(19) Europäisches Patentamt
European Patent Office
Office européen des brevets

(11) Publication number: 0 518 313 A2

(12) # EUROPEAN PATENT APPLICATION

(21) Application number: 92109812.5

(22) Date of filing: 11.06.92

(51) Int. Cl.5: C12N 15/51, C07K 15/00, C12Q 1/70, A61K 39/29, G01N 33/576

A request for correction of the sequence listing has been filed pursuant to Rule 88 EPC. A decision on the request will be taken during the proceedings before the Examining Division (Guidelines for Examination in the EPO, A-V, 2.2).

(30) Priority: 11.06.91 JP 139268/91
12.07.91 JP 172794/91
07.10.91 JP 287008/91
16.12.91 JP 332329/91
20.04.92 JP 99957/92

(43) Date of publication of application:
16.12.92 Bulletin 92/51

(84) Designated Contracting States:
AT BE CH DE DK ES FR GB GR IT LI LU MC NL PT SE

(71) Applicant: MITSUBISHI KASEI CORPORATION
5-2, Marunouchi 2-chome Chiyoda-ku
Tokyo 100(JP)

(72) Inventor: Seki, Makoto
10-406, Machida-cooptown, 2-11-3, Ogawa
Machida-shi, Tokyo-to(JP)
Inventor: Honda, Yoshikazu
2-3-16, Nishimikado
Kamakura-shi, Kanagawa-ken(JP)
Inventor: Takahashi, Kazuhiro
10-201, Popuragaoka-coop, 2-10-1, Naruse
Machida-shi, Tokyo-to(JP)
Inventor: Murakami, Tomoko
3-2-9, Narusegaoka
Machida-shi, Tokyo-to(JP)
Inventor: Teranishi, Yutaka
1-14-2, Takane, Sagamihara-shi
Kanagawa-ken(JP)
Inventor: Hayashi, Norio
1-1-112, Suimeidai
Kawanishi-shi, Hyogo-ken(JP)

(74) Representative: Hansen, Bernd, Dr. Dipl.-Chem. et al
Hoffmann, Eitle & Partner Patent- und Rechtsanwälte Arabellastrasse 4 Postfach 81 04 20
W-8000 München 81(DE)

(54) **Gene of hepatitis C virus or fragment thereof, polypeptide encoded by the same.**

(57) A novel gene encoding HCV polypeptide including HCV-associated antigen, a polypeptide encoded by the same, an expression vector containing the gene, a transformant transformed with the vector, a process for producing HCV polypeptide by culturing the transformant, which polypeptide produced by the process is useful for serodiagnosis of hepatitis C and for the preparation of vaccine against hepatitis C virus.

EP 0 518 313 A2

## Field of the invention

This invention relates to an isolated gene encoding a polypeptide of human hepatitis C virus (hereinafter, referred to as HCV), or a fragment thereof, and a polypeptide encoded thereby.

## Background of the invention

Hepatitis viruses A, B and D have been identified and the serodiagnosis for each virus has been established before the present invention. However, there was at least one hepatitis whose cause remained unknown (Digestive Diseases and Sciences, 31: 122S-132S (1986); and Seminars in Liver Diseases, 6: 56-66 (1986)).

Serodiagnosis for hepatitis A virus (HAV) or hepatitis B virus (HBV) has been established and clinically employed since middle of 1970's, which revealed that most of the blood-transfusion-associated hepatitis are caused by unknown pathogen(s) other than viruses capable of growing in hepatocytes, such as HAV or HBV. The hepatitis caused by unknown pathogen was designated as "non-A, non-B hepatitis (NANBH)". In the United States, the incidence of hepatitis following the "transfusion" is about 1 to 10% of the total patients undergone transfusion, and more than 90% of said post-transfusional hepatitis are reported to be NANBH (Jikken Igaku, 8,3: 15-18 (1990)). In Japan, about 200,000 patients, corresponding to about 10 - 20% of those undergone transfusion, are suffering from the post-transfusional hepatitis every year, and about 95% of them are diagnosed as NANBH. Furthermore, about 300,000 people are diagnosed as sporadic hepatitis every year and about 40 to 50% of them are considered to be NANBH. There are also epidemic NANBH in Japan. Although infectious route for NANBH has not been established in contrast with hepatitis A or B, it is likely different from those for hepatitis A and B (Jikken Igaku, 8, 3: 13-14 (1990)).

Chiron Corp. (May, 1988) has succeeded in isolating a gene fragment of a virus responsible for NANBH by means of an unique technique quite different from conventional ones and designated said virus as hepatitis C virus (HCV). Many researchers followed the work and sequenced the entire gene encoding both of non-structural and structural proteins of HCV (Shimotohno et al., Proc. Natl. Acad. Sci. USA, 87: 9524-9526 (1990); and Takamizawa et al., Journal of Virology 65, 3: 1105-1113 (1991)).

Many Patent Applications directed to HCV gene have been done so far, for example, European Patent Publication Nos.318216, 388232, 398748, 419182, 450931, 464287, 463848, 468657, WO 91/01376, WO 91/15516 and British Patent No.2239245, and the like.

Chiron corp. and Ortho, Inc. have developed an Enzyme-linked Immunosorbent Assay (ELISA) for HCV and a kit therefor, using a recombinant antigen (clone C100-3) which was obtained by transforming yeast cells with an expression plasmid encoding a fused peptide comprising a human super-oxide dismutase and a 363 amino acid polypeptide encoded by a gene encoding a region from NS3 to NS4 encoding a part of non-structural protein, growing transformants under a condition to allow the transformants express said fused peptide (WO No. 89/04669; and European Patent Publication No.318216).

The Japanese Welfare Ministry (KOSEI-SHO), leading other nations in the world, decided to introduce said Chiron's kit into the screening and detection of anti-HCV antibody and the import thereof started on December 26, 1989. From the next day, the Japanese Red Cross began screening for anti-HCV antibody in blood offered by volunteers using the kit. About 1.7 million of people are estimated to undergo blood transfusion yearly. Before the screening, the incidence of post-transfusional hepatitis among them was about 12.3% (about 173,000), and thereafter, it reduced to about 3%.

As an outstanding and critical feature, the variability of HC-associated antigen is often suggested. For instance, homology in amino acid and base sequences between C100-3 clone and a clone obtained in Japan was reported to be about 80%. The difference is only 20% though, it can affect on the accuracy of the detection of HCV. In another aspects, homology between HC-associated antigens varies from a region to region, for example, it is only 70% regarding the all or a part of NS1, NS2, NS3 and NS5 regions (according to the designation by Chiron Corp.), which indicates that some substances may be overlooked by Chiron's kit. As is often the case with virus, especially that has RNA genome, a genetic mutation occurs at a high frequency, which leads to a change in antigen determinant sites. As a result, HC-associated antigen presented by antigen-presenting cells in serum and antibody raised against it also change in the course of disease.

The another kit provided by Ortho, Inc. is accurate in detecting anti-HCV antibodies raised during a restricted period of disease, that is, antibodies raised during a period while the disease progresses from an acute stage to a chronic stage, which begins about 24.7 weeks after the infection (SAISHIN IGAKU, 45, 12: 2331-2336 (19909); IGAKUNOAYUMI, 151: 892-896). Thus, the Ortho's kit is not effective for detecting antibodies raised against all the HC-associated antigens throughout the disease, especially those presented during acute and chronic stages.

Accordingly, an assay method useful for the detection of any anti-HCV antibodies raised against various HC-associated antigens exist in serum of a patient throughout the disease has been needed. HCV is detectable in hepatocytes of patients in various phases of disease, including acute, chronic hepatitis, hepatocirrhosis, and hepatoma. Recently, interests are concentrated on the pathogenetic relationship between HCV infection and hepatoma because about 50 - 60% of patients of hepatoma are HCV positive. Although the pathogenetic relationship between HCV infection and hepatoma has not been established, it is generally accepted that there are some relationships between chronic hepatitis, hepatocirrhosis and hepatoma. Therefore, screening for anti-HCV antibody in serum of a subject susceptible to them may helpful for preventing such serious diseases. Thus, more accurate and efficient screening method, as well as serodiagnosis, is strongly desired to prevent HCV-related diseases. For this purpose, a reagent and a kit having a extended utility in, for example, the assay of serum of a variety of subjects including carriers of HCV without manifesting symptoms, patients suffering from HC of various stages, such as acute, chronic, or progressed hepatitis, is necessary. As the number of HCV-infected patients increases, the% of HCV-contamination in blood offered by volunteers increases. This causes a serious problem all over the world, for instance, the% of HCV positive blood in total blood offered by volunteers is about 10, 0.8, 1.5, and 1.2% in Japan, USA, Italy, and Spain, respectively (TANPAKUSITU, KAKUSAN, KOSO, 36, 10: 1679-1691 (1991)-). However, there are no effective methods for treating HCV infection, and therefore a method for detection of HCV in serum of suspected subjects is strongly required to prevent HCV-related diseases.

## Summary of the Invention

As previously mentioned, HCV gene is extremely liable to vary and subtypes of HCV should differ to a great extent at various sites including surface antigenic sites and others responsible for the determination of significant features of HCV protein. As these mutated viruses induce hepatitis C of different symptoms depending on the type when infected to human, variants low in homology are considered to be different from each other.

In this regard, the present inventors isolated plural viruses which differ from each other in terms of amino acid and DNA sequences from sera of patients of HC (HC patients).

The present invention was established by isolating a novel hepatitis C virus, separating RNA encoding viral protein, converting RNA into cDNA using reverse transcriptase, and cloning and sequencing the resultant DNA. When the isolated DNA was transformed into host cells after ligating to an appropriate expression vector, transformants expressed HC-associated antigen.

DNA obtained by transcribing the RNA of HCV encodes recombinant antigen which is immunochemically the same as HCV-associated antigen. Therefore, for the purpose of the invention, the terms "cDNA", "DNA" and "gene" are used interchangeably, as far as they encode the same protein(s) or antigens as those encoded by RNA gene of HCV. As one of skill will easily appreciate, a DNA fragment encoding an epitopic site of HCV-associated antigen is also useful to produce a polypeptide capable of specifically reacting with anti-HCV antibody in the same manner as intact HC-associated antigen. Therefore such a DNA fragment is also useful for the purpose of the invention.

Thus, the present invention provides an isolated gene of a novel hepatitis C virus and a fragment thereof. The HCV gene and its fragment of the invention are useful for the development of a diagnostic method which is more accurate and effective than conventional ones in the detection of antibodies raised against a wide range of HCVs which have been hardly detected before the present invention. The gene and fragments thereof are also useful for the preparation of a novel vaccine.

In another aspects of the invention, an in vitro screening system for a substance capable of specifically suppressing or controlling a proteolytic processing of a precursor polypeptide of HCV can be obtained. The screening system can be established by analyzing viral protease intimately. The analysis can be carried out by synthesizing a + strand of RNA from a double-stranded DNA containing HCV-originated protease gene and its adjoining regions, producing a polypeptide comprising viral protease in vitro, characterizing said protease as to the activity, specificity, function, and the like.

In another aspects, the present invention provides an in vivo screening system for the substance capable of suppressing the processing of viral precursor protein. The screening can be carried out using a transformant, for example, eucaryotic cells such as animal cells which have been transformed with DNA fragment of the invention, and can express a precursor polypeptide of HCV and process the product intracellularly.

Specifically, the present invention provides an isolated DNA (gene) encoding all or a part of polypeptide having an amino acid sequence of any of SEQ ID NO 1 to 43, 64 to 75 and 101 to 104, or fragment thereof.

The present invention further provides polypeptide having all or a part of amino acid sequence of any of SEQ ID. Nos. 1 to 43, 64 to 75 and 101 to 104.

The polypeptides of the invention have an ability to immunochemically and specifically react with antiserum obtained from patients suffering from hepatitis C.

As the amino acid and DNA sequences of polypeptide of HCV are determined, it is easy to obtain active derivatives of viral protein which falls within the scope of the present invention by conventional methods which leads to the insertion, deletion, replacement or addition of amino acids without changing the specific reactivity with sera from patients suffering from hepatitis C. This can be conducted by, for example, a site-specific mutagenesis of DNA.

Therefore, the present invention also provides active derivatives of HCV protein obtained by conventional methods, and DNA fragments encoding it, which can immunochemically react with antiserum raised against HC-associated antigen.

In this regard, the present invention provides a polypeptide fragment having a modified amino acid sequence derived from polypeptides having amino acid sequence of SEQ ID NO 1 - 104, and being capable of reacting with serum of HC patients with a different specificity, for example, those claimed in Claims 119, 121, 123, 125, 127, 129, 131 - 136, 138, 140 - 154, 157 - 179, 185 - 199, wherein the modification has been done by deletion, insertion, modification or addition of amino acid(s) subject to that the ability to react with antiserum from HC patients is not decreased:

Furthermore, the present invention provides an expression vector which comprises DNA shown by either of SEQ ID NO 1 to 43, 64 to 75 and 101 to 104 or a fragment thereof and has an ability to allow a host cell to express said DNA when transformed into the same.

The present invention also provides a transformant transformed with the expression vector.

The present invention further provides a method for preparing HCV protein or HCV-associated antigen by culturing a transformant in a medium and recovering the product from the cultured broth.

## Definition

For the purpose of the invention, the following terms are defined below.

HCAg: HCV- or HC-associated antigen. For the purpose of the invention, as hepatitis C is caused by HCV, the terms "HC-associated antigen" and "HCV-associated antigen" are used exchangeably.

HCAb: antibody raised against HCV-associated antigen.

HCV protein or HCV polypeptide: protein or polypeptide encoded by HCV gene.

HCV gene: generally, it is RNA gene of HCV. However, for the purpose of the invention, it refers to gene encoding HCV polypeptide or protein encoded by RNA gene. Therefore, the terms "gene", "cDNA", and "DNA" obtained from RNA gene are used exchangeably.

Recombinant HCAg: a product (protein or polypeptide, including glycosylated ones) produced in host cells transformed by DNA of the invention and is capable of immunochemically reacting with HCAb.

Recombinant polypeptide: polypeptide expressed by host cells transformed by HCV gene of the invention.

HC patient: a patient suffering from hepatitis C.

## Detailed Description of the Invention

### [1] Gene Encoding Core-envelope Region

#### (1) Preparation of cDNA clone of SEQ ID NO 1 - 12 and sequencing thereof

The cDNA clones of SEQ ID NO 1 - 12 which encode a novel polypeptide of core-envelope region of HCV protein were cloned from serum from HC patients as follows.

The cloning and sequencing of cDNA encoding HCV polypeptide can be carried out using any of known methods. However, it is hardly accomplished by known "Okayama-Berg" or "Gubler-Hoffman" method because the content of HCV in serum is only a slight amount and HCV gene is liable to variation. The present inventors succeeded in the cloning of gene from a slight amount of serum as will be hereinafter described in Example 1. Briefly, it was conducted by extracting nucleic acids from a serum of a patient suffering from HC. It is preferable to use serum showing OD value of 3.5 on a screening kit of Ortho. Before the extraction, it is desirable to add tRNA or polyribonucleoside to the serum as a carrier for viral RNA. For the purpose of the invention, tRNA is preferable because the degradation of RNA can be easily detected, at least after the addition of tRNA, by monitoring the existence of a sufficient amounts of tRNA having an intact length on electrophoresis.

The resultant RNA is converted into cDNA using transcriptase in the presence of an appropriate oligonucleotide primer. The cDNA is then cloned and amplified by modified polymerase chain reaction (PCR) (Saiki et al., Nature 324: 126 (1986)) in the presence of a pair of primers. Although commercially available random primers can be used in the PCR, synthetic primers having the following base sequences are suitable for the present invention.

Synthetic Primers

5′ 3′

S1:CTCCACCATAGATCACTCC(SEQ ID NO:105)

S2:AGGTCTAGTAGACCGTGC(SEQ ID NO:106)

S3:AGGAAGACTTCCGAGCGG(SEQ ID NO:107)

S4:CGTGAACTATGCAACAGGG(SEQ ID NO:108)

AS1:ACCGCTCGGAAGTCTTCC(SEQ ID NO:109)

AS2:GGGCAAGTTCCCTGTTGC(SEQ ID NO:110)

AS3:GCTGGATTCTCTGAGACG(SEQ ID NO:111)

PCR can be conducted under appropriate conditions, for example, those described in Example 2 using the first complemental DNA (1st cDNA) as a template. The condition may vary depending on the primers used such as base sequence or combination, length to be amplified, or the like. Examples of pair of primers are: S1 - AS1; S1 - AS2; S1 - AS3; S2 - AS1; S2 - AS2; S2 - AS3; S3 - AS2; S3 - AS3; and S4 - AS3.

The minimum amount of serum required for the cloning described in Example 2 [2] varies depending on the content of virus in serum used, however, it may be about 5 to 7 μl when the serum shows OD 3 or more on aforementioned Ortho's kit. The base sequence of cDNA obtained using random primer in the synthesis of the 1st strand cDNA was the same as that of cDNA obtained using antisence primer which was designed and synthesized (Example 8).

Thus, a region (clone N1-1) was obtained by two different methods. Three clones independently obtained from a serum of a patient using random primers are shown as a clone of SEQ ID NO 1. When synthetic DNA (S1 and AS1) was used as primers, two clones of three clones obtained independently have the same base sequence as that of SEQ ID NO 1 and one clone had a modified base sequence wherein three amino acids of SEQ ID NO 1 were changed, i.e., No.345 A to C, No.332 A to T, and No. 95 A to C, which shows that there are more than one virus in one patient.

The resultant DNA fragment is then subjected to the determination of base sequence. Generally, three clones obtained independently are employed and the base sequence of the both strands are determined to obtain an entire base sequence. The base sequence is conveniently determined using a fluorescence sequencer GENESIS 2000 (DUPONT) according to the protocol attached thereto. Alternatively, a conventional subcloning can be used when the DNA fragment consists more than 180 nucleotides or contains a region which is hardly determined by fluorescence sequencer.

Thus obtained base sequences are shown in SEQ ID NO 1 to 12.

For the purpose of the invention, a part of base sequences may be changed, for example, No. 345 A to C, No. 332 A to T, and No. 95 A to C, respectively.

(2) Expression of Polypeptides Encoded by Clones

DNA fragments of SEQ ID NO 1 to 12 can be used to produce a recombinant HCAg by constructing an expression vector containing DNA encoding a clone, by inserting the DNA into a known expression vector at an appropriate site of the vector, downstream from a promoter, using a well known method per se, and introducing the expression vector harboring the DNA into a host cell such as Escherichia coli cell, yeast cell, animal cell or the like according to the method known to one of skill, culturing the transformant in a medium under an appropriate condition, and recovering a product from the cultured broth.

The present invention can be accomplished using any expression vectors which have a promoter at an appropriate site to direct the expression of a DNA encoding HCV polypeptide or a fragment thereof. Expression vectors preferably contain promoter, ribosome binding (SD) sequence, gene encoding HCV polypeptide, transcription termination factor, and a regulator gene.

Expression vectors functional in microorganisms such as Escherichia coli, Bacillus subtilis or the like will preferably comprise promoter, ribosome binding (SD) sequence, HCV-associated-protein-encoding gene, transcription termination factor, and a regulator gene.

Examples of promoters include those derived from Escherichia coli or phages such as tryptophane synthetase (trp), lactose operon (lac), λphage $P_L$ and $P_R$, $T_5$ early gene $P_{25}$, $P_{26}$ promoter and the like. These promoter may have modified or designed sequence for each expression vector such as pac promoter.

Although the SD sequence may be derived from Escherichia coli or phage, a sequence which has been designed to contain a consensus sequence consisting of more than 4 bases, which is complementary to the sequence at the 3' terminal region of 16S ribosome RNA, may also be used.

The transcription termination factor is not essential. However, it is preferable that an expression vector contains a $\rho$-independent factor such as lipoprotein terminator, trp operon terminator or the like.

Preferably, these sequences required for the expression of the a gene encoding HCAg originated from HCV are located, in an appropriate expression plasmid, in the order of promoter, SD sequence, said gene and transcription termination factor from 5' to 3' direction.

Typical example of expression vectors is commercially available pKK233-2 (Pharmacia). However, a series of plasmids pGEX (Pharmacia), which are provided for the expression of a fused protein, are also employable for the expression of HCAg-encoding gene of the present invention.

A suitable host cell such as Escherichia coli can be transformed with an expression vector comprising a DNA of the invention by any of known methods such as protocol provided by TOYOBO Japan (Example 3).

The cultivation of the transformants can be carried out using any of well known procedures in literatures such as Molecular Cloning, 1982, and the like. The cultivation is preferably conducted at a temperature from about 28°C to 42°C.

Expression vectors used for transforming other host cells, such as those derived from insects or animals including mammals, consist of substantially the same elements as those described in the above. However, there are certain preferable factors as follows.

When insect cells are used, a commercially available kit MAXBAC™ is employed according to the teaching of the supplier (MAXBAC™ BACULOVIRUS EXPRESSION SYSTEM MANUAL VERSION 1.4). In this case, it is desirable to make a modification to reduce the distance between the promoter of polyhedrin gene and the initiation codon so as to improve the expression of the gene.

When animal cells are used as hosts, expression vectors preferably contain SV40 early promoter, SV40 late promoter, apolipoprotein E gene promoter, or the like. Specifically, known expression vectors such as pKCR (Proc. Natl. Acad. Sci. USA, 78: 1528 (1981)), pBPV MT1 (Proc. Natl. Acad. Sci. USA, 80: 398 (1983)-), or the like may be employed after minimum modification, for example, an insertion of cloning site as described in a literature (Nature, 307: 604 (1984)), so that the resultant vectors maintain essential functions to serve as expression vectors.

Animal cells usable in the present invention are CHO cell, COS cell, mouse L cell, mouse C127 cell, mouse FM3A cell and the like.

The recombinant polypeptide expressed by host cells such as microorganisms including E. coli, insect cells and animal cells can be recovered from the cultured broth by known methods and identified by, for example, immunoreactions between the expressed product and antiserum obtained from HC patients using a conventional method such as Western blot analysis.

As the result, polypeptides having amino acid sequence of SEQ ID NO 1 to 12 were obtained as expression products of cDNA obtained from serum of HC patients and identified as HCAg. Among them, polypeptides having 191 amino acid sequence from No. 1 to No. 191 of SEQ ID NO 5, 6 and 8 are assumed to be polypeptides which were expressed and cleaved by processing in insect cells. Thus, the sequences of SEQ ID NO 5, 6 and 8 comprise: from No. 174 to No. 188 (region A), amino acid sequence containing mainly hydrophobic amino acids having a large side chain of higher molecular weight; and at Nos. 189 and 191, alanine, a residue having a small side chain of lower molecular weight. This pattern of sequence keeps a feature of signal region which is recognized by signal peptidase in animal (including insect) cell. The 5'- and 3'- regions of said sequence contain many variations in amino acid sequence resulting from variations in base sequence due to the replacement of a part of said sequence, when compared with known HCV genes cloned before the present invention. However, the regions A and B contain less variations which indicates that the polypeptide may be cleaved at C-terminus of the No. 191 alanine by signal peptidase.

Polypeptide having amino acid sequence from No. 1 to No.191 of SEQ ID NO 5, 6, and 8 is assumed to be core or matrix protein on the basis of the homology between said amino acid sequence and a known sequence of core or matrix region of viral protein of Japanese encephalitis virus or yellow fever virus. The polypeptide comprising said 191 amino acid sequence is herein referred to as "core protein" or "core region".

Polypeptides having 18 amino acid sequence from No. 1 to No.18 of SEQ ID NO 1, 9, 10, 11 and 12, 34 amino acid sequence from No. 40 to No.73 of SEQ ID NO 3, and 35 amino acid sequence from No. 81 to No.115 of SEQ ID NO 3 are relatively highly hydrophilic and highly homologous to polypeptides having amino acid sequences deduced from known HCV genes cloned by Chiron, Shimotohno or Takamizawa (ibid) and are useful as HCV-associated antigenic peptide in diagnosis and/or for the preparation of vaccine.

Polypeptides having 18 amino acid sequence from No. 40 to No.57 of SEQ ID NO 4, and 12 amino acid sequence from No. 240 to No.251 of SEQ ID NO 4 are relatively highly hydrophilic and extremely low in homology with polypeptides having amino acid sequences deduced from known HCV genes cloned before the present invention and are useful as HCV-associated antigenic peptide in diagnosis. These polypeptides can be produced by chemical synthesis, as well as by DNA recombinant technique.

Furthermore, a polypeptide having 115 amino acid sequence from No. 1 to No. 115 of SEQ ID NO 3, corresponding to an epitopic region of core protein, and a polypeptide having 191 amino acid sequence from No. 1 to No.191 of SEQ ID NO 3, corresponding to the total region of core protein, can be produced in large scale by DNA recombinant technique and are useful as diagnostic reagent and/or vaccine.

Among them, a polypeptide having 192 amino acid sequence from No. 31 to No. 222 of SEQ ID NO 4 is assumed to be a polypeptide which was expressed and cleaved by processing in insect cells. Thus, the sequence of SEQ ID NO 4 comprises: from No. 13 to No. 29 (region A), amino acid sequence containing mainly hydrophobic amino acids having a side chain of higher molecular weight; at No. 30, alanine, a residue having a side chain of lower molecular weight; from No. 210 to No. 221 (region B), amino acid sequence containing mainly hydrophobic amino acids having a side chain of higher molecular weight; at No. 222, glycine, a residue having a side chain of lower molecular weight. This pattern of sequence keeps a feature of signal region which is recognized by signal peptidase in animal (including insect) cell.

The 5'- and 3'- regions of said sequence contain many variations in amino acid sequence resulting from those in base sequence due to the replacement of base sequences, when compared with known HCV gene cloned before the present invention. However, the regions A and B contain less variations, indicating that the polypeptide may be cleaved by signal peptidase at C-terminus of the No. 30 alanine.

The polypeptide is assumed to be envelope protein of HCV or a fragment thereof on the basis of the low homology between the base sequence encoding said polypeptide and a known base sequence which encodes a corresponding region of HCV protein. The polypeptide comprising said 192 amino acid sequence is herein referred to as "M-gp35 protein" or "M-gp35 region".

Polypeptides having 19 amino acid sequence from No. 134 to No.152 of SEQ ID NO 4, 17 amino acid sequence from No. 223 to 239 of SEQ ID NO 4, and 18 amino acid sequence from No. 92 to No.109 of SEQ ID NO 4 are relatively highly hydrophilic and highly homologous to polypeptides having amino acid sequences deduced from known HCV genes and are useful as HCV-associated antigenic peptide in diagnosis and/or for the preparation of vaccine.

Polypeptides having 18 amino acid sequence from No. 40 to No.54 of SEQ ID NO 4, and 12 amino acid sequence from No. 240 to No.251 of SEQ ID NO 4 are relatively highly hydrophilic and extremely low in homology with polypeptides having amino acid sequences deduced from known HCV genes cloned before the present invention and are useful as HCV-associated antigenic peptide for diagnosis.

These polypeptides can be produced by chemical synthesis, as well as by DNA recombinant technique.

Furthermore, polypeptides having 76 amino acid sequence from No. 31 to No. 106 of SEQ ID NO 4; and 36 amino acid sequence from No. 134 to No. 169 of SEQ ID NO 4, which correspond to an epitopic region of M-gp35 protein can be produced in large scale by DNA recombinant technique and are useful as diagnostic reagent and/or vaccine.

[2] Gene Encoding NS1(gp70) Region

(1) Preparation of cDNA clone of SEQ ID NO 13 - 27 and sequencing thereof

The cDNA clones of SEQ ID NO 13 - 27 which encode a novel polypeptide of NS1(gp70) region of HCV protein and fragments thereof were cloned from serum from HC patients as follows.

The cloning and sequencing of cDNA encoding HCV polypeptide can be carried out using any of known methods. However, it is hardly accomplished by known "Okayama-Berg" or "Gubler-Hoffman" method because the content of HCV in serum is only a slight amount and HCV gene is liable to variation. The present inventors succeeded in the cloning of gene from a slight amount of serum as will be hereinafter described in Example 1. Briefly, it was conducted by extracting nucleic acids from a serum of a patient suffering from HC. It is preferable to use serum showing OD value of 3.5 on a screening kit of Ortho. Before the extraction, it is desirable to add tRNA or polyribonucleoside to the serum as a carrier for viral RNA. For the purpose of the invention, tRNA is preferable because the degradation of RNA can be easily detected, at least after the addition of tRNA, by monitoring the existence of a sufficient amounts of tRNA having an intact length on electrophoresis.

The resultant RNA is converted into cDNA using transcriptase in the presence of an appropriate oligonucleotide primer. The cDNA is then cloned and amplified by modified PCR (Saiki et al., Nature 324: 126 (1986)) in the presence of a pair of primers. Although commercially available random primers can be used in the above procedures, synthetic primers having the following base sequences are suitable for the present invention.

Synthetic Primers for Cloning of HCV Gene

5′ 3′

MS122:GAGGCCGTGAACTGCGATGA(SEQ ID NO:112)

MS148:TTCTCTAAGGTGGCNTCNGCNTG(SEQ ID NO:113)

MS157:CCGGACGCGTTGAANCTNTGNGT(SEQ ID NO:114)

MS123:CATCCAGGTACAACCGAACCA(SEQ ID NO:115)

MS146:AACACACGGCCGCCNCANGGNAA(SEQ ID NO:116)

MS156:CCGGATCCCACAAGCCGTNGTNGA(SEQ ID NO:117)

In the above sequences, the letter "N" refers to inosine. The above sequences are only illustrative and these base sequences are not critical. They can be modified by replacing nucleotide(s) with other(s), or deleting or inserting nucleotide(s). The replacement may be preferably introduced within 10 bases from 5' terminus involving 1 to several nucleotides, more preferably, within 5 bases involving less than 5 nucleotides. The deletion may occur in the 5' terminal region involving 4 to 5 nucleotides, preferably, within several bases from the 5' terminus involving a few nucleotides. In case of insertion, it may be an addition of 8 to 12, preferably 5 to 6, more preferably, a few nucleotides in 5' terminal region.

PCR can be conducted under appropriate conditions, for example, those described in Example 9 using the first complemental DNA (1st cDNA) as a template. The condition may vary depending on the primers used such as base sequence or combination, length to be amplified, or the like. Examples of pair of primers are : MS122 - MS123; MS157 - MS156; and MS148 - MS146. The resultant cDNA is then inserted into an appropriate site of a cloning vector such as at SmaI site of pUC19. A cloning vector harboring the DNA fragment is subjected to the determination of base sequence. Generally, three clones obtained independently are employed and the base sequence of the both strands are determined to obtain an entire base sequence. The sequence is conveniently determined using a fluorescence sequencer GENESIS 2000 (DUPONT) according to the protocol attached thereto. Alternatively, a conventional subcloning can be used when the DNA fragment consists more than 180 nucleotides or contains a region which is hardly determined by fluorescence sequencer.

Thus obtained base sequences of DNA fragments are shown in SEQ ID NO 13 to 27.

Clones N19-1, 2, 3, N27-1, 2 and 3 were obtained from serum of a patient N, and clones H19-2, 4, 10, Y19-4, 6 and 7 were obtained independently from patients H, and Y, respectively. Clones MX24-4, 5 and 13 were obtained from a pool comprising sera from multiple patients.

Clones of SEQ ID NO 13 to 15 were obtained using primers MS157 and MS156 represent the same region of HCV gene designated as N27. Clones of SEQ ID NO 16 to 24 were obtained using primers MS122 and MS123 also represent the same region of HCV gene designated as N19, and clones of SEQ ID NO 25 to 27 were obtained using primers MS148 and MS146 also represent the same region of HCV gene designated as MX24. The comparison between base sequence of each clone and that of known HCV gene (Kato et al., Proc. Natl. Acad. Sci. USA, 87: 9524-9528 (1990); and Takamizawa et al., Journal of Virology, 65,3: 1105-1113 (1991)) indicates that clones align on HCV gene in the other of N27, N19 and MX24, from 5' to 3'. As there are overlapping regions between clones, these regions were used to ligate clones each other as will be hereinafter described.

(2) Ligation of Clones of SEQ ID NO 13 to 27

CDNA clones obtained from serum of HC patients shown by sequences of SEQ ID NO 13 to 27 were ligated in the following manners.

1) Ligation of Clones of SEQ ID NO 13 to 15, and Clones of SEQ ID NO 16 to 24

Each clones of SEQ ID NO 13 to 15 was cleaved at MluI site at Nos. 330 to 335 from 5' terminus of base sequences of SEQ ID NO 13 to 15 and ligated to the MluI site at No. 71 from 5' terminus of base sequences of SEQ ID NO 16 to 24 by ligation reaction to yield 27 clones having a DNA fragment which comprises, at 5' region, a DNA fragment from clone N27-1, 2 or 3, and at 3' region, a DNA fragment from clone N19-1, 2, 3, clone H19-2, 4, 10, clone Y19-4, 6 or 7. Thus, by the ligation reaction between N27-3 and N19-1, a clone N27N19-1 of SEQ ID NO 28 was obtained.

2) Ligation of Clones of SEQ ID NO 16 to 24, and Clones of SEQ ID NO 25 to 27

Each clones of SEQ ID NO 16 to 24 was ligated to each clones of SEQ ID NO 25 to 27 by PCR. There obtained 54 kinds of clones. Thus, 27 clones have a DNA fragment which encodes either of polypeptides which contain: from N-terminus (amino-terminus) to amino acid No.131, amino acid sequence comprising 131 amino acid residues from N- to C-termini of SEQ ID NO 16 to 24, and from amino acid No. 132 to C-terminus (carboxy-terminus), amino acid sequence from No. 16 to C-terminus of SEQ ID NO 25 to 27. Thus, a clone obtained by the ligation reaction between N19-1 and MX24-4 is the clone N19MX24A-1 of SEQ ID NO 29. The others have a DNA fragment which encodes either of polypeptides which contain: at N-terminal region, amino acid sequence from N-terminus to amino acid No. 116 of SEQ ID NO 16 to 24, and from amino acid No. 117 to C-terminus (carboxy-terminus), amino acid sequence comprising 209 amino acid sequence from N- to C-termini of SEQ ID NO 25 to 27. Thus, a clone obtained by the ligation reaction between N19-1 and MX24-4 is the clone N19MX24B-1 of SEQ ID NO 30.

3) Ligation of Clones of SEQ ID NO 13 to 27

Each clones of SEQ ID NO 13 to 15 was cleaved at MluI site at base Nos. 330 to 335 from 5' terminus of base sequences of SEQ ID NO 13 to 15 and ligated to the MluI site at base Nos. 71 to 76 from 5' of the base sequences of the above (2), 2) by ligation reaction to yield clones of N27MX24 series. Thus, a clone obtained by the ligation reaction between N27-3 and N19MX24A-1 is the clone N27MX24A-1 of SEQ ID NO 31 and a clone obtained by the ligation reaction between N27-3 and N19MX24B-1 is the clone N27MX24B-1 of SEQ ID NO 32.

On the basis of the homology between the amino acid sequence of the clone and that reported previously (Kato et al., Proc. Natl. Acad. Sci. USA, 88: 5547-5551 (1991); and Hijikata et al., in: Congress of Association of Japan Molecular Biology, November 29, 1990), the clone N27N19MX24A-1 proved to be the entire region of a gene encoding gp70 polypeptide reported by Kato et al. Thus, polypeptide comprising amino acids from Nos. 46 to 395 of SEQ ID NO 31 and 32 corresponds to the gp protein presented by Kato et al.

On the other hand, polypeptide comprising amino acids from Nos. 1 to 45 and polypeptide comprising amino acids from No. 46 to the C-terminus of SEQ ID NO 13 to 15 correspond to the C-terminal region of gp 35 polypeptide and N-terminal region of gp70 polypeptide reported by Hijikata et al, respectively. Further, the amino acid sequence from No. 46 to C-terminus of SEQ ID NO 13 to 15 corresponds to a sequence from N-terminus to amino acid No.67 of gp70 reported by Kato et al, and the amino acid sequence from N- to C-termini of SEQ ID NO 16 to 24 corresponds to a sequence from amino acid Nos. 42 to 172 of gp70 and represents a fragment of gp70 protein presented by Kato et al.

The amino acid No.1 of SEQ ID NO 25 to 27 corresponds to the amino acid No.158 from N-terminus of a sequence reported by Hijikata et al., and also the amino acid No. 350 of SEQ ID NO 25 to 27 corresponds to C-terminal amino acids of gp70 reported by Shimotohno et al., and a polypeptide comprising amino acids from Nos. 194 to C-terminus of SEQ ID NO 25 to 27 corresponds to the N-terminal region of non-structural protein of HCV (NS2).

The ligation products prepared in 2) code all or a part of gp70 polypeptide reported by Hijikata et al. For example, the polypeptide from amino acid Nos. 46 to 395 of SEQ ID NO 31 or 32 corresponds to gp70 protein of Hijikata et al. Although a protein expressed from a HCV gene encoding a polypeptide from amino acid Nos. 46 to 395 of SEQ ID NO 31 or 32 is gp70 protein, said expression product is herein referred to as M-gp70, in contrast with gp70 reported by Hijikata et al in: Congress of Association of Japan Molecular Biology, November 29, 1990.

(3) Expression of Polypeptides Encoded by Clones

DNA fragments obtained in the above 1) and 2) can be used to produce a recombinant HCAg by constructing an expression vector containing DNA encoding a clone, by inserting the DNA into a known expression vector at an appropriate site of the vector, downstream from a promoter, using a well known method per se, and introducing the expression vector harboring the DNA into a host cell such as Escherichia coli cell, yeast cell or the like according to the method known to one of skill, culturing the transformant in a medium under an appropriate condition, and recovering a product from the cultured broth.

The present invention can be accomplished using any expression vectors which have a promoter at an appropriate site to direct the expression of a DNA encoding HCV polypeptide or a fragment thereof. Expression vectors preferably contain promoter, ribosome binding (SD) sequence, gene encoding HCV polypeptide, transcription termination factor, and a regulator gene.

Expression vectors functional in microorganisms such as Escherichia coli, Bacillus subtilis or the like will preferably comprise promoter, ribosome binding (SD) sequence, HCV-associated-protein-encoding gene, transcription termination factor, and a regulator gene.

Examples of promoters include those derived from Escherichia coli or phages such as tryptophane synthetase (trp), lactose operon (lac), λphage $P_L$ and $P_R$, $T_5$ early gene $P_{25}$, $P_{26}$ promoter and the like. These promoter may have modified or designed sequence for each expression vector such as pac promoter.

Although the SD sequence may be derived from Escherichia coli or phage, a sequence which has been designed to contain a consensus sequence consisting of more than 4 bases, which is complementary to the sequence at the 3' terminal region of 16S ribosome RNA, may also be used.

The transcription termination factor is not essential. However, it is preferable that an expression vector contains a $\rho$-independent factor such as lipoprotein terminator, trp operon terminator or the like.

Preferably, these sequences required for the expression of a gene encoding HCAg originated from HCV are located, in an appropriate expression plasmid, in the order of promoter, SD sequence, said gene and transcription termination factor from 5' to 3' direction.

Typical example of expression vectors is commercially available pKK233-2 (Pharmacia). However, a series of plasmids pGEX (Pharmacia), which are provided for the expression of fused protein, are also employable for the expression of HCAg-encoding gene of the present invention.

A suitable host cell such as Escherichia coli can be transformed with an expression vector comprising a DNA of the invention by any of known methods such as protocol provided by TOYOBO Japan as described in Example 10.

The cultivation of the transformants can be carried out using any of well known procedures in literatures such as Molecular Cloning, 1982, and the like. The cultivation is preferably conducted at a temperature from about 28°C to 42°C.

Expression vectors used for transforming other host cells, such as those derived from insects, consist of substantially the same elements as those described in the above. However, there are certain preferable factors as follows.

When insect cells are used, a commercially available kit, MAXBAC™ is employed according to the teaching of the supplier (MAXBAC™ BACULOVIRUS EXPRESSION SYSTEM MANUAL VERSION 1.4). In this case, it is desirable to make a modification to reduce the distance between the promoter of polyhedrin gene and the initiation codon so as to improve the expression of the gene.

A clone of the invention can be inserted into an expression vector for procaryotic cells such as E.coli or eucaryotic cells such as animal cells after modifying the DNA sequence to bring it in conformity with a frame of initiation codon of said vector. Alternatively, an initiation codon is added at the 5' terminus of DNA so as to an appropriate translational frame can be produced. The term "a translational frame" of a clone refers to a frame of a base sequence in which bases are described as triplets capable of encoding amino acid sequence as illustrated in SEQ ID NO 13 to 32.

The recombinant polypeptide expressed by host cells such as microorganisms including E. coli and insect cells and animal cells can be recovered from the cultured broth by known methods and identified by, for example, immunoreactions between the expressed product and antiserum obtained from HC patients using a conventional method such as Western blot analysis.

E. coli cells transformed with any of clones obtained in the above (1) and (2) express polypeptides encoded thereby as a single polypeptide without cleaving between regions gp35, gp70 and NS2.

When any of clones obtained in the above (1) and (2) is expressed in insect cells, the expressed polypeptide is cleaved between regions gp35, gp70 and NS2. Thus, clone N27MX24A-1 or N27MX24B-1 was transformed into insect or animal cells, polypeptide M-gp70 derived from each clone N27MX24A-1 or N27MX24B-1 was expressed as a glycoprotein after processing.

The following polypeptides comprising amino acid sequence of SEQ ID NO 31 or 32 are relatively highly hydrophilic and homologous to amino acid sequence deduced from known HCV gene cloned before the present invention: a polypeptide consisting of 13 amino acids from amino acid Nos. 143 to 155; a polypeptide consisting of 21 amino acids from amino acid Nos. 171 to 191 subject to that it contains at least amino acids from Nos. 182 to 187; a polypeptide consisting of 14 amino acids from amino acid Nos. 202 to 215 subject to that it contains at least amino acids from Nos. 202 to 209; a polypeptide consisting of 13 amino acids from amino acid Nos. 244 to 256; and a polypeptide consisting of 21 amino acids from amino acid Nos. 299 to 319.

The M-gp70 is a glycoprotein which located adjacent to C-terminus of envelope protein (M-gp35) on HCV gene and contains potential trans-membrane region. These facts lead to an assumption that all or a part of gp70, whose function has not been established yet, may be a part of envelope protein. On the basis of this assumption, the above five kinds of polypeptide fragments, as well as a polypeptide consisting of 106 amino acids from Nos. 109 to 214 and that consisting of 92 amino acids of amino acid sequence from Nos. 233 to 324 of SEQ ID NO 31 or 32, which include said fragments, are useful as vaccine.

Furthermore, the following polypeptides which comprise amino acid sequence of SEQ ID NO 31 or 32 and are expected to be epitopic region of M-gp70 are also useful as vaccine: a polypeptide consisting of 10 amino acids from amino acid Nos. 252 to 261 subject to that it contains at least amino acids from Nos. 252 to 256; a polypeptide consisting of 34 or less than 34 amino acids from amino acid Nos. 250 to 283 subject to that it contains at least amino acids from Nos. 273 to 279; a polypeptide consisting of 20 amino acids from amino acid Nos. 77 to 96; a polypeptide consisting of 18 amino acids from amino acid Nos. 306 to 323; and a polypeptide consisting of 16 amino acids from amino acid Nos. 122 to 137.

The following polypeptides comprising amino acid sequence of SEQ ID NO 31 or 32 are relatively highly hydrophilic and low in homology with amino acid sequences deduced from known HCV genes cloned before the present invention: a polypeptide consisting of 12 amino acids from amino acid Nos. 136 to 147 subject to that it contains at least amino acids from Nos. 136 to 142; a polypeptide consisting of 27 amino acids from amino acid Nos. 45 to 71 subject to that it contains at least amino acids from Nos. 53 to 69; a polypeptide consisting of 9 amino acids from amino acid Nos. 193 to 201.

These polypeptides can be produced by chemical synthesis, as well as by DNA recombinant technique.

Furthermore, a polypeptide having 106 amino acid sequence from Nos. 109 to 214 and a polypeptide having 92 amino acid sequence from Nos. 233 to 324 of SEQ ID NO 31 or 32 can be produced in large scale by DNA recombinant technique.

[3] Genes Encoding NS2 - NS4 Regions

(1) Preparation of cDNA clone of SEQ ID NO 33 - 44 and sequencing thereof

The cDNA clones of SEQ ID NO 33 - 44 which encode a novel polypeptide of NS2 - NS4 regions of HCV protein and fragments thereof were cloned from serum from HC patients as follows.

The cloning and sequencing of cDNA encoding HCV polypeptide can be carried out using any of known methods. However, it is hardly accomplished by known "Okayama-Berg" or "Gubler-Hoffman" method because the content of HCV in serum is only a slight amount and HCV gene is liable to variation. The present inventors succeeded in the cloning of gene from a slight amount of serum as will be hereinafter described in Example 1. Briefly, it was conducted by extracting nucleic acids from a serum of a patient suffering from HC. It is preferable to use serum showing OD value of 3.5 on a screening kit of Ortho. Before the extraction, it is desirable to add tRNA or polyribonucleoside to the serum as a carrier for viral RNA. For the purpose of the invention, tRNA is preferable because the degradation of RNA can be easily detected, at least after the addition of tRNA, by monitoring the existence of a sufficient amounts of tRNA having an intact length on electrophoresis.

The resultant RNA is converted into cDNA using transcriptase in the presence of an appropriate oligonucleotide primer. The cDNA is then cloned and amplified by modified PCR (Saiki et al., Nature 324: 126 (1986)) in the presence of a pair of primers. Although commercially available random primers can be used in the above PCR, synthetic primers having the following base sequences are suitable for the present invention.

Synthetic Primers for Cloning of HCV Gene

5′ 3′

MS49:GACATGCATGTCATGATGTA(SEQ ID NO:118)

MS88:GGCTGCAGCCGGTTCATCCACTGCAC(SEQ ID NO:119)

MS100:GCGGATCCTGCTTCGCCCAGAAGGTC(SEQ ID NO:120)

MS132:GACACATGTGTTGCAGTCGATC(SEQ ID NO:121)

MS152:CGGTCCNAGNAGTATCTCNTTNCC(SEQ ID NO:122)

MS158:ATGGGCCCGGGNGANAGNAGNCTCCCCCTNCTNTC(SEQ ID NO:123)

MS48:GGCTATACCGGCGACTTCGA(SEQ ID NO:124)

MS86:GCGGATCCGGCCTCACCCACATAGATG(SEQ ID NO:125)

MS97:GCGGATCCTCCACCTCCATCGTG(SEQ ID NO:126)

MS135:CTGCTGTCGCCCNGNCCCAT(SEQ ID NO:127)

MS151:ATCACGTGGGGNGCAGANACNGC(SEQ ID NO:128)

MS155:TGTGCCTGNTTNTGGATGATG(SEQ ID NO:129)

In the above sequences, the letter "N" refers to inosine. The above sequences are only illustrative and these base sequences are not critical. They can be modified by replacing nucleotide(s) with other(s), or deleting or inserting nucleotide(s). The replacement may be preferably introduced within 10 bases from 5' terminus involving 1 to several nucleotides, more preferably, within 5 bases involving less than 5 nucleotides. The deletion may occur in the 5' terminal region involving 4 to 5 nucleotides, preferably, within several bases from the 5' terminus involving a few nucleotides. In case of insertion, it may be an addition of 8 to 12, preferably 5 to 6, more preferably, a few nucleotides in 5' terminal region. Primers MS86, MS97, and MS100 contains additional 8 nucleotides encoding a restriction site at 5' terminus (MS88: 5' GGCTGCAG 3'; MS86, MS97 and MS100: 5' GCGGATCC 3'), however, these are not critical for the isolation of the desired DNA fragments.

PCR can be conducted under appropriate conditions, for example, those described in Example 15 using the first complemental DNA (1st cDNA) as a template. The condition may vary depending on the primers used such as base sequence or combination, length to be amplified, or the like. Examples of pair of primers are : MS48 - MS49; MS86 - MS100; MS97 - MS88; MS135 - MS132; MS155 - MS152; and MS151 - MS158. The resultant cDNA is then inserted into an appropriate site of a cloning vector such as at SmaI site of pUC19. A cloning vector harboring the DNA fragment is subjected to the determination of base sequence. Generally, three clones obtained independently are employed and the base sequence of the both strands are determined to obtain an entire base sequence. The sequence is conveniently determined using a fluorescence sequencer GENESIS 2000 (DUPONT) according to the protocol attached thereto. Alternatively, a conventional subcloning can be used when the DNA fragment consists more than 180 nucleotides or contains a region which is hardly determined by fluorescence sequencer. Thus obtained base sequences of DNA fragments are shown in SEQ ID NO 33 - 39, 44 - 55, and 103 and 104.

Clones N13-1, N15-1, N16 and N23 were obtained from serum of a patient N, clone O26 from patient O, clone U16-4 from patient U, and clone MX25 from a pool comprising sera from multiple patients. Clone of SEQ ID NO 37 obtained from primers MS48 and MS49 and clones of SEQ ID. Nos. 53 to 55 represent the same region on HCV gene (N16 region). The region of clones of SEQ ID NO 44 to 46 obtained using primers MS155 and MS152 on HCV gene was designated as MX25 region. In the same manner, regions of clones of SEQ ID NO 47 to 49, and regions of clones of SEQ ID NO 50 to 52, each obtained by primers MS151 and MS158, or MS135 and MS132, were designated as O26 and N23 regions, respectively.

Clones N13-1, N15-1, O15-1, and O15-2 of SEQ ID NO 38, 39, 103, and 104, which were obtained by primers MS86 and MS100, MS97 and MS88, were designated as regions N13 and N15.

The comparison between base sequences of each clone and known HCV gene (Kato et al., Proc. Natl. Acad. Sci. USA, 87:9524-9528 (1990); and Takamizawa et al., Journal of Virology, 65,3: 1105-1113 (1991)) indicates that clones align in the other of MX25, O26, N23, N16, N13 and N15, from 5' to 3' on the gene.

The clone N16 of SEQ ID NO 36 was obtained by isolating independently three plasmids containing DNA fragment of N16 region, and determining the entire base sequence of DNA fragment originated from HCV.

As there are overlapping region between clones, these regions were used to ligate clones each other.

Clones are highly homologous though, they are distinguishable from each other in terms of nucleotide and amino acid sequences (e.g., clones of SEQ ID NO 33, 34 and 35), which indicates that one patient may carry more than one HCVs at the same time. It is generally accepted that core protein is well conserved even in HCV. When core-protein-encoding gene was cloned in the same manner as that used for the cloning of gene encoding HCV polypeptide, few variations were observed between clones. Among regions on HCV gene, MX25, O26, N23 and N16 regions, especially MX25 region, appear to be highly liable compared with core-protein-encoding region and upperstream region thereof.

(2) Ligation of Clones of SEQ ID NO 33 to 39

cDNA clones obtained from serum of HC patients shown by sequences of SEQ ID NO 33 to 37 and 39 were ligated in the following manners.

1) Ligation of Clone N16 of SEQ ID NO 36 and clone N15-1 of SEQ ID NO 39

The ligation was conducted at restriction sites common to both clones. Thus, clone 16 was digested with restriction enzyme to cleave at the BstEII site located at nucleotide Nos. 576-582 of SEQ ID NO 36 and ligated to the BstEII site of clone N15-1 at Nos. 114 to 120 of SEQ ID NO 39 to obtain a DNA fragment consisting of DNA fragments from clones N16 and N15-1 from 5' to 3'. The resultant clones are summarized as clone of SEQ ID NO 41.

2) Ligation of Clones MX25 (SEQ ID NO 33) and O26 (SEQ ID NO 34)

Clones MX25 and O26 were ligated by PCR. By this procedure, multiple DNA fragments encoding different polypeptides were obtained, for example, a DNA fragment encoding a polypeptide which comprises, at the N-terminal region, 284 amino acids of N- to C-termini of SEQ ID NO 33 and, from amino acid No. 285 to the C-terminus, amino acids from No. 32 to the C-terminus of SEQ ID NO 34; a DNA fragment encoding a polypeptide which comprises, at the N-terminal region, amino acid residues of N-terminus to amino acid No. 252 of SEQ ID NO 33 and, from amino acid No. 253 to the C-terminus, 174 amino acid residues from N- to C- termini of SEQ ID NO 34. Thus obtained fused clones were inclusively shown in SEQ ID NO 40.

Clones of SEQ ID NO 36 and 39 or clones of DEQ ID NO 37 and 39 can be ligated by PCR and the resultant clone is shown in SEQ ID NO 41 together with a base sequence obtained in the above 1). Clone MX25 of SEQ ID NO 33 and clone O26 of SEQ ID NO 34, both of which contain different DNA fragments from those used in the above, were ligated to give multiple DNA fragments having different base sequences. These base sequences are summarized in SEQ ID NO 40.

3) Ligation of Clones of SEQ ID NO 35 and 41

Ligation of clones N23 and N16N15 can be conducted in the same manner as the above 1) to obtain various clones which are designated as N23N15 of SEQ ID NO 42 inclusively. The following illustrative DNA fragments were obtained: a DNA fragment encoding a polypeptide comprising, at the N-terminal region, 307 amino acid residues from N-to C-termini of SEQ ID NO 35 (clone N23) and, from amino acid No. 308 to the C-terminus, amino acids from No.17 to C-terminus of SEQ ID NO 41; a DNA fragment encoding a polypeptide comprising, at the N-terminal region, amino acids from N-terminus to amino acid No. 291 of SEQ ID NO 33 and from amino acid No. 292 to the C-terminus, 477 amino acid residues from N- to C-termini of SEQ ID NO 41.

4) Ligation of Clones of SEQ ID NO 40 and 42

Ligation of clones MX25O26 and N23N15 can be conducted in the same manner as the above 1) to obtain clones shown in SEQ ID NO 43 inclusively.

The protease activity of viral protein of Flavivirus, a related strain of HCV, exists in the N-terminal domain of non-structural protein of said virus (see, Proc. Natl. Acad. Sci. USA, 87: 8898-8902 (1990)). It is likely that the protease activity of HCV protein also exists in the presumed N-terminal region, NS3. It was confirmed that clone MX25N15 comprises the known entire amino acid sequence encoded by HCV gene (Kato et al., Proc. Natl. Acad. Sci. USA, 87: 9524-9528 (1990)), and a region responsible for the protease activity reported by Hijikata et al (in: Congress of Japan Cancer Association (NIHON Gan-Gakkai (1991)).

Although the both of N- and C-termini of NS3 domain of HCV protein had not been established, it can be presumed to be a region between amino acid Nos. 276 and 884 of SEQ ID NO 43 (clone MX25N15) on the basis of the primary structure of regions to be cleaved by protease and hydrophilic and hydrophobic patterns of Flavivirus protein, referring to a literature (Houghton et al. Hepatology, 14, 2: 381-388 (1991)). The presumed NS3 region of clone MX25N15 is hereinafter referred to as MK/NS3 region.

In the same manner, the NS2 region was presumed to be a polypeptide region between amino acid Nos. 3 and 275 of SEQ ID NO 43 (clone MX25N15) and 40 (clone MX25O26). The presumed NS2 region is hereinafter referred to as MK/NS2 region.

(3) Expression of Polypeptides Encoded by Clones

DNA fragments obtained in the above 1) and 2) can be used to produce a recombinant HCAg by constructing an expression vector containing DNA encoding a clone, by inserting the DNA into a known expression vector at an appropriate site of the vector, downstream from a promoter, using a well known method per se, and introducing the expression vector harboring the DNA into a host cell such as Escherichia coli cell, yeast cell, animal cell or the like according to the method known to one of skill, culturing the transformant in a medium under an appropriate condition, and recovering a product from the cultured broth.

The present invention can be accomplished using any expression vectors which have a promoter at an appropriate site to direct the expression of a DNA encoding HCV polypeptide or a fragment thereof. Expression vectors preferably contain promoter, ribosome binding (SD) sequence, gene encoding HCV polypeptide, transcription termination factor, and a regulator gene.

Expression vectors functional in microorganisms such as Escherichia coli, Bacillus subtilis or the like will preferably comprise promoter, ribosome binding (SD) sequence, HCV-associated-protein-encoding gene, transcription termination factor, and a regulator gene.

Examples of promoters include those derived from Escherichia coli or phages such as tryptophane synthetase (trp), lactose operon (lac), λphage $P_L$ and $P_R$, $T_5$ early gene $P_{25}$, $P_{26}$ promoter and the like. These promoter may have modified or designed sequence for each expression vector such as pac promoter.

Although the SD sequence may be derived from Escherichia coli or phage, a sequence which has been designed to contain a consensus sequence consisting of more than 4 bases, which is complementary to the sequence at the 3' terminal region of 16S ribosome RNA, may also be used.

The transcription termination factor is not essential. However, it is preferable that an expression vector

contains a $\rho$-independent factor such as lipoprotein terminator, trp operon terminator or the like.

Preferably, these sequences required for the expression of a gene encoding HCAg originated from HCV are located, in an appropriate expression plasmid, in the order of promoter, SD sequence, HCV-associated-protein-encoding gene and transcription termination factor from 5' to 3' direction.

Typical example of expression vectors is commercially available pKK233-2 (Pharmacia). However, a series of plasmids pGEX (Pharmacia), which are provided for the expression of fused protein, are also employable for the expression of HCAg-encoding gene of the present invention.

A suitable host cell such as Escherichia coli can be transformed with an expression vector comprising a DNA of the invention by any of known methods such as protocol provided by TOYOBO Japan as described in Example 16.

The cultivation of the transformants can be carried out using any of well known procedures in literatures such as Molecular Cloning, 1982, and the like. The cultivation is preferably conducted at a temperature from about 28°C to 42°C.

Expression vectors used for transforming other host cells, such as those derived from insects or animals including mammals, consist of substantially the same elements as those described in the above. However, there are certain preferable factors as follows.

When insect cells are used, a commercially available kit, MAXBAC™ is employed according to the teaching of the supplier (MAXBAC™ BACULOVIRUS EXPRESSION SYSTEM MANUAL VERSION 1.4). In this case, it is desirable to make a modification to reduce the distance between the promoter of polyhedrin gene and the initiation codon so as to improve the expression of the gene.

When animal cells are used as hosts, expression vectors preferably contain active-type promotor from adenovirus EIA gene (ZOKUSEIKAGAKU JIKKEN KOZA I, IDENSHI KENKYU-HO II, 189-190, 1986), SV40 early promoter, SV40 late promoter, apolipoprotein E gene promoter, SR$\alpha$ promotor (Molecular and Cellular Biology, 8, 1, 466-472, 1988) or the like. Specifically, known expression vectors such as pKCR (Proc. Natl. Acad. Sci. USA, 78: 1528 (1981)) or a derivative thereof, pBPV MT1 (Proc. Natl. Acad. Sci. USA, 80: 398 (1983)), which prepared by modifying pKCR maintaining its essential functions, pBPV MT1 (Proc. Natl. Acad. Sci. USA, 80: 398 (1983)), or the like may be employed.

Animal cells usable in the present invention are CHO cell, COS cell, mouse L cell, mouse C127 cell, mouse FM3A cell and the like.

A clone of the invention can be inserted into an expression vector for procaryotic cells such as E.coli or eucaryotic cells such as animal cells after modifying the DNA sequence to bring it in conformity with a frame of initiation codon of said vector. Alternatively, an initiation codon is added at the 5' terminus of DNA so as to an appropriate translational frame can be produced. The term "a translational frame" of a clone refers to a frame of a base sequence in which bases are described as triplets capable of encoding amino acid sequence as illustrated in SEQ ID NO 33 to 43.

The recombinant polypeptide expressed by the host cells such as microorganisms including E. coli, insect cells and animal cells can be recovered from the cultured broth by known methods and identified by, for example, immunoreactions between the expressed product and antiserum obtained from HC patients using a conventional method such as Western blot analysis.

Hydrophilic study and prediction of higher-order structure of protein, the following peptide fragments contained in a polypeptide having amino acid sequence of SEQ ID NO 43 appeared to be highly hydrophilic and can take so-called "turn structure" not $\alpha$-helix or $\beta$-sheet structure in high probability. Therefore, these fragments possibly represent antigen determinants, or can contain at least one antigen determinant of HCAg. Although the higher-order structure in serum and the specific reactivity of each fragment are not established, it can be concluded that the following peptide fragments are highly reactive with antiserum raised against HCV-associated antigens. A polypeptide consisting of 19 amino acids from amino acid Nos. 247 to 265 of SEQ ID NO 43; a polypeptide consisting of 8 to 25 amino acids subject to that it contains at least 8 amino acids from Nos. 300 to 307; a polypeptide consisting of 13 to 25 amino acids subject to that it contains at least 13 amino acids from Nos. 410 to 428; a polypeptide consisting of 10 amino acids from Nos. 283 to 292; a polypeptide consisting of 14 amino acids from Nos. 477 to 490; a polypeptide consisting of 14 amino acids from Nos. 498 to 512; a polypeptide consisting of 12 amino acids from Nos. 538 to 550; a polypeptide consisting of at least 21 amino acids from Nos. 747 to 767; a polypeptide consisting of at least 12 amino acids from Nos. 841 to 852; a polypeptide consisting of at least 12 amino acids from Nos. 867 to 878; a polypeptide consisting of 8 to 25 amino acids subject to that it contains at least 8 amino acids from Nos. 665 to 672; and a polypeptide consisting of 15 amino acids from Nos. 315 to 327.

The above polypeptide fragments can be obtained by means of chemical synthesis, as well as DNA recombinant technique.

Other polypeptide fragments of clone of SEQ ID NO 43, that is, a polypetides containing the entire or a part of a polypeptide consisting of 266 amino acids from Nos. 461 to 726; a polypeptide consisting of 74 amino acids from Nos. 477 to 550; a polypeptide consisting of 42 amino acids from Nos. 963 to 1004; and a polypeptide consisting of 45 amino acids from Nos. 283 to 327, can be prepared in a large scale by recombinant DNA technique.

[4] Gene Encoding NS4 to NS5 Regions

(1) Preparation of cDNA clone of SEQ ID NO 64 - 75 and sequencing thereof

The cDNA clones of SEQ ID NO 64 - 75 which encode a novel polypeptide of NS4 to NS5 regions of HCV protein and fragments thereof were cloned from serum from HC patients as follows.

The cloning and sequencing of cDNA encoding HCV polypeptide can be carried out using any of known methods. However, it is hardly accomplished by known "Okayama-Berg" or "Gubler-Hoffman" method because the content of HCV in serum is only a slight amount and HCV gene is liable to variation. The present inventors succeeded in the cloning of gene from a slight amount of serum as will be hereinafter described in Example 1. Briefly, it was conducted by extracting nucleic acids from a serum of a patient suffering from HC. It is preferable to use serum showing OD value of 3.5 on a screening kit of Ortho. Before the extraction, it is desirable to add tRNA or polyribonucleoside to the serum as a carrier for viral RNA. For the purpose of the invention, tRNA is preferable because the degradation of RNA can be easily detected, at least after the addition of tRNA, by monitoring the existence of a sufficient amounts of tRNA having an intact length on electrophoresis.

The resultant RNA is converted into cDNA using transcriptase in the presence of an appropriate oligonucleotide primer. The cDNA is then cloned and amplified by means of polymerase chain reaction (PCR) (Saiki et al., Nature 324: 126 (1986)) in the presence of a pair of primers. Although commercially available random primers can be used in the PCR, synthetic primers having the following base sequences are suitable for the present invention.

Synthetic Primers for Cloning of HCV Gene

5' 3'

MS126:GGTGAGCATGGAGGTGACCAC(SEQ ID NO:130)

MS119:TCATCCTCCTCCGCTCGAAGC(SEQ ID NO:131)

MS161:GTGGACGCCTTNGCCTTCATNTC(SEQ ID NO:132)

MS162:ACGGATGTCNTTCTCNGTNAC(SEQ ID NO:133)

MS121:GGCGGAATTCCTGGTCATAGCCTCCGTGAA(SEQ ID NO:134)

MS163:GGGGNATGGCCTATTGGCCTG(SEQ ID NO:135)

MS127:GGCATGTGGGCCCAGGGGAGG(SEQ ID NO:136)

MS118:TGTGAGCCCGAACCGGATGT(SEQ ID NO:137)

MS159:GTGGTANTCCTGGACTCNTTNGA(SEQ ID NO:138)

MS160:ACTACCGNGACGTGCTNAANGA(SEQ ID NO:139)

MS120:TGGGGATCCCGTATGATACCCGCTGCTTTG(SEQ ID NO:140)

MS174:ATTGTCAGATCTACGGGGCCACTT(SEQ ID NO:141)

MS175:GCAAGCTTAAAAAAAAAAAAGGGGGATGGCCTATTGGCCTGGA(SEQ ID NO:142)

In the above sequences, the letter "N" refers to inosine. The above sequences are only illustrative and these base sequences are not critical. They can be modified by replacing nucleotide(s) with other(s), or deleting or inserting nucleotide(s). The replacement may be preferably introduced within 10 bases from 5' terminus involving 1 to several nucleotides, more preferably, within 5 bases involving less than 5 nucleotides. The deletion may occur in the 5' terminal region involving 4 to 5 nucleotides, preferably, within several bases from the 5' terminus involving a few nucleotides. In case of insertion, it may be an addition of 8 to 12, preferably 5 to 6, more preferably, a few nucleotides in 5' terminal region.

PCR can be conducted under appropriate conditions, for example, those described in Example 21 using the first complemental DNA (1st cDNA) as a template. The condition may vary depending on the primers used such as base sequence or combination, length to be amplified, or the like. Examples of pair of primers are : MS127 - MS126; MS118 - MS119; MS159 - MS161; MS160 - MS162; MS120 - MS163; and MS120 - MS121. The resultant cDNA is then inserted into an appropriate site of a cloning vector such as at SmaI site of pUC19. A cloning vector harboring the DNA fragment is subjected to the determination of base sequence. Generally, three clones obtained independently are employed and the base sequence of the both strands are determined to obtain an entire base sequence. The sequence is conveniently determined using a fluorescence sequencer GENESIS 2000 (DUPONT) according to the protocol attached thereto. Alternatively, a conventional subcloning can be used when the DNA fragment consists more than 180 nucleotides or contains a region which is hardly determined by fluorescence sequencer. Thus obtained base sequences of DNA fragments are shown in SEQ ID NO 64 - 69, and 76 - 100.

Clones N22-1, 3, N17-1, 2, 3, N29-1, 2, 3, N18-2, 3 and 4 were obtained from serum of a patient N, clone H22-3, 8, 9, H17-1, 3, H18-1, 2 and 3 from patient H, clone O28-1, 2, 4, O30-2, 3 and 4 from patient O. It is generally accepted that region encoding core protein or its 5' region generally contain few variations and are well conserved even in HCV. When regions encoding core protein and/or a upstream region thereof were cloned in the same manner as the above, variations were hardly observed between clones. In the present invention, clones obtained from a same region on HCV gene were highly homologous though, they proved to be DNA fragments distinguishable from each other in terms of nucleotide and amino acid sequences. This indicates that one patient may carry more than one HCVs at the same time.

From the above fact, N22, N17, O28, N18, N29, and O30 regions assumed to be highly liable compared with core-protein-encoding region and upstream region thereof.

Region on HCV gene which corresponds to each clone was designated as follows. The region of clones N22-1, 3, H-22, 3, 8 and 9 obtained using primers MS127 and MS126 was designated as N22. In the same manner, the regions on HCV gene corresponding to clones N17-1, 2, 3, H17-1 and 3 obtained using primers MS118 and MS119, clones O28-1, 2 and 4 obtained using primers MS159 and MS161, clones N29-1, 2 and 3 obtained using primers MS160 and MS162, clones N18-2, 3, 4, H18-1, 2 and 3 obtained using primers MS120 and MS121, clones O30-2, 3 and 4 obtained using primers MS120 and MS163 were designated as N17, O28, N29, N18 and O30, respectively.

The comparison between base sequences of each clone and known HCV gene (Kato et al., Proc. Natl. Acad. Sci. USA, 87:9524-9528 (1990); and Takamizawa et al., Journal of Virology, 65,3: 1105-1113 (1991)) indicates that clones align in the other of N22, N17, O28, N29, and O30, from 5' to 3' on the gene (N18 is included in O30 region).

There are overlapping region between clones, which were used to ligate clones each other.

(2) Ligation of Clones of SEQ ID NO 64 to 69

Regions N22, N17, O28, N29, and O30 of clone N15 (see, the above [3]), a cDNA clone obtained from serum of HC patients, were ligated in the following manners.

1) Ligation of N17 and O28 Regions

The ligation of N17 and O28 regions can be conducted using, for instance, clones N17-3 (SEQ ID NO 81) and O28-1 (SEQ ID NO 86). The ligation was carried out by PCR. Thus, about equimolar of DNA fragments (as template) of clones N17-3 and O28-1 in a solution were subjected to PCR in the presence of primers MS118 and MS161 to yield clone 1728.

2) Ligation of N29 and N18 Regions

In the same manner as the above 1), N29 and N18 regions were ligated using clones N29-1 (SEQ ID NO 89) and N18-4 (SEQ ID NO 92), and primers MS160 and MS121 to yield clone 2918.

3) Ligation of Regions N 17 to N18

PCR was carried out using DNA fragments of clones 1728 and 2918, primers MS118 and MS121 to yield clone 1718 which contains clones N17, O28, N29, N18 from 5' to 3'. The clone 1718 was cloned into SmaI site of PUC19 to give plasmid 1718 in which EcoRI site from pUC19, clone N17-3 and N18 regions on HCV gene are aligned in this order from 5' to 3'.

4) Ligation of Regions N 22 to N17

In the same manner as the above 1), DNA fragments of clones N22-1 (SEQ ID NO 76) and N17-3 (SEQ ID NO 81) were ligated by PCR using primers MS127 and MS119 to yield a DNA fragment designated as clone 2217 which contains N22 and N17 from 5' to 3'. The clone 2217 was cloned into SmaI site of pUC19 in the same manner as the above 3) to give plasmid 2217 in which EcoRI site located at 5' terminus.

5) Ligation of Clones 2217 and 1718

Upon digestion with restriction enzyme XbaI, clone 1718 is cleaved at one site. Plasmid pUC1718 was digested with XbaI and a DNA fragment comprising DNA fragment of clone 1718 and XbaI site of pUC19 was isolated. The DNA fragment derived from clone 2217 was inserted into XbaI site of pUC2217 such that the XbaI site in N17 region of pUC2217 and XbaI site from pUC19 are ligated to obtain plasmid pUC2218.

6) Ligation of N15 Region and O30 Region Corresponding to 3' Terminal Region of HCV Gene

An example of DNA fragment of O30 region is clone O30-3 of SEQ ID NO 98. Plasmid pUCO30 contains the DNA fragment of O30-3 at SmaI site of pUC19 in the order of, from 5' to 3', EcoRI site and clone O30-3. Plasmid pUCN15 contains a DNA fragment of HCV gene, clone N15 (see, [3]), forwardly at SmaI site of pUC19 in the order of, from 5' to 3', EcoRI site and clone N15.

Plasmid pUCO30 was cleaved by SacI and blunt ended, which was followed by the cleavage at another cloning site, HindIII, to isolate a DNA fragment derived from HCV gene, which was ligated to a DNA fragment from plasmid pUCN15 which was digested with XbaI, blunt ended, digested with HindIII to yield plasmid pUC15-30. Taking advantage of the fact that said plasmid pUC15-30 has only one site which can be cleaved by restriction enzymes BglII and HindIII, it was subjected to PCR using a primer MS174 having a BglII site in sequence derived from clone O30-3 in order to add poly U at 3' terminus of clone O30-3.

PCR was conducted using, as a template, pUC15-30 and primers MS174 and MS175. PCR fragment was then digested with BglII and HindIII and the resultant fragment ligated to a BglII-HindIII fragment of pUCO30 containing the vector fragment of pUCO30 to obtain plasmid pUC15-30U having polyU attached to the 3' terminus of clone O30-3.

7) Ligation of N15 to O30 Regions

There is an ApaI site within a region common to N15 and N22 regions. There is an ApaI site within a region common to N18 and O30. A DNA fragment isolated from pUC2218 with ApaI was inserted into ApaI site of pUC15-30U appropriately to obtain plasmid pUC1530U.

The ligated N15 to O30 regions encodes amino acid sequence which is highly homologous to amino acid sequence of NS5, a part of non-structural protein NS4 of Flavivirus, a related strain of HCV. It was also confirmed that said region is homologous to a sequence encoding a part of NS4 region AND NS5 region by comparison with a known sequence of HCV gene disclosed by aforementioned Chiron, Shimotohno, or Takamizawa. As a conclusion, clone disclosed in Seq. Lis. represents DNA sequence assumed to be NS4 and NS5 regions of HCV gene. The clone was then inserted into an expression plasmid to produce polypeptide encoded by said clone. The polypeptide was then evaluated as to the ability to react immunologically with antiserum of HC patients.

(3) Expression of Polypeptides Encoded by Clones

DNA fragments obtained in the above (2) can be used to produce a recombinant HCAg by constructing an expression vector containing DNA encoding a clone, by inserting the DNA into a known expression vector at an appropriate site of the vector, downstream from a promoter, using a well known method per se, and introducing the expression vector harboring the DNA into a host cell such as Escherichia coli cell, yeast cell, animal cell or the like according to the method known to one of skill, culturing the transformant in a medium under an appropriate condition, and recovering a product from the cultured broth.

The present invention can be accomplished using any expression vectors which have a promoter at an appropriate site to direct the expression of a DNA encoding HCV polypeptide or a fragment thereof. Expression vectors preferably contain promoter, ribosome binding (SD) sequence, gene encoding HCV polypeptide, transcription termination factor, and a regulator gene.

Expression vectors functional in microorganisms such as Escherichia coli, Bacillus subtilis or the like will preferably comprise promoter, ribosome binding (SD) sequence, HCV-associated-protein-encoding gene, transcription termination factor, and a regulator gene.

Examples of promoters include those derived from Escherichia coli or phages such as tryptophane synthetase (trp), lactose operon (lac), λphage $P_L$ and $P_R$, $T_5$ early gene $P_{25}$, $P_{26}$ promoter and the like. These promoter may have modified or designed sequence for each expression vector such as pac promoter.

Although the SD sequence may be derived from Escherichia coli or phage, a sequence which has been designed to contain a consensus sequence consisting of more than 4 bases, which is complementary to the sequence at the 3' terminal region of 16S ribosome RNA, may also be used.

The transcription termination factor is not essential. However, it is preferable that an expression vector contains a ρ-independent factor such as lipoprotein terminator, trp operon terminator or the like.

Preferably, these sequences required for the expression of a gene encoding HCAg originated from HCV are located, in an appropriate expression plasmid, in the order of promoter, SD sequence, said gene and transcription termination factor from 5' to 3' direction.

Typical example of expression vectors is commercially available pKK233-2 (Pharmacia). However, a series of plasmids pGEX (Pharmacia), which are provided for the expression of fused protein, are also employable for the expression of HCAg-encoding gene of the present invention.

A suitable host cell such as Escherichia coli can be transformed with an expression vector comprising a DNA of the invention by any of known methods such as protocol provided by TOYOBO Japan as described in Example 22.

The cultivation of the transformants can be carried out using any of well known procedures in literatures such as Molecular Cloning, 1982, and the like. The cultivation is preferably conducted at a temperature from about 28° C to 42° C.

Expression vectors used for transforming other host cells, such as those derived from insects or animals including mammals, consist of substantially the same elements as those described in the above. However, there are certain preferable factors as follows.

When insect cells are used, a commercially available kit, MAXBAC™ is employed according to the teaching of the supplier (MAXBAC™ BACULOVIRUS EXPRESSION SYSTEM MANUAL VERSION 1.4). In this case, it is desirable to make a modification to reduce the distance between the promoter of polyhedrin gene and the initiation codon so as to improve the expression of the gene.

When animal cells are used as hosts, expression vectors preferably contain active-type promotor from adenovirus EIA gene (ZOKUSEIKAGAKU JIKKEN KOZA I, IDENSHI KENKYU-HO II, 189-190, 1986), SV40 early promoter, SV40 late promoter, apolipoprotein E gene promoter, SRα promotor (Molecular and Cellular Biology, 8, 1, 466-472, 1988) or the like. Specifically, known expression vectors such as pKCR (Proc. Natl. Acad. Sci. USA, 78: 1528 (1981)) or a derivative thereof, pBPV MT1 (Proc. Natl. Acad. Sci. USA, 80: 398 (1983)), which prepared by modifying pKCR maintaining its essential functions, pBPV MT1 (Proc. Natl. Acad. Sci. USA, 80: 398 (1983)), or the like may be employed.

Animal cells usable in the present invention are CHO cell, COS cell, mouse L cell, mouse C127 cell, mouse FM3A cell and the like.

A clone of the invention can be inserted into an expression vector for procaryotic cells such as E.coli or eucaryotic cells such as animal cells after modifying the DNA sequence to bring it in conformity with a frame of initiation codon of said vector. Alternatively, an initiation codon is added at the 5' terminus of DNA so as to an appropriate translational frame can be produced. The term "a translational frame" of a clone refers to a frame of a base sequence in which bases are described as triplets capable of encoding amino acid sequence as illustrated in SEQ ID NO 64 to 75.

The recombinant polypeptide expressed by host cells such as microorganisms including E. coli, insect cells and animal cells can be recovered from the cultured broth by known methods and identified by, for example, immunoreactions between the expressed product and antiserum obtained from HC patients using a conventional method such as Western blot analysis.

Hydrophilic study and prediction of higher-order structure of protein, the following peptide fragments contained in a polypeptide having amino acid sequence of SEQ ID NO 75 appeared to be highly hydrophilic and can take so-called "turn structure" not α-helix or β-sheet structure in high probability. Therefore, these fragments possibly represent antigen determinants, or can contain at least one antigen determinant of HCAg. Although the higher-order structure in serum and the specific reactivity of each fragment are not established, it can be concluded that the following peptide fragments are highly reactive with antiserum raised against HCV-associated antigens. A polypeptide comprising at least 20 amino acids from amino acid Nos. 324 to 343; a polypeptide comprising at least 14 amino acids from Nos. 356 to 369; a polypeptide comprising at least 18 amino acids from Nos. 584 to 601; a polypeptide comprising 10 amino acids from Nos. 588 to 597; a polypeptide consisting of 10 amino acids from Nos. 620 to 629; a polypeptide consisting of 18 amino acids from Nos. 901 to 918; and a polypeptide which contains at least any of those described in the above and comprises 25 or less amino acids of SEQ ID NO 75.

The above polypeptide fragments can be obtained by means of chemical synthesis, as well as DNA recombinant technique.

Other polypeptide fragments of SEQ ID NO 75, that is, a polypetides containing the entire or a part of a polypeptide consisting of 74 amino acids from Nos. 413 to 486; a polypeptide consisting of 997 amino acids from Nos. 415 to 1411; a polypeptide consisting of 74 amino acids from Nos. 655 to 728; a polypeptide consisting of 98 amino acids from Nos. 858 to 955; a polypeptide consisting of 92 amino acids from Nos. 1009 to 1100; a polypeptide consisting of 66 amino acids from Nos. 1160 to 1225; and a polypeptide consisting of 54 amino acids from Nos. 763 to 816 can be prepared in a large scale by recombinant DNA technique.

[5] Preparation of a cDNA Clone T7N1-30U Originated from Serum of HC Patient (SEQ ID NO 101)

The gene or a DNA fragment encoding a novel polypeptide of SEQ ID NO 101 can be obtained by following procedures.

The ligation of clones N19MX24A-1 and MX25-1 by PCR gives a DNA fragment in which either of the 3' sequence of MX24 region and 5' sequence of MX25 region, which are overlapping each other, is preferentially used (Clone 1925). A synthetic DNA was synthesized in order to introduce into clone N1-1, from 5' to 3', restriction sites HindIII and SpeI and T7 promoter and clone T7N1-1 was obtained by cassette ligation. Clone T7N1N3N10 was obtained in the same manner as that used for the preparation of clone N1N3N10 except that clone T7N1-1 was used instead of clone N1-1. This clone was ligated to clone N27N19-1 by restriction enzyme BamHI to obtain clone T7N119. The clones T7N119 and 1925 have N19 regions and the both clones were ligated using PvuI restriction site in the N19 region to yield clone T7N1-25.

A EcoRI-NotI-BamHI adapter (Toyobo) was ligated to plasmid pUC1530U at the HindIII site in its 3' terminal region to obtain Clone 1530UNot which contains NotI site at 3' terminus of clone 1530U.

For the ligation of clones T7N1-25, 1530UNot, and MX25N15-1, prepared in [3], the three clones were ligated at PstI site in MX25 region common to clones T7N1-25 and MX25N15-1 and EcoT22I site in N15 region common to clones 1530UNot and MX25N15-1. Clone T7N1-25 has SpeI site at 5' terminus and clone 1530UNot has NotI site at 3' terminus.

HCV gene can be prepared by ligating clones T7N1-25, MX25N15-1 and 1530UNot in this order without overlapping. Thus, clone T7N1-25 is digested with SpecI and PstI, clone MX25N15-1 with PstI and EcoT221, clone 1530UNot with EcoT22I and NotI, λZapII (Strategene) with SpecI and NotI, respectively, and the resultant fragments were ligated to yield a phage in which a single DNA fragment having a sequence of HCV gene between SpeI and NotI sites of λZapII (from 5' to 3': clone T7N1-25, MX25N15-1 and 1530UNot). The resultant HCV derived clone was designated as T7NI-30U. Ligation to λZapII (Strategene), isolation of phage DNA, subcloning into pBluescriptII can be conducted according to the protocol attached to the kit. The packaging for the preparation of phage particles were carried out using Gigapack II Packaging Extracts (Strategene) according to the protocol attached thereto. The clone T7N1-30U is a DNA fragment which comprises a cDNA originated from HCV having an inserted T7 phage promoter at 5' terminus, and poly T at 3' terminus.

[6] Expression of Fused Polypeptides Encoded by cDNA Originated from Serum of HC Patients

Recombinant HCV-associated antigen can be obtained by expressing all or a part of clones prepared in [1], [2], [3] or [4], or DNA sequence encoding all the protein of HCV prepared in [5].

The present invention can be accomplished using any expression vectors which have a promoter at an appropriate site to direct the expression of a DNA encoding HCV polypeptide or a fragment thereof. Expression vectors preferably contain promoter, ribosome binding (SD) sequence, gene encoding HCV polypeptide, transcription termination factor, and a regulator gene.

Expression vectors functional in microorganisms such as Escherichia coli, Bacillus subtilis or the like will preferably comprise promoter, ribosome binding (SD) sequence, HCV-associated-protein-encoding gene, transcription termination factor, and a regulator gene.

Examples of promoters include those derived from Escherichia coli or phages such as tryptophane synthetase (trp), lactose operon (lac), λphage $P_L$ and $P_R$, $T_5$ early gene $P_{25}$, $P_{26}$ promoter and the like. These promoter may have modified or designed sequence for each expression vector such as pac promoter.

Although the SD sequence may be derived from Escherichia coli or phage, a sequence which has been designed to contain a consensus sequence consisting of more than 4 bases, which is complementary to the sequence at the 3' terminal region of 16S ribosome RNA, may also be used.

The transcription termination factor is not essential. However, it is preferable that an expression vector contains a $\rho$-independent factor such as lipoprotein terminator, trp operon terminator or the like.

Preferably, these sequences required for the expression of a gene encoding HCAg originated from HCV are located, in an appropriate expression plasmid, in the order of promoter, SD sequence, said gene and transcription termination factor from 5' to 3' direction.

Typical example of expression vectors is commercially available pKK233-2 (Pharmacia). However, a series of plasmids pGEX (Pharmacia), which are provided for the expression of fused protein, are also employable for the expression of HCAg-encoding gene of the present invention.

A suitable host cell such as Escherichia coli can be transformed with an expression vector comprising a DNA of the invention by any of known methods such as protocol provided by TOYOBO Japan as described in Example 30.

The cultivation of the transformants can be carried out using any of well known procedures in literatures such as Molecular Cloning, 1982, and the like. The cultivation is preferably conducted at a temperature from about 28 ° C to 42 ° C.

Expression vectors used for transforming other host cells, such as those derived from insects or animals including mammals, consist of substantially the same elements as those described in the above. However, there are certain preferable factors as follows.

When insect cells are used, a commercially available kit, MAXBAC™ is employed according to the teaching of the supplier (MAXBAC™ BACULOVIRUS EXPRESSION SYSTEM MANUAL VERSION 1.4). In this case, it is desirable to make a modification to reduce the distance between the promoter of polyhedrin gene and the initiation codon so as to improve the expression of the gene.

When animal cells are used as hosts, expression vectors preferably contain active-type promotor from adenovirus EIA gene (ZOKUSEIKAGAKU JIKKEN KOZA I, IDENSHI KENKYU-HO II, 189-190, 1986), SV40 early promoter, SV40 late promoter, apolipoprotein E gene promoter, SRα promotor (Molecular and Cellular Biology, 8, 1, 466-472, 1988) or the like. Specifically, known expression vectors such as pKCR (Proc. Natl. Acad. Sci. USA, 78: 1528 (1981)) or a derivative thereof, pBPV MT1 (Proc. Natl. Acad. Sci. USA, 80: 398 (1983)), which prepared by modifying pKCR maintaining its essential functions, pBPV MT1 (Proc. Natl. Acad. Sci. USA, 80: 398 (1983)), or the like may be employed.

Animal cells usable in the present invention are CHO cell, COS cell, mouse L cell, mouse C127 cell, mouse FM3A cell and the like.

A clone of the invention can be inserted into an expression vector for procaryotic cells such as E.coli or eucaryotic cells such as animal cells after modifying the DNA sequence to bring it in conformity with a frame of initiation codon of said vector. Alternatively, an initiation codon is added at the 5' terminus of DNA so as to an appropriate translational frame can be produced. The term "a translational frame" of a clone refers to a frame of a base sequence in which bases are described as triplets capable of encoding amino acid sequence as illustrated in SEQ ID NO 1 to 104.

The recombinant polypeptide expressed by host cells such as microorganisms including E. coli, insect cells and animal cells can be recovered from the cultured broth by known methods and identified by, for example, immunoreactions between the expressed product and antiserum obtained from HC patients using a conventional method such as Western blot analysis.

Polypeptide encoded by gene of the invention contains region(s) which seem to be immunologically highly reactive with antiserum of HC. These regions were ligated and expressed in various cells as fused protein. For example, polypeptide having amino acids from Nos. 1 to 115 of SEQ ID NO 3 was expressed using expression vector pCZCORE. The expression vector was modified to replace the 3' region from the epitopic region of said polypeptide with clone N23 which encodes a desired polypeptide to express a fused protein. It was followed by the ligation of a polypeptide having amino acids from Nos. 963 to 1005 of SEQ ID NO 43 to the C-terminus of polypeptide encoded by N23 region. Thus, regions encoding polypeptides which seem to be immunologically highly reactive with antiserum of HC patients were ligated to cDNA and inserted into an expression vector to express said polypeptides.

Specifically, as shown in Example 30, a polypeptide CN23 which contains an epitopic region of core protein of HCV and a region comprising an epitope which is encoded by clone N23, a part of non-structural protein region NS3 and is seem to be immunologically highly reactive with antiserum of HC patients, was expressed directly in E. coli

Thus, clone N23, from No. 107 (G), was inserted in frame into pCZCORE at the SacII site within core gene. Expression vector pCZCN23 capable of expressing epitopic regions of core protein and a polypeptide encoded by N23 as a fused protein was constructed by ligating a part of N23 to the 3' terminus of the N-terminal gene of core protein. A DNA fragment which encodes HCV protein and has SD sequence at 5' terminus was ligated in tandem to the vector, resulting in the expression of desired polypeptide in large scale.

The resultant fused protein comprising epitopic regions of core protein and N23 region reacted with antiserum of HC patient in high probability.

Thus, the present invention provides a novel gene of HCV or a fragment thereof and polypeptide encoded by the same. The recombinant polypeptide is highly reactive with HCAb and can be used for the development of a method for detecting HCAb efficiently, and for the preparation of vaccine. DNA and polypeptides of the invention are also useful for the development of in vivo or in vitro system for the estimation of protease activity of HCV.

The following Examples further illustrate and detail the invention disclosed, but should not be construed to limit the invention. Throughout the Examples concerning the isolation of RNA and cloning of cDNA, tip or pipet used for the preparation of samples and/or reagents employed for reaction was changed to cleaned and/or sterilized one every time for preventing the sample from contamination. The procedures which are not specifically described were conducted substantially in accordance with the teachings of literatures given in parentheses.

Electrophoresis of nucleic acids (Molecular Cloning (1982), Cold Spring Harbor): cleavage of DNA fragment with restriction enzymes (Molecular Cloning (1982), Cold Spring Harbor); or a catalogue "IDENSHIKOGAKU KENKYU-YO SIYAKU SOGO KATALIOGU", Toyobo): ligation reaction of DNA fragments (TAKARA Biotechnology Catolog, 1991, vol. 1, Takara Shuzo): extraction of DNA from acrylamide gel or agarose gel (Molecular Cloning (1982), Cold Spring Harbor): cultivation of E. coli transformants transformed with a plasmid on agarose plate and isolation of colony therefrom (Molecular Cloning (1982), Cold Spring Harbor).

Example 1

Extraction of Nucleic Acids from Serum of a Patient Suffering from Hepatitis C

To 10 ml of a serum from a patient of HC (OD = 3.5 or more on HCV EIA kit of Ortho & Co.) was added 25 ml of Tris buffer (50 mM Tris-HCl (pH 8.0), 1 mM EDTA, 100 mM NaCl), mixed and centrifuged (20,000 x g, 20 min) at 20 °C. The supernatant was centrifuged (100,000 x g, 5 hr) at 20 °C. The pellet was dissolved into 1.5 ml of Protenase K solution (1% sodium dodecyl sulfate, 10 mM EDTA, 10 mM Tris-HCl (pH 7.5), 2 mg/ml Protenase K (Pharmacia), and 6.6 µg yeast tRNA mixture) and the solution incubated at 45 °Cfor 90 min. The solution was then subjected to the phenol/chloroform extraction (more than 4 times) which was carried out by adding an equal volume of phenol/chloroform to the solution, vigorously mixing, and centrifuging to recover the aqueous layer containing nucleic acids. It was followed by chloroform treatments (more than two times) and ethanol precipitation. The ethanol precipitation was carried out by mixing the aqueous solution with 2.5 volumes of ethanol containing either of 1/10 volume of 3M sodium acetate or an equal volume of 4 M ammonium acetate, allowing to stand for overnight at -20 °C, or more than 15 min at -80 °C, centrifuging (35,000 rpm, 4 hr) by SW41 Ti Rotor (Beckman) to pellet nucleic acids, and recovering the pellet. The pellet of nucleic acid was then dried for the subsequent use.

Example 2

Synthesis of cDNA

[1] Preparation of RNA Sample Solution

RNA sample solution was prepared by resolving the dried nucleic acid obtained in Example 1 in 30 µl of water containing 10 µl of ribonuclease inhibitor (100 U/µl, Takara Shuzo, Japan).

[2] Synthesis of cDNA Using Random Primer

To 2 µl of RNA sample solution was added 2.7 µl of random primer (0.170D, Amersham), 2 µl of 10 x PCR (Mg) buffer (100 mM Tris-HCl (pH 8.3), 500 mM KCl, 60 mM $MgCl_2$), 8 µl of 1.25 mM 4dNTPs, 2 µl of water and the mixture incubated at 65 °C for 5 min then at 25 °C for 5 min. To the mixture was added l µl of reverse transcriptase (25 U, Life Science), 1 µl of ribonuclease inhibitor (100 U/µl, Takara Shuzo) and the mixture incubated at 37 °C for 20 min, at 42 °C for 30 min, and finally at 95 °C for 5 min, which was followed by prompt cooling to 0 °C (synthesis of cDNA).

Amplification of DNA having specific sequences was conducted substantial in accordance with the polymerase chain reaction (PCR) of Saiki et al. (Nature 324: 126 (1986)). Throughout the specification, the expression that PCR was carried out according to Saiki's method means that the PCR was conducted substantial in accordance with the polymerase chain reaction (PCR) of Saiki et al. For the PCR, a 100 µl of a mixture containing 2 µl of cDNA solution, 10 µl of 10 x PCR buffer (100 mM Tris-HCl (pH 8.3), 500 mM KCl, 150 mM $MgCl_2$, 1% gelatin), 8 µl of 2.5 mM 4 dNTPs, 50 pmol each of two synthetic primers (the pair of primers consists of S1 - AS1, S2 - AS1, S2 - AS2, or S4 - AS3) and water was incubated at 95 °C for 5 min, then immediately cooled to 0 °C. One minute later, it was mixed with 0.5 µl of Taq DNA polymerase (7 U/µl, AmpliTaq™, Takara Shuzo) and overlaid with mineral oil. The resultant sample was then subjected to PCR. PCR was conducted by repeating 25 times of reaction cycle, which comprises the following treatments: at 95 °C for 1 min; at 40 - 55 °C for 1 min; and at 72 °C for 1 - 5 min in DNA Thermal Cycler (Parkin Elmer Cetus). The reaction mixture was then subjected to phenol/chloroform extraction and ethanol precipitation to obtain amplified DNA fragments. The ethanol precipitation was generally carried out by adding 2.5 volumes of ethanol and either of about 1/10 volume of 3 M sodium acetate or an equal volume of 4 M ammonium acetate to the aqueous solution, mixing, centrifuging at 15,000 rpm for 15 min using a rotor of about 5 cm in diameter under cooling at 4 °C to pellet the precipitates, and drying the pellet. Throughout the specification, the procedure "etanol precipitation" meanes the above-mentioned procedures. In the same manner as the above, various DNA fragments were obtained using different pair of primers in PCR.

[3] Synthesis of cDNA Using Antisense Primer

To 2 μl of RNA sample solution prepared in above [1] was added 1 μl of 15 pmol/μl anti-sense primer (synthesized primer AS1, AS2 or AS3), 2 μl of 10 x RT buffer (100 mM Tris-HCl (pH8.3), 500 mM KCl), 4 μl of 25 mM $MgCl_2$, 8 μl of 2.5 mM 4dNTPs, 1 μl of water and the mixture incubated at 65 °C for 5 min then at room temperature for 5 min. To the mixture was added 1 μl of reverse transcriptase (25 U, Life Science), 1 μl of ribonuclease inhibitor (100 U/μl, Takara Shuzo) and the mixture incubated at 37 °C for 20 min, at 42 °C for 30 min, and finally at 95 °C for 2 min, which was followed by an immediate cooling to 0 °C (synthesis of cDNA).

Amplification of DNA containing specific sequences was conducted by PCR (Saiki et al., Nature 324: 126 (1986)). Thus, 100 μl mixture containing 10 μl of cDNA solution, 10 μl of 10 x PCR buffer (100 mM Tris-HCl (pH 8.3), 500 mM KCl, 15 mM $MgCl_2$, 1% gelatin), 8 μl of 2.5 mM 4 dNTPs, 2 μl of 15 pmol/μl synthetic DNA primer (the same primer as used in the synthesis of cDNA), 3 μl of 15 pmol/μl synthetic DNA primer (a counterpart of paired primers) and water was incubated at 95 °C for 5 min, then immediately cooled to 0 °C. One minute later, it was mixed with 0.5 μl of Taq DNA polymerase (7 U/μl, AmpliTaq™ Takara Shuzo) and overlaid with mineral oil. The resultant sample was then subjected to PCR. PCR was conducted by repeating 25 times of reaction cycle, which comprises the following treatments: at 95 °C for 1 min; at 40 - 55 °C for 1 min; and at 72 °C for 1 - 5 min in DNA Thermal Cycler (Parkin Elmer Cetus). Finally, the reaction mixture was incubated at 72 °C for 7 min, which was followed by phenol/chloroform extraction and ethanol precipitation to obtain different amplified DNA fragments derived from either of above-mentioned pairs of primers.

Example 3

Cloning and Sequencing of Amplified DNA Fragments

Dried DNA fragment (at least 1 pmole) obtained in the above Example 2, [2] or [3] was blunt-ended with T4 DNA polymerase (Toyobo) and 5'-end phosphorylated with polynucleotide kinase (Toyobo) and ligated into SmaI site of multi-cloning sites of 5 ng to 10 ng of pUC19 cloning vector. The cloning vector had been previously treated as follows: digestion with a restriction enzyme SmaI (Toyobo), phenol/chloroform extraction, ethanol precipitation, 5'-end dephosphorylation with alkaline phosphatase (Behringer-Manheim), phenol/chloroform extraction, and ethanol-precipitation. The ligated DNA was used to transform into a competent E.coli JM 109 or DH5 cells (Toyobo). The transformation was carried out according to the protocol of the manufacture's instruction (COMPETENT HIGH, Toyobo). Plasmid clones were recovered from transformed cells conventionally. At least 20 transformants were obtained using pUC19 cloning vectors containing either of DNA fragments obtained in the above Example 2, [2] and [3] using each pair of primers.

The determination of base sequence of DNA fragment was conducted by Fluorescent DNA Sequencer (GENESIS 2000, Dupont) using, as sequence primer, the following synthetic primers:
5' d(GTAAAACGACGGCCAGT)3' (SEQ ID NO 143) and
5'd(CAGGAAACAGCTATGAC)3' (SEQ ID NO 144) for the + and - strands of DNA fragment to be sequenced.

Base sequences of clones is given in SEQ ID NO 1 to 4 and 9 to 12. Base sequences of SEQ ID NO 1, 2, 3, 4, 9, 10, 11 and 12 correspond to that of + strand of clones N1-1, N2-1, N3-1, N10-1, N1-2, S1-1, S1-2 and S1-3 of transformants, respectively. These clones are double stranded DNA which were prepared in the same manner as those described in Examples 2 and 3 using 4 kinds of pairs of primers shown in Example 2, [2]. Plasmid used for sequencing the clones were designated as pUCN1-1, pUCN2-1, pUCN3-1, pUCN10-1, pUCN1-2, pUCS1-1, pUCS1-2 and pUCS1-3, respectively. Each plasmid contained one DNA molecule corresponding to each DNA fragment.

These base sequences represents base sequences of clones obtained by cloning the cDNA synthesized from RNA isolated from serum of patient(s) suffering from HC. Therefore, these sequences are specific for clones originated from serum of HCV-infected patients but can not be found or obtained from serum of healthy subjects. Thus, cDNA prepared from RNA (if there are any) obtained from a healthy subject under more strict conditions, for instance, by increasing (3 or 4 folds) the reaction cycles of PCR in Example 2, [2] and [3], by repeating them 60 - 100 times, did not show any homology in base sequence with those shown in SEQ ID NO 1 to 4. Consequently, base sequences of clones N1-1, N2-1, N3-1, N10-1, N1-2, S1-1, S1-2 and S1-3 are specific for those obtained from serum of patients suffering from HC.

As the next step, the resultant DNA fragment was modified so that a polypeptide encoded by a open reading frame should be expressed in a host cell transformed by the modified DNA, and the resultant product was then evaluated as to the ability to react, as a antigenic polypeptide of HCV, with HCAb in serum of HC patients.

Example 4

Preparation of Clone N1N3N10 or N3N10

[1] Preparation of Clone N3N10

One μl of each DNA fragments (about 200 - 300 ng) from clones N3-1 and N10-1 was added into a reaction mixture containing 10 μl of 10 x PCR buffer (100 mM Tris-HCl (pH8.3), 500 mM KCl, 15 mM $MgCl_2$, 1% gelatin), 8 μl of 2.5 mM 4 dNTPs, 5 μl each of 20 pmol/μl synthetic primers S2 and AS3, and 76.5 μl of water. After an intimate mixing, the mixture was heated at 95 °C for 5 min, then immediately cooled to 0 °C. One minute later, to the mixture was added 0.5 μl of Taq DNA polymerase (7 U/μl, AmpliTaq™ Takara Shuzo), mixed and overlaid with mineral oil. The sample was then subjected to PCR. PCR was conducted by repeating 25 times of reaction cycle, which comprises the following treatments: at 95 °C for 1 min; at 40 °C for 1 min; and at 72 °C for 2 min in DNA Thermal Cycler (Parkin Elmer Cetus). It was followed by an incubation at 97 °C for 2 min. The mixture was immediately cooled to 0 °C, kept at 0 °C for 2 min, mixed with 0.5 μl of Taq DNA polymerase (7 U/μl, AmpliTaq™ Takara Shuzo). The sample was then treated in the same manner as the above by repeating 25 times of reaction cycle, which comprises the following treatments: at 95 °C for 1 min; at 50 °C for 1 min; and at 72 °C for 2 min. After the final treatment at 72 °C for 7 min, the resultant reaction solution was treated with phenol/chloroform then precipitated with ethanol. The amplified DNA samples were fractionated on agarose gel electrophoresis and a gel containing a desired fragment having an expected length was removed (Molecular Cloning (1982) Cold Spring Harbor) to isolate the DNA fragment therefrom conventionally. The resultant DNA fragment was then modified as described in Example 3 and ligated into SmaI site of multi-cloning sites of pUC19, cloned and screened as described in Example 3 to obtain plasmid pUCN3N10. The resultant cDNA derived from serum of HC patient was referred to as clone N3N10 whose base sequence is given in SEQ ID NO 5.

[2] Preparation of Clone N1N3N10

Two overlapping clones N1-1 and N3N10 were ligated by taking advantage of unique restriction site which exists in the overlapping regions of the both clones. Upon digestion with restriction enzyme BssH11, clone N1-1 is cleaved at the 3' site of a nucleotide No. 455 G and clone N3N10 at the 3' site of a nucleotide No.159 G. The ligation of two clones N1-1 and N3N10 was accomplished on the basis of an assumption that plasmids pUCN1 and pUCN3N10 contain each clone in the same orientation. Thus, plasmid pUCN1 was digested with HindIII and BssHII to yield a 492 bp DNA fragment comprising a HindIII-SmaI DNA fragment of plasmid pUC19 attached to the 5' end of the No. 455 bp nucleotide of clone N1-1 derived from serum of HC patient, which fragment was then exchanged with 159 bp HindIII - BssHII fragment of Plasmid pUCN3N10, cloned and screened to obtain a plasmid pUCN1N3N10. The plasmid pUCN1N3N10 contained the desired clone N1N3N10 comprising clones N1-1, N3-1 and N10-1 ligated without overlapping. The base sequence of clone N1N3N10 is shown in SEQ ID NO 6.

Example 5

Modification of DNA for the Expression of HCV Polypeptide Encoded by Clones N3-1 or N3N10

[1] Modification of DNA for the Expression of HCV Polypeptide Encoded by Clone N3-1 in E.coli

Clone N3-1 contains a DNA fragment capable of encoding a structural protein of HCV which begins at nucleotide No. 22 (A). The DNA can be expressed utilizing ATG codon at nucleotides Nos. 22 to 24. The modification of DNA was carried out using PCR. The following synthetic oligonucleotide primers were used.

5' primer:
5' GCAAGCTTATGAGCACAAATCCAAAACCCCAAAGA 3' (SEQ ID NO 145)
3' primer:
5' GCGAATTCAGATCTTCACCTACGCCGGGGGTCCGTGGG 3' (SEQ ID NO 146)

The synthetic DNA was adjusted to 20 pmol/ml before use.

PCR was carried out in the same manner as described in the above according to Saiki's method in a total volume of 100 µl containing 100 ng of plasmid pUCN3, as a template, and 2 µl each of 3' and 5' primers. The reaction mixture was heated at 95 °C for 5 min and quenched at 0 °C. One minute later, to the mixture was added 0.5 µl of Taq DNA polymerase (7 U/ml, AmpliTaq™ Takara Shuzo), mixed thoroughly and overlaid with mineral oil. The sample was reacted by repeating 25 cycles of treatments which comprises: at 95 °C for 1 minute; at 60 °C for 1 min; and at 72 °C for 5 min in DNA Thermal Cycler (Parkin Elmer Cetus). The resultant reaction solution was extracted with phenol/chloroform, and precipitated with ethanol conventionally. The amplified DNA samples were digested with HindIII and EcoRI, and fractionated on acrylamide gel electrophoresis and extracted (Molecular Cloning, Cold Spring Harbor (1982)).

The resultant DNA fragment was then ligated into HindIII and EcoRI sites of a cloning vector pUC19, cloned and screened to obtain plasmid pUCHN3. The resultant plasmid was sequenced and shown in SEQ ID NO 7. The sequence shows that it contains, at the 5'-terminus, a HindIII site followed by ATG initiation codon, and at the 3'-terminus, a termination codon TGA, BglII and EcoRI restriction sites, from 5' to 3'.

[2] Modification of DNA for the Expression of HCV Polypeptide Encoded by Clone N3N10 in E.coli

Clone N3N10 contains a DNA fragment capable of encoding structural protein of HCV which begins at nucleotide No. 22 (A). The DNA can be expressed utilizing ATG codon at nucleotides Nos. 22 to 24. The modification of DNA was carried out using PCR. The following synthetic oligonucleotide primers were used.
5' primer:
5' GCAAGCTTATGAGCACAAATCCAAAACCCCAAAGA 3' (SEQ ID NO 145)
3' primer:
5' GCGAATTCAGATCTTCAGATTCTCTGAGACGGCCCTCGT 3' (SEQ ID NO 147)

The synthetic DNA was adjusted to 20 pmol/ml before use.

PCR was carried out in the same manner as the above [1] except that the above two primers and plasmid pUCN3N10, as a template, were used and PCR was conducted by repeating 10 cycles of treatments which comprises: at 95 °Cfor 1 minute; at 50 °C for 1 min; and at 72 °C for 5 min, and then 20 cycles of treatments which comprises: at 95 °Cfor 1 minute; at 65 °C for 1 min; and at 72 °C for 5 min.

The amplified DNA sample was digested with HindIII and EcoRI, and fractionated on acrylamide gel electrophoresis and extracted the gel containing a DNA fragment of desired length (Molecular Cloning, Cold Spring Harbor (1982)). The resultant DNA fragments were then ligated into HindIII and EcoRI sites of cloning vector pUC19, cloned and screened conventionally to obtain plasmid pUCHN3N10. The plasmid pUCHN3N10 was then sequenced.

Thus obtained clone HN3N10 contains, at the 5'-terminus, a HindIII site followed by ATG initiation codon, and at the 3'-terminus, a termination codon TGA, BglII and EcoRI restriction sites, from 5' to 3'.

For the removal or BamHI site from the clone HN3N10, a nucleotide sequence: 5'GGATCC3' was converted to 5'GGATAC3' by PCR using the following synthetic DNA fragments as primers.
5' primer:
5' GCTACTCCGGATACCAC 3' (SEQ ID NO 148)
3' primer:
5' GTAAAACGACGGCCAGT 3' (SEQ ID NO 143)

The synthetic DNA was adjusted to 20 pmol/ml before use.

The nucleotide "G" at the 5'-terminus of 5' primer corresponds to the No.1016 G of the base sequence of clone N3N10. The 3' primer is derived from plasmid pUC19 and the same as one of primers used for sequencing in Example 3. The PCR was conducted by repeating 25 cycles of treatments which comprises: at 95 °C for 1 minute; at 55 °C for 1 min; and at 72 °C for 1 min. For the reaction, 3 µl of each primer and 100 ng of plasmid pUCHN3N10, as a template DNA, were used. The reaction mixture was then subjected to phenol/chloroform extraction and ethanol precipitation as conventionally. The amplified DNA sample was digested with MroI, BglII, and BamHI, fractionated on acrylamide gel electrophoresis, and extracted the gel containing a desired 226 bp DNA fragment (Molecular Cloning, Cold Spring Harbor (1982)). The resultant DNA fragments were then ligated into MroI and BglII sites of plasmid pUCHN3N10, cloned and screened by conventional method to obtain plasmid pUCHN3N10ΔB. The resultant plasmid pUCHN3N10ΔB was then sequenced and base sequence of clone HN3N10ΔB is shown in SEQ ID NO 8.

[3] Modification of DNA for the Expression of HCV Polypeptide Encoded by Clone N3N10 in Insect Cells

Clone N3N10 appears to contain entire viral protein-encoding genes including those encoding core, envelope (M-gp35) proteins. The region beginning at nucleotide No. 22 (A) which encodes structural protein was expressed in insect cells utilizing ATG codon at nucleotides Nos. 22 to 24. When insect cells were transfected with the DNA and cultivated, core andenvelope (M-gp35) proteins were expressed in the fused form as a precursor polypeptide, which was then processed to separate core and envelope (M-gp35). At least the latter envelope (M-gp35) was then glycosylated incompletely and accumulated intracellurarly. The modification of DNA of clone N3N10 for the construction of expression vector was carried out by PCR using following synthetic oligonucleotide primers.

5' primers:

MS106: 5' GCGTCGACGCTAGCATGAGCACAAATCCAAAACCC 3' (SEQ ID NO 149)

MS107: 5' GCGTCGACGCTAGCAGGTCTCGTAGACCGTGCATC 3' (SEQ ID NO 150)

3' primer:

MS108: 5' GCGAATTCGCTAGCTCAGGATTCTCTGAGACGGCCCTCGA 3' (SEQ ID NO 151)

These three synthetic DNAs were separately adjusted to 20 pmol/ml before use.

The PCR was carried out using the same reaction solution and worked up in the same manner as described in the above [1] except that plasmid pUCN3N10 was used as a template plasmid, and, as 5'primer, primer MS106 or MS107 and, as 3'primer, MS108 were used. PCR was accomplished by repeating 10 times of reaction cycles consisting of: 1 min at 95 °C; 1 min at 50 °C and 5 min at 72 °C ; and then 20 times of reaction cycles consisting of: 1 min at 95 °C; 1 min at 65 °C; 5 min at 72 °C. A combination of primers MS106 and MS108 gave a desired 1265 bp DNA fragment 106-108 and that of primers MS107 and MS108 gave a desired 1286 bp DNA fragment 107-108.

These DNA fragments were digested with NheI, fractionated on acrylamide gel electrophoresis and extracted by convenional means (Molecular Cloning, Cold Spring Harbor (1982)) to obtain DNA fragments of desired length. Each of the resultant DNA fragments was then ligated into NheI restriction site of a transfer vector pBlueBac (Invitrogen), cloned and screened by the usual method to yield plasmids pBlueN3N10-1 and pBlueN3N10-2, which are derived from DNA fragments 106-108 and 107-108, respectively.

Plasmids pBlueN3N10-1 and pBlueN3N10-2 were digested with NheI or BamHI completely to confirm that each plasmid contains only one DNA fragment, either of 106-108 or 107-108 inserted at NheI site. Furthermore, taking account of the instruction provided by the manufacture (Invitrogen), the expression unit of these plasmid contain a gene encoding HCV structural polypeptide (core and envelope) oriented forward and ligated to the NheI cloning site down stream from a polyhedrin promoter.

## Example 6

### Expression of HCV Polypeptides Encoded by Clones HN3, HN3N10ΔB

[1] Expression of Polypeptide Encoded by Clone HN3 in E.coli

Clone HN3 encodes a part of polypeptide encoded by cDNA originated from serum of HC patient. The polypeptide encoded by clone HN3 was expressed directly in E.coli, as it is, by subcloning said clone into an expression vector pCZ44 (Japanese Patent Publication No. 124387/1989).

Clone HN3 was digested thoroughly with restriction enzymes HindIII and BglII, extracted with phenol/chloroform, precipitated with ethanol, separated on acrylamide gel electrophoresis. From the gel was extracted a DNA fragment having cohesive HindIII- and BglII-restricted ends (Molecular Cloning, Cold Spring Harbor, 1982). The expression vector pCZ44 was digested with HindIII and BglII. The larger DNA fragment containing a region functional for the replication in E.coli was separated, treated in the same manner, ligated to the HindIII-BglII fragment of clone HN3 so as to have only one insertion, and cloned by conventional method to yield plasmid pCZCORE.

Alternatively, an expression vector was constructed using an expression vector pGEX-2T (Pharmacia) for the expression of a fused protein of a desired polypeptide and β-glutathione-S-transferase (GST). The construction was carried out substantial in accordance with the protocol taught by the manufacture (Pharmacia). Thus, the expression vector pGEX-2T was digested with BamHI. The linearized vector was ligated with a HindIII linker to obtain a DNA fragment having EcoRI and HindIII restriction sites at the 3'- and 5'-termini. The fragment was ligated to HindIII-EcoRI fragment of HN3 such that every reading frame of codon is consistent with an amino acid of clone N3-1 to yield an expression vector pGEXCORE.

E.coli K12 strains (e.g., JM109, KS476) or those derived from B strains transformed with plasmid pCZCORE was grown in L-Broth at 37 °C overnight (Molecular Cloning, Cold Spring Harbor, 1982). The cultured broth was diluted 50-folds by inoculating it into a freshly prepared L-Broth and the cultivation continued with shaking at 30 °C for 2 hr. At this time, IPTG (isopropyl-$\beta$-D-galactopyranoside) was added to the culture to a final concentration of 1 or 2 mM in order to induce the expression of DNA encoding HCV-originated CORE-N3 polypeptide by single-clone-derived transformants (E.coli cells transformed solely by plasmid pCZCORE derived from clone HN3). Base sequence and deduced amino acid sequence of clone HN3 is shown in SEQ ID NO 7.

As mentioned in the above, plasmid pGEXCORE can be used to obtain transformants capable of expressing a fused protein include desired polypeptide and GST. The plasmid encodes a fused protein GST-CORE comprising GST, which has a thrombin-cleaving site at its C-terminus, and a polypeptide derived from a clone HN3, the same polypeptide as that encoded by plasmid pCZCORE. The transformants containing pGEXCORE were grown in the presence of IPTG using the same protocol as that used for the expression of CORE-N3 polypeptide of HCV from transformants harboring pCZCORE to produce the fused polypeptide GST-CORE.

[2] Expression of Polypeptides Encoded by Clone HN3N10ΔB

Clone HN3N10ΔB encoding a part of polypeptide encoded by cDNA originated from serum of HC patient was expressed in E.coli to give polypeptide CME-N3N10ΔB in the same manner as the above [1]. The cDNA used was that contained in clone HN3N10ΔB obtained from serum of HC patient, which clone had been previously isolated and sequenced as described in Examples 3, Example 4 [1], and Example 5 [2]. The expression plasmid pCZCMEΔB was constructed by subcloniong a DNA fragment isolated from plasmid pUCHN3N10ΔB by ligating its HindIII and BglII cohesive ends to HindIII and BglII sites of plasmid pCZ44 such that only one DNA fragment should be inserted in an appropriate orientation by the same method used for the preparation of plasmid pCZCORE. Plasmid pCZCMEΔB was then subjected to the sequencing and restriction enzyme mapping to confirm that an expression unit of plasmid pCZCMEΔB was reconstructed properly.

The cultivation of transformants was carried out in the presence of IPTG in order to induce the expression of HCV-originated CME-N3N10ΔB polypeptide by single-clone-derived transformants (E.coli JM 109 cells transformed solely by plasmid pCZCMEΔB derived from clone HN3N10ΔB, a variant of clone N3N10). Base sequence and deduced amino acid sequence of cDNA obtained from serum of HC patient contained in clone HN3N10ΔB is shown in SEQ ID NO 8. The amino acid sequences deduced from base sequences of a clone HN3N10ΔB and its original clone N3N10 were exactly the same.

In the same manner as the above [1], plasmid pGEXCMEΔB was constructed, transformed into host cells. The transformants, when grown under a same condition for transformants harboring plasmid pCZCMEΔB inducing by IPTG, expressed a fused protein GST-CME-N3N10ΔB.

[3] Expression of Polypeptide Encoded by Clone N3N10 in Insect Cells

The expression of structural polypeptide (core, envelope (M-gp35) of HCV encoded by plasmid pBlueN3N10-1 prepared in Example 5 [3] was conducted substantial in accordance with a known expression manual for baculovirus (MAXBAC™ BACULOVIRUS EXPRESSION SYSTEM MANUAL VERSION 1.4, hereinafter, referred to as Maxbac, Invitrogen).

Plasmids pBlueN3N10-1 and pBlueN3N10-2, plasmids prepared by inserting DNA fragment containing HCV structural gene at the NheI site of a transfer vector pBlueBac (Maxbac, pp.37), were recovered from E.coli host cells transformed thereby, and purified according to the method of Maniatis et al.(Molecular Cloning, Cold Spring Harbor Laboratory, pp.86 - 96 (1982)). Thus, a large amount of HCV structural gene-containing transfer plasmid DNA was obtained. Sf9 cells were co-transfected with 2 $\mu$g of either of plasmids pBlueN3N10-1 or pBlueN3N10-2 and 1 $\mu$g of AcNPV viral DNA (Maxbac, pp.27). Sf9 cells were grown in TMN-FH medium (Invitrogen) containing 10% FCS (fetal calf serum) in a 6 cm dish (60 x 15 mm, FALCON$^R$; Nippon Becton Dickinson Co., Ltd.) until a cell density reached to about 2 x $10^6$/plate. The TMN-F medium was removed and a 0.75 ml Grace medium (Gibco) containing 10% FCS was added thereto. To the DNA mixture described in the above was added 0.75 ml of transfection buffer (attached to the kit) was thoroughly mixed by vortex and gradually added dropwise onto the Grace medium. After the culture being allowed to stand for 4 hr at 27 °C, Grace medium was replaced with 3 ml of TMN-FH medium containing 10% FCS and the dish incubated at 27 °C for 6 days. Three days from the incubation, there observed a few multinucleate cells and on sixth day, almost all the cells were multinuclear. The supernatant was taken into a centrifuging tube and centrifuged at 1,000 rpm, 10 min to obtain the supernatant as a cotransfected viral solution.

The cotransfected viral solution contains about $10^8$ viruses/ml and 0.5% of which were recombinant viruses. The isolation of recombinant virus was carried out by a plaque isolation method described below.

Thus, cells were adsorbed onto a 6 cm dish by seeding 1.5 x $10^6$ cells on medium and removing the medium completely. To the dish was added 100 µl of a diluted viral solution ($10^{-4}$ and $10^{-5}$ folds), separately and incubated at room temperature for 1 hr while slanting the 6 cm dish every 15 min to spread the virus extensively. X-gal medium containing agarose was prepared by adding 5-bromo-4-chloro-3-indolyl-β-D-galactoside to a final concentration of 150 µg/l (Maxbac, pp. 16-17) to a warm medium which had been prepared by autoclaving 2.5% baculovirus agarose (Invitrogen) at 105 °C for 10 min, mixing with TMN-FH medium containing 10% FCS preheated at 46 °C at the mixing ratio of 1 : 3, and keeping the temperature at 46 °C.

After the completion of infection, virus solution was aspirated thoroughly from the 6 cm dish and 4 ml of the warm X-gal medium containing agarose (previously prepared) was gently added to every 6 cm dish not to peel off cells. The dish kept open by slightly sliding a lid until the agarose solidified and dried, and thereafter the dish covered, turned upside down, and incubated at 27 °C for 6 days. The plaques were observed under a phase difference microscope to find blue plaques which do not form multinucleate cells. Agarose containing blue recombinant plaques were removed with a Pasteur pipet and suspended into 1 ml of TMN-FH medium by pipetting many times. The above process which comprised: infection, 6-day incubation, and isolation of virus containing transfer plasmid DNA is called the "plaque method". The plaque method was repeated using 100 µl of viral suspension. After repeating said process three times, there obtained a recombinant virus having a gene encoding structural protein derived from HCV free from contamination with that of wild-type strain.

A viral solution of the primary recombinant virus was prepared by aspirating plaques with a Pasteur pipet, and mixing thoroughly with 1 ml of TMN-FH medium. Because the primary viral solution was low in virus density for infection, it required further treatments for concentration. Thus, 100 µl of viral solution was adsorbed onto Sf9 cells grown in 6 cm dish to a semi-confluent, and 4 ml of TMN-FH medium was added thereto and incubated three days. The culture supernatant was recovered to yield a recombinant viral solution for infection.

For the production of HCV structural protein, a suspension of Sf9 cells in TMN-FH medium containing 10% FCS (5 x $10^6$ cells/10 ml medium) was added into a 9 cm dish and kept 1 hr for adsorption. After the removal of medium, 250 µl of recombinant viral solution was added to the 9 cm dish and spread extensively. To the dish was added 10 ml TMN-FH medium containing 10% FCS and incubated at 27 °C for 4 days. The cells expressing recombinant glycoprotein of HCV were harvested by scraping up and suspended into 1,000 ml of phosphate buffered saline.

Thus, HCV structural gene was expressed in Sf9 cells transfected with said virus. The transformants transformed with plasmids pBlueN3N10-1 and pBlueN3N10-2 expressed the same HCV polypeptide.

## Example 7

## Identification of Expression Products as HCAg

The expression products obtained in Example 6, which are CORE-N3 and CME-N3N10ΔB polypeptides, and HCV polypeptide encoded by clone N3N10 expressed in insect cells, were immunologically reactive with antiserum obtained from HC patients, demonstrating that these expression products are HC associated antigens.

Identification of these expression products as HCAg were carried out by Western blot as follows. E. coli cells transformed with either of plsmids pCZCORE and pCZCMEΔB encoding CORE-N3 and CME-N3N10ΔB polypeptides, respectively were grown under the presence of IPTG for 3 hr or a overnight in the same manner as described in Example 6.

Recombinant strains were harvested by centrifuging 1,000 µl of the cultured broth at 6,500 rpm, 10 min. The pellet was dissolved into a sample solution (50 mM Tris-HCl, pH6.8 containing 2% SDS, 5% mercaptoethanol, 10% glycerin, and 0.005% bromophenol blue) for SDS-polyacrylamide gel electrophoresis to a final volume of 0.2 ml. Sf9 cells infected with viruses which had been treated more than 3 times by plaque method were collected by scraping up and suspended into 1,000 ml of phosphate-buffered saline physiological saline (PBS) and 100 µl of the suspension was centrifuged at 6,500 rpm, 10 min to pellet the cells. The pellet was dissolved into a sample solution for SDS-polyacrylamide gel electrophoresis to a final volume of 0.2 ml.

The sample solutions were then boiled at 100 °C for 10 min. Ten µl of the boiled solution was loaded onto 0.1% SDS-15% polyacrylamide gel (70 x 85 x 1 mm) together with a marker protein LMW Kit E (low-molecular weight marker protein, Pharmacia). Electrophoresis was carried out at a constant current of 30 mA for 45 min in Tris buffer (25 mM Tris, pH 8.3, 192 mM glycine, 0.1% SDS) as electrode buffer. Thereafter, DNA was transferred electophoretically to a nitrocellulose filter by superposing the gel onto a filter BA-83 (S & S), impressing a constant current of 120 mA for about 20 min between gel (cathode) and the filter (anode) as conventionally.

The transcribed filter was cut to remove a part containing a marker protein (referred to as marker filter) and that containing the sample (referred to as sample filter). The former was stained with 0.1% (w/v) amideblack 10B and the latter immersed into 0.01 M PBS (pH 7.4) containing 5% (w/v) bovine serum albumin (BSA). Serum from a HC patient was diluted 50 times with 0.01 M PBS (pH 7.4) containing 5% (w/v) BSA. To the sample filter was added 10 μl of diluted serum and the filter allowed to stand for 2 hr at room temperature. Thereafter, the filter was washed with PBS containing 0.1% (v/v) Tween 20 for 20 min (x3).

The sample filter was then reacted with 10 ml of horseradish peroxidase conjugated anti human IgG (Gappel) at 37 °C for 1 hr and washed with PBS containing 0.1% (v/v) Tween 20 for 20 min (x3). The filter was then immersed into peroxidase-color-producing solution (60 mg 4-chloro-1-naphthol, 20 ml methanol, 80 ml PBS, and 20 μl aqueous hydrogen peroxide). The colored filter was washed with distilled water and compared with the marker filter, demonstrating that polypeptides CORE-N3 and CME-N3N10ΔB contain only one colored protein having a reasonable molecular weight as an expression product of cDNAs originated from serum of HC patients and contained in plasmid pCZCORE and pCZCMEΔB, respectively.

Cells transformed with pBlueN3N10-1 or plasmid pBlueN3N10-2, both of which encode polypeptide encoded by clone N3N10, expressed HCV polypeptides showing the same pattern on the detection. A protein of molecular weight of about 22 kD was expressed which corresponds to calculated molecular weight of an expression product from core-encoding gene contained in clone N3N10. Thus, said core-encoding gene, when expressed, gives a protein of calculated molecular weight of about 22 kD (without modification). As the result, the expressed product was identified as hepatitis C associated antigenic polypeptide presumably derived from HCV core protein.

Example 8

Comparison of Clones Obtained in Example 2 [2] and [3]

Three clones corresponding to SEQ ID NO 1 were separately cloned using serum from a HC patient according to the method described in Example 2 [2] (using random primers) and sequenced. On the other hand, three clones corresponding to SEQ ID NO 1 were separately cloned using serum from the same HC patient according to the method described in Example 2 [3] (using antisense primers) and sequenced.

Clones obtained using random primers had the same base sequence as that shown by SEQ ID NO 1, whereas the synthetic primers S1 and AS1 were used, two of three clones obtained independently had the base sequence of SEQ ID NO 1, and one clone had a base sequence which differed from that of SEQ ID NO 1 as to three nucleotides. Thus, at No. 345, A was changed to C, No.322 A changed to T, and No. 95 A changed to C. These differences indicate that a patient is infected at least 2 kinds of viruses.

The above facts demonstrate that there are no substantial difference between clones obtained by methods in Example 2 [2] and those obtained in Example 2 [3].

Example 9

Synthesis of cDNA

[1] Preparation of RNA Sample Solution

RNA sample solution was prepared by resolving the dried nucleic acid obtained in Example 1 in 30 μl of water containing 10 μl of ribonuclease inhibitor (100 U/μl, Takara Shuzo, Japan).

[2] Synthesis of cDNA Using Antisense Primer

To 2 μl of RNA sample solution prepared in above [1] was added 1 μl of 15 pmol/μl anti-sense primer (synthesized primer MS122, MS157 or MS148), 2 μl of 10 x RT buffer (100 mM Tris-HCl (pH8.3), 500 mM KCl), 4 μl of 25 mM $MgCl_2$, 8 μl of 2.5 mM 4dNTPs, 1 μl of water and the mixture incubated at 65 °C for 5

min then at room temperature for 5 min. To the mixture was added 1 µl of reverse transcriptase (25 U, Life Science), 1 µl of ribonuclease inhibitor (100 U/µl, Takara Shuzo) and the mixture incubated at 37 °C for 20 min, at 42 °C for 30 min, and finally at 95 °C for 2 min, which was followed by an immediate cooling to 0 °C (synthesis of cDNA).

Amplification of DNA containing specific sequences was conducted by PCR (Saiki et al., Nature 324: 126 (1986)). Thus, 100 µl mixture containing ten µl of cDNA solution, 10 µl of 10 x PCR buffer (100 mM Tris-HCl (pH8.3), 500 mM KCl, 15 mM $MgCl_2$, 1% gelatin), 8 µl of 2.5 mM 4 dNTPs, 2 µl of 150 pmol/µl synthetic DNA primer (the same primer as used in the synthesis of cDNA), 3 µl of 15 pmol/µl synthetic DNA primer (a counterpart of pair of primers, i.e.,MS122-MS123, MS157-MS156, or MS148-MS146) and water was incubated at 95 °C for 5 min, then immediately cooled to 0 °C. One minute later, it was mixed with 0.5 µl of Taq DNA polymerase (7 U/µl, AmpliTaq™ Takara Shuzo) and overlaid with mineral oil. The resultant sample was then subjected to PCR. PCR was conducted by repeating 25 times of reaction cycle, which comprises the following treatments: at 95 °C for 1 min; at 40 - 55 °C for 1 min; and at 72 °C for 1 - 5 min in DNA Thermal Cycler (Parkin Elmer Cetus). Finally, the reaction mixture was incubated at 72 °C for 7 min, which was followed by phenol/chloroform extraction and ethanol precipitation to obtain different amplified DNA fragments derived from either of above-mentioned pairs of primers.

Example 10

Cloning and Sequencing of Amplified DNA Fragments

Dried DNA fragment (at least 1 pmole) obtained in the above Example 9, [2] was blunt-ended with T4 DNA polymerase (Toyobo) and 5'-end phosphorylated with polynucleotide kinase (Toyobo) and ligated into SmaI site of multi-cloning sites of 5 ng to 10 ng of pUC19 cloning vector. The cloning vector had been previously treated as follows: digestion with a restriction enzyme SmaI (Toyobo), phenol/chloroform extraction, ethanol precipitation, 5'-end dephosphorylation with alkaline phosphatase (Behringer-Manheim) (Molecular Cloning (1982) Cold Spring Harbor), phenol/chloroform extraction, and ethanol-precipitation. The ligated DNA was used to transform a competent E.coli JM 109 or DH5 cells (Toyobo). The transformation was carried out according to the protocol of the manufacture's instruction (COMPETENT HIGH, Toyobo). Plasmid clones were recovered from transformed cells conventionally. At least 20 transformants were obtained using pUC19 cloning vectors containing either of DNA fragments obtained using either of pairs of primers in the same manner as that described in Example 9, [2].

Plasmid DNA was isolated from corresponding transformant by an usual method and sequenced. The determination of base sequence was conducted by means of Fluorescent DNA Sequencer (GENESIS 2000, Dupont) using, as sequence primer, the following synthetic primers:
5' d(GTAAAACGACGGCCAGT)3' (SEQ ID NO 143) and
5'd(CAGGAAACAGCTATGAC)3' (SEQ ID NO 144) for the + and - strands of DNA fragment to be sequenced.

Base sequences of DNA fragments are given in SEQ ID NO 13 to 27, which show the base sequences of + strand of HCV genes inserted into each plasmid used for the transformation. These clones are double stranded DNA. Plasmids used for the sequencing of clones N19-1, N19-2 and N19-3 were designated as plasmids pUCN19-1, pUCN19-2 and pUCN19-3, respectively. Each plasmid contained one DNA molecule corresponding to each DNA fragment. In the same manner, a plasmid which contains a single clone and is used for the sequencing of the same is designated by adding a prefix "pUC" to the name of the clone.

These base sequences represents base sequences of clones obtained by cloning the cDNA synthesized from RNA isolated from serum of patient(s) suffering from HC. Therefore, these sequences are specific for clones originated from serum of HCV-infected patients but can not be found or obtained from serum of healthy subjects. Thus, cDNA prepared from RNA (if there are any) obtained from a healthy subject under more strict conditions, for instance, by increasing (3 or 4 folds) the reaction cycles of PCR in Example 9 [2] and [3], by repeating them 60 - 100 times, did not show any homology in base sequence with those shown in SEQ ID NO 13 to 27. Consequently, base sequences of clones shown in SEQ ID NO 13 to 27 are specific for those obtained from serum of HC patient.

The base sequences of DNA fragments were compared with a known base sequence of HCV gene. As can be seen from the fact that three clones N19-1, N19-2 and N-193 were obtained from serum of one HC patient in Example 9 [2] using primers MS122 and MS123, there must be more than one virus in a patient.

Example 11

Preparation of Clones N27MX24A-1 and N27MX24B-1

[1] Preparation of Clones N19MX24A-1 and N19MX24B-1

One μl (about o.5 to 1 μg/μl) of each DNA fragment from clones N19-1 and MX24-4 was added into a reaction mixture containing 10 μl of 10 x PCR buffer (100 mM Tris-HCl (pH8.3), 500 mM KCl, 15 mM $MgCl_2$, 1% gelatin), 8 μl of 2.5 mM 4 dNTPs, 5 μl each of 20 pmol/μl synthetic primers S2 and AS3, and 76.5 μl of water. After an intimate mixing, the mixture was heated at 95 °C for 5 min, then immediately cooled to 0 °C. One minute later, to the mixture was added 0.5 μl of Taq DNA polymerase (7 U/μl, AmpliTaq™ Takara Shuzo), mixed and overlaid with mineral oil. The sample was then subjected to PCR. PCR was conducted by repeating 25 times of reaction cycle, which comprises the following treatments: at 95 °C for 1 min; at 40 °C for 1 min; and at 72 °C for 2 min in DNA Thermal Cycler (Parkin Elmer Cetus). It was followed by an incubation at 97 °C for 2 min. The mixture was immediately cooled to 0 °C, kept at 0 °C for 2 min, mixed with 0.5 μl of Taq DNA polymerase (7 U/μl, AmpliTaq™ Takara Shuzo). The sample was then treated in the same manner as the above by repeating 25 times of reaction cycle, which comprises the following treatments: at 95°C for 1 min; at 50 °C for 1 min; and at 72 °C for 2 min. After the final treatment at 72 °C for 7 min, the resultant reaction solution was treated with phenol/chloroform then precipitated with ethanol. The amplified DNA samples were fractionated on agarose gel electrophoresis and a gel containing a fragment having a desired length was removed (Molecular Cloning (1982) Cold Spring Harbor) to isolate the DNA fragment therefrom conventionally. The resultant DNA fragment was then modified as described in Example 10 and ligated into SmaI site of multi-cloning sites of pUC19, cloned and screened as described in Example 10 to obtain plasmids pUCN19MX24A-1 and pUCN19MX24B-1. The resultant cDNAs derived from serum of HC patient were referred to as clones N19MX24A-1 and N19MX24B-1, of which base sequences are given in SEQ ID NO 29 and 30.

[2] Preparation of Clone N27N19-1

Two overlapping clones N27-3 and N19-1 were ligated by taking advantage of unique restriction site which exists in the overlapping regions of the both clones. Upon digestion with restriction enzyme MluI, clone N27-3 is cleaved at the 3' site of a nucleotide No. 330 (A) and clone N19-1 at the 3' site of a nucleotide No.51 (A). The ligation of clones N27-3 and N19-1 was accomplished on the basis of an assumption that plasmids pUCN27-3 and pUCN19-1 contain each DNA fragment in the same orientation. Thus, plasmid pUCN27-3 was digested with HindIII and MluI to isolate a DNA fragment containing 5' region of clone N27-3 which comprises a HindIII-SmaI DNA fragment of plasmid pUC19 attached to the 5' end of the clone N27-3, a cDNA derived from serum of HC patient. The DNA fragment was then exchanged with a HindIII-MluI fragment of clone N19-1 containing 3' region of said clone, cloned and screened to obtain a plasmid pUCN27N19-1. The plasmid pUCN27N19-1 contained the desired clone N27N19-1 comprising clones N27-3 and N19-1 ligated without overlapping. The base sequence of clone N27N19-1 is shown in SEQ ID NO 28.

[3] Preparation of Clones N27MX24A-1 and N27MX24B-1

Overlapping clones N27-3 and either of clones N19MX24A-1 and N19MX24B-1 were ligated by taking advantage of unique restriction site which exists in the overlapping regions of the both clones. Upon digestion with restriction enzyme MluI, clone N27-3 is cleaved at the 3' site of a nucleotide No. 330 (A) and clones N19MX24A-1 and N19MX24B-1 at the 3' site of a nucleotide No.71 (A). The ligation of clones was accomplished on the basis of an assumption that plasmids pUCN27-3, pUCN19MX24A-1 and pUCN19MX24B-1 contain each DNA fragment in the same orientation. Thus, plasmid pUCN27-3 was digested with HindIII and MluI to isolate a 363 bp DNA fragment which comprises a HindIII-SmaI DNA fragment of plasmid pUC19 attached to the 5' end of the clone N27-3, a cDNA derived from serum of HC patient. The DNA fragment was then exchanged with a 363 bp DNA fragment of clone N19MX24A-1 or N19MX24B-1 which were excised from plasmids pUCN19MX24A-1 and pUCN19MX24B-1 with HindIII and MluI restriction enzymes, followed by cloning and screening. The resultant plasmids pUCN27MX24A-1 and pUCN27MX24B-1 contained the desired clones N27MX24A-1 and N27MX24B-1, each comprising a clone N27-3 and either of clones N19MX24A-1 and N19MX24B-1 ligated without overlapping. The base sequences of clones N27MX24A-1 and N27MX24B-1 are shown in SEQ ID NO 31 and 32, respectively.

Example 12

Modification of DNA for the Expression of HCV Polypeptide Encoded by Clones N27MX24A-1 and N27MX24B-1

[1] Modification of DNA for the Expression of HCV Polypeptide Encoded by Clones N27MX24A-1 and N27MX24B-1 in E.coli

Clones N27MX24A-1 and N27MX24B-1 appeared to encode an open reading frame from the nucleotide No.2 (C) derived from HCV gene, which can be expressed by inserting an ATG initiation codon inframe and upperstream from said gene so that the expression of the DNA might be properly effected in host cells. The insertion of an ATG initiation codon at the upperstream from 5' terminus of said gene may be accompanied by an addition of a foreign polypeptide which is not encoded by HCV gene to the N' terminus (amino terminus) of an amino acid sequence of SEQ ID NO 31 or 32. When an expression vector containing an initiation codon for E. coli. is used, a DNA fragment from the clone is ligated to the vector such that frame of said DNA is in fonfirmity with that of the ATG codon. The modification of DNA can be carried out by PCR. The modification procedures are hereinafter illustrated using clone N27MX24A-1. It will be appreciated that clone N27MX24B-1 can be modified just in the same manner.

The following synthetic oligonucleotide primers were used.

5' primer:
MS2724-1; 5' GCAAGCTTATGCGGATCCCACAAGCCGTGGTGGAT 3' (SEQ ID NO 152)
5' primer for inserting DNA fragment into a vector containing initiation codon.
MS2724-2; 5' CGGATCCCACAAGCCGTGGTGGAT 3' (SEQ ID NO 153)
3' primer:
MS2724-3; 5' GCGAATTCAGATCTTCATCACTCTAAGGTGGCGTCGGCGTGGG 3' (SEQ ID NO 154)

The synthetic DNA was adjusted to 20 pmol/ml before use.

PCR was carried out in the same manner as described in the above according to Saiki's method in a total volume of 100 µl containing 100 ng of plasmid pUCN27MX24A-1, as a template, and 2 µl each of 3' and 5' primers. The reaction mixture was heated at 95 °C for 5 min and quenched at 0 °C. One minute later, to the mixture was added 0.5 µl of Taq DNA polymerase (7 U/ml, AmpliTaq™ Takara Shuzo), mixed thoroughly and overlaid with mineral oil. The sample was reacted by repeating 25 cycles of treatments which comprises: at 95 °C for 1 minute; at 60 °C for 1 min; and at 72 °C for 5 min in DNA Thermal Cycler (Parkin Elmer Cetus). The resultant reaction solution was extracted with phenol/chloroform and precipitated with ethanol conventionally. The amplified DNA samples were digested with HindIII and EcoRI (when MS2724-2 was used as 5'primer, the DNA was blunt ended with T4 DNA polymerase and digested with EcoRI), and fractionated on acrylamide gel electrophoresis and the gel containing a DNA fragment of desired length was extracted (Molecular Cloning, Cold Spring Harbor (1982)).

The resultant DNA fragment was then ligated into HindIII (when MS2724-2 was used as 5'primer, SmaI) and EcoRI sites of a cloning vector pUC19, cloned and screened to obtain plasmid pUCHN27MX24A-1 (plasmid pUCH2N27MX24A-1, when MS2724-2 was used). The resultant plasmid was sequenced. Clone CHN27MX24A-1 comprises a DNA fragment shown by a base sequence of SEQ ID No 31, 32 except that the 5' terminal C was removed and the following DNA fragment:

```
5′ GCAAGCTTATG 3′
3′ CGTTCGAATAC 5′ (SEQ ID NO 155)
```

which comprises a HindIII restriction site followed by an initiation codon ATG, was added thereto, and 3' terminal two bases (AA) were removed from the base sequence of SEQ ID NO 31 and the following DNA fragment:

```
5′ TGATGAAGATCTGAATTCGC 3′
3′ ACTACTTCTAGACTTAAGCG 5′ (SEQ ID NO 156)
```

which comprises two termination codons, and EcoRI sites from 5' to 3', was added thereto.

Another clone H2N27MX24A-1 obtained using primers MS2724-2 and MS2724-3 was sequenced showing that said clone has no additional DNA fragment at the 5' terminus but, at the 3' terminus, has the same additional DNA fragment as that of the above clone HN27MX24A-1.

[2] Modification of a DNA Fragment for the Expression of HCV Polypeptide Comprising 106 Amino Acid Sequence from No. 109 to 214 of SEQ ID NO 31, 32 in E.coli

A DNA fragment encoding a polypeptide comprising 106 amino acid sequence from Nos. 109 to 214 amino acids of SEQ ID NO 31, 32 appeared to encode an open reading frame (ORF) from HCV gene, which can be expressed by inserting an ATG initiation codon in frame and upperatream from said gene. The insertion of an ATG initiation codon at the upperstream from 5' terminus of said gene may be accompanied by an addition of a foreign polypeptide to the N' terminus (amino terminus) of said polypeptide. When an expression vector containing an initiation codon for E. coli. is used, a DNA fragment from the clone is ligated to the vector such that the frame of said DNA is in confirmity with that of the codon. The modification of DNA can be carried out by PCR using the following synthetic oligonucleotide primers.

5' primer:
MSHNS1-1: 5' GCAAGCTTATGTTCAACGCGTCCGGATGTCCGGA 3' (SEQ ID NO 157)
5' primer for inserting DNA fragment into a vector containing initiation codon.
MSHNS1-2: 5' TTCAACGCGTCCGGATGTCCGGA 3' (SEQ ID NO 158)
3' primer:
MSHNS1-3: 5' GCGAATTCAGATCTTCATCAACAACCGAACCAGTTGCCCTGCG 3' (SEQ ID NO 159)

The synthetic DNA was adjusted to 20 pmol/ml before use.

PCR was carried out in the same manner as described in the above according to Saiki's method in a total volume of 100 µl containing 100 ng of plasmid pUCN27MX24A-1 (or plasmid pUCN27MX24B-1), as a template, and 2 µl each of 3' and 5' primers. The reaction mixture was heated at 95 °C for 5 min and quenched at 0 °C. One minute later, to the mixture was added 0.5 µl of Taq DNA polymerase (7 U/ml, AmpliTaq™ Takara Shuzo), mixed thoroughly and overlaid with mineral oil. The sample was reacted by repeating 25 cycles of treatments which comprises: at 95 °C for 1 minute; at 60 °C for 1 min; and at 72 °C for 5 min in DNA Thermal Cycler (Parkin Elmer Cetus). The resultant reaction solution was extracted with phenol/chloroform and precipitated with ethanol conventionally. The amplified DNA samples were digested with HindIII and EcoRI (when MSHNS1-2 was used as 5'primer, the DNA was blunt ended with T4 DNA polymerase and digested with EcoRI), and fractionated on acrylamide gel electrophoresis and extracted (Molecular Cloning, Cold Spring Harbor (1982)).

The resultant DNA fragment was then ligated into HindIII (when MSHNS1-2 was used as 5'primer, SmaI) and EcoRI sites of a cloning vector pUC19, cloned and screened to obtain plasmid pUCH48 (plasmid pUCH48-2, when primers MSHNS1-2 and MSHNS1-3 were used). The resultant plasmid was sequenced, demonstrating that the clone H48 has a modified base sequence of SEQ ID NO 31, 32 wherein, at the 5' site of No. 326 T, the following DNA fragment:

5′ GCAAGCTTATG 3′

3′ CGTTCGAATAC 5′ (SEQ ID NO 155)

which fragment comprises a HindIII restriction site at 5' terminus and ATG initiation codon, followed by an initiation codon ATG, was added, and, at the 3' terminus, the following DNA fragment:

5′ TGATGAAGATCTGAATTCGC 3′

3′ ACTACTTCTAGACTTAAGCG 5′ (SEQ ID NO 156)

which fragment comprises two termination codons, BglII and EcoRI sites from 5' to 3', was added.

Another clone H48-2 obtained using primers MSHNS1-2 and MSHNS1-3 was sequenced showing that said clone has no additional DNA fragment at the 5' site of No. 326 T, while has the same additional DNA fragment as that of clone H48.

[3] Modification of a DNA Fragment for the Expression of HCV Polypeptide Comprising 92 Amino Acid Sequence from No. 233 to 324 of SEQ ID NO 31, 32 in E.coli

A DNA fragment encoding a polypeptide comprising 92 amino acid sequence from Nos. 233 to 324 amino acids of SEQ ID NO 31, 32 appeared to encode an open reading frame (ORF) from HCV gene. The modification of DNA fragment was conducted in the same manner as that used for the modification of DNA fragment encoding a polypeptide of 106 amino acid sequence from amino acid Nos. 109 to 214 of SEQ ID NO 31, 32 in the above [2] except that the following primers were employed.

5' primer:

MSNS1-4: 5' GCAAGCTTATGATCGGGGGGGTCGGCAACAATAC 3' (SEQ ID NO 160)

5' primer for inserting DNA fragment into a vector containing initiation codon.

MSNS1-5: 5' ATCGGGGGGGTCGGCAACAATAC 3' (SEQ ID NO 161)

3' primer:

MSNS1-6: 5' GCGAATTCAGATCTTCATCAAAGCTCTGATCTATCCCTGTCCT 3' (SEQ ID NO 162)

Each synthetic DNA was adjusted to 20 pmole/μl.

The resultant clones are H49 (primers MSNS1-4 and MSNS1-6) and H49-2 (primers MSNS1-5 and MSNS1-6).

[4] Modification of DNA for the Expression of HCV Polypeptide Encoded by Clones N27MX24A-1, N27MX24B-1, H48-2 and H49-2 in Insect Cells

Clones N27MX24A-1 and N27MX24B-1 appears to contain an ORF which starts from the nucleotide No.2 (C). Clones H48-2 and H49-2 contain an ORF which starts from the nucleotide No.1. For the expression of polypeptide encoded by these ORF, an initiation codon ATG is inserted in frame and at an appropriate site upperstream from said gene so that the expression of the DNA might be properly effected in insect cells. The insertion of an ATG initiation codon at the upperstream from 5' terminus of said gene may be accompanied by an addition of a foreign polypeptide which is not encoded by HCV gene to the N' terminus (amino terminus) of amino acid sequence encoded by clones N27MX24A-1, N27MX24B-1, H48-2 and H49-2. When an expression vector containing an initiation codon for insect cells is used, a DNA fragment from the clone is ligated to the vector such that the frame of said DNA is in confirmity with that of the initiation codon on said vector. It also can be accompanied by an addition of a foreign polypeptide which is not encoded by HCV gene to the N' terminus (amino terminus) of amino acid sequence encoded by clones N27MX24A-1, N27MX24B-1, H48-2 and H49-2. The modification of vector DNA was carried out by PCR. Although the modification procedures are described using clone N27MX24A-1, it can be conducted as well using clone N27MX24B-1. When insect cells were transfected with the DNA and cultivated, clones N27MX24A-1, N27MX24B-1 were expressed as in the fused form as a precursor, which was then processed, glycosylated incompletely to give a mature glycoprotein of about 70 kD accumulated intracellularly. The modification of DNA of clones N27MX24A-1, N27MX24B-1, H48-2 and H49-2 was carried out by PCR using the following synthetic DNA as primers.

5' primers:

MS2724-4: 5' GCGTCGACGCTAGCATGCGGATCCCACAAGCCGTGGTGGAT 3' (SEQ ID NO 163)

MSNS1-7: 5' GCGTCGACGCTAGCATGTTCAACGCGTCCGGATGTCCGGA 3' (SEQ ID NO 164)

MSNS1-8: 5' GCGTCGACGCTAGCATGATCGGGGGGGTCGGCAACAATAC 3' (SEQ ID NO 165)

3' primer:

MS2724-5: 5' GCGAATTCGCTAGCTCACTCTAAGGTGGCGTCGGCGTGGG 3' (SEQ ID NO 166)

MSNS1-9: 5' GCGAATTCGCTAGCTCAACAACCGAACCAGTTGCCCTGCG 3' (SEQ ID NO 167)

MSNS1-10: 5' GCGAATTCGCTAGCTCAAAGCTCTGATCTATCCCTGTCCT 3' (SEQ ID NO 168)

These three synthetic DNAs were separately adjusted to 20 pmol/ml before use.

The PCR was carried out using the same reaction solution and worked up in the same manner as described in the above [1] except that plasmid pUCHN27MX24A-1 (primers MS2724-4 and MS2724-5), pUCH48 (primers MSNS1-7 and MSNS1-9) or pUCH49 (primers MSNS1-8 and MSNS1-10) was used as a template plasmid. PCR was accomplished by repeating 10 times of reaction cycles consisting of: 1 min at 95 °C; 1 min at 50 °C and 5 min at 72 °C ; and then 20 times of reaction cycles consisting of: 1 min at 95 °C; 1 min at 65 °C; 5 min at 72 °C. When plasmid pUCHN27MX24A-1, as a template DNA, and primers MS2724-4 and MS2724-5 were used, a desired 1268 bp DNA fragment was obtained. The other combination of plasmid pUCH48 and primers MSNS1-7 and MSNS1-9 gave a desired 352 bp DNA fragment and that of pUCH49 and primers MSNS1-8 and MSNS1-10 gave a desired 322 bp DNA fragment.

Each DNA fragment was digested with NheI, fractionated on acrylamide gel electrophoresis and extracted by convenional means (Molecular Cloning, Cold Spring Harbor (1982)) to obtain a DNA fragment of desired length. The resultant DNA fragment was then ligated into NheI restriction site of a transfer vector pBlueBac (Invitrogen), cloned and screened for a clone which contains a single DNA fragment inserted at NheI site. Thus, plasmids pBlueN27MX24A-1 derived from 1268 bp DNA obtained by primers MS2724-4 and MS2724-5, pBlueH48 derived from 352 bp DNA fragment obtained by primers MSNS1-7 and MSNS1-9, and pBlueH49 derived from 322 bp DNA fragment obtained by primers MSNS1-8 and MSNS1-10 were prepared.

According to the teaching shown in the protocol given by Invitrogen, the expression unit of these plasmid contains DNA fragment derived from HCV gene oriented forward and ligated to the NheI cloning site downstream from a poyhedrin promoter.

Example 13

Expression of HCV Polypeptides Encoded by Clones HN27MX24A-1, HN27MX24B-1, H2N27MX24A-1, H2N27MX24B-1, H48, H48-2, H49, and H49-2

[1] Expression of Polypeptide Encoded by Clone HN27MX24A-1, HN27MX24B-1, H48, or H49 in E.coli

Each clone encodes a part of polypeptide encoded by cDNA originated from serum of HC patient. The polypeptide encoded by each clone was expressed directly in E.coli, as it is, by subcloning said clone into an expression vector pCZ44 (Japanese Patent Publication No. 124387/1989).

A clone was digested thoroughly with restriction enzymes HindIII and BglII, extracted with phenol/chloroform, precipitated with ethanol, separated on acrylamide gel electrophoresis. From the gel was extracted a larger DNA fragment having cohesive HindIII- and BglII-restricted ends (Molecular Cloning, Cold Spring Harbor, 1982). The expression vector pCZ44 was digested with HindIII and BglII. The larger fragment containing a region functional for the replication in E.coli was separated, treated in the same manner, ligated to the HindIII-BglII fragment obtained from a clone such that the vector contains only one insertion, and cloned conventionally. The resultant plasmids were designated as plasmid pCZ2724A-1, pCZ2724B-1, pCZ48 andpCZ49 after clones HN27MX24A-1, HN27MX24B-1, H48, or H49, respectively.

Alternatively, an expression vector was constructed using an expression vector pGEX-2T (Pharmacia), in stead of pCZ44, for the expression of a fused protein between a desired polypeptide and GST. The construction was carried out substantial in accordance with the protocol taught by the manufacture (Pharmacia). Thus, the expression vector pGEX-2T was digested with BamHI. The linearized vector was ligated with a HindIII linker, and ligated with a HindIII-EcoRI DNA fragment prepared from a clone to yield expression vectors pGEX2724A-1, pGEX2724B-1, pGEX48 and pGEX49.

E.coli JM109 strain transformed with plasmid pCZ2724A-1, pCZ2724B-1, pCZ48 or pCZ49 was grown in L-Broth at 37 °C overnight (Molecular Cloning, Cold Spring Harbor, 1982). The cultured broth was diluted 50-folds by inoculating it into a freshly prepared L-Broth and the cultivation continued with shaking at 30 °C for 2 hr. At this time, IPTG was added to the culture to a final concentration of 2 mM and cultured for more than 3 hr in order to induce the expression of DNA encoding HCV-originated polypeptide by single-clone-derived transformants (E.coli JM 109 cells transformed solely by one plasmid derived from corresponding clone). Base sequences of cDNA contained in clones HN27MX24A-1 and HN27MX24B-1, and amino acid sequences deduced therefrom are shown in SEQ ID NO 31. Base sequences of cDNA contained in clones H48 and H49, and deduced amino acid sequence are shown by amino acid sequences from amino acid No. 109 to 214 and from amino acid No. 233 to 324 of in SEQ ID NO 31, respectively.

In the same manner as the above, clone HN27MX24B-1 can be used in stead of clone HN27MX24A-1 to give a polypeptide encoded by said clone. The deduced amino acid sequence of the polypeptide is shown in SEQ ID NO 32.

As mentioned in the above, plasmids pGEX2724A-1, pGEX2724B-1, pGEX48 and pGEX49 can be used to obtain transformants capable of expressing a fused protein include desired polypeptide and GST. The plasmid encodes a fused protein GST-CORE comprising GST, which has a thrombin-cleaving site at its C-terminus, and a polypeptide derived from a clone HN27MX24A-1, HN27MX24B-1, H48 or H49. Fused protein comprising at C-terminal region a HCV polypeptide was produced in E.coli transformant transformed with either of plasmids pGEX2724A-1, pGEX2724B-1, pGEX48 and pGEX49, by culturing the cells in the same manner as that used to produce polypeptide in transformants harboring plasmid pCZ2724A-1, pCZ2724B-1, pCZ48 or pCZ49 in the presence of IPTG.

[2] Expression of Polypeptides Encoded by Clones H2N27MX24A-1, H2N27MX24B-1, H48-2, and H49-2

Polypeptides were expressed in E.coli using cDNA contained in clones H2N27MX24A-1, H2N27MX24B-1, H48-2, and H49-2 obtained from serum of HC patient in the same manner as the above [1]. The cDNA used was that contained in clone H2N27MX24A-1, H2N27MX24B-1, H48-2, or H49-2, which clone had been previously isolated and sequenced.

Expression plasmid for each clone was constructed using pOFA (Japanese Patent Publication (KOKAI) No.84195/1990). DNA fragment from each clone was blunt-ended with T4 DNA polymerase. The expression vector pOFA was digested with KpnI and blunt-ended with T4DNA polymerase. Thus obtained DNA fragments were ligated, cloned and screened for a clone having a insertion of one DNA fragment. Thus, the desired plasmids pOFA2724A-1, pOFA2724B-1, pOFA48 and pOFA49 were prepared by subcloning a clone so that the + strand capable of expressing HCV protein should be inserted appropriately for the correct translation of said strand. It was confirmed that the cDNA from HCV was properly reconstructed by the determination of base sequence and restriction enzyme mapping of each plasmid.

Cultivation was carried out in the presence of IPTG in order to induce the expression of DNA encoding HCV-originated polypeptide by single-clone-derived transformants (E.coli JM 109 cells transformed solely by one plasmid). Base sequences of cDNAs derived from serum of a HC patient contained in clones H2N27MX24A-1, H2N27MX24B-1, H48-2 and H49-2 and amino acid sequences deduced therefrom are shown by the amino acid sequences of SEQ ID NO 31, 32, amino acid sequence from No. 109 to 214 of SEQ ID NO 31, and that from No. 233 to 324 of SEQ ID NO 31, respectively.

[3] Expression of Polypeptide Encoded by Clones N27MX24A-1, N27MX24B-1, H48-2 and H49-2 in Insect Cells

The expression of HCV-originated glycoprotein encoded by plasmid pBlueN27MX24A-1, pBlueH48 and pBlueH49 prepared in Example 12 [4] was conducted substantial in accordance with a known expression manual for baculovirus (MAXBAC™ BACULOVIRUS EXPRESSION SYSTEM MANUAL VERSION 1.4, hereinafter, referred to as Maxbac, Invitrogen).

Plasmids pBlueN27MX24A-1, pBlueH48 and pBlueH49 prepared in Example 12 [4] by inserting DNA fragment containing HCV gene at the NheI site of a transfer vector pBlueBac (Maxbac, pp.37), were recovered from E.coli host cells transformed thereby, and purified according to the method of Maniatis et al.(Molecular Cloning, Cold Spring Harbor Laboratory, pp.86 - 96 (1982)). Thus, a large amount of HCV gene-containing transfer plasmid DNA was obtained. Sf9 cells were cotransfected with 2 $\mu$g of a plasmid containing a DNA fragment from HCV gene and 1 $\mu$g of AcNPV viral DNA (Maxbac, pp.27). Sf9 cells were grown in TMN-FH medium (Invitrogen) containing 10% FCS (fetal calf serum) in a 6 cm dish until a cell density reached to about 2 x $10^6$/plate. The TMN-F medium was removed and a 0.75 ml Grace medium (Gibco) containing 10% FCS was added thereto. To the DNA mixture described in the above was added 0.75 ml of transfection buffer (attached to the kit) was thoroughly mixed by vortex and gradually added dropwise onto the Grace medium. After the culture being allowed to stand for 4 hr at 27 °C, Grace medium was replaced with 3 ml of TMN-FH medium containing 10% FCS and the dish incubated at 27 °Cfor 6 days. Three days from the incubation, there observed a few multinucleate cells and on sixth day, almost all the cells were multinuclear. The supernatant was taken into a centrifuging tube and centrifuged at 1,000 rpm, 10 min to obtain the supernatant as a cotransfected viral solution.

The cotransfected viral solution contains about $10^8$ viruses/ml and 0.5% of which were recombinant viruses. The isolation of recombinant virus was carried out by a plaque isolation method described below.

Thus, cells were adsorbed onto a 6 cm dish by seeding 1.5 x $10^6$ cells on medium and removing the medium completely. To the dish was added 100 $\mu$l of a diluted viral solution ($10^{-4}$ and $10^{-5}$ folds), separately and incubated at room temperature for 1 hr while slanting the dish every 15 min to spread the virus extensively. X-gal medium containing agarose was prepared by adding 5-bromo-4-chloro-3-indolyl-$\beta$-D-galactoside to a final concentration of 150 $\mu$g/l (Maxbac, pp.16-17) to a warm medium which had been prepared by autoclaving 2.5% baculovirus agarose (Invitrogen) at 105 °C for 10 min, mixing with TMN-FH medium containing 10% FCS preheated at 46 °C at the mixing ratio of 1 : 3, and keeping the temperature at 46 °C.

After the completion of infection, virus solution was aspirated thoroughly from the dish and 4 ml of the warm X-gal medium containing agarose (previously prepared) was gently added to every dish not to peel off cells. The dish kept open by slightly sliding a lid until the agarose solidified and dried, and thereafter the dish covered, turned upside down, and incubated at 27 °C for 6 days. The plaques were observed under a phase difference microscope to find blue plaques which do not form multinucleate cells. Agarose containing blue recombinant plaques were removed with a Pasteur pipet and suspended into 1 ml of TMN-FH medium by pipetting many times. The above process whcih comprises: infection, 6-day incubation, and isolation of virus containing transfer plasmid DNA is called the "plaque method". The plaque method was repeated using 100 μl of viral suspension. After repeating said process three times, there obtained a recombinant virus having a gene encoding HCV glycoprotein free from contamination with that of wild-type strain.

A viral solution of the primary recombinant virus was prepared by aspirating plaques with a Pasteur pipet, and mixing thoroughly with 1 ml of TMN-FH medium. Because the primary viral solution was low in virus density for infection, it required further treatments for concentration. Thus, 100 μl of viral solution was adsorbed onto Sf9 cells grown in a 6 cm dish to a semi-confluent, and 4 ml of TMN-FH medium was added thereto and incubated three days. The culture supernatant was recovered to yield a recombinant viral solution for infection.

For the production of HCV structural protein , a suspension of Sf9 cells in TMN-FH medium containing 10% FCS (5 x $10^6$ cells/10 ml medium) was added into a 9 cm dish and kept 1 hr for adsorption. After the removal of medium, 250 μl of recombinant viral solution was added to the dish and spread extensively. To the dish was added 10 ml TMN-FH medium containing 10% FCS and incubated at 27 °C for 4 days. The cells expressing recombinant glycoprotein of HCV were harvested by scraping up and suspended into 1,000 ml of phosphate buffered saline.

Thus, HCV-derived glycoprotein was expressed in Sf9 cells transfected with said virus.

Example 14

Identification of Expression Products as HCAg

Each expression product obtained in Example 13 was identified as HCAg because it reacted immunologically with antiserum obtained from HC patients.

Identification was conducted by Western blot technique.

E. coli cells transformed with either of expression plasmids pCZ2724A-1, pCZ2724B-1, pCZ48, pCZ49, pOFA2724A-1, pOFA2724B-1, pOFA48, pOFA49, pGEX2724A-1, pGEX2724B-1, pGEX48, pGEX49, pBlueN27MX24A-1, pBlueH48 and pBlueH49 for polypeptides encodid by clones HN27MX24A-1, HN27MX24B-1, H2N27MX24A-1, H2N27MX24B-1, H48, H48-2, H49, and H49-2 were grown in the presence of IPTG for 3 hr or a overnight in the same manner as described in Example 13.

Recombinant strains were harvested by centrifuging 1,000 μl of the cultured broth at 6,500 rpm, 10 min. The pellet was dissolved into a sample solution (50 mM Tris-HCl, pH6.8 containing 2% SDS, 5% mercaptoethanol, 10% glycerin, and 0.005% bromophenol blue) for SDS-polyacrylamide gel electrophoresis to a final volume of 0.2 ml. Sf9 cells infected with viruses which had been treated more than 3 times by plaque method were collected by scraping up and suspended into 1,000 ml of PBS and 100 μl of the suspension was centrifuged at 6,500 rpm, 10 min to pellet the cells. The pellet was dissolved into a sample solution for SDS-polyacrylamide gel electrophoresis to a final volume of 0.2 ml.

The sample solutions were then boiled at 100 °C for 10 min. Ten μl of the boiled solution was loaded onto 0.1% SDS-15% polyacrylamide gel (70 x 85 x 1 mm) together with a marker protein LMW Kit E (low-molecular weight marker protein, Pharmacia). Electrophoresis was carried out at a constant current of 30 mA for 45 min in Tris buffer (25 mM Tris, pH 8.3, 192 mM glycine, 0.1% SDS) as electrode buffer. Thereafter, DNA was transferred electophoretically to a nitrocellulose filter by superposing the gel onto a filter BA-83 (S & S), impressing a constant current of 120 mA for about 20 min between gel (cathode) and the filter (anode) as conventionally.

The transcribed filter was cut to remove a part containing a marker protein (referred to as marker filter) and that containing the sample (referred to as sample filter). The former was stained with 0.1% (w/v) amideblack 10B and the latter immersed into 0.01 M PBS (pH 7.4) containing 5% (w/v) bovine serum albumin (BSA). Serum from a HC patient was diluted 50 times with 0.01 M PBS (pH 7.4) containing 5% (w/v) BSA. To the sample filter was added 10 μl of diluted serum and the filter allowed to stand for 2 hr at room temperature. Thereafter, the filter was washed with PBS containing 0.1% (v/v) Tween 20 for 20 min (x3).

The sample filter was then reacted with 10 ml of horseradish peroxidase conjugated anti human IgG (Gappel) at 37 °C for 1 hr and washed with PBS containing 0.1% (v/v) Tween 20 for 20 min (x3). The filter was then immersed into peroxidase-color-producing solution (60 mg 4-chloro-1-naphthol, 20 ml methanol, 80 ml PBS, and 20 μl aqueous hydrogen peroxide). The colored filter was washed with distilled water and compared with the marker filter, demonstrating that colored protein expressed by transformants transformed with plasmid pCZ2724A-1, pCZ2724B-1, pCZ48 and pCZ49 had a reasonable molecular weight as an expression product of inserted HCV gene and was identified as HCAg.

The expression product from host cells transformed with pOFA-derived plasmids such as pOFA2724A-1, pOFA2724B-1, pOFA48 or pOFA49 is a fused protein consisting of HCV originated polypeptide and OmpF signal peptide of E.coli and the product from host cells transformed with pGEX-derived plasmid such as pGEX2724A-1, pGEX2724B-1, pGEX48 or pGEX49 is also a fused protein consisting of HCV originated polypeptide and GST and thrombin cleaving site wherein the latter two attached at the N-terminus of the former. Each fused protein has a reasonable molecular weight and was also identified as HCAg.

Insect cells transfected with pBlueN27MX24A-1 and pBlueN27MX24B-1, as shown in Example 13 [3], expressed HCV polypeptide encoded by clones N27MX24A-1, N27MX24B-1, H48-2, and H49-2. The expression product (M-gp70) was a glycoprotein of molecular weight of about 70 kD, which has a base sequence corresponding to the base sequence from about No.46 to about No. 395 of SEQ ID NO 31 or 32. Also glycoprotein of HCV was expressed in insect cells tranformed with plasmid pBlueH48 or pBlueH49. Thus produced glycoproteins were encoded by clones H48-2 and H49-2 and had amino acid sequences which correspond to a polypeptide having 106 amino acids from No. 109 to 214 and that of 96 amino acids from No. 233 to 324 of SEQ ID NO 31 and 32, respectively. As a result, theses glycoproteins were identified as HCAg.

## Example 15

## Synthesis of DNA

### [1] Preparation of RNA Sample Solution

RNA sample solution was prepared by resolving the dried nucleic acid obtained in Example 1 in 30 µl of water containing 10 µl of ribonuclease inhibitor (100 U/µl, Takara Shuzo, Japan).

Oligonucleotide primers of the following base sequences were synthesized using a method well known to one of skill. Among them, antisence primers such as MS49, MS88, MS100, MS132, MS152, and MS158 were used for cloning of cDNA.

### [2] Synthesis of cDNA Using Antisence Primer

To 2 µl of RNA sample solution was added 1 µl of 15 pmol/µl anti-sense primer (e.g., synthetic DNA primer such as MS158, MS152, MS132, MS49, MS88, or MS100) 2 µl of 10 x RT buffer (100 mM Tris-HCl (pH8.3), 500 mM KCl), 4 µl of 25 mM $MgCl_2$, 8 µl of 2.5 mM 4dNTPs, 1 µl of water and the mixture incubated at 65 °C for 5 min then at room temperature for 5 min. To the mixture was added 1 µl of reverse transcriptase (25 U, Life Science), 1 µl of ribonuclease inhibitor (100 U/µl, Takara Shuzo) and the mixture incubated at 37 °C for 20 min, at 42 °C for 30 min, and finally at 95 °C for 2 min, which was followed by an immediate cooling to 0 °C to yield cDNA.

Amplification of DNA encoding HCAg was conducted by polymerase chain reaction (PCR) (Saiki et al., Nature 324: 126 (1986)). For the PCR, primers synthesized in the above were used as a pair of: MS48 - MS49; MS86 - MS100; MS97 - MS88; MS135 - MS132; MS155 - MS152; or MS151 - MS158.

A 100 µl mixture containing ten µl of cDNA solution, 10 µl of 10 x PCR buffer (100 mM Tris-HCl (pH8.3), 500 mM KCl, 15 mM $MgCl_2$, 1% gelatin), 8 µl of 2.5 mM 4 dNTPs, 2 µl of 15 pmol/µl synthetic primer (the same primer as used in the preparation of cDNA), 3 µl of 15 pmol/µl synthetic primer (a counterpart of pairs of primers) and water was incubated at 95 °C for 5 min, then immediately cooled to 0 °C. One minute later, it was mixed with 0.5 µl of Taq DNA polymerase (7 U/µl, AmpliTaq™ Takara Shuzo) and overlaid with mineral oil. The resultant sample was then subjected to PCR. PCR was conducted by repeating 25 times of reaction cycle, which comprises the following treatments: at 95 °C for 1 min; at 40 - 55 °C for 1 min; and at 72 °C for 1 - 5 min in DNA Thermal Cycler (Parkin Elmer Cetus). Finally, the reaction mixture was incubated at 72 °C for 7 min, which was followed by phenol/chloroform extraction and ethanol precipitation. The ethanol precipitation was carried out by adding 2.5 volumes of ethanol and either of about 1/10 volume of 3 M acetic acid or an equal volume of 4 M ammonium acetate to the aqueous solution, mixing, centrifuging at 15,000 rpm for 15 min using a rotor of about 5 cm in diameter under cooling at 4 °C to pellet the precipitates, and drying the pellet. Various amplified DNA fragments were obtained using different primers, for the cloning of cDNA and amplification thereof.

## Example 16

## Cloning and Sequencing of Amplified DNA Fragments

The cloning was carried out substantial in accordance with the method of Molecular Cloning, Cold Spring Harbor (1982).

Dried DNA fragment (at least 1 pmole) obtained in the above Example 15 [2] was blunt-ended with T4 DNA polymerase (Toyobo) and 5'-end phosphorylated with polynucleotide kinase (Toyobo) and ligated into smaI site of multi-cloning sites of 5 ng to 10 ng of pUC19 cloning vector. The cloning vector had been previously treated as follows: digestion with a restriction enzyme SmaI (Toyobo), phenol/chloroform-extraction, ethanol-precipitation, 5'-end dephosphorylation with alkaline phosphatase (Behringer-Manheim) (Molecular Cloning (1982) Cold Spring Harbor), phenol/chloroform extraction, and ethanol-precipitation. The ligated DNA was used to transfect into a competent E.coli JM 109 or DH5 cells (Toyobo). The transfection was carried out according to the protocol of the manufacture's instruction (COMPETENT HIGH, Toyobo). Plasmid clones were recovered from transformed cells conventionally. At least 20 transformants were obtained using pUC19 cloning vectors containing either of DNA fragments obtained using either of pairs of primers in the same manner as that described in the above Example 15 [2].

The determination of base sequence of DNA fragment was conducted by Fluorescent DNA Sequencer (GENESIS 2000, Dupont) using, as sequence primer, the following synthetic primers:
5' d(GTAAAACGACGGCCAGT)3' (SEQ ID NO 143) and
5'd(CAGGAAACAGCTATGAC)3' (SEQ ID NO 144) for the + and - strands of DNA fragment to be sequenced. Base sequences of each clone are shown in SEQ ID NO 37 - 39, 44 - 55, 103 and 104. Clones belong to the same region of HCV gene were summarized and of which sequences are shown in SEQ ID NO 33 to 36. For example, clones MX25-1, MX25-2 and MX25-3 are summarized and shown by SEQ ID NO 33.

In the same manner, clones shown by SEQ ID NO 47 to 55 are summarized as clones shown by SEQ ID NO 34 to 36.

In the Sequence Listings, base sequences shown in SEQ ID NO 33 to 39, 44 and 55 are those of + strand of HCV gene inserted into cloning vector to transfect into host cells. All the clones are double-stranded and a plasmid containing one clone and used for the sequencing of said clone is designated by adding a prefix "pUC" to the name of clone. Thus, plasmid containing clone MX25-1 is pUCMX25-1, containing MX25-2 is pUCMX25-2, containing MX25-3 is pUCMX25-3, and so on.

The base sequence shown in the Sequence Listing represents a specific sequence of cDNA corresponding to RNA isolated from serum of patient(s) suffering from HC and differs from that of cDNA obtained from RNA in serum of a healthy subject in the same manner. It was confirmed that cDNA prepared from RNA obtained from a healthy subject under more strict conditions, for instance, by repeating a reaction cycles in Example 15 [2] and [3] about 60 - 100 times (= about 3- or 4-folds), did not show any homology in base sequence with those shown in SEQ ID NO 33 to 43. Consequently, base sequences of clones shown in SEQ ID NO 33 to 43 are specific for those obtained from serum of HC patient.

The base sequences of DNA fragments were compared with a known base sequence of HCV gene. The fact that three clones MX25-1, MX25-2 and MX25-3 were obtained from serum of one HC patient in Example 9 [2] using primers MS155 and MS152 strongly suggests that there must be more than one virus in a patient.

## Example 17

### Preparation of Fused Clones MX25O26A-1, MX25026B-1, N16N15A-1 and N16N15B-1, U16N15A-1 U16N15B-1, N23N15A-1,N23N15B-1, MX25N15A-1, and MX25N15B-1

#### [1] Preparation of Clones MX25026A-1 and MX25026B-1

One µl (about 0.5 to 1 µg/µl) each of DNA fragments from clones MX25-1 and O26-1 (prepared in Example 16) was added into a reaction mixture containing 10 µl of 10 x PCR buffer (100 mM Tris-HCl (pH8.3), 500 mM KCl, 15 mM $MgCl_2$, 1% gelatin), 8 µl of 2.5 mM 4 dNTPs, 5 µl each of 20 pmol/µl synthetic primers MS155 and MS158, and 76.5 µl of water. After an intimate mixing, the mixture was heated at 95 °C for 5 min, then immediately cooled to 0 °C. One minute later, to the mixture was added 0.5 µl of Taq DNA polymerase (7 U/µl, AmpliTaq™ Takara Shuzo), mixed and overlaid with mineral oil. The sample was then subjected to PCR. PCR was conducted by repeating 25 times of reaction cycle, which comprises the following treatments: at 95 °C for 1 min; at 37 °C for 1 min; and at 72 °C for 2 min in DNA Thermal Cycler (Parkin Elmer Cetus). It was followed by an incubation at 97 °C for 2 min. The mixture was immediately cooled to 0 °C, kept at the same temperature for 2 min, mixed with 0.5 µl of Taq DNA polymerase (7 U/µl, AmpliTaq™ Takara Shuzo), and overlaid with mineral oil. The sample was then treated in the same manner as the above by repeating 25 times of reaction cycle, which comprises the following treatments: at 95 °Cfor 1 min; at 50 - 55 °C for 1 min; and at 72 °C for 2 min. After the final treatment at 72 °C for 7 min, the resultant reaction solution was treated with phenol/chloroform then precipitated with ethanol. The amplified DNA samples were fractionated on agarose gel electrophoresis and a gel containing a desired fragment having an expected length was removed (Molecular Cloning (1982) Cold Spring Harbor) to isolate the DNA fragment therefrom conventionally. The resultant DNA fragment was then modified at the N-terminal region as described in Example 16 and ligated into SmaI site of multi-cloning sites of pUC19, cloned and screened as described in Example 3 to obtain plasmids pUCMX25026A-1 and pUCMX25026B-1. CDNAs derived from serum of HC patient contained in said plasmids were designated as clones MX25026A-1 and MX25026B-1, respectively and of which base sequences are summarized in SEQ ID NO 40. Base and deduced amino acid sequences of each clone MX25026A-1 and MX25026B-1 are shown in SEQ ID NO 56 and 57. Overlapping region in clone MX25026A-1 is derived from clone MX25-1 and that of MX25026B-1 from O26-1.

In the same manner as the above, clones N16N15A-1 and N16N15B-1, and U16N15A-1 and U16N15B-1 were prepared using clones N15-1 (SEQ ID NO 39) and either of N16 (SEQ ID NO 36) and U16-4 (SEQ ID NO 37). Base sequences of clones are summarized in SEQ ID NO 41. Base and amino acid sequences of clones N16N15A-1 and N16N-15B-1 are shown in SEQ ID NO 26 and 27, respectively.

[2] Preparation of Clone N16N15-1

Two overlapping clones N16-1 and N15-1 were ligated by taking advantage of unique restriction site which exists in the overlapping regions of the both clones. Upon digestion with restriction enzyme BstE11, clone N16-1 is cleaved at the 3' site of a nucleotide No. 576 and clone N15-1 at the 3' site of a nucleotide No.114. The ligation of A clones N16-1 and N15-1 were conducted on the basis of assumption that plasmids pUCN16-1 and pUCN15-1 contains each clone in the same orientation. As a result, a clone N16N15-1 in which clones N16-1 and N15-1 are ligated without overlapping was conducted. Thus, plasmid pUCN16-1 was digested with HindIII and BstEII to obtain a 609 bp DNA fragment comprising a HindIII-SmaI DNA fragment of plasmid pUC19 attached to the 5' end of clone N16-1 (a cDNA clone from serum of a HC patient). Plasmid pUCN15-1 was digested with HindIII and BstE11 to obtain a 147 bp DNA fragment containing clone N15-1. These 609 bp and 147 bp HindIII-BstE11 fragments are then exchanged each other, cloned and screened to obtain plasmid pUCN16N15-1 containing the desired clone N16N15-1. Clones obtainable in the same manner are summarized in SEQ ID NO 41. The base and amino acid sequences of clone N16N15-1 are shown in SEQ ID NO 60.

[3] Preparation of Clones N23N15A-1 and N23N15B-1

One µl (about 0.5 to 1 µg/µl) of DNA fragment from each clone N23-1 (Example 16), and N16N15A-1, N16N15-B-1 and N16N15-1 (Example 17 [1],[2]) was added to a reaction solution containing 10 µl of 10 x PCR buffer (100 mM Tris-HCl (pH8.3), 500 mM KCl, 15 mM $MgCl_2$, 1% gelatin), 8 µl of 2.5 mM 4 dNTPs, 5 µl each of 20 pmol/µl synthetic primers MS135 and MS88, and 76.5 µl of water. After an intimate mixing, the mixture was heated at 95 °C for 5 min, then immediately cooled to 0 °C. One minute later, to the mixture was added 0.5 µl of Taq DNA polymerase (7 U/µl, AmpliTaq™ Takara Shuzo), mixed and overlaid with mineral oil. The sample was then subjected to PCR. PCR was conducted by repeating 25 times of reaction cycle, which comprises the following steps: at 95 °C for 1 min; at 37 °C for 1 min; at 72 °C for 3.5 - 4 min in DNA Thermal Cycler (Parkin Elmer Cetus). After the final incubation at 92 °Cfor 2 min, the mixture was immediately cooled to 0 °C, kept at the same temperature for 2 min, mixed with 0.5 µl of Taq DNA polymerase (7 U/µl, AmpliTaq™ Takara Shuzo), and overlaid with mineral oil. The sample was then treated in the same manner as the above by repeating 25 times of reaction cycle, which comprises the following treatments: at 95 °C for 1 min; at 50 - 55 °C for 1 min; and at 72 °C for 3.5 - 4 min. After the final treatment at 72 °C for 7 min, the resultant reaction solution was treated with phenol/chloroform then precipitated with ethanol. The amplified DNA samples were fractionated on agarose gel electrophoresis and a gel containing a desired fragment having an expected length was removed (Molecular Cloning (1982) Cold Spring Harbor) to isolate the DNA fragment therefrom conventionally. The resultant DNA fragment was then modified at the N-terminal region and ligated into SmaI site of the multi-cloning sites on pUC19, cloned and screened as described in Example 16 to obtain plasmids pUCN23N15A-1 and pUCN23N15B-1. cDNAs obtained from these plasmids are designated as clones N23N15A-1 and N23N15B-1, whose base and deduced amino acid sequences are shown in SEQ ID NO 61 and 62, respectively and are summarized in SEQ ID NO 42. The overlapping region in clones N23N15A-1, a fused clone of N23-1, N16N15A-1, N16N15B-1 and N16N15A-1, is originated from clone N23-1, and that of clone N23N15B-1 is originated from clones N16N15A-1, N16N15B-1 and N16N15-1.

[4] Preparation of Clone MX25N15-1

Two overlapping clones MX25O26A-1 and N23N15A-1 were ligated by taking advantage of a unique restriction site which exists in the overlapping regions of the both clones. Upon digestion with restriction enzyme ApaI, clone MX25O26A-1 was cleaved at the 3' site of C at nucleotide No. 1277 and clone N23N15A-1 at the 3' site of nucleotide No.17. A plasmid pUCMX25N15-1 in which clones MX25O26A-1 and N23N15A-1 are ligated without overlapping was constructed in the following manner by taking advantage of the fact that plasmids pUCMX25026A and pUCN23N15A-1 contain clones MX25O26A-1 and N23N15A-1 in the same orientation at SmaI site of multi-cloning sites of pUC19. Plasmid pUCMX25O26A-1 was digested with HindIII and ApaI to obtain a 1310 bp DNA fragment comprising a HindIII-SmaI DNA fragment of plasmid pUC19 attached to the 5' end of clone MX25O26A-1 (a cDNA clone from serum of a HC patient). Plasmid pUCN23N15A-1 was digested with HindIII and ApaI to obtain a 50 bp DNA fragment containing clone N15-1. These 1310 bp and 50 bp HindIII-ApaI fragments are then exchanged each other, cloned and screened to obtain plasmid pUCMX25N15-1 containing desired clone MX25N15-1. The base and amino acid sequences of clone MX25N15-1 shown in SEQ ID NO 63.

The clones MX25O26A-1 and N23N15A-1 are ligated by PCR. The resultant base sequences are summarized in SEQ ID NO 43.

Example 18

Modification of DNA for the Expression of HCV Polypeptide Encoded by MX25N15-1

[1] Modification of DNA for the Expression of HCV Polypeptide Encoded by clone MX25N15-1 in E.coli

Clone MX25N15-1 appeared to contain multiple open reading frames each originated from HCV gene such as NS2 ORF (hereinafter, referred to as MK/NS2) from No. 7 (T) to 825 (G), and NS3 ORF (MK/NS3) from No. 826(G) to 2652(G) of base sequence of SEQ ID NO 43. Genes contained therein can be expressed by inserting an ATG initiation codon in frame and upperatream from said gene so that the expression thereof might be properly effected in host cells. When a partial DNA fragment derived from MK/NS2 or MK/NS3 is to be expressed, an ATG initiation codon and a termination codon are inserted upperatream and downstream from the DNA to be expressed, respectively, such that the frame of each inserted codon is in confirmity with that of the DNA. The insertion of an ATG initiation codon at the upperstream from 5' terminus of a gene may be accompanied by an addition of a foreign polypeptide which is not encoded by HCV gene to the N' terminus (amino terminus) of an amino acid sequence of SEQ ID NO 43. When an expression vector containing an initiation codon for E. coli. is used, a DNA fragment from the clone is ligated to the vector such that the frame of said DNA is in confirmity with that of the codon. The modification of DNA can be carried out by PCR.

For the expression of MK/NS2, the following synthetic oligonucleotide primers were used.

5' primer:

MSNS2-1: 5' GCAAGCTTATGTGGTTGTGGATGATGCTGCTG 3' (SEQ ID NO 169)

5' primer for the insertion of said DNA fragment into a vector having an initiation codon from a procaryotic expression vector:

MSNS2-2: 5' TGGTTGTGGATGATGCTGCTG 3' (SEQ ID NO 170)

3' primer:

MSNS2-3: 5' GCGAATTCAGATCTTCATCACCTCCGGGCGGAGACNGGNAGNCC 3' (SEQ ID NO 171)

The synthetic DNA was adjusted to 20 pmol/ml before use.

PCR was carried out in the same manner as described in the above according to Saiki's method in a total volume of 100 $\mu$l containing 100 ng of plasmid pUCMX25N15-1 (or plasmid pUCMX25026A-1), as a template, and 2 $\mu$l each of 3' and 5' primers MSNS2-1 and MSNS2-3. The reaction mixture was heated at 95 °C for 5 min and quenched at 0 °C. One minute later, to the mixture was added 0.5 $\mu$l of Taq DNA polymerase (7 U/ml, AmpliTaq™ Takara Shuzo), mixed thoroughly and overlaid with mineral oil. The sample was reacted by repeating 25 cycles of treatments which comprises: at 95 °C for 1 minute; at 60 °C for 1 min; and at 72 °C for 3 min in DNA Thermal Cycler (Parkin Elmer Cetus). The resultant reaction solution was extracted with phenol/chloroform, and precipitated with ethanol conventionally. The amplified DNA samples were digested with HindIII and EcoRI, and fractionated on acrylamide gel electrophoresis and extracted (Molecular Cloning, Cold Spring Harbor (1982)).

The DNA fragment is then ligated into HindIII (in case of MSNS2-2 primer, SmaI site) and EcoRI sites of cloning vector pUC19 to obtain plasmid pUCHNS2-1. The base sequence of said plasmid is determined to show that it comprises a DNA fragment shown by a base sequence from No. 7 to 825 in SEQ ID No 43 having additional DNA fragments attached to the both 5'- and 3'-termini. That is, at its 5'-terminus, the following DNA fragment comprises a HindIII restriction site followed by an initiation codon ATG was attached.

5′ GCAAGCTTATG 3′

3′ CGTTCGAATAC 5′ (SEQ ID NO 155)

And at its 3'-terminus, the following DNA fragment comprises two termination codons, BglII and EcoRI sites from 5' to 3' was attached.

5′ TGATGAAGATCTGAATTCGC 3′

3′ ACTACTTCTAGACTTAAGCG 5′ (SEQ ID NO 156)

For an expression vector containing E.coli-derived initiation codon, DNA was modified in the same manner as the above except that primers MSNS2-2 and MSNS2-3 are employed and the amplified DNA is first blunt-ended with T4 DNA polymerase and then digested with EcoRI instead of the digestion with HindIII and EcoRI to obtain plasmid pUCH2NS2-1. The sequencing of resultant clone H2NS2-1 showed that said clone has no additional DNA fragment at the 5' terminus but, at the 3' terminus, has the same DNA fragment as that of the above clone HNS2-1.

Modification of DNA for the expression of MK/NS3 was conducted substantially in the same manner as the above except that primers MSNS3-1, 3-2 and 3-3 are used in stead of MSNS2-1, 2-2, and 2-3, respectively to obtain plasmids pUCHNS3-1 and pUCH2NS3-1, corresponding to the above plasmids pUCHNS2-1 and pUCH2NS2-1.

MSNS3-1: 5' GCAAGCTTATGGGCAACGAGNTNCTNCTIGG 3' (SEQ ID NO 172)
MSNS3-2: 5' GGCAACGAGNTNCTNCTNGG 3' (SEQ ID NO 173)
MSNS3-3: 5' GCGAATTCAGATCTTCATCACTTCAGCCGTATGAGACACTT 3' (SEQ ID NO 174)

[2] Modification of DNA for the Expression of DNA encoding HCV Polypeptide MK1 in E. coli

DNA encoding MK1 polypeptide shown by 305 amino acid sequence from No. 422 to 726 in SEQ ID NO 43 was modified in the same manner as the above [1] by inserting an initiation codon ATG in frame and the upperstream from 5' terminus of said DNA in ORF encoding MK1. The insertion of an ATG initiation codon at the upperstream from 5' terminus of said gene may be accompanied by an addition of a foreign polypeptide which is not encoded by HCV gene. When an expression vector containing an initiation codon for E. coli. is used, a DNA fragment from the clone is ligated to the vector such that the frame of said DNA is in confirmity with that of the codon. The modification of DNA can be carried out by PCR using the following synthetic oligonucleotide primers.

5' primer:
MSMK1-1: 5' GCAAGCTTATGCTGTCGCCCGGGCCCATCTC 3' (SEQ ID NO 175)
5' primer for the insertion of a DNA fragment into a vector having an initiation codon from procaryotic expression vector:
MSMK1-2: 5' CTGTCGCCCGGGCCCATCTC 3' (SEQ ID NO 176)
3' primer:
MSMK1-3: 5' GCGAATTCAGATCTTCATCAACATGTGTTGCAGTCGATCAC 3' (SEQ ID NO 177)

The synthetic DNA was adjusted to 20 pmol/ml before use.

PCR, cloning and subcloning were carried out in the same manner as described in the above [1].

Plasmid pUCMK1 was prepared by cloning a DNA fragment obtained by PCR using MSMK1-1 and MSMK1-3. The base sequence of clone MK-1 contained in plasmid pUCMK1 is determined to show that it comprises a DNA fragment having a base sequence from No. 1264 (G) to 2178 (G) in SEQ ID No 43 having additional DNA fragments attached to the both 5'- and 3'-termini. That is, at its 5'-terminal G, a DNA fragment comprises a HindIII restriction site followed by an initiation codon ATG as follows:

5′ GCAAGCTTATG 3′

3′ CGTTCGAATAC 5′ (SEQ ID NO 155).

And at its 3'-terminal G, a DNA fragment comprises two termination codons, BglII and EcoRI sites from 5' to 3' as follows:

5′ TGATGAAGATCTGAATTCGC 3′

3′ ACTACTTCTAGACTTAAGCG 5′ (SEQ ID NO 156)

Another plasmid pUCMK1-2 was constructed in the same manner as the above except that primers MSMK1-2 and MSMK1-3 were employed. The sequencing of resultant clone MK1-2 showed that said clone has no additional DNA fragment at the 5' terminus but has the same additional DNA fragment as that of the above clone MK1 at its 3' terminus.

[3] Modification of DNA for the Expression of DNA Encoding HCV Polypeptide MK2

MK2 polypeptide shown by 322 amino acid sequence from No. 712 to 1033 in SEQ ID NO 43 appears to be HCV-derived antigenic protein which is highly reactive with antiserum from a HC patient. For the expression of DNA encoding MK2 in E.coli, said DNA was modified in the same manner as the above [2] using the following synthetic oligonucleotide primers.
5' primer:
MSMK2-1: 5' GCAAGCTTATGGGCTATACCGGNGACTTNGAC 3' (SEQ ID NO 178)
5' primer for the insertion of a DNA fragment into a vector having an initiation codon from procaryotic expression vector:
MSMK2-2: 5' GGCTATACCGGNGACTTNGAC 3' (SEQ ID NO 179)
3' primer:
MSMK2-3: 5' GCGAATTCAGATCTTCAGTGCTTCGCCCAGAAGGT 3' (SEQ ID NO 180)

The synthetic DNA was adjusted to 20 pmol/ml before use. The resultant clones were designated as clone MK2 (prepared using primers MSMK2-1 and MSMK2-3) and MK2-2 (prepared using primers MSMK2-2 and MSMK2-3).

[4] Modification of DNA for the Expression of HCV Polypeptide Encoded by Clone MX25N15-1 in Insect Cells

Clone MX25N15 contains an open reading frame which starts from the nucleotide No.1 (T). For the construction of expressing plasmids for insect cells, DNA was modified essentially in the same manner as described in the above by inserting an initiation codon ATG to the upperstream from 5' terminus of said DNA in ORF. The insertion of an ATG initiation codon at the upperstream from 5' terminus of said gene may be accompanied by an addition of a foreign polypeptide which is not encoded by HCV gene to the N-terminus (amino-terminus) of the total or a part of amino acid sequence which is encoded by clone MK25N15. When an expression vector containing an initiation codon for insect cells is used, a DNA fragment from the clone is ligated to the vector such that the expression of said DNA can be initiated at the codon. In this case, the modification may be accompanied by an addition of a foreign polypeptide which is not encoded by HCV gene to the N-terminus (amino-terminus) of the total or a part of amino acid sequence which is encoded by clone MK25N15. When an insect cell transformed with an expression plasmid containing MK25N15, said clone was expressed as a precursor polypeptide having an amino acid sequence, which at least contains amino acids from No. 167 to 502, which was then processed, glycosylated and accumulated intracellurarly.

The modification of clone MX25N15 DNA was carried out by PCR employing the following synthetic oligonucleotides as primers.
5' primer:
MS2515-1: 5' GCGCTAGCATGTGGTTGTGGATGATGCTG 3' (SEQ ID NO 181)
3' primer:
MS2515-2: 5' GCGAATTCGCTAGCTCACAGCCGGTTCATCCACTGCAC 3' (SEQ ID NO 182)

The synthetic DNA was adjusted to 20 pmol/ml before use.

The PCR was carried out using the same reaction solution and worked up in the same manner as described in the above except that plasmid pUCMX25N15-1 was used as a template plasmid, primers MS2515-1 and MS2515-2 were used as primers, and the PCR was carried out repeating 10 times the following reaction cycles consisting of: 1 min at 95 °C; 1 min at 50 °C and 5 min at 72 °C ; and then repeating 20 times of the following reaction cycle consisting of: 1 min at 95 °C; 1 min at 65 °C; 5 min at 72 °C to obtain a desired 3586 bp DNA fragment.

The amplified DNA samples were digested with NheI and fractionated on acrylamide gel electrophoresis and extracted as conventionally (Molecular Cloning, Cold Spring Harbor (1982)). The DNA fragment was then ligated into NheI restriction site of a transfer vector pBlueBac (Invitrogen) , cloned and screened for a clone containing a single DNA insert at NheI site conventionally to obtain a plasmid pBlueMX25N15-1.

Taking account of the instruction provided by the manufacture (Invitrogen), the expression unit of the resultant plasmid contains a DNA fragment derived from HCV gene oriented forward and ligated to the NheI cloning site down stream from a poyhedrin promoter.

Example 19

Expression of HCV Polypeptides Encoded by MX25N15, and Polypeptides MK/NS2, MK/NS3, MK1 and MK3

[1] Expression of Polypeptide Encoded by Clones HNS2-1, HNS3-1, MK1 or MK2

Clones HNS2-1, HNS3-1, MK1 and MK2 encode polypeptide fragments derived from a polypeptide encoded by cDNA obtained from a serum of a HC patient and were expressed as it is in E.coli. Construction of expression vector for each clone was carried out by subcloning it into an expression vector pCZ44 (Japanese Patent Publication No. 1-124387/1989).

Each clone was digested thoroughly with restriction enzymes HindIII and BglII, extracted with phenol/chloroform, precipitated with ethanol, separated on acrylamide gel electrophoresis, and extracted a DNA fragment having cohesive HindIII- and BglII-restricted ends from the gel (Molecular Cloning, Cold Spring Harbor, 1982). The expression vector pCZ44 was digested with HindIII and BglII and the larger fragment containing functional region for expressing DNA was separated and treated in the same manner. The both DNA fragments were ligated at their HindIII and BglII sites and cloned. The resultant plasmids were named plasmids pCZHNS2-1, pCZHNS3-1, pCZMK1 and pCZMK2 after clones HNS2-1, HNS3-1, MK1 and MK2, respectively.

Alternatively, expression vectors encoding polypeptides encoded by clones HNS2-1, HNS3-1, MK1 and MK2 were constructed using an expression vector pGEX-2T (Pharmacia) designed to express a fused protein of desired peptide and $\beta$-glutathione-S-transferase (GST). The construction was carried out substantial in accordance with the protocol taught by the manufacture (Pharmacia).

The expression vector pGEX-2T was digested with BamHI. To the linearized vector was ligated a HindIII linker to obtain a DNA fragment having EcoRI and HindIII restriction sites at its 3'- and 5'-termini. Each clone was digested with HindIII and EcoRI to obtain DNA fragments encoding desired HCV polypeptides. The two fragments were then ligated at their HindIII and EcoRI sites to obtain expression vectors pGEXHNS2-1, pGEXHNS3-1, pGEXMK1, and pGEXMK2, respectively. These plasmids contain DNAs encoding GST, thrombin-cleaving sequence, and desired clone from upperstream to downstream.

E.coli JM109 strain was transformed with a plasmid pCZHNS2-1, pCZHNS3-1, pCZMK1 or pCZMK2 and transformant was grown in L-Broth at 37 °C conventionally (Molecular Cloning, Cold Spring Harbor, 1982). The cultured broth was inoculated into a fresh L-Broth to decrease the concentration to 1/50 and cultured with shaking at 30 °C for 2 hr. IPTG (isopropyl-$\beta$-D-galactopyranoside) was added to the culture to a final concentration of 2 mM in order to induce exclusively the expression of DNA by single-clone-derived transformant (E.coli transformed with a single plasmid) and cultivation continued for more than 3 hr. Thus, the transformant produced a polypeptide encoded by the clone. Deduced amino acid sequences of polypeptides encoded by cDNA derived from clones HNS2-1, HNS3-1, MK1 and MK2 are shown by amino acid sequences from 422 to 726 and 712 to 1033 in SEQ ID NO 43.

E.coli JM 109 cells were transformed with expression vector pGEXHNS2-1, pGEXHNS3-1, pGEXMK1, or pGEXMK2 and cultured in the same manner as the above. The expression of gene encoding a fused polypeptide was induced by IPTG. The resultant fused protein comprises GST, a thrombin-cleaving site at its C-terminus, and a polypeptide derived from a clone HNS2-1, HNS3-1, MK1 or MK2.

[2] Expression of Polypeptides Encoded by Clones H2NS2-1, H2NS3-1, MK1-2, and MK2-2

Clones H2NS2-1, H2NS3-1, MK1-2, and MK2-2, whcih had been isolated and sequenced, were expressed in E.coli to give an fused protein using a substantially the same manner as the above.

The fused protein comprises, for instance, a signal peptide of OmpF, an outer membrane protein of E.coli, and a polypeptide encoded by either of the above-mentioned clones can be expressed using, as the expression vector, pOFA (Japanese Patent Publication No. 84195/1990). DNA fragment from each clone H2NS2-1, H2NS3-1, MK1-2, or MK2-2 was blunt-ended with T4DNA polymerase. The expression vector pOFA was digested with KpnI and blunt-ended with T4DNA polymerase. Thus obtained DNA fragments were ligated, cloned and screened for a clone having a insertion of one DNA fragment. Thus, the desired plasmids pOFANS2-1, pOFANS3-1, pOFAMK1, and pOFAMK2 were prepared by subcloning a clone so that the + strand responsible for the expression of HCV protein should be inserted appropriately for the correct translation of the clone. It was confirmed by the determination of base sequence and mapping of each plasmid that the HCV-derived cDNA was reconstructed properly.

E.coli JM109 cells were transformed with an expression vector obtained in the above and induced the expression of DNA by growing host cells under the presence of IPTG as previously described. Transformants expressed a fused protein of a signal peptide of OmpF and a HCV polypeptide encoded by each clone. DNA sequences and deduced amino acid sequences of polypeptides encoded by clones HNS2-1, HNS3-1, MK1 and MK2 are shown by amino acid sequences from 422 to 726 and 712 to 1033 in SEQ ID NO 43. Thus, according to this method, HCV polypeptide was expressed as a fused protein between OmpF signal peptide and polypeptide encoded by each clone.

[3] Expression of Polypeptide Encoded by Clone MX25N15 in Insect Cells

The expression of HCV polypeptide encoded by plasmid pBlueMX25N25 prepared in Example 18, [4] was conducted substantial in accordance with a known expression manual for baculovirus (MAXBAC™ BACULOVIRUS EXPRESSION SYSTEM MANUAL VERSION 1.4, hereinafter, referred to as Maxbac, Invitrogen).

Plasmid pBlueMX25N15, a plasmid prepared by inserting DNA fragment containing HCV gene at the NheI site of a transfer vector pBlueBac (Maxbac, pp.37) was recovered from E.coli/pBlueMX25N15, and purified according to the method of Maniatis et al.(Molecular Cloning, Cold Spring Harbor Laboratory, pp.86 - 96 (1982)) to obtain a large amount of HCV gene-containing transfer plasmid DNA. Sf9 cells were cotransfected with 2 $\mu$g of plasmid pBlueMX25N15 and 1 $\mu$g of AcNPV viral DNA (Maxbac, pp.27). Thus, Sf9 cells were grown in TMN-FH medium (Invitrogen) containing 10% FCS (fetal calf serum) in 6 cm dish until a cell density reached to about 2 x $10^6$/plate. The TMN-F medium was removed and a 0.75 ml Grace medium (Gibco) containing 10% FCS was added thereto. A DNA solution of 2 $\mu$g plasmid pBlueMX25N15 DNA and 1 $\mu$g AcNPV viral DNA in 0.75 ml of transfection buffer (attached to the kit) was thoroughly mixed by vortex and gradually added dropwise onto the Grace medium. After allowing to stand for 4 hr at 27 °C, the Grace medium was replaced with 3 ml of TMN-FH medium containing 10% FCS and the dish incubated at 27 °C for 6 days. Three days from the incubation, there observed a few multinucleate cells and on sixth day, almost all the cells were multinuclear. The supernatant was taken into a centrifuging tube and centrifuged at 1,000 rpm, 10 min to obtain the supernatant as a cotransfected viral solution.

The cotransfected viral solution contains about $10^8$ virus/ml and 0.5% of which were recombinant virus. The isolation of recombinant virus was carried out by plaque isolation method described below.

Thus, cells were adsorbed onto a 6 cm dish by seeding 1.5 x $10^6$ cells on medium and removing the medium. To the dish was added 100 $\mu$l of a diluted viral solution ($10^{-4}$ and $10^{-5}$ folds), separately and incubated at room temperature for 1 hr while slanting the dish every 15 min to spread the virus extensively. X-gal medium containing agarose was prepared by adding 5-bromo-4-chloro-3-indolyl-$\beta$-D-galactoside to a final concentration of 150 $\mu$g/l to a warm medium which had been prepared by autoclaving 2.5% baculovirus agarose (Invitrogen) at 105 °C for 10 min, mixing with TMN-FH medium containing 10% FCS preheated at 46 °C at the mixing ration of 1 : 3, and keeping the temperature at 46 °C.

After the completion of infection, virus solution was aspirated thoroughly from the dish and 4 ml of the warm X-gal medium containing agarose (previously prepared) was gently added to every dish not to peel off cells. The dish kept open by slightly sliding a lid until the agarose solidified and dried, and thereafter the dish covered, turned upside down, and incubated at 27 °C for 6 days. The plaques were observed under a phase difference microscope to find blue plaques which do not form multinucleate cells. Agarose containing blue recombinant plaques were removed by an aspirating pipet and suspended into 1 ml of TMN-FH medium by pipetting many times. The above process whcih comprises: infection, 6-day incubation, and isolation of virus containing transfer plasmid DNA is called the "plaque method". The plaque method was repeated using 100 µl of viral suspension. After repeating said process three times, there obtained a recombinant virus having a gene encoding HCV glucoprotein free from contamination with wild-type strain.

A viral solution of the primary recombinant virus was prepared by aspirating plaques with a Pasteur pipet, and mixing thoroughly with 1 ml of TMN-FH medium. Because the primary viral solution was low in virus density for infection, it was further treated. Thus, 100 µl of viral solution was adsorbed onto Sf9 cells grown in a petri dish (6 cm in diameter) to a semi-confluent, and 4 ml of TMN-FH medium was added thereto and incubated three days. The culture supernatant was recovered to yield a recombinant viral solution for infection.

2. Infection of Sf9 Cells with Recombinant Viral Solution

A suspension of Sf9 cells in TMN-FH medium containing 10% FCS (5 x $10^6$ cells/10 ml medium) were added into a Petri dish (9 cm, in diameter) and kept 1 hr for adsorption. After the removal of medium, 250 µl of recombinant viral solution was added to the dish and spread extensively. To the dish was added 10 ml TMN-FH medium containing 10% FCS and incubated at 27 µC for 4 days. The cells expressing recombinant glycoprotein of HCV were harvested by scraping up and suspended into 1,000 ml of phosphate buffered saline. Thus, HCV glycoproteins were expressed by Sf9 cells infected with said viral solution.

## Example 20

### Identification of Expression Product as HCAg

Each expression product obtained in Example 19 was identified as HCAg because it reacted immunologically with antiserum obtained from HC patient. Identification of the expression product as HCAg was conducted by Western blot as follows. E. coli cells were transformed with either of plasmids described in Example 19 [1], [2], such as plasmids pCZHNS2-1, pCZHNS3-1, pCZMK1, pCZMK2 pGEXHNS2-1, pGEXHNS3-1, pGEXMK1, pGEXMK2, pOFANS2-1, pOFANS3-1, pOFAMK1, and pOFAMK2 for polypeptides encoded by clones HNS2-1, HNS3-1, MK1, MK2 HNS2-1, HNS3-1, MK1, MK2, H2NS2-1, H2NS3-1, MK1-2, and MK2-2, respectively and grown under the presence of IPTG for 3 hr or a overnight.

Recombinant strains were harvested by centrifuging 1,000 µl of the cultured broth at 6,500 rpm, 10 min. The pellet was dissolved into a sample solution (50 mM Tris-HCl, pH6.8 containing 2% SDS, 5% mercaptoethanol, 10% glycerin, and 0.005% bromophenol blue) for SDS-polyacrylamide gel electrophoresis to a final volume of 0.2 ml. The sample solution was then boiled at 100 °C for 10 min. Ten µl of the boiled solution was loaded on 0.1% SDS-15% polyacrylamide gel (70 x 85 x 1 mm) together with a marker protein LMW Kit E (low-molecular weight marker protein, Pharmacia). Electrophoresis was carried out at a constant current of 30 mA for 45 min in Tris buffer (25 mM Tris, pH 8.3, 192 mM glycine, 0.1% SDS) as electrode buffer. Thereafter, DNA was transferred electophoretically to a nitrocellulose filter by superposing the gel onto a filter BA-83 (S & S), impressing a constant current of 120 mA for about 20 min between gel (cathode) and the filter (anode) as conventionally.

The transcribed filter was cut to separate a region containing a marker protein (marker filter) and that containing the sample (sample filter) and the former was stained with 0.1% (w/v) amideblack 10B and the latter immersed into 0.01 M PBS (pH 7.4) containing 5% (w/v) bovine serum albumin (BSA). A serum from a patient suffering from hepatitis C was diluted 50 times with 0.01 M PBS (pH 7.4) containing 5% (w/v) BSA. To the sample filter was added 10 µl of diluted serum and the filter allowed to stand for 2 hr at room temperature. Thereafter, the filter was washed with PBS containing 0.1% (v/v) Tween 20 for 20 min (x3).

The sample filter was then reacted with 10 ml of horseradish peroxidase conjugated anti human IgG (Gappel) at 37 °C for 1 hr and washed with PBS containing 0.1% (v/v) Tween 20 for 20 min (x3). The filter was then immersed into peroxidase-color-producing solution (60 mg 4-chloro-1-naphthol, 20 ml methanol, 80 ml PBS, and 20 µl aqueous hydrogen peroxide). The colored filter was washed with distilled water and compared with the marker filter to demonstrate that the product developed color had a reasonable molecular weight as an expression product of HCV gene contained in expression plasmid pCZHNS2-1, pCZHNS3-1. pCZMK1 or pCZMK2, and was identified as HC-associated antigens.

The expression product from host cells transformed with plasmid pOFANS2-1, pOFANS3-1, pOFAMK1 or pOFAMK2 is a fused protein consisting of HCV originated polypeptide and OmpF signal peptide of E.coli wherein the latter two attached at the N-terminus of the former. The expression product from host cells transformed with plasmid pGEXHNS2-1, pGEXHNS3-1, pGEXMK1 or pGEXMK2 is also a fused protein consisting of HCV originated polypeptide and GST and thrombin cleaving site wherein the latter attached at the N-terminus of the former. These fused proteins have reasonable molecular weight and was also identified as HC-associated antigens.

Example 21

Synthesis of cDNA

In the present Example 21, water used is ultra-pure water which was preparaed by autoclaving (x 2) distilled water.

[1] Preparation of RNA Sample Solution

RNA isolated in Example 1 was dried, dissolved into 0.3 M (pH 7.0) sodium acetate, treated with phenol/chloroform (x 1), mixed with 2.5 volumes of ethanol, and centrifuged (15000 rpm, 20 min, at room temperature) with a rotor of about 5 cm in diameter to yield a pellet of nucleic acid. The pellet was then dried and dissolved into 30 µl of water containing 10 µl of ribonuclease inhibitor (100 U/µl, Takara Shuzo, Japan) to give a nucleic acid solution, which was then subjected to the cDNA synthesis.

[2] Synthesis of cDNA Using Antisense Primer

To 2 µl of RNA sample solution prepared in above [1] was added 1 µl of 15 pmol/µl anti-sense primer selected from a group of synthesized primers MS126, MS119, MS161, MS162, MS121, and MS163 shown in Table, 2 µl of 10 x RT buffer (100 mM Tris-HCl (pH8.3), 500 mM KCl), 4 µl of 25 mM $MgCl_2$, 8 µl of 2.5 mM 4dNTPs, 1 µl of water and the mixture incubated at 65 - 70 °C for 5 min then at room temperature for 5 min. To the mixture was added 1 µl of reverse transcriptase (25 U, Life Science), 1 µl of ribonuclease inhibitor (100 U/µl, Takara Shuzo) and the mixture incubated at 37 °C for 20 min, at 42 °C for 30 min, and finally at 95 °C for 2 min, which was followed by an immediate cooling to 0 °C (synthesis of cDNA).

Amplification of DNA containing specific sequences was conducted by PCR (Saiki et al., Nature 324: 126 (1986)). Thus, 100 µl mixture containing ten µl of cDNA solution, 10 µl of 10 x PCR buffer (100 mM Tris-HCl (pH8.3), 500 mM KCl, 15 mM $MgCl_2$, 1% gelatin), 8 µl of 2.5 mM 4 dNTPs, 2 µl of 15 pmol/µl synthetic DNA primer (the same primer as used in the synthesis of cDNA), 3 µl of 15 pmol/µl synthetic DNA primer (a counterpart of pair of primers, i.e.,MS127-MS126, MS118-MS119, MS159-MS161, MS160-MS162, MS120-MS163, or MS120-MS121) and water was incubated at 95 °C for 5 min, then immediately cooled to 0 °C. One minute later, it was mixed with 0.5 µl of Taq DNA polymerase (7 U/µl, AmpliTaq™ Takara Shuzo) and overlaid with mineral oil. The resultant sample was then subjected to PCR. PCR was conducted by repeating 25 times of reaction cycle, which comprises the following treatments: at 95 °C for 1 min; at 40 - 55 °C for 1 min; and at 72 °C for 1 - 5 min in DNA Thermal Cycler (Parkin Elmer Cetus). Finally, the reaction mixture was incubated at 72 °C for 7 min, which was followed by phenol/chloroform extraction and ethanol precipitation to obtain different amplified DNA fragments derived from either of above-mentioned pairs of primers. The ethanol precipitation was carried out by adding 2.5 volumes of ethanol and either of about 1/10 volume of 3 M acetic acid or an equal volume of 4 M ammonium acetate to the aqueous solution, mixing, centrifuging at 15,000 rpm for 15 min using a rotor of about 5 cm in diameter under cooling at 4 °C to pellet the precipitates, and drying the pellet.

Example 22

Cloning and Sequencing of Amplified DNA Fragments

The cloning was carried out substantial in accordance with the method of Molecular Cloning, Cold Spring Harbor (1982).

Dried DNA fragment (at least 1 pmole) obtained in the above Example 21, [2] was blunt-ended with T4 DNA polymerase (Toyobo) and 5'-end phosphorylated with polynucleotide kinase (Toyobo) and ligated into smaI site of multi-cloning sites of 5 ng to 10 ng of pUC19 cloning vector. The cloning vector had been previously treated as follows: digestion with a restriction enzyme SmaI (Toyobo), phenol/chloroform extraction, ethanol precipitation, 5'-end dephosphorylation with alkaline phosphatase (Behringer-Manheim) (Molecular Cloning (1982) Cold Spring Harbor), phenol/chloroform extraction, and ethanol-precipitation. The ligated DNA was used to transfect into a competent E.coli JM 109 or DH5 cells (Toyobo). The transfection was carried out according to the protocol of the manufacture's instruction (COMPETENT HIGH, Toyobo). Plasmid clones were recovered from transformed cells conventionally. At least 20 transformants were obtained using pUC19 cloning vectors containing either of DNA fragments obtained using either of pairs of primers in the same manner as that described in Example 21, [2].

Plasmid DNA was isolated from corresponding transformant by an usual method and sequenced. The determination of base sequence was conducted by means of Fluorescent DNA Sequencer (GENESIS 2000, Dupont) using, as sequence primer, the following synthetic primers:
5' d(GTAAAACGACGGCCAGT)3' (SEQ ID NO 143) and
5'd(CAGGAAACAGCTATGAC)3' (SEQ ID NO 144) for the + and - strands of DNA fragment to be sequenced. When the DNA fragment is longer than about 200 bp, the determination was conducted by subcloning said DNA into a clone of dilation mutant in order to make sure the sequencing.

DNA fragment obtained using either of pairs of primers shown in Example 21 and whose base sequence was determined is listed below.

| Pair of primers | clone(s) |
|---|---|
| MS127-MS126 | N22-1, N22-3, N22-4, H22-3, H22-8, H22-9 |
| MS118-MS119 | N17-1, N17-2, N17-3, H17-1, H17-3 |
| MS159-MS161 | O28-1, O28-2, 028-4 |
| MS160-MS162 | N29-1, N29-2, N29-3 |
| MS120-MS163 | O30-2, O30-3, O30-4 |
| MS120-MS121 | N18-2, N18-3, N18-4, H18-1, H18-2, H18-3 |

The alphabet letter used to express each clone represents the serum of HC patient used in Example 1. The base sequence of clones proved to have a homology with a known base sequence of HCV gene. The region on HCV gene corresponding to each clone was designated as follows.

| Pair of primers | region on HCV gene |
|---|---|
| MS127-MS126 | N22 |
| MS118-MS119 | N17 |
| MS159-MS161 | O28 |
| MS160-MS162 | N29 |
| MS120-MS163 | O30 |
| MS120-MS121 | N18 |

Among resultant clones, base and amino acid sequences of clones N22-1, N17-3, O28-1, N29-1, N18-4, O30-3 are shown in SEQ ID NO 76, 81, 86, 89, 92, and 98, respectively. Base sequences of other clones obtained in the same manner are listed below in alignment with a base sequence of a clone which disclosed in Seq. Lis. In the list, the base sequence of a clone disclosed in the Seq. Lis. is given at the uppermost column, which is follwed by others in the same region, showing only the bases which are different from those of the clone to be referred to (that shown in the uppermost column). The figure following the name of clone represents the nucleotide number of the base at 5' terminus of the sequence. The nucleotide is numbered from 5' terminus (base No. 1) conventionally.

BASE SEQUENCE OF EACH CLONE IN N22 REGION

```
N22-1.NUC      1 : GGCATGTGGGCCCAGGGGAGGGGGCTGTGCAGTGGATGAACCGGCTGATA
 (SEQ ID NO: 76)
N22-3.NUC      1 : ••••••••••••••••••••••••••••••••••••••••••••••••••
 (SEQ ID NO: 77)
H22-3.NUC      1 : ••••••••••••••••••••••••••••••••••••••••••••••••••
 (SEQ ID NO: 78)
H22-8.NUC      1 : ••••••••••••••••••••••••••••••••••••••••••••••••••
 (SEQ ID NO: 79)
H22-9.NUC      1 : ••••••••••••••••••••••••••••••••••••••••••••••••••
 (SEQ ID NO: 80)
N22-1.NUC     51 : GCGTTTGCTTCGCGGGGCAACCATGTCTCCCCCACGCACTATGTGCCTGA
N22-3.NUC     51 : ••••••••••••••••••••••••••••••••••••••••••••••••••
H22-3.NUC     51 : •••••C•••••••••••T•••••C••••••••••••••T•••••••••••
H22-8.NUC     51 : •••••C••C••••••••T•••••C••••••••••••••T•••••••••••
H22-9.NUC     51 : •••••C•••••••••••T•••••C••••••••••••••T•••••••••••
N22-1.NUC    101 : AAGCGACGCCGCAGCGCGCGTCACCCAGATCCTCTCCAACCTTACCATCA
N22-3.NUC    101 : ••••••••••••••••••••••••••••••••••••••••••••••••••
H22-3.NUC    101 : G•••••••••••••••••T•••••••••••••••••••G•••••••••••
H22-8.NUC    101 : G•••••••••••G•••••T•••••••••••••••••••G•••C•••••••
H22-9.NUC    101 : G•••••••••••••••••T•••••••••••••••••••G•••••••••••
N22-1.NUC    151 : CTCAGCTGTTGAAGAGGCTTCACCAGTGGATTAATGAGGACTGCTCCACG
N22-3.NUC    151 : •••••T•••••••••••••C••••••••••••••••••••••••••••••
H22-3.NUC    151 : ••••••••C••••••••••C••••••••••••G•••••••••••••••••
H22-8.NUC    151 : ••••••••C••••••••••C••••••••••••••••••••••••••••••
```

| | | |
|---|---|---|
| H22-9.NUC | 151 : | •••••••••••••••••••C•••••••••••••••••T•••••••••••• |
| N22-1.NUC | 201 : | CCATGCTCCGGCTCGTGGCTCAGGGATGTTTGGGACTGGATATGCACGGT |
| N22-3.NUC | 201 : | •••••••••••••••••••••••••••••••••••••••••••••••••• |
| H22-3.NUC | 201 : | •••••T••T••T•••••••••••••••••••••••••••••••••••••• |
| H22-8.NUC | 201 : | •••••T••T••T•••••••••••••••••••••••••••••••••••••• |
| H22-9.NUC | 201 : | •••••T••T••T•••••••••••••••••••••••••••••••••••••• |
| N22-1.NUC | 251 : | ATTGGCTGATTGCAAGACCTGGCTCCAGTCCAAGCTCCTGCCGCGGTTAC |
| N22-3.NUC | 251 : | •••••••••••T•••••••••••••••••••••••••••••••••••••• |
| H22-3.NUC | 251 : | G•••AG•••C•T••••••••••••••••••••••••••••••••••C••• |
| H22-8.NUC | 251 : | G•••AG•••C•T••••••••••••••••••••••••••••••••••C••• |
| H22-9.NUC | 251 : | G•••AG•••C•T••••••••••••••••••••••••••••••••••C••• |
| N22-1.NUC | 301 : | CGGGGGTCCCTTTTTTCTCATGCCAGCGTGGGTACAAGGGGGTTTGGCGG |
| N22-3.NUC | 301 : | •••••••••••••C•••••••••••••••••••••••••••••••••••• |
| H22-3.NUC | 301 : | ••••A••••••••CC••••••••••A••••••••••••••A••C•••••• |
| H22-8.NUC | 301 : | ••••A••••••••CC•T••••••••A••••••••••••••A••C•••••• |
| H22-9.NUC | 301 : | ••••A••••••••CC••••••••••A••••••••••••••A••C•••••• |
| N22-1.NUC | 351 : | GGAGATGGCATCATGTATACCACCTGCCCATGTGGAGCACAAATCACCGG |
| N22-3.NUC | 351 : | •••••C•••••••••••••••••••••••••••••••••••••••••••• |
| H22-3.NUC | 351 : | •••••••••••••••C•G••••••••••••••C••••••••••••G•••• |
| H22-8.NUC | 351 : | •••••••••••••••C•A••••••••••••••C••••••••••••G•••• |
| H22-9.NUC | 351 : | •••••••••••••••C••••••••••••••••C••••••••••••G•••• |
| N22-1.NUC | 401 : | ACATGTCAAAAACGGTTCTATGAGGATCGTTGGGCCTAGAACCTGTAGCA |
| N22-3.NUC | 401 : | •••••••••••••••••••••••••••••••••••T•••••••••••••• |
| H22-3.NUC | 401 : | ••••••••••••T•••••••••••••••AC•••C••C••••••••••••• |
| H22-8.NUC | 401 : | ••••••••••••T•••••C•••••••••AC•••C••C••••••••••••• |

| | | | |
|---|---|---|---|
| H22-9.NUC | 401 | : | ••••••••••••T•••••C•••••••••AC•••C••C••••••••••••• |
| N22-1.NUC | 451 | : | ACACGTGGCACGGAACATTTCCCATCAACGCGTACACCACAGGCCCCTGC |
| N22-3.NUC | 451 | : | •••••••••••••••••••C•••••••••••••••••••••••••••••• |
| H22-3.NUC | 451 | : | ••••••••••••••••G••C•••••••••••••••••••••••••••••• |
| H22-8.NUC | 451 | : | ••••••••••••••••G••C•••••••••••••••••••••••••••••• |
| H22-9.NUC | 451 | : | •••••••••G••••••G••C•••••••••••••••••••••••••••••• |
| N22-1.NUC | 501 | : | ACACCCTCCCCGGCGCCAAACTATTCCAGGGCGTTGTGGCGGGTGGCCAT |
| N22-3.NUC | 501 | : | ••••••••T••A•••••G•••••C•••••••••••A••••••••A•••GC |
| H22-3.NUC | 501 | : | •••••••••••A•••••G•••••C•••••••••••A••••••••A••TGC |
| H22-8.NUC | 501 | : | •••••••••••A•••••G••••••••T•••••••••••••••••A••TGC |
| H22-9.NUC | 501 | : | •••••••••••A•••••G••••••••T•A•••••••••••••••A••TGC |
| N22-1.NUC | 551 | : | TGAGGAGTATGTGGAGGTCACGCGGGTGGGGATTTCCACTACGTGACGG |
| N22-3.NUC | 551 | : | •••••••••••••••••••••••••••••••••••••••••••••••••• |
| H22-3.NUC | 551 | : | •••••••••••••••••••••••••••••••••C•••••••••••••••• |
| H22-8.NUC | 551 | : | •••••••••••••••••••••••••••••••••••••••••••••••••• |
| H22-9.NUC | 551 | : | •••••••••••••••••••••••••••••••••••••••••••••••••• |
| N22-1.NUC | 601 | : | GCATGACCACTGACAACGTGAAATGCCCATGCCAGGTTCCGGCCCCCGAA |
| N22-3.NUC | 601 | : | •••••••••••••••••••A•••••••••••••••••••••••••••••• |
| H22-3.NUC | 601 | : | •••••••••••••••••T•••••••••••••••••••C•••••••••••• |
| H22-8.NUC | 601 | : | •••••••••••••••••T•••••••••••••••••••C•••••••••••• |
| H22-9.NUC | 601 | : | •••••••••••••••••T•••••••••••••••••••C•••••••••••• |
| N22-1.NUC | 651 | : | TTCTTCACAGAATTGGATGGGGTGCGGCTGCACAGGTACGCTCCGGCGTG |
| N22-3.NUC | 651 | : | •••••••••••••••••••••••••••••••G•••••••••••••••••• |
| H22-3.NUC | 651 | : | ••••••••G••G•••••••••••A•••••A•••••••••••••••••••• |
| H22-8.NUC | 651 | : | ••••••••G••G•••••••••••A•••••A•••••A•••••••••••••• |

```
H22-9.NUC    651 : ••T•••••G••G•••••••••••A•••••A••••••••••••••••••••
N22-1.NUC    701 : CAAACCTCTCCTGCGGGATGAGGTCACATTCCAGGTCGGGCTCAACCAAT
N22-3.NUC    701 : ••••••••••••••••••••••••••••••••••••••••••••••••••
H22-3.NUC    701 : ••••••••••••A•••••••••••••••••••••••••••••••••••••
H22-8.NUC    701 : ••••••••••••A•••••••••••••••••••••••••••••••••••••
H22-9.NUC    701 : ••••••••••••A•••••••••••••••••••••••••••••••••••••
N22-1.NUC    751 : ATACGGTTGGGTCACAGCTCCCATGTGAGCCCGAACCGGATGTAACAGTG
N22-3.NUC    751 : ••••••••••••••••••••••••••••••••••••••••••••••G•••
H22-3.NUC    751 : TCC••••••••••G•••••••••••C•••••••••••••••••••T••••
H22-8.NUC    751 : TCC••••••••••G•••••••••••C••••••••••••••••••••••••
H22-9.NUC    751 : TCC••••••••••G•••••A•••••C••••••••••••••••••G•••••
N22-1.NUC    801 : GTCACCTCCATGCTCACC
N22-3.NUC    801 : ••••••••••••••••••
H22-3.NUC    801 : ••••••••••••••••••
H22-8.NUC    801 : ••••••••••••••••••
H22-9.NUC    801 : ••••••••••••••••••
```

BASE SEQUENCE OF EACH CLONE IN N17 REGION

```
N17-3.NUC      1 : TGTGAGCCCGAACCGGATGTAACAGTGGTCACCTCCATGCTCACCGACCC
(SEQ ID NO: 81)
N17-1.NUC      1 : ••••••••••••••••••••••••••••••••••••••••••••••••••
(SEQ ID NO: 82)
N17-2.NUC      1 : ••••••••••••••••••••••••••••••••••••••••••••••••••
(SEQ ID NO: 83)
H17-1.NUC      1 : •••••••••••••••••••••••••••C••••T•••••••••••••••••
(SEQ ID NO: 84)
```

H17-3.NUC 1 : ••••••••••••••••••••••••••••••••T•••••••••••••••••

(SEQ ID NO: 85)

N17-3.NUC 51 : CTCCCACATTACAGCAGAGGCGGCTAGGCGTAGGCTGACCAGAGGGTCTC

N17-1.NUC 51 : •••••••••C•••••••••••••••••••••••••••G••••••••••••

N17-2.NUC 51 : •••••••••C•••••••••••••••••••••••••••G••••••••••••

H17-1.NUC 51 : •••••••••••••••••••A••••••A••••••••••G••••••••••••

H17-3.NUC 51 : •••••••••••••••••••••••••G•••••••••••G••••••••••••

N17-3.NUC 101 : CCCCTTCCTCGACCAGTTCTTCAGCTAGTCAGTTGTCTGCGCTTTCTTCG

N17-1.NUC 101 : •T•••••T•••G••••C•••••••••••C•••••••••••••CA••••T•

N17-2.NUC 101 : •T•••••T•T•G••••C•••••••••••••••••••••••••CA••••T•

H17-1.NUC 101 : •••••C•••T•G••••C•••••••••••••••••••••••••CC••CCT•

H17-3.NUC 101 : •••••••••T•G••••C•••••••••••••••••••••••••CC•••CT•

N17-3.NUC 151 : CAGGCAACATGCACTACCCATCAGGGCGCCCCAGACACTGACCTCATCGA

N17-1.NUC 151 : A••••G•••••T•••••••••••A•A•T••••••••G•••••••••••••

N17-2.NUC 151 : A••••G•••••••••••••••••T•A•T••••••••G•••••••••••••

H17-1.NUC 151 : A••••G•••••••••••••••••T•A•T••••G•••G•••••••••••••

H17-3.NUC 151 : A••••G•••••••••••••••••T•A•T••••G•••G•••••••••••••

N17-3.NUC 201 : GGCCAACCTCCTGTGGCGGCAGGAGATGGGCGGAAACATCACCCGCGTGG

N17-1.NUC 201 : •••••••••••••••••••••••••••••••••G••••••••••••••••

N17-2.NUC 201 : •••••••••••••••••••••••••••••••••G••••••••••••••••

H17-1.NUC 201 : ••••••••••••••••••••••••••••••A••G•••••••••••T••••

H17-3.NUC 201 : ••••••••••••A•••••••••••••••••A••G••••••••••••••••

N17-3.NUC 251 : AGTCAGAGAACAAGATAGTAATTCTAGACTCTTTTGAACCGCTTCGAGCG

N17-1.NUC 251 : •••••••••••••••••••G•••••••••••••C••••••••••••••••

N17-2.NUC 251 : •••T••••••••••••••••••••••••••••••••••••••••••••••

H17-1.NUC 251 : ••••••••••••••G••••••••••G••••••••C••C••••••••••••

H17-3.NUC 251 : •••••••••G••••G••••••••••G••••••••C••C••••••••••••

N17-3.NUC 301 : GAGGAGGATGA

N17-1.NUC 301 : •••••••••••

N17-2.NUC 301 : •••••••••••

H17-1.NUC 301 : •••••••••••

H17-3.NUC 301 : •••••••••••

BASE SEQUENCE OF EACH CLONE IN 028 REGION

O28-1.NUC 1 : GTGGTAGTCCTGGACTCGTTGGAGCCGCTTCAAGCGAAGGAAGGTGAGAG
(SEQ ID NO: 86)

O28-2.NUC 1 : •••••••••••••••••••••••C•••••••G••••G••••••A••••••
(SEQ ID NO: 87)

O28-4.NUC 1 : •••••••••••••••••••••••C•••••••G••••G••••••A••••••
(SEQ ID NO: 88)

O28-1.NUC 51 : GGAAGTGTCCGTTGCGGCGGAGATCCTGCGGAAGACCAGGAAATTCCCCG

O28-2.NUC 51 : ••••••••••••••••••••••••••••••A•••••••A•••••••••••

O28-4.NUC 51 : ••••••••••••••••••••••••••••••A•••••••A•••••••••••

O28-1.NUC 101 : CAGCGATGCCCGTATGGGCACGCCCGGACTACAACCCACCATTACTAGAG

O28-2.NUC 101 : ••••••••••••••••••••••••••••••••••••••••••••••••••

O28-4.NUC 101 : ••••••••••••••••••••••••••••••••••••••••••••••••••

O28-1.NUC 151 : TCTTGGAAGAACCCGGACTACGTCCCTCCAGTGGTACACGGGTGCCCATT

O28-2.NUC 151 : •••••••••••••••••••••••••••••G••••••••••••••••••••

O28-4.NUC 151 : •••••••••••••••••••••••••••••G••••••••••••••••••••

O28-1.NUC 201 : GCCGCCTACCAAGGCCCCTCCAATACCACCTCCACGAAGAAAGAGAACGG

O28-2.NUC 201 : ••••••••••••••••••••••••••••••••••••G••••••••G••••

| | | | |
|---|---|---|---|
| O28-4.NUC | 201 | : | ••••••••T•••••••••••••••••••••••••••G••••••••G•••• |
| O28-1.NUC | 251 | : | TTGTCCTGACAGAATCCTCCGTGTCCTCTGCCTTGGCGGAGCTTGCTACA |
| O28-2.NUC | 251 | : | •••C•••••••••••••A•••••••••••••••••••••••••••••••• |
| O28-4.NUC | 251 | : | •••••••••••••••••A•••••••••••••••••••••••••••••••• |
| O28-1.NUC | 301 | : | AAGACCTTTGGCAGTTCCGGATCGTCGGCCGTCGACAGCGGCACGGCGAC |
| O28-2.NUC | 301 | : | •••••••••••••••••••••••••••••••••••••••••••••••••• |
| O28-4.NUC | 301 | : | •••••••••••••••••••••••••••••••••••••••••••••••••• |
| O28-1.NUC | 351 | : | CGGCCCTCCTGACCAGGCCTCCGCCGAAGGAGATGCAGGATCCGACGCTG |
| O28-2.NUC | 351 | : | T••••••••••••••••••••••••••••••••••••••••••••••••• |
| O28-4.NUC | 351 | : | •••••••••••••••••••••••••••••••••••••••••••••••••• |
| O28-1.NUC | 401 | : | AGTCGTACTCCTCCATGCCCCCCCTTGAGGGAGAGCCGGGGGACCCCGAT |
| O28-2.NUC | 401 | : | ••••••••••••••••••••••••••••••••••••••••••••••T••• |
| O28-4.NUC | 401 | : | ••••••••••••••••••••••••••••••••••••••••••••••T••• |
| O28-1.NUC | 451 | : | CTCAGCGACGGGTCTTGGTCTACCGTAAGCGAGGAGGCCAGCGAGGACGT |
| O28-2.NUC | 451 | : | •••••••••••••••••••••••T•••••••••••••••G•••••••••• |
| O28-4.NUC | 451 | : | •••••••••••••••••••••••T•••••••••••••••G•••••••••• |
| O28-1.NUC | 501 | : | CGTCTGCTGCTCGATGTCCTACACATGGACAGGCGCCTTAATTACACCAT |
| O28-2.NUC | 501 | : | •••••••••••••••••••••••••••••••••••••••••••••••••• |
| O28-4.NUC | 501 | : | •••••••••••••••••••••••••••••••••••••••••••••••••• |
| O28-1.NUC | 551 | : | GCGCCGCGGAGGAGAGCAAGCTGCCCATTAATGCGCTGAGCAACCCTTTG |
| O28-2.NUC | 551 | : | ••••••••••••••••••••••••••••••••••••••••••••T••••• |
| O28-4.NUC | 551 | : | ••A•••••••••••••••••••••••••••••••••••••••••T••••• |
| O28-1.NUC | 601 | : | CTGCGCCACCACAACATGGTCTATGCCACAACATCCCGCAGCGCAAGCCA |
| O28-2.NUC | 601 | : | •••••••••••••••••••••••••••••••••••••••••••••••••• |
| O28-4.NUC | 601 | : | •••••T•••••••••••••••••••••••••••••••••••••••••••• |

```
O28-1.NUC    651 : GCGGCAGAAAAAGGTCACATTTGACAGACTGCAAGTCCTGGATGACCACT
O28-2.NUC    651 : ••••••••••••••••••••••••••••••••••••••••••••••••••
O28-4.NUC    651 : ••••••••••••••••••••••••••••••••••••••••••••••••••
O28-1.NUC    701 : ACCGGGACGTGCTCAAGGACATGAAGGCCAAGGCGTCCAC
O28-2.NUC    701 : ••••••••••••••••••••••••••••••••••••••••
O28-4.NUC    701 : ••••••••••••••••••••••••••••••••••••••••
```

BASE SEQUENCE OF EACH CLONE IN N29 REGION

```
N29-1.NUC      1 : ACTACCGGGACGTGCTGAAGGAGATGAAGGCGAAGGCGTCCACAGTTAAG
 (SEQ ID NO: 89)
N29-2.NUC      1 : ••••••••••••••••••••••••••••••••••••••••••••••••••
 (SEQ ID NO: 90)
N29-3.NUC      1 : ••••••••••••••••••••••••••••••••••••••••••••••••••
 (SEQ ID NO: 91)
N29-1.NUC     51 : GCTAAACTTCTATCTGTAGAGGAAGCCTGCAAGCTGACGCCCCCACACTC
N29-2.NUC     51 : •••••••••••••••••••••••••••••T••••••••••••••••••••
N29-3.NUC     51 : •••••••••••••••••••••••••••••T••••••••••••••••••••
N29-1.NUC    101 : GGCCAGATCTAAATTTGGCTACGGGGCAAAGGACGTCCGGAGCCTGTCCA
N29-2.NUC    101 : ••••••••••••••••••••••••••••••••••••••••••••••••••
N29-3.NUC    101 : ••••••••••••G•••••••••••••••••••••••••••••••••••••
N29-1.NUC    151 : GCAAGGCCGTTAACCACATCCGCTCCGTGTGGAAGGACTTGCTGGAAGAC
N29-2.NUC    151 : ••••••••••••••••••••••••••••••••••••••••••••••••••
N29-3.NUC    151 : •••••••••••••••••••••••••••••••••G••••••••••••••••
N29-1.NUC    201 : ACTGAGACACCAATTGACACCACCATCATGGCAAAAAATGAGGTTTTCTG
N29-2.NUC    201 : ••••••••••••••••••••••••••••••••••••••••••••••••••
N29-3.NUC    201 : •••••A••••••••••••••••••••••••••••••••••••••••••••
```

N29-1.NUC 251 : TGTTCAACCAGAGAAAGGAGGCCGCAAGCCAGCTCGCCTTATCGTATTCC

N29-2.NUC 251 : ••••••••••••••••••••••••••••••••••••••••••••••••••

N29-3.NUC 251 : ••••••••••••••••••••••••••••••••••••••••••••••••••

N29-1.NUC 301 : CAGACTTGGGGGTTCGTGTGTGCGAGAAAATGGCCCTCTACGACGTGGTC

N29-2.NUC 301 : ••••••••••••••••••••••••••••••••••••••••••••••••••

N29-3.NUC 301 : ••••••••••••••••••••••••••••••••••••••••••••••••••

N29-1.NUC 351 : TCCACTCTTCCTCAGGCCGTGATGGGCTCCTCATACGGATTCCAGTACTC

N29-2.NUC 351 : ••••••••••••••••••••••••••••••••••••••••••••••••••

N29-3.NUC 351 : ••••••••••••••••••••••••••••••••••••••••••••••••••

N29-1.NUC 401 : CCCTGGACAGCGGGTCGAGTTCCTGGTGAATGCCTGGAAGTCAAAGAAGA

N29-2.NUC 401 : ••••••••••••••••••••••••••••••••••••••••••••••••••

N29-3.NUC 401 : ••••••••••••••••••••••••••••••••••••••••••••••••••

N29-1.NUC 451 : GCCCTATGGGCTTTGCATATGACACCCGCTGTTTTGACTCAACGGTCACC

N29-2.NUC 451 : •T••••••••••••••••••••••••••••••••••••••••••••••••

N29-3.NUC 451 : •T••••••••••••T•••••••••••••••••••••••••••••••••••

N29-1.NUC 501 : GAGAACGACATCCGT

N29-2.NUC 501 : •••••••••••••••

N29-3.NUC 501 : •••

BASE SEQUENCE OF EACH CLONE IN O30 REGION

O30-3.NUC 1 : TGGGGATCCCGTATGATACCCGCTGCTTTGACTCAACGGTCACTGAGAAT

(SEQ ID NO: 98)

O30-2.NUC 1 : •••••••••••••••••••••••••••••••••••••••••••••••••

(SEQ ID NO: 99)

O30-4.NUC 1 : •••••••••••••••••••••••••••••••••••••A••••••••••••

(SEQ ID NO: 100)

| | | | |
|---|---|---|---|
| O30-3.NUC | 51 | : | GACATCCGTGTCGAGGAGTCAATTTACCAATGTTGTGACTTGGCCCCCGA |
| O30-2.NUC | 50 | : | ...........T...................................... |
| O30-4.NUC | 51 | : | ...........T...................................... |
| O30-3.NUC | 101 | : | GGCCAGACAGGCCATAAGGTCACTCACAGAGCGGCTTTACATCGGGGGCC |
| O30-2.NUC | 100 | : | .....................G............................ |
| O30-4.NUC | 101 | : | .....................G............................ |
| O30-3.NUC | 151 | : | CCCTGACTAATTCAAAGGGGCAGAACTGCGGTTATCGCCGGTGCCGCGTC |
| O30-2.NUC | 150 | : | ................A..............C.................. |
| O30-4.NUC | 151 | : | ................................................C. |
| O30-3.NUC | 201 | : | AGCGGCGTGCTGACGACTAGCTGCGGTAATACCCTCACATGTTACTTGAA |
| O30-2.NUC | 200 | : | ..........................C....................... |
| O30-4.NUC | 201 | : | .................................................. |
| O30-3.NUC | 251 | : | GGCCTCTGCAGCCTGTCGAGCTGCAAAGCTCCAGGACTGCACGATGCTTG |
| O30-2.NUC | 250 | : | .................................................. |
| O30-4.NUC | 251 | : | .................................................. |
| O30-3.NUC | 301 | : | TGTGCGGAGACGACCTTGTCGTTATCTGTGATAGCGCGGGAACTCAGGAG |
| O30-2.NUC | 300 | : | ...............................A.................. |
| O30-4.NUC | 301 | : | ...............................A.................. |
| O30-3.NUC | 351 | : | GACGCGGCGAGCCTACGAGTCTTCACGGAGGCTATGACTAGGTACTCTGC |
| O30-2.NUC | 350 | : | .................................................. |
| O30-4.NUC | 351 | : | .................................................. |
| O30-3.NUC | 401 | : | CCCCCCCGGGGACCCGCCCCAACCAGAATACGACTTGGAGCTGATAACAT |
| O30-2.NUC | 400 | : | .................................................. |
| O30-4.NUC | 401 | : | .................................................. |
| O30-3.NUC | 451 | : | CATGTTCCTCCAATGTGTCGGTCGCGCACGACGCATCAGGCAAACGGGTG |

```
O30-2.NUC   450 : ....C........C....................................
O30-4.NUC   451 : ....C.............................................
O30-3.NUC   501 : TACTATCTCACCCGTGACCCCACCACCCCCCTAGCGCGGGCTGCGTGGGA
O30-2.NUC   500 : .....C..........................T.................
O30-4.NUC   501 : .....................C..........T.................
O30-3.NUC   551 : GACAGCTAGACACACTCCAGTCAACTCCTGGCTAGGCAACATCATCATGT
O30-2.NUC   550 : ..................................................
O30-4.NUC   551 : ..................................................
O30-3.NUC   601 : ACGCGCCCACCTTATGGGCAAGGATGATTCTGATGACCCACTTCTTCTCC
O30-2.NUC   600 : .T................................................
O30-4.NUC   601 : ..................................................
O30-3.NUC   651 : ATCCTTCTAGCCCAGGAGCAACTTGAAAAAGCCCTAGATTGTCAGATCTA
O30-2.NUC   650 : ..................................................
O30-4.NUC   651 : ..................................................
O30-3.NUC   701 : CGGGGCCACTTACTCCATTGAGCCACTTGACCTACCTCAGATCATTCAAC
O30-2.NUC   700 : T.................................................
O30-4.NUC   701 : ..................................................
O30-3.NUC   751 : GACTCCACGGTCTTAGCGCATTTTCACTCCATAGTTACTCTCCAGGTGAG
O30-2.NUC   750 : .......T..........................................
O30-4.NUC   751 : .......T..........................................
O30-3.NUC   801 : ATCAATAGGGTGGCTTCATGCCTCAGGAAACTTGGGGTACCGCCCTTGCG
O30-2.NUC   800 : ..................................................
O30-4.NUC   801 : ..................................................
O30-3.NUC   851 : AGTCTGGAGACATCGGGCCAGAAGCGTCCGCGCTAAGCTACTGTCCCAGG
O30-2.NUC   850 : ..................................................
```

```
O30-4.NUC    851 : ••••••••••••••••••••••••••••••••••••••••••••••••••
O30-3.NUC    901 : GGGGGAGGGCCGCCACCTGTGGCAAATACCTCTTCAACTGGGCAGTAAAG
O30-2.NUC    900 : ••••••••••••••••••••••••••••••••••••••••••••••••••
O30-4.NUC    901 : ••••••••••••••••••••••••••••••••••••••••••••••••••
O30-3.NUC    951 : ACCAAGCTCAAACTCACTCCAATCCCAGAAGCGTCCCAGCTGGACTTGTC
O30-2.NUC    950 : •••••••••••••C•••••••••••••G••••••••••••••••••••••
O30-4.NUC    951 : •••••••••••••••••••••••••••G••••••••••••••••••••••
O30-3.NUC   1001 : CGGCTGGTTCGTTGCTGGTTACAGCGGGGGAGACATATATCACAGCCTGT
O30-2.NUC   1000 : ••••••••••••••••••••••••••••••••••••••••••••••••••
O30-4.NUC   1001 : ••••••••••••••••••••••••••••••••••••••••••••••••••
O30-3.NUC   1051 : CTCGTGCCCGACCCCGCTGGTTCATGTGGTGCCTACTCCTACTTTCCGTA
O30-2.NUC   1050 : ••••••••••••••••••••••T•••••••••••••••••••••••••••
O30-4.NUC   1051 : ••••••••••••••••••••••••••••••••••••••••••••••••••
O30-3.NUC   1101 : GGGGTAGGCATCTACCTGCTCCCCAACCGATGAGCGGGGAGCTAAACACT
O30-2.NUC   1100 : ••••••••••••••••••••••••••••••••••••••••••••••••••
O30-4.NUC   1101 : ••••••••••••••••••••••••••••••••••••••••••••••••••
O30-3.NUC   1151 : CCAGGCCAATAGGCCATCCCC
O30-2.NUC   1150 : •••••••••••••••••••••
O30-4.NUC   1151 : •••••••••••••••••••••
```

BASE SEQUENCE OF EACH CLONE IN N18 REGION

```
N18-4.NUC      1 : TGGGGATCCCGTATGATACCCGCTGCTTTGACTCAACGGTCACTGAGAAT
(SEQ ID NO: 92)
N18-2.NUC      1 : •••••••••••••••••••••••••••••••••••••A•••••••••••C
(SEQ ID NO: 93)
N18-3.NUC      1 : ••••••••••••••••••••••••••••••••••••••••••••••••••
```

(SEQ ID NO: 94)

H18-1.NUC 1 : •••••••••••••••••••••••••••••••••••••A••••••••••G•

(SEQ ID NO: 95)

H18-2.NUC 1 : •••••••••••••••••••••••••••••••••••••A••••••••••G•

(SEQ ID NO: 96)

H18-3.NUC 1 : •••••••••••••••••••••••••••••••••••••A•••••C••••G•

(SEQ ID NO: 97)

N18-4.NUC 51 : GACATCCGTACTGAGGAGTCAATTTATCAATGTTGTGACTTGGACCCCGA

N18-2.NUC 51 : ••••••••••T•••••••••••••••••••••C••••••••••T••••••

N18-3.NUC 51 : ••••••••••••••••••••••••••••••••••••••••••••••••••

H18-1.NUC 51 : ••T••••••GT••••••••••••C••C••••••••••••••••C••••••

H18-2.NUC 51 : ••T••••••GT••••••••••••C••C••••••••••••••••C••••••

H18-3.NUC 51 : ••T••••••GT••••••••••••C••C••••••••••••••••C••••••

N18-4.NUC 101 : GGCCAGACAGGCCATAAGGTCGCTCACAGAGCGGCTTTATATCGGGGGCC

N18-2.NUC 101 : ••••••••••••••••••••••••••••••••••••••••••••••••••

N18-3.NUC 101 : ••••••••••••••••••••••••••••••••••••••••••••••••••

H18-1.NUC 101 : ••••••••••••••••••••••••••••••••••••••••••••••••••

H18-2.NUC 101 : ••••••••••••••••••••••••••••••••••••••••••••••••••

H18-3.NUC 101 : ••••••••••••T•••••••••••••••••••••••••••••••••••••

N18-4.NUC 151 : CCTTGACCAATTCAAAAGGGCAAAACTGCGGCTATCGCCGGTGCCGCGCC

N18-2.NUC 151 : ••••••••••••••••••••••••••••••••••••••••••••••••••

N18-3.NUC 151 : ••••••••••••••••••••••G••••••••T••••••••••••••••••

H18-1.NUC 151 : ••C••••T••••••••••••••G••••••••T••••••••••••••••T•

H18-2.NUC 151 : ••C••••T••••••••G•••••G••••••••T••••••••••••••••T•

H18-3.NUC 151 : ••C••••T••••••••••••••G••••••••T••••••••••••••••T•

| | | |
|---|---|---|
| N18-4.NUC | 201 : | AGCGGCGTGCTGACGACTAGCTGCGGTAATACCCTCACATGTTACTTGAA |
| N18-2.NUC | 201 : | •••••••••••••••••••••••••••••••••••••••••••••••••• |
| N18-3.NUC | 201 : | •••••••••••••••••••••••••••••••••••T•••••••••••••• |
| H18-1.NUC | 201 : | •••••••••••••••••C••••••••••••••T••T•••••••••••••• |
| H18-2.NUC | 201 : | •••••••••••••••••C•••••••••••••••••T•••••••••••••• |
| H18-3.NUC | 201 : | •••••••••••••••••C•••••••••••••••••T•••••••••••••• |
| N18-4.NUC | 251 : | GGCCTCTGCAGCCTGTCGAGCTGCGAAGCTCCAGGACTGCACGATGCTCG |
| N18-2.NUC | 251 : | ••••••••••••••••••••••••••••••••G••••••••••••••••• |
| N18-3.NUC | 251 : | •••••••••••••••••••••••••••••••••••••••••••••••••• |
| H18-1.NUC | 251 : | ••••••••••••••••••••••••A•••••••••••••••••A••••••• |
| H18-2.NUC | 251 : | ••••••••••••••••••••••••A•••••••••••••••••A••••••• |
| H18-3.NUC | 251 : | ••••••••••••••••••••••••A•••••••••••••••••A••••••• |
| N18-4.NUC | 301 : | TGTGCGGAGACGACCTTGTCGTTATCTGTGAAAGCGCGGGAACCCAGGAG |
| N18-2.NUC | 301 : | ••••••••••••••••••••••••••••••••••••••••G••••••••• |
| N18-3.NUC | 301 : | •••••••••••••••••••••••••••••••••••••••••••••••••• |
| H18-1.NUC | 301 : | •••••••G••••••••••••••C••••••••G•••••••••••••••••• |
| H18-2.NUC | 301 : | •••••••G••••••••••••••C••••••••••••••••••••••••••• |
| H18-3.NUC | 301 : | •••••••G••••••••••••••C••••••••••••••••••••••••••• |
| N18-4.NUC | 351 : | GACGCGGCAAACCTACGAGTCTTCACGGAGGCTATGACCAGGAATTCCGC |
| N18-2.NUC | 351 : | ••••••••••G••••••••••••••••••••••••••••••••••••••• |
| N18-3.NUC | 351 : | •••••••••••••••••••••••••••••••••••••••••••••••••• |
| H18-1.NUC | 351 : | ••••••••G••••••••••••••••••••••••••••••••••••••••• |
| H18-2.NUC | 351 : | ••••••••G••••••••••••••••••••••••••••••••••••••••• |
| H18-3.NUC | 351 : | ••••••••G••••••••••••••••••••••••••••••••••••••••• |
| N18-4.NUC | 401 : | C |
| N18-2.NUC | 401 : | • |
| N18-3.NUC | 401 : | • |
| H18-1.NUC | 401 : | • |
| H18-2.NUC | 401 : | • |
| H18-3.NUC | 401 : | • |

Base sequences of clones in each of six regions are summarized in SEQ ID NO 64 to 69. Base sequences of SEQ ID NO 64 to 69, 76 - 100 show the base sequences of + - strand of DNA fragments which were derived from HCV gene and inserted into each plasmid used for the transformation. These clones are double stranded DNA. Plasmids used for the sequencing of clones were designated by adding a prefix "pUC" to the name of each clone, for example, plasmid used for sequencing the clone N22-1 was designated as plasmid pUCN22-1. Each plasmid contained one DNA molecule.

These base sequences represents those of clones obtained by cloning the cDNA synthesized from RNA isolated from serum of patient(s) suffering from HC. Therefore, these sequences are specific for clones originated from serum of HCV-infected patients but can not be found or obtained from serum of healthy subjects. Thus, cDNA prepared from RNA (if there are any) obtained from a healthy subject under more strict conditions, for instance, by increasing (3 or 4 folds) the reaction cycles of PCR in Example 21 [2], by repeating them 60 - 100 times, did not show any homology in base sequence with those shown in SEQ ID NO 64 - 69, and 76 - 100. Consequently, base sequences of clones shown in SEQ ID NO 64 - 69, and 76 - 100 are specific for those obtained from serum of HC patient.

The above table indicates that there must be more than one virus in a patient.

## Example 23

## Preparation of Clone 1530U

### [1] Preparation of Clones 1728, 2217, and 2918

Clones N17-3 and O28-1 were ligated using overlapping region by PCR. One μl (about 0.5 to 1 μg/μl) of each DNA fragment from clones N17-3 and O28-1 (311 and 740 bp, respectively) was added into a reaction mixture containing 10 μl of 10 x PCR buffer (100 mM Tris-HCl (pH8.3), 500 mM KCl, 15 mM $MgCl_2$, 1% gelatin), 8 μl of 2.5 mM 4 dNTPs, 5 μl each of 20 pmol/μl synthetic primers MS118 and MS161, and 76.5 μl of water. After an intimate mixing, the mixture was heated at 95 °C for 5 min, then immediately cooled to 0 °C. One minute later, to the mixture was added 0.5 μl of Taq DNA polymerase (7 U/μl, AmpliTaq™ Takara Shuzo), mixed and overlaid with mineral oil. The sample was then subjected to PCR. PCR was conducted by repeating 25 times of reaction cycle, which comprises the following treatments: at 95 °C for 1 min; at 37 °C for 1 min; and at 72 °C for 2 min in DNA Thermal Cycler (Parkin Elmer Cetus). It was followed by an incubation at 97 °C for 2 min. The mixture was immediately cooled to 0 °C, kept at the same temperature for 2 min, mixed with 0.5 μl of Taq DNA polymerase (7 U/μl, AmpliTaq™ Takara Shuzo), and overlaid with mineral oil. The sample was then treated in the same manner as the above by repeating 25 times of reaction cycle, which comprises the following treatments: at 95 °Cfor 1 min; at 55 °C for 1 min; and at 72 °C for 2 min. After the final treatment at 72 °C for 7 min, the resultant reaction solution was treated with phenol/chloroform then precipitated with ethanol. The two DNA fragments were ligated and amplified by PCR. The ligated DNA sample was fractionated on agarose gel electrophoresis and a gel containing about 1000 bp fragment was excised from the gel (Molecular Cloning (1982) Cold Spring Harbor). The resultant DNA fragment was then modified as described in Example 22 and ligated into SmaI site of multi-cloning sites of pUC19, cloned and screened as described in Example 22 to obtain plasmid pUC1728. The resultant clone derived from serum of HC patient was designated as clone 1728 and whose base sequence is given in SEQ ID NO 8.

In the same manner as the above, plasmid pUC2217 was obtained from clones N22-1 and N17-3, which plasmid contains at SmaI site a DNA fragment derived from serum of HC patient in the following order from 5' to 3' site: EcoRI restriction site from pUC19, DNA from clone N22-1, DNA from clone N17-3, and HindIII restriction site. Base and amino acid sequences of clone 2217 are given in SEQ ID NO 70.

In the same manner as the above, clone 2918 was obtained from clones N29-1 and N18-4 whose base and amino acid sequences are given in SEQ ID NO 72.

### [2] Preparation of Clone 1718

There is a 43 bp sequence common to clones 1728 and 2918. These fragments of 1004 and 857 bp were ligated by PCR substantial in accordance with the procedures as those described in the above [1] except that the elongation step in PCR reaction using Taq polymerase was conducted at 72 °C for 5 min. The resultant plasmid pUC1718 contained a DNA fragment having a base sequence derived from HCV gene at SmaI site in which EcoRI site of pUC19 is located to the 5' site of clone N17-3. (N17 region is located to 5' site of N18 region on HCV gene). Base and amino acid sequences of clone 1718 is given in SEQ ID NO 73.

### [3] Preparation of Clone 2218

Overlapping clones 2217 and 2218 were ligated by taking advantage of unique restriction site which exists in the overlapping regions of the both clones. Upon digestion with restriction enzyme XbaI, pUC2217 was cleaved at two sites, i.e., in a sequence from clone N17-3 and the other in a sequence from pUC19, and a small fragment of less than about 40 bp and a large fragment containing most of the sequences from vector and clone 2217 were separated on agarose gel electrophoresis and the larger fragment, pUC2217/XbaI, was extracted. Plasmid pUC1718 was also cleaved at two sites within a sequence from clone N17-3 and one from pUC19, and a larger DNA fragment 1718/XbaI of about 1545 bp containing most of the sequences from vector and clone 1718 was separated on agarose gel electrophoresis and extracted. The ligation of clones 2217 and 1718 was accomplished on the basis of an assumption that plasmids pUC2217 and pUC1718 contain each DNA fragment in the same orientation. Thus, 10 ng of pUC2217/XbaI and 50 ng of 1718/XbaI was ligated in the presence of T4DNA ligase and the ligation mixture was incubated with competent E.coli JM109 cells and cloned in the same manner as Example 22. Transformants containing plasmid pUC2218 which contains clone 17-3 religated at XbaI site. The plasmid pUC2218 contains at its SmaI site, EcoRI site and the following regions without overlapping: clones N22-1, N17-3, O28-1, N29-1, N18-4. Base and amino acid sequences of the resultant clone 2218 is given in SEQ ID NO 74.

[4] Ligation of N15 Region and O30 Region Corresponding to 3' Terminal Region of HCV Gene

Clone O30-3 is shown in SEQ ID NO 98. Plasmid pUCO30 contains a DNA fragment having a sequence corresponding to 3' terminal region of HCV gene at SmaI site of pUC19 in the order of, from 5' to 3', EcoRI site and clone O30-3. Plasmid pUCN15 contains a DNA fragment of HCV gene, clone N15, forwardly at SmaI site of pUC19 in the order of, from 5' to 3', EcoRI site and clone N15.

Plasmid pUCO30 was cleaved at a cloning site, SacI, of pUC19 and blunt ended with T4 DNA polymerase conventionally, which was followed by the cleavage at another cloning site, HindIII, of pUC19 to obtain a DNA fragment O30 (SacT4/Hind) derived from HCV gene. Plasmid pUCN15 was digested with XbaI, blunt ended, and digested with HindIII to obtain a larger DNA fragment pUCN15 (XbaT4/Hind) which contains a sequence from clone N15-1 and all the region of HindIII fragment of pUC19. About 80 ng of DNA fragment O30 (SacT4/Hind) and about 20 ng of DNA fragment pUCN15 (XbaT4/Hind) were ligated in the presence of T4DNA ligase in 20 $\mu$l of reaction mixture. The ligation mixture was incubated with COMPETENT HIGH JM109 (Toyobo) according to the protocol provided by the manufacture and transformants containing desired plasmid pUC15-30 were isolated. Taking advantage of the fact that said plasmid pUC15-30 has only one site which can be cleaved by restriction enzymes BglII and HindIII, it was subjected to PCR using a primer MS174 having a BglII site in sequence derived from clone O30-3.

PCR was conducted using, as a template, 10 ng of pUC1530 and primers MS174 and MS175. PCR fragment was then digested with BglII and HindIII and the resultant fragment ligated to a BglII-HindIII fragment containing the vector fragment of pUCO30 to obtain plasmid pUC15-30U having polyU attached to the 3' terminus of clone 30-3.

[5] Ligation of N15 to O30 Regions

There is an ApaI site within a region common to N15 and N22 regions. There also is an ApaI site within a region common to N18 and O30 regions. A DNA fragment isolated from pUC2218 with ApaI was inserted into ApaI site of pUC15-30 appropriately to obtain plasmid pUC1530U. Thus, plasmid pUC2218 was digested with ApaI and 30 ng of desired DNA fragment, pUC2218/ApaI, was isolated by agarose gel electrophoresis conventionally. Plasmid pUC15-30 was digested with ApaI completely and desired DNA fragment was isolated and dephosphorylated. Ligation was conducted using 30 ng of pUC2218/ApaI and 20 ng of dephosphorylated DNA fragment in a final volume of 10 $\mu$l. All the ligation mixture was used to transform COMPETENT HIGH JM109 (Toyobo). From transformants, desired plasmid pUC1530U which contains at the cloning site, SmaI, a clone 1530U having a sequence from regions N15 to O30 without overlapping was prepared. Base and amino acid sequences of clone 1530 were determined in the same manner as that used in Example 22 and shown in SEQ ID NO 75.

The amino acid sequence of the ligated region comprising N15 to O30 regions has a high homology with a part of non-structural protein NS4 and NS5 of Flavivirus, a related strain of HCV. It was also confirmed that said region is homologous to a sequence encoding a part of NS4 region and all of the NS5 region by comparison with a known sequence of entire HCV gene disclosed by aforementioned Chiron, Shimotohno, or Takamizawa. As a conclusion, clones herein disclosed and whose sequence are shown in Seq Lis correspond to a part of NS4 and all of NS5. As the next step, polypeptides encoded by said clone was evaluated as to the ability to react immunologically with antiserum of HC patients.

Example 24

Modification of DNA for the Expression of HCV Polypeptide Encoded by Clone 1530U

The expression of all or a part of regions of clone 1530U which encodes HCV polypeptide can be accomplished using any of methods which will be hereinafter described.

[1] Modification of DNA for the Expression of a part of HCV Polypeptide Encoded by Clone 1530U in E.coli

This method is used to express a desired polypeptide free from additional amino acid sequence.

Clone 1530U appears to encode an ORF derived from HCV gene (hereinafter, referred to as NS5N) from No.1246 (C) to 1692 (C) of base sequence of SEQ ID NO 75, which can be expressed by inserting an ATG initiation codon at 5' site of said gene in frame. When a part of amino acid sequence of NS5N is desired to be expressed, ATG initiation codon and termination codon were inserted to 5' and 3' site of a gene encoding said amino acid sequence such that the frame of these codons are in confirmity with that of the gene. The insertion of an ATG initiation codon at the upperstream from 5' terminus of said gene may be accompanied by an addition of a foreign polypeptide which is not encoded by HCV gene to the N' terminus (amino terminus) of an amino acid sequence of SEQ ID NO 75. This may happen when a sequence of pUC19 is inserted between ATG codon and DNA encoding HCV polypeptide at the time of insertion of ATG codon. The modification of DNA was carried out by PCR using the following synthetic DNAs as primer.

5' primer:

MSNS5-1: 5' GCAAGCTTATGCAGCGTGGGTACAAGGGGGTT 3' (SEQ ID NO 183)

3' primer:

MSNS5-2: 5' GCGAATTCAGATCTTCATCAGAGCTGTGACCCAACCGTATATTGGTT 3' (SEQ ID NO 184)

The synthetic DNA was adjusted to 20 pmol/ml before use.

PCR was carried out according to Saiki's method in a total volume of 100 $\mu$l containing 100 ng of plasmid pUC2217 (or pUCN22-1 which contains the same region), and 2 $\mu$l each of the above 3' and 5' primers. The reaction mixture was heated at 95 °C for 5 min and quenched at 0 °C. One minute later, to the mixture was added 0.5 $\mu$l of Taq DNA polymerase (7 U/ml, AmpliTaq™ Takara Shuzo), mixed thoroughly and overlaid with mineral oil. The sample was reacted by repeating 25 cycles of treatments which comprises: at 95 °C for 1 minute; at 55 °C for 1 min; and at 72 °C for 1 min in DNA Thermal Cycler (Parkin Elmer Cetus). The resultant reaction solution was extracted with phenol/chloroform and precipitated with ethanol conventionally. The amplified DNA samples were digested with HindIII and EcoRI and fractionated on acrylamide gel electrophoresis and the desired DNA fragment was extracted. The resultant DNA fragment was then ligated into HindIII and EcoRI sites of a cloning vector pUC19, cloned and screened to obtain plasmid pUCNS5N, which was then sequenced. The clone NS5N has a modified base sequence of that from No.1246 (C) to 1692 (C) of SEQ ID NO 75, wherein, at the 5' site of said sequence, the following DNA fragment:

5′ GCAAGCTTATG 3′

3′ CGTTCGAATAC 5′ (SEQ ID NO 155)

which comprises a HindIII restriction site followed by an initiation codon ATG was added, and, at the 3' site of said sequence, the following DNA fragment:

5′ TGATGAAGATCTGAATTCGC 3′

3′ ACTACTTCTAGACTTAAGCG 5′ (SEQ ID NO 156)

which comprises two termination codons, BglII and EcoRI sites from 5' to 3' was added.

[2] Modification of DNA for the Expression of HCV Polypeptide Encoded by MKCNS5 Region in Insect Cells

MKCNS5 region is an ORF derived from HCV gene encoding an amino acid sequence from No. 415 to No. 1411 of SEQ ID NO 75. For the expression of polypeptide, an initiation codon ATG is inserted at 5' site of said gene in frame so that the expression of the gene might be properly effected in insect cells. The insertion of an ATG initiation codon at the upperstream from 5' terminus of said gene may be accompanied by an addition of a foreign polypeptide which is not encoded by HCV gene to the N' terminus (amino terminus) of all or a part of the amino acid sequence encoded by HCV gene. When an expression vector containing an initiation codon for insect cells is used, a DNA fragment from the clone is ligated to the vector such that the frame of said DNA is in confirmity with that of the initiation codon on said vector. It also can be accompanied by an addition of a foreign polypeptide which is not encoded by HCV gene to the N' terminus (amino terminus) of all or a part of the amino acid sequence encoded by HCV gene. Polypeptides encoded by MKCNS5 was expressed in insect cells as a single precursor polypeptide subject to that said polypeptide comprises, at least, the amino acid sequence from No. 415 to 1411 of SEQ ID NO 75, which precursor was then processed by, for example, glycosylation and accumulated intracellurarly. The modification of DNA of clone MKCNS5 region was carried out by PCR using the following synthetic DNA as primers.

5' primers:

MKCNS5-1: 5' GCGCTAGCATGGGGTACAAGGGGGTTTGGCGGG 3' (SEQ ID NO 185)

3' primer:

MKCNS5-2: 5' GCGCTAGCTCATCGGTTGGGGAGCAGGTAGAT 3' (SEQ ID NO 186)

These primers were designed to introduce NheI site at both ends of said gene in order to insert said gene into NheI site of transfer vector pBlueBac (Invitrogen). Therefore, the use of these primers are not critical and others can be used which are designed for introducing said gene into any other transfer vectors for insect cells. The above two synthetic DNAs were adjusted to 20 pmol/ml before use.

The PCR was carried out using the same reaction solution and worked up in the same manner as described in the above [1] except that primers MKCNS5-1 and MKCNS5-2 and, as a template plasmid, 20 ng of plasmid pUC1530U were used. PCR was accomplished by repeating 10 times of reaction cycles consisting of: 1 min at 95 °C; 1 min at 50 °C and 5 min at 72 °C ; and then 20 times of reaction cycles consisting of: 1 min at 95 °C; 1 min at 65 °C; 5 min at 72 °C to yield a desired 3013 bp DNA fragment.

The DNA fragment was digested with NheI, fractionated on acrylamide gel electrophoresis and a DNA fragment of desired length was extracted. The resultant DNA fragment was then ligated into NheI site of a transfer vector pBlueBac (Invitrogen), cloned and screened for a clone which contains a single DNA fragment inserted at NheI site to obtain plasmid pBlueMKCNS5.

According to the teaching shown in the protocol given by Invitrogen, the expression unit of said plasmid contains DNA fragment derived from HCV gene oriented forward and ligated to the NheI cloning site downstream from a poyhedrin promoter.

## Example 25

Expression of HCV Polypeptides Encoded by Clones NS5N, MKCNS4bNS5 in E.coli

Each clone encodes a part of polypeptide encoded by cDNA originated from serum of HC patient. The polypeptide encoded by each clone was expressed in E.coli, as it is, by subcloning said clone into an expression vector pCZ44 (Japanese Patent Publication (KOKAI) No. 124387/1989).

A DNA fragment having a sequence of clone NS5N obtained in Example 24 was digested thoroughly with restriction enzymes HindIII and BglII, extracted with phenol/chloroform, precipitated with ethanol, separated on acrylamide gel electrophoresis. From the gel was extracted a DNA fragment having cohesive HindIII- and BglII-restricted ends. The expression vector pCZ44 was digested with HindIII and BglII. The larger fragment containing a region functional for the expression of DNA was separated, treated in the same manner, ligated to the HindIII-BglII fragment obtained from a clone and cloned conventionally. The resultant plasmid was designated as plasmid pCZNS5N after the clone.

Alternatively, expression vectors were constructed using an expression vector pGEX-2T (Pharmacia) designed to express a fused protein substantial in accordance with the protocol taught by the manufacture (Pharmacia). The expression vector pGEX-2T was digested with BamHI. To the linearized vector was ligated a HindIII linker to obtain a DNA fragment having EcoRI and HindIII restriction sites at its 3'- and 5'-termini. Each clone was digested with HindIII and EcoRI to obtain DNA fragments encoding desired HCV polypeptides. The two fragments were then ligated at their HindIII and EcoRI sites such that the frame of the codon is in confirmity with the amino acid of the clone.

For example, the following region corresponding to HCV polypeptide (hereinafter, referred to as clone MKCNS4bNS5) having a 863 amino acid sequence from No. 306 to 1168 of SEQ ID NO 75 was expressed in E.coli. A DNA fragment encodes MKCNS4bNS5 is named as clone MKCNS4bNS5.

The above region appears to be a HCAg which can immunologically react with antiserum from HC patients in high efficiency. This region can be expressed using pCZ44 for the construction of expression vector. However, it also can be expressed as a fused polypeptide with GST.

Plasmid pUC2218 (2 ng) was digested thoroughly with restriction enzymes HindIII, PvuII and SspI and separated on acrylamide gel electrophoresis. From the gel was extracted about 200 ng of DNA fragment containing a region from clone 2218, which fragment was then blunt-ended. The DNA fragment 2218 (Hin/Pvu/T4) was inserted into HindIII site of pGEXH10 which has a modified sequence of pGEX-2T, wherein the sequence between BamHI and EcoRI sites of pGEX-2T is changed as follows:

```
5'  GGATCCCCCCAAGCTTGGGGGAATTC 3'

    BamHI     HindIII    EcoRI     (SEQ ID NO 187)
```

The expression vector pGEXH10 (1 ng) was digested with HindIII completely and blunt-ended. DNA fragment from pGEXH10 (20 ng) was ligated to 50 ng of DNA fragment 2218 (Hin/Pvu/T4), transformed, and cloned conventionally. The resultant plasmid pGEX2218 encodes a fused polypeptide comprising GST linked to the N22 region of DNA fragment 2218 (Hin/Pvu/T4).

E.coli JM109 strain transformed with plasmid pCZNS5N was grown in L-Broth at 37 °C overnight (Molecular Cloning, Cold Spring Harbor, 1982). The cultured broth was diluted 50-folds by inoculating it into a freshly prepared L-Broth and the cultivation continued with shaking at 30 °Cfor 2 hr. At this time, IPTG was added to the culture to a final concentration of 2 mM in order to induce the expression of DNA encoding HCV-originated polypeptide by single-clone-derived transformants (E.coli JM 109 cells transformed solely by said plasmid). Deduced amino acid sequence of cDNA derived from clone NS5N corresponds to that of No. 1246 to 1692 of SEQ ID NO 75.

In the same manner as the above, plasmid pGEX2218 can be used to express a fused protein between polypeptide MKCNS4bNS5 and GST. The plasmid , as instructed by Pharmacia, contains a sequence encoding a region specifically cleaved by thrombin at C-terminal region of GST, followed by a sequence of clone 2218 ( it also contains a short sequence derived from pUC19). The fused protein can be expressed in the same manner used for the expression of HCV polypeptide encoded by plasmid pCZNS5. Thus, E.coli transformants transformed with plasmid pGEX2218 were grown in the presence of IPTG.

## Example 26

### Expression of MKCNS5 Region in Insect Cells

The expression of HCV-originated protein encoded by plasmid pBlueMKCNS5 prepared in Example 24 [2] was conducted substantial in accordance with a known expression manual for baculovirus (MAXBAC™ BACULOVIRUS EXPRESSION SYSTEM MANUAL VERSION 1.4, hereinafter, referred to as Maxbac, Invitrogen).

Plasmid pBlueMKCNS5 prepared in Example 24 [2] by inserting DNA fragment containing HCV gene at the NheI site of a transfer vector pBlueBac (Maxbac, pp.37), was recovered from E.coli host cells transformed thereby, and purified according to the method of Maniatis et al (Molecular Cloning, Cold Spring Harbor Laboratory, pp.86 - 96 (1982)). Thus, a large amount of HCV gene-containing transfer plasmid DNA was obtained. Sf9 cells were cotransfected with 2 $\mu$g of a plasmid containing a DNA fragment from HCV gene and 1 $\mu$g of AcNPV viral DNA (Maxbac, pp.27). Sf9 cells were grown in TMN-FH medium (Invitrogen) containing 10% FCS (fetal calf serum) in a Petri dish (6 cm diameter) until a cell density reached to about 2 x $10^6$/plate. The TMN-F medium was removed and a 0.75 ml Grace medium (Gibco) containing 10% FCS was added thereto. To the DNA mixture described in the above was added 0.75 ml of transfection buffer (attached to the kit) was thoroughly mixed by vortex and gradually added dropwise onto the Grace medium. After the culture being allowed to stand for 4 hr at 27 °C, Grace medium was replaced with 3 ml of TMN-FH medium containing 10% FCS and the dish incubated at 27 °Cfor 6 days. Three days from the incubation, there observed a few multinucleate cells and on sixth day, almost all the cells were multinuclear. The supernatant was taken into a centrifuging tube and centrifuged at 1,000 rpm, 10 min to obtain the supernatant as a cotransfected viral solution.

The cotransfected viral solution contains about $10^8$ viruses/ml and 0.5% of which were recombinant viruses. The isolation of recombinant virus was carried out by a plaque isolation method described below.

Thus, cells were adsorbed onto a Petri dishes (6 cm diameter) by seeding 1.5 x $10^6$ cells on medium and removing the medium completely. To the dish was added 100 $\mu$l of a diluted viral solution ($10^{-4}$ and $10^{-5}$ folds), separately and incubated at room temperature for 1 hr while slanting the dish every 15 min to spread the virus extensively. X-gal medium containing agarose was prepared by adding 5-bromo-4-chloro-3-indolyl-$\beta$-D-galactoside to a final concentration of 150 $\mu$g/l to a warm medium which had been prepared by autoclaving 2.5% baculovirus agarose (Invitrogen) at 105 °C for 10 min, mixing with TMN-FH medium containing 10% FCS preheated at 46 °C at the mixing ratio of 1 : 3, and keeping the temperature at 46 °C.

After the completion of infection, virus solution was aspirated thoroughly from the dish and 4 ml of the warm X-gal medium containing agarose (previously prepared) was gently added to every dish not to peel off cells. The dish kept open by slightly sliding a lid until the agarose solidified and dried, and thereafter the dish covered, turned upside down, and incubated at 27 °C for 6 days. The plaques were observed under a phase difference microscope to find blue plaques which do not form multinucleate cells. Agarose containing blue recombinant plaques were removed with an aspirating pipet and suspended into 1 ml of TMN-FH medium by pipetting many times. The above process whcih comprises: infection, 6-day incubation, and isolation of virus containing transfer plasmid DNA is called the "plaque method". The plaque method was repeated using 100 $\mu$l of viral suspension. After repeating said process three times, there obtained a recombinant virus having a gene encoding HCV glucoprotein free from contamination with that of wild-type strain.

A viral solution of the primary recombinant virus was prepared by aspirating plaques with a Pasteur pipet, and mixing thoroughly with 1 ml of TMN-FH medium. Because the primary viral solution was low in virus density for infection, it required further treatments for concentration. Thus, 100 $\mu$l of viral solution was adsorbed onto Sf9 cells grown in a petri dish (6 cm in diameter) to a semi-confluent, and 4 ml of TMN-FH medium was added thereto and incubated three days. The culture supernatant was recovered to yield a recombinant viral solution for infection.

For the production of HCV structural protein , a suspension of Sf9 cells in TMN-FH medium containing 10% FCS (5 x $10^6$ cells/10 ml medium) was added into a Petri dish (9 cm, in diameter) and kept 1 hr for adsorption. After the removal of medium, 250 $\mu$l of recombinant viral solution was added to the dish and spread extensively. To the dish was added 10 ml TMN-FH medium containing 10% FCS and incubated at 27 °C for 4 days. The cells expressing recombinant glycoprotein of HCV were harvested by scraping up and suspended into 1,000 ml of phosphate buffered saline.

Thus, HCV-derived glycoprotein was expressed in Sf9 cells transfected with said virus.

## Example 27

### Identification of Expression Products as HCAg

Each expression product obtained in Examples 25 and 26 was identified as HCAg because it reacted immunologically with antiserum obtained from HC patients. Identification was conducted by Western blot technique.

E. coli cells transformed with expression plasmid pCZNS5N or pGEX2218 were grown in the presence of IPTG for 3 hr or a overnight in the same manner as described in Example 25.

Recombinant strains were harvested by centrifuging 1,000 $\mu$l of the cultured broth at 6,500 rpm, 10 min. The pellet was dissolved into a sample solution (50 mM Tris-HCl, pH6.8 containing 2% SDS, 5% mercaptoethanol, 10% glycerin, and 0.005% bromophenol blue) for SDS-polyacrylamide gel electrophoresis to a final volume of 0.2 ml. Sf9 cells infected with viruses which had been treated more than 3 times by plaque method were collected by scraping up and suspended into 1,000 ml of PBS and 100 $\mu$l of the suspension was centrifuged at 6,500 rpm, 10 min to pellet the cells. The pellet was dissolved into a sample solution for SDS-polyacrylamide gel electrophoresis to a final volume of 0.2 ml.

The sample solutions were then boiled at 100 °C for 10 min. Ten $\mu$l of the boiled solution was loaded onto 0.1% SDS-15% polyacrylamide gel (70 x 85 x 1 mm) together with a marker protein LMW Kit E (low-molecular weight marker protein, Pharmacia). Electrophoresis was carried out at a constant current of 30 mA for 45 min in Tris buffer (25 mM Tris, pH 8.3, 192 mM glycine, 0.1% SDS) as electrode buffer. Thereafter, DNA was transferred electophoretically to a nitrocellulose filter by superposing the gel onto a filter BA-83 (S & S), impressing a constant current of 120 mA for about 20 min between gel (cathode) and the filter (anode) as conventionally.

The transcribed filter was cut to remove a part containing a marker protein (referred to as marker filter) and that containing the sample (referred to as sample filter). The former was stained with 0.1% (w/v) amideblack 10B and the latter immersed into 0.01 M PBS (pH 7.4) containing 5% (w/v) bovine serum albumin (BSA). Serum from a HC patient was diluted 50 times with 0.01 M PBS (pH 7.4) containing 5% (w/v) BSA. To the sample filter was added 10 μl of diluted serum and the filter allowed to stand for 2 hr at room temperature. Thereafter, the filter was washed with PBS containing 0.1% (W/V) Tween 20 for 20 min (x3).

The sample filter was then reacted with 10 ml of horseradish peroxidase conjugated anti human IgG (Gappel) at 37 °C for 1 hr and washed with PBS containing 0.1% (w/v) Tween 20 for 20 min (x3). The filter was then immersed into peroxidase-color-producing solution (60 mg 4-chloro-1-naphthol, 20 ml methanol, 80 ml PBS, and 20 μl aqueous hydrogen peroxide). The colored filter was washed with distilled water and compared with the marker filter, demonstrating that colored protein expressed by transformants transformed with plasmid pCZNS5N or pGEX2218 had a reasonable molecular weight as an expression product of inserted HCV gene and was identified as HCAg. The expression product from transformants transformed with pGEX2218 was a fused protein consisting of HCV originated polypeptide and GST and thrombin cleaving site wherein the latter two attached at the N-terminus of the former.

Example 28

Preparation of Clone T7N1-25

[1] Preparation of Clone 1925

Clones N19MX24A-1 (prepared in Example 11[1]) and MX25-1 were ligated using overlapping region by PCR. One μl (about 0.5 to 1 μg/μl) of each DNA fragment from clones N19MX24A-1 and MX25-1 (977 and 849 bp, respectively) was added into a reaction mixture containing 10 μl of 10 x PCR buffer (100 mM Tris-HCl (pH8.3), 500 mM KCl, 15 mM $MgCl_2$, 1% gelatin), 8 μl of 2.5 mM 4 dNTPs, 5 μl each of 20 pmol/μl synthetic primers MS122 and MS152, and 76.5 μl of water. After an intimate mixing, the mixture was heated at 95 °Cfor 5 min, then immediately cooled to 0 °C. One minute later, to the mixture was added 0.5 μl of Taq DNA polymerase (7 U/μl, AmpliTaq™ Takara Shuzo), mixed and overlaid with mineral oil. The sample was then subjected to PCR. PCR was conducted by repeating 10 times of reaction cycle, which comprises the following treatments: at 95 °C for 1 min; at 37 °C for 1 min; and at 72 °C for 2 min in DNA Thermal Cycler (Parkin Elmer Cetus). It was followed by an incubation at 97 °C for 2 min. The mixture was immediately cooled to 0 °C, kept at the same temperature for 2 min, mixed with 0.5 μl of Taq DNA polymerase (7 U/μl, AmpliTaq™ Takara Shuzo), and overlaid with mineral oil. The sample was then treated in the same manner as the above by repeating 15 times of reaction cycle, which comprises the following treatments: at 95 °Cfor 1 min; at 55 °C for 1 min; and at 72 °C for 2 min. After the final treatment at 72 °C for 7 min, the resultant reaction solution was treated with phenol/chloroform then precipitated with ethanol. The two DNA fragments were ligated and amplified by PCR. The ligated DNA sample was fractionated on agarose gel electrophoresis and a gel containing about 1000 bp fragment was excised from the gel (Molecular Cloning (1982) Cold Spring Harbor). The resultant DNA fragment was then modified as described in Example 3 and ligated into SmaI site of multi-cloning sites of pUC19, cloned and screened as described in Example 3 to obtain plasmid pUC1925. The resultant clone derived from serum of HC patient was designated as clone 1925.

[2] Preparation of Clone T7N119

Plasmid pUCN1-1 contains cDNA clone N1-1 at SmaI site of pUC19 from 5' to 3', HindIII site of pUC19 and HCV gene. The plasmid pUCN1-1 was digested with HindIII and NcoI completely and the larger fragment pUCN1HN containing the vector function was isolated. Ten ng of said DNA fragment was ligated to the following synthetic DNAs:

MS168: AGCTTACTAGTTAATACGACTCACTATAGGG (31base pairs, SEQ ID NO: 188)

MS169: CTGGCACCCTATAGTGAGTCGTATTAACTAGTA (33base pairs, SEQ ID NO: 189)

MS170: TGCCAGCCCCCTGATGGGGGCGACACTCCACCATAGATCACTCC (44base pairs, SEQ ID NO: 190)

MS171: TCACAGGGGAGTGATCTATGGTGGAGTGTCGCCCCCATCAGGGGG(45base pairs, SEQ ID NO: 191)

MS172: CCTGTGAGGAACTACTGTCTTCACGCAGAAAGCGTCTAGC(40base pairs, SEQ ID NO: 192)

MS173: CATGGCTAGACGCTTTCTGCGTGAAGACAGTAGTTCC(37base pairs, SEQ ID NO: 193)

The above DNA fragments are shown from 5' to 3' termini.

DNA fragments except MS168 and MS173 were kinased at 5' terminus. A 100 pmol of each of 5'-kinased MS169, MS170, MS171 and MS172, and 20 pmol of each of MS168 and MS173 were ligated in the presence of T4 DNA ligase, and the reaction mixture treated with phenol treatment and ethanol precipitation, conventionally. A quarter of the precipitated DNA sample was ligated to 10 ng of pUCN1HN to obtain plasmid pUCT7N1 which comprises from 5, to 3', HindIII site, SpeI site, promoter sequence derived from T7RNA polymerase, 5' non-translational region of HCV gene, DNA fragment of a gene encoding the N-terminal region of HCV core protein, at the 5' site of clone N1-1. The resultant plasmid pUCT7N1 contains clone T7N1 between HindIII and SmaI sites. Clone T7N1N3N10 was prepared in the same manner as that described in Example 4 [2] except that plasmid pUCT7N1 was used instead of pUCN1-1 having clone N1-1.

Clones T7N1N3N10 and N27N19-1 prepared in Example 11 [2] were ligated by taking advantage of unique restriction site which exists in the overlapping regions of the both clones. Upon digestion with restriction enzyme BamHI, T7N1N3N10 and N27N19-1 were cleaved at 3' site of No. 1332 (G) and No. 3 (G), respectively. The ligation was accomplished on the basis of an assumption that plasmids pUCN1N3N10 and pUCN27N19-1 contain each DNA fragment in the same orientation (on the HCV gene, HindIII site of pUC19 located at 5' site). Thus, plasmid pUCN119 was prepared by digesting pUCN27N19-1 with EcoRI and BamHI to isolate a DNA fragment containing the 5' region of clone N27N19-1 (the fragment comprises clone N27N19-1 attached at the 3' terminus by EcoRI-SmaI fragment of plasmid pUC19), ligating said fragment to the EcoRI-BamHI fragment containing the vector function of plasmid pUCN1N3N10, cloning and screening. Plasmid pUCN119 contains the desired clone T7N119 comprising, from 5' to 3', HindIII site, SpeI site, promoter sequence derived from T7RNA polymerase, a part of 5' non-translational region of HCV gene, clones N1-1, N3-1, N10-1, N27-3, N19-1 without overlapping.

[3] Preparation of Clone T7N1-25

Clones T7N119 and 1925 prepared in the above [1] were ligated by taking advantage of unique restriction site which exists in the overlapping regions of the both clones. Upon digestion with restriction enzyme PvuI, clone T7N119 was cleaved at 3' site of No. 288 (T) of basse sequence of clone N19-1 in N19 region which is shown by SEQ ID NO 16, and clone 1925 was cleaved at 3' of No.288 (T). The ligation of T7N119 and 1925 clones was accomplished on the basis of an assumption that plasmids pUCT7N119 and pUC1925 contain each DNA fragment in the same orientation. Thus, plasmid pUCTN119 was prepared by digesting pUC1925 with PvuII and EcoRI to isolate a DNA fragment encoding HCV originated gene (said DNA fragment contains at 3' of said cDNA a EcoRI-SmaI fragment of plasmid pUC19), exchanging the PvuII-EcoRI fragment containing 3' region of N19 region of plasmid pUCT7N119 with the fragment obtained from plasmid pUCTN1-25, cloning, and screening.

Plasmid pUCT7N1-25 contains the desired clone T7N1-25 comprising clones T7N119 and 1925 ligated at PvuI site without overlapping.

## Example 29

### Preparation of Clone T7N1-30U

[1] Preparation of Clone 1530UNot

The clone 1530U prepared in Example 23[5] contains HindIII site adjacent to 3' site of cDNA of HCV. Plasmid pUC1530U was digested completely with HindIII, blunt ended with T4DNA polymerase conventionally. Ten ng of resultant DNA fragment was ligated to an excess amount of EcoRI-NotI-BamHI adapter (x 100 molar, Toyobo) in the presence of T4 DNA ligase, conventionally. After the phenol treatment and ethanol precipitation, the fragment was digested with NotI, ligated, cloned and screened to yield plasmid pUC1530UNot.

[2] Preparation of Clone T7N1-30U

Clones T7N1-25 prepared in Example 28, MX25N15-1 prepared in Example 17 [4], and 1530UNot obtained in the present Example were ligated by taking advantage of unique restriction site which exists in the overlapping regions of the clones. PUCT7N1-25 was digested with SpeI and PstI and about 1 ng of a DNA fragment T7N1-325SP containing the majority of clone T7N1-25 was extracted from gel. Plasmid pUCMX25N15-1 was digested with PstI and EcoT221 and about 1 ng of a DNA fragment MX25N15-1PE containing the majority of clone MX25N15-1 was extracted from gel. Plasmid pUC1530UNot was digested with EcoT22I and NotI and about 1 ng of a DNA fragment 1530UEN containing the majority of clone 1530UNot was isolated from gel.

About 200 ng of each of the above fragments T7N1-25SP, MX25N15-1PE, 1530UEN, and 1 ng of SpecI-NotI fragment of λZapII (Strategene) were ligated according to the protocol attached to the kit. It was followed by packaging with GIGAPACKII PACKING EXTRACTS, GOLD (Strategnen). All the procedures including ligation, titer check, amplification of λDNA, isolation and packaging were conducted according to the teaching of protocol attached thereto. The screening of recombinant phage was carried out for the inserted DNA fragment by isolating 20 white plaques, subcloning into plasmid pBBLUESCRIPT SK (-). Among 2 clones of 20 clones subcloned into plasmid pBBLUESCRIPT SK (-) contained a DNA fragment having three sequences of HCV gene between SpecI and NotI site of said plasmid λZapII (from 5' SpecI site to 3': clone T7N1-25SP, MX25N15-1PE and 1530UEN). The resultant plasmid was designated as pT7NI-30U.

The plasmid pT7N1-30U contains a clone T7N1-30U comprising three DNA fragments originated from HCV ligated without overlapping SpecI and NotI sites. Base and amino acid sequence of polypeptide encoded by said clone are shown in SEQ ID NO 101.

Example 30

Large-Scale-Expression of Polypeptides CORE and C + N23

[1] Preparation of clone CN23

A region of clone N23-1 to be expressed was obtained by PCR using as a template, pUCN23-1 having clone N23-1 prepared in Example 16. The following synthetic DNAs were used as primers.

5' primer:

MS165: 5' GCAAGCTTATGCTGCTGTCGCCCGGGCCCATCT3' (SEQ ID NO: 194)

3' primer:

MS166: 5' GCGAATTCAGATCTTCATCATGTGTTGCAGTCGATCAC 3' (SEQ ID NO: 195)

The synthetic DNA was adjusted to 20 pmol/ml before use.

PCR was carried out in the same manner as described in the above according to Saiki's method in a total volume of 100 μl containing 100 ng of plasmid pUCN23-1, as a template, and 2 μl each of 3' and 5' primers. The reaction mixture was heated at 95 °C for 5 min and quenched at 0 °C. One minute later, to the mixture was added 0.5 μl of Taq DNA polymerase (7 U/ml, AmpliTaq™ Takara Shuzo), mixed thoroughly and overlaid with mineral oil. The sample was reacted by repeating 8 cycles of treatments which comprises: at 95 °C for 1 minute; at 55 °C for 1 min; and at 72 °C for 1 min in DNA Thermal Cycler (Parkin Elmer Cetus). It was followed by 17 times of reaction cycles comprising, at 95 °C for 1 minute; at 65 °C for 1 min; and at 72 °C for 1 min. The resultant reaction solution was extracted with phenol/chloroform, and precipitated with ethanol conventionally. The amplified DNA samples were digested with HindIII and EcoRI, and fractionated on acrylamide gel electrophoresis and extracted.

The DNA fragment was then ligated into HindIII and EcoRI sites of cloning vector pUC19, cloned screened to obtain plasmid pUCN23A. The base sequence of clone N23A shows that it comprises a DNA fragment shown by a base sequence from Nos. 1 to 915 of SEQ ID No 50 having additional DNA fragments attached to the both 5'- and 3'-termini. That is, at its 5'-terminus, the following DNA fragment comprises a HindIII restriction site followed by an initiation codon ATG was attached.

```
5' GCAAGCTTATG 3'
3' CGTTCGAATAC 5' (SEQ ID NO 155)
```

And at its 3'-terminus, the following DNA fragment comprises two termination codons, BglII and EcoRI sites from 5' to 3' was attached.

5' TGATGAAGATCTGAATTCGC 3'

3' ACTACTTCTAGACTTAAGCG 5' (SEQ ID NO 156)

Plasmid pCZCORE obtained in Example 6 [1] was digested with SacII and blunt ended with T4DNA polymerase conventionally, which was followed by the digestion with BglII and subjected to acrylamide gel electrophoresis. From the gel, a DNA fragment pCZCORE/SB containing a vector part of vector pCZ and the N-terminal region of core protein of HCV was extracted.

In the same manner, plasmid pUCN23A was digested with SmaI and BglII completely and subjected to acrylamide gel electrophoresis. From the gel, a DNA fragment N23A/SB containing the sequence of clone N23-1 was extracted, which fragment contains, from 5' terminus, a base sequence from No.107 (G) to No. 915 (A) of SEQ ID NO 50 and two stop codons and a BglII site.

Ten ng of a DNA fragment pCZCORE/SB and 100 ng of N23A/SB were ligated conventionally to obtain plasmid pCZCN23, which contains clone CN23 of SEQ ID NO 102between HindIII and BglII sites.

[2] Modification of expression vector

The improvement of the efficiency of expression was accomplished by making the expression unit in expression vector multiple. Thus, plasmids pCZCORE and pCZCN23 were digested thoroughly with restriction enzymes BamHI and BglII, and the resultant DNA fragments CORE/BB and CN23/BB encoding a polypeptide derived from HCV was recovered.

The DNA fragment CORE/BB (100 ng) was ligated by T4DNA ligase at 12 °C for 30 minutes according to a conventional method. The resultant material was worked up with phenol treatment and ethanol precipitation, digested with restriction enzymes BamHI and BglII, digested thoroughly with BglII, and ligated to plasmid pCZCORE (10 ng) previously dephosphorylated with alkali phosphatase by a conventional method to obtain plasmid pCZCORE tandem 2, 3, 4, 8, 16, in which 2, 3, 4, 6, 12 expression units of polypeptide CORE between BamHI and BglII sites of plasmid pCZCORE are ligated forwardly in tandem. The same procedure was conducted with the DNA fragment CN23/BB and plasmid pCACN23 to obtain plasmid pCZCN23 tandem 2, 3, 4, 6.

[3] Direct Expression of polypeptides CORE and CN23 in Large Scale

Expression of polypeptide CORE and CN23 in E. coli was conducted using each of expression vector obtained in the above [2] in the same method in Example 6 [1].

For this purpose, conditions such as the timing for induction, species or strains of host cells, number of tandem and the temperature of the culture in the system transformed with pCZCORE were studied.

For example, hosts derived from K12 strain such as JM109, DH5, KS476 and hosts derived from B strain were studied. The degree of expression varies depending on the host. The host derived from B strain and KS476 gave an excellent expression, and the expression amount per culture medium was about 8 to 10 times larger than that obtained using DH5, as host cells. The quantities also varied depending on the time for induction. Thus, 0.5 ml of overnight culture containing transformants (OD 600 = about 1.5) was inoculated into 10 ml bactopepton medium (Difco; 20 g/l bactpepton, 0.2% v/v glycerin, 0.1 M $MgSO_4$, 10 g/l NaCl, 160 µl/l of 0.1% thiamin chloride, 100 mg/l ampicillin) in 10 ml L-shaped tube and cultured at 30°C. IPTG was added either of the time when the conductivity (OD 600) reached to about 0.5, 0.8, 1.2, 2.0 and 3.0 for induction. The cultured broth which was induced when the OD 600 reached to about 0.5 gave the best expression and the amounts of the expression product was highest. The expression was not directly proportional to the number of tandem. For example, when cells transformed with expression plasmid containing in tandem three units of an expression unit CORE/BB, the expression efficiency was low, whereas, it was drastically increased when the plasmid contains 4 units in tandem and kept increase until the number of units becomes 8. However, significant improvement was no more observed and the expression amount was almost the same between cultures containing host cells transformed with tandem 8 and 16. The above studies provided the condition for large-scale-expression of polypeptide CORE as follows. A host cell derived from B strain or KS476 was transformed with pCZCORE tandem 8 and cultured 30°C overnight, inducing the expression when the density reached to about 0.5 (OD 600). Among the plasmid pCZCN23 tandem 2, 3, 4, 6 prepared for the expression of polypeptide CN23, tandem 6 was used and the expression was carried out under the same condition as that used for pCZCORE tandem.

SEQ ID NO:1
SEQUENCE LENGTH: 483 base pairs
SEQUENCE TYPE: nucleic acid
STRANDEDNESS: double
TOPOLOGY: linear
ANTI-SENSE: No
MOLECULE TYPE: cDNA to genomic RNA
ORIGINAL SOURCE
ORGANISM: Hepatitis C virus
IMMEDIATE EXPERIMENTAL SOURCE
CLONE: N1-1

```
CTCCACCATA GATCACTCCC CTGTGAGGAA CTACTGTCTT CACGCAGAAA GCGTCTAGCC   60
ATGGCGTTAG TATGAGTGTC GTGCAGCCTC CAGGACCCCC CCTCCCGGGA GAGCCATAGT  120
GGTCTGCGGA ACCGGTGAGT ACACCGGAAT TGCCAGGACG ACCGGGTCCT TTCTTGGATC  180
AACCCGCTCA ATGCCTGGAG ATTTGGGCGT GCCCCCGCGA GACTGCTAGC CGAGTAGTGT  240
TGGGTCGCGA AAGGCCTTGT GGTACTGCCT GATAGGGTGC TTGCGAGTGC CCCGGGAGGT  300
CTCGTAGACC GTGCATC ATG AGC ACA AAT CCA AAA CCC CAA AGA AAA ACC     350
                   Met Ser Thr Asn Pro Lys Pro Gln Arg Lys Thr
                     1               5                   10
AAA CGT AAC ACC AAC CGC CGC CCA CAG GAC GTC AAG TTC CCG GGC GGT    398
Lys Arg Asn Thr Asn Arg Arg Pro Gln Asp Val Lys Phe Pro Gly Gly
            15                  20                  25
GGT CAG ATC GTT GGT GGA GTT TAC CTG TTG CCG CGC AGG GGC CCC AGG    446
Gly Gln Ile Val Gly Gly Val Tyr Leu Leu Pro Arg Arg Gly Pro Arg
        30                  35                  40
TTG GGT GTG CGC GCG ACT AGG AAG ACT TCC GAG CGG T                  483
Leu Gly Val Arg Ala Thr Arg Lys Thr Ser Glu Arg
```

SEQ ID NO:2
SEQUENCE LENGTH: 187 base pairs
SEQUENCE TYPE: nucleic acid
STRANDEDNESS: double
TOPOLOGY: linear
ANTI-SENSE: No
MOLECULE TYPE: cDNA to genomic RNA
ORIGINAL SOURCE

ORGANISM: Hepatitis C virus
IMMEDIATE EXPERIMENTAL SOURCE
CLONE: N2-1

```
AGGTCTCGTA GACCGTGCAT C ATG AGC ACA AAT CCA AAA CCC CAA AGA AAA      51
                        Met Ser Thr Asn Pro Lys Pro Gln Arg Lys
                          1               5                  10
ACC AAA CGT AAC ACC AAC CGC CGC CCA CAG GAC GTC AAG TTC CCG GGC      99
Thr Lys Arg Asn Thr Asn Arg Arg Pro Gln Asp Val Lys Phe Pro Gly
                 15                  20                  25
GGT GGT CAG ATC GTT GGT GGA GTT TAC CTG TTG CCG CGC AGG GGC CCC     147
Gly Gly Gln Ile Val Gly Gly Val Tyr Leu Leu Pro Arg Arg Gly Pro
             30                  35                  40
AGG TTG GGT GTG CGC GCG ACT AGG AAG ACT TCC GAG CGG T               187
Arg Leu Gly Val Arg Ala Thr Arg Lys Thr Ser Glu Arg
         45                  50                  55
```

SEQ ID NO:3
SEQUENCE LENGTH: 531 base pairs
SEQUENCE TYPE: nucleic acid
STRANDEDNESS: double
TOPOLOGY: linear
MOLECULE TYPE: cDNA to genomic RNA
ANTI-SENSE: No
ORIGINAL SOURCE
ORGANISM: Hepatitis C virus
IMMEDIATE EXPERIMENTAL SOURCE
CLONE: N3-1

```
AGGTCTCGTA GACCGTGCAT C ATG AGC ACA AAT CCA AAA CCC CAA AGA AAA ACC 54
                        Met Ser Thr Asn Pro Lys Pro Gln Arg Lys Thr
                          1               5                  10
AAA CGT AAC ACC AAC CGC CGC CCA CAG GAC GTC AAG TTC CCG GGC GGT     102
Lys Arg Asn Thr Asn Arg Arg Pro Gln Asp Val Lys Phe Pro Gly Gly
             15                  20                  25
GGT CAG ATC GTT GGT GGA GTT TAC CTG TTG CCG CGC AGG GGC CCC AGG     150
Gly Gln Ile Val Gly Gly Val Tyr Leu Leu Pro Arg Arg Gly Pro Arg
```

```
        30                  35                  40
TTG GGT GTG CGC GCG ACT AGG AAG ACT TCC GAG CGG TCG CAA CCT CGT     198
Leu Gly Val Arg Ala Thr Arg Lys Thr Ser Glu Arg Ser Gln Pro Arg
    45                  50                  55
GGA AGG CGA CAA CCT ATC CCC AAG GCT CGC CAA CCC GAG GGC AGG GCC     246
Gly Arg Arg Gln Pro Ile Pro Lys Ala Arg Gln Pro Glu Gly Arg Ala
60                  65                  70                  75
TGG GCT CAG CCC GGG TAC CCT TGG CCC CTC TAT GGC AAT GAG GGC TTG     294
Trp Ala Gln Pro Gly Tyr Pro Trp Pro Leu Tyr Gly Asn Glu Gly Leu
                80                  85                  90
GGG TGG GCA GGA TGG CTC CTG TCA CCC CGC GGC TCC CGG CCT AGT TGG     342
Gly Trp Ala Gly Trp Leu Leu Ser Pro Arg Gly Ser Arg Pro Ser Trp
            95                  100                 105
GGC CCC ACG GAC CCC CGG CGT AGG TCG CGT AAT TTG GGT AAG GTC ATC     390
Gly Pro Thr Asp Pro Arg Arg Arg Ser Arg Asn Leu Gly Lys Val Ile
        110                 115                 120
GAT ACC CTC ACA TGC GGC TTC GCC GAT CTC ATG GGT ACA TTC CGC TCG     438
Asp Thr Leu Thr Cys Gly Phe Ala Asp Leu Met Gly Tyr Ile Pro Leu
    125                 130                 135
GTC GGC GCC CCC CTA GGG GGC GCT GCC AGG GCT CTA GCG CAT GGC GTC     486
Val Gly Ala Pro Leu Gly Gly Ala Ala Arg Ala Leu Ala His Gly Val
140                 145                 150                 155
CGG GTT CTG GAG GAC GGC GTG AAC TAC GCA ACA GGG AAC TTG CCC         531
Arg Val Leu Glu Asp Gly Val Asn Tyr Ala Thr Gly Asn Leu Pro
                160                 165                 170
```

SEQ ID NO:4
SEQUENCE LENGTH: 755 base pairs
SEQUENCE TYPE: nucleic acid
STRANDEDNESS: double
TOPOLOGY: linear
MOLECULE TYPE: cDNA to genomic RNA
ANTI-SENSE: No
ORIGINAL SOURCE
ORGANISM: Hepatitis C virus
IMMEDIATE EXPERIMENTAL SOURCE
CLONE: N10-1

```
C GTG AAC TAT GCA ACA GGG AAT CTG CCT GGT TGC TCC TTT TCT ATC TTC    49
  Val Asn Tyr Ala Tyr Gly Asn Leu Pro Gly Cys Ser Phe Ser Ile Phe
    1               5                  10                  15
CTT TTG GCT TTG CTG TCC TGT TTG ACC ATC CCA GCT TCC GCC TAC CAA      97
Leu Leu Ala Leu Leu Ser Cys Leu Thr Ile Pro Ala Ser Ala Tyr Gln
              20                  25                  30
GTG CGC AAC GCG TCC GGG GTG TAC CAT GTC ACG AAC GAC TGC TCC AAC     145
Val Arg Asn Ala Ser Gly Val Tyr His Val Thr Asn Asp Cys Ser Asn
          35                  40                  45
TCA AGT ATT GTG TAT GAG GCG GCG GAC GTG ATT ATG CAC ACC CCC GGG     193
Ser Ser Ila Val Thr Glu Ala Ala Asp Val Ile Met His Thr Pro Gly
     50                  55                  60
TGC GTG CCC TGC GTC CGG GAG AAC AAT TCC TCC CGC TGC TGG GTA GCG     241
Cys Val Pro Cys Val Arg Glu Asn Asn Ser Ser Arg Cys Trp Val Ala
 65                  70                  75                  80
CTC ACT CCC ACG CTT GCG GCC AGG AAC AGC AGC ATC CCC ACT ACG ACA     289
Leu Thr Pro Thr Leu Ala Ala Arg Asn Ser Ser Ile Pro Thr Thr Thr
                  85                  90                  95
ATA CGG CGT CAT GTC GAC TTG CTC GTT GGG GCA GCT GTC CTC TGT TCC     337
Ile Arg Arg His Val Asp Leu Leu Val Gly Ala Ala Ala Leu Cys Ser
              100                 105                 110
GCT ATG TAT GTG GGG GAT TTT TGC GGA TCT GTT TTC CTC GTC TCC CAG     385
Ala Met Tyr Val Gly Asp Phe Cys Gly Ser Val Phe Leu Val Ser Gln
          115                 120                 125
CTG TTC ACT TTC TCA CCT CGC CGG TAT GAG ACG GTG CAA GAC TGC AAT     433
Leu Phe Thr Phe Ser Pro Arg Arg Tyr Glu Thr Val Gln Asp Cys Asn
      130                 135                 140
TGC TCA ATC TAT CCC GGC CAT GTA TCA GGC CAT CGC ATG GCT TGG GAT     481
Cys Ser Ile Tyr Pro Gly His Val Ser Gly His Arg Met Ala Trp Asp
145                 150                 155                 160
ATG ATA ATG AAT TGG TCA CCT ACA ACA GCC CTA GTG GTA TCG CAG CTA     529
Met Ile Met Asn Trp Ser Pro Thr Thr Ala Leu Val Val Ser Gln Leu
                165                 170                 175
CTC CGG ATC CCA CAA GCC GTC GTG GAT ATG GTG GCG GGG GCC CAC TGG     577
Leu Arg Ile Pro Gln Ala Val Val Asp Met Val Ala Gly Ala His Trp
              180                 185                 190
GGA GTC CTG GCG GGC CTT GCC TAC TAT TCC ATG GTG GGG AAC TGG GCT     625
```

```
Gly Val Leu Ala Gly Leu Ala Tyr Tyr Ser Met Val Gly Asn Trp Ala
        195                 200                 205
AAG GTC TTG GTT GTG ATG CTG CTC TTC GCC GGT GTT GAC GGG GGG ACC      673
Lys Val Leu Val Val Met Leu Leu Phe Ala Gly Val Asp Gly Gly Thr
    210                 215                 220
CAC GTG ACA GGG GGA AAG GTA GCC TAC ACC ACC CAG AGC TTT ACA TCC      721
His Val Thr Gly Gly Lys Val Ala Tyr Thr Thr Gln Ser Phe Thr Ser
225                 230                 235                 240
TTC TTT TCA CGA GGG CCG TCT CAG AGA ATC CAG C
Phe Phe Ser Arg Gly Pro Ser Gln Arg Ile Gln
                245                 250
```

SEQ ID NO:5
SEQUENCE LENGTH: 1258 base pairs
SEQUENCE TYPE: nucleic acid
STRANDEDNESS: double
TOPOLOGY: linear
MOLECULE TYPE: cDNA to genomic RNA
ANTI-SENSE: No
ORIGINAL SOURCE
ORGANISM: Hepatitis C virus
IMMEDIATE EXPERIMENTAL SOURCE
CLONE: N3N10

```
AGGTCTCGTA GACCGTGCAT C ATG AGC ACA AAT CCA AAA CCC CAA AGA AAA ACC 54
                        Met Ser Thr Asn Pro Lys Pro Gln Arg Lys Thr
                         1               5                  10
AAA CGT AAC ACC AAC CGC CGC CCA CAG GAC GTC AAG TTC CCG GGC GGT      102
Lys Arg Asn Thr Asn Arg Arg Pro Gln Asp Val Lys Phe Pro Gly Gly
            15                  20                  25
GGT CAG ATC GTT GGT GGA GTT TAC CTG TTG CCG CGC AGG GGC CCC AGG      150
Gly Gln Ile Val Gly Gly Val Tyr Leu Leu Pro Arg Arg Gly Pro Arg
        30                  35                  40
TTG GGT GTG CGC GCG ACT AGG AAG ACT TCC GAG CGG TCG CAA CCT CGT      198
Leu Gly Val Arg Ala Thr Arg Lys Thr Ser Glu Arg Ser Gln Pro Arg
    45                  50                  55
GGA AGG CGA CAA CCT ATC CCC AAG GCT CGC CAA CCC GAG GGC AGG GCC      246
```

```
Gly Arg Arg Gln Pro Ile Pro Lys Ala Arg Gln Pro Glu Gly Arg Ala
 60                 65                  70                  75
TGG GCT CAG CCC GGG TAC CCT TGG CCC CTC TAT GGC AAT GAG GGC TTG    294
Trp Ala Gln Pro Gly Tyr Pro Trp Pro Leu Tyr Gly Asn Glu Gly Leu
                80                  85                  90
GGG TGG GCA GGA TGG CTC CTG TCA CCC CGC GGC TCC CGG CCT AGT TGG    342
Gly Trp Ala Gly Trp Leu Leu Ser Pro Arg Gly Ser Arg Pro Ser Trp
            95                  100                 105
GGC CCC ACG GAC CCC CGG CGT AGG TCG CGT AAT TTG GGT AAG GTC ATC    390
Gly Pro Thr Asp Pro Arg Arg Arg Ser Arg Asn Leu Gly Lys Val Ile
        110                 115                 120
GAT ACC CTC ACA TGC GGC TTC GCC GAT CTC ATG GGT ACA TTC CGC TCG    438
Asp Thr Leu Thr Cys Gly Phe Ala Asp Leu Met Gly Tyr Ile Pro Leu
    125                 130                 135
GTC GGC GCC CCC CTA GGG GGC GCT GCC AGG GCT CTA GCG CAT GGC GTC    486
Val Gly Ala Pro Leu Gly Gly Ala Ala Arg Ala Leu Ala His Gly Val
140                 145                 150                 155
CGG GTT CTG GAG GAC GGC GTG AAC TAT GCA ACA GGG AAC CTG CCT GGT    534
Arg Val Leu Glu Asp Gly Val Asn Tyr Ala Thr Gly Asn Leu Pro Gly
                160                 165                 170
TGC TCC TTT TCT ATC TTC CTT TTG GCT TTG CTG TCC TGT TTG ACC ATC    582
Cys Ser Phe Ser Ile Phe Leu Leu Ala Leu Leu Ser Cys Leu Thr Ile
            175                 180                 185
CCA GCT TCC GCC TAC CAA GTG CGC AAC GCG TCC GGG GTG TAC CAT GTC    630
Pro Ala Ser Ala Tyr Gln Val Arg Asn Ala Ser Gly Val Tyr His Val
        190                 195                 200
ACG AAC GAC TGC TCC AAC TCA AGT ATT GTG TAT GAG GCG GCG GAC GTG    678
Thr Asn Asp Cys Ser Asn Ser Ser Ile Val Tyr Glu Ala Ala Asp Val
    205                 210                 215
ATT ATG CAC ACC CCC GGG TGC GTG CCC TGC GTC CGG GAG AAC AAT TCC    726
Ile Met His Tyr Pro Gly Cys Val Pro Cys Val Arg Glu Asn Asn Ser
220                 225                 230                 235
TCC CGC TGC TGG GTA GCG CTC ACT CCC ACG CTT GCG GCC AGG AAC AGC    774
Ser Arg Cys Trp Val Ala Leu Thr Pro Thr Leu Ala Ala Arg Asn Ser
                240                 245                 250
AGC ATC CCC ACT ACG ACA ATA CGG CGT CAT GTC GAC TTG CTC GTT GGG    822
Ser Ile Pro Thr Thr Thr Ile Arg Arg His Val Asp Leu Leu Val Gly
```

```
            255                     260                     265
GCA GCT GCT CTC TGT TCC GCT ATG TAT GTG GGG GAT TTT TGC GGA TCT    870
Ala Ala Ala Leu Cys Ser Ala Met Tyr Val Gly Asp Phe Cys Gly Ser
        270                     275                     280
GTT TTC CTC GTC TCC CAG CTG TTC ACT TTC TCA CCT CGC CGG TAT GAG    918
Val Phe Leu Val Ser Gln Leu Phe Thr Phe Ser Pro Arg Arg Tyr Glu
    285                     290                     295
ACG GTG CAA GAC TGC AAT TGC TCA ATC TAT CCC GGC CAT GTA TCA GGC    966
Thr Val Gln Asp Cys Asn Cys Ser Ile Tyr Pro Gly His Val Ser Gly
300                     305                     310                 315
CAT CGC ATG GCT TGG GAT ATG ATA ATG AAT TGG TCA CCT ACA ACA GCC   1014
His Arg Met Ala Trp Asp Met Ile Met Asn Trp Ser Pro Thr Thr Ala
                320                     325                     330
CTA GTG GTA TCG CAG CTA CTC CGG ATC CCA CAA GCC GTC GTG GAT ATG   1062
Leu Val Val Ser Gln Leu Leu Arg Ile Pro Gln Ala Val Val Asp Met
            335                     340                     345
GTG GCG GGG GCC CAC TGG GGA GTC CTG GCG GGC CTT GCC TAC TAT TCC   1110
Val Ala Gly Ala His Trp Gly Val Leu Ala Gly Leu Ala Tyr Tyr Ser
        350                     355                     360
ATG GTG GGG AAC TGG GCT AAG GTC TTG GTT GTG ATG CTG CTC TTC GCC   1158
Met Val Gly Asn Trp Ala Lys Val Leu Val Val Met Leu Leu Phe Ala
    365                     370                     375
GGT GTT GAC GGG GGG ACC CAC GTG ACA GGG GGA AAG GTA GCC TAC ACC   1206
Gly Val Asp Gly Gly Thr His Val Thr Gly Gly Lys Val Ala Tyr Thr
380                     385                     390                 395
ACC CAG AGC TTT ACA TCC TTC TTT TCA CGA GGG CCG TCT CAG AGA ATC   1254
Thr Gln Ser Phe Thr Ser Phe Phe Ser Arg Gly Pro Ser Gln Arg Ile
                400                     405                     410
CAGC                                                              1258
Gln
```

SEQ ID NO:6

SEQUENCE LENGTH: 1554 base pairs

SEQUENCE TYPE: nucleic acid

STRANDEDNESS: double

TOPOLOGY: linear

MOLECULE TYPE: cDNA to genomic RNA

ANTI-SENSE: No
ORIGINAL SOURCE
ORGANISM: Hepatitis C virus
IMMEDIATE EXPERIMENTAL SOURCE
CLONE: N1N3N10

```
CTCCACCATA GATCACTCCC CTGTGAGGAA CTACTGTCTT CACGCAGAAA GCGTCTAGCC   60
ATGGCGTTAG TATGAGTGTC GTGCAGCCTC CAGGACCCCC CCTCCCGGGA GAGCCATAGT  120
GGTCTGCGGA ACCGGTGAGT ACACCGGAAT TGCCAGGACG ACCGGGTCCT TTCTTGGATC  180
AACCCGCTCA ATGCCTGGAG ATTTGGGCGT GCCCCCGCGA GACTGCTAGC CGAGTAGTGT  240
TGGGTCGCGA AAGGCCTTGT GGTACTGCCT GATAGGGTGC TTGCGAGTGC CCCGGGAGGT  300
CTCGTAGACC GTGCATC ATG AGC ACA AAT CCA AAA CCC CAA AGA AAA ACC     350
                   Met Ser Thr Asn Pro Lys Pro Gln Arg Lys Thr
                    1               5                  10
AAA CGT AAC ACC AAC CGC CGC CCA CAG GAC GTC AAG TTC CCG GGC GGT    398
Lys Arg Asn Thr Asn Arg Arg Pro Gln Asp Val Lys Phe Pro Gly Gly
            15                  20                  25
GGT CAG ATC GTT GGT GGA GTT TAC CTG TTG CCG CGC AGG GGC CCC AGG    446
Gly Gln Ile Val Gly Gly Val Tyr Leu Leu Pro Arg Arg Gly Pro Arg
        30                  35                  40
TTG GTT GTG CGC GCG ACT AGG AAG ACT TCC GAG CGG TCG CAA CCT CGT    494
Leu Gly Val Arg Ala Thr Arg Lys Thr Ser Glu Arg Ser Gln Pro Arg
    45                  50                  55
GGA AGG CGA CAA CCT ATC CCC AAG GCT CGC CAA CCC GAG GGC AGG GCC    542
Gly Arg Arg Gln Pro Ile Pro Lys Ala Arg Gln Pro Glu Gly Arg Ala
60                  65                  70                  75
TGG GCT CAG CCC GGG TAC CCT TGG CCC CTC TAT GGC AAT GAG GGC TTG    590
Trp Ala Gln Pro Gly Tyr Pro Trp Pro Leu Tyr Gly Asn Glu Gly Leu
                80                  85                  90
GGG TGG GCA GGA TGG CTC CTG TCA CCC CGC GGC TCC CGG CCT AGT TGG    638
Gly Trp Ala Gly Trp Leu Leu Ser Pro Arg Gly Ser Arg Pro Ser Trp
            95                  100                 105
GGC CCC ACG GAC CCC CGG CGT AGG TCG CGT AAT TTG GGT AAG GTC ATC    686
Gly Pro Thr Asp Pro Arg Arg Arg Ser Arg Asn Leu Gly Lys Val Ile
        110                 115                 120
GAT ACC CTC ACA TGC GGC TTC GCC GAT CTC ATG GGT ACA TTC CGC TCG    734
Asp Thr Leu Thr Cys Gly Phe Ala Asp Leu Met Gly Tyr Ile Pro Leu
```

```
     125                 130                 135
GTC GGC GCC CCC CTA GGG GGC GCT GCC AGG GCT CTA GCG CAT GGC GTC     782
Val Gly Ala Pro Leu Gly Gly Ala Ala Arg Ala Leu Ala His Gly Val
140                 145                 150                 155
CGG GTT CTG GAG GAC GGC GTG AAC TAT GCA ACA GGG AAT CTG CCT GGT     830
Arg Val Leu Glu Asp Gly Val Asn Tyr Ala Thr Gly Asn Leu Pro Gly
                160                 165                 170
TGC TCC TTT TCT ATC TTC CTT TTG GCT TTG CTG TCC TGT TTG ACC ATC     878
Cys Ser Phe Ser Ile Phe Leu Leu Ala Leu Leu Ser Cys Leu Thr Ile
            175                 180                 185
CCA GCT TCC GCC TAC CAA GTG CGC AAC GCG TCC GGG GTG TAC CAT GTC     926
Pro Ala Ser Ala Tyr Gln Val Arg Asn Ala Ser Gly Val Tyr His Val
        190                 195                 200
ACG AAC GAC TGC TCC AAC TCA AGT ATT GTG TAT GAG GCG GCG GAC GTG     974
Thr Asn Asp Cys Ser Asn Ser Ser Ile Val Tyr Glu Ala Ala Asp Val
    205                 210                 215
ATT ATG CAC ACC CCC GGG TGC GTG CCC TGC GTC CGG GAG AAC AAT TCC    1022
Ile Met His Thr Pro Gly Cys Val Pro Cys Val Arg Glu Asn Asn Ser
220                 225                 230                 235
TCC CGC TGC TGG GTA GCG CTC ACT CCC ACG CTT GCG GCC AGG AAC AGC    1070
Ser Arg Cys Trp Val Ala Leu Thr Pro Thr Leu Ala Ala Arg Asn Ser
                240                 245                 250
AGC ATC CCC ACT ACG ACA ATA CGG CGT CAT GTC GAC TTG CTC GTT GGG    1118
Ser Ile Pro Thr Thr Thr Ile Arg Arg His Val Asp Leu Leu Val Gly
            255                 260                 265
GCA GCT GCT CTC TGT TCC GCT ATG TAT GTG GGG GAT TTT TGC GGA TCT    1166
Ala Ala Ala Leu Cys Ser Ala Met Tyr Val Gly Asp Phe Cys Gly Ser
        270                 275                 280
GTT TTC CTC GTC TCC CAG CTG TTC ACT TTC TCA CCT CGC CGG TAT GAG    1214
Val Phe Leu Val Ser Gln Leu Phe Thr Phe Ser Pro Arg Arg Tyr Glu
    285                 290                 295
ACG GTG CAA GAC TGC AAT TGC TCA ATC TAT CCC GGC CAT GTA TCA GGC    1262
Thr Val Gln Asp Cys Asn Cys Ser Ile Tyr Pro Gly His Val Ser Gly
300                 305                 310                 315
CAT CGC ATG GCT TGG GAT ATG ATA ATG AAT TGG TCA CCT ACA ACA GCC    1310
His Arg Met Ala Trp Asp Met Ile Met Asn Trp Ser Pro Thr Thr Ala
                320                 325                 330
```

```
CTA GTG GTA TCG CAG CTA CTC CGG ATC CCA CAA GCC GTC GTG GAT ATG  1358
Leu Val Val Ser Gln Leu Leu Arg Ile Pro Gln Ala Val Val Asp Met
            335                 340                 345
GTG GCG GGG GCC CAC TGG GGA GTC CTG GCG GGC CTT GCC TAC TAT TCC  1406
Val Ala Gly Ala His Trp Gly Val Leu Ala Gly Leu Ala Tyr Tyr Ser
        350                 355                 360
ATG GTG GGG AAC TGG GCT AAG GTC TTG GTT GTG ATG CTG CTC TTC GCC  1454
Met Val Gly Asn Trp Ala Lys Val Leu Val Val Met Leu Leu Phe Ala
    365                 370                 375
GGT GTT GAC GGG GGG ACC CAC GTG ACA GGG GGA AAG GTA GCC TAC ACC  1502
Gly Val Asp Gly Gly Thr His Val Thr Gly Gly Lys Val Ala Tyr Thr
380                 385                 390                 395
ACC CAG AGC TTT ACA TCC TTC TTT TCA CGA GGG CCG TCT CAG AGA ATC  1550
Thr Gln Ser Phe Thr Ser Phe Phe Ser Arg Gly Pro Ser Gln Arg Ile
                400                 405                 410
CAG C                                                            1554
Gln
```

SEQ ID NO:7

SEQUENCE LENGTH: 370 base pairs

SEQUENCE TYPE: nucleic acid

STRANDEDNESS: double

TOPOLOGY: linear

MOLECULE TYPE: cDNA to genomic RNA

ANTI-SENSE: No

ORIGINAL SOURCE

ORGANISM: Hepatitis C virus

IMMEDIATE EXPERIMENTAL SOURCE

CLONE: HN3

```
GCAAGCTT ATG AGC ACA AAT CCA AAA CCC CAA AGA AAA ACC AAA CGT     47
         Met Ser Thr Asn Pro Lys Pro Gln Arg Lys Thr Lys Arg
           1               5                  10
AAC ACC AAC CGC CGC CCA CAG GAC GTC AAG TTC CCG GGC GGT GGT CAG   95
Asn Thr Asn Arg Arg Pro Gln Asp Val Lys Phe Pro Gly Gly Gly Gln
    15                  20                  25
ATC GTT GGT GGA GTT TAC CTG TTG CCG CGC AGG GGC CCC AGG TTG GGT  143
```

```
Ile Val Gly Gly Val Tyr Leu Leu Pro Arg Arg Gly Pro Arg Leu Gly
 30                 35                  40                  45
GTG CGC GCG ACT AGG AAG ACT TCC GAG CGG CCG CAA CCT CGT GGA AGG   191
Val Arg Ala Thr Arg Lys Thr Ser Glu Arg Pro Gln Pro Arg Gly Arg
                 50                  55                  60
CGA CAA CCT ATC CCC AAG GCT CGC CAA CCC GAG GGT AGG GCC TGG GCT   239
Arg Gln Pro Ile Pro Lys Ala Arg Gln Pro Glu Gly Arg Ala Trp Ala
             65                  70                  75
CAG CCC GGG TAC CCT TGG CCC CTC TAT GGC AAT GAG GGC TTG GGG TGG   287
Gln Pro Gly Tyr Pro Trp Pro Leu Tyr Gly Asn Glu Gly Leu Gly Trp
         80                  85                  90
GCA GGA TGG CTC CTG TCA CCC CGC GGC TCC CGG CCT AGT TGG GGC CCC   335
Ala Gly Trp Leu Leu Ser Pro Arg Gly Ser Arg Pro Ser Trp Gly Pro
     95                 100                 105
ACG GAC CCC CGG CGT AGG TGAAGATCTG AATTCGC                        370
Thr Asp Pro Arg Arg Arg
110                 115
```

SEQ ID NO:8

SEQUENCE LENGTH: 1264 base pairs

SEQUENCE TYPE: nucleic acid

STRANDEDNESS: double

TOPOLOGY: linear

MOLECULE TYPE: cDNA to genomic RNA

ANTI-SENSE: No

ORIGINAL SOURCE

ORGANISM: Hepatitis C virus

IMMEDIATE EXPERIMENTAL SOURCE

CLONE: HN3N10ΔB

```
GCAAGCTT ATG AGC ACA AAT CCA AAA CCC CAA AGA AAA ACC AAA CGT       47
         Met Ser Thr Asn Pro Lys Pro Gln Arg Lys Thr Lys Arg
           1               5                  10
AAC ACC AAC CGC CGC CCA CAG GAC GTC AAG TTC CCG GGC GGT GGT CAG    95
Asn Thr Asn Arg Arg Pro Gln Asp Val Lys Phe Pro Gly Gly Gly Gln
     15                  20                  25
ATC GTT GGT GGA GTT TAC CTG TTG CCG CGC AGG GGC CCC AGG TTG GGT   143
```

```
Ile Val Gly Gly Val Tyr Leu Leu Pro Arg Arg Gly Pro Arg Leu Gly
 30                 35                  40                  45
GTG CGC GCG ACT AGG AAG ACT TCC GAG CGG TCG CAA CCT CGT GGA AGG    191
Val Arg Ala Thr Arg Lys Thr Ser Glu Arg Ser Gln Pro Arg Gly Arg
                50                  55                  60
CGA CAA CCT ATC CCC AAG GCT CGC CAA CCC GAG GGC AGG GCC TGG GCT    239
Arg Gln Pro Ile Pro Lys Ala Arg Gln Pro Glu Gly Arg Ala Trp Ala
            65                  70                  75
CAG CCC GGG TAC CCT TGG CCC CTC TAT GGC AAT GAG GGC TTG GGG TGG    287
Gln Pro Gly Tyr Pro Trp Pro Leu Tyr Gly Asn Glu Gly Leu Gly Trp
        80                  85                  90
GCA GGA TGG CTC CTG TCA CCC CGC GGC TCC CGG CCT AGT TGG GGC CCC    335
Ala Gly Trp Leu Leu Ser Pro Arg Gly Ser Arg Pro Ser Trp Gly Pro
    95                  100                 105
ACG GAC CCC CGG CGT AGG TCG CGT AAT TTG GGT AAG GTC ATC GAT ACC    383
Thr Asp Pro Arg Arg Arg Ser Arg Asn Leu Gly Lys Val Ile Asp Thr
110                 115                 120                 125
CTC ACA TGC GGC TTC GCC GAT CTC ATG GGT ACA TTC CGC TCG GTC GGC    431
Leu Thr Cys Gly Phe Ala Asp Leu Met Gly Tyr Ile Pro Leu Val Gly
                130                 135                 140
GCC CCC CTA GGG GGC GCT GCC AGG GCT CTA GCG CAT GGC GTC CGG GTT    479
Ala Pro Leu Gly Gly Ala Ala Arg Ala Leu Ala His Gly Val Arg Val
            145                 150                 155
CTG GAG GAC GGC GTG AAC TAT GCA ACA GGG AAC CTG GGT GGT TGC TCC    527
Leu Glu Asp Gly Val Asn Tyr Ala Thr Gly Asn Leu Pro Gly Cys Ser
        160                 165                 170
TTT TCT ATC TTC CTT TTG GCT TTG CTG TCC TGT TTG ACC ATC CCA GCT    575
Phe Ser Ile Phe Leu Leu Ala Leu Leu Ser Cys Leu Thr Ile Pro Ala
    175                 180                 185
TCC GCC TAC CAA GTG CGC AAC GCG TCC GGG GTG TAC CAT GTC ACG AAC    623
Ser Ala Tyr Gln Val Arg Asn Ala Ser Gly Val Tyr His Val Thr Asn
190                 195                 200                 205
GAC TGC TCC AAC TCA AGT ATT GTG TAT GAG GCG GCG GAC GTG ATT ATG    671
Asp Cys Ser Asn Ser Ser Ile Val Tyr Glu Ala Ala Asp Val Ile Met
                210                 215                 220
CAC ACC CCC GGG TGC GTG CCC TGC GTC CGG GAG AAC AAT TCC TCC CGC    719
His Thr Pro Gly Cys Val Pro Cys Val Arg Glu Asn Asn Ser Ser Arg
```

225 230 235
TGC TGG GTA GCG CTC ACT CCC ACG CTT GCG GCC AGG AAC AGC AGC ATC 767
Cys Trp Val Ala Leu Thr Pro Thr Leu Ala Ala Arg Asn Ser Ser Ile
240 245 250
CCC ACT ACG ACA ATA CGG CGT CAT GTC GAC TTG CTC GTT GGG GCA GCT 815
Pro Thr Thr Thr Ile Arg Arg His Val Asp Leu Leu Val Gly Ala Ala
255 260 265
GCT CTC TGT TCC GCT ATG TAT GTG GGG GAT TTT TGC GGA TCT GTT TTC 963
Ala Leu Cys Ser Ala Met Tyr Val Gly Asp Phe Cys Gly Ser Val Phe
270 275 280 285
CTC GTC TCC CAG CTG TTC ACT TTC TCA CCT CGC CGG TAT GAG ACG GTG 911
Leu Val Ser Gln Leu Phe Thr Phe Ser Pro Arg Arg Tyr Glu Thr Val
290 295 300
CAA GAC TGC AAT TGC TCA ATC TAT CCC GGC CAT GTA TCA GGC CAT CGC 959
Gln Asp Cys Asn Cys Ser Ile Tyr Pro Gly His Val Ser Gly His Arg
305 310 315
ATG GCT TGG GAT ATG ATA ATG AAT TGG TCA CCT ACA ACA GCC CTA GTG 1007
Met Ala Trp Asp Met Ile Met Asn Trp Ser Pro Thr Thr Ala Leu Val
320 325 330
GTA TCG CAG CTA CTC CGG ATA CCA CAA GCC GTC GTG GAT ATG GTG GCG 1015
Val Ser Gln Leu Leu Arg Ile Pro Gln Ala Val Val Asp Met Val Ala
335 340 345
GGG GCC CAC TGG GGA GTC CTG GCG GGC CTT GCC TAC TAT TCC ATG GTG 1103
Gly Ala His Trp Gly Val Leu Ala Gly Leu Ala Tyr Tyr Ser Met Val
350 355 360 365
GGG AAC TGG GCT AAG GTC TTG GTT GTG ATG CTG CTC TTC GCC GGT GTT 1151
Gly Asn Trp Ala Lys Val Leu Val Val Met Leu Leu Phe Ala Gly Val
370 375 380
GAC GGG GGG ACC CAC GTG ACA GGG GGA AAG GTA GCC TAC ACC ACC CAG 1199
Asp Gly Gly Thr His Val Thr Gly Gly Lys Val Ala Tyr Thr Thr Gln
385 390 395
AGC TTT ACA TCC TTC TTT TCA CGA GGG CCG TCT CAG AGA ATC 1247
Ser Phe Thr Ser Phe Phe Ser Arg Gly Pro Ser Gln Arg Ile
400 405 410
TGAAGATCTG AATTCGC 1264

SEQ ID NO:9

SEQUENCE LENGTH: 483 base pairs
SEQUENCE TYPE: nucleic acid
STRANDEDNESS: double
TOPOLOGY: linear
ANTI-SENSE: No
MOLECULE TYPE: cDNA to genomic RNA
ORIGINAL SOURCE
ORGANISM: Hepatitis C virus
IMMEDIATE EXPERIMENTAL SOURCE
CLONE: N1-2

```
CTCCACCATA GATCACTCCC CTGTGAGGAA CTACTGTCTT CACGCAGAAA GCGTCTAGCC    60
ATGGCGTTAG TATGAGTGTC GTGCAGCCTC CAGGCCCCCC CCTCCCGGGA GAGCCATAGT   120
GGTCTGCGGA ACCGGTGAGT ACACCGGAAT TGCCAGGACG ACCGGGTCCT TTCTTGGATC   180
AACCCGCTCA ATGCCTGGAG ATTTGGGCGT GCCCCCGCGA GACTGCTAGC CGAGTAGTGT   240
TGGGTCGCGA AAGGCCTTGT GGTACTGCCT GATAGGGTGC TTGCGAGTGC CCCGGGAGGT   300
CTCGTAGACC GTGCATC ATG AGC ACA AAT CCT AAA CCC CAA AGA CAA ACC      350
                   Met Ser Thr Asn Pro Lys Pro Gln Arg Gln Thr
                    1               5                   10
AAA CGT AAC ACC AAC CGC CGC CCA CAG GAC GTC AAG TTC CCG GGC GGT     398
Lys Arg Asn Thr Asn Arg Arg Pro Gln Asp Val Lys Phe Pro Gly Gly
            15                  20                  25
GGT CAG ATC GTT GGT GGA GTT TAC CTG TTG CCG CGC AGG GGC CCC AGG     446
Gly Gln Ile Val Gly Gly Val Tyr Leu Leu Pro Arg Arg Gly Pro Arg
        30                  35                  40
TTG GGT GTG CGC GCG ACT AGG AAG ACT TCC GAG CGG T                   483
Leu Gly Val Arg Ala Thr Arg Lys Thr Ser Glu Arg
    45                  50
```

SEQ ID NO:10
SEQUENCE LENGTH: 483 base pairs
SEQUENCE TYPE: nucleic acid
STRANDEDNESS: double
TOPOLOGY: linear
ANTI-SENSE: No
MOLECULE TYPE: cDNA to genomic RNA
ORIGINAL SOURCE

ORGANISM: Hepatitis C virus
IMMEDIATE EXPERIMENTAL SOURCE
CLONE: S1-1

```
CTCCACCATA GATCACTCCC CTGTGAGGAA CTACTGTCTT CACGCAGAAA GCGTCTAGCC    60
ATGGCGTTAG TATGAGTGTC GTGCAGCCTC CAGGACCCCC CCTCCCGGGA GAGCCATAGT   120
GGTCTGCGGA ACCGGTGAGT ACACCGGAAT TGCCAGGACG ACCGGGTCCT TTCTTGGATT   180
AACCCGCTCA ATGCCTGGAG ATTTGGGCGT GCCCCCGCGA GACCGCTAGC CGAGTAGTGT   240
TGGGTCGCGA AAGGCCTTGT GGTACTGCCT GATAGGGTGC TTGCGAGTGC CCCGGGAGGT   300
CTCGTAGACC GTGCACC ATG AGC ACG AAT CCT AAA CCT CAA AGA AAA ACC      350
                   Met Ser Thr Asn Pro Lys Pro Gln Arg Lys Thr
                    1               5                   10
AAA CGT AAC ACC AAC CGC CGC CCA CAG GAC GTC AAG TTC CCG GGT GGT     398
Lys Arg Asn Thr Asn Arg Arg Pro Gln Asp Val Lys Phe Pro Gly Gly
         15                  20                  25
GGT CAG ATC GTT GGT GGA GTT TAC CTG TTG CCG CGC AGG GGC CCC AGG     446
Gly Gln Ile Val Gly Gly Val Tyr Leu Leu Pro Arg Arg Gly Pro Arg
     30                  35                  40
TTG GGT GTG CGC GCG ACT AGG AAG ACT TCC GAG CGG T                   483
Leu Gly Val Arg Ala Thr Arg Lys Thr Ser Glu Arg
 45                  50
```

SEQ ID NO:11
SEQUENCE LENGTH: 483 base pairs
SEQUENCE TYPE: nucleic acid
STRANDEDNESS: double
TOPOLOGY: linear
ANTI-SENSE: No
MOLECULE TYPE: cDNA to genomic RNA
ORIGINAL SOURCE
ORGANISM: Hepatitis C virus
IMMEDIATE EXPERIMENTAL SOURCE
CLONE: S1-2

```
CTCCACCATA GATCACTCCC CTGTGAGGAA CTACTGTCTT CACGCAGAAA GCGTCTAGCC    60
ATGGCGTTAG TATGAGTGTC GTGCAGCCTC CAGGACCCCC CCTCCCGGGA GAGCCATAGT   120
GGTCTGCGGA ACCGGTGAGT ACACCGGAAT TGCCAGGACG ACCGGGTCCT TTCTTGGATT   180
```

AACCCGCTCA ATGCCTGGAG ATTTGGGCGT GCCCCCGCGA GACCGCTAGC CGAGTAGTGT 240
TGGGTCGCGA AAGGCCTTGT GGTACTGCCT GATAGGGTGC TTGCGAGTGC CCCGGGAGGT 300
CTCGTAGACC GTGCACC ATG AGC ACG AAT CCT AAA CCT CAA AGA AAA ACC 350
Met Ser Thr Asn Pro Lys Pro Gln Arg Lys Thr
1 5 10
AAA CGT AAC ACC AAC CGC CGC CCA CAG GAC GTC AAG TTC CCG GGT GGT 398
Lys Arg Asn Thr Asn Arg Arg Pro Gln Asp Val Lys Phe Pro Gly Gly
15 20 25
GGT CAG ATC GTT GGT GGA GTT TAC CTG TTG CCG CGC AGG GGC CCC AGG 446
Gly Gln Ile Val Gly Gly Val Tyr Leu Leu Pro Arg Arg Gly Pro Arg
30 35 40
TTG GGT GTG CGC GCG ACT AGG AAG ACT TCC GAG CGG T 483
Leu Gly Val Arg Ala Thr Arg Lys Thr Ser Glu Arg
45 50

SEQ ID NO:12
SEQUENCE LENGTH: 483 base pairs
SEQUENCE TYPE: nucleic acid
STRANDEDNESS: double
TOPOLOGY: linear
ANTI-SENSE: No
MOLECULE TYPE: cDNA to genomic RNA
ORIGINAL SOURCE
ORGANISM: Hepatitis C virus
IMMEDIATE EXPERIMENTAL SOURCE
CLONE: S1-3

CTCCACCATA GATCACTCCC CTGTGAGGAA CTACTGTCTT CACGCAGAAA GCGTCTAGCC 60
ATGGCGTTAG TATGAGTGTC GTGCAGCCTC CAGGACCCCC CCTCCCGGGA GAGCCATAGT 120
GGTCTGCGGA ACCGGTGAGT ACACCGGAAT TGCCAGGACG ACCGGGTCCT TTCTTGGATT 180
AACCCGCTCA ATGCCTGGAG ATTTGGGCGT GCCCCCGCGA GACCGCTAGC CGAGTAGTGT 240
TGGGTCGCGA AAGGCCTTGT GGTACTGCCT GATGGGGTGC TTGCGAGTGC CCCGGGAGGT 300
CTCGTAGACC GTGCACC ATG AGC ACG AAT CCT AAA CCT CAA AGA AAA ACC 350
Met Ser Thr Asn Pro Lys Pro Gln Arg Lys Thr
1 5 10
AAA CGT AAC ACC AAC CGC CGC CCA CAG GAC GTC AAG TTC CCG GGT GGT 398
Lys Arg Asn Thr Asn Arg Arg Pro Gln Asp Val Lys Phe Pro Gly Gly

```
            15                  20                  25
GGT CAG ATC GTT GGT GGA GTT TAC CTG TTG CCG CGC AGG GGC CCC AGG      446
Gly Gln Ile Val Gly Gly Val Tyr Leu Leu Pro Arg Arg Gly Pro Arg
        30                  35                  40
TTG GGT GTG CGC GCG ACT AGG AAG ACT TCC GAG CGG T                    483
Leu Gly Val Arg Ala Thr Arg Lys Thr Ser Glu Arg
    45                  50
```

SEQ ID NO:13

SEQUENCE LENGTH: 339 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: N27-1

```
C CGG ATC CCA CAA GCC GTG GTG GAT ATG GTG GCG GGG GCC CAC TGG GGA 49
  Arg Ile Pro Gln Ala Val Val Asp Met Val Ala Gly Ala His Trp Gly
                      5                  10                  15
GTC CTA GCG GGC CTT GCC TAC TAT TCC ATG GTG GGG AAC TGG GCT AAG   97
Val Leu Ala Gly Leu Ala Tyr Tyr Ser Met Val Gly Asn Trp Ala Lys
                20                  25                  30
GTC TTG GTT GTG ATG CTG CTC TTC GCC GGT GTT GAC GGG AGG ACC CAC  145
Val Leu Val Val Met Leu Leu Phe Ala Gly Val Asp Gly Arg Thr His
            35                  40                  45
GTG ACA GGA GGG AAG GTA GCC TAC ACC ACC CAG AGG TTT ACA TCC TTC  193
Val Thr Gly Gly Lys Val Ala Tyr Thr Thr Gln Arg Phe Thr Ser Phe
    50                  55                  60
TTT TCA CGA GGG CCG TCC CAG AAA ATC CAA CTT GTA AAC ACT AAC GGC  241
Phe Ser Arg Gly Pro Ser Gln Lys Ile Gln Leu Val Asn Thr Asn Gly
65                  70                  75                  80
AGC TGG CAC ATC AAC AGG ACT GCC CTG AAT TGC AAT GAC TCC CTT AAC  289
Ser Trp His Ile Asn Arg Thr Ala Leu Asn Cys Asn Asp Ser Leu Asn
                  85                  90                  95
```

```
ACC GGG TTC CTT GCC GCG CTG TTC TAC ACC CAC AGC TTC AAC GCG TCC GG  339
Thr Gly Phe Leu Ala Ala Leu Phe Tyr Thr His Ser Phe Asn Ala Ser
            100                 105                 110
```

SEQ ID NO:14
SEQUENCE LENGTH: 339 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N27-2

```
C CGG ATC CCA CAA GCC GTG GTG GAT ATG GTG GCG GGG GCC CAC TGG GGA 49
  Arg Ile Pro Gln Ala Val Val Asp Met Val Ala Gly Ala His Trp Gly
                  5                  10                  15
GTC CTG GCG GGC CTT GCC TAC TAT TCC ATG GTG GGG AAC TGG GCT AAG   97
Val Leu Ala Gly Leu Ala Tyr Tyr Ser Met Val Gly Asn Trp Ala Lys
             20                  25                  30
GTC TTG GTT GTG ATG CTG CTT TTC GCC GGT GTT GAC GGG GGG ACC CAC  145
Val Leu Val Val Met Leu Leu Phe Ala Gly Val Asp Gly Gly Thr His
         35                  40                  45
GTG ACA GGG GGG AAG GTA GCC TAC ACC ACC CAG AGC TTC ACA TCC TTC  193
Val Thr Gly Gly Lys Val Ala Tyr Thr Thr Gln Ser Phe Thr Ser Phe
     50                  55                  60
TTT TCA CGA GGG CCG TCT CAG AGG ATC CAA CTT GTA AAC ACT AAC GGC  241
Phe Ser Arg Gly Pro Ser Gln Arg Ile Gln Leu Val Asn Thr Asn Gly
 65                  70                  75                  80
AGC TGG CAC ATC AAT AGG ACT GCC CTG AAT TGC AAT GAC TCC CTT AAC  289
Ser Trp His Ile Asn Arg Thr Ala Leu Asn Cys Asn Asp Ser Leu Asn
                 85                  90                  95
ACC GGG TTC CTT GCC GCG CTG TTC TAC ACC CAC AGC TTC AAC GCG TCC GG 339
Thr Gly Phe Leu Ala Ala Leu Phe Tyr Thr His Ser Phe Asn Ala Ser
            100                 105                 110
```

SEQ ID NO:15
SEQUENCE LENGTH: 339 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N27-3

```
C CGG ATC CCA CAA GCC GTG GTG GAT ATG GTG GCA GGG GCC CAC TGG GGA 49
  Arg Ile Pro Gln Ala Val Val Asp Met Val Ala Gly Ala His Trp Gly
                      5                  10                  15
GTC CTG GCG GGC CTT GCC TAC TAT TCC ATG GTG GGG AAC TGG GCT AAG   97
Val Leu Ala Gly Leu Ala Tyr Tyr Ser Met Val Gly Asn Trp Ala Lys
             20                  25                  30
GTC TTG GTT GTG ATG CTG CTC TTC GCC GGT GTT GAC GGG GGG ACC CAC  145
Val Leu Val Val Met Leu Leu Phe Ala Gly Val Asp Gly Gly Thr His
         35                  40                  45
GTG ACA GGG GGG AAG GTA GCC TAC ACC ACC CAG GGC TTT ACA CCC TTC  193
Val Thr Gly Gly Lys Val Ala Tyr Thr Thr Gln Gly Phe Thr Pro Phe
     50                  55                  60
TTT TCA CGA GGG CCG TCT CAG AAA ATC CAA CTT GTA AAC ACT AAC GGC  241
Phe Ser Arg Gly Pro Ser Gln Lys Ile Gln Leu Val Asn Thr Asn Gly
 65                  70                  75                  80
AGC TGG CAC ATC AAT AGG ACT GCC CTC AAT TGC AAT GAC TCC CTT AAC  289
Ser Trp His Ile Asn Arg Thr Ala Leu Asn Cys Asn Asp Ser Leu Asn
                 85                  90                  95
ACC GGG TTC CTT GCC GCG CTG TTC TAC ACC CAC AGC TTC AAC GCG TCC GG 339
Thr Gly Phe Leu Ala Ala Leu Phe Tyr Thr His Ser Phe Asn Ala Ser
            100                 105                 110
```

SEQ ID NO:16
SEQUENCE LENGTH: 393 base pairs
SEQUENCE TYPE: nucleic acid
STRANDEDNESS: double

TOPOLOGY: linear
ANTI-SENSE: No
ORIGINAL SOURCE
ORGANISM: Hepatitis C virus
IMMEDIATE EXPERIMENTAL SOURCE
CLONE: N19-1

```
GAG GCC GTG AAC TGC GAT GAC TCC CTT AAC ACC GGG TTC CTT GCC GCG    48
Glu Ala Val Asn Cys Asp Asp Ser Leu Asn Thr Gly Phe Leu Ala Ala
  1               5                  10                  15
CTG TTC TAC ACG CAC AGG TTC AAC GCG TCC GGA TGT CCG GAG CGT ATG    96
Leu Phe Tyr Thr His Arg Phe Asn Ala Ser Gly Cys Pro Glu Arg Met
             20                  25                  30
GCC GGT TGC CGC CCC ATT GAC GAG TTC GCT CAG GGG TGG GGT CCC ATC   144
Ala Gly Cys Arg Pro Ile Asp Glu Phe Ala Gln Gly Trp Gly Pro Ile
         35                  40                  45
ACT CAT GTT GTG CCT AAC ATC TCG GAC CAG AGG CCC TAT TGC TGG CAC   192
Thr His Val Val Pro Asn Ile Ser Asp Gln Arg Pro Tyr Cys Trp His
     50                  55                  60
TAC GCG CCT CGA CCG TGT GGT ATC GTA CCC GCG TCG CAG GTG TGT GGT   240
Tyr Ala Pro Arg Pro Cys Gly Ile Val Pro Ala Ser Gln Val Cys Gly
 65                  70                  75                  80
CCG GTG TAT TGC TTC ACC CCA AGC CCT GTT GTG GTG GGG ACG ACC GAT   288
Pro Val Tyr Cys Phe Thr Pro Ser Pro Val Val Val Gly Thr Thr Asp
                 85                  90                  95
CGT TTC GGC GCC CCC ACG TAC AAC TGG GGA AAC AAT GAG ACG GAT GTG   336
Arg Phe Gly Ala Pro Thr Tyr Asn Trp Gly Asn Asn Glu Thr Asp Val
            100                 105                 110
CTA CTC CTC AAC AAC ACA CGG CCG CCG CAG GGC AAC TGG TTC GGT TGT   384
Leu Leu Leu Asn Asn Thr Arg Pro Pro Gln Gly Asn Trp Phe Gly Cys
        115                 120                 125
ACC TGG ATG                                                       393
Thr Trp Met
    130
```

SEQ ID NO:17
SEQUENCE LENGTH: 393 base pairs

SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N19-2

```
GAG GCC GTG AAC TGC GAT GAC TCC CTT AAC ACC GGG TTC CTT GCC GCG    48
Glu Ala Val Asn Cys Asp Asp Ser Leu Asn Thr Gly Phe Leu Ala Ala
 1               5                  10                  15
CTG TTC TAC ACG CAC AGG TTC AAC GCG TCC GGA TGT CCG GAG CGT ATG    96
Leu Phe Tyr Thr His Arg Phe Asn Ala Ser Gly Cys Pro Glu Arg Met
                20                  25                  30
GCC AGT TGC CGC CCC ATT GAC GAG TTC GCT CAG GGG TGG GGT CCC ATC   144
Ala Ser Cys Arg Pro Ile Asp Glu Phe Ala Gln Gly Trp Gly Pro Ile
            35                  40                  45
ACT CAT GTT GTG CCT AAC ATC TCG GAC CAG AGG CCC TAT TGC TGG CAC   192
Thr His Val Val Pro Asn Ile Ser Asp Gln Arg Pro Tyr Cys Trp His
        50                  55                  60
TAC GCG CCT CGA CCG TGT GGT ATC GTA CCC GCG TCG CAG GTG TGT GGT   240
Tyr Ala Pro Arg Pro Cys Gly Ile Val Pro Ala Ser Gln Val Cys Gly
 65                  70                  75                  80
CCG GTG TAT TGC TTC ACC CCA AGC CCT GTT GTG GTG GGG ACG ACC GAT   288
Pro Val Tyr Cys Phe Thr Pro Ser Pro Val Val Val Gly Thr Thr Asp
                85                  90                  95
CGT TTC GGC GCC CCC ACG TAT AAC TGG GGG AAC AAT GAG ACG GAT GTG   336
Arg Phe Gly Ala Pro Thr Tyr Asn Trp Gly Asn Asn Glu Thr Asp Val
            100                 105                 110
CTA CTC CTC AAC AAC ACA CGG CCG CCG CAA GGC AAC TGG TTC GGT TGT   384
Leu Leu Leu Asn Asn Thr Arg Pro Pro Gln Gly Asn Trp Phe Gly Cys
        115                 120                 125
ACC TGG ATG                                                       393
Thr Trp Met
    130
```

SEQ ID NO:18
SEQUENCE LENGTH: 393 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N19-3

```
GAG GCC GTG AAC TGC GAT GAC TCC CTT AAC ACC GGG TTC CTT GCC GCG   48
Glu Ala Val Asn Cys Asp Asp Ser Leu Asn Thr Gly Phe Leu Ala Ala
 1               5                  10                  15
CTG TTC TAC ACG CAC AGG TTC AAC GCG TCC GGA TGT CCG GAG CGT ATG   96
Leu Phe Tyr Thr His Arg Phe Asn Ala Ser Gly Cys Pro Glu Arg Met
            20                  25                  30
GCC AGT TGC CGC CCC ATT GAT GAG TTC GCT CAG GGG TGG GGT CCC ATC  144
Ala Ser Cys Arg Pro Ile Asp Glu Phe Ala Gln Gly Trp Gly Pro Ile
        35                  40                  45
ACT CAT GTT GTG CCT AAC ATC TCG GAC CAG AGG CCC TAT TGC TGG CAC  192
Thr His Val Val Pro Asn Ile Ser Asp Gln Arg Pro Tyr Cys Trp His
    50                  55                  60
TAC GCG CCT CGA CCG TGT GGT ATC GTA CCC GCG TGG CAG GTG TGT GGT  240
Tyr Ala Pro Arg Pro Cys Gly Ile Val Pro Ala Trp Gln Val Cys Gly
65                  70                  75                  80
CCG GTG TAT TGC TTC ACC CCA AGC CCT GTT GTG GTG GGG ACG ACC GAT  288
Pro Val Tyr Cys Phe Thr Pro Ser Pro Val Val Val Gly Thr Thr Asp
                85                  90                  95
CGT TTC GGC GCC CCC ACG TAT AAC TGG GGG AAC AAT GAG ACG GAT GTG  336
Arg Phe Gly Ala Pro Thr Tyr Asn Trp Gly Asn Asn Glu Thr Asp Val
            100                 105                 110
CTA CTC CTC AAC AAC ACA CGG CCG CCG CAA GGC AAC TGG TTC GGT TGT  384
Leu Leu Leu Asn Asn Thr Arg Pro Pro Gln Gly Asn Trp Phe Gly Cys
        115                 120                 125
ACC TGG ATG                                                      393
Thr Trp Met
```

130

SEQ ID NO:19
SEQUENCE LENGTH: 393 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H19-2

```
GAG GCC GTG AAC TGC GAT GAC TCC CTC CAG ACT GGG TTC CTT GCC GCG    48
Glu Ala Val Asn Cys Asp Asp Ser Leu Gln Thr Gly Phe Leu Ala Ala
 1               5                  10                  15
CTG TTC TAC AGG CAC AGG TTC AAC GCA TCC GGG TGC CCA GAA CGC ATG    96
Leu Phe Tyr Arg His Arg Phe Asn Ala Ser Gly Cys Pro Glu Arg Met
            20                  25                  30
GCC AGC TGC CGC CCC ATT AGC GAG TTC GCT CAG GGG TGG GGT CCT ATC   144
Ala Ser Cys Arg Pro Ile Ser Glu Phe Ala Gln Gly Trp Gly Pro Ile
        35                  40                  45
ACT CAT GTT GTG CCT GAC GTG TCG GAC CAG AGG CCT TAT TGC TGG CAC   192
Thr His Val Val Pro Asp Val Ser Asp Gln Arg Pro Tyr Cys Trp His
    50                  55                  60
TAC GCG CCT CGA CCG TGC GGT ATC GTA CCC GCG TCG CAG GTG TGT GGT   240
Tyr Ala Pro Arg Pro Cys Gly Ile Val Pro Ala Ser Gln Val Cys Gly
65                  70                  75                  80
CCA GTG TAT TGC TTC ACC CCA AGC CCT GTC GTG GTG GGG ACG ACC GAT   288
Pro Val Tyr Cys Phe Thr Pro Ser Pro Val Val Val Gly Thr Thr Asp
                85                  90                  95
CGT TCT GGC GCC CCC ACG TAC ACC TGG GGG GCG AAT GAG ACG GAC GTG   336
Arg Ser Gly Ala Pro Thr Tyr Thr Trp Gly Ala Asn Glu Thr Asp Val
            100                 105                 110
CTA CTC CTT AAC AAC ACG CGT CCG CCA CAG GGC AAC TGG TTC GGT TGT   384
Leu Leu Leu Asn Asn Thr Arg Pro Pro Gln Gly Asn Trp Phe Gly Cys
        115                 120                 125
```

ACC TGG ATG 393
Thr Trp Met
130

SEQ ID NO:20
SEQUENCE LENGTH: 393 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H19-4

```
GAG GCC GTG AAC TGC GAT GAC TCC CTC CAG ACT GGG TTC CTT GCC GCG   48
Glu Ala Val Asn Cys Asp Asp Ser Leu Gln Thr Gly Phe Leu Ala Ala
  1               5                  10                  15
CTG TTC TAC AGG CAC AGG TTC AAC GCA TCC GGG TGC CCA GAA CGC ATG   96
Leu Phe Tyr Arg His Arg Phe Asn Ala Ser Gly Cys Pro Glu Arg Met
                 20                  25                  30
GCC AGC TGT CGC CCC ATT AGC GAG TTC GCT CAG GGG TGG GGC CCT ATC  144
Ala Ser Cys Arg Pro Ile Ser Glu Phe Ala Gln Gly Trp Gly Pro Ile
             35                  40                  45
ACT CAT GTT GTG CCT GAC GTG TCG GAC CAG AGG CCT TAT TGC TGG CAC  192
Thr His Val Val Pro Asp Val Ser Asp Gln Arg Pro Tyr Cys Trp His
         50                  55                  60
TAC GCG CCT CGA CCG TGC GGT ATC GTA CCC GCG TCG CAG GTG TGT GGT  240
Tyr Ala Pro Arg Pro Cys Gly Ile Val Pro Ala Ser Gln Val Cys Gly
 65                  70                  75                  80
CCA GTG TAT TGC TTC ACC CCA AGC CCT GTC GTG GTG GGG ACG ACC GAT  288
Pro Val Tyr Cys Phe Thr Pro Ser Pro Val Val Val Gly Thr Thr Asp
                 85                  90                  95
CGC TCT GGC GCC CCC ACG TAC ACC TGG GGG GCG AAT GAG ACG GAC GTG  336
Arg Ser Gly Ala Pro Thr Tyr Thr Trp Gly Ala Asn Glu Thr Asp Val
            100                 105                 110
CTA CTC CTT AAC AAC ACG CGT CCG CCA CAG GGC AAC TGG TTC GGT TGT  384
```

```
Leu Leu Leu Asn Asn Thr Arg Pro Pro Gln Gly Asn Trp Phe Gly Cys
        115                 120                 125
ACC TGG ATG                                                         393
Thr Trp Met
    130
```

SEQ ID NO:21
SEQUENCE LENGTH: 393 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H19-10

```
GAG GCC GTG AAC TGC GAT GAC TCC CTC CAG ACT GGG TTC CTT GCC GCG   48
Glu Ala Val Asn Cys Asp Asp Ser Leu Gln Thr Gly Phe Leu Ala Ala
  1               5                  10                  15
CTG TTC TAC AGG CAC AGG TTC AAC GCA TCC GGG TGC CCA GAA CGC ATG   96
Leu Phe Tyr Arg His Arg Phe Asn Ala Ser Gly Cys Pro Glu Arg Met
             20                  25                  30
GCC AGC TGC CGC CCC ATT AGC GAG TTC GCT CAG GGG TGG GGC CCT ATC  144
Ala Ser Cys Arg Pro Ile Ser Glu Phe Ala Gln Gly Trp Gly Pro Ile
         35                  40                  45
ACT CAT GTT GTG CCT GAC GTG TCG GAC CAG AGG CCT TAT TGC TGG CAC  192
Thr His Val Val Pro Asp Val Ser Asp Gln Arg Pro Tyr Cys Trp His
     50                  55                  60
TAC GCA CCT CGA CCG TGC GGT GTC GTA CCC GCG TCG CAG GTG TGT GGT  240
Tyr Ala Pro Arg Pro Cys Gly Val Val Pro Ala Ser Gln Val Cys Gly
 65                  70                  75                  80
CCA GTG TAT TGC TTC ACC CCA AGC CCT GTC GTG GTG GGG ACG ACC GAT  288
Pro Val Tyr Cys Phe Thr Pro Ser Pro Val Val Val Gly Thr Thr Asp
                 85                  90                  95
CGC TCT GGC GCC CCC ACG TAC ACC TGG GGG GCG AAT GAG ACG GAC GTG  336
Arg Ser Gly Ala Pro Thr Tyr Thr Trp Gly Ala Asn Glu Thr Asp Val
```

```
              100                      105                      110
    CTA CTC CTT AAC AAC ACG CGT CCG CCA CAG GGC AAC TGG TTC GGT TGT   384
    Leu Leu Leu Asn Asn Thr Arg Pro Pro Gln Gly Asn Trp Phe Gly Cys
            115                     120                     125
    ACC TGG ATG                                                       393
    Thr Trp Met
        130
```

SEQ ID NO:22
SEQUENCE LENGTH: 393 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: Y19-4

```
    GAG GCC GTG AAC TGC GAT GAC TCC CTC CAG ACT GGG TTC CTT GCC GCG    48
    Glu Ala Val Asn Cys Asp Asp Ser Leu Gln Thr Gly Phe Leu Ala Ala
      1               5                  10                  15
    CTG TTC TAC AGG CAC AGG TTC AAC GCA TCC GGG TGC CCA GAA CGC ATG    96
    Leu Phe Tyr Arg His Arg Phe Asn Ala Ser Gly Cys Pro Glu Arg Met
                     20                  25                  30
    GCC AGC TGT CGC CCC ATT AGC GAG TTC GCT CAG GGG TGG GGC CCT ATC   144
    Ala Ser Cys Arg Pro Ile Ser Glu Phe Ala Gln Gly Trp Gly Pro Ile
                 35                  40                  45
    ACT CAT GTT GTG CCT GAC GTG TCG GAC CAG AGG CCT TAT TGC TGG CAC   192
    Thr His Val Val Pro Asp Val Ser Asp Gln Arg Pro Tyr Cys Trp His
             50                  55                  60
    TAC GCG CCT CGA CCG TGC GGT ATC GTA CCC GCG TCG CAG GTG TGT GGT   240
    Tyr Ala Pro Arg Pro Cys Gly Ile Val Pro Ala Ser Gln Val Cys Gly
     65                  70                  75                  80
    CCA GTG TAT TGC TTC ACC CCA AGC CCT GTC GTG GTG GGG ACG ACC GAT   288
    Pro Val Tyr Cys Phe Thr Pro Ser Pro Val Val Val Gly Thr Thr Asp
                     85                  90                  95
```

```
CGC TCT GGC GCC CCC ACG TAC ACC TGG GGG GCG AAT GAG ACG GAC GTG   336
Arg Ser Gly Ala Pro Thr Tyr Thr Trp Gly Ala Asn Glu Thr Asp Val
            100                 105                 110
CTA CTC CTT AAC AAC ACG CGT CCG CCA CAG GGC AAC TGG TTC GGT TGT   384
Leu Leu Leu Asn Asn Thr Arg Pro Pro Gln Gly Asn Trp Phe Gly Cys
        115                 120                 125
ACC TGG ATG                                                       393
Thr Trp Met
    130
```

SEQ ID NO:23
SEQUENCE LENGTH: 393 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: Y19-6

```
GAG GCC GTG AAC TGC GAT GAC TCC CTC CAG ACT GGG TTC CTT GCC ACG   48
Glu Ala Val Asn Cys Asp Asp Ser Leu Gln Thr Gly Phe Leu Ala Thr
  1               5                  10                  15
CTG TTC TAC AGG CAC AGG TTC AAC GCA TCC GGG TGC CCA GAA CGC ATG   96
Leu Phe Tyr Arg His Arg Phe Asn Ala Ser Gly Cys Pro Glu Arg Met
            20                  25                  30
GCC AGC TGT CGC CCC ATT AGC GAG TTC GCT CAG GGG TGG GAC CCT ATC   144
Ala Ser Cys Arg Pro Ile Ser Glu Phe Ala Gln Gly Trp Asp Pro Ile
        35                  40                  45
ACT CAT GTT GTG CCT GAC GTG TCG GAC CAG AGG CCT TAT TGC TGG CAC   192
Thr His Val Val Pro Asp Val Ser Asp Gln Arg Pro Tyr Cys Trp His
    50                  55                  60
TAC GCG CCT CGA CCG TGC GGT ATC GTA CCC GCG TCG CAG GTG TGT GGT   240
Tyr Ala Pro Arg Pro Cys Gly Ile Val Pro Ala Ser Gln Val Cys Gly
 65                  70                  75                  80
CCA GTG TAT TGC TTC ACC CCA AGC CCT GTC GTG GTG GGG ACG ACC GAT   288
```

```
Pro Val Tyr Cys Phe Thr Pro Ser Pro Val Val Val Gly Thr Thr Asp
                 85                      90                  95
CGC TCT GGC GCC CCC ACG TAC ACC TGG GGG GCG AAT GAG ACG GAC GTG   336
Arg Ser Gly Ala Pro Thr Tyr Thr Trp Gly Ala Asn Glu Thr Asp Val
            100                     105                 110
CTA CTC CTT AAC AAC ACG CGT CCG CCA CAG GGC AAC TGG TTC GGT TGT   384
Leu Leu Leu Asn Asn Thr Arg Pro Pro Gln Gly Asn Trp Phe Gly Cys
        115                     120                 125
ACC TGG ATG                                                       393
Thr Trp Met
    130
```

SEQ ID NO:24
SEQUENCE LENGTH: 393 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: Y19-7

```
GAG GCC GTG AAC TGC GAT GAC TCC CTC CAG ACT GGG TTC CTT GCC GCG   48
Glu Ala Val Asn Cys Asp Asp Ser Leu Gln Thr Gly Phe Leu Ala Ala
  1               5                  10                  15
CTG TTC TAC AGG CAT AGG TTC GAC GCA TCC GGG TGC CCA GAA CGC ATG   96
Leu Phe Tyr Arg His Arg Phe Asp Ala Ser Gly Cys Pro Glu Arg Met
            20                  25                  30
GCC AGC TGT CGC CCC ATT AGC GAG TTC GCT CAG GGG TGG GGC CCT ATC   144
Ala Ser Cys Arg Pro Ile Ser Glu Phe Ala Gln Gly Trp Gly Pro Ile
        35                  40                  45
ACT CAT GTT GTG CCT GAC GTG TCG GAC CAG AGG CCT TAT TGC TGG CAC   192
Thr His Val Val Pro Asp Val Ser Asp Gln Arg Pro Tyr Cys Trp His
    50                  55                  60
TAC GCG CCT CGA CCG TGC GGT ATC GTA CCC GCG TCG CAG GTG TGT GGT   240
Tyr Ala Pro Arg Pro Cys Gly Ile Val Pro Ala Ser Gln Val Cys Gly
```

```
 65                  70                  75                  80
CCA GTG TAT TGC TTC ACC CCA AGC CCT GTC GTG GTG GGG ACG ACC GAT     288
Pro Val Tyr Cys Phe Thr Pro Ser Pro Val Val Val Gly Thr Thr Asp
                 85                  90                  95
CGC TCT GGC GCC CCC ACG TAC ACC TGG GGG GCG AAT GAG ACG GAC GTG     336
Arg Ser Gly Ala Pro Thr Tyr Thr Trp Gly Ala Asn Glu Thr Asp Val
           100                 105                 110
CTA CTC CTT AAC AAC ACG CGT CCG CCA CAG GGC AAC TGG TTC GGT TGT     384
Leu Leu Leu Asn Asn Thr Arg Pro Pro Gln Gly Asn Trp Phe Gly Cys
        115                 120                 125
ACC TGG ATG                                                         393
Thr Trp Met
    130
```

SEQ ID NO:25
SEQUENCE LENGTH: 629 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX24-4

```
AAC ACA CGG CCG CCG CAG GGG AAC TGG TTT GGC TGT ACA TGG ATG AAT     48
Asn Thr Arg Pro Pro Gln Gly Asn Trp Phe Gly Cys Thr Trp Met Asn
 1               5                  10                  15
GGC ACT GGG TTC ACA AAG ACG TGC GGG GGC CCC CCG TGC AAC ATC GGG     96
Gly Thr Gly Phe Thr Lys Thr Cys Gly Gly Pro Pro Cys Asn Ile Gly
             20                  25                  30
GGG GTC GGC AAC AAT ACC TTG ACT TGC CCC ACG GAC TGC TTC CGG AAG     144
Gly Val Gly Asn Asn Thr Leu Thr Cys Pro Thr Asp Cys Phe Arg Lys
         35                  40                  45
CAC CCC GAG GCC ACT TAC ACA AAA TGT GGT TCG GGG CCT TGG TTG ACG     192
His Pro Glu Ala Thr Tyr Thr Lys Cys Gly Ser Gly Pro Trp Leu Thr
     50                  55                  60
```

```
CCT AGG TGC CTA GTT CAT TAC CCA TAC AGG CTC TGG CAC TAT CCC TGC   240
Pro Arg Cys Leu Val His Tyr Pro Tyr Arg Leu Trp His Tyr Pro Cys
 65                 70                  75                  80
ACT GTC AAC TTT ACC ATC TTC AAG GTT AGG ATG TAT GTG GGG GGC GTG   288
Thr Val Asn Phe Thr Ile Phe Lys Val Arg Met Tyr Val Gly Gly Val
                85                  90                  95
GAA CAC AGG CTT GAA GCT GCA TGC AAT TGG ACC CGA GGA GAG CGT TGT   336
Glu His Arg Leu Glu Ala Ala Cys Asn Trp Thr Arg Gly Glu Arg Cys
            100                 105                 110
GAC TTG GAG GAC AGG GAT AGA TCA GAG CTT AGC CCG CTA TTG CTG TCC   384
Asp Leu Glu Asp Arg Asp Arg Ser Glu Leu Ser Pro Leu Leu Leu Ser
        115                 120                 125
ACA ACA GAG TGG CAG GTA CTG CCC TGT TCC TTC ACC ACC CTG CCG GCT   432
Thr Thr Glu Trp Gln Val Leu Pro Cys Ser Phe Thr Thr Leu Pro Ala
    130                 135                 140
CTG TCC ACT GGT TTG ATT CAT CTC CAT CAG AAC ATC GTG GAC GTG CAA   480
Leu Ser Thr Gly Leu Ile His Leu His Gln Asn Ile Val Asp Val Gln
145                 150                 155                 160
TAT CTG TAC GGC ATA GGG TCG GCG GTT GTC TCC TTC GCA ATC AAA TGG   528
Tyr Leu Tyr Gly Ile Gly Ser Ala Val Val Ser Phe Ala Ile Lys Trp
                165                 170                 175
GAA TAT ATT CTG TTG CTT TTC CTC CTC CTG GCG GAC GCG CGC GTC TGT   576
Glu Tyr Ile Leu Leu Leu Phe Leu Leu Leu Ala Asp Ala Arg Val Cys
            180                 185                 190
GCC TGC TTG TGG ATG ATG CTG CTG ATA GCC CAC GCC GAC GCC ACC TTA   624
Ala Cys Leu Trp Met Met Leu Leu Ile Ala His Ala Asp Ala Thr Leu
        195                 200                 205
GAG AA                                                            629
Glu
```

SEQ ID NO:26
SEQUENCE LENGTH: 629 base pairs
SEQUENCE TYPE: nucleic acid
STRANDEDNESS: double
TOPOLOGY: linear
ANTI-SENSE: No
ORIGINAL SOURCE

ORGANISM: Hepatitis C virus
IMMEDIATE EXPERIMENTAL SOURCE
CLONE: MX24-5

```
AAC ACA CGG CCG CCG CAG GGG AAC TGG TTT GGC TGT ACA TGG ATG AAT    48
Asn Thr Arg Pro Pro Gln Gly Asn Trp Phe Gly Cys Thr Trp Met Asn
 1               5                  10                  15
GGC ACT GGG TTC ACA AAG ACG TGC GGG GGC CCC CCG TGC AAC ATC GGG    96
Gly Thr Gly Phe Thr Lys Thr Cys Gly Gly Pro Pro Cys Asn Ile Gly
            20                  25                  30
GGG GTC GGC AAC AAT ACC TTG ACT TGC CCC ACG GAC TGC TTC CGG AAG   144
Gly Val Gly Asn Asn Thr Leu Thr Cys Pro Thr Asp Cys Phe Arg Lys
        35                  40                  45
CAC CCC GAG GCC ACT TAC ACA AAA TGT GGT TCG GGG CCT TGG TTG ACG   192
His Pro Glu Ala Thr Tyr Thr Lys Cys Gly Ser Gly Pro Trp Leu Thr
    50                  55                  60
CCT AGG TGC CTA GTT CAT TAC CCA TAC AGG CTC TGG CAC TAT CCC TGC   240
Pro Arg Cys Leu Val His Tyr Pro Tyr Arg Leu Trp His Tyr Pro Cys
 65                 70                  75                  80
ACT GTC AAC TTT ACC ATC TTC AAG GTT AGG ATG TAT GTG GGG GGC GTG   288
Thr Val Asn Phe Thr Ile Phe Lys Val Arg Met Tyr Val Gly Gly Val
                85                  90                  95
GAA CAC AGG CTT GAA GCT GCA TGC AAT TGG ACC CGA GGA GAG CGT TGT   336
Glu His Arg Leu Glu Ala Ala Cys Asn Trp Thr Arg Gly Glu Arg Cys
            100                 105                 110
GAC TTG GAG GAC AGG GAT AGA TCA GAG CTT AGC CCG CTA TTG CTG TCT   384
Asp Leu Glu Asp Arg Asp Arg Ser Glu Leu Ser Pro Leu Leu Leu Ser
        115                 120                 125
ACA ACA GAG TGG CAG GTA CTG CCC TGT TCC TTC ACC ACC CTG CCG GCT   432
Thr Thr Glu Trp Gln Val Leu Pro Cys Ser Phe Thr Thr Leu Pro Ala
    130                 135                 140
CTG TCC ACT GGT TTG ATT CAT CTC CAT CAG AAC ATC GTG GAC GTG CAA   480
Leu Ser Thr Gly Leu Ile His Leu His Gln Asn Ile Val Asp Val Gln
145                 150                 155                 160
TAT TTG TAC GGC ATA GGG TCG GCG GTT GTC TCC TTC GCA ATC AAA TGG   528
Tyr Leu Tyr Gly Ile Gly Ser Ala Val Val Ser Phe Ala Ile Lys Trp
                165                 170                 175
```

```
GAA TAT ATT CTG TTG CTT TTC CTT CTC CTG GCG GAC GCG CGC GTC TGT   576
Glu Tyr Ile Leu Leu Leu Phe Leu Leu Leu Ala Asp Ala Arg Val Cys
            180                 185                 190
GCC TGC TTG TGG ATG ATG CTG CTG ATA GCC CAC GCC GAC GCC ACC TTA   624
Ala Cys Leu Trp Met Met Leu Leu Ile Ala His Ala Asp Ala Thr Leu
        195                 200                 205
GAG AA                                                            629
Glu
```

SEQ ID NO:27
SEQUENCE LENGTH: 629 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX24-13

```
AAC ACA CGG CCG CCG CAG GGG AAC TGG TTT GGC TGT ACA TGG ATG AAT   48
Asn Thr Arg Pro Pro Gln Gly Asn Trp Phe Gly Cys Thr Trp Met Asn
  1               5                  10                  15
GGC ACT GGG TTC ACA AAG ACG TGC GGG GGC CCC CCG TGC AAC ATC GGG   96
Gly Thr Gly Phe Thr Lys Thr Cys Gly Gly Pro Pro Cys Asn Ile Gly
                 20                  25                  30
GGG GTC GGC AAC AAT ACC TTG ACT TGC CCC ACG GAC TGC TTC CGG AAG   144
Gly Val Gly Asn Asn Thr Leu Thr Cys Pro Thr Asp Cys Phe Arg Lys
             35                  40                  45
CAC CCC GAG GCC ACT TAC ACA AAA TGT GGT TCG GGG CCT TGG CTG ACG   192
His Pro Glu Ala Thr Tyr Thr Lys Cys Gly Ser Gly Pro Trp Leu Thr
     50                  55                  60
CCT AGG TGC CTA GTT CAT TAC CCA TAC AGG CTC TGG CAC TAT CCC TGC   240
Pro Arg Cys Leu Val His Tyr Pro Tyr Arg Leu Trp His Tyr Pro Cys
 65                  70                  75                  80
ACT GTC AAC TTT ACC ATC TTC AAG GTT AGG ATG TAT GTG GGG GGC GTG   288
Thr Val Asn Phe Thr Ile Phe Lys Val Arg Met Tyr Val Gly Gly Val
```

```
                    85                       90                       95
GAA CAC AGG CTT GAA GCT GCA TGC AAT TGG ACC CGC GGA GAG CGT TGT    336
Glu His Arg Leu Glu Ala Ala Cys Asn Trp Thr Arg Gly Glu Arg Cys
                100                      105                      110
GAC TTG GAG GAC AGG GAT AGA TCA GAG CTT AGC CCG CTA TTG CTG TCT    384
Asp Leu Glu Asp Arg Asp Arg Ser Glu Leu Ser Pro Leu Leu Leu Ser
            115                      120                      125
ACA ACA GAG TGG CAG GTA CTG CCC TGT TCC TTC ACC ACC CTG CCG GCT    432
Thr Thr Glu Trp Gln Val Leu Pro Cys Ser Phe Thr Thr Leu Pro Ala
        130                      135                      140
CTG TCC ACT GGT TTG ATT CAT CTC CAT CAG AAC ATC GTG GAC GTG CAA    480
Leu Ser Thr Gly Leu Ile His Leu His Gln Asn Ile Val Asp Val Gln
145                      150                      155                 160
TAT CTG TAC GGC ATA GGG TCG GCG GTT GTC TCC TTC GCA ATC AAA TGG    528
Tyr Leu Tyr Gly Ile Gly Ser Ala Val Val Ser Phe Ala Ile Lys Trp
                    165                      170                      175
GAA TAT ATT CTG TTG CTT TTC CTT CTC CTG GCG GAC GCA CGC GTC TGT    576
Glu Tyr Ile Leu Leu Leu Phe Leu Leu Leu Ala Asp Ala Arg Val Cys
                180                      185                      190
GCC TGC TTG TGG ATG ATG CTG CTG ATA GCC CAC GCC GAC GCC ACC TTA    624
Ala Cys Leu Trp Met Met Leu Leu Ile Ala His Ala Asp Ala Thr Leu
            195                      200                      205
GAG AA                                                             629
Glu
```

SEQ ID NO:28
SEQUENCE LENGTH: 652 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N27N19-1

```
C CGG ATC CCA CAA GCC GTG GTG GAT ATG GTG GCA GGG GCC CAC TGG GGA 49
```

```
 Arg Ile Pro Gln Ala Val Val Asp Met Val Ala Gly Ala His Trp Gly
   1               5                  10                  15
GTC CTG GCG GGC CTT GCC TAC TAT TCC ATG GTG GGG AAC TGG GCT AAG     97
Val Leu Ala Gly Leu Ala Tyr Tyr Ser Met Val Gly Asn Trp Ala Lys
              20                  25                  30
GTC TTG GTT GTG ATG CTG CTC TTC GCC GGT GTT GAC GGG GGG ACC CAC    145
Val Leu Val Val Met Leu Leu P(e Ala GLy VAl Asp Gly Gly THr His
          35                  40                  45
GTG ACA GGG GGG AAG GTA GCC TAC ACC ACC CAG GGC TTT ACA CCC TTC    193
Val Thr Gly Gly Lys Val Ala Tyr Thr Thr Gln Gly Phe Thr Pro Phe
     50                  55                  60
TTT TCA CGA GGG CCG TCT CAG AAA ATC CAA CTT GTA AAC ACT AAC GGC    241
Phe Ser Arg Gly Pro Ser Gln Lys Ile Gln Leu Val Asn Thr Asn Gly
 65                  70                  75                  80
AGC TGG CAC ATC AAT AGG ACT GCC CTC AAT TGC AAT GAC TCC CTT AAC    289
Ser Trp His Ile Asn Arg Thr Ala Leu Asn Cys Asn Asp Ser Leu Asn
                 85                  90                  95
ACC GGG TTC CTT GCC GCG CTG TTC TAC ACC CAC AGC TTC AAC GCG TCC    337
Thr Gly Phe Leu Ala Ala Leu Phe Tyr Thr His Ser Phe Asn Ala Ser
            100                 105                 110
GGA TGT CCG GAG CGT ATG GCC GGT TGC CGC CCC ATT GAC GAG TTC GCT    385
Gly Cys Pro Glu Arg Met Ala Gly Cys Arg Pro Ile Asp Glu Phe Ala
        115                 120                 125
CAG GGG TGG GGT CCC ATC ACT CAT GTT GTG CCT AAC ATC TCG GAC CAG    433
Gln Gly Trp Gly Pro Ile Thr His Val Val Pro Asn Ile Ser Asp Gln
    130                 135                 140
AGG CCC TAT TGC TGG CAC TAC GCG CCT CGA CCG TGT GGT ATC GTA CCC    481
Arg Pro Tyr Cys Trp His Tyr Ala Pro Arg Pro Cys Gly Ile Val Pro
145                 150                 155                 160
GCG TCG CAG GTG TGT GGT CCG GTG TAT TGC TTC ACC CCA AGC CCT GTT    529
Ala Ser Gln Val Cys Gly Pro Val Tyr Cys Phe Thr Pro Ser Pro Val
                165                 170                 175
GTG GTG GGG ACG ACC GAT CGT TTC GGC GCC CCC ACG TAC AAC TGG GGA    577
Val Val Gly Thr Thr Asp Arg Phe Gly Ala Pro Thr Tyr Asn Trp Gly
            180                 185                 190
AAC AAT GAG ACG GAT GTG CTA CTC CTC AAC AAC ACA CGG CCG CCG CAG    625
Asn Asn Glu Thr Asp Val Leu Leu Leu Asn Asn Thr Arg Pro Pro Gln
```

```
        195                 200                 205
    GGC AAC TGG TTC GGT TGT ACC TGG ATG                                 652
    Gly Asn Trp Phe Gly Cys Thr Trp Met
        210                 215
```

SEQ ID NO:29
SEQUENCE LENGTH: 977 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N19MX24A-1

```
GAG GCC GTG AAC TGC GAT GAC TCC CTT AAC ACC GGG TTC CTT GCC GCG   48
Glu Ala Val Asn Cys Asp Asp Ser Leu Asn Thr Gly Phe Leu Ala Ala
  1               5                  10                  15
CTG TTC TAC ACG CAC AGG TTC AAC GCG TCC GGA TGT CCG GAG CGT ATG   96
Leu Phe Tyr Thr His Arg Phe Asn Ala Ser Gly Cys Pro Glu Arg Met
             20                  25                  30
GCC GGT TGC CGC CCC ATT GAC GAG TTC GCT CAG GGG TGG GGT CCC ATC  144
Ala Gly Cys Arg Pro Ile Asp Glu Phe Ala Gln Gly Trp Gly Pro Ile
         35                  40                  45
ACT CAT GTT GTG CCT AAC ATC TCG GAC CAG AGG CCC TAT TGC TGG CAC  192
Thr His Val Val Pro Asn Ile Ser Asp Gln Arg Pro Tyr Cys Trp His
     50                  55                  60
TAC GCG CCT CGA CCG TGT GGT ATC GTA CCC GCG TCG CAG GTG TGT GGT  240
Tyr Ala Pro Arg Pro Cys Gly Ile Val Pro Ala Ser Gln Val Cys Gly
 65                  70                  75                  80
CCG GTG TAT TGC TTC ACC CCA AGC CCT GTT GTG GTG GGG ACG ACC GAT  288
Pro Val Tyr Cys Phe Thr Pro Ser Pro Val Val Val Gly Thr Thr Asp
                 85                  90                  95
CGT TTC GGC GCC CCC ACG TAC AAC TGG GGA AAC AAT GAG ACG GAT GTG  336
Arg Phe Gly Ala Pro Thr Tyr Asn Trp Gly Asn Asn Glu Thr Asp Val
            100                 105                 110
```

CTA CTC CTC AAC AAC ACA CGG CCG CCG CAG GGC AAC TGG TTC GGT TGT 384
Leu Leu Leu Asn Asn Thr Arg Pro Pro Gln Gly Asn Trp Phe Gly Cys
115 120 125

ACC TGG ATG AAT GGC ACT GGG TTC ACA AAG ACG TGC GGG GGC CCC CCG 432
Thr Trp Met Asn Gly Thr Gly Phe Thr Lys Thr Cys Gly Gly Pro Pro
130 135 140

TGC AAC ATC GGG GGG GTC GGC AAC AAT ACC TTG ACT TGC CCC ACG GAC 480
Cys Asn Ile Gly Gly Val Gly Asn Asn Thr Leu Thr Cys Pro Thr Asp
145 150 155 160

TGC TTC CGG AAG CAC CCC GAG GCC ACT TAC ACA AAA TGT GGT TCG GGG 528
Cys Phe Arg Lys His Pro Glu Ala Thr Tyr Thr Lys Cys Gly Ser Gly
165 170 175

CCT TGG TTG ACG CCT AGG TGC CTA GTT CAT TAC CCA TAC AGG CTC TGG 576
Pro Trp Leu Thr Pro Arg Cys Leu Val His Tyr Pro Tyr Arg Leu Trp
180 185 190

CAC TAT CCC TGC ACT GTC AAC TTT ACC ATC TTC AAG GTT AGG ATG TAT 624
His Tyr Pro Cys Thr Val Asn Phe Thr Ile Phe Lys Val Arg Met Tyr
195 200 205

GTG GGG GGC GTG GAA CAC AGG CTT GAA GCT GCA TGC AAT TGG ACC CGA 672
Val Gly Gly Val Glu His Arg Leu Glu Ala Ala Cys Asn Trp Thr Arg
210 215 220

GGA GAG CGT TGT GAC TTG GAG GAC AGG GAT AGA TCA GAG CTT AGC CCG 720
Gly Glu Arg Cys Asp Leu Glu Asp Arg Asp Arg Ser Glu Leu Ser Pro
225 230 235 240

CTA TTG CTG TCC ACA ACA GAG TGG CAG GTA CTG CCC TGT TCC TTC ACC 768
Leu Leu Leu Ser Thr Thr Glu Trp Gln Val Leu Pro Cys Ser Phe Thr
245 250 255

ACC CTG CCG GCT CTG TCC ACT GGT TTG ATT CAT CTC CAT CAG AAC ATC 816
Thr Leu Pro Ala Leu Ser Thr Gly Leu Ile His Leu His Gln Asn Ile
260 265 270

GTG GAC GTG CAA TAT CTG TAC GGC ATA GGG TCG GCG GTT GTC TCC TTC 864
Val Asp Val Gln Tyr Leu Tyr Gly Ile Gly Ser Ala Val Val Ser Phe
275 280 285

GCA ATC AAA TGG GAA TAT ATT CTG TTG CTT TTC CTC CTC CTG GCG GAC 912
Ala Ile Lys Trp Glu Tyr Ile Leu Leu Leu Phe Leu Leu Leu Ala Asp
290 295 300

GCG CGC GTC TGT GCC TGC TTG TGG ATG ATG CTG CTG ATA GCC CAC GCC 960

```
Ala Arg Val Cys Ala Cys Leu Trp Met Met Leu Leu Ile Ala His Ala
305                 310                 315                 320
GAC GCC ACC TTA GAG AA                                            977
Asp Ala Thr Leu Glu
                325
```

SEQ ID NO:30
SEQUENCE LENGTH: 977 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N19MX24B-1

```
GAG GCC GTG AAC TGC GAT GAC TCC CTT AAC ACC GGG TTC CTT GCC GCG    48
Glu Ala Val Asn Cys Asp Asp Ser Leu Asn Thr Gly Phe Leu Ala Ala
  1               5                  10                  15
CTG TTC TAC ACG CAC AGG TTC AAC GCG TCC GGA TGT CCG GAG CGT ATG    96
Leu Phe Tyr Thr His Arg Phe Asn Ala Ser Gly Cys Pro Glu Arg Met
                 20                  25                  30
GCC GGT TGC CGC CCC ATT GAC GAG TTC GCT CAG GGG TGG GGT CCC ATC   144
Ala Gly Cys Arg Pro Ile Asp Glu Phe Ala Gln Gly Trp Gly Pro Ile
             35                  40                  45
ACT CAT GTT GTG CCT AAC ATC TCG GAC CAG AGG CCC TAT TGC TGG CAC   192
Thr His Val Val Pro Asn Ile Ser Asp Gln Arg Pro Tyr Cys Trp His
         50                  55                  60
TAC GCG CCT CGA CCG TGT GGT ATC GTA CCC GCG TCG CAG GTG TGT GGT   240
Tyr Ala Pro Arg Pro Cys Gly Ile Val Pro Ala Ser Gln Val Cys Gly
 65                  70                  75                  80
CCG GTG TAT TGC TTC ACC CCA AGC CCT GTT GTG GTG GGG ACG ACC GAT   288
Pro Val Tyr Cys Phe Thr Pro Ser Pro Val Val Val Gly Thr Thr Asp
                 85                  90                  95
CGT TTC GGC GCC CCC ACG TAC AAC TGG GGA AAC AAT GAG ACG GAT GTG   336
Arg Phe Gly Ala Pro Thr Tyr Asn Trp Gly Asn Asn Glu Thr Asp Val
```

```
                100                     105                     110
CTA CTC CTC AAC AAC ACA CGG CCG CCG CAG GGG AAC TGG TTT GGC TGT      384
Leu Leu Leu Asn Asn Thr Arg Pro Pro Gln Gly Asn Trp Phe Gly Cys
        115                     120                     125
ACA TGG ATG AAT GGC ACT GGG TTC ACA AAG ACG TGC GGG GGC CCC CCG      432
Thr Trp Met Asn Gly Thr Gly Phe Thr Lys Thr Cys Gly Gly Pro Pro
    130                     135                     140
TGC AAC ATC GGG GGG GTC GGC AAC AAT ACC TTG ACT TGC CCC ACG GAC      480
Cys Asn Ile Gly Gly Val Gly Asn Asn Thr Leu Thr Cys Pro Thr Asp
145                     150                     155                 160
TGC TTC CGG AAG CAC CCC GAG GCC ACT TAC ACA AAA TGT GGT TCG GGG      528
Cys Phe Arg Lys His Pro Glu Ala Thr Tyr Thr Lys Cys Gly Ser Gly
                165                     170                     175
CCT TGG TTG ACG CCT AGG TGC CTA GTT CAT TAC CCA TAC AGG CTC TGG      576
Pro Trp Leu Thr Pro Arg Cys Leu Val His Tyr Pro Tyr Arg Leu Trp
                180                     185                     190
CAC TAT CCC TGC ACT GTC AAC TTT ACC ATC TTC AAG GTT AGG ATG TAT      624
His Tyr Pro Cys Thr Val Asn Phe Thr Ile Phe Lys Val Arg Met Tyr
        195                     200                     205
GTG GGG GGC GTG GAA CAC AGG CTT GAA GCT GCA TGC AAT TGG ACC CGA      672
Val Gly Gly Val Glu His Arg Leu Glu Ala Ala Cys Asn Trp Thr Arg
    210                     215                     220
GGA GAG CGT TGT GAC TTG GAG GAC AGG GAT AGA TCA GAG CTT AGC CCG      720
Gly Glu Arg Cys Asp Leu Glu Asp Arg Asp Arg Ser Glu Leu Ser Pro
225                     230                     235                 240
CTA TTG CTG TCC ACA ACA GAG TGG CAG GTA CTG CCC TGT TCC TTC ACC      768
Leu Leu Leu Ser Thr Thr Glu Trp Gln Val Leu Pro Cys Ser Phe Thr
                245                     250                     255
ACC CTG CCG GCT CTG TCC ACT GGT TTG ATT CAT CTC CAT CAG AAC ATC      816
Thr Leu Pro Ala Leu Ser Thr Gly Leu Ile His Leu His Gln Asn Ile
                260                     265                     270
GTG GAC GTG CAA TAT CTG TAC GGC ATA GGG TCG GCG GTT GTC TCC TTC      864
Val Asp Val Gln Tyr Leu Tyr Gly Ile Gly Ser Ala Val Val Ser Phe
        275                     280                     285
GCA ATC AAA TGG GAA TAT ATT CTG TTG CTT TTC CTC CTC CTG GCG GAC      912
Ala Ile Lys Trp Glu Tyr Ile Leu Leu Leu Phe Leu Leu Leu Ala Asp
    290                     295                     300
```

```
GCG CGC GTC TGT GCC TGC TTG TGG ATG ATG CTG CTG ATA GCC CAC GCC   960
Ala Arg Val Cys Ala Cys Leu Trp Met Met Leu Leu Ile Ala His Ala
305                 310                 315                 320
GAC GCC ACC TTA GAG AA                                           977

Asp Ala Thr Leu Glu
                325
```

SEQ ID NO:31

SEQUENCE LENGTH: 1236 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: N27MX24A-1

```
C CGG ATC CCA CAA GCC GTG GTG GAT ATG GTG GCA GGG GCC CAC TGG GGA 49
  Arg Ile Pro Gln Ala Val Val Asp Met Val Ala Gly Ala His Trp Gly
   1               5                  10                  15
GTC CTG GCG GGC CTT GCC TAC TAT TCC ATG GTG GGG AAC TGG GCT AAG   97
Val Leu Ala Gly Leu Ala Tyr Tyr Ser Met Val Gly Asn Trp Ala Lys
            20                  25                  30
GTC TTG GTT GTG ATG CTG CTC TTC GCC GGT GTT GAC GGG GGG ACC CAC   145
Val Leu Val Val Met Leu Leu Phe Ala Gly Val Asp Gly Gly Thr His
        35                  40                  45
GTG ACA GGG GGG AAG GTA GCC TAC ACC ACC CAG GGC TTT ACA CCC TTC   193
Val Thr Gly Gly Lys Val Ala Tyr Thr Thr Gln Gly Phe Thr Pro Phe
    50                  55                  60
TTT TCA CGA GGG CCG TCT CAG AAA ATC CAA CTT GTA AAC ACT AAC GGC   241
Phe Ser Arg Gly Pro Ser Gln Lys Ile Gln Leu Val Asn Thr Asn Gly
65                  70                  75                  80
AGC TGG CAC ATC AAT AGG ACT GCC CTC AAT TGC AAT GAC TCC CTT AAC   289
Ser Trp His Ile Asn Arg Thr Ala Leu Asn Cys Asn Asp Ser Leu Asn
                85                  90                  95
```

```
ACC GGG TTC CTT GCC GCG CTG TTC TAC ACC CAC AGC TTC AAC GCG TCC    337
Thr Gly Phe Leu Ala Ala Leu Phe Tyr Thr His Ser Phe Asn Ala Ser
            100                 105                 110
GGA TGT CCG GAG CGT ATG GCC GGT TGC CGC CCC ATT GAC GAG TTC GCT    385
Gly Cys Pro Glu Arg Met Ala Gly Cys Arg Pro Ile Asp Glu Phe Ala
        115                 120                 125
CAG GGG TGG GGT CCC ATC ACT CAT GTT GTG CCT AAC ATC TCG GAC CAG    433
Gln Gly Trp Gly Pro Ile Thr His Val Val Pro Asn Ile Ser Asp Gln
    130                 135                 140
AGG CCC TAT TGC TGG CAC TAC GCG CCT CGA CCG TGT GGT ATC GTA CCC    481
Arg Pro Tyr Cys Trp His Tyr Ala Pro Arg Pro Cys Gly Ile Val Pro
145                 150                 155                 160
GCG TCG CAG GTG TGT GGT CCG GTG TAT TGC TTC ACC CCA AGC CCT GTT    529
Ala Ser Gln Val Cys Gly Pro Val Tyr Cys Phe Thr Pro Ser Pro Val
                165                 170                 175
GTG GTG GGG ACG ACC GAT CGT TTC GGC GCC CCC ACG TAC AAC TGG GGA    577
Val Val Gly Thr Thr Asp Arg Phe Gly Ala Pro Thr Tyr Asn Trp Gly
            180                 185                 190
AAC AAT GAG ACG GAT GTG CTA CTC CTC AAC AAC ACA CGG CCG CCG CAG    625
Asn Asn Glu Thr Asp Val Leu Leu Leu Asn Asn Thr Arg Pro Pro Gln
        195                 200                 205
GGC AAC TGG TTC GGT TGT ACC TGG ATG AAT GGC ACT GGG TTC ACA AAG    673
Gly Asn Trp Phe Gly Cys Thr Trp Met Asn Gly Thr Gly Phe Thr Lys
    210                 215                 220
ACG TGC GGG GGC CCC CCG TGC AAC ATC GGG GGG GTC GGC AAC AAT ACC    721
Thr Cys Gly Gly Pro Pro Cys Asn Ile Gly Gly Val Gly Asn Asn Thr
225                 230                 235                 240
TTG ACT TGC CCC ACG GAC TGC TTC CGG AAG CAC CCC GAG GCC ACT TAC    769
Leu Thr Cys Pro Thr Asp Cys Phe Arg Lys His Pro Glu Ala Thr Tyr
                245                 250                 255
ACA AAA TGT GGT TCG GGG CCT TGG TTG ACG CCT AGG TGC CTA GTT CAT    817
Thr Lys Cys Gly Ser Gly Pro Trp Leu Thr Pro Arg Cys Leu Val His
            260                 265                 270
TAC CCA TAC AGG CTC TGG CAC TAT CCC TGC ACT GTC AAC TTT ACC ATC    865
Tyr Pro Tyr Arg Leu Trp His Tyr Pro Cys Thr Val Asn Phe Thr Ile
        275                 280                 285
TTC AAG GTT AGG ATG TAT GTG GGG GGC GTG GAA CAC AGG CTT GAA GCT    913
```

```
Phe Lys Val Arg Met Tyr Val Gly Gly Val Glu His Arg Leu Glu Ala
    290                 295                 300
GCA TGC AAT TGG ACC CGA GGA GAG CGT TGT GAC TTG GAG GAC AGG GAT   961
Ala Cys Asn Trp Thr Arg Gly Glu Arg Cys Asp Leu Glu Asp Arg Asp
305                 310                 315                 320
AGA TCA GAG CTT AGC CCG CTA TTG CTG TCC ACA ACA GAG TGG CAG GTA  1009
Arg Ser Glu Leu Ser Pro Leu Leu Leu Ser Thr Thr Glu Trp Gln Val
                325                 330                 335
CTG CCC TGT TCC TTC ACC ACC CTG CCG GCT CTG TCC ACT GGT TTG ATT  1057
Leu Pro Cys Ser Phe Thr Thr Leu Pro Ala Leu Ser Thr Gly Leu Ile
            340                 345                 350
CAT CTC CAT CAG AAC ATC GTG GAC GTG CAA TAT CTG TAC GGC ATA GGG  1105
His Leu His Gln Asn Ile Val Asp Val Gln Tyr Leu Tyr Gly Ile Gly
        355                 360                 365
TCG GCG GTT GTC TCC TTC GCA ATC AAA TGG GAA TAT ATT CTG TTG CTT  1153
Ser Ala Val Val Ser Phe Ala Ile Lys Trp Glu Tyr Ile Leu Leu Leu
    370                 375                 380
TTC CTC CTC CTG GCG GAC GCG CGC GTC TGT GCC TGC TTG TGG ATG ATG  1201
Phe Leu Leu Leu Ala Asp Ala Arg Val Cys Ala Cys Leu Trp Met Met
385                 390                 395                 400
CTG CTG ATA GCC CAC GCC GAC GCC ACC TTA GAG AA                   1236
Leu Leu Ile Ala His Ala Asp Ala Thr Leu Glu
                405                 410
```

SEQ ID NO:32
SEQUENCE LENGTH: 1236 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N27MX24B-1

```
C CGG ATC CCA CAA GCC GTG GTG GAT ATG GTG GCA GGG GCC CAC TGG GGA 49
  Arg Ile Pro Gln Ala Val Val Asp Met Val Ala Gly Ala His Trp Gly
```

1 5 10 15
GTC CTG GCG GGC CTT GCC TAC TAT TCC ATG GTG GGG AAC TGG GCT AAG 97
Val Leu Ala Gly Leu Ala Tyr Tyr Ser Met Val Gly Asn Trp Ala Lys
20 25 30
GTC TTG GTT GTG ATG CTG CTC TTC GCC GGT GTT GAC GGG GGG ACC CAC 145
Val Leu Val Val Met Leu Leu Phe Ala Gly Val Asp Gly Gly Thr His
35 40 45
GTG ACA GGG GGG AAG GTA GCC TAC ACC ACC CAG GGC TTT ACA CCC TTC 193
Val Thr Gly Gly Lys Val Ala Tyr Thr Thr Gln Gly Phe Thr Pro Phe
50 55 60
TTT TCA CGA GGG CCG TCT CAG AAA ATC CAA CTT GTA AAC ACT AAC GGC 241
Phe Ser Arg Gly Pro Ser Gln Lys Ile Gln Leu Val Asn Thr Asn Gly
65 70 75 80
AGC TGG CAC ATC AAT AGG ACT GCC CTC AAT TGC AAT GAC TCC CTT AAC 289
Ser Trp His Ile Asn Arg Thr Ala Leu Asn Cys Asn Asp Ser Leu Asn
85 90 95
ACC GGG TTC CTT GCC GCG CTG TTC TAC ACC CAC AGC TTC AAC GCG TCC 337
Thr Gly Phe Leu Ala Ala Leu Phe Tyr Thr His Ser Phe Asn Ala Ser
100 105 110
GGA TGT CCG GAG CGT ATG GCC GGT TGC CGC CCC ATT GAC GAG TTC GCT 385
Gly Cys Pro Glu Arg Met Ala Gly Cys Arg Pro Ile Asp Glu Phe Ala
115 120 125
CAG GGG TGG GGT CCC ATC ACT CAT GTT GTG CCT AAC ATC TCG GAC CAG 433
Gln Gly Trp Gly Pro Ile Thr His Val Val Pro Asn Ile Ser Asp Gln
130 135 140
AGG CCC TAT TGC TGG CAC TAC GCG CCT CGA CCG TGT GGT ATC GTA CCC 481
Arg Pro Tyr Cys Trp His Tyr Ala Pro Arg Pro Cys Gly Ile Val Pro
145 150 155 160
GCG TCG CAG GTG TGT GGT CCG GTG TAT TGC TTC ACC CCA AGC CCT GTT 529
Ala Ser Gln Val Cys Gly Pro Val Tyr Cys Phe Thr Pro Ser Pro Val
165 170 175
GTG GTG GGG ACG ACC GAT CGT TTC GGC GCC CCC ACG TAC AAC TGG GGA 577
Val Val Gly Thr Thr Asp Arg Phe Gly Ala Pro Thr Tyr Asn Trp Gly
180 185 190
AAC AAT GAG ACG GAT GTG CTA CTC CTC AAC AAC ACA CGG CCG CCG CAG 625
Asn Asn Glu Thr Asp Val Leu Leu Leu Asn Asn Thr Arg Pro Pro Gln
195 200 205

```
GGG AAC TGG TTT GGC TGT ACA TGG ATG AAT GGC ACT GGG TTC ACA AAG    673
Gly Asn Trp Phe Gly Cys Thr Trp Met Asn Gly Thr Gly Phe Thr Lys
    210                 215                 220
ACG TGC GGG GGC CCC CCG TGC AAC ATC GGG GGG GTC GGC AAC AAT ACC    721
Thr Cys Gly Gly Pro Pro Cys Asn Ile Gly Gly Val Gly Asn Asn Thr
225                 230                 235                 240
TTG ACT TGC CCC ACG GAC TGC TTC CGG AAG CAC CCC GAG GCC ACT TAC    769
Leu Thr Cys Pro Thr Asp Cys Phe Arg Lys His Pro Glu Ala Thr Tyr
                245                 250                 255
ACA AAA TGT GGT TCG GGG CCT TGG TTG ACG CCT AGG TGC CTA GTT CAT    817
Thr Lys Cys Gly Ser Gly Pro Trp Leu Thr Pro Arg Cys Leu Val His
            260                 265                 270
TAC CCA TAC AGG CTC TGG CAC TAT CCC TGC ACT GTC AAC TTT ACC ATC    865
Tyr Pro Tyr Arg Leu Trp His Tyr Pro Cys Thr Val Asn Phe Thr Ile
        275                 280                 285
TTC AAG GTT AGG ATG TAT GTG GGG GGC GTG GAA CAC AGG CTT GAA GCT    913
Phe Lys Val Arg Met Tyr Val Gly Gly Val Glu His Arg Leu Glu Ala
    290                 295                 300
GCA TGC AAT TGG ACC CGA GGA GAG CGT TGT GAC TTG GAG GAC AGG GAT    961
Ala Cys Asn Trp Thr Arg Gly Glu Arg Cys Asp Leu Glu Asp Arg Asp
305                 310                 315                 320
AGA TCA GAG CTT AGC CCG CTA TTG CTG TCC ACA ACA GAG TGG CAG GTA   1009
Arg Ser Glu Leu Ser Pro Leu Leu Leu Ser Thr Thr Glu Trp Gln Val
                325                 330                 335
CTG CCC TGT TCC TTC ACC ACC CTG CCG GCT CTG TCC ACT GGT TTG ATT   1057
Leu Pro Cys Ser Phe Thr Thr Leu Pro Ala Leu Ser Thr Gly Leu Ile
            340                 345                 350
CAT CTC CAT CAG AAC ATC GTG GAC GTG CAA TAT CTG TAC GGC ATA GGG   1105
His Leu His Gln Asn Ile Val Asp Val Gln Tyr Leu Tyr Gly Ile Gly
        355                 360                 365
TCG GCG GTT GTC TCC TTC GCA ATC AAA TGG GAA TAT ATT CTG TTG CTT   1153
Ser Ala Val Val Ser Phe Ala Ile Lys Trp Glu Tyr Ile Leu Leu Leu
    370                 375                 380
TTC CTC CTC CTG GCG GAC GCG CGC GTC TGT GCC TGC TTG TGG ATG ATG   1201
Phe Leu Leu Leu Ala Asp Ala Arg Val Cys Ala Cys Leu Trp Met Met
385                 390                 395                 400
CTG CTG ATA GCC CAC GCC GAC GCC ACC TTA GAG AA                    1236
```

```
Leu Leu Ile Ala His Ala Asp Ala Thr Leu Glu
                405                 410
```

SEQ ID NO:33
SEQUENCE LENGTH: 849 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX25

```
TGT GCC TGG TTG TGG ATG ATG CTG CTG ATA GCC CAA GCT GAG GCC GCC    48
Cys Ala Trp Leu Trp Met Met Leu Leu Ile Ala Gln Ala Glu Ala Ala
  1               5                  10                  15
TTG GAG AAC CTG GTG GTC CTC AAT GCA GCA TCC ATG GCS GGA GCG CAT    96
Leu Glu Asn Leu Val Val Leu Asn Ala Ala Ser Met Ala Gly Ala His
                 20                  25                  30
GGC ATC CTC TCT TTC CTT GTG TTC TTC TGT GCC GCC TGG TAC ATC AAA   144
Gly Ile Leu Ser Phe Leu Val Phe Phe Cys Ala Ala Trp Tyr Ile Lys
             35                  40                  45
GGC AGG CTG GTC CCY GGG GCG RCA TAY GCT YTC TAT GGC GTA TGG CCG   192
Gly Arg Leu Val Pro Gly Ala Xaa Tyr Ala Xab Tyr Gly Val Trp Pro
         50                  55                  60
CTG CTC CTG CTC TTG MTG GCG CTA CCS SCA CGG GCG TAC GCC ATG GAC   240
Leu Leu Leu Leu Leu Xac Ala Leu Pro Xad Arg Ala Tyr Ala Met Asp
 65                  70                  75                  80
CGG GAS ATG GCT GCA TCG TGC GGA GGC GCG GTT TTT GTA GGT CTG GTA   288
Arg Xae Met Ala Ala Ser Cys Gly Gly Ala Val Phe Val Gly Leu Val
                 85                  90                  95
CTC YTG ACC TTG TCA CCA TAC TAC AAA GTG TTC CTC GCT ARG CTC ATA   336
Leu Leu Thr Leu Ser Pro Tyr Tyr Lys Val Phe Leu Ala Xaf Leu Ile
            100                 105                 110
TGG TGG TTR CAA TAT CTC ATC ACC AGR GCC GAG GCG CAC YTG CAA GTG   384
Trp Trp Leu Gln Tyr Leu Ile Thr Arg Ala Glu Ala His Leu Gln Val
```

```
        115                 120                 125
TGG ATY CCC CCY CTY AAC GTY CGG GGR GGC CGC GAY GCC ATC ATC CTY    432
Trp Ile Pro Pro Leu Asn Val Arg Gly Gly Arg Asp Ala Ile Ile Leu
    130                 135                 140
CTC ACR TGT GCG GTC CAY CCR GAG CTR ATY TTT GAC ATC ACC AAR CTY    480
Leu Tre Cys Ala Val His Pro Glu Leu Ile Phe Asp Ile Thr Lys Leu
145                 150                 155                 160
YTG CTC GCC ATA CTC GGT CCG CTC ATG GTR CTC CAG GCT GSC MTA ACY    528
Leu Leu Ala Ile Leu Gly Pro Leu Met Val Leu Gln Ala Xag Xah Thr
                165                 170                 175
MRA RTG CCG TAC TTY GTR CGY GCT CAA GGG CTC ATY CGT RYG TGC ATG    576
Xai Xaj Pro Tyr Phe Val Arg Ala Gln Gly Leu Ile Arg Xak Cys Met
            180                 185                 190
TTR GTG CGG AAA GYC GCY GGR GGT CAT TAT GTY CAR ATG GCY YTY RTG    624
Leu Val Arg Lys Xal Ala Gly Gly His Tyr Val Gln Met Ala Xam Xan
        195                 200                 205
AAG CTG GCY GCR CTG ACA GGT ACG TAC RTT TAT GWC CAT CTT RCY CCA    672
Lys Leu Ala Ala Leu Thr Gly Thr Tyr Xao Tyr Xap His Leu Xaq Pro
    210                 215                 220
CTG CAG SAY TGG GCC CAY GCG GGC CTA CGR GAC CTT GCG GTR GCR GTW    720
Leu Gln Xar Trp Ala His Ala Gly Leu Arg Asp Leu Ala Val Ala Val
225                 230                 235                 240
GAG CCC GTT GYC TTC TCT GAY ATG GAG ACY AAG ATC ATC ACC TGG GGG    768
Glu Pro Val Xas Phe Ser Asp Met Glu Tre Lys Ile Ile Thr Trp Gly
                245                 250                 255
GCA GAC ACY GCG GCG TGT GGG GAC ATC ATT TTG GGC CTA CCW GTC TCC    816
Ala Asp Thr Ala Ala Cys Gly Asp Ile Ile Leu Gly Leu Pro Val Ser
            260                 265                 270
GCC CGG AGG GGC AAC GAG ATA CTC CTC GGA CCG                        849
Ala Arg Arg Gly Asn Glu Ile Leu Leu Gly Pro
        275                 280
```

| | | | |
|---|---|---|---|
| Y : C or T | R : A or G | M : A or C | K : G or T |
| S : G or C | W : A or T | H : A or C or T | B : G or T or C |

| | | |
|---|---|---|
| Xaa : Ala or Thr | Xab : Phe or Leu | Xac : Met or Leu |
| Xad : Ala or Pro | Xae : Glu or Asp | Xaf : Lys or Arg |
| Xag : Gly or Ala | Xah : Leu or Ile | Xai : Gln or Arg |
| Xaj : Met or Val | Xak : Met or Ala | Xal : Ala or Val |

Xam : Leu or Phe    Xan : Met or Val    Xao : Val or Ile
Xap : Asp or Val    Xaq : Thr or Ala    Xar : Asp or His
Xas : Ala or Val

SEQ ID NO:34
SEQUENCE LENGTH: 524 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O26

```
ATC ACG TGG GGG GCA GAG ACG GCG GCG TGT GGG GAC ATC ATC TCG GGT   48
Ile Thr Trp Gly Ala Glu Thr Ala Ala Cys Gly Asp Ile Ile Ser Gly
 1               5                  10                  15
CTA CCC GTT TCC GCC CGA AGG GGG ARG GAG CTG CTT TTG GGR CCG GCC   96
Leu Pro Val Ser Ala Arg Arg Gly Xaa Glu Leu Leu Leu Gly Pro Ala
                20                  25                  30
GAT AGT TTT GAC GGG CAG GGG TGG CGA CTC CTT GCG CCT ATC ACG GCC  144
Asp Ser Phe Asp Gly Gln Gly Trp Arg Leu Leu Ala Pro Ile Thr Ala
            35                  40                  45
TAC TCC CAG CAR ACG CGG GGC CTG CTT GGT TGC ATC ATC ACY AGC CTT  192
Tyr Ser Gln Gln Thr Arg Gly Leu Leu Gly Cys Ile Ile Tre Ser Leu
    50                  55                  60
ACG GGC CGG GAT AAR AAC CAG GTC GAG GGG GAG GTT CAA GTG GTC TCT  240
Thr Gly Arg Asp Lys Asn Gln Val Glu Gly Glu Val Gln Val Val Ser
65                  70                  75                  80
ACC GCA ACA CAA TCT TTC CTG GCG ACC TGY RTC AAC GGC GTK TGC TGG  288
Thr Ala Thr Gln Ser Phe Leu Ala Thr Cys Xab Asn Gly Val Cys Trp
                85                  90                  95
ACT GTT TTC CAC GGY GCC GGC TCG AAG ACC TTA GCC GGC CCA AAA GGC  336
Thr Val Phe His Gly Ala Gly Ser Lys Thr Leu Ala Gly Pro Lys Gly
            100                 105                 110
CCA ATC ACC CAA ATG TAC ACC AAT GTR GAT CAG GAC CTC GTC GGY TGG  384
```

```
Pro Ile Thr Gln Met Tyr Thr Asn Val Asp Gln Asp Leu Val Gly Trp
        115                 120                 125
TCG GCG CCC CCC SGG GCG CGT TCC TTG ACA CCW TGC ACC TGC GGC AGC    432
Ser Ala Pro Pro Xac Ala Arg Ser Leu Thr Pro Cys Thr Cys Gly Ser
    130                 135                 140
TCG GAC CTT TAT TTG GTC ACG AGR CAT GCT GAT GTC ATT CCG GTG CAC    480
Ser Asp Leu Tyr Leu Val Thr Arg His Ala Asp Val Ile Pro Val His
145                 150                 155                 160
CGG CGG GGC GAC AGC AGG GGG AGC CTC CTC TCC CCC GGG CCC AT         524
Arg Arg Gly Asp Ser Arg Gly Ser Leu Leu Ser Pro Gly Pro
                165                 170
Y : C or T      R : A or G       M : A or C        K : G or T
S : G or C      W : A or T       H : A or C or T   B : G or T or C
Xaa : Arg or Lys          Xab : Val or Ile        Xac : Gly or Arg
```

SEQ ID NO:35
SEQUENCE LENGTH: 921 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N23

```
CTG CTG TCG CCC GGG CCC ATC TCY TAC YTG AAG GGY TCC TCG GGT GGT     48
Leu Leu Ser Pro Gly Pro Ile Ser Tyr Leu Lys Gly Ser Ser Gly Gly
  1               5                  10                  15
CCG CTG CYT TGC CCC TCG GGC CRT GTT GTG GGC ATC TTC CGG GCT GCY     96
Pro Leu Xaa Cys Pro Ser Gly Xab Val Val Gly Ile Phe Arg Ala Ala
             20                  25                  30
GTG TGC ACC CGG GGG GTT GCG AAG GCG GTR GAC TTT GTG CCC GTT GAG    144
Val Cys Thr Arg Gly Val Ala Lys Ala Val Asp Phe Val Pro Val Glu
        35                  40                  45
TCT ATG GAA ACC ACY ATG CGG TCT CCG GTC TTC RCG GAT AAC TCA ACC    192
Ser Met Glu Thr Thr Met Arg Ser Pro Val Phe Xac Asp Asn Ser Thr
```

```
        50                  55                  60
CCC CCG GCC GTA CCG CAG WCA TTC CAA GTG GCC CAC CTA CAC GCT CCC     240
Pro Pro Ala Val Pro Gln Xad Phe Gln Val Ala His Leu His Ala Pro
 65                  70                  75                  80
ACT GGC AGC GGC AAA AGC ACC ARG GTG CCG GCT GCG TAT GCG GCC CAA     288
Thr Gly Ser Gly Lys Ser Thr Xae Val Pro Ala Ala Tyr Ala Ala Gln
                 85                  90                  95
GGG TAC AAG GTA CTC GTC CTG AAC CCG TCC GTT GCT GCC ACT TTG GGC     336
Gly Tyr Lys Val Leu Val Leu Asn Pro Ser Val Ala Ala Thr Leu Gly
            100                 105                 110
TTT GGG GCG TAY ATG TCC AAG GCA CAT GGT GTT GAC CCT AAC ATC AGA     384
Phe Gly Ala Tyr Met Ser Lys Ala His Gly Val Asp Pro Asn Ile Arg
        115                 120                 125
ACT GGG GTG AGG ACC ATC ACC ACG GGC GCT CCC RTC ACG TAC TCC ACC     432
Thr Gly Val Arg Thr Ile Thr Thr Gly Ala Pro Xaf Thr Tyr Ser Thr
    130                 135                 140
TAC GGT AAG TTC CTC GCC GAC GGT GGC TGT TCT GGG GGT GCC TAT GAC     480
Tyr Gly Lys Phe Leu Ala Asp Gly Gly Cys Ser Gly Gly Ala Tyr Asp
145                 150                 155                 160
ATC ATA ATA TGT GAT GAG TGT CAT TCA ACT GAC TCG ACT TCC ATC TTG     528
Ile Ile Ile Cys Asp Glu Cys His Ser Thr Asp Ser Thr Ser Ile Leu
                165                 170                 175
GGC ATT GGT ACA GTC CTG GAC CAA GCG GAG ACG GCT GGA GCG CGC CTT     576
Gly Ile Gly Thr Val Leu Asp Gln Ala Glu Thr Ala Gly Ala Arg Leu
            180                 185                 190
GTC GTG CTC GCC ACC GCT ACG CCT CCG GGA TCG GTC ACC GTG CCG CAT     624
Val Val Leu Ala Thr Ala Thr Pro Pro Gly Ser Val Thr Val Pro His
        195                 200                 205
CCT AAT ATT GAG GAG GTG GCC TTG TCC AAC ACT GGA GAG ATC CCC TTC     672
Pro Asn Ile Glu Glu Val Ala Leu Ser Asn Thr Gly Glu Ile Pro Phe
    210                 215                 220
TAT GGC AAG GCC ATC CCC CTC GAG GCC ATC AAG GGG GGG AGG CAT CTC     720
Tyr Gly Lys Ala Ile Pro Leu Glu Ala Ile Lys Gly Gly Arg His Leu
225                 230                 235                 240
AYT TTC TGC CAT TCC AAG AAG AAA TGT GAC GAG CTC GCT GCG AAG CTG     768
Xag Phe Cys His Ser Lys Lys Lys Cys Asp Glu Leu Ala Ala Lys Leu
                245                 250                 255
```

```
TCG GCC CTC GGA GTC AAY GCT GTA GCA TAY TAC CGG GGT CTT GAT GTG   816
Ser Ala Leu Gly Val Asn Ala Val Ala Tyr Tyr Arg Gly Leu Asp Val
            260                 265                 270
TCC RTC ATA CCG ACA AGC GGG GAC GTC GTT GTC GTG GCA ACW GAC GCT   864
Ser Xah Ile Pro Thr Ser Gly Asp Val Val Val Val Ala Thr Asp Ala
        275                 280                 285
CTA ATG ACG GGC TAT ACC GGT GAC TTT GAC TCR GTG ATC GAC TGY AAC   912
Leu Met Thr Gly Tyr Thr Gly Asp Phe Asp Ser Val Ile Asp Cys Asn
    290                 295                 300
ACA TGT GTC                                                       921
Thr Cys Val
305
```

Y : C or T  R : A or G  M : A or C  K : G or T
S : G or C  W : A or T  H : A or C or T  B : G or T or C

Xaa : Leu or Pro  Xab : His or Arg  Xac : Thr or Ala
Xad : Ser or Thr  Xae : Lys or Arg  Xaf : Ile or Val
Xag : Thr or Ile  Xah : Val or Ile

SEQ ID NO:36
SEQUENCE LENGTH: 623 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N16

```
GGC TAT ACC GGC GAC TTC GAC TCA GTG ATC GAC TGC AAC ACA TGT GTC   48
Gly Tyr Thr Gly Asp Phe Asp Ser Val Ile Asp Cys Asn Thr Cys Val
  1               5                  10                  15
ACC CAA ACA GTC GAT TTC AGC TTG GAC CCT ACT TTC ACC ATY GAG ACG   96
Thr Gln Thr Val Asp Phe Ser Leu Asp Pro Thr Phe Thr Ile Glu Thr
```

```
                20                       25                       30
ACG ACC GTA CCC CAA GAT GCG GTG TCG CGC TCG CAG CGG CGA GGC AGG    144
Thr Thr Val Pro Gln Asp Ala Val Ser Arg Ser Gln Arg Arg Gly Arg
            35                       40                       45
ACT GGT AGG GGC AGR GGG GGC ATA TAC AGG TTT GTA ACT CCA GGG GAA    192
Thr Gly Arg Gly Arg Gly Gly Ile Tyr Arg Phe Val Thr Pro Gly Glu
        50                       55                       60
CGG CCC TCA GGC ATG TTC GAT TCT TCG GTC CTG TGT GAA TGT TAT GAC    240
Arg Pro Ser Gly Met Phe Asp Ser Ser Val Leu Cys Glu Cys Tyr Asp
 65                      70                       75                       80
GCG GGC TGT GCT TGG TAC GAG CTC ACG YCC GCC GAG ACC TCG GTT AGG    288
Ala Gly Cys Ala Trp Tyr Glu Leu Thr Xaa Ala Glu Thr Ser Val Arg
                    85                       90                       95
TTG CGG GCT TAC CTA AAY ACA CCT GGG CTG CCC GTC TGC CAG GAC CAT    336
Leu Arg Ala Tyr Leu Asn Thr Pro Gly Leu Pro Val Cys Gln Asp His
                100                      105                      110
CTG GAG TTC TGG GAG AGC GTC TTC ACC GGC CTC ACC CAC ATA GAT GCC    384
Leu Glu Phe Trp Glu Ser Val Phe Thr Gly Leu Thr His Ile Asp Ala
            115                      120                      125
CAC TTC TTG TCC CAG ACY AAA CAG GCA GGA GAC AAC TTC CCC TAC CTG    432
His Phe Leu Ser Gln Thr Lys Gln Ala Gly Asp Asn Phe Pro Tyr Leu
        130                      135                      140
GTA GCA TAC CAG GCT ACA GTG TGC GCC AGG GCC AAG GCT CCA CCT CCA    480
Val Ala Tyr Gln Ala Thr Val Cys Ala Arg Ala Lys Ala Pro Pro Pro
145                     150                      155                      160
TCG TGG GAT CAG ATG TGG AAG TGT CTC ATA CGG CTG AAG CCT ACG CTA    528
Ser Trp Asp Gln Met Trp Lys Cys Leu Ile Arg Leu Lys Pro Thr Leu
                    165                      170                      175
CAC GGG CCA ACG CCC CTG TTG YAT AGG TTA GGA GCC GTT CAG AAC RAG    576
His Gly Pro Thr Pro Leu Leu Xab Arg Leu Gly Ala Val Gln Asn Xac
                180                      185                      190
GTT RCC CTY ACA CAC CCY ATA ACC AAG TAC ATC ATG ACA TGC ATG TC     623
Val Xad Leu Thr His Pro Ile Thr Lys Tyr Ile Met Thr Cys Met
            195                      200                      205
Y : C or T       R : A or G        M : A or C         K : G or T
S : G or C       W : A or T        H : A or C or T    B : G or T or C
Xaa : Pro or Ser           Xab : Tyr or His           Xac : Glu or Lys
```

Xad : Thr or Ala

SEQ ID NO:37
SEQUENCE LENGTH: 623 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: U16-4

```
GGC TAT ACC GGC GAC TTC GAC TCG GTG ATC GAC TGT AAT ACA TGT GTC   48
Gly Tyr Thr Gly Asp Phe Asp Ser Val Ile Asp Cys Asn Thr Cys Val
  1               5                  10                  15
ATC CAG ACA GTC GAC TTC AGC TTG GAC CCC ACC TTC ACC ATC GAG ACG   96
Ile Gln Thr Val Asp Phe Ser Leu Asp Pro Thr Phe Thr Ile Glu Thr
             20                  25                  30
ACT ACC GTG CCC CAA GAC GCG GTG TCA CGC TCG CAA CGG CGA GGC AGG  144
Thr Thr Val Pro Gln Asp Ala Val Ser Arg Ser Gln Arg Arg Gly Arg
         35                  40                  45
ACT GGC AGG GGC AGG CAA GGC ATT TAC AGG TTT GTG ACT CCA GGA GAA  192
Thr Gly Arg Gly Arg Gln Gly Ile Tyr Arg Phe Val Thr Pro Gly Glu
     50                  55                  60
CGG CCC TCG GGC ATG TTC GAT TCC TCG GTC CTG TGC GAG TGC TAT GAC  240
Arg Pro Ser Gly Met Phe Asp Ser Ser Val Leu Cys Glu Cys Tyr Asp
 65                  70                  75                  80
GCG GGC TGT GCT TGG TAC GAG CTC CCG CCC GCC GAG ACC ACG GTC AGG  288
Ala Gly Cys Ala Trp Tyr Glu Leu Pro Pro Ala Glu Thr Thr Val Arg
                 85                  90                  95
TTG CGG GCT TAC CTG AAC ACC CCA GGG CTG CCC GTC TGC CAG GAC CAT  336
Leu Arg Ala Tyr Leu Asn Thr Pro Gly Leu Pro Val Cys Gln Asp His
            100                 105                 110
CTG GAG TTC TGG GAG AGC GTC TTC ACA GGC CTC ACC CAC ATA GAT GCC  384
Leu Glu Phe Trp Glu Ser Val Phe Thr Gly Leu Thr His Ile Asp Ala
        115                 120                 125
```

```
CAC TTC TTG TCC CAG ACC AAG CAA GCA GGA GAC AAT CTC CCT TAC CTG   432
His Phe Leu Ser Gln Thr Lys Gln Ala Gly Asp Asn Leu Pro Tyr Leu
    130                 135                 140
GTA GCG TAC CAA GCA ACA GTG TGC GCT AGA GCT CAG GCT CCA CCT CCA   480
Val Ala Tyr Gln Ala Thr Val Cys Ala Arg Ala Gln Ala Pro Pro Pro
145                 150                 155                 160
TCA TGG GAT CAA ATG TGG AAG TGT CTC ATA CGG CTA AAA CCT ACA CTA   528
Ser Trp Asp Gln Met Trp Lys Cys Leu Ile Arg Leu Lys Pro Thr Leu
                165                 170                 175
CGC GGG CCA ACG CCC CTG CTG TAT AGG CTG GGA GCC GTC CAA AAT GAG   576
Arg Gly Pro Thr Pro Leu Leu Tyr Arg Leu Gly Ala Val Gln Asn Glu
            180                 185                 190
GTC AAC CTC ACG CAC CCC GTA ACC AAA TAC ATC ATG ACA TGC ATG TC    623
Val Asn Leu Thr His Pro Val Thr Lys Tyr Ile Met Thr Cys Met
        195                 200                 205
```

SEQ ID NO:38
SEQUENCE LENGTH: 618 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N13-1

```
GCGGATCC GGC CTC ACC CAC ATA GAT GCC CAC TTC CTG TCC CAG ACC AAA   50
         Gly Leu Thr His Ile Asp Ala His Phe Leu Ser Gln Thr Lys
           1               5                  10
CAG GCA GGA GAC AAC TTC CCC TAC CTG GTA GCA TAC CAG GCT ACA GTG   98
Gln Ala Gly Asp Asn Phe Pro Tyr Leu Val Ala Tyr Gln Ala Thr Val
 15                 20                  25                  30
TGC GCC AGG GCC AAG GCT CCA CCT CCA TCG TGG GAT CAG ATG TGG AAG   146
Cys Ala Arg Ala Lys Ala Pro Pro Pro Ser Trp Asp Gln Met Trp Lys
                35                  40                  45
TGT CTC ATA CGG CTG AAG CCT ACG CTA CAC GGG CCA ACG CCC CTG TTG   194
```

```
Cys Leu Ile Arg Leu Lys Pro Thr Leu His Gly Pro Thr Pro Leu Leu
                50                  55                  60
TAT AGG TTA GGA GCC GTT CAG AAC GAG GTT ACC CTC ACA CAC CCC ATA     242
Tyr Arg Leu Gly Ala Val Gln Asn Glu Val Thr Leu Thr His Pro Ile
            65                  70                  75
ACC AAG TTC ATC ATG GCA TGC ATG TCG GCT GAC CTA GAG GTC GTC ACT     290
Thr Lys Phe Ile Met Ala Cys Met Ser Ala Asp Leu Glu Val Val Thr
        80                  85                  90
AGC ACT TGG GTG CTG GTA GGC GGG GTC CTC GCG GCT CTG GCC GCG TAC     338
Ser Thr Trp Val Leu Val Gly Gly Val Leu Ala Ala Leu Ala Ala Tyr
 95                 100                 105                 110
TGC CTG ACA ACG GGC AGC GTG GTC ATT GTG GGC AGG ATC GTC TTG TCC     386
Cys Leu Thr Thr Gly Ser Val Val Ile Val Gly Arg Ile Val Leu Ser
                115                 120                 125
GGG AGG CCG GTT GTT ATT CCC GAC AGG GAA GTT CTC TAC CAA GAG TTC     434
Gly Arg Pro Val Val Ile Pro Asp Arg Glu Val Leu Tyr Gln Glu Phe
            130                 135                 140
GAT GAA ATG GAA GAG TGC GCC TCG CAC CTC CCT TAC ATC GAA CAA GGA     482
Asp Glu Met Glu Glu Cys Ala Ser His Leu Pro Tyr Ile Glu Gln Gly
        145                 150                 155
ATG CAG CTC GCC GAG CAA TTC AAG CAG AAG GCG CTC GGT TTG CTG CAA     530
Met Gln Leu Ala Glu Gln Phe Lys Gln Lys Ala Leu Gly Leu Leu Gln
    160                 165                 170
ACA GCC ACC AAG CAA GCG GAG GCT GCT GCT CCC GTG GTG GAG TCC AAG     578
Thr Ala Thr Lys Gln Ala Glu Ala Ala Ala Pro Val Val Glu Ser Lys
175                 180                 185                 190
TGG CGA GCC CTT GAG ACC TTC TGG GCG AAG CA GGATCCGC                 618
Trp Arg Ala Leu Glu Thr Phe Trp Ala Lys
                195                 200
```

SEQ ID NO:39
SEQUENCE LENGTH: 969 base pairs
SEQUENCE TYPE: nucleic acid
STRANDEDNESS: double
TOPOLOGY: linear
ANTI-SENSE: No
ORIGINAL SOURCE

ORGANISM: Hepatitis C virus
IMMEDIATE EXPERIMENTAL SOURCE
CLONE: N15-1

GCGGATCCT CCA CCT CCA TCG TGG GAT CAA ATG TGG AAG TGT CTC ATA CGG 51
Pro Pro Pro Ser Trp Asp Gln Met Trp Lys Cys Leu Ile Arg
1 5 10
CTG AAG CCT ACG CTA CAC GGG CCA ACG CCC CTG TTG TAT AGG TTA GGA 99
Leu Lys Pro Thr Leu His Gly Pro Thr Pro Leu Leu Tyr Arg Leu Gly
15 20 25 30
GCC GTT CAG AAC GAG GTT ACC CTC ACA CAC CCC ATA ACC AAG TTC ATC 147
Ala Val Gln Asn Glu Val Thr Leu Thr His Pro Ile Thr Lys Phe Ile
35 40 45
ATG GCA TGC ATG TCG GCT GAC CTA GAG GTC GTC ACT AGC ACT TGG GTG 195
Met Ala Cys Met Ser Ala Asp Leu Glu Val Val Thr Ser Thr Trp Val
50 55 60
CTG GTA GGC GGG GTC CTC GCG GCT CTG GCC GCG TAC TGC CTG ACA ACG 243
Leu Val Gly Gly Val Leu Ala Ala Leu Ala Ala Tyr Cys Leu Thr Thr
65 70 75
GGC AGC GTG GTC ATT GTG GGC AGG ATC ATC TTG TCC GGG AGG CCG GCC 291
Gly Ser Val Val Ile Val Gly Arg Ile Ile Leu Ser Gly Arg Pro Ala
80 85 90
GTT ATT CCC GAC AGG GAA GTT CTC TAC CAA GAG TTC GAT GAA ATG GAA 339
Val Ile Pro Asp Arg Glu Val Leu Tyr Gln Glu Phe Asp Glu Met Glu
95 100 105 110
GAG TGC GCC TCG CAC CTC CCT TAC ATC GAA CAA GGA ATG CAG CTC GCC 387
Glu Cys Ala Ser His Leu Pro Tyr Ile Glu Gln Gly Met Gln Leu Ala
115 120 125
GAG CAA TTC AAG CAG AAG GCG CTC GGT TTG CTG CAA ACA GCC ACC AAG 435
Glu Gln Phe Lys Gln Lys Ala Leu Gly Leu Leu Gln Thr Ala Thr Lys
130 135 140
CAA GCG GAG GCT GCT GCT CCC GTG GTG GAG TCC AAG TGG CGA GCC CTT 483
Gln Ala Glu Ala Ala Ala Pro Val Val Glu Ser Lys Trp Arg Ala Leu
145 150 155
GAG ACC TTC TGG GCG AAG CAC ATG TGG AAT TTC ATC AGC GGG ATA CAG 531
Glu Thr Phe Trp Ala Lys His Met Trp Asn Phe Ile Ser Gly Ile Gln
160 165 170

```
TAC TTA GCA GGC TTG TCC ACT CTG CCT GGA AAC CCC GCA ATA GCA TCA    579
Tyr Leu Ala Gly Leu Ser Thr Leu Pro Gly Asn Pro Ala Ile Ala Ser
175                 180                 185                 190
CTG ATG GCA TTC ACA GCC TCT ATC ACC AGC CCG CTC ACC ACC CAA TAT    627
Leu Met Ala Phe Thr Ala Ser Ile Thr Ser Pro Leu Thr Thr Gln Tyr
                195                 200                 205
ACC CTC CTG TTT AAC ATC TTG GGG GGA TGG GTG GCC GCC CAA CTC GCC    675
Thr Leu Leu Phe Asn Ile Leu Gly Gly Trp Val Ala Ala Gln Leu Ala
            210                 215                 220
CCC CCC AGT GCC GCT TCA GCC TTC GTG GGC GCC GGT ATA GCT GGC GCG    723
Pro Pro Ser Ala Ala Ser Ala Phe Val Gly Ala Gly Ile Ala Gly Ala
        225                 230                 235
GCT GTT GGC AGC ATA GGC CTC GGG AAG GTG CTT GTG GAC ATT CTG GCG    771
Ala Val Gly Ser Ile Gly Leu Gly Lys Val Leu Val Asp Ile Leu Ala
    240                 245                 250
GGT TAT GGA GCA GGG GTG GCA GGC GCG CTC GTG GCC TTT AAG GTC ATG    819
Gly Tyr Gly Ala Gly Val Ala Gly Ala Leu Val Ala Phe Lys Val Met
255                 260                 265                 270
AGC GGT GAC ATG CCC TCC ACC GAG GAC CTG GTC AAC TTA CTC CCC GCC    867
Ser Gly Asp Met Pro Ser Thr Glu Asp Leu Val Asn Leu Leu Pro Ala
                275                 280                 285
ATC CTC TCT CCT GGT GCC CTG GTC GTC GGG GTC GTG TGC GCA GCA ATA    915
Ile Leu Ser Pro Gly Ala Leu Val Val Gly Val Val Cys Ala Ala Ile
            290                 295                 300
CTG CGT CGG CAT GTG GGC CCA GGG GAG GGG GCT GTG CAG TGG ATG AAC    963
Leu Arg Arg His Val Gly Pro Gly Glu Gly Ala Val Gln Trp Met Asn
        305                 310                 315
CGG CTG C AGCC                                                     974
Arg Leu
    320
```

SEQ ID NO:40
SEQUENCE LENGTH: 1280 base pairs
SEQUENCE TYPE: nucleic acid
STRANDEDNESS: double
TOPOLOGY: linear
ANTI-SENSE: No

ORIGINAL SOURCE
ORGANISM: Hepatitis C virus
IMMEDIATE EXPERIMENTAL SOURCE
CLONE: MX25026

```
TGT GCC TGG TTG TGG ATG ATG CTG CTG ATA GCC CAA GCT GAG GCC GCC    48
Cys Ala Trp Leu Trp Met Met Leu Leu Ile Ala Gln Ala Glu Ala Ala
 1               5                  10                  15
TTG GAG AAC CTG GTG GTC CTC AAT GCA GCA TCC ATG GCS GGA GCG CAT    96
Leu Glu Asn Leu Val Val Leu Asn Ala Ala Ser Met Ala Gly Ala His
            20                  25                  30
GGC ATC CTC TCT TTC CTT GTG TTC TTC TGT GCC GCC TGG TAC ATC AAA   144
Gly Ile Leu Ser Phe Leu Val Phe Phe Cys Ala Ala Trp Tyr Ile Lys
        35                  40                  45
GGC AGG CTG GTC CCY GGG GCG RCA TAY GCT YTC TAT GGC GTA TGG CCG   192
Gly Arg Leu Val Pro Gly Ala Xaa Tyr Ala Xab Tyr Gly Val Trp Pro
    50                  55                  60
CTG CTC CTG CTC TTG MTG GCG CTA CCS SCA CGG GCG TAC GCC ATG GAC   240
Leu Leu Leu Leu Leu Xac Ala Leu Pro Xad Arg Ala Tyr Ala Met Asp
65                  70                  75                  80
CGG GAS ATG GCT GCA TCG TGC GGA GGC GCG GTT TTT GTA GGT CTG GTA   288
Arg Xae Met Ala Ala Ser Cys Gly Gly Ala Val Phe Val Gly Leu Val
                85                  90                  95
CTC YTG ACC TTG TCA CCA TAC TAC AAA GTG TTC CTC GCT ARG CTC ATA   336
Leu Leu Thr Leu Ser Pro Tyr Tyr Lys Val Phe Leu Ala Xaf Leu Ile
            100                 105                 110
TGG TGG TTR CAA TAT CTC ATC ACC AGR GCC GAG GCG CAC YTG CAA GTG   384
Trp Trp Leu Gln Tyr Leu Ile Thr Arg Ala Glu Ala His Leu Gln Val
        115                 120                 125
TGG ATY CCC CCY CTY AAC GTY CGG GGR GGC CGC GAY GCC ATC ATC CTY   432
Trp Ile Pro Pro Leu Asn Val Arg Gly Gly Arg Asp Ala Ile Ile Leu
    130                 135                 140
CTC ACR TGT GCG GTC CAY CCR GAG CTR ATY TTT GAC ATC ACC AAR CTY   480
Leu Thr Cys Ala Val His Pro Glu Leu Ile Phe Asp Ile Thr Lys Leu
145                 150                 155                 160
YTG CTC GCC ATA CTC GGT CCG CTC ATG GTR CTC CAG GCT GSC MTA ACY   528
Leu Leu Ala Ile Leu Gly Pro Leu Met Val Leu Gln Ala Xag Xah Thr
```

```
                    165                     170                     175
MRA RTG CCG TAC TTY GTR CGY GCT CAA GGG CTC ATY CGT RYG TGC ATG    576
Xai Xaj Pro Tyr Phe Val Arg Ala Gln Gly Leu Ile Arg Xak Cys Met
            180                     185                     190
TTR GTG CGG AAA GYC GCY GGR GGT CAT TAT GTY CAR ATG GCY YTY RTG    624
Leu Val Arg Lys Xal Ala Gly Gly His Tyr Val Gln Met Ala Xam Xan
        195                     200                     205
AAG CTG GCY GCR CTG ACA GGT ACG TAC RTT TAT GWC CAT CTT RCY CCA    672
Lys Leu Ala Ala Leu Thr Gly Thr Tyr Xao Tyr Xap His Leu Xaq Pro
    210                     215                     220
CTG CAG SAY TGG GCC CAY GCG GGC CTA CGR GAC CTT GCG GTR GCR GTW    720
Leu Gln Xar Trp Ala His Ala Gly Leu Arg Asp Leu Ala Val Ala Val
225                     230                     235                     240
GAG CCC GTT GYC TTC TCT GAY ATG GAG ACY AAG ATC ATC ACS TGG GGG    768
Glu Pro Val Xas Phe Ser Asp Met Glu Thr Lys Ile Ile Thr Trp Gly
                    245                     250                     255
GCA GAS ACB GCG GCG TGT GGG GAC ATC ATY TYG GGY CTA CCH GTY TCC    816
Ala Xat Thr Ala Ala Cys Gly Asp Ile Ile Xau Gly Leu Pro Val Ser
            260                     265                     270
GCC CGR AGG GGY ARS GAG MTR CTY YTS GGR CCG GCC GAT AGT TTT GAC    864
Ala Arg Arg Gly Xav Glu Xaw Leu Xax Gly Pro Ala Asp Ser Phe Asp
        275                     280                     285
GGG CAG GGG TGG CGA CTC CTT GCG CCT ATC ACG GCC TAC TCC CAG CAR    912
Gly Gln Gly Trp Arg Leu Leu Ala Pro Ile Thr Ala Tyr Ser Gln Gln
    290                     295                     300
ACG CGG GGC CTG CTT GGT TGC ATC ATC ACY AGC CTT ACG GGC CGG GAT    960
Thr Arg Gly Leu Leu Gly Cys Ile Ile Thr Ser Leu Thr Gly Arg Asp
305                     310                     315                     320
AAR AAC CAG GTC GAG GGG GAG GTT CAA GTG GTC TCT ACC GCA ACA CAA   1008
Lys Asn Gln Val Glu Gly Glu Val Gln Val Val Ser Thr Ala Thr Gln
                    325                     330                     335
TCT TTC CTG GCG ACC TGY RTC AAC GGC GTK TGC TGG ACT GTT TTC CAC   1056
Ser Phe Leu Ala Thr Cys Xay Asn Gly Val Cys Trp Thr Val Phe His
            340                     345                     350
GGY GCC GGC TCG AAG ACC TTA GCC GGC CCA AAA GGC CCA ATC ACC CAA   1104
Gly Ala Gly Ser Lys Thr Leu Ala Gly Pro Lys Gly Pro Ile Thr Gln
        355                     360                     365
```

```
ATG TAC ACC AAT GTR GAT CAG GAC CTC GTC GGY TGG TCG GCG CCC CCC  1152
Met Tyr Thr Asn Val Asp Gln Asp Leu Val Gly Trp Ser Ala Pro Pro
    370                 375                 380
SGG GCG CGT TCC TTG ACA CCW TGC ACC TGC GGC AGC TCG GAC CTT TAT  1200
Xaz Ala Arg Ser Leu Thr Pro Cys Thr Cys Gly Ser Ser Asp Leu Tyr
385                 390                 395                 400
TTG GTC ACG AGR CAT GCT GAT GTC ATT CCG GTG CAC CGG CGG GGC GAC  1248
Leu Val Thr Arg His Ala Asp Val Ile Pro Val His Arg Arg Gly Asp
                405                 410                 415
AGC AGG GGG AGC CTC CTC TCC CCC GGG CCC AT                       1280
Ser Arg Gly Ser Leu Leu Ser Pro Gly Pro
            420                 425
```

```
Y : C or T       R : A or G        M : A or C          K : G or T
S : G or C       W : A or T        H : A or C or T     B : G or T or C
Xaa : Ala or Thr          Xab : Phe or Leu          Xac : Met or Leu
Xad : Ala or Pro          Xae : Glu or Asp          Xaf : Lys or Arg
Xag : Gly or Ala          Xah : Leu or Ile          Xai : Gln or Arg
Xaj : Met or Val          Xak : Met or Ala          Xal : Ala or Val
Xam : Leu or Phe          Xan : Met or Val          Xao : Val or Ile
Xap : Asp or Val          Xaq : Thr or Ala          Xar : Asp or His
Xas : Ala or Val          Xat : Asp or Glu          Xau : Leu or Ser
Xav : Asn or Arg or Lys   Xaw : Ile or Leu          Xax : Leu or Phe
Xay : Ile or Val          Xaz : Gly or Arg
```

SEQ ID NO:41
SEQUENCE LENGTH: 1431 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N16N15

```
GGC TAT ACC GGC GAC TTC GAC TCA GTG ATC GAC TGC AAC ACA TGT GTC   48
Gly Tyr Thr Gly Asp Phe Asp Ser Val Ile Asp Cys Asn Thr Cys Val
```

```
1               5                   10                  15
ACC CAA ACA GTC GAT TTC AGC TTG GAC CCT ACT TTC ACC ATY GAG ACG    96
Thr Gln Thr Val Asp Phe Ser Leu Asp Pro Thr Phe Thr Ile Glu Thr
            20                  25                  30
ACG ACC GTA CCC CAA GAT GCG GTG TCG CGC TCG CAG CGG CGA GGC AGG   144
Thr Thr Val Pro Gln Asp Ala Val Ser Arg Ser Gln Arg Arg Gly Arg
        35                  40                  45
ACT GGT AGG GGC AGR GGG GGC ATA TAC AGG TTT GTA ACT CCA GGG GAA   192
Thr Gly Arg Gly Arg Gly Gly Ile Tyr Arg Phe Val Thr Pro Gly Glu
    50                  55                  60
CGG CCC TCA GGC ATG TTC GAT TCT TCG GTC CTG TGT GAA TGT TAT GAC   240
Arg Pro Ser Gly Met Phe Asp Ser Ser Val Leu Cys Glu Cys Tyr Asp
65                  70                  75                  80
GCG GGC TGT GCT TGG TAC GAG CTC ACG YCC GCC GAG ACC TCG GTT AGG   288
Ala Gly Cys Ala Trp Tyr Glu Leu Thr Xaa Ala Glu Thr Ser Val Arg
                85                  90                  95
TTG CGG GCT TAC CTA AAY ACA CCT GGG CTG CCC GTC TGC CAG GAC CAT   336
Leu Arg Ala Tyr Leu Asn Thr Pro Gly Leu Pro Val Cys Gln Asp His
            100                 105                 110
CTG GAG TTC TGG GAG AGC GTC TTC ACC GGC CTC ACC CAC ATA GAT GCC   384
Leu Glu Phe Trp Glu Ser Val Phe Thr Gly Leu Thr His Ile Asp Ala
        115                 120                 125
CAC TTC TTG TCC CAG ACY AAA CAG GCA GGA GAC AAC TTC CCC TAC CTG   432
His Phe Leu Ser Gln Thr Lys Gln Ala Gly Asp Asn Phe Pro Tyr Leu
    130                 135                 140
GTA GCA TAC CAG GCT ACA GTG TGC GCC AGG GCC AAG GCT CCA CCT CCA   480
Val Ala Tyr Gln Ala Thr Val Cys Ala Arg Ala Lys Ala Pro Pro Pro
145                 150                 155                 160
TCG TGG GAT CAR ATG TGG AAG TGT CTC ATA CGG CTG AAG CCT ACG CTA   528
Ser Trp Asp Gln Met Trp Lys Cys Leu Ile Arg Leu Lys Pro Thr Leu
                165                 170                 175
CAC GGG CCA ACG CCC CTG TTG YAT AGG TTA GGA GCC GTT CAG AAC RAG   576
His Gly Pro Thr Pro Leu Leu Xab Arg Leu Gly Ala Val Gln Asn Xac
            180                 185                 190
GTT RCC CTY ACA CAC CCY ATA ACC AAG TWC ATC ATG RCA TGC ATG TCG   624
Val Xad Leu Thr His Pro Ile Thr Lys Xae Ile Met Xaf Cys Met Ser
        195                 200                 205
```

```
GCT GAC CTA GAG GTC GTC ACT AGC ACT TGG GTG CTG GTA GGC GGG GTC    672
Ala Asp Leu Glu Val Val Thr Ser Thr Trp Val Leu Val Gly Gly Val
    210                 215                 220
CTC GCG GCT CTG GCC GCG TAC TGC CTG ACA ACG GGC AGC GTG GTC ATT    720
Leu Ala Ala Leu Ala Ala Tyr Cys Leu Thr Thr Gly Ser Val Val Ile
225                 230                 235                 240
GTG GGC AGG ATC ATC TTG TCC GGG AGG CCG GCC GTT ATT CCC GAC AGG    768
Val Gly Arg Ile Ile Leu Ser Gly Arg Pro Ala Val Ile Pro Asp Arg
                245                 250                 255
GAA GTT CTC TAC CAA GAG TTC GAT GAA ATG GAA GAG TGC GCC TCG CAC    816
Glu Val Leu Tyr Gln Glu Phe Asp Glu Met Glu Glu Cys Ala Ser His
            260                 265                 270
CTC CCT TAC ATC GAA CAA GGA ATG CAG CTC GCC GAG CAA TTC AAG CAG    864
Leu Pro Tyr Ile Glu Gln Gly Met Gln Leu Ala Glu Gln Phe Lys Gln
        275                 280                 285
AAG GCG CTC GGT TTG CTG CAA ACA GCC ACC AAG CAA GCG GAG GCT GCT    912
Lys Ala Leu Gly Leu Leu Gln Thr Ala Thr Lys Gln Ala Glu Ala Ala
    290                 295                 300
GCT CCC GTG GTG GAG TCC AAG TGG CGA GCC CTT GAG ACC TTC TGG GCG    960
Ala Pro Val Val Glu Ser Lys Trp Arg Ala Leu Glu Thr Phe Trp Ala
305                 310                 315                 320
AAG CAC ATG TGG AAT TTC ATC AGC GGG ATA CAG TAC TTA GCA GGC TTG   1008
Lys His Met Trp Asn Phe Ile Ser Gly Ile Gln Tyr Leu Ala Gly Leu
                325                 330                 335
TCC ACT CTG CCT GGA AAC CCC GCA ATA GCA TCA CTG ATG GCA TTC ACA   1056
Ser Thr Leu Pro Gly Asn Pro Ala Ile Ala Ser Leu Met Ala Phe Thr
            340                 345                 350
GCC TCT ATC ACC AGC CCG CTC ACC ACC CAA TAT ACC CTC CTG TTT AAC   1104
Ala Ser Ile Thr Ser Pro Leu Thr Thr Gln Tyr Thr Leu Leu Phe Asn
        355                 360                 365
ATC TTG GGG GGA TGG GTG GCC GCC CAA CTC GCC CCC CCC AGT GCC GCT   1152
Ile Leu Gly Gly Trp Val Ala Ala Gln Leu Ala Pro Pro Ser Ala Ala
    370                 375                 380
TCA GCC TTC GTG GGC GCC GGT ATA GCT GGC GCG GCT GTT GGC AGC ATA   1200
Ser Ala Phe Val Gly Ala Gly Ile Ala Gly Ala Ala Val Gly Ser Ile
385                 390                 395                 400
GGC CTC GGG AAG GTG CTT GTG GAC ATT CTG GCG GGT TAT GGA GCA GGG   1248
```

```
Gly Leu Gly Lys Val Leu Val Asp Ile Leu Ala Gly Tyr Gly Ala Gly
                405                     410                     415
GTG GCA GGC GCG CTC GTG GCC TTT AAG GTC ATG AGC GGT GAC ATG CCC   1296
Val Ala Gly Ala Leu Val Ala Phe Lys Val Met Ser Gly Asp Met Pro
            420                     425                     430
TCC ACC GAG GAC CTG GTC AAC TTA CTC CCC GCC ATC CTC TCT CCT GGT   1344
Ser Thr Glu Asp Leu Val Asn Leu Leu Pro Ala Ile Leu Ser Pro Gly
        435                     440                     445
GCC CTG GTC GTC GGG GTC GTG TGC GCA GCA ATA CTG CGT CGG CAT GTG   1392
Ala Leu Val Val Gly Val Val Cys Ala Ala Ile Leu Arg Arg His Val
    450                     455                     460
GGC CCA GGG GAG GGG GCT GTG CAG TGG ATG AAC CGG CTG               1431
Gly Pro Gly Glu Gly Ala Val Gln Trp Met Asn Arg Leu
465                     470                     475
Y : C or T      R : A or G        M : A or C          K : G or T
S : G or C      W : A or T        H : A or C or T     B : G or T or C
Xaa : Pro or Ser          Xab : Tyr or His          Xac : Glu or Lys
Xad : Thr or Ala          Xae : Tyr or Phe          Xaf : Thr or Ala
```

SEQ ID NO:42
SEQUENCE LENGTH: 2304 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N23N15

```
CTG CTG TCG CCC GGG CCC ATC TCY TAC YTG AAG GGY TCC TCG GGT GGT    48
Leu Leu Ser Pro Gly Pro Ile Ser Tyr Leu Lys Gly Ser Ser Gly Gly
 1               5                  10                  15
CCG CTG CYT TGC CCC TCG GGC CRT GTT GTG GGC ATC TTC CGG GCT GCY    96
Pro Leu Xaa Cys Pro Ser Gly Xab Val Val Gly Ile Phe Arg Ala Ala
            20                  25                  30
GTG TGC ACC CGG GGG GTT GCG AAG GCG GTR GAC TTT GTG CCC GTT GAG   144
```

```
Val Cys Thr Arg Gly Val Ala Lys Ala Val Asp Phe Val Pro Val Glu
        35                  40                  45
TCT ATG GAA ACC ACY ATG CGG TCT CCG GTC TTC RCG GAT AAC TCA ACC    192
Ser Met Glu Thr Thr Met Arg Ser Pro Val Phe Xac Asp Asn Ser Thr
    50                  55                  60
CCC CCG GCC GTA CCG CAG WCA TTC CAA GTG GCC CAC CTA CAC GCT CCC    240
Pro Pro Ala Val Pro Gln Xad Phe Gln Val Ala His Leu His Ala Pro
65                  70                  75                  80
ACT GGC AGC GGC AAA AGC ACC ARG GTG CCG GCT GCG TAT GCG GCC CAA    288
Thr Gly Ser Gly Lys Ser Thr Xae Val Pro Ala Ala Tyr Ala Ala Gln
                85                  90                  95
GGG TAC AAG GTA CTC GTC CTG AAC CCG TCC GTT GCT GCC ACT TTG GGC    336
Gly Tyr Lys Val Leu Val Leu Asn Pro Ser Val Ala Ala Thr Leu Gly
            100                 105                 110
TTT GGG GCG TAY ATG TCC AAG GCA CAT GGT GTT GAC CCT AAC ATC AGA    384
Phe Gly Ala Tyr Met Ser Lys Ala His Gly Val Asp Pro Asn Ile Arg
        115                 120                 125
ACT GGG GTG AGG ACC ATC ACC ACG GGC GCT CCC RTC ACG TAC TCC ACC    432
Thr Gly Val Arg Thr Ile Thr Thr Gly Ala Pro Xaf Thr Tyr Ser Thr
    130                 135                 140
TAC GGT AAG TTC CTC GCC GAC GGT GGC TGT TCT GGG GGT GCC TAT GAC    480
Tyr Gly Lys Phe Leu Ala Asp Gly Gly Cys Ser Gly Gly Ala Tyr Asp
145                 150                 155                 160
ATC ATA ATA TGT GAT GAG TGT CAT TCA ACT GAC TCG ACT TCC ATC TTG    528
Ile Ile Ile Cys Asp Glu Cys His Ser Thr Asp Ser Thr Ser Ile Leu
                165                 170                 175
GGC ATT GGT ACA GTC CTG GAC CAA GCG GAG ACG GCT GGA GCG CGC CTT    576
Gly Ile Gly Thr Val Leu Asp Gln Ala Glu Thr Ala Gly Ala Arg Leu
            180                 185                 190
GTC GTG CTC GCC ACC GCT ACG CCT CCG GGA TCG GTC ACC GTG CCG CAT    624
Val Val Leu Ala Thr Ala Thr Pro Pro Gly Ser Val Thr Val Pro His
        195                 200                 205
CCT AAT ATT GAG GAG GTG GCC TTG TCC AAC ACT GGA GAG ATC CCC TTC    672
Pro Asn Ile Glu Glu Val Ala Leu Ser Asn Thr Gly Glu Ile Pro Phe
    210                 215                 220
TAT GGC AAG GCC ATC CCC CTC GAG GCC ATC AAG GGG GGG AGG CAT CTC    720
Tyr Gly Lys Ala Ile Pro Leu Glu Ala Ile Lys Gly Gly Arg His Leu
```

```
225                 230                 235                 240
AYT TTC TGC CAT TCC AAG AAG AAA TGT GAC GAG CTC GCT GCG AAG CTG    768
Xag Phe Cys His Ser Lys Lys Lys Cys Asp Glu Leu Ala Ala Lys Leu
                245                 250                 255
TCG GCC CTC GGA GTC AAY GCT GTA GCA TAY TAC CGG GGT CTT GAT GTG    816
Ser Ala Leu Gly Val Asn Ala Val Ala Tyr Tyr Arg Gly Leu Asp Val
            260                 265                 270
TCC RTC ATA CCG ACA AGC GGG GAC GTC GTT GTC GTG GCA ACW GAC GCT    864
Ser Xah Ile Pro Thr Ser Gly Asp Val Val Val Val Ala Thr Asp Ala
        275                 280                 285
CTA ATG ACG GGC TAT ACC GGY GAC TTY GAC TCR GTG ATC GAC TGY AAC    912
Leu Met Thr Gly Tyr Thr Gly Asp Phe Asp Ser Val Ile Asp Cys Asn
    290                 295                 300
ACA TGT GTC ACC CAA ACA GTC GAT TTC AGC TTG GAC CCT ACT TTC ACC    960
Thr Cys Val Thr Gln Thr Val Asp Phe Ser Leu Asp Pro Thr Phe Thr
305                 310                 315                 320
ATY GAG ACG ACG ACC GTA CCC CAA GAT GCG GTG TCG CGC TCG CAG CGG   1008
Ile Glu Thr Thr Thr Val Pro Gln Asp Ala Val Ser Arg Ser Gln Arg
                325                 330                 335
CGA GGC AGG ACT GGT AGG GGC AGR GGG GGC ATA TAC AGG TTT GTA ACT   1056
Arg Gly Arg Thr Gly Arg Gly Arg Gly Gly Ile Tyr Arg Phe Val Thr
            340                 345                 350
CCA GGG GAA CGG CCC TCA GGC ATG TTC GAT TCT TCG GTC CTG TGT GAA   1104
Pro Gly Glu Arg Pro Ser Gly Met Phe Asp Ser Ser Val Leu Cys Glu
        355                 360                 365
TGT TAT GAC GCG GGC TGT GCT TGG TAC GAG CTC ACG YCC GCC GAG ACC   1152
Cys Tyr Asp Ala Gly Cys Ala Trp Tyr Glu Leu Thr Xai Ala Glu Thr
    370                 375                 380
TCG GTT AGG TTG CGG GCT TAC CTA AAY ACA CCT GGG CTG CCC GTC TGC   1200
Ser Val Arg Leu Arg Ala Tyr Leu Asn Thr Pro Gly Leu Pro Val Cys
385                 390                 395                 400
CAG GAC CAT CTG GAG TTC TGG GAG AGC GTC TTC ACC GGC CTC ACC CAC   1248
Gln Asp His Leu Glu Phe Trp Glu Ser Val Phe Thr Gly Leu Thr His
                405                 410                 415
ATA GAT GCC CAC TTC TTG TCC CAG ACY AAA CAG GCA GGA GAC AAC TTC   1296
Ile Asp Ala His Phe Leu Ser Gln Thr Lys Gln Ala Gly Asp Asn Phe
            420                 425                 430
```

```
CCC TAC CTG GTA GCA TAC CAG GCT ACA GTG TGC GCC AGG GCC AAG GCT  1344
Pro Tyr Leu Val Ala Tyr Gln Ala Thr Val Cys Ala Arg Ala Lys Ala
        435                 440                 445
CCA CCT CCA TCG TGG GAT CAR ATG TGG AAG TGT CTC ATA CGG CTG AAG  1392
Pro Pro Pro Ser Trp Asp Gln Met Trp Lys Cys Leu Ile Arg Leu Lys
    450                 455                 460
CCT ACG CTA CAC GGG CCA ACG CCC CTG TTG YAT AGG TTA GGA GCC GTT  1440
Pro Thr Leu His Gly Pro Thr Pro Leu Leu Xaj Arg Leu Gly Ala Val
465                 470                 475                 480
CAG AAC RAG GTT RCC CTY ACA CAC CCY ATA ACC AAG TWC ATC ATG RCA  1488
Gln Asn Xak Val Xal Leu Thr His Pro Ile Thr Lys Xam Ile Met Xan
                485                 490                 495
TGC ATG TCG GCT GAC CTA GAG GTC GTC ACT AGC ACT TGG GTG CTG GTA  1536
Cys Met Ser Ala Asp Leu Glu Val Val Thr Ser Thr Trp Val Leu Val
            500                 505                 510
GGC GGG GTC CTC GCG GCT CTG GCC GCG TAC TGC CTG ACA ACG GGC AGC  1584
Gly Gly Val Leu Ala Ala Leu Ala Ala Tyr Cys Leu Thr Thr Gly Ser
        515                 520                 525
GTG GTC ATT GTG GGC AGG ATC ATC TTG TCC GGG AGG CCG GCC GTT ATT  1632
Val Val Ile Val Gly Arg Ile Ile Leu Ser Gly Arg Pro Ala Val Ile
    530                 535                 540
CCC GAC AGG GAA GTT CTC TAC CAA GAG TTC GAT GAA ATG GAA GAG TGC  1680
Pro Asp Arg Glu Val Leu Tyr Gln Glu Phe Asp Glu Met Glu Glu Cys
545                 550                 555                 560
GCC TCG CAC CTC CCT TAC ATC GAA CAA GGA ATG CAG CTC GCC GAG CAA  1728
Ala Ser His Leu Pro Tyr Ile Glu Gln Gly Met Gln Leu Ala Glu Gln
                565                 570                 575
TTC AAG CAG AAG GCG CTC GGT TTG CTG CAA ACA GCC ACC AAG CAA GCG  1776
Phe Lys Gln Lys Ala Leu Gly Leu Leu Gln Thr Ala Thr Lys Gln Ala
            580                 585                 590
GAG GCT GCT GCT CCC GTG GTG GAG TCC AAG TGG CGA GCC CTT GAG ACC  1824
Glu Ala Ala Ala Pro Val Val Glu Ser Lys Trp Arg Ala Leu Glu Thr
        595                 600                 605
TTC TGG GCG AAG CAC ATG TGG AAT TTC ATC AGC GGG ATA CAG TAC TTA  1872
Phe Trp Ala Lys His Met Trp Asn Phe Ile Ser Gly Ile Gln Tyr Leu
    610                 615                 620
GCA GGC TTG TCC ACT CTG CCT GGA AAC CCC GCA ATA GCA TCA CTG ATG  1920
```

```
Ala Gly Leu Ser Thr Leu Pro Gly Asn Pro Ala Ile Ala Ser Leu Met
625                 630                 635                 640
GCA TTC ACA GCC TCT ATC ACC AGC CCG CTC ACC ACC CAA TAT ACC CTC  1968
Ala Phe Thr Ala Ser Ile Thr Ser Pro Leu Thr Thr Gln Tyr Thr Leu
                645                 650                 655
CTG TTT AAC ATC TTG GGG GGA TGG GTG GCC GCC CAA CTC GCC CCC CCC  2016
Leu Phe Asn Ile Leu Gly Gly Trp Val Ala Ala Gln Leu Ala Pro Pro
            660                 665                 670
AGT GCC GCT TCA GCC TTC GTG GGC GCC GGT ATA GCT GGC GCG GCT GTT  2064
Ser Ala Ala Ser Ala Phe Val Gly Ala Gly Ile Ala Gly Ala Ala Val
        675                 680                 685
GGC AGC ATA GGC CTC GGG AAG GTG CTT GTG GAC ATT CTG GCG GGT TAT  2112
Gly Ser Ile Gly Leu Gly Lys Val Leu Val Asp Ile Leu Ala Gly Tyr
    690                 695                 700
GGA GCA GGG GTG GCA GGC GCG CTC GTG GCC TTT AAG GTC ATG AGC GGT  2160
Gly Ala Gly Val Ala Gly Ala Leu Val Ala Phe Lys Val Met Ser Gly
705                 710                 715                 720
GAC ATG CCC TCC ACC GAG GAC CTG GTC AAC TTA CTC CCC GCC ATC CTC  2208
Asp Met Pro Ser Thr Glu Asp Leu Val Asn Leu Leu Pro Ala Ile Leu
                725                 730                 735
TCT CCT GGT GCC CTG GTC GTC GGG GTC GTG TGC GCA GCA ATA CTG CGT  2256
Ser Pro Gly Ala Leu Val Val Gly Val Val Cys Ala Ala Ile Leu Arg
            740                 745                 750
CGG CAT GTG GGC CCA GGG GAG GGG GCT GTG CAG TGG ATG AAC CGG CTG  2304
Arg His Val Gly Pro Gly Glu Gly Ala Val Gln Trp Met Asn Arg Leu
        755                 760                 765
Y : C or T     R : A or G      M : A or C        K : G or T
S : G or C     W : A or T      H : A or C or T   B : G or T or C
Xaa : Leu or Pro       Xab : His or Arg       Xac : Thr or Ala
Xad : Ser or Thr       Xae : Lys or Arg       Xaf : Ile or Val
Xag : Thr or Ile       Xah : Val or Ile       Xai : Pro or Ser
Xaj : Tyr or His       Xak : Gln or Lys       Xal : Thr or Ala
Xam : Tyr or Phe       Xan : Thr or Ala
```

SEQ ID NO:43

SEQUENCE LENGTH: 3564 base pairs

SEQUENCE TYPE: nucleic acid

STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX25N15

```
TGT GCC TGG TTG TGG ATG ATG CTG CTG ATA GCC CAA GCT GAG GCC GCC   48
Cys Ala Trp Leu Trp Met Met Leu Leu Ile Ala Gln Ala Glu Ala Ala
  1               5                  10                  15
TTG GAG AAC CTG GTG GTC CTC AAT GCA GCA TCC ATG GCS GGA GCG CAT   96
Leu Glu Asn Leu Val Val Leu Asn Ala Ala Ser Met Ala Gly Ala His
             20                  25                  30
GGC ATC CTC TCT TTC CTT GTG TTC TTC TGT GCC GCC TGG TAC ATC AAA   144
Gly Ile Leu Ser Phe Leu Val Phe Phe Cys Ala Ala Trp Tyr Ile Lys
         35                  40                  45
GGC AGG CTG GTC CCY GGG GCG RCA TAY GCT YTC TAT GGC GTA TGG CCG   192
Gly Arg Leu Val Pro Gly Ala Xaa Tyr Ala Xab Tyr Gly Val Trp Pro
     50                  55                  60
CTG CTC CTG CTC TTG MTG GCG CTA CCS SCA CGG GCG TAC GCC ATG GAC   240
Leu Leu Leu Leu Leu Xac Ala Leu Pro Xad Arg Ala Tyr Ala Met Asp
 65                  70                  75                  80
CGG GAS ATG GCT GCA TCG TGC GGA GGC GCG GTT TTT GTA GGT CTG GTA   288
Arg Xae Met Ala Ala Ser Cys Gly Gly Ala Val Phe Val Gly Leu Val
                 85                  90                  95
CTC YTG ACC TTG TCA CCA TAC TAC AAA GTG TTC CTC GCT ARG CTC ATA   336
Leu Leu Thr Leu Ser Pro Tyr Tyr Lys Val Phe Leu Ala Xaf Leu Ile
            100                 105                 110
TGG TGG TTR CAA TAT CTC ATC ACC AGR GCC GAG GCG CAC YTG CAA GTG   384
Trp Trp Leu Gln Tyr Leu Ile Thr Arg Ala Glu Ala His Leu Gln Val
        115                 120                 125
TGG ATY CCC CCY CTY AAC GTY CGG GGR GGC CGC GAY GCC ATC ATC CTY   432
Trp Ile Pro Pro Leu Asn Val Arg Gly Gly Arg Asp Ala Ile Ile Leu
    130                 135                 140
CTC ACR TGT GCG GTC CAY CCR GAG CTR ATY TTT GAC ATC ACC AAR CTY   480
Leu Thr Cys Ala Val His Pro Glu Leu Ile Phe Asp Ile Thr Lys Leu
```

```
145                 150                 155                 160
YTG CTC GCC ATA CTC GGT CCG CTC ATG GTR CTC CAG GCT GSC MTA ACY    528
Leu Leu Ala Ile Leu Gly Pro Leu Met Val Leu Gln Ala Xag Xah Thr
                165                 170                 175
MRA RTG CCG TAC TTY GTR CGY GCT CAA GGG CTC ATY CGT RYG TGC ATG    576
Xai Xaj Pro Tyr Phe Val Arg Ala Gln Gly Leu Ile Arg Xak Cys Met
            180                 185                 190
TTR GTG CGG AAA GYC GCY GGR GGT CAT TAT GTY CAR ATG GCY YTY RTG    624
Leu Val Arg Lys Xal Ala Gly Gly His Tyr Val Gln Met Ala Xam Xan
        195                 200                 205
AAG CTG GCY GCR CTG ACA GGT ACG TAC RTT TAT GWC CAT CTT RCY CCA    672
Lys Leu Ala Ala Leu Thr Gly Thr Tyr Xao Tyr Xap His Leu Xaq Pro
    210                 215                 220
CTG CAG SAY TGG GCC CAY GCG GGC CTA CGR GAC CTT GCG GTR GCR GTW    720
Leu Gln Xar Trp Ala His Ala Gly Leu Arg Asp Leu Ala Val Ala Val
225                 230                 235                 240
GAG CCC GTT GYC TTC TCT GAY ATG GAG ACY AAG ATC ATC ACS TGG GGG    768
Glu Pro Val Xas Phe Ser Asp Met Glu Thr Lys Ile Ile Thr Trp Gly
                245                 250                 255
GCA GAS ACB GCG GCG TGT GGG GAC ATC ATY TYG GGY CTA CCH GTY TCC    816
Ala Xat Thr Ala Ala Cys Gly Asp Ile Ile Xau Gly Leu Pro Val Ser
            260                 265                 270
GCC CGR AGG GGS ARS GAG MTR CTY YTS GGR CCG GCC GAT AGT TTT GAC    864
Ala Arg Arg Gly Xav Glu Xaw Leu Xax Gly Pro Ala Asp Ser Phe Asp
        275                 280                 285
GGG CAG GGG TGG CGA CTC CTT GCG CCT ATC ACG GCC TAC TCC CAG CAR    912
Gly Gln Gly Trp Arg Leu Leu Ala Pro Ile Thr Ala Tyr Ser Gln Gln
    290                 295                 300
ACG CGG GGC CTG CTT GGT TGC ATC ATC ACY AGC CTT ACG GGC CGG GAT    960
Thr Arg Gly Leu Leu Gly Cys Ile Ile Thr Ser Leu Thr Gly Arg Asp
305                 310                 315                 320
AAR AAC CAG GTC GAG GGG GAG GTT CAA GTG GTC TCT ACC GCA ACA CAA   1008
Lys Asn Gln Val Glu Gly Glu Val Gln Val Val Ser Thr Ala Thr Gln
                325                 330                 335
TCT TTC CTG GCG ACC TGY RTC AAC GGC GTK TGC TGG ACT GTT TTC CAC   1056
Ser Phe Leu Ala Thr Cys Xay Asn Gly Val Cys Trp Thr Val Phe His
            340                 345                 350
```

```
GGY GCC GGC TCG AAG ACC TTA GCC GGC CCA AAA GGC CCA ATC ACC CAA  1104
Gly Ala Gly Ser Lys Thr Leu Ala Gly Pro Lys Gly Pro Ile Thr Gln
        355                 360                 365
ATG TAC ACC AAT GTR GAT CAG GAC CTC GTC GGY TGG TCG GCG CCC CCC  1152
Met Tyr Thr Asn Val Asp Gln Asp Leu Val Gly Trp Ser Ala Pro Pro
    370                 375                 380
SGG GCG CGT TCC TTG ACA CCW TGC ACC TGC GGC AGC TCG GAC CTT TAT  1200
Xaz Ala Arg Ser Leu Thr Pro Cys Thr Cys Gly Ser Ser Asp Leu Tyr
385                 390                 395                 400
TTG GTC ACG AGR CAT GCT GAT GTC ATT CCG GTG CAC CGG CGG GGC GAC  1248
Leu Val Thr Arg His Ala Asp Val Ile Pro Val His Arg Arg Gly Asp
                405                 410                 415
AGC AGG GGG AGC CTS CTS TCS CCC GGG CCC ATC TCY TAC YTG AAG GGY  1296
Ser Arg Gly Ser Leu Leu Ser Pro Gly Pro Ile Ser Tyr Leu Lys Gly
            420                 425                 430
TCC TCG GGT GGT CCG CTG CYT TGC CCC TCG GGC CRT GTT GTG GGC ATC  1344
Ser Ser Gly Gly Pro Leu Xba Cys Pro Ser Gly Xbb Val Val Gly Ile
        435                 440                 445
TTC CGG GCT GCY GTG TGC ACC CGG GGG GTT GCG AAG GCG GTR GAC TTT  1392
Phe Arg Ala Ala Val Cys Thr Arg Gly Val Ala Lys Ala Val Asp Phe
    450                 455                 460
GTG CCC GTT GAG TCT ATG GAA ACC ACY ATG CGG TCT CCG GTC TTC RCG  1440
Val Pro Val Glu Ser Met Glu Thr Thr Met Arg Ser Pro Val Phe Xbc
465                 470                 475                 480
GAT AAC TCA ACC CCC CCG GCC GTA CCG CAG WCA TTC CAA GTG GCC CAC  1488
Asp Asn Ser Thr Pro Pro Ala Val Pro Gln Xbd Phe Gln Val Ala His
                485                 490                 495
CTA CAC GCT CCC ACT GGC AGC GGC AAA AGC ACC ARG GTG CCG GCT GCG  1536
Leu His Ala Pro Thr Gly Ser Gly Lys Ser Thr Xbe Val Pro Ala Ala
            500                 505                 510
TAT GCG GCC CAA GGG TAC AAG GTA CTC GTC CTG AAC CCG TCC GTT GCT  1584
Tyr Ala Ala Gln Gly Tyr Lys Val Leu Val Leu Asn Pro Ser Val Ala
        515                 520                 525
GCC ACT TTG GGC TTT GGG GCG TAY ATG TCC AAG GCA CAT GGT GTT GAC  1632
Ala Thr Leu Gly Phe Gly Ala Tyr Met Ser Lys Ala His Gly Val Asp
    530                 535                 540
CCT AAC ATC AGA ACT GGG GTG AGG ACC ATC ACC ACG GGC GCT CCC RTC  1680
```

```
Pro Asn Ile Arg Thr Gly Val Arg Thr Ile Thr Thr Gly Ala Pro Xbf
545                 550                 555                 560
ACG TAC TCC ACC TAC GGT AAG TTC CTC GCC GAC GGT GGC TGT TCT GGG   1728
Thr Tyr Ser Thr Tyr Gly Lys Phe Leu Ala Asp Gly Gly Cys Ser Gly
                565                 570                 575
GGT GCC TAT GAC ATC ATA ATA TGT GAT GAG TGT CAT TCA ACT GAC TCG   1776
Gly Ala Tyr Asp Ile Ile Ile Cys Asp Glu Cys His Ser Thr Asp Ser
            580                 585                 590
ACT TCC ATC TTG GGC ATT GGT ACA GTC CTG GAC CAA GCG GAG ACG GCT   1824
Thr Ser Ile Leu Gly Ile Gly Thr Val Leu Asp Gln Ala Glu Thr Ala
        595                 600                 605
GGA GCG CGC CTT GTC GTG CTC GCC ACC GCT ACG CCT CCG GGA TCG GTC   1872
Gly Ala Arg Leu Val Val Leu Ala Thr Ala Thr Pro Pro Gly Ser Val
    610                 615                 620
ACC GTG CCG CAT CCT AAT ATT GAG GAG GTG GCC TTG TCC AAC ACT GGA   1920
Thr Val Pro His Pro Asn Ile Glu Glu Val Ala Leu Ser Asn Thr Gly
625                 630                 635                 640
GAG ATC CCC TTC TAT GGC AAG GCC ATC CCC CTC GAG GCC ATC AAG GGG   1968
Glu Ile Pro Phe Tyr Gly Lys Ala Ile Pro Leu Glu Ala Ile Lys Gly
                645                 650                 655
GGG AGG CAT CTC AYT TTC TGC CAT TCC AAG AAG AAA TGT GAC GAG CTC   2016
Gly Arg His Leu Xbg Phe Cys His Ser Lys Lys Lys Cys Asp Glu Leu
            660                 665                 670
GCT GCG AAG CTG TCG GCC CTC GGA GTC AAY GCT GTA GCA TAY TAC CGG   2064
Ala Ala Lys Leu Ser Ala Leu Gly Val Asn Ala Val Ala Tyr Tyr Arg
        675                 680                 685
GGT CTT GAT GTG TCC RTC ATA CCG ACA AGC GGG GAC GTC GTT GTC GTG   2112
Gly Leu Asp Val Ser Xbh Ile Pro Thr Ser Gly Asp Val Val Val Val
    690                 695                 700
GCA ACW GAC GCT CTA ATG ACG GGC TAT ACC GGY GAC TTY GAC TCA GTG   2160
Ala Thr Asp Ala Leu Met Thr Gly Tyr Thr Gly Asp Phe Asp Ser Val
705                 710                 715                 720
ATC GAC TGC AAC ACA TGT GTC ACC CAA ACA GTC GAT TTC AGC TTG GAC   2208
Ile Asp Cys Asn Thr Cys Val Thr Gln Thr Val Asp Phe Ser Leu Asp
                725                 730                 735
CCT ACT TTC ACC ATY GAG ACG ACG ACC GTA CCC CAA GAT GCG GTG TCG   2256
Pro Thr Phe Thr Ile Glu Thr Thr Thr Val Pro Gln Asp Ala Val Ser
```

```
              740                       745                       750
CGC TCG CAG CGG CGA GGC AGG ACT GGT AGG GGC AGR GGG GGC ATA TAC   2304
Arg Ser Gln Arg Arg Gly Arg Thr Gly Arg Gly Arg Gly Gly Ile Tyr
        755                       760                       765
AGG TTT GTA ACT CCA GGG GAA CGG CCC TCA GGC ATG TTC GAT TCT TCG   2352
Arg Phe Val Thr Pro Gly Glu Arg Pro Ser Gly Met Phe Asp Ser Ser
    770                       775                       780
GTC CTG TGT GAA TGT TAT GAC GCG GGC TGT GCT TGG TAC GAG CTC ACG   2400
Val Leu Cys Glu Cys Tyr Asp Ala Gly Cys Ala Trp Tyr Glu Leu Thr
785                     790                       795                 800
YCC GCC GAG ACC TCG GTT AGG TTG CGG GCT TAC CTA AAY ACA CCT GGG   2448
Xbi Ala Glu Thr Ser Val Arg Leu Arg Ala Tyr Leu Asn Thr Pro Gly
                805                       810                       815
CTG CCC GTC TGC CAG GAC CAT CTG GAG TTC TGG GAG AGC GTC TTC ACC   2496
Leu Pro Val Cys Gln Asp His Leu Glu Phe Trp Glu Ser Val Phe Thr
            820                       825                       830
GGC CTC ACC CAC ATA GAT GCC CAC TTC TTG TCC CAG ACY AAA CAG GCA   2544
Gly Leu Thr His Ile Asp Ala His Phe Leu Ser Gln Thr Lys Gln Ala
        835                       840                       845
GGA GAC AAC TTC CCC TAC CTG GTA GCA TAC CAG GCT ACA GTG TGC GCC   2592
Gly Asp Asn Phe Pro Tyr Leu Val Ala Tyr Gln Ala Thr Val Cys Ala
    850                       855                       860
AGG GCC AAG GCT CCA CCT CCA TCG TGG GAT CAR ATG TGG AAG TGT CTC   2640
Arg Ala Lys Ala Pro Pro Pro Ser Trp Asp Gln Met Trp Lys Cys Leu
865                     870                       875                 880
ATA CGG CTG AAG CCT ACG CTA CAC GGG CCA ACG CCC CTG TTG YAT AGG   2688
Ile Arg Leu Lys Pro Thr Leu His Gly Pro Thr Pro Leu Leu Xbj Arg
                885                       890                       895
TTA GGA GCC GTT CAG AAC RAG GTT RCC CTY ACA CAC CCY ATA ACC AAG   2736
Leu Gly Ala Val Gln Asn Xbk Val Xbl Leu Thr His Pro Ile Thr Lys
            900                       905                       910
TWC ATC ATG RCA TGC ATG TCG GCT GAC CTA GAG GTC GTC ACT AGC ACT   2784
Xbm Ile Met Xbn Cys Met Ser Ala Asp Leu Glu Val Val Thr Ser Thr
        915                       920                       925
TGG GTG CTG GTA GGC GGG GTC CTC GCG GCT CTG GCC GCG TAC TGC CTG   2832
Trp Val Leu Val Gly Gly Val Leu Ala Ala Leu Ala Ala Tyr Cys Leu
    930                       935                       940
```

ACA ACG GGC AGC GTG GTC ATT GTG GGC AGG ATC ATC TTG TCC GGG AGG 2880
Thr Thr Gly Ser Val Val Ile Val Gly Arg Ile Ile Leu Ser Gly Arg
945 950 955 960
CCG GCC GTT ATT CCC GAC AGG GAA GTT CTC TAC CAA GAG TTC GAT GAA 2928
Pro Ala Val Ile Pro Asp Arg Glu Val Leu Tyr Gln Glu Phe Asp Glu
965 970 975
ATG GAA GAG TGC GCC TCG CAC CTC CCT TAC ATC GAA CAA GGA ATG CAG 2976
Met Glu Glu Cys Ala Ser His Leu Pro Tyr Ile Glu Gln Gly Met Gln
980 985 990
CTC GCC GAG CAA TTC AAG CAG AAG GCG CTC GGT TTG CTG CAA ACA GCC 3024
Leu Ala Glu Gln Phe Lys Gln Lys Ala Leu Gly Leu Leu Gln Thr Ala
995 1000 1005
ACC AAG CAA GCG GAG GCT GCT GCT CCC GTG GTG GAG TCC AAG TGG CGA 3072
Thr Lys Gln Ala Glu Ala Ala Ala Pro Val Val Glu Ser Lys Trp Arg
1010 1015 1020
GCC CTT GAG ACC TTC TGG GCG AAG CAC ATG TGG AAT TTC ATC AGC GGG 3120
Ala Leu Glu Thr Phe Trp Ala Lys His Met Trp Asn Phe Ile Ser Gly
1025 1030 1035 1040
ATA CAG TAC TTA GCA GGC TTG TCC ACT CTG CCT GGA AAC CCC GCA ATA 3168
Ile Gln Tyr Leu Ala Gly Leu Ser Thr Leu Pro Gly Asn Pro Ala Ile
1045 1050 1055
GCA TCA CTG ATG GCA TTC ACA GCC TCT ATC ACC AGC CCG CTC ACC ACC 3216
Ala Ser Leu Met Ala Phe Thr Ala Ser Ile Thr Ser Pro Leu Thr Thr
1060 1065 1070
CAA TAT ACC CTC CTG TTT AAC ATC TTG GGG GGA TGG GTG GCC GCC CAA 3264
Gln Tyr Thr Leu Leu Phe Asn Ile Leu Gly Gly Trp Val Ala Ala Gln
1075 1080 1085
CTC GCC CCC CCC AGT GCC GCT TCA GCC TTC GTG GGC GCC GGT ATA GCT 3312
Leu Ala Pro Pro Ser Ala Ala Ser Ala Phe Val Gly Ala Gly Ile Ala
1090 1095 1100
GGC GCG GCT GTT GGC AGC ATA GGC CTC GGG AAG GTG CTT GTG GAC ATT 3360
Gly Ala Ala Val Gly Ser Ile Gly Leu Gly Lys Val Leu Val Asp Ile
1105 1110 1115 1120
CTG GCG GGT TAT GGA GCA GGG GTG GCA GGC GCG CTC GTG GCC TTT AAG 3408
Leu Ala Gly Tyr Gly Ala Gly Val Ala Gly Ala Leu Val Ala Phe Lys
1125 1130 1135
GTC ATG AGC GGT GAC ATG CCC TCC ACC GAG GAC CTG GTC AAC TTA CTC 3456

```
Val Met Ser Gly Asp Met Pro Ser Thr Glu Asp Leu Val Asn Leu Leu
            1140                1145                1150
CCC GCC ATC CTC TCT CCT GGT GCC CTG GTC GTC GGG GTC GTG TGC GCA  3504
Pro Ala Ile Leu Ser Pro Gly Ala Leu Val Val Gly Val Val Cys Ala
        1155                1160                1165
GCA ATA CTG CGT CGG CAT GTG GGC CCA GGG GAG GGG GCT GTG CAG TGG  3552
Ala Ile Leu Arg Arg His Val Gly Pro Gly Glu Gly Ala Val Gln Trp
    1170                1175                1180
ATG AAC CGG CTG                                                  3564
Met Asn Arg Leu
1185
```

| | | | |
|---|---|---|---|
| Y : C or T | R : A or G | M : A or C | K : G or T |
| S : G or C | W : A or T | H : A or C or T | B : G or T or C |

| | | |
|---|---|---|
| Xaa : Ala or Thr | Xab : Phe or Leu | Xac : Met or Leu |
| Xad : Ala or Pro | Xae : Glu or Asp | Xaf : Lys or Arg |
| Xag : Gly or Ala | Xah : Leu or Ile | Xai : Gln or Arg |
| Xaj : Met or Val | Xak : Met or Ala | Xal : Ala or Val |
| Xam : Leu or Phe | Xan : Met or Val | Xao : Val or Ile |
| Xap : Asp or Val | Xaq : Thr or Ala | Xar : Asp or His |
| Xas : Ala or Val | Xat : Asp or Glu | Xau : Leu or Ser |
| Xav : Asn or Arg or Lys | Xaw : Ile or Leu | Xax : Leu or Phe |
| Xay : Ile or Val | Xaz : Gly or Arg | Xba : Leu or Pro |
| Xbb : His or Arg | Xbc : Thr or Ala | Xbd : Ser or Thr |
| Xbe : Lys or Arg | Xbf : Ile or Val | Xbg : Thr or Ile |
| Xbh : Val or Ile | Xbi : Pro or Ser | Xbj : Tyr or His |
| Xbk : Glu or Lys | Xbl : Thr or Ala | Xbm : Tyr or Phe |
| Xbn : Thr or Ala | | |

SEQ ID NO:44
SEQUENCE LENGTH: 849 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE

CLONE: MX25-1

```
TGT GCC TGG TTG TGG ATG ATG CTG CTG ATA GCC CAA GCT GAG GCC GCC   48
Cys Ala Trp Leu Trp Met Met Leu Leu Ile Ala Gln Ala Glu Ala Ala
 1               5                  10                  15
TTG GAG AAC CTG GTG GTC CTC AAT GCA GCA TCC ATG GCG GGA GCG CAT   96
Leu Glu Asn Leu Val Val Leu Asn Ala Ala Ser Met Ala Gly Ala His
                20                  25                  30
GGC ATC CTC TCT TTC CTT GTG TTC TTC TGT GCC GCC TGG TAC ATC AAA  144
Gly Ile Leu Ser Phe Leu Val Phe Phe Cys Ala Ala Trp Tyr Ile Lys
            35                  40                  45
GGC AGG CTG GTC CCT GGG GCG GCA TAC GCT TTC TAT GGC GTA TGG CCG  192
Gly Arg Leu Val Pro Gly Ala Ala Tyr Ala Phe Tyr Gly Val Trp Pro
        50                  55                  60
CTG CTC CTG CTC TTG ATG GCG CTA CCC GCA CGG GCG TAC GCC ATG GAC  240
Leu Leu Leu Leu Leu Met Ala Leu Pro Ala Arg Ala Tyr Ala Met Asp
 65                  70                  75                  80
CGG GAG ATG GCT GCA TCG TGC GGA GGC GCG GTT TTT GTA GGT CTG GTA  288
Arg Glu Met Ala Ala Ser Cys Gly Gly Ala Val Phe Val Gly Leu Val
                    85                  90                  95
CTC TTG ACC TTG TCA CCA TAC TAC AAA GTG TTC CTC GCT AAG CTC ATA  336
Leu Leu Thr Leu Ser Pro Tyr Tyr Lys Val Phe Leu Ala Lys Leu Ile
                100                 105                 110
TGG TGG TTG CAA TAT CTC ATC ACC AGG GCC GAG GCG CAC TTG CAA GTG  384
Trp Trp Leu Gln Tyr Leu Ile Thr Arg Ala Glu Ala His Leu Gln Val
            115                 120                 125
TGG ATC CCC CCC CTC AAC GTT CGG GGG GGC CGC GAT GCC ATC ATC CTT  432
Trp Ile Pro Pro Leu Asn Val Arg Gly Gly Arg Asp Ala Ile Ile Leu
        130                 135                 140
CTC ACA TGT GCG GTC CAC CCG GAG CTG ATC TTT GAC ATC ACC AAG CTC  480
Leu Thr Cys Ala Val His Pro Glu Leu Ile Phe Asp Ile Thr Lys Leu
145                 150                 155                 160
TTG CTC GCC ATA CTC GGT CCG CTC ATG GTA CTC CAG GCT GGC CTA ACC  528
Leu Leu Ala Ile Leu Gly Pro Leu Met Val Leu Gln Ala Gly Leu Thr
                165                 170                 175
CAA ATG CCG TAC TTT GTG CGT GCT CAA GGG CTC ATT CGT ATG TGC ATG  576
Gln Met Pro Tyr Phe Val Arg Ala Gln Gly Leu Ile Arg Met Cys Met
```

```
              180                          185                          190
     TTG GTG CGG AAA GCC GCT GGG GGT CAT TAT GTC CAG ATG GCT CTC ATG    624
     Leu Val Arg Lys Ala Ala Gly Gly His Tyr Val Gln Met Ala Leu Met
             195                          200                          205
     AAG CTG GCT GCA CTG ACA GGT ACG TAC GTT TAT GAC CAT CTT ACT CCA    672
     Lys Leu Ala Ala Leu Thr Gly Thr Tyr Val Tyr Asp His Leu Thr Pro
         210                          215                          220
     CTG CAG GAC TGG GCC CAC GCG GGC CTA CGA GAC CTT GCG GTA GCA GTT    720
     Leu Gln Asp Trp Ala His Ala Gly Leu Arg Asp Leu Ala Val Ala Val
     225                          230                          235                 240
     GAG CCC GTT GCC TTC TCT GAT ATG GAG ACT AAG ATC ATC ACC TGG GGG    768
     Glu Pro Val Ala Phe Ser Asp Met Glu Thr Lys Ile Ile Thr Trp Gly
                      245                          250                          255
     GCA GAC ACT GCG GCG TGT GGG GAC ATC ATT TTG GGC CTA CCT GTC TCC    816
     Ala Asp Thr Ala Ala Cys Gly Asp Ile Ile Leu Gly Leu Pro Val Ser
                  260                          265                          270
     GCC CGG AGG GGC AAC GAG ATA CTC CTC GGA CCG                        849
     Ala Arg Arg Gly Asn Glu Ile Leu Leu Gly Pro
             275                          280
```

SEQ ID NO:45
SEQUENCE LENGTH: 849 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX25-2

```
     TGT GCC TGG TTG TGG ATG ATG CTG CTG ATA GCC CAA GCT GAG GCC GCC     48
     Cys Ala Trp Leu Trp Met Met Leu Leu Ile Ala Gln Ala Glu Ala Ala
       1               5                  10                  15
     TTG GAG AAC CTG GTG GTC CTC AAT GCA GCA TCC ATG GCG GGA GCG CAT     96
     Leu Glu Asn Leu Val Val Leu Asn Ala Ala Ser Met Ala Gly Ala His
                  20                          25                          30
```

```
GGC ATC CTC TCT TTC CTT GTG TTC TTC TGT GCC GCC TGG TAC ATC AAA    144
Gly Ile Leu Ser Phe Leu Val Phe Phe Cys Ala Ala Trp Tyr Ile Lys
        35                  40                  45
GGC AGG CTG GTC CCT GGG GCG ACA TAC GCT CTC TAT GGC GTA TGG CCG    192
Gly Arg Leu Val Pro Gly Ala Thr Tyr Ala Leu Tyr Gly Val Trp Pro
    50                  55                  60
CTG CTC CTG CTC TTG ATG GCG CTA CCG CCA CGG GCG TAC GCC ATG GAC    240
Leu Leu Leu Leu Leu Met Ala Leu Pro Pro Arg Ala Tyr Ala Met Asp
 65                  70                  75                  80
CGG GAC ATG GCT GCA TCG TGC GGA GGC GCG GTT TTT GTA GGT CTG GTA    288
Arg Asp Met Ala Ala Ser Cys Gly Gly Ala Val Phe Val Gly Leu Val
                85                  90                  95
CTC TTG ACC TTG TCA CCA TAC TAC AAA GTG TTC CTC GCT AGG CTC ATA    336
Leu Leu Thr Leu Ser Pro Tyr Tyr Lys Val Phe Leu Ala Arg Leu Ile
            100                 105                 110
TGG TGG TTA CAA TAT CTC ATC ACC AGA GCC GAG GCG CAC CTG CAA GTG    384
Trp Trp Leu Gln Tyr Leu Ile Thr Arg Ala Glu Ala His Leu Gln Val
        115                 120                 125
TGG ATT CCC CCT CTC AAC GTC CGG GGA GGC CGC GAC GCC ATC ATC CTC    432
Trp Ile Pro Pro Leu Asn Val Arg Gly Gly Arg Asp Ala Ile Ile Leu
    130                 135                 140
CTC ACG TGT GCG GTC CAT CCA GAG CTA ATT TTT GAC ATC ACC AAA CTT    480
Leu Thr Cys Ala Val His Pro Glu Leu Ile Phe Asp Ile Thr Lys Leu
145                 150                 155                 160
CTG CTC GCC ATA CTC GGT CCG CTC ATG GTG CTC CAG GCT GCC ATA ACT    528
Leu Leu Ala Ile Leu Gly Pro Leu Met Val Leu Gln Ala Ala Ile Thr
                165                 170                 175
AGA GTG CCG TAC TTC GTA CGC GCT CAA GGG CTC ATC CGT GCG TGC ATG    576
Arg Val Pro Tyr Phe Val Arg Ala Gln Gly Leu Ile Arg Ala Cys Met
            180                 185                 190
TTA GTG CGG AAA GCC GCC GGA GGT CAT TAT GTT CAA ATG GCC TTT GTG    624
Leu Val Arg Lys Ala Ala Gly Gly His Tyr Val Gln Met Ala Phe Val
        195                 200                 205
AAG CTG GCC GCG CTG ACA GGT ACG TAC ATT TAT GAC CAT CTT GCC CCA    672
Lys Leu Ala Ala Leu Thr Gly Thr Tyr Ile Tyr Asp His Leu Ala Pro
    210                 215                 220
CTG CAG CAT TGG GCC CAT GCG GGC CTA CGG GAC CTT GCG GTG GCG GTA    720
```

```
Leu Gln His Trp Ala His Ala Gly Leu Arg Asp Leu Ala Val Ala Val
225                 230                 235                 240
GAG CCC GTT GTC TTC TCT GAC ATG GAG ACC AAG ATC ATC ACC TGG GGG   768
Glu Pro Val Val Phe Ser Asp Met Glu Thr Lys Ile Ile Thr Trp Gly
                245                 250                 255
GCA GAC ACC GCG GCG TGT GGG GAC ATC ATT TTG GGC CTA CCA GTC TCC   816
Ala Asp Thr Ala Ala Cys Gly Asp Ile Ile Leu Gly Leu Pro Val Ser
            260                 265                 270
GCC CGG AGG GGC AAC GAG ATA CTC CTC GGA CCG                       849
Ala Arg Arg Gly Asn Glu Ile Leu Leu Gly Pro
        275                 280
```

SEQ ID NO:46
SEQUENCE LENGTH: 849 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX25-3

```
TGT GCC TGG TTG TGG ATG ATG CTG CTG ATA GCC CAA GCT GAG GCC GCC    48
Cys Ala Trp Leu Trp Met Met Leu Leu Ile Ala Gln Ala Glu Ala Ala
  1               5                  10                  15
TTG GAG AAC CTG GTG GTC CTC AAT GCA GCA TCC ATG GCC GGA GCG CAT    96
Leu Glu Asn Leu Val Val Leu Asn Ala Ala Ser Met Ala Gly Ala His
            20                  25                  30
GGC ATC CTC TCT TTC CTT GTG TTC TTC TGT GCC GCC TGG TAC ATC AAA   144
Gly Ile Leu Ser Phe Leu Val Phe Phe Cys Ala Ala Trp Tyr Ile Lys
        35                  40                  45
GGC AGG CTG GTC CCC GGG GCG GCA TAT GCT TTC TAT GGC GTA TGG CCG   192
Gly Arg Leu Val Pro Gly Ala Ala Tyr Ala Phe Tyr Gly Val Trp Pro
    50                  55                  60
CTG CTC CTG CTC TTG CTG GCG CTA CCC GCA CGG GCG TAC GCC ATG GAC   240
Leu Leu Leu Leu Leu Leu Ala Leu Pro Ala Arg Ala Tyr Ala Met Asp
```

```
 65                  70                   75                   80
CGG GAG ATG GCT GCA TCG TGC GGA GGC GCG GTT TTT GTA GGT CTG GTA    288
Arg Glu Met Ala Ala Ser Cys Gly Gly Ala Val Phe Val Gly Leu Val
                    85                  90                  95
CTC CTG ACC TTG TCA CCA TAC TAC AAA GTG TTC CTC GCT AAG CTC ATA    336
Leu Leu Thr Leu Ser Pro Tyr Tyr Lys Val Phe Leu Ala Lys Leu Ile
            100                 105                 110
TGG TGG TTG CAA TAT CTC ATC ACC AGG GCC GAG GCG CAC TTG CAA GTG    384
Trp Trp Leu Gln Tyr Leu Ile Thr Arg Ala Glu Ala His Leu Gln Val
        115                 120                 125
TGG ATC CCC CCC CTT AAC GTT CGG GGG GGC CGC GAT GCC ATC ATC CTT    432
Trp Ile Pro Pro Leu Asn Val Arg Gly Gly Arg Asp Ala Ile Ile Leu
    130                 135                 140
CTC ACA TGT GCG GTC CAC CCG GAG CTG ATC TTT GAC ATC ACC AAG CTC    480
Leu Thr Cys Ala Val His Pro Glu Leu Ile Phe Asp Ile Thr Lys Leu
145                 150                 155                 160
TTG CTC GCC ATA CTC GGT CCG CTC ATG GTA CTC CAG GCT GGC CTA ACC    528
Leu Leu Ala Ile Leu Gly Pro Leu Met Val Leu Gln Ala Gly Leu Thr
                165                 170                 175
CAA ATG CCG TAC TTT GTG CGT GCT CAA GGG CTC ATT CGT ATG TGC ATG    576
Gln Met Pro Tyr Phe Val Arg Ala Gln Gly Leu Ile Arg Met Cys Met
            180                 185                 190
TTG GTG CGG AAA GTC GCT GGG GGT CAT TAT GTC CAG ATG GCT CTC ATG    624
Leu Val Arg Lys Val Ala Gly Gly His Tyr Val Gln Met Ala Leu Met
        195                 200                 205
AAG CTG GCT GCA CTG ACA GGT ACG TAC GTT TAT GTC CAT CTT ACT CCA    672
Lys Leu Ala Ala Leu Thr Gly Thr Tyr Val Tyr Val His Leu Thr Pro
    210                 215                 220
CTG CAG GAC TGG GCC CAC GCG GGC CTA CGA GAC CTT GCG GTA GCA GTT    720
Leu Gln Asp Trp Ala His Ala Gly Leu Arg Asp Leu Ala Val Ala Val
225                 230                 235                 240
GAG CCC GTT GTC TTC TCT GAT ATG GAG ACT AAG ATC ATC ACC TGG GGG    768
Glu Pro Val Val Phe Ser Asp Met Glu Thr Lys Ile Ile Thr Trp Gly
                245                 250                 255
GCA GAC ACC GCG GCG TGT GGG GAC ATC ATT TTG GGC CTA CCT GTC TCC    816
Ala Asp Thr Ala Ala Cys Gly Asp Ile Ile Leu Gly Leu Pro Val Ser
            260                 265                 270
```

```
GCC CGG AGG GGC AAC GAG ATA CTC CTC GGA CCG                          849
Ala Arg Arg Gly Asn Glu Ile Leu Leu Gly Pro
        275                 280
```

SEQ ID NO:47
SEQUENCE LENGTH: 524 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O26-1

```
ATC ACG TGG GGG GCA GAG ACG GCG GCG TGT GGG GAC ATC ATC TCG GGT   48
Ile Thr Trp Gly Ala Glu Thr Ala Ala Cys Gly Asp Ile Ile Ser Gly
  1               5                  10                  15
CTA CCC GTT TCC GCC CGA AGG GGG AGG GAG CTG CTT TTG GGG CCG GCC   96
Leu Pro Val Ser Ala Arg Arg Gly Arg Glu Leu Leu Leu Gly Pro Ala
         20                  25                  30
GAT AGT TTT GAC GGG CAG GGG TGG CGA CTC CTT GCG CCT ATC ACG GCC  144
Asp Ser Phe Asp Gly Gln Gly Trp Arg Leu Leu Ala Pro Ile Thr Ala
         35                  40                  45
TAC TCC CAG CAG ACG CGG GGC CTG CTT GGT TGC ATC ATC ACT AGC CTT  192
Tyr Ser Gln Gln Thr Arg Gly Leu Leu Gly Cys Ile Ile Thr Ser Leu
     50                  55                  60
ACG GGC CGG GAT AAG AAC CAG GTC GAG GGG GAG GTT CAA GTG GTC TCT  240
Thr Gly Arg Asp Lys Asn Gln Val Glu Gly Glu Val Gln Val Val Ser
 65                  70                  75                  80
ACC GCA ACA CAA TCT TTC CTG GCG ACC TGT GTC AAC GGC GTG TGC TGG  288
Thr Ala Thr Gln Ser Phe Leu Ala Thr Cys Val Asn Gly Val Cys Trp
                 85                  90                  95
ACT GTT TTC CAC GGC GCC GGC TCG AAG ACC TTA GCC GGC CCA AAA GGC  336
Thr Val Phe His Gly Ala Gly Ser Lys Thr Leu Ala Gly Pro Lys Gly
            100                 105                 110
CCA ATC ACC CAA ATG TAC ACC AAT GTA GAT CAG GAC CTC GTC GGC TGG  384
```

```
Pro Ile Thr Gln Met Tyr Thr Asn Val Asp Gln Asp Leu Val Gly Trp
        115                 120                 125
TCG GCG CCC CCC CGG GCG CGT TCC TTG ACA CCT TGC ACC TGC GGC AGC    432
Ser Ala Pro Pro Arg Ala Arg Ser Leu Thr Pro Cys Thr Cys Gly Ser
    130                 135                 140
TCG GAC CTT TAT TTG GTC ACG AGG CAT GCT GAT GTC ATT CCG GTG CAC    480
Ser Asp Leu Tyr Leu Val Thr Arg His Ala Asp Val Ile Pro Val His
145                 150                 155                 160
CGG CGG GGC GAC AGC AGG GGG AGC CTC CTC TCC CCC GGG CCC AT         524
Arg Arg Gly Asp Ser Arg Gly Ser Leu Leu Ser Pro Gly Pro
                165                 170
```

SEQ ID NO:48
SEQUENCE LENGTH: 514 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O26-2

```
ATC ACG TGG GGG GCA GAG ACG GCG GCG TGT GGG GAC ATC ATC TCG GGT     48
Ile Thr Trp Gly Ala Glu Thr Ala Ala Cys Gly Asp Ile Ile Ser Gly
  1               5                  10                  15
CTA CCC GTT TCC GCC CGA AGG GGG AGG GAG CTG CTT TTG GGA CCG GCC     96
Leu Pro Val Ser Ala Arg Arg Gly Arg Glu Leu Leu Leu Gly Pro Ala
             20                  25                  30
GAT AGT TTT GAC GGG CAG GGG TGG CGA CTC CTT GCG CCT ATC ACG GCC    144
Asp Ser Phe Asp Gly Gln Gly Trp Arg Leu Leu Ala Pro Ile Thr Ala
         35                  40                  45
TAC TCC CAG CAG ACG CGG GGC CTG CTT GGT TGC ATC ATC ACC AGC CTT    192
Tyr Ser Gln Gln Thr Arg Gly Leu Leu Gly Cys Ile Ile Thr Ser Leu
     50                  55                  60
ACG GGC CGG GAT AAG AAC CAG GTC GAG GGG GAG GTT CAA GTG GTC TCT    240
Thr Gly Arg Asp Lys Asn Gln Val Glu Gly Glu Val Gln Val Val Ser
```

```
 65                   70                   75                   80
ACC GCA ACA CAA TCT TTC CTG GCG ACC TGC ATC AAC GGC GTT TGC TGG   288
Thr Ala Thr Gln Ser Phe Leu Ala Thr Cys Ile Asn Gly Val Cys Trp
                    85                   90                   95
ACT GTT TTC CAC GGC GCC GGC TCG AAG ACC TTA GCC GGC CCA AAA GGC   336
Thr Val Phe His Gly Ala Gly Ser Lys Thr Leu Ala Gly Pro Lys Gly
            100                  105                  110
CCA ATC ACC CAA ATG TAC ACC AAT GTA GAT CAG GAC CTC GTC GGC TGG   384
Pro Ile Thr Gln Met Tyr Thr Asn Val Asp Gln Asp Leu Val Gly Trp
        115                  120                  125
TCG GCG CCC CCC GGG GCG CGT TCC TTG ACA CCT TGC ACC TGC GGC AGC   432
Ser Ala Pro Pro Gly Ala Arg Ser Leu Thr Pro Cys Thr Cys Gly Ser
    130                  135                  140
TCG GAC CTT TAT TTG GTC ACG AGG CAT GCT GAT GTC ATT CCG GTG CAC   480
Ser Asp Leu Tyr Leu Val Thr Arg His Ala Asp Val Ile Pro Val His
145                  150                  155                  160
CGG CGG GGC GAC AGC AGG GGG AGC CTC CTC TCC C                     514
Arg Arg Gly Asp Ser Arg Gly Ser Leu Leu Ser
                165                  170
```

SEQ ID NO:49
SEQUENCE LENGTH: 523 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O26-3

```
ATC ACG TGG GGG GCA GAG ACG GCG GCG TGT GGG GAC ATC ATC TCG GGT   48
Ile Thr Trp Gly Ala Glu Thr Ala Ala Cys Gly Asp Ile Ile Ser Gly
  1               5                  10                  15
CTA CCC GTT TCC GCC CGA AGG GGG AAG GAG CTG CTT TTG GGA CCG GCC   96
Leu Pro Val Ser Ala Arg Arg Gly Lys Glu Leu Leu Leu Gly Pro Ala
            20                   25                   30
```

```
GAT AGT TTT GAC GGG CAG GGG TGG CGA CTC CTT GCG CCT ATC ACG GCC    144
Asp Ser Phe Asp Gly Gln Gly Trp Arg Leu Leu Ala Pro Ile Thr Ala
         35                  40                  45
TAC TCC CAG CAA ACG CGG GGC CTG CTT GGT TGC ATC ATC ACT AGC CTT    192
Tyr Ser Gln Gln Thr Arg Gly Leu Leu Gly Cys Ile Ile Thr Ser Leu
     50                  55                  60
ACG GGC CGG GAT AAA AAC CAG GTC GAG GGG GAG GTT CAA GTG GTC TCT    240
Thr Gly Arg Asp Lys Asn Gln Val Glu Gly Glu Val Gln Val Val Ser
 65                  70                  75                  80
ACC GCA ACA CAA TCT TTC CTG GCG ACC TGT GTC AAC GGC GTG TGC TGG    288
Thr Ala Thr Gln Ser Phe Leu Ala Thr Cys Val Asn Gly Val Cys Trp
                 85                  90                  95
ACT GTT TTC CAC GGT GCC GGC TCG AAG ACC TTA GCC GGC CCA AAA GGC    336
Thr Val Phe His Gly Ala Gly Ser Lys Thr Leu Ala Gly Pro Lys Gly
            100                 105                 110
CCA ATC ACC CAA ATG TAC ACC AAT GTG GAT CAG GAC CTC GTC GGT TGG    384
Pro Ile Thr Gln Met Tyr Thr Asn Val Asp Gln Asp Leu Val Gly Trp
        115                 120                 125
TCG GCG CCC CCC GGG GCG CGT TCC TTG ACA CCA TGC ACC TGC GGC AGC    432
Ser Ala Pro Pro Gly Ala Arg Ser Leu Thr Pro Cys Thr Cys Gly Ser
    130                 135                 140
TCG GAC CTT TAT TTG GTC ACG AGA CAT GCT GAT GTC ATT CCG GTG CAC    480
Ser Asp Leu Tyr Leu Val Thr Arg His Ala Asp Val Ile Pro Val His
145                 150                 155                 160
CGG CGG GGC GAC AGC AGG GGG AGC CTC CTC TCC CCC GGG CCC A          523
Arg Arg Gly Asp Ser Arg Gly Ser Leu Leu Ser Pro Gly Pro
                165                 170
```

SEQ ID NO:50
SEQUENCE LENGTH: 921 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE

CLONE: N23-1

```
CTG CTG TCG CCC GGG CCC ATC TCT TAC TTG AAG GGT TCC TCG GGT GGT    48
Leu Leu Ser Pro Gly Pro Ile Ser Tyr Leu Lys Gly Ser Ser Gly Gly
  1               5                  10                  15
CCG CTG CCT TGC CCC TCG GGC CGT GTT GTG GGC ATC TTC CGG GCT GCC    96
Pro Leu Pro Cys Pro Ser Gly Arg Val Val Gly Ile Phe Arg Ala Ala
                 20                  25                  30
GTG TGC ACC CGG GGG GTT GCG AAG GCG GTG GAC TTT GTG CCC GTT GAG   144
Val Cys Thr Arg Gly Val Ala Lys Ala Val Asp Phe Val Pro Val Glu
             35                  40                  45
TCT ATG GAA ACC ACC ATG CGG TCT CCG GTC TTC ACG GAT AAC TCA ACC   192
Ser Met Glu Thr Thr Met Arg Ser Pro Val Phe Thr Asp Asn Ser Thr
         50                  55                  60
CCC CCG GCC GTA CCG CAG ACA TTC CAA GTG GCC CAC CTA CAC GCT CCC   240
Pro Pro Ala Val Pro Gln Thr Phe Gln Val Ala His Leu His Ala Pro
 65                  70                  75                  80
ACT GGC AGC GGC AAA AGC ACC AGG GTG CCG GCT GCG TAT GCG GCC CAA   288
Thr Gly Ser Gly Lys Ser Thr Arg Val Pro Ala Ala Tyr Ala Ala Gln
                 85                  90                  95
GGG TAC AAG GTA CTC GTC CTG AAC CCG TCC GTT GCT GCC ACT TTG GGC   336
Gly Tyr Lys Val Leu Val Leu Asn Pro Ser Val Ala Ala Thr Leu Gly
            100                 105                 110
TTT GGG GCG TAC ATG TCC AAG GCA CAT GGT GTT GAC CCT AAC ATC AGA   384
Phe Gly Ala Tyr Met Ser Lys Ala His Gly Val Asp Pro Asn Ile Arg
        115                 120                 125
ACT GGG GTG AGG ACC ATC ACC ACG GGC GCT CCC ATC ACG TAC TCC ACC   432
Thr Gly Val Arg Thr Ile Thr Thr Gly Ala Pro Ile Thr Tyr Ser Thr
    130                 135                 140
TAC GGT AAG TTC CTC GCC GAC GGT GGC TGT TCT GGG GGT GCC TAT GAC   480
Tyr Gly Lys Phe Leu Ala Asp Gly Gly Cys Ser Gly Gly Ala Tyr Asp
145                 150                 155                 160
ATC ATA ATA TGT GAT GAG TGT CAT TCA ACT GAC TCG ACT TCC ATC TTG   528
Ile Ile Ile Cys Asp Glu Cys His Ser Thr Asp Ser Thr Ser Ile Leu
                165                 170                 175
GGC ATT GGT ACA GTC CTG GAC CAA GCG GAG ACG GCT GGA GCG CGC CTT   576
Gly Ile Gly Thr Val Leu Asp Gln Ala Glu Thr Ala Gly Ala Arg Leu
```

```
                    180                           185                           190
        GTC GTG CTC GCC ACC GCT ACG CCT CCG GGA TCG GTC ACC GTG CCG CAT     624
        Val Val Leu Ala Thr Ala Thr Pro Pro Gly Ser Val Thr Val Pro His
                195                           200                           205
        CCT AAT ATT GAG GAG GTG GCC TTG TCC AAC ACT GGA GAG ATC CCC TTC     672
        Pro Asn Ile Glu Glu Val Ala Leu Ser Asn Thr Gly Glu Ile Pro Phe
            210                           215                           220
        TAT GGC AAG GCC ATC CCC CTC GAG GCC ATC AAG GGG GGG AGG CAT CTC     720
        Tyr Gly Lys Ala Ile Pro Leu Glu Ala Ile Lys Gly Gly Arg His Leu
        225                           230                           235                 240
        ATT TTC TGC CAT TCC AAG AAG AAA TGT GAC GAG CTC GCT GCG AAG CTG     768
        Ile Phe Cys His Ser Lys Lys Lys Cys Asp Glu Leu Ala Ala Lys Leu
                        245                           250                           255
        TCG GCC CTC GGA GTC AAC GCT GTA GCA TAT TAC CGG GGT CTT GAT GTG     816
        Ser Ala Leu Gly Val Asn Ala Val Ala Tyr Tyr Arg Gly Leu Asp Val
                    260                           265                           270
        TCC ATC ATA CCG ACA AGC GGG GAC GTC GTT GTC GTG GCA ACA GAC GCT     864
        Ser Ile Ile Pro Thr Ser Gly Asp Val Val Val Val Ala Thr Asp Ala
                275                           280                           285
        CTA ATG ACG GGC TAT ACC GGT GAC TTT GAC TCG GTG ATC GAC TGC AAC     912
        Leu Met Thr Gly Tyr Thr Gly Asp Phe Asp Ser Val Ile Asp Cys Asn
            290                           295                           300
        ACA TGT GTC                                                         921
        Thr Cys Val
        305
```

SEQ ID NO:51
SEQUENCE LENGTH: 921 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N23-2

```
CTG CTG TCG CCC GGG CCC ATC TCC TAC CTG AAG GGT TCC TCG GGT GGT    48
Leu Leu Ser Pro Gly Pro Ile Ser Tyr Leu Lys Gly Ser Ser Gly Gly
  1               5                  10                  15
CCG CTG CTT TGC CCC TCG GGC CAT GTT GTG GGC ATC TTC CGG GCT GCT    96
Pro Leu Leu Cys Pro Ser Gly His Val Val Gly Ile Phe Arg Ala Ala
                 20                  25                  30
GTG TGC ACC CGG GGG GTT GCG AAG GCG GTA GAC TTT GTG CCC GTT GAG   144
Val Cys Thr Arg Gly Val Ala Lys Ala Val Asp Phe Val Pro Val Glu
             35                  40                  45
TCT ATG GAA ACC ACT ATG CGG TCT CCG GTC TTC ACG GAT AAC TCA ACC   192
Ser Met Glu Thr Thr Met Arg Ser Pro Val Phe Thr Asp Asn Ser Thr
         50                  55                  60
CCC CCG GCC GTA CCG CAG TCA TTC CAA GTG GCC CAC CTA CAC GCT CCC   240
Pro Pro Ala Val Pro Gln Ser Phe Gln Val Ala His Leu His Ala Pro
 65                  70                  75                  80
ACT GGC AGC GGC AAA AGC ACC AAG GTG CCG GCT GCG TAT GCG GCC CAA   288
Thr Gly Ser Gly Lys Ser Thr Lys Val Pro Ala Ala Tyr Ala Ala Gln
                     85                  90                  95
GGG TAC AAG GTA CTC GTC CTG AAC CCG TCC GTT GCT GCC ACT TTG GGC   336
Gly Tyr Lys Val Leu Val Leu Asn Pro Ser Val Ala Ala Thr Leu Gly
                100                 105                 110
TTT GGG GCG TAT ATG TCC AAG GCA CAT GGT GTT GAC CCT AAC ATC AGA   384
Phe Gly Ala Tyr Met Ser Lys Ala His Gly Val Asp Pro Asn Ile Arg
            115                 120                 125
ACT GGG GTG AGG ACC ATC ACC ACG GGC GCT CCC ATC ACG TAC TCC ACC   432
Thr Gly Val Arg Thr Ile Thr Thr Gly Ala Pro Ile Thr Tyr Ser Thr
        130                 135                 140
TAC GGT AAG TTC CTC GCC GAC GGT GGC TGT TCT GGG GGT GCC TAT GAC   480
Tyr Gly Lys Phe Leu Ala Asp Gly Gly Cys Ser Gly Gly Ala Tyr Asp
145                 150                 155                 160
ATC ATA ATA TGT GAT GAG TGT CAT TCA ACT GAC TCG ACT TCC ATC TTG   528
Ile Ile Ile Cys Asp Glu Cys His Ser Thr Asp Ser Thr Ser Ile Leu
                165                 170                 175
GGC ATT GGT ACA GTC CTG GAC CAA GCG GAG ACG GCT GGA GCG CGC CTT   576
Gly Ile Gly Thr Val Leu Asp Gln Ala Glu Thr Ala Gly Ala Arg Leu
            180                 185                 190
GTC GTG CTC GCC ACC GCT ACG CCT CCG GGA TCG GTC ACC GTG CCG CAT   624
```

Val Val Leu Ala Thr Ala Thr Pro Pro Gly Ser Val Thr Val Pro His
195 200 205

CCT AAT ATT GAG GAG GTG GCC TTG TCC AAC ACT GGA GAG ATC CCC TTC 672
Pro Asn Ile Glu Glu Val Ala Leu Ser Asn Thr Gly Glu Ile Pro Phe
210 215 220

TAT GGC AAG GCC ATC CCC CTC GAG GCC ATC AAG GGG GGG AGG CAT CTC 720
Tyr Gly Lys Ala Ile Pro Leu Glu Ala Ile Lys Gly Gly Arg His Leu
225 230 235 240

ACT TTC TGC CAT TCC AAG AAG AAA TGT GAC GAG CTC GCT GCG AAG CTG 768
Thr Phe Cys His Ser Lys Lys Lys Cys Asp Glu Leu Ala Ala Lys Leu
245 250 255

TCG GCC CTC GGA GTC AAC GCT GTA GCA TAC TAC CGG GGT CTT GAT GTG 816
Ser Ala Leu Gly Val Asn Ala Val Ala Tyr Tyr Arg Gly Leu Asp Val
260 265 270

TCC GTC ATA CCG ACA AGC GGG GAC GTC GTT GTC GTG GCA ACT GAC GCT 864
Ser Val Ile Pro Thr Ser Gly Asp Val Val Val Val Ala Thr Asp Ala
275 280 285

CTA ATG ACG GGC TAT ACC GGT GAC TTT GAC TCA GTG ATC GAC TGC AAC 912
Leu Met Thr Gly Tyr Thr Gly Asp Phe Asp Ser Val Ile Asp Cys Asn
290 295 300

ACA TGT GTC 921
Thr Cys Val
305

SEQ ID NO:52
SEQUENCE LENGTH: 921 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N23-3

CTG CTG TCG CCC GGG CCC ATC TCT TAC TTG AAG GGC TCC TCG GGT GGT 48
Leu Leu Ser Pro Gly Pro Ile Ser Tyr Leu Lys Gly Ser Ser Gly Gly

```
 1               5                  10                  15
CCG CTG CTT TGC CCC TCG GGC CAT GTT GTG GGC ATC TTC CGG GCT GCC    96
Pro Leu Leu Cys Pro Ser Gly His Val Val Gly Ile Phe Arg Ala Ala
            20                  25                  30
GTG TGC ACC CGG GGG GTT GCG AAG GCG GTG GAC TTT GTG CCC GTT GAG   144
Val Cys Thr Arg Gly Val Ala Lys Ala Val Asp Phe Val Pro Val Glu
        35                  40                  45
TCT ATG GAA ACC ACC ATG CGG TCT CCG GTC TTC GCG GAT AAC TCA ACC   192
Ser Met Glu Thr Thr Met Arg Ser Pro Val Phe Ala Asp Asn Ser Thr
    50                  55                  60
CCC CCG GCC GTA CCG CAG ACA TTC CAA GTG GCC CAC CTA CAC GCT CCC   240
Pro Pro Ala Val Pro Gln Thr Phe Gln Val Ala His Leu His Ala Pro
65                  70                  75                      80
ACT GGC AGC GGC AAA AGC ACC AGG GTG CCG GCT GCG TAT GCG GCC CAA   288
Thr Gly Ser Gly Lys Ser Thr Arg Val Pro Ala Ala Tyr Ala Ala Gln
                85                  90                  95
GGG TAC AAG GTA CTC GTC CTG AAC CCG TCC GTT GCT GCC ACT TTG GGC   336
Gly Tyr Lys Val Leu Val Leu Asn Pro Ser Val Ala Ala Thr Leu Gly
            100                 105                 110
TTT GGG GCG TAC ATG TCC AAG GCA CAT GGT GTT GAC CCT AAC ATC AGA   384
Phe Gly Ala Tyr Met Ser Lys Ala His Gly Val Asp Pro Asn Ile Arg
        115                 120                 125
ACT GGG GTG AGG ACC ATC ACC ACG GGC GCT CCC GTC ACG TAC TCC ACC   432
Thr Gly Val Arg Thr Ile Thr Thr Gly Ala Pro Val Thr Tyr Ser Thr
    130                 135                 140
TAC GGT AAG TTC CTC GCC GAC GGT GGC TGT TCT GGG GGT GCC TAT GAC   480
Tyr Gly Lys Phe Leu Ala Asp Gly Gly Cys Ser Gly Gly Ala Tyr Asp
145                 150                 155                     160
ATC ATA ATA TGT GAT GAG TGT CAT TCA ACT GAC TCG ACT TCC ATC TTG   528
Ile Ile Ile Cys Asp Glu Cys His Ser Thr Asp Ser Thr Ser Ile Leu
                165                 170                 175
GGC ATT GGT ACA GTC CTG GAC CAA GCG GAG ACG GCT GGA GCG CGC CTT   576
Gly Ile Gly Thr Val Leu Asp Gln Ala Glu Thr Ala Gly Ala Arg Leu
            180                 185                 190
GTC GTG CTC GCC ACC GCT ACG CCT CCG GGA TCG GTC ACC GTG CCG CAT   624
Val Val Leu Ala Thr Ala Thr Pro Pro Gly Ser Val Thr Val Pro His
        195                 200                 205
```

```
CCT AAT ATT GAG GAG GTG GCC TTG TCC AAC ACT GGA GAG ATC CCC TTC   672
Pro Asn Ile Glu Glu Val Ala Leu Ser Asn Thr Gly Glu Ile Pro Phe
    210                 215                 220
TAT GGC AAG GCC ATC CCC CTC GAG GCC ATC AAG GGG GGG AGG CAT CTC   720
Tyr Gly Lys Ala Ile Pro Leu Glu Ala Ile Lys Gly Gly Arg His Leu
225                 230                 235                 240
ATT TTC TGC CAT TCC AAG AAG AAA TGT GAC GAG CTC GCT GCG AAG CTG   768
Ile Phe Cys His Ser Lys Lys Lys Cys Asp Glu Leu Ala Ala Lys Leu
                245                 250                 255
TCG GCC CTC GGA GTC AAT GCT GTA GCA TAT TAC CGG GGT CTT GAT GTG   816
Ser Ala Leu Gly Val Asn Ala Val Ala Tyr Tyr Arg Gly Leu Asp Val
            260                 265                 270
TCC ATC ATA CCG ACA AGC GGG GAC GTC GTT GTC GTG GCA ACA GAC GCT   864
Ser Ile Ile Pro Thr Ser Gly Asp Val Val Val Val Ala Thr Asp Ala
        275                 280                 285
CTA ATG ACG GGC TAT ACC GGT GAC TTT GAC TCG GTG ATC GAC TGT AAC   912
Leu Met Thr Gly Tyr Thr Gly Asp Phe Asp Ser Val Ile Asp Cys Asn
    290                 295                 300
ACA TGT GTC                                                       921
Thr Cys Val
305
```

SEQ ID NO:53
SEQUENCE LENGTH: 623 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N16-1

```
GGC TAT ACC GGC GAC TTC GAC TCA GTG ATC GAC TGC AAC ACA TGT GTC   48
Gly Tyr Thr Gly Asp Phe Asp Ser Val Ile Asp Cys Asn Thr Cys Val
  1               5                  10                  15
ACC CAA ACA GTC GAT TTC AGC TTG GAC CCT ACT TTC ACC ATC GAG ACG   96
```

```
Thr Gln Thr Val Asp Phe Ser Leu Asp Pro Thr Phe Thr Ile Glu Thr
                20                  25                  30
ACG ACC GTA CCC CAA GAT GCG GTG TCG CGC TCG CAG CGG CGA GGC AGG    144
Thr Thr Val Pro Gln Asp Ala Val Ser Arg Ser Gln Arg Arg Gly Arg
            35                  40                  45
ACT GGT AGG GGC AGG GGG GGC ATA TAC AGG TTT GTA ACT CCA GGG GAA    192
Thr Gly Arg Gly Arg Gly Gly Ile Tyr Arg Phe Val Thr Pro Gly Glu
        50                  55                  60
CGG CCC TCA GGC ATG TTC GAT TCT TCG GTC CTG TGT GAA TGT TAT GAC    240
Arg Pro Ser Gly Met Phe Asp Ser Ser Val Leu Cys Glu Cys Tyr Asp
65                  70                  75                  80
GCG GGC TGT GCT TGG TAC GAG CTC ACG CCC GCC GAG ACC TCG GTT AGG    288
Ala Gly Cys Ala Trp Tyr Glu Leu Thr Pro Ala Glu Thr Ser Val Arg
                85                  90                  95
TTG CGG GCT TAC CTA AAT ACA CCT GGG CTG CCC GTC TGC CAG GAC CAT    336
Leu Arg Ala Tyr Leu Asn Thr Pro Gly Leu Pro Val Cys Gln Asp His
            100                 105                 110
CTG GAG TTC TGG GAG AGC GTC TTC ACC GGC CTC ACC CAC ATA GAT GCC    384
Leu Glu Phe Trp Glu Ser Val Phe Thr Gly Leu Thr His Ile Asp Ala
        115                 120                 125
CAC TTC TTG TCC CAG ACC AAA CAG GCA GGA GAC AAC TTC CCC TAC CTG    432
His Phe Leu Ser Gln Thr Lys Gln Ala Gly Asp Asn Phe Pro Tyr Leu
    130                 135                 140
GTA GCA TAC CAG GCT ACA GTG TGC GCC AGG GCC AAG GCT CCA CCT CCA    480
Val Ala Tyr Gln Ala Thr Val Cys Ala Arg Ala Lys Ala Pro Pro Pro
145                 150                 155                 160
TCG TGG GAT CAG ATG TGG AAG TGT CTC ATA CGG CTG AAG CCT ACG CTA    528
Ser Trp Asp Gln Met Trp Lys Cys Leu Ile Arg Leu Lys Pro Thr Leu
                165                 170                 175
CAC GGG CCA ACG CCC CTG TTG TAT AGG TTA GGA GCC GTT CAG AAC GAG    576
His Gly Pro Thr Pro Leu Leu Tyr Arg Leu Gly Ala Val Gln Asn Glu
            180                 185                 190
GTT ACC CTT ACA CAC CCC ATA ACC AAG TAC ATC ATG ACA TGC ATG TC     623
Val Thr Leu Thr His Pro Ile Thr Lys Tyr Ile Met Thr Cys Met
        195                 200                 205
```

SEQ ID NO:54

SEQUENCE LENGTH: 623 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N16-2

```
GGC TAT ACC GGC GAC TTC GAC TCA GTG ATC GAC TGC AAC ACA TGT GTC    48
Gly Tyr Thr Gly Asp Phe Asp Ser Val Ile Asp Cys Asn Thr Cys Val
  1               5                  10                  15
ACC CAA ACA GTC GAT TTC AGC TTG GAC CCT ACT TTC ACC ATC GAG ACG    96
Thr Gln Thr Val Asp Phe Ser Leu Asp Pro Thr Phe Thr Ile Glu Thr
                20                  25                  30
ACG ACC GTA CCC CAA GAT GCG GTG TCG CGC TCG CAG CGG CGA GGC AGG   144
Thr Thr Val Pro Gln Asp Ala Val Ser Arg Ser Gln Arg Arg Gly Arg
            35                  40                  45
ACT GGT AGG GGC AGG GGG GGC ATA TAC AGG TTT GTA ACT CCA GGG GAA   192
Thr Gly Arg Gly Arg Gly Gly Ile Tyr Arg Phe Val Thr Pro Gly Glu
        50                  55                  60
CGG CCC TCA GGC ATG TTC GAT TCT TCG GTC CTG TGT GAA TGT TAT GAC   240
Arg Pro Ser Gly Met Phe Asp Ser Ser Val Leu Cys Glu Cys Tyr Asp
 65                  70                  75                  80
GCG GGC TGT GCT TGG TAC GAG CTC ACG CCC GCC GAG ACC TCG GTT AGG   288
Ala Gly Cys Ala Trp Tyr Glu Leu Thr Pro Ala Glu Thr Ser Val Arg
                 85                  90                  95
TTG CGG GCT TAC CTA AAT ACA CCT GGG CTG CCC GTC TGC CAG GAC CAT   336
Leu Arg Ala Tyr Leu Asn Thr Pro Gly Leu Pro Val Cys Gln Asp His
            100                 105                 110
CTG GAG TTC TGG GAG AGC GTC TTC ACC GGC CTC ACC CAC ATA GAT GCC   384
Leu Glu Phe Trp Glu Ser Val Phe Thr Gly Leu Thr His Ile Asp Ala
        115                 120                 125
CAC TTC TTG TCC CAG ACC AAA CAG GCA GGA GAC AAC TTC CCC TAC CTG   432
His Phe Leu Ser Gln Thr Lys Gln Ala Gly Asp Asn Phe Pro Tyr Leu
    130                 135                 140
```

```
GTA GCA TAC CAG GCT ACA GTG TGC GCC AGG GCC AAG GCT CCA CCT CCA   480
Val Ala Tyr Gln Ala Thr Val Cys Ala Arg Ala Lys Ala Pro Pro Pro
145                 150                 155                 160
TCG TGG GAT CAG ATG TGG AAG TGT CTC ATA CGG CTG AAG CCT ACG CTA   528
Ser Trp Asp Gln Met Trp Lys Cys Leu Ile Arg Leu Lys Pro Thr Leu
                165                 170                 175
CAC GGG CCA ACG CCC CTG TTG TAT AGG TTA GGA GCC GTT CAG AAC GAG   576
His Gly Pro Thr Pro Leu Leu Tyr Arg Leu Gly Ala Val Gln Asn Glu
            180                 185                 190
GTT ACC CTC ACA CAC CCT ATA ACC AAG TAC ATC ATG ACA TGC ATG TC    623
Val Thr Leu Thr His Pro Ile Thr Lys Tyr Ile Met Thr Cys Met
        195                 200                 205
```

SEQ ID NO:55
SEQUENCE LENGTH: 623 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N16-3

```
GGC TAT ACC GGC GAC TTC GAC TCA GTG ATC GAC TGC AAC ACA TGT GTC   48
Gly Tyr Thr Gly Asp Phe Asp Ser Val Ile Asp Cys Asn Thr Cys Val
  1               5                  10                  15
ACC CAA ACA GTC GAT TTC AGC TTG GAC CCT ACT TTC ACC ATT GAG ACG   96
Thr Gln Thr Val Asp Phe Ser Leu Asp Pro Thr Phe Thr Ile Glu Thr
            20                  25                  30
ACG ACC GTA CCC CAA GAT GCG GTG TCG CGC TCG CAG CGG CGA GGC AGG   144
Thr Thr Val Pro Gln Asp Ala Val Ser Arg Ser Gln Arg Arg Gly Arg
        35                  40                  45
ACT GGT AGG GGC AGA GGG GGC ATA TAC AGG TTT GTA ACT CCA GGG GAA   192
Thr Gly Arg Gly Arg Gly Gly Ile Tyr Arg Phe Val Thr Pro Gly Glu
    50                  55                  60
CGG CCC TCA GGC ATG TTC GAT TCT TCG GTC CTG TGT GAA TGT TAT GAC   240
```

```
Arg Pro Ser Gly Met Phe Asp Ser Ser Val Leu Cys Glu Cys Tyr Asp
 65                 70                  75                  80
GCG GGC TGT GCT TGG TAC GAG CTC ACG TCC GCC GAG ACC TCG GTT AGG     288
Ala Gly Cys Ala Trp Tyr Glu Leu Thr Ser Ala Glu Thr Ser Val Arg
                85                  90                  95
TTG CGG GCT TAC CTA AAC ACA CCT GGG CTG CCC GTC TGC CAG GAC CAT     336
Leu Arg Ala Tyr Leu Asn Thr Pro Gly Leu Pro Val Cys Gln Asp His
            100                 105                 110
CTG GAG TTC TGG GAG AGC GTC TTC ACC GGC CTC ACC CAC ATA GAT GCC     384
Leu Glu Phe Trp Glu Ser Val Phe Thr Gly Leu Thr His Ile Asp Ala
        115                 120                 125
CAC TTC TTG TCC CAG ACT AAA CAG GCA GGA GAC AAC TTC CCC TAC CTG     432
His Phe Leu Ser Gln Thr Lys Gln Ala Gly Asp Asn Phe Pro Tyr Leu
    130                 135                 140
GTA GCA TAC CAG GCT ACA GTG TGC GCC AGG GCC AAG GCT CCA CCT CCA     480
Val Ala Tyr Gln Ala Thr Val Cys Ala Arg Ala Lys Ala Pro Pro Pro
145                 150                 155                 160
TCG TGG GAT CAG ATG TGG AAG TGT CTC ATA CGG CTG AAG CCT ACG CTA     528
Ser Trp Asp Gln Met Trp Lys Cys Leu Ile Arg Leu Lys Pro Thr Leu
                165                 170                 175
CAC GGG CCA ACG CCC CTG TTG CAT AGG TTA GGA GCC GTT CAG AAC AAG     576
His Gly Pro Thr Pro Leu Leu His Arg Leu Gly Ala Val Gln Asn Lys
            180                 185                 190
GTT GCC CTC ACA CAC CCC ATA ACC AAG TAC ATC ATG ACA TGC ATG TC      623
Val Ala Leu Thr His Pro Ile Thr Lys Tyr Ile Met Thr Cys Met
        195                 200                 205
```

SEQ ID NO:56
SEQUENCE LENGTH: 1280 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX25026A-1

```
TGT GCC TGG TTG TGG ATG ATG CTG CTG ATA GCC CAA GCT GAG GCC GCC    48
Cys Ala Trp Leu Trp Met Met Leu Leu Ile Ala Gln Ala Glu Ala Ala
  1               5                  10                  15
TTG GAG AAC CTG GTG GTC CTC AAT GCA GCA TCC ATG GCG GGA GCG CAT    96
Leu Glu Asn Leu Val Val Leu Asn Ala Ala Ser Met Ala Gly Ala His
             20                  25                  30
GGC ATC CTC TCT TTC CTT GTG TTC TTC TGT GCC GCC TGG TAC ATC AAA   144
Gly Ile Leu Ser Phe Leu Val Phe Phe Cys Ala Ala Trp Tyr Ile Lys
         35                  40                  45
GGC AGG CTG GTC CCT GGG GCG GCA TAC GCT TTC TAT GGC GTA TGG CCG   192
Gly Arg Leu Val Pro Gly Ala Ala Tyr Ala Phe Tyr Gly Val Trp Pro
     50                  55                  60
CTG CTC CTG CTC TTG ATG GCG CTA CCC GCA CGG GCG TAC GCC ATG GAC   240
Leu Leu Leu Leu Leu Met Ala Leu Pro Ala Arg Ala Tyr Ala Met Asp
 65                  70                  75                  80
CGG GAG ATG GCT GCA TCG TGC GGA GGC GCG GTT TTT GTA GGT CTG GTA   288
Arg Glu Met Ala Ala Ser Cys Gly Gly Ala Val Phe Val Gly Leu Val
                 85                  90                  95
CTC TTG ACC TTG TCA CCA TAC TAC AAA GTG TTC CTC GCT AAG CTC ATA   336
Leu Leu Thr Leu Ser Pro Tyr Tyr Lys Val Phe Leu Ala Lys Leu Ile
            100                 105                 110
TGG TGG TTG CAA TAT CTC ATC ACC AGG GCC GAG GCG CAC TTG CAA GTG   384
Trp Trp Leu Gln Tyr Leu Ile Thr Arg Ala Glu Ala His Leu Gln Val
        115                 120                 125
TGG ATC CCC CCC CTC AAC GTT CGG GGG GGC CGC GAT GCC ATC ATC CTT   432
Trp Ile Pro Pro Leu Asn Val Arg Gly Gly Arg Asp Ala Ile Ile Leu
    130                 135                 140
CTC ACA TGT GCG GTC CAC CCG GAG CTG ATC TTT GAC ATC ACC AAG CTC   480
Leu Thr Cys Ala Val His Pro Glu Leu Ile Phe Asp Ile Thr Lys Leu
145                 150                 155                 160
TTG CTC GCC ATA CTC GGT CCG CTC ATG GTA CTC CAG GCT GGC CTA ACC   528
Leu Leu Ala Ile Leu Gly Pro Leu Met Val Leu Gln Ala Gly Leu Thr
                165                 170                 175
CAA ATG CCG TAC TTT GTG CGT GCT CAA GGG CTC ATT CGT ATG TGC ATG   576
Gln Met Pro Tyr Phe Val Arg Ala Gln Gly Leu Ile Arg Met Cys Met
            180                 185                 190
TTG GTG CGG AAA GCC GCT GGG GGT CAT TAT GTC CAG ATG GCT CTC ATG   624
```

Leu Val Arg Lys Ala Ala Gly Gly His Tyr Val Gln Met Ala Leu Met
195 200 205
AAG CTG GCT GCA CTG ACA GGT ACG TAC GTT TAT GAC CAT CTT ACT CCA 672
Lys Leu Ala Ala Leu Thr Gly Thr Tyr Val Tyr Asp His Leu Thr Pro
210 215 220
CTG CAG GAC TGG GCC CAC GCG GGC CTA CGA GAC CTT GCG GTA GCA GTT 720
Leu Gln Asp Trp Ala His Ala Gly Leu Arg Asp Leu Ala Val Ala Val
225 230 235 240
GAG CCC GTT GCC TTC TCT GAT ATG GAG ACT AAG ATC ATC ACC TGG GGG 768
Glu Pro Val Ala Phe Ser Asp Met Glu Thr Lys Ile Ile Thr Trp Gly
245 250 255
GCA GAC ACT GCG GCG TGT GGG GAC ATC ATT TTG GGC CTA CCT GTC TCC 816
Ala Asp Thr Ala Ala Cys Gly Asp Ile Ile Leu Gly Leu Pro Val Ser
260 265 270
GCC CGG AGG GGC AAC GAG ATA CTC CTC GGA CCG GCC GAT AGT TTT GAC 864
Ala Arg Arg Gly Asn Glu Ile Leu Leu Gly Pro Ala Asp Ser Phe Asp
275 280 285
GGG CAG GGG TGG CGA CTC CTT GCG CCT ATC ACG GCC TAC TCC CAG CAG 912
Gly Gln Gly Trp Arg Leu Leu Ala Pro Ile Thr Ala Tyr Ser Gln Gln
290 295 300
ACG CGG GGC CTG CTT GGT TGC ATC ATC ACT AGC CTT ACG GGC CGG GAT 960
Thr Arg Gly Leu Leu Gly Cys Ile Ile Thr Ser Leu Thr Gly Arg Asp
305 310 315 320
AAG AAC CAG GTC GAG GGG GAG GTT CAA GTG GTC TCT ACC GCA ACA CAA 1008
Lys Asn Gln Val Glu Gly Glu Val Gln Val Val Ser Thr Ala Thr Gln
325 330 335
TCT TTC CTG GCG ACC TGT GTC AAC GGC GTG TGC TGG ACT GTT TTC CAC 1056
Ser Phe Leu Ala Thr Cys Val Asn Gly Val Cys Trp Thr Val Phe His
340 345 350
GGC GCC GGC TCG AAG ACC TTA GCC GGC CCA AAA GGC CCA ATC ACC CAA 1104
Gly Ala Gly Ser Lys Thr Leu Ala Gly Pro Lys Gly Pro Ile Thr Gln
355 360 365
ATG TAC ACC AAT GTA GAT CAG GAC CTC GTC GGC TGG TCG GCG CCC CCC 1152
Met Tyr Thr Asn Val Asp Gln Asp Leu Val Gly Trp Ser Ala Pro Pro
370 375 380
CGG GCG CGT TCC TTG ACA CCT TGC ACC TGC GGC AGC TCG GAC CTT TAT 1200
Arg Ala Arg Ser Leu Thr Pro Cys Thr Cys Gly Ser Ser Asp Leu Tyr

```
385                 390                 395                 400
TTG GTC ACG AGG CAT GCT GAT GTC ATT CCG GTG CAC CGG CGG GGC GAC  1248
Leu Val Thr Arg His Ala Asp Val Ile Pro Val His Arg Arg Gly Asp
                405                 410                 415
AGC AGG GGG AGC CTC CTC TCC CCC GGG CCC AT                       1280
Ser Arg Gly Ser Leu Leu Ser Pro Gly Pro
            420                 425
```

SEQ ID NO:57
SEQUENCE LENGTH: 1280 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX25026B-1

```
TGT GCC TGG TTG TGG ATG ATG CTG CTG ATA GCC CAA GCT GAG GCC GCC   48
Cys Ala Trp Leu Trp Met Met Leu Leu Ile Ala Gln Ala Glu Ala Ala
  1               5                  10                  15
TTG GAG AAC CTG GTG GTC CTC AAT GCA GCA TCC ATG GCG GGA GCG CAT   96
Leu Glu Asn Leu Val Val Leu Asn Ala Ala Ser Met Ala Gly Ala His
                20                  25                  30
GGC ATC CTC TCT TTC CTT GTG TTC TTC TGT GCC GCC TGG TAC ATC AAA  144
Gly Ile Leu Ser Phe Leu Val Phe Phe Cys Ala Ala Trp Tyr Ile Lys
            35                  40                  45
GGC AGG CTG GTC CCT GGG GCG GCA TAC GCT TTC TAT GGC GTA TGG CCG  192
Gly Arg Leu Val Pro Gly Ala Ala Tyr Ala Phe Tyr Gly Val Trp Pro
        50                  55                  60
CTG CTC CTG CTC TTG ATG GCG CTA CCC GCA CGG GCG TAC GCC ATG GAC  240
Leu Leu Leu Leu Leu Met Ala Leu Pro Ala Arg Ala Tyr Ala Met Asp
 65                  70                  75                  80
CGG GAG ATG GCT GCA TCG TGC GGA GGC GCG GTT TTT GTA GGT CTG GTA  288
Arg Glu Met Ala Ala Ser Cys Gly Gly Ala Val Phe Val Gly Leu Val
                85                  90                  95
```

```
CTC TTG ACC TTG TCA CCA TAC TAC AAA GTG TTC CTC GCT AAG CTC ATA    336
Leu Leu Thr Leu Ser Pro Tyr Tyr Lys Val Phe Leu Ala Lys Leu Ile
            100                 105                 110
TGG TGG TTG CAA TAT CTC ATC ACC AGG GCC GAG GCG CAC TTG CAA GTG    384
Trp Trp Leu Gln Tyr Leu Ile Thr Arg Ala Glu Ala His Leu Gln Val
        115                 120                 125
TGG ATC CCC CCC CTC AAC GTT CGG GGG GGC CGC GAT GCC ATC ATC CTT    432
Trp Ile Pro Pro Leu Asn Val Arg Gly Gly Arg Asp Ala Ile Ile Leu
    130                 135                 140
CTC ACA TGT GCG GTC CAC CCG GAG CTG ATC TTT GAC ATC ACC AAG CTC    480
Leu Thr Cys Ala Val His Pro Glu Leu Ile Phe Asp Ile Thr Lys Leu
145                 150                 155                 160
TTG CTC GCC ATA CTC GGT CCG CTC ATG GTA CTC CAG GCT GGC CTA ACC    528
Leu Leu Ala Ile Leu Gly Pro Leu Met Val Leu Gln Ala Gly Leu Thr
                165                 170                 175
CAA ATG CCG TAC TTT GTG CGT GCT CAA GGG CTC ATT CGT ATG TGC ATG    576
Gln Met Pro Tyr Phe Val Arg Ala Gln Gly Leu Ile Arg Met Cys Met
            180                 185                 190
TTG GTG CGG AAA GCC GCT GGG GGT CAT TAT GTC CAG ATG GCT CTC ATG    624
Leu Val Arg Lys Ala Ala Gly Gly His Tyr Val Gln Met Ala Leu Met
        195                 200                 205
AAG CTG GCT GCA CTG ACA GGT ACG TAC GTT TAT GAC CAT CTT ACT CCA    672
Lys Leu Ala Ala Leu Thr Gly Thr Tyr Val Tyr Asp His Leu Thr Pro
    210                 215                 220
CTG CAG GAC TGG GCC CAC GCG GGC CTA CGA GAC CTT GCG GTA GCA GTT    720
Leu Gln Asp Trp Ala His Ala Gly Leu Arg Asp Leu Ala Val Ala Val
225                 230                 235                 240
GAG CCC GTT GCC TTC TCT GAT ATG GAG ACT AAG ATC ATC ACG TGG GGG    768
Glu Pro Val Ala Phe Ser Asp Met Glu Thr Lys Ile Ile Thr Trp Gly
                245                 250                 255
GCA GAG ACG GCG GCG TGT GGG GAC ATC ATC TCG GGT CTA CCC GTT TCC    816
Ala Glu Thr Ala Ala Cys Gly Asp Ile Ile Ser Gly Leu Pro Val Ser
            260                 265                 270
GCC CGA AGG GGG AGG GAG CTG CTT TTG GGG CCG GCC GAT AGT TTT GAC    864
Ala Arg Arg Gly Arg Glu Leu Leu Leu Gly Pro Ala Asp Ser Phe Asp
        275                 280                 285
GGG CAG GGG TGG CGA CTC CTT GCG CCT ATC ACG GCC TAC TCC CAG CAG    912
```

Gly Gln Gly Trp Arg Leu Leu Ala Pro Ile Thr Ala Tyr Ser Gln Gln
290 295 300

ACG CGG GGC CTG CTT GGT TGC ATC ATC ACT AGC CTT ACG GGC CGG GAT 960
Thr Arg Gly Leu Leu Gly Cys Ile Ile Thr Ser Leu Thr Gly Arg Asp
305 310 315 320

AAG AAC CAG GTC GAG GGG GAG GTT CAA GTG GTC TCT ACC GCA ACA CAA 1008
Lys Asn Gln Val Glu Gly Glu Val Gln Val Val Ser Thr Ala Thr Gln
325 330 335

TCT TTC CTG GCG ACC TGT GTC AAC GGC GTG TGC TGG ACT GTT TTC CAC 1056
Ser Phe Leu Ala Thr Cys Val Asn Gly Val Cys Trp Thr Val Phe His
340 345 350

GGC GCC GGC TCG AAG ACC TTA GCC GGC CCA AAA GGC CCA ATC ACC CAA 1104
Gly Ala Gly Ser Lys Thr Leu Ala Gly Pro Lys Gly Pro Ile Thr Gln
355 360 365

ATG TAC ACC AAT GTA GAT CAG GAC CTC GTC GGC TGG TCG GCG CCC CCC 1152
Met Tyr Thr Asn Val Asp Gln Asp Leu Val Gly Trp Ser Ala Pro Pro
370 375 380

CGG GCG CGT TCC TTG ACA CCT TGC ACC TGC GGC AGC TCG GAC CTT TAT 1200
Arg Ala Arg Ser Leu Thr Pro Cys Thr Cys Gly Ser Ser Asp Leu Tyr
385 390 395 400

TTG GTC ACG AGG CAT GCT GAT GTC ATT CCG GTG CAC CGG CGG GGC GAC 1248
Leu Val Thr Arg His Ala Asp Val Ile Pro Val His Arg Arg Gly Asp
405 410 415

AGC AGG GGG AGC CTC CTC TCC CCC GGG CCC AT 1280
Ser Arg Gly Ser Leu Leu Ser Pro Gly Pro
420 425

SEQ ID NO:58
SEQUENCE LENGTH: 1431 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N16N15A-1

```
GGC TAT ACC GGC GAC TTC GAC TCA GTG ATC GAC TGC AAC ACA TGT GTC    48
Gly Tyr Thr Gly Asp Phe Asp Ser Val Ile Asp Cys Asn Thr Cys Val
 1               5                  10                  15
ACC CAA ACA GTC GAT TTC AGC TTG GAC CCT ACT TTC ACC ATC GAG ACG    96
Thr Gln Thr Val Asp Phe Ser Leu Asp Pro Thr Phe Thr Ile Glu Thr
            20                  25                  30
ACG ACC GTA CCC CAA GAT GCG GTG TCG CGC TCG CAG CGG CGA GGC AGG   144
Thr Thr Val Pro Gln Asp Ala Val Ser Arg Ser Gln Arg Arg Gly Arg
        35                  40                  45
ACT GGT AGG GGC AGG GGG GGC ATA TAC AGG TTT GTA ACT CCA GGG GAA   192
Thr Gly Arg Gly Arg Gly Gly Ile Tyr Arg Phe Val Thr Pro Gly Glu
    50                  55                  60
CGG CCC TCA GGC ATG TTC GAT TCT TCG GTC CTG TGT GAA TGT TAT GAC   240
Arg Pro Ser Gly Met Phe Asp Ser Ser Val Leu Cys Glu Cys Tyr Asp
65                  70                  75                  80
GCG GGC TGT GCT TGG TAC GAG CTC ACG CCC GCC GAG ACC TCG GTT AGG   288
Ala Gly Cys Ala Trp Tyr Glu Leu Thr Pro Ala Glu Thr Ser Val Arg
                85                  90                  95
TTG CGG GCT TAC CTA AAT ACA CCT GGG CTG CCC GTC TGC CAG GAC CAT   336
Leu Arg Ala Tyr Leu Asn Thr Pro Gly Leu Pro Val Cys Gln Asp His
            100                 105                 110
CTG GAG TTC TGG GAG AGC GTC TTC ACC GGC CTC ACC CAC ATA GAT GCC   384
Leu Glu Phe Trp Glu Ser Val Phe Thr Gly Leu Thr His Ile Asp Ala
        115                 120                 125
CAC TTC TTG TCC CAG ACC AAA CAG GCA GGA GAC AAC TTC CCC TAC CTG   432
His Phe Leu Ser Gln Thr Lys Gln Ala Gly Asp Asn Phe Pro Tyr Leu
    130                 135                 140
GTA GCA TAC CAG GCT ACA GTG TGC GCC AGG GCC AAG GCT CCA CCT CCA   480
Val Ala Tyr Gln Ala Thr Val Cys Ala Arg Ala Lys Ala Pro Pro Pro
145                 150                 155                 160
TCG TGG GAT CAG ATG TGG AAG TGT CTC ATA CGG CTG AAG CCT ACG CTA   528
Ser Trp Asp Gln Met Trp Lys Cys Leu Ile Arg Leu Lys Pro Thr Leu
                165                 170                 175
CAC GGG CCA ACG CCC CTG TTG TAT AGG TTA GGA GCC GTT CAG AAC GAG   576
His Gly Pro Thr Pro Leu Leu Tyr Arg Leu Gly Ala Val Gln Asn Glu
            180                 185                 190
GTT ACC CTT ACA CAC CCC ATA ACC AAG TAC ATC ATG ACA TGC ATG TCG   624
```

```
Val Thr Leu Thr His Pro Ile Thr Lys Tyr Ile Met Thr Cys Met Ser
        195                 200                 205
GCT GAC CTA GAG GTC GTC ACT AGC ACT TGG GTG CTG GTA GGC GGG GTC    672
Ala Asp Leu Glu Val Val Thr Ser Thr Trp Val Leu Val Gly Gly Val
    210                 215                 220
CTC GCG GCT CTG GCC GCG TAC TGC CTG ACA ACG GGC AGC GTG GTC ATT    720
Leu Ala Ala Leu Ala Ala Tyr Cys Leu Thr Thr Gly Ser Val Val Ile
225                 230                 235                 240
GTG GGC AGG ATC ATC TTG TCC GGG AGG CCG GCC GTT ATT CCC GAC AGG    768
Val Gly Arg Ile Ile Leu Ser Gly Arg Pro Ala Val Ile Pro Asp Arg
                245                 250                 255
GAA GTT CTC TAC CAA GAG TTC GAT GAA ATG GAA GAG TGC GCC TCG CAC    816
Glu Val Leu Tyr Gln Glu Phe Asp Glu Met Glu Glu Cys Ala Ser His
            260                 265                 270
CTC CCT TAC ATC GAA CAA GGA ATG CAG CTC GCC GAG CAA TTC AAG CAG    864
Leu Pro Tyr Ile Glu Gln Gly Met Gln Leu Ala Glu Gln Phe Lys Gln
        275                 280                 285
AAG GCG CTC GGT TTG CTG CAA ACA GCC ACC AAG CAA GCG GAG GCT GCT    912
Lys Ala Leu Gly Leu Leu Gln Thr Ala Thr Lys Gln Ala Glu Ala Ala
    290                 295                 300
GCT CCC GTG GTG GAG TCC AAG TGG CGA GCC CTT GAG ACC TTC TGG GCG    960
Ala Pro Val Val Glu Ser Lys Trp Arg Ala Leu Glu Thr Phe Trp Ala
305                 310                 315                 320
AAG CAC ATG TGG AAT TTC ATC AGC GGG ATA CAG TAC TTA GCA GGC TTG   1008
Lys His Met Trp Asn Phe Ile Ser Gly Ile Gln Tyr Leu Ala Gly Leu
                325                 330                 335
TCC ACT CTG CCT GGA AAC CCC GCA ATA GCA TCA CTG ATG GCA TTC ACA   1056
Ser Thr Leu Pro Gly Asn Pro Ala Ile Ala Ser Leu Met Ala Phe Thr
            340                 345                 350
GCC TCT ATC ACC AGC CCG CTC ACC ACC CAA TAT ACC CTC CTG TTT AAC   1104
Ala Ser Ile Thr Ser Pro Leu Thr Thr Gln Tyr Thr Leu Leu Phe Asn
        355                 360                 365
ATC TTG GGG GGA TGG GTG GCC GCC CAA CTC GCC CCC CCC AGT GCC GCT   1152
Ile Leu Gly Gly Trp Val Ala Ala Gln Leu Ala Pro Pro Ser Ala Ala
    370                 375                 380
TCA GCC TTC GTG GGC GCC GGT ATA GCT GGC GCG GCT GTT GGC AGC ATA   1200
Ser Ala Phe Val Gly Ala Gly Ile Ala Gly Ala Ala Val Gly Ser Ile
```

```
385                 390                 395                 400
GGC CTC GGG AAG GTG CTT GTG GAC ATT CTG GCG GGT TAT GGA GCA GGG  1248
Gly Leu Gly Lys Val Leu Val Asp Ile Leu Ala Gly Tyr Gly Ala Gly
                405                 410                 415
GTG GCA GGC GCG CTC GTG GCC TTT AAG GTC ATG AGC GGT GAC ATG CCC  1296
Val Ala Gly Ala Leu Val Ala Phe Lys Val Met Ser Gly Asp Met Pro
            420                 425                 430
TCC ACC GAG GAC CTG GTC AAC TTA CTC CCC GCC ATC CTC TCT CCT GGT  1344
Ser Thr Glu Asp Leu Val Asn Leu Leu Pro Ala Ile Leu Ser Pro Gly
        435                 440                 445
GCC CTG GTC GTC GGG GTC GTG TGC GCA GCA ATA CTG CGT CGG CAT GTG  1392
Ala Leu Val Val Gly Val Val Cys Ala Ala Ile Leu Arg Arg His Val
    450                 455                 460
GGC CCA GGG GAG GGG GCT GTG CAG TGG ATG AAC CGG CTG              1431
Gly Pro Gly Glu Gly Ala Val Gln Trp Met Asn Arg Leu
465                 470                 475
```

SEQ ID NO:59
SEQUENCE LENGTH: 1431 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N16N15B-1

```
GGC TAT ACC GGC GAC TTC GAC TCA GTG ATC GAC TGC AAC ACA TGT GTC    48
Gly Tyr Thr Gly Asp Phe Asp Ser Val Ile Asp Cys Asn Thr Cys Val
  1               5                  10                  15
ACC CAA ACA GTC GAT TTC AGC TTG GAC CCT ACT TTC ACC ATC GAG ACG    96
Thr Gln Thr Val Asp Phe Ser Leu Asp Pro Thr Phe Thr Ile Glu Thr
                20                  25                  30
ACG ACC GTA CCC CAA GAT GCG GTG TCG CGC TCG CAG CGG CGA GGC AGG   144
Thr Thr Val Pro Gln Asp Ala Val Ser Arg Ser Gln Arg Arg Gly Arg
            35                  40                  45
```

```
ACT GGT AGG GGC AGG GGG GGC ATA TAC AGG TTT GTA ACT CCA GGG GAA   192
Thr Gly Arg Gly Arg Gly Gly Ile Tyr Arg Phe Val Thr Pro Gly Glu
     50                  55                  60
CGG CCC TCA GGC ATG TTC GAT TCT TCG GTC CTG TGT GAA TGT TAT GAC   240
Arg Pro Ser Gly Met Phe Asp Ser Ser Val Leu Cys Glu Cys Tyr Asp
 65                  70                  75                  80
GCG GGC TGT GCT TGG TAC GAG CTC ACG CCC GCC GAG ACC TCG GTT AGG   288
Ala Gly Cys Ala Trp Tyr Glu Leu Thr Pro Ala Glu Thr Ser Val Arg
                 85                  90                  95
TTG CGG GCT TAC CTA AAT ACA CCT GGG CTG CCC GTC TGC CAG GAC CAT   336
Leu Arg Ala Tyr Leu Asn Thr Pro Gly Leu Pro Val Cys Gln Asp His
            100                 105                 110
CTG GAG TTC TGG GAG AGC GTC TTC ACC GGC CTC ACC CAC ATA GAT GCC   384
Leu Glu Phe Trp Glu Ser Val Phe Thr Gly Leu Thr His Ile Asp Ala
        115                 120                 125
CAC TTC TTG TCC CAG ACC AAA CAG GCA GGA GAC AAC TTC CCC TAC CTG   432
His Phe Leu Ser Gln Thr Lys Gln Ala Gly Asp Asn Phe Pro Tyr Leu
    130                 135                 140
GTA GCA TAC CAG GCT ACA GTG TGC GCC AGG GCC AAG GCT CCA CCT CCA   480
Val Ala Tyr Gln Ala Thr Val Cys Ala Arg Ala Lys Ala Pro Pro Pro
145                 150                 155                 160
TCG TGG GAT CAA ATG TGG AAG TGT CTC ATA CGG CTG AAG CCT ACG CTA   528
Ser Trp Asp Gln Met Trp Lys Cys Leu Ile Arg Leu Lys Pro Thr Leu
                165                 170                 175
CAC GGG CCA ACG CCC CTG TTG TAT AGG TTA GGA GCC GTT CAG AAC GAG   576
His Gly Pro Thr Pro Leu Leu Tyr Arg Leu Gly Ala Val Gln Asn Glu
            180                 185                 190
GTT ACC CTC ACA CAC CCC ATA ACC AAG TTC ATC ATG GCA TGC ATG TCG   624
Val Thr Leu Thr His Pro Ile Thr Lys Phe Ile Met Ala Cys Met Ser
        195                 200                 205
GCT GAC CTA GAG GTC GTC ACT AGC ACT TGG GTG CTG GTA GGC GGG GTC   672
Ala Asp Leu Glu Val Val Thr Ser Thr Trp Val Leu Val Gly Gly Val
    210                 215                 220
CTC GCG GCT CTG GCC GCG TAC TGC CTG ACA ACG GGC AGC GTG GTC ATT   720
Leu Ala Ala Leu Ala Ala Tyr Cys Leu Thr Thr Gly Ser Val Val Ile
225                 230                 235                 240
GTG GGC AGG ATC ATC TTG TCC GGG AGG CCG GCC GTT ATT CCC GAC AGG   768
```

```
Val Gly Arg Ile Ile Leu Ser Gly Arg Pro Ala Val Ile Pro Asp Arg
                245                 250                 255
GAA GTT CTC TAC CAA GAG TTC GAT GAA ATG GAA GAG TGC GCC TCG CAC    816
Glu Val Leu Tyr Gln Glu Phe Asp Glu Met Glu Glu Cys Ala Ser His
            260                 265                 270
CTC CCT TAC ATC GAA CAA GGA ATG CAG CTC GCC GAG CAA TTC AAG CAG    864
Leu Pro Tyr Ile Glu Gln Gly Met Gln Leu Ala Glu Gln Phe Lys Gln
        275                 280                 285
AAG GCG CTC GGT TTG CTG CAA ACA GCC ACC AAG CAA GCG GAG GCT GCT    912
Lys Ala Leu Gly Leu Leu Gln Thr Ala Thr Lys Gln Ala Glu Ala Ala
    290                 295                 300
GCT CCC GTG GTG GAG TCC AAG TGG CGA GCC CTT GAG ACC TTC TGG GCG    960
Ala Pro Val Val Glu Ser Lys Trp Arg Ala Leu Glu Thr Phe Trp Ala
305                 310                 315                 320
AAG CAC ATG TGG AAT TTC ATC AGC GGG ATA CAG TAC TTA GCA GGC TTG   1008
Lys His Met Trp Asn Phe Ile Ser Gly Ile Gln Tyr Leu Ala Gly Leu
                325                 330                 335
TCC ACT CTG CCT GGA AAC CCC GCA ATA GCA TCA CTG ATG GCA TTC ACA   1056
Ser Thr Leu Pro Gly Asn Pro Ala Ile Ala Ser Leu Met Ala Phe Thr
            340                 345                 350
GCC TCT ATC ACC AGC CCG CTC ACC ACC CAA TAT ACC CTC CTG TTT AAC   1104
Ala Ser Ile Thr Ser Pro Leu Thr Thr Gln Tyr Thr Leu Leu Phe Asn
        355                 360                 365
ATC TTG GGG GGA TGG GTG GCC GCC CAA CTC GCC CCC CCC AGT GCC GCT   1152
Ile Leu Gly Gly Trp Val Ala Ala Gln Leu Ala Pro Pro Ser Ala Ala
    370                 375                 380
TCA GCC TTC GTG GGC GCC GGT ATA GCT GGC GCG GCT GTT GGC AGC ATA   1200
Ser Ala Phe Val Gly Ala Gly Ile Ala Gly Ala Ala Val Gly Ser Ile
385                 390                 395                 400
GGC CTC GGG AAG GTG CTT GTG GAC ATT CTG GCG GGT TAT GGA GCA GGG   1248
Gly Leu Gly Lys Val Leu Val Asp Ile Leu Ala Gly Tyr Gly Ala Gly
                405                 410                 415
GTG GCA GGC GCG CTC GTG GCC TTT AAG GTC ATG AGC GGT GAC ATG CCC   1296
Val Ala Gly Ala Leu Val Ala Phe Lys Val Met Ser Gly Asp Met Pro
            420                 425                 430
TCC ACC GAG GAC CTG GTC AAC TTA CTC CCC GCC ATC CTC TCT CCT GGT   1344
Ser Thr Glu Asp Leu Val Asn Leu Leu Pro Ala Ile Leu Ser Pro Gly
```

```
        435                 440                 445
GCC CTG GTC GTC GGG GTC GTG TGC GCA GCA ATA CTG CGT CGG CAT GTG  1392
Ala Leu Val Val Gly Val Val Cys Ala Ala Ile Leu Arg Arg His Val
    450                 455                 460
GGC CCA GGG GAG GGG GCT GTG CAG TGG ATG AAC CGG CTG              1431
Gly Pro Gly Glu Gly Ala Val Gln Trp Met Asn Arg Leu
465                 470                 475
```

SEQ ID NO:60

SEQUENCE LENGTH: 1431 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: N16N15-1

```
GGC TAT ACC GGC GAC TTC GAC TCA GTG ATC GAC TGC AAC ACA TGT GTC   48
Gly Tyr Thr Gly Asp Phe Asp Ser Val Ile Asp Cys Asn Thr Cys Val
  1               5                  10                  15
ACC CAA ACA GTC GAT TTC AGC TTG GAC CCT ACT TTC ACC ATC GAG ACG   96
Thr Gln Thr Val Asp Phe Ser Leu Asp Pro Thr Phe Thr Ile Glu Thr
                20                  25                  30
ACG ACC GTA CCC CAA GAT GCG GTG TCG CGC TCG CAG CGG CGA GGC AGG  144
Thr Thr Val Pro Gln Asp Ala Val Ser Arg Ser Gln Arg Arg Gly Arg
            35                  40                  45
ACT GGT AGG GGC AGG GGG GGC ATA TAC AGG TTT GTA ACT CCA GGG GAA  192
Thr Gly Arg Gly Arg Gly Gly Ile Tyr Arg Phe Val Thr Pro Gly Glu
    50                  55                  60
CGG CCC TCA GGC ATG TTC GAT TCT TCG GTC CTG TGT GAA TGT TAT GAC  240
Arg Pro Ser Gly Met Phe Asp Ser Ser Val Leu Cys Glu Cys Tyr Asp
65                  70                  75                  80
GCG GGC TGT GCT TGG TAC GAG CTC ACG CCC GCC GAG ACC TCG GTT AGG  288
Ala Gly Cys Ala Trp Tyr Glu Leu Thr Pro Ala Glu Thr Ser Val Arg
                85                  90                  95
```

```
TTG CGG GCT TAC CTA AAT ACA CCT GGG CTG CCC GTC TGC CAG GAC CAT   336
Leu Arg Ala Tyr Leu Asn Thr Pro Gly Leu Pro Val Cys Gln Asp His
            100                 105                 110
CTG GAG TTC TGG GAG AGC GTC TTC ACC GGC CTC ACC CAC ATA GAT GCC   384
Leu Glu Phe Trp Glu Ser Val Phe Thr Gly Leu Thr His Ile Asp Ala
        115                 120                 125
CAC TTC TTG TCC CAG ACC AAA CAG GCA GGA GAC AAC TTC CCC TAC CTG   432
His Phe Leu Ser Gln Thr Lys Gln Ala Gly Asp Asn Phe Pro Tyr Leu
    130                 135                 140
GTA GCA TAC CAG GCT ACA GTG TGC GCC AGG GCC AAG GCT CCA CCT CCA   480
Val Ala Tyr Gln Ala Thr Val Cys Ala Arg Ala Lys Ala Pro Pro Pro
145                 150                 155                 160
TCG TGG GAT CAG ATG TGG AAG TGT CTC ATA CGG CTG AAG CCT ACG CTA   528
Ser Trp Asp Gln Met Trp Lys Cys Leu Ile Arg Leu Lys Pro Thr Leu
                165                 170                 175
CAC GGG CCA ACG CCC CTG TTG TAT AGG TTA GGA GCC GTT CAG AAC GAG   576
His Gly Pro Thr Pro Leu Leu Tyr Arg Leu Gly Ala Val Gln Asn Glu
            180                 185                 190
GTT ACC CTC ACA CAC CCC ATA ACC AAG TTC ATC ATG GCA TGC ATG TCG   624
Val Thr Leu Thr His Pro Ile Thr Lys Phe Ile Met Ala Cys Met Ser
        195                 200                 205
GCT GAC CTA GAG GTC GTC ACT AGC ACT TGG GTG CTG GTA GGC GGG GTC   672
Ala Asp Leu Glu Val Val Thr Ser Thr Trp Val Leu Val Gly Gly Val
    210                 215                 220
CTC GCG GCT CTG GCC GCG TAC TGC CTG ACA ACG GGC AGC GTG GTC ATT   720
Leu Ala Ala Leu Ala Ala Tyr Cys Leu Thr Thr Gly Ser Val Val Ile
225                 230                 235                 240
GTG GGC AGG ATC ATC TTG TCC GGG AGG CCG GCC GTT ATT CCC GAC AGG   768
Val Gly Arg Ile Ile Leu Ser Gly Arg Pro Ala Val Ile Pro Asp Arg
                245                 250                 255
GAA GTT CTC TAC CAA GAG TTC GAT GAA ATG GAA GAG TGC GCC TCG CAC   816
Glu Val Leu Tyr Gln Glu Phe Asp Glu Met Glu Glu Cys Ala Ser His
            260                 265                 270
CTC CCT TAC ATC GAA CAA GGA ATG CAG CTC GCC GAG CAA TTC AAG CAG   864
Leu Pro Tyr Ile Glu Gln Gly Met Gln Leu Ala Glu Gln Phe Lys Gln
        275                 280                 285
AAG GCG CTC GGT TTG CTG CAA ACA GCC ACC AAG CAA GCG GAG GCT GCT   912
```

```
Lys Ala Leu Gly Leu Leu Gln Thr Ala Thr Lys Gln Ala Glu Ala Ala
    290                 295                 300
GCT CCC GTG GTG GAG TCC AAG TGG CGA GCC CTT GAG ACC TTC TGG GCG    960
Ala Pro Val Val Glu Ser Lys Trp Arg Ala Leu Glu Thr Phe Trp Ala
305                 310                 315                 320
AAG CAC ATG TGG AAT TTC ATC AGC GGG ATA CAG TAC TTA GCA GGC TTG   1008
Lys His Met Trp Asn Phe Ile Ser Gly Ile Gln Tyr Leu Ala Gly Leu
                325                 330                 335
TCC ACT CTG CCT GGA AAC CCC GCA ATA GCA TCA CTG ATG GCA TTC ACA   1056
Ser Thr Leu Pro Gly Asn Pro Ala Ile Ala Ser Leu Met Ala Phe Thr
            340                 345                 350
GCC TCT ATC ACC AGC CCG CTC ACC ACC CAA TAT ACC CTC CTG TTT AAC   1104
Ala Ser Ile Thr Ser Pro Leu Thr Thr Gln Tyr Thr Leu Leu Phe Asn
        355                 360                 365
ATC TTG GGG GGA TGG GTG GCC GCC CAA CTC GCC CCC CCC AGT GCC GCT   1152
Ile Leu Gly Gly Trp Val Ala Ala Gln Leu Ala Pro Pro Ser Ala Ala
    370                 375                 380
TCA GCC TTC GTG GGC GCC GGT ATA GCT GGC GCG GCT GTT GGC AGC ATA   1200
Ser Ala Phe Val Gly Ala Gly Ile Ala Gly Ala Ala Val Gly Ser Ile
385                 390                 395                 400
GGC CTC GGG AAG GTG CTT GTG GAC ATT CTG GCG GGT TAT GGA GCA GGG   1248
Gly Leu Gly Lys Val Leu Val Asp Ile Leu Ala Gly Tyr Gly Ala Gly
                405                 410                 415
GTG GCA GGC GCG CTC GTG GCC TTT AAG GTC ATG AGC GGT GAC ATG CCC   1296
Val Ala Gly Ala Leu Val Ala Phe Lys Val Met Ser Gly Asp Met Pro
            420                 425                 430
TCC ACC GAG GAC CTG GTC AAC TTA CTC CCC GCC ATC CTC TCT CCT GGT   1344
Ser Thr Glu Asp Leu Val Asn Leu Leu Pro Ala Ile Leu Ser Pro Gly
        435                 440                 445
GCC CTG GTC GTC GGG GTC GTG TGC GCA GCA ATA CTG CGT CGG CAT GTG   1392
Ala Leu Val Val Gly Val Val Cys Ala Ala Ile Leu Arg Arg His Val
    450                 455                 460
GGC CCA GGG GAG GGG GCT GTG CAG TGG ATG AAC CGG CTG               1431
Gly Pro Gly Glu Gly Ala Val Gln Trp Met Asn Arg Leu
465                 470                 475
```

SEQ ID NO:61

SEQUENCE LENGTH: 2304 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N23N15A-1

```
CTG CTG TCG CCC GGG CCC ATC TCT TAC TTG AAG GGT TCC TCG GGT GGT    48
Leu Leu Ser Pro Gly Pro Ile Ser Tyr Leu Lys Gly Ser Ser Gly Gly
  1               5                  10                  15
CCG CTG CCT TGC CCC TCG GGC CGT GTT GTG GGC ATC TTC CGG GCT GCC    96
Pro Leu Pro Cys Pro Ser Gly Arg Val Val Gly Ile Phe Arg Ala Ala
                 20                  25                  30
GTG TGC ACC CGG GGG GTT GCG AAG GCG GTG GAC TTT GTG CCC GTT GAG   144
Val Cys Thr Arg Gly Val Ala Lys Ala Val Asp Phe Val Pro Val Glu
             35                  40                  45
TCT ATG GAA ACC ACC ATG CGG TCT CCG GTC TTC ACG GAT AAC TCA ACC   192
Ser Met Glu Thr Thr Met Arg Ser Pro Val Phe Thr Asp Asn Ser Thr
         50                  55                  60
CCC CCG GCC GTA CCG CAG ACA TTC CAA GTG GCC CAC CTA CAC GCT CCC   240
Pro Pro Ala Val Pro Gln Thr Phe Gln Val Ala His Leu His Ala Pro
 65                  70                  75                  80
ACT GGC AGC GGC AAA AGC ACC AGG GTG CCG GCT GCG TAT GCG GCC CAA   288
Thr Gly Ser Gly Lys Ser Thr Arg Val Pro Ala Ala Tyr Ala Ala Gln
                 85                  90                  95
GGG TAC AAG GTA CTC GTC CTG AAC CCG TCC GTT GCT GCC ACT TTG GGC   336
Gly Tyr Lys Val Leu Val Leu Asn Pro Ser Val Ala Ala Thr Leu Gly
            100                 105                 110
TTT GGG GCG TAC ATG TCC AAG GCA CAT GGT GTT GAC CCT AAC ATC AGA   384
Phe Gly Ala Tyr Met Ser Lys Ala His Gly Val Asp Pro Asn Ile Arg
        115                 120                 125
ACT GGG GTG AGG ACC ATC ACC ACG GGC GCT CCC ATC ACG TAC TCC ACC   432
Thr Gly Val Arg Thr Ile Thr Thr Gly Ala Pro Ile Thr Tyr Ser Thr
    130                 135                 140
```

```
TAC GGT AAG TTC CTC GCC GAC GGT GGC TGT TCT GGG GGT GCC TAT GAC   480
Tyr Gly Lys Phe Leu Ala Asp Gly Gly Cys Ser Gly Gly Ala Tyr Asp
145                 150                 155                 160
ATC ATA ATA TGT GAT GAG TGT CAT TCA ACT GAC TCG ACT TCC ATC TTG   528
Ile Ile Ile Cys Asp Glu Cys His Ser Thr Asp Ser Thr Ser Ile Leu
                165                 170                 175
GGC ATT GGT ACA GTC CTG GAC CAA GCG GAG ACG GCT GGA GCG CGC CTT   576
Gly Ile Gly Thr Val Leu Asp Gln Ala Glu Thr Ala Gly Ala Arg Leu
            180                 185                 190
GTC GTG CTC GCC ACC GCT ACG CCT CCG GGA TCG GTC ACC GTG CCG CAT   624
Val Val Leu Ala Thr Ala Thr Pro Pro Gly Ser Val Thr Val Pro His
        195                 200                 205
CCT AAT ATT GAG GAG GTG GCC TTG TCC AAC ACT GGA GAG ATC CCC TTC   672
Pro Asn Ile Glu Glu Val Ala Leu Ser Asn Thr Gly Glu Ile Pro Phe
    210                 215                 220
TAT GGC AAG GCC ATC CCC CTC GAG GCC ATC AAG GGG GGG AGG CAT CTC   720
Tyr Gly Lys Ala Ile Pro Leu Glu Ala Ile Lys Gly Gly Arg His Leu
225                 230                 235                 240
ATT TTC TGC CAT TCC AAG AAG AAA TGT GAC GAG CTC GCT GCG AAG CTG   768
Ile Phe Cys His Ser Lys Lys Lys Cys Asp Glu Leu Ala Ala Lys Leu
                245                 250                 255
TCG GCC CTC GGA GTC AAC GCT GTA GCA TAT TAC CGG GGT CTT GAT GTG   816
Ser Ala Leu Gly Val Asn Ala Val Ala Tyr Tyr Arg Gly Leu Asp Val
            260                 265                 270
TCC ATC ATA CCG ACA AGC GGG GAC GTC GTT GTC GTG GCA ACA GAC GCT   864
Ser Ile Ile Pro Thr Ser Gly Asp Val Val Val Val Ala Thr Asp Ala
        275                 280                 285
CTA ATG ACG GGC TAT ACC GGT GAC TTT GAC TCG GTG ATC GAC TGC AAC   912
Leu Met Thr Gly Tyr Thr Gly Asp Phe Asp Ser Val Ile Asp Cys Asn
    290                 295                 300
ACA TGT GTC ACC CAA ACA GTC GAT TTC AGC TTG GAC CCT ACT TTC ACC   960
Thr Cys Val Thr Gln Thr Val Asp Phe Ser Leu Asp Pro Thr Phe Thr
305                 310                 315                 320
ATC GAG ACG ACG ACC GTA CCC CAA GAT GCG GTG TCG CGC TCG CAG CGG  1008
Ile Glu Thr Thr Thr Val Pro Gln Asp Ala Val Ser Arg Ser Gln Arg
                325                 330                 335
CGA GGC AGG ACT GGT AGG GGC AGG GGG GGC ATA TAC AGG TTT GTA ACT  1056
```

```
Arg Gly Arg Thr Gly Arg Gly Arg Gly Gly Ile Tyr Arg Phe Val Thr
            340                 345                 350
CCA GGG GAA CGG CCC TCA GGC ATG TTC GAT TCT TCG GTC CTG TGT GAA   1104
Pro Gly Glu Arg Pro Ser Gly Met Phe Asp Ser Ser Val Leu Cys Glu
        355                 360                 365
TGT TAT GAC GCG GGC TGT GCT TGG TAC GAG CTC ACG CCC GCC GAG ACC   1152
Cys Tyr Asp Ala Gly Cys Ala Trp Tyr Glu Leu Thr Pro Ala Glu Thr
    370                 375                 380
TCG GTT AGG TTG CGG GCT TAC CTA AAT ACA CCT GGG CTG CCC GTC TGC   1200
Ser Val Arg Leu Arg Ala Tyr Leu Asn Thr Pro Gly Leu Pro Val Cys
385                 390                 395                 400
CAG GAC CAT CTG GAG TTC TGG GAG AGC GTC TTC ACC GGC CTC ACC CAC   1248
Gln Asp His Leu Glu Phe Trp Glu Ser Val Phe Thr Gly Leu Thr His
                405                 410                 415
ATA GAT GCC CAC TTC TTG TCC CAG ACC AAA CAG GCA GGA GAC AAC TTC   1296
Ile Asp Ala His Phe Leu Ser Gln Thr Lys Gln Ala Gly Asp Asn Phe
            420                 425                 430
CCC TAC CTG GTA GCA TAC CAG GCT ACA GTG TGC GCC AGG GCC AAG GCT   1344
Pro Tyr Leu Val Ala Tyr Gln Ala Thr Val Cys Ala Arg Ala Lys Ala
        435                 440                 445
CCA CCT CCA TCG TGG GAT CAG ATG TGG AAG TGT CTC ATA CGG CTG AAG   1392
Pro Pro Pro Ser Trp Asp Gln Met Trp Lys Cys Leu Ile Arg Leu Lys
    450                 455                 460
CCT ACG CTA CAC GGG CCA ACG CCC CTG TTG TAT AGG TTA GGA GCC GTT   1440
Pro Thr Leu His Gly Pro Thr Pro Leu Leu Tyr Arg Leu Gly Ala Val
465                 470                 475                 480
CAG AAC GAG GTT ACC CTC ACA CAC CCC ATA ACC AAG TTC ATC ATG GCA   1488
Gln Asn Glu Val Thr Leu Thr His Pro Ile Thr Lys Phe Ile Met Ala
                485                 490                 495
TGC ATG TCG GCT GAC CTA GAG GTC GTC ACT AGC ACT TGG GTG CTG GTA   1536
Cys Met Ser Ala Asp Leu Glu Val Val Thr Ser Thr Trp Val Leu Val
            500                 505                 510
GGC GGG GTC CTC GCG GCT CTG GCC GCG TAC TGC CTG ACA ACG GGC AGC   1584
Gly Gly Val Leu Ala Ala Leu Ala Ala Tyr Cys Leu Thr Thr Gly Ser
        515                 520                 525
GTG GTC ATT GTG GGC AGG ATC ATC TTG TCC GGG AGG CCG GCC GTT ATT   1632
Val Val Ile Val Gly Arg Ile Ile Leu Ser Gly Arg Pro Ala Val Ile
```

```
    530                 535                 540
CCC GAC AGG GAA GTT CTC TAC CAA GAG TTC GAT GAA ATG GAA GAG TGC   1680
Pro Asp Arg Glu Val Leu Tyr Gln Glu Phe Asp Glu Met Glu Glu Cys
545                 550                 555                 560
GCC TCG CAC CTC CCT TAC ATC GAA CAA GGA ATG CAG CTC GCC GAG CAA   1728
Ala Ser His Leu Pro Tyr Ile Glu Gln Gly Met Gln Leu Ala Glu Gln
                565                 570                 575
TTC AAG CAG AAG GCG CTC GGT TTG CTG CAA ACA GCC ACC AAG CAA GCG   1776
Phe Lys Gln Lys Ala Leu Gly Leu Leu Gln Thr Ala Thr Lys Gln Ala
            580                 585                 590
GAG GCT GCT GCT CCC GTG GTG GAG TCC AAG TGG CGA GCC CTT GAG ACC   1824
Glu Ala Ala Ala Pro Val Val Glu Ser Lys Trp Arg Ala Leu Glu Thr
        595                 600                 605
TTC TGG GCG AAG CAC ATG TGG AAT TTC ATC AGC GGG ATA CAG TAC TTA   1872
Phe Trp Ala Lys His Met Trp Asn Phe Ile Ser Gly Ile Gln Tyr Leu
    610                 615                 620
GCA GGC TTG TCC ACT CTG CCT GGA AAC CCC GCA ATA GCA TCA CTG ATG   1920
Ala Gly Leu Ser Thr Leu Pro Gly Asn Pro Ala Ile Ala Ser Leu Met
625                 630                 635                 640
GCA TTC ACA GCC TCT ATC ACC AGC CCG CTC ACC ACC CAA TAT ACC CTC   1968
Ala Phe Thr Ala Ser Ile Thr Ser Pro Leu Thr Thr Gln Tyr Thr Leu
                645                 650                 655
CTG TTT AAC ATC TTG GGG GGA TGG GTG GCC GCC CAA CTC GCC CCC CCC   2016
Leu Phe Asn Ile Leu Gly Gly Trp Val Ala Ala Gln Leu Ala Pro Pro
            660                 665                 670
AGT GCC GCT TCA GCC TTC GTG GGC GCC GGT ATA GCT GGC GCG GCT GTT   2064
Ser Ala Ala Ser Ala Phe Val Gly Ala Gly Ile Ala Gly Ala Ala Val
        675                 680                 685
GGC AGC ATA GGC CTC GGG AAG GTG CTT GTG GAC ATT CTG GCG GGT TAT   2112
Gly Ser Ile Gly Leu Gly Lys Val Leu Val Asp Ile Leu Ala Gly Tyr
    690                 695                 700
GGA GCA GGG GTG GCA GGC GCG CTC GTG GCC TTT AAG GTC ATG AGC GGT   2160
Gly Ala Gly Val Ala Gly Ala Leu Val Ala Phe Lys Val Met Ser Gly
705                 710                 715                 720
GAC ATG CCC TCC ACC GAG GAC CTG GTC AAC TTA CTC CCC GCC ATC CTC   2208
Asp Met Pro Ser Thr Glu Asp Leu Val Asn Leu Leu Pro Ala Ile Leu
                725                 730                 735
```

```
TCT CCT GGT GCC CTG GTC GTC GGG GTC GTG TGC GCA GCA ATA CTG CGT  2256
Ser Pro Gly Ala Leu Val Val Gly Val Val Cys Ala Ala Ile Leu Arg
            740                 745                 750
CGG CAT GTG GGC CCA GGG GAG GGG GCT GTG CAG TGG ATG AAC CGG CTG  2304
Arg His Val Gly Pro Gly Glu Gly Ala Val Gln Trp Met Asn Arg Leu
        755                 760                 765
```

SEQ ID NO:62
SEQUENCE LENGTH: 2304 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N23N15B-1

```
CTG CTG TCG CCC GGG CCC ATC TCT TAC TTG AAG GGT TCC TCG GGT GGT   48
Leu Leu Ser Pro Gly Pro Ile Ser Tyr Leu Lys Gly Ser Ser Gly Gly
  1               5                  10                  15
CCG CTG CCT TGC CCC TCG GGC CGT GTT GTG GGC ATC TTC CGG GCT GCC   96
Pro Leu Pro Cys Pro Ser Gly Arg Val Val Gly Ile Phe Arg Ala Ala
            20                  25                  30
GTG TGC ACC CGG GGG GTT GCG AAG GCG GTG GAC TTT GTG CCC GTT GAG  144
Val Cys Thr Arg Gly Val Ala Lys Ala Val Asp Phe Val Pro Val Glu
        35                  40                  45
TCT ATG GAA ACC ACC ATG CGG TCT CCG GTC TTC ACG GAT AAC TCA ACC  192
Ser Met Glu Thr Thr Met Arg Ser Pro Val Phe Thr Asp Asn Ser Thr
    50                  55                  60
CCC CCG GCC GTA CCG CAG ACA TTC CAA GTG GCC CAC CTA CAC GCT CCC  240
Pro Pro Ala Val Pro Gln Thr Phe Gln Val Ala His Leu His Ala Pro
 65                  70                  75                  80
ACT GGC AGC GGC AAA AGC ACC AGG GTG CCG GCT GCG TAT GCG GCC CAA  288
Thr Gly Ser Gly Lys Ser Thr Arg Val Pro Ala Ala Tyr Ala Ala Gln
                 85                  90                  95
GGG TAC AAG GTA CTC GTC CTG AAC CCG TCC GTT GCT GCC ACT TTG GGC  336
```

```
Gly Tyr Lys Val Leu Val Leu Asn Pro Ser Val Ala Ala Thr Leu Gly
            100                 105                 110
TTT GGG GCG TAC ATG TCC AAG GCA CAT GGT GTT GAC CCT AAC ATC AGA    384
Phe Gly Ala Tyr Met Ser Lys Ala His Gly Val Asp Pro Asn Ile Arg
        115                 120                 125
ACT GGG GTG AGG ACC ATC ACC ACG GGC GCT CCC ATC ACG TAC TCC ACC    432
Thr Gly Val Arg Thr Ile Thr Thr Gly Ala Pro Ile Thr Tyr Ser Thr
    130                 135                 140
TAC GGT AAG TTC CTC GCC GAC GGT GGC TGT TCT GGG GGT GCC TAT GAC    480
Tyr Gly Lys Phe Leu Ala Asp Gly Gly Cys Ser Gly Gly Ala Tyr Asp
145                 150                 155                 160
ATC ATA ATA TGT GAT GAG TGT CAT TCA ACT GAC TCG ACT TCC ATC TTG    528
Ile Ile Ile Cys Asp Glu Cys His Ser Thr Asp Ser Thr Ser Ile Leu
                165                 170                 175
GGC ATT GGT ACA GTC CTG GAC CAA GCG GAG ACG GCT GGA GCG CGC CTT    576
Gly Ile Gly Thr Val Leu Asp Gln Ala Glu Thr Ala Gly Ala Arg Leu
            180                 185                 190
GTC GTG CTC GCC ACC GCT ACG CCT CCG GGA TCG GTC ACC GTG CCG CAT    624
Val Val Leu Ala Thr Ala Thr Pro Pro Gly Ser Val Thr Val Pro His
        195                 200                 205
CCT AAT ATT GAG GAG GTG GCC TTG TCC AAC ACT GGA GAG ATC CCC TTC    672
Pro Asn Ile Glu Glu Val Ala Leu Ser Asn Thr Gly Glu Ile Pro Phe
    210                 215                 220
TAT GGC AAG GCC ATC CCC CTC GAG GCC ATC AAG GGG GGG AGG CAT CTC    720
Tyr Gly Lys Ala Ile Pro Leu Glu Ala Ile Lys Gly Gly Arg His Leu
225                 230                 235                 240
ATT TTC TGC CAT TCC AAG AAG AAA TGT GAC GAG CTC GCT GCG AAG CTG    768
Ile Phe Cys His Ser Lys Lys Lys Cys Asp Glu Leu Ala Ala Lys Leu
                245                 250                 255
TCG GCC CTC GGA GTC AAC GCT GTA GCA TAT TAC CGG GGT CTT GAT GTG    816
Ser Ala Leu Gly Val Asn Ala Val Ala Tyr Tyr Arg Gly Leu Asp Val
            260                 265                 270
TCC ATC ATA CCG ACA AGC GGG GAC GTC GTT GTC GTG GCA ACA GAC GCT    864
Ser Ile Ile Pro Thr Ser Gly Asp Val Val Val Val Ala Thr Asp Ala
        275                 280                 285
CTA ATG ACG GGC TAT ACC GGC GAC TTC GAC TCA GTG ATC GAC TGC AAC    912
Leu Met Thr Gly Tyr Thr Gly Asp Phe Asp Ser Val Ile Asp Cys Asn
```

```
    290                     295                     300
ACA TGT GTC ACC CAA ACA GTC GAT TTC AGC TTG GAC CCT ACT TTC ACC    960
Thr Cys Val Thr Gln Thr Val Asp Phe Ser Leu Asp Pro Thr Phe Thr
305                 310                     315                 320
ATC GAG ACG ACG ACC GTA CCC CAA GAT GCG GTG TCG CGC TCG CAG CGG   1008
Ile Glu Thr Thr Thr Val Pro Gln Asp Ala Val Ser Arg Ser Gln Arg
                325                     330                 335
CGA GGC AGG ACT GGT AGG GGC AGG GGG GGC ATA TAC AGG TTT GTA ACT   1056
Arg Gly Arg Thr Gly Arg Gly Arg Gly Gly Ile Tyr Arg Phe Val Thr
            340                     345                 350
CCA GGG GAA CGG CCC TCA GGC ATG TTC GAT TCT TCG GTC CTG TGT GAA   1104
Pro Gly Glu Arg Pro Ser Gly Met Phe Asp Ser Ser Val Leu Cys Glu
        355                     360                 365
TGT TAT GAC GCG GGC TGT GCT TGG TAC GAG CTC ACG CCC GCC GAG ACC   1152
Cys Tyr Asp Ala Gly Cys Ala Trp Tyr Glu Leu Thr Pro Ala Glu Thr
    370                     375                 380
TCG GTT AGG TTG CGG GCT TAC CTA AAT ACA CCT GGG CTG CCC GTC TGC   1200
Ser Val Arg Leu Arg Ala Tyr Leu Asn Thr Pro Gly Leu Pro Val Cys
385                 390                     395                 400
CAG GAC CAT CTG GAG TTC TGG GAG AGC GTC TTC ACC GGC CTC ACC CAC   1248
Gln Asp His Leu Glu Phe Trp Glu Ser Val Phe Thr Gly Leu Thr His
                405                     410                 415
ATA GAT GCC CAC TTC TTG TCC CAG ACC AAA CAG GCA GGA GAC AAC TTC   1296
Ile Asp Ala His Phe Leu Ser Gln Thr Lys Gln Ala Gly Asp Asn Phe
            420                     425                 430
CCC TAC CTG GTA GCA TAC CAG GCT ACA GTG TGC GCC AGG GCC AAG GCT   1344
Pro Tyr Leu Val Ala Tyr Gln Ala Thr Val Cys Ala Arg Ala Lys Ala
        435                     440                 445
CCA CCT CCA TCG TGG GAT CAG ATG TGG AAG TGT CTC ATA CGG CTG AAG   1392
Pro Pro Pro Ser Trp Asp Gln Met Trp Lys Cys Leu Ile Arg Leu Lys
    450                     455                 460
CCT ACG CTA CAC GGG CCA ACG CCC CTG TTG TAT AGG TTA GGA GCC GTT   1440
Pro Thr Leu His Gly Pro Thr Pro Leu Leu Tyr Arg Leu Gly Ala Val
465                 470                     475                 480
CAG AAC GAG GTT ACC CTC ACA CAC CCC ATA ACC AAG TTC ATC ATG GCA   1488
Gln Asn Glu Val Thr Leu Thr His Pro Ile Thr Lys Phe Ile Met Ala
                485                     490                 495
```

```
TGC ATG TCG GCT GAC CTA GAG GTC GTC ACT AGC ACT TGG GTG CTG GTA  1536
Cys Met Ser Ala Asp Leu Glu Val Val Thr Ser Thr Trp Val Leu Val
            500                 505                 510
GGC GGG GTC CTC GCG GCT CTG GCC GCG TAC TGC CTG ACA ACG GGC AGC  1584
Gly Gly Val Leu Ala Ala Leu Ala Ala Tyr Cys Leu Thr Thr Gly Ser
        515                 520                 525
GTG GTC ATT GTG GGC AGG ATC ATC TTG TCC GGG AGG CCG GCC GTT ATT  1632
Val Val Ile Val Gly Arg Ile Ile Leu Ser Gly Arg Pro Ala Val Ile
    530                 535                 540
CCC GAC AGG GAA GTT CTC TAC CAA GAG TTC GAT GAA ATG GAA GAG TGC  1680
Pro Asp Arg Glu Val Leu Tyr Gln Glu Phe Asp Glu Met Glu Glu Cys
545                 550                 555                 560
GCC TCG CAC CTC CCT TAC ATC GAA CAA GGA ATG CAG CTC GCC GAG CAA  1728
Ala Ser His Leu Pro Tyr Ile Glu Gln Gly Met Gln Leu Ala Glu Gln
                565                 570                 575
TTC AAG CAG AAG GCG CTC GGT TTG CTG CAA ACA GCC ACC AAG CAA GCG  1776
Phe Lys Gln Lys Ala Leu Gly Leu Leu Gln Thr Ala Thr Lys Gln Ala
            580                 585                 590
GAG GCT GCT GCT CCC GTG GTG GAG TCC AAG TGG CGA GCC CTT GAG ACC  1824
Glu Ala Ala Ala Pro Val Val Glu Ser Lys Trp Arg Ala Leu Glu Thr
        595                 600                 605
TTC TGG GCG AAG CAC ATG TGG AAT TTC ATC AGC GGG ATA CAG TAC TTA  1872
Phe Trp Ala Lys His Met Trp Asn Phe Ile Ser Gly Ile Gln Tyr Leu
    610                 615                 620
GCA GGC TTG TCC ACT CTG CCT GGA AAC CCC GCA ATA GCA TCA CTG ATG  1920
Ala Gly Leu Ser Thr Leu Pro Gly Asn Pro Ala Ile Ala Ser Leu Met
625                 630                 635                 640
GCA TTC ACA GCC TCT ATC ACC AGC CCG CTC ACC ACC CAA TAT ACC CTC  1968
Ala Phe Thr Ala Ser Ile Thr Ser Pro Leu Thr Thr Gln Tyr Thr Leu
                645                 650                 655
CTG TTT AAC ATC TTG GGG GGA TGG GTG GCC GCC CAA CTC GCC CCC CCC  2016
Leu Phe Asn Ile Leu Gly Gly Trp Val Ala Ala Gln Leu Ala Pro Pro
            660                 665                 670
AGT GCC GCT TCA GCC TTC GTG GGC GCC GGT ATA GCT GGC GCG GCT GTT  2064
Ser Ala Ala Ser Ala Phe Val Gly Ala Gly Ile Ala Gly Ala Ala Val
        675                 680                 685
GGC AGC ATA GGC CTC GGG AAG GTG CTT GTG GAC ATT CTG GCG GGT TAT  2112
```

```
Gly Ser Ile Gly Leu Gly Lys Val Leu Val Asp Ile Leu Ala Gly Tyr
    690                 695                 700
GGA GCA GGG GTG GCA GGC GCG CTC GTG GCC TTT AAG GTC ATG AGC GGT  2160
Gly Ala Gly Val Ala Gly Ala Leu Val Ala Phe Lys Val Met Ser Gly
705                 710                 715                 720
GAC ATG CCC TCC ACC GAG GAC CTG GTC AAC TTA CTC CCC GCC ATC CTC  2208
Asp Met Pro Ser Thr Glu Asp Leu Val Asn Leu Leu Pro Ala Ile Leu
                725                 730                 735
TCT CCT GGT GCC CTG GTC GTC GGG GTC GTG TGC GCA GCA ATA CTG CGT  2256
Ser Pro Gly Ala Leu Val Val Gly Val Val Cys Ala Ala Ile Leu Arg
            740                 745                 750
CGG CAT GTG GGC CCA GGG GAG GGG GCT GTG CAG TGG ATG AAC CGG CTG  2304
Arg His Val Gly Pro Gly Glu Gly Ala Val Gln Trp Met Asn Arg Leu
        755                 760                 765
```

SEQ ID NO:63
SEQUENCE LENGTH: 3564 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: MX25N15-1

```
TGT GCC TGG TTG TGG ATG ATG CTG CTG ATA GCC CAA GCT GAG GCC GCC    48
Cys Ala Trp Leu Trp Met Met Leu Leu Ile Ala Gln Ala Glu Ala Ala
  1               5                  10                  15
TTG GAG AAC CTG GTG GTC CTC AAT GCA GCA TCC ATG GCG GGA GCG CAT    96
Leu Glu Asn Leu Val Val Leu Asn Ala Ala Ser Met Ala Gly Ala His
             20                  25                  30
GGC ATC CTC TCT TTC CTT GTG TTC TTC TGT GCC GCC TGG TAC ATC AAA   144
Gly Ile Leu Ser Phe Leu Val Phe Phe Cys Ala Ala Trp Tyr Ile Lys
         35                  40                  45
GGC AGG CTG GTC CCT GGG GCG GCA TAC GCT TTC TAT GGC GTA TGG CCG   192
Gly Arg Leu Val Pro Gly Ala Ala Tyr Ala Phe Tyr Gly Val Trp Pro
```

```
    50                  55                  60
CTG CTC CTG CTC TTG ATG GCG CTA CCC GCA CGG GCG TAC GCC ATG GAC   240
Leu Leu Leu Leu Leu Met Ala Leu Pro Ala Arg Ala Tyr Ala Met Asp
65                  70                  75                  80
CGG GAG ATG GCT GCA TCG TGC GGA GGC GCG GTT TTT GTA GGT CTG GTA   288
Arg Glu Met Ala Ala Ser Cys Gly Gly Ala Val Phe Val Gly Leu Val
                85                  90                  95
CTC TTG ACC TTG TCA CCA TAC TAC AAA GTG TTC CTC GCT AAG CTC ATA   336
Leu Leu Thr Leu Ser Pro Tyr Tyr Lys Val Phe Leu Ala Lys Leu Ile
            100                 105                 110
TGG TGG TTG CAA TAT CTC ATC ACC AGG GCC GAG GCG CAC TTG CAA GTG   384
Trp Trp Leu Gln Tyr Leu Ile Thr Arg Ala Glu Ala His Leu Gln Val
        115                 120                 125
TGG ATC CCC CCC CTC AAC GTT CGG GGG GGC CGC GAT GCC ATC ATC CTT   432
Trp Ile Pro Pro Leu Asn Val Arg Gly Gly Arg Asp Ala Ile Ile Leu
    130                 135                 140
CTC ACA TGT GCG GTC CAC CCG GAG CTG ATC TTT GAC ATC ACC AAG CTC   480
Leu Thr Cys Ala Val His Pro Glu Leu Ile Phe Asp Ile Thr Lys Leu
145                 150                 155                 160
TTG CTC GCC ATA CTC GGT CCG CTC ATG GTA CTC CAG GCT GGC CTA ACC   528
Leu Leu Ala Ile Leu Gly Pro Leu Met Val Leu Gln Ala Gly Leu Thr
                165                 170                 175
CAA ATG CCG TAC TTT GTG CGT GCT CAA GGG CTC ATT CGT ATG TGC ATG   576
Gln Met Pro Tyr Phe Val Arg Ala Gln Gly Leu Ile Arg Met Cys Met
            180                 185                 190
TTG GTG CGG AAA GCC GCT GGG GGT CAT TAT GTC CAG ATG GCT CTC ATG   624
Leu Val Arg Lys Ala Ala Gly Gly His Tyr Val Gln Met Ala Leu Met
        195                 200                 205
AAG CTG GCT GCA CTG ACA GGT ACG TAC GTT TAT GAC CAT CTT ACT CCA   672
Lys Leu Ala Ala Leu Thr Gly Thr Tyr Val Tyr Asp His Leu Thr Pro
    210                 215                 220
CTG CAG GAC TGG GCC CAC GCG GGC CTA CGA GAC CTT GCG GTA GCA GTT   720
Leu Gln Asp Trp Ala His Ala Gly Leu Arg Asp Leu Ala Val Ala Val
225                 230                 235                 240
GAG CCC GTT GCC TTC TCT GAT ATG GAG ACT AAG ATC ATC ACC TGG GGG   768
Glu Pro Val Ala Phe Ser Asp Met Glu Thr Lys Ile Ile Thr Trp Gly
                245                 250                 255
```

```
GCA GAC ACT GCG GCG TGT GGG GAC ATC ATT TTG GGC CTA CCT GTC TCC    816
Ala Asp Thr Ala Ala Cys Gly Asp Ile Ile Leu Gly Leu Pro Val Ser
            260                 265                 270
GCC CGG AGG GGC AAC GAG ATA CTC CTC GGA CCG GCC GAT AGT TTT GAC    864
Ala Arg Arg Gly Asn Glu Ile Leu Leu Gly Pro Ala Asp Ser Phe Asp
        275                 280                 285
GGG CAG GGG TGG CGA CTC CTT GCG CCT ATC ACG GCC TAC TCC CAG CAG    912
Gly Gln Gly Trp Arg Leu Leu Ala Pro Ile Thr Ala Tyr Ser Gln Gln
    290                 295                 300
ACG CGG GGC CTG CTT GGT TGC ATC ATC ACT AGC CTT ACG GGC CGG GAT    960
Thr Arg Gly Leu Leu Gly Cys Ile Ile Thr Ser Leu Thr Gly Arg Asp
305                 310                 315                 320
AAG AAC CAG GTC GAG GGG GAG GTT CAA GTG GTC TCT ACC GCA ACA CAA   1008
Lys Asn Gln Val Glu Gly Glu Val Gln Val Val Ser Thr Ala Thr Gln
                325                 330                 335
TCT TTC CTG GCG ACC TGT GTC AAC GGC GTG TGC TGG ACT GTT TTC CAC   1056
Ser Phe Leu Ala Thr Cys Val Asn Gly Val Cys Trp Thr Val Phe His
            340                 345                 350
GGC GCC GGC TCG AAG ACC TTA GCC GGC CCA AAA GGC CCA ATC ACC CAA   1104
Gly Ala Gly Ser Lys Thr Leu Ala Gly Pro Lys Gly Pro Ile Thr Gln
        355                 360                 365
ATG TAC ACC AAT GTA GAT CAG GAC CTC GTC GGC TGG TCG GCG CCC CCC   1152
Met Tyr Thr Asn Val Asp Gln Asp Leu Val Gly Trp Ser Ala Pro Pro
    370                 375                 380
CGG GCG CGT TCC TTG ACA CCT TGC ACC TGC GGC AGC TCG GAC CTT TAT   1200
Arg Ala Arg Ser Leu Thr Pro Cys Thr Cys Gly Ser Ser Asp Leu Tyr
385                 390                 395                 400
TTG GTC ACG AGG CAT GCT GAT GTC ATT CCG GTG CAC CGG CGG GGC GAC   1248
Leu Val Thr Arg His Ala Asp Val Ile Pro Val His Arg Arg Gly Asp
                405                 410                 415
AGC AGG GGG AGC CTC CTC TCC CCC GGG CCC ATC TCT TAC TTG AAG GGT   1296
Ser Arg Gly Ser Leu Leu Ser Pro Gly Pro Ile Ser Tyr Leu Lys Gly
            420                 425                 430
TCC TCG GGT GGT CCG CTG CCT TGC CCC TCG GGC CGT GTT GTG GGC ATC   1344
Ser Ser Gly Gly Pro Leu Pro Cys Pro Ser Gly Arg Val Val Gly Ile
        435                 440                 445
TTC CGG GCT GCC GTG TGC ACC CGG GGG GTT GCG AAG GCG GTG GAC TTT   1392
```

```
Phe Arg Ala Ala Val Cys Thr Arg Gly Val Ala Lys Ala Val Asp Phe
    450                 455                 460
GTG CCC GTT GAG TCT ATG GAA ACC ACC ATG CGG TCT CCG GTC TTC ACG  1440
Val Pro Val Glu Ser Met Glu Thr Thr Met Arg Ser Pro Val Phe Thr
465                 470                 475                 480
GAT AAC TCA ACC CCC CCG GCC GTA CCG CAG ACA TTC CAA GTG GCC CAC  1488
Asp Asn Ser Thr Pro Pro Ala Val Pro Gln Thr Phe Gln Val Ala His
                485                 490                 495
CTA CAC GCT CCC ACT GGC AGC GGC AAA AGC ACC AGG GTG CCG GCT GCG  1536
Leu His Ala Pro Thr Gly Ser Gly Lys Ser Thr Arg Val Pro Ala Ala
            500                 505                 510
TAT GCG GCC CAA GGG TAC AAG GTA CTC GTC CTG AAC CCG TCC GTT GCT  1584
Tyr Ala Ala Gln Gly Tyr Lys Val Leu Val Leu Asn Pro Ser Val Ala
        515                 520                 525
GCC ACT TTG GGC TTT GGG GCG TAC ATG TCC AAG GCA CAT GGT GTT GAC  1632
Ala Thr Leu Gly Phe Gly Ala Tyr Met Ser Lys Ala His Gly Val Asp
    530                 535                 540
CCT AAC ATC AGA ACT GGG GTG AGG ACC ATC ACC ACG GGC GCT CCC ATC  1680
Pro Asn Ile Arg Thr Gly Val Arg Thr Ile Thr Thr Gly Ala Pro Ile
545                 550                 555                 560
ACG TAC TCC ACC TAC GGT AAG TTC CTC GCC GAC GGT GGC TGT TCT GGG  1728
Thr Tyr Ser Thr Tyr Gly Lys Phe Leu Ala Asp Gly Gly Cys Ser Gly
                565                 570                 575
GGT GCC TAT GAC ATC ATA ATA TGT GAT GAG TGT CAT TCA ACT GAC TCG  1776
Gly Ala Tyr Asp Ile Ile Ile Cys Asp Glu Cys His Ser Thr Asp Ser
            580                 585                 590
ACT TCC ATC TTG GGC ATT GGT ACA GTC CTG GAC CAA GCG GAG ACG GCT  1824
Thr Ser Ile Leu Gly Ile Gly Thr Val Leu Asp Gln Ala Glu Thr Ala
        595                 600                 605
GGA GCG CGC CTT GTC GTG CTC GCC ACC GCT ACG CCT CCG GGA TCG GTC  1872
Gly Ala Arg Leu Val Val Leu Ala Thr Ala Thr Pro Pro Gly Ser Val
    610                 615                 620
ACC GTG CCG CAT CCT AAT ATT GAG GAG GTG GCC TTG TCC AAC ACT GGA  1920
Thr Val Pro His Pro Asn Ile Glu Glu Val Ala Leu Ser Asn Thr Gly
625                 630                 635                 640
GAG ATC CCC TTC TAT GGC AAG GCC ATC CCC CTC GAG GCC ATC AAG GGG  1968
Glu Ile Pro Phe Tyr Gly Lys Ala Ile Pro Leu Glu Ala Ile Lys Gly
```

```
                     645                                650                                655
GGG AGG CAT CTC ATT TTC TGC CAT TCC AAG AAG AAA TGT GAC GAG CTC   2016
Gly Arg His Leu Ile Phe Cys His Ser Lys Lys Lys Cys Asp Glu Leu
                660                 665                 670
GCT GCG AAG CTG TCG GCC CTC GGA GTC AAC GCT GTA GCA TAT TAC CGG   2064
Ala Ala Lys Leu Ser Ala Leu Gly Val Asn Ala Val Ala Tyr Tyr Arg
            675                 680                 685
GGT CTT GAT GTG TCC ATC ATA CCG ACA AGC GGG GAC GTC GTT GTC GTG   2112
Gly Leu Asp Val Ser Ile Ile Pro Thr Ser Gly Asp Val Val Val Val
        690                 695                 700
GCA ACA GAC GCT CTA ATG ACG GGC TAT ACC GGT GAC TTT GAC TCG GTG   2160
Ala Thr Asp Ala Leu Met Thr Gly Tyr Thr Gly Asp Phe Asp Ser Val
705                 710                 715                 720
ATC GAC TGC AAC ACA TGT GTC ACC CAA ACA GTC GAT TTC AGC TTG GAC   2208
Ile Asp Cys Asn Thr Cys Val Thr Gln Thr Val Asp Phe Ser Leu Asp
                725                 730                 735
CCT ACT TTC ACC ATC GAG ACG ACG ACC GTA CCC CAA GAT GCG GTG TCG   2256
Pro Thr Phe Thr Ile Glu Thr Thr Thr Val Pro Gln Asp Ala Val Ser
            740                 745                 750
CGC TCG CAG CGG CGA GGC AGG ACT GGT AGG GGC AGG GGG GGC ATA TAC   2304
Arg Ser Gln Arg Arg Gly Arg Thr Gly Arg Gly Arg Gly Gly Ile Tyr
        755                 760                 765
AGG TTT GTA ACT CCA GGG GAA CGG CCC TCA GGC ATG TTC GAT TCT TCG   2352
Arg Phe Val Thr Pro Gly Glu Arg Pro Ser Gly Met Phe Asp Ser Ser
    770                 775                 780
GTC CTG TGT GAA TGT TAT GAC GCG GGC TGT GCT TGG TAC GAG CTC ACG   2400
Val Leu Cys Glu Cys Tyr Asp Ala Gly Cys Ala Trp Tyr Glu Leu Thr
785                 790                 795                 800
CCC GCC GAG ACC TCG GTT AGG TTG CGG GCT TAC CTA AAT ACA CCT GGG   2448
Pro Ala Glu Thr Ser Val Arg Leu Arg Ala Tyr Leu Asn Thr Pro Gly
                805                 810                 815
CTG CCC GTC TGC CAG GAC CAT CTG GAG TTC TGG GAG AGC GTC TTC ACC   2496
Leu Pro Val Cys Gln Asp His Leu Glu Phe Trp Glu Ser Val Phe Thr
            820                 825                 830
GGC CTC ACC CAC ATA GAT GCC CAC TTC TTG TCC CAG ACC AAA CAG GCA   2544
Gly Leu Thr His Ile Asp Ala His Phe Leu Ser Gln Thr Lys Gln Ala
        835                 840                 845
```

```
GGA GAC AAC TTC CCC TAC CTG GTA GCA TAC CAG GCT ACA GTG TGC GCC  2592
Gly Asp Asn Phe Pro Tyr Leu Val Ala Tyr Gln Ala Thr Val Cys Ala
    850                 855                 860
AGG GCC AAG GCT CCA CCT CCA TCG TGG GAT CAG ATG TGG AAG TGT CTC  2640
Arg Ala Lys Ala Pro Pro Pro Ser Trp Asp Gln Met Trp Lys Cys Leu
865                 870                 875                 880
ATA CGG CTG AAG CCT ACG CTA CAC GGG CCA ACG CCC CTG TTG TAT AGG  2688
Ile Arg Leu Lys Pro Thr Leu His Gly Pro Thr Pro Leu Leu Tyr Arg
                885                 890                 895
TTA GGA GCC GTT CAG AAC GAG GTT ACC CTC ACA CAC CCC ATA ACC AAG  2736
Leu Gly Ala Val Gln Asn Glu Val Thr Leu Thr His Pro Ile Thr Lys
            900                 905                 910
TTC ATC ATG GCA TGC ATG TCG GCT GAC CTA GAG GTC GTC ACT AGC ACT  2784
Phe Ile Met Ala Cys Met Ser Ala Asp Leu Glu Val Val Thr Ser Thr
        915                 920                 925
TGG GTG CTG GTA GGC GGG GTC CTC GCG GCT CTG GCC GCG TAC TGC CTG  2832
Trp Val Leu Val Gly Gly Val Leu Ala Ala Leu Ala Ala Tyr Cys Leu
    930                 935                 940
ACA ACG GGC AGC GTG GTC ATT GTG GGC AGG ATC ATC TTG TCC GGG AGG  2880
Thr Thr Gly Ser Val Val Ile Val Gly Arg Ile Ile Leu Ser Gly Arg
945                 950                 955                 960
CCG GCC GTT ATT CCC GAC AGG GAA GTT CTC TAC CAA GAG TTC GAT GAA  2928
Pro Ala Val Ile Pro Asp Arg Glu Val Leu Tyr Gln Glu Phe Asp Glu
                965                 970                 975
ATG GAA GAG TGC GCC TCG CAC CTC CCT TAC ATC GAA CAA GGA ATG CAG  2976
Met Glu Glu Cys Ala Ser His Leu Pro Tyr Ile Glu Gln Gly Met Gln
            980                 985                 990
CTC GCC GAG CAA TTC AAG CAG AAG GCG CTC GGT TTG CTG CAA ACA GCC  3024
Leu Ala Glu Gln Phe Lys Gln Lys Ala Leu Gly Leu Leu Gln Thr Ala
        995                1000                1005
ACC AAG CAA GCG GAG GCT GCT GCT CCC GTG GTG GAG TCC AAG TGG CGA  3072
Thr Lys Gln Ala Glu Ala Ala Ala Pro Val Val Glu Ser Lys Trp Arg
   1010                1015                1020
GCC CTT GAG ACC TTC TGG GCG AAG CAC ATG TGG AAT TTC ATC AGC GGG  3120
Ala Leu Glu Thr Phe Trp Ala Lys His Met Trp Asn Phe Ile Ser Gly
1025                1030                1035                1040
ATA CAG TAC TTA GCA GGC TTG TCC ACT CTG CCT GGA AAC CCC GCA ATA  3168
```

```
Ile Gln Tyr Leu Ala Gly Leu Ser Thr Leu Pro Gly Asn Pro Ala Ile
                1045                1050                1055
GCA TCA CTG ATG GCA TTC ACA GCC TCT ATC ACC AGC CCG CTC ACC ACC   3216
Ala Ser Leu Met Ala Phe Thr Ala Ser Ile Thr Ser Pro Leu Thr Thr
            1060                1065                1070
CAA TAT ACC CTC CTG TTT AAC ATC TTG GGG GGA TGG GTG GCC GCC CAA   3264
Gln Tyr Thr Leu Leu Phe Asn Ile Leu Gly Gly Trp Val Ala Ala Gln
        1075                1080                1085
CTC GCC CCC CCC AGT GCC GCT TCA GCC TTC GTG GGC GCC GGT ATA GCT   3312
Leu Ala Pro Pro Ser Ala Ala Ser Ala Phe Val Gly Ala Gly Ile Ala
    1090                1095                1100
GGC GCG GCT GTT GGC AGC ATA GGC CTC GGG AAG GTG CTT GTG GAC ATT   3360
Gly Ala Ala Val Gly Ser Ile Gly Leu Gly Lys Val Leu Val Asp Ile
1105                1110                1115                1120
CTG GCG GGT TAT GGA GCA GGG GTG GCA GGC GCG CTC GTG GCC TTT AAG   3408
Leu Ala Gly Tyr Gly Ala Gly Val Ala Gly Ala Leu Val Ala Phe Lys
                1125                1130                1135
GTC ATG AGC GGT GAC ATG CCC TCC ACC GAG GAC CTG GTC AAC TTA CTC   3456
Val Met Ser Gly Asp Met Pro Ser Thr Glu Asp Leu Val Asn Leu Leu
            1140                1145                1150
CCC GCC ATC CTC TCT CCT GGT GCC CTG GTC GTC GGG GTC GTG TGC GCA   3504
Pro Ala Ile Leu Ser Pro Gly Ala Leu Val Val Gly Val Val Cys Ala
        1155                1160                1165
GCA ATA CTG CGT CGG CAT GTG GGC CCA GGG GAG GGG GCT GTG CAG TGG   3552
Ala Ile Leu Arg Arg His Val Gly Pro Gly Glu Gly Ala Val Gln Trp
    1170                1175                1180
ATG AAC CGG CTG                                                   3564
Met Asn Arg Leu
1185
```

SEQ ID NO:64
SEQUENCE LENGTH: 818 base pairs
SEQUENCE TYPE: nucleic acid
STRANDEDNESS: double
TOPOLOGY: linear
ANTI-SENSE: No
ORIGINAL SOURCE

ORGANISM: Hepatitis C virus
IMMEDIATE EXPERIMENTAL SOURCE
CLONE: N22-1, N22-3, H22-8, H22-9

```
 GG CAT GTG GGC CCA GGG GAG GGG GCT GTG CAG TGG ATG AAC CGG CTG    47
    His Val Gly Pro Gly Glu Gly Ala Val Gln Trp Met Asn Arg Leu
      1               5                  10                  15
ATA GCG TTY GCY TCG CGG GGY AAC CAY GTC TCC CCC ACG CAY TAT GTG    95
Ile Ala Phe Ala Ser Arg Gly Asn His Val Ser Pro Thr His Tyr Val
                    20                  25                  30
CCT GAR AGC GAC GCC GCR GCG CGY GTC ACC CAG ATC CTC TCC ARC CTY   143
Pro Glu Ser Asp Ala Ala Ala Arg Val Thr Gln Ile Leu Ser Xaa Leu
                35                  40                  45
ACC ATC ACT CAG YTG YTG AAG AGG CTY CAC CAG TGG ATT RAT GAK GAC   191
Thr Ile Thr Gln Leu Leu Lys Arg Leu His Gln Trp Ile Asx Xac Asp
            50                  55                  60
TGC TCC ACG CCA TGY TCY GGY TCG TGG CTC AGG GAT GTT TGG GAC TGG   239
Cys Ser Thr Pro Cys Ser Gly Ser Trp Leu Arg Asp Val Trp Asp Trp
     65                  70                  75
ATA TGC ACG GTR TTG RST GAY TKC AAG ACC TGG CTC CAG TCC AAG CTC   287
Ile Cys Thr Val Leu Xad Asp Xae Lys Thr Trp Leu Gln Ser Lys Leu
 80                  85                  90                  95
CTG CCG CGG YTA CCG GGR GTC CCT TTY YTY TCA TGC CAR CGT GGG TAC   335
Leu Pro Arg Leu Pro Gly Val Pro Phe Xaf Ser Cys Gln Arg Gly Tyr
                100                 105                 110
AAG GGR GTY TGG CGG GGA GAY GGC ATC ATG YAD ACC ACC TGC CCA TGY   383
Lys Gly Val Trp Arg Gly Asp Gly Ile Met Xag Thr Thr Cys Pro Cys
            115                 120                 125
GGA GCA CAA ATC RCC GGA CAT GTC AAA AAY GGT TCY ATG AGG ATC RYT   431
Gly Ala Gln Ile Xah Gly His Val Lys Asn Gly Ser Met Arg Ile Xai
        130                 135                 140
GGS CYY AGA ACC TGT AGC AAC ACG TGG CRC GGA ACR TTY CCC ATC AAC   479
Gly Xaj Arg Thr Cys Ser Asn Thr Trp Xak Gly Thr Phe Pro Ile Asn
    145                 150                 155
GCG TAC ACC ACA GGC CCC TGC ACA CCC TCY CCR GCG CCR AAC TAY TCY   527
Ala Tyr Thr Thr Gly Pro Cys Thr Pro Ser Pro Ala Pro Asn Tyr Ser
160                 165                 170                 175
```

```
ARG GCG TTR TGG CGG GTR GCY RYT GAG GAG TAT GTG GAG GTC ACG CGG   575
Xal Ala Leu Trp Arg Val Ala Xam Glu Glu Tyr Val Glu Val Thr Arg
                180                 185                 190
GTG GGG GAY TTC CAC TAC GTG ACG GGC ATG ACC ACT GAC AAC KTR AAA   623
Val Gly Asp Phe His Tyr Val Thr Gly Met Thr Thr Asp Asn Xan Lys
            195                 200                 205
TGC CCA TGC CAG GTY CCG GCC CCC GAA TTY TTC ACR GAR TTG GAT GGG   671
Cys Pro Cys Gln Val Pro Ala Pro Glu Phe Phe Thr Glu Leu Asp Gly
        210                 215                 220
GTR CGG CTR CRC AGR TAC GCT CCG GCG TGC AAA CCT CTC CTR CGG GAT   719
Val Arg Leu Xak Arg Tyr Ala Pro Ala Cys Lys Pro Leu Leu Arg Asp
    225                 230                 235
GAG GTC ACA TTC CAG GTC GGG CTC AAC CAA TWY MCG GTT GGG TCR CAG   767
Glu Val Thr Phe Gln Val Gly Leu Asn Gln Xao Xap Val Gly Ser Gln
240                 245                 250                 255
CTM CCA TGY GAG CCC GAA CCG GAT GTA RYR GTG GTC ACC TCC ATG CTC   815
Leu Pro Cys Glu Pro Glu Pro Asp Val Xaq Val Val Thr Ser Met Leu
                260                 265                 270
ACC                                                               818
Thr
```

| | | | |
|---|---|---|---|
| Y : C or T | R : A or G | M : A or C | K : G or T |
| S : G or C | W : A or T | D : G or T or A | |

| | | |
|---|---|---|
| Xaa : Asn or Ser | Asx : Asn or Asp | Xac : Glu or Asp |
| Xad : Ala or Ser | Xae : Cys or Phe | Xaf : Phe or Leu |
| Xag : Tyr or Gln or His | Xah : Thr or Ala | Xai : Val or Thr |
| Xaj : Pro or Leu | Xak : His or Arg | Xal : Arg or Lys |
| Xam : Ile or Ala | Xan : Val or Leu | Xao : Tyr or Phe |
| Xap : Thr or Pro | Xaq : Thr or Met or Ala | |

SEQ ID NO:65
SEQUENCE LENGTH: 311 base pairs
SEQUENCE TYPE: nucleic acid
STRANDEDNESS: double
TOPOLOGY: linear
ANTI-SENSE: No
ORIGINAL SOURCE

ORGANISM: Hepatitis C virus
IMMEDIATE EXPERIMENTAL SOURCE
CLONE: N17-1, N17-2, N17-3, H17-1, H17-3

```
TGT GAG CCC GAA CCG GAT GTA ACA GTG STC ACY TCC ATG CTC ACC GAC    48
Cys Glu Pro Glu Pro Asp Val Thr Val Xaa Thr Ser Met Leu Thr Asp
 1               5                  10                  15
CCC TCC CAC ATY ACA GCA GAG RCG GCT RRG CGT AGG CTG RCC AGA GGG    96
Pro Ser His Ile Thr Ala Glu Xab Ala Xac Arg Arg Leu Xab Arg Gly
            20                  25                  30
TCT CCY CCT YCY TYG RCC AGY TCT TCA GCT AGY CAG TTG TCT GCG CYH   144
Ser Pro Pro Xad Xae Xab Ser Ser Ser Ala Ser Gln Leu Ser Ala Xaf
        35                  40                  45
TCY YYG MAG GCR ACA TGY ACT ACC CAT CAD GRC KCC CCR GAC RCT GAC   192
Ser Xae Xag Ala Thr Cys Thr Thr His Xah Xai Xaj Pro Asp Xab Asp
    50                  55                  60
CTC ATC GAG GCC AAC CTC CTR TGG CGG CAG GAG ATG GGM GGR AAC ATC   240
Leu Ile Glu Ala Asn Leu Leu Trp Arg Gln Glu Met Gly Gly Asn Ile
 65                 70                  75                  80
ACC CGY GTG GAG TYA GAG ARC AAG RTA GTR ATT CTR GAC TCT TYY GAM   288
Thr Arg Val Glu Xae Glu Xak Lys Xal Val Ile Leu Asp Ser Xam Xan
                85                  90                  95
CCG CTT CGA GCG GAG GAG GAT G A                                   311
Pro Leu Arg Ala Glu Glu Asp
            100
```

```
Y : C or T        R : A or G            M : A or C          K : G or T
S : G or C        H : A or T or C       D : G or T or A
Xaa : Val or Leu             Xab : Ala or Thr
Xac : Arg or Lys or Gly      Xad : Pro or Ser         Xae : Ser or Leu
Xaf : Pro or Leu             Xag : Gln or Lys         Xah : Gln or His
Xai : Gly or Asp             Xaj : Ala or Ser         Xak : Asn or Ser
Xal : Ile or Val             Xam : Phe or Ser         Xan : Glu or Asp
```

SEQ ID NO:66
SEQUENCE LENGTH: 740 base pairs
SEQUENCE TYPE: nucleic acid

STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O28-1, O28-2, O28-4

```
GTG GTA GTC CTG GAC TCG TTG GAS CCG CTT CRA GCG RAG GAA GRT GAG   48
Val Val Val Leu Asp Ser Leu Xaa Pro Leu Xab Ala Xac Glu Xad Glu
  1               5                  10                  15
AGG GAA GTG TCC GTT GCG GCG GAG ATC CTG CGR AAG ACC ARG AAA TTC   96
Arg Glu Val Ser Val Ala Ala Glu Ile Leu Arg Lys Thr Xae Lys Phe
                 20                  25                  30
CCC GCA GCG ATG CCC GTA TGG GCA CGC CCG GAC TAC AAC CCA CCA TTA  144
Pro Ala Ala Met Pro Val Trp Ala Arg Pro Asp Tyr Asn Pro Pro Leu
             35                  40                  45
CTA GAG TCT TGG AAG AAC CCG GAC TAC GTC CCT CCR GTG GTA CAC GGG  192
Leu Glu Ser Trp Lys Asn Pro Asp Tyr Val Pro Pro Val Val His Gly
         50                  55                  60
TGC CCA TTG CCG CCT AYC AAG GCC CCT CCA ATA CCA CCT CCA CGR AGA  240
Cys Pro Leu Pro Pro Xaf Lys Ala Pro Pro Ile Pro Pro Pro Arg Arg
 65                  70                  75                  80
AAG AGR ACG GTT GYC CTG ACA GAA TCC WCC GTG TCC TCT GCC TTG GCG  288
Lys Arg Thr Val Xag Leu Thr Glu Ser Xah Val Ser Ser Ala Leu Ala
                 85                  90                  95
GAG CTT GCT ACA AAG ACC TTT GGC AGT TCC GGA TCG TCG GCC GTC GAC  336
Glu Leu Ala Thr Lys Thr Phe Gly Ser Ser Gly Ser Ser Ala Val Asp
            100                 105                 110
AGC GGC ACG GCG ACY GGC CCT CCT GAC CAG GCC TCC GCC GAA GGA GAT  384
Ser Gly Thr Ala Thr Gly Pro Pro Asp Gln Ala Ser Ala Glu Gly Asp
        115                 120                 125
GCA GGA TCC GAC GCT GAG TCG TAC TCC TCC ATG CCC CCC CTT GAG GGA  432
Ala Gly Ser Asp Ala Glu Ser Tyr Ser Ser Met Pro Pro Leu Glu Gly
    130                 135                 140
GAG CCG GGG GAC CCY GAT CTC AGC GAC GGG TCT TGG TCT ACY GTA AGC  480
Glu Pro Gly Asp Pro Asp Leu Ser Asp Gly Ser Trp Ser Thr Val Ser
```

```
145                  150                  155                  160
GAG GAG GCC RGC GAG GAC GTC GTC TGC TGC TCG ATG TCC TAC ACA TGG  528
Glu Glu Ala Xai Glu Asp Val Val Cys Cys Ser Met Ser Tyr Thr Trp
                165                  170                  175
ACA GGC GCC TTA ATT ACA CCA TGC RCC GCG GAG GAG AGC AAG CTG CCC  576
Thr Gly Ala Leu Ile Thr Pro Cys Xaj Ala Glu Glu Ser Lys Leu Pro
            180                  185                  190
ATT AAT GCG CTG AGC AAC YCT TTG CTG CGY CAC CAC AAC ATG GTC TAT  624
Ile Asn Ala Leu Ser Asn Xak Leu Leu Arg His His Asn Met Val Tyr
        195                  200                  205
GCC ACA ACA TCC CGC AGC GCA AGC CAG CGG CAG AAA AAG GTC ACA TTT  672
Ala Thr Thr Ser Arg Ser Ala Ser Gln Arg Gln Lys Lys Val Thr Phe
    210                  215                  220
GAC AGA CTG CAA GTC CTG GAT GAC CAC TAC CGG GAC GTG CTC AAG GAC  720
Asp Arg Leu Gln Val Leu Asp Asp His Tyr Arg Asp Val Leu Lys Asp
225                  230                  235                  240
ATG AAG GCC AAG GCG TCC AC                                       740
Met Lys Ala Lys Ala Ser
                245
Y : C or T      R : A or G        S : G or C       W : A or T
Xaa : Glu or Asp          Xab : Gln or Arg    Xac : Lys or Glu
Xad : Gly or Asp          Xae : Arg or Lys    Xaf : Thr or Ile
Xag : Val or Ala          Xah : Ser or Thr    Xai : Ser or Gly
Xaj : Ala or Thr          Xak : Pro or Ser
```

SEQ ID NO:67
SEQUENCE LENGTH: 515 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N29-1, N29-2, N29-3

```
AC TAC CGG GAC GTG CTG AAG GAG ATG AAG GCG AAG GCG TCC ACA GTT   47
```

```
    Tyr Arg Asp Val Leu Lys Glu Met Lys Ala Lys Ala Ser Thr Val
      1               5                  10                  15
AAG GCT AAA CTT CTA TCT GTA GAG GAA GCC TGY AAG CTG ACG CCC CCA   95
Lys Ala Lys Leu Leu Ser Val Glu Glu Ala Cys Lys Leu Thr Pro Pro
                    20                  25                  30
CAC TCG GCC AGA TCT AAR TTT GGC TAC GGG GCA AAG GAC GTC CGG AGC  143
His Ser Ala Arg Ser Lys Phe Gly Tyr Gly Ala Lys Asp Val Arg Ser
                35                  40                  45
CTG TCC AGC AAG GCC GTT AAC CAC ATC CGC TCC GTG TGG ARG GAC TTG  191
Leu Ser Ser Lys Ala Val Asn His Ile Arg Ser Val Trp Xaa Asp Leu
            50                  55                  60
CTG GAA GAC ACT GAR ACA CCA ATT GAC ACC ACC ATC ATG GCA AAA AAT  239
Leu Glu Asp Thr Glu Thr Pro Ile Asp Thr Thr Ile Met Ala Lys Asn
    65                  70                  75
GAG GTT TTC TGT GTT CAA CCA GAG AAA GGA GGC CGC AAG CCA GCT CGC  287
Glu Val Phe Cys Val Gln Pro Glu Lys Gly Gly Arg Lys Pro Ala Arg
80                  85                  90                  95
CTT ATC GTA TTC CCA GAC TTG GGG GTT CGT GTG TGC GAG AAA ATG GCC  335
Leu Ile Val Phe Pro Asp Leu Gly Val Arg Val Cys Glu Lys Met Ala
                100                 105                 110
CTC TAC GAC GTG GTC TCC ACT CTT CCT CAG GCC GTG ATG GGC TCC TCA  383
Leu Tyr Asp Val Val Ser Thr Leu Pro Gln Ala Val Met Gly Ser Ser
            115                 120                 125
TAC GGA TTC CAG TAC TCC CCT GGA CAG CGG GTC GAG TTC CTG GTG AAT  431
Tyr Gly Phe Gln Tyr Ser Pro Gly Gln Arg Val Glu Phe Leu Val Asn
        130                 135                 140
GCC TGG AAG TCA AAG AAG AGY CCT ATG GGC TTT KCA TAT GAC ACC CGC  479
Ala Trp Lys Ser Lys Lys Ser Pro Met Gly Phe Xab Tyr Asp Thr Arg
    145                 150                 155
TGT TTT GAC TCA ACG GTC ACC GAG AAC GAC ATC CGT                  515
Cys Phe Asp Ser Thr Val Thr Glu Asn Asp Ile Arg
160                 165                 170
Y : C or T      R : A or G        K : G or T
Xaa : Lys or Glu          Xab : Ala or Ser
```

SEQ ID NO:68

SEQUENCE LENGTH: 401 base pairs

SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N18-2, N18-3, N18-4, H18-1, H18-2, H18-3

```
TG GGG ATC CCG TAT GAT ACC CGC TGC TTT GAC TCA ACR GTC ACY GAG     47
   Gly Ile Pro Tyr Asp Thr Arg Cys Phe Asp Ser Thr Val Thr Glu
    1               5                  10                  15
ARY GAY ATC CGT RYT GAG GAG TCA ATY TAY CAA TGY TGT GAC TTG GHC    95
Xaa Asp Ile Arg Xab Glu Glu Ser Ile Tyr Gln Cys Cys Asp Leu Xac
                 20                  25                  30
CCC GAG GCC AGA CAG GCY ATA AGG TCG CTC ACA GAG CGG CTT TAT ATC   143
Pro Glu Ala Arg Gln Ala Ile Arg Ser Leu Thr Glu Arg Leu Tyr Ile
             35                  40                  45
GGG GGC CCC YTG ACY AAT TCA AAR GGG CAR AAC TGC GGY TAT CGC CGG   191
Gly Gly Pro Leu Thr Asn Ser Lys Gly Gln Asn Cys Gly Tyr Arg Arg
         50                  55                  60
TGC CGC GYC AGC GGC GTG CTG ACG ACY AGC TGC GGT AAT ACY CTY ACA   239
Cys Arg Xad Ser Gly Val Leu Thr Thr Ser Cys Gly Asn Thr Leu Thr
     65                  70                  75
TGT TAC TTG AAG GCC TCT GCA GCC TGT CGA GCT GCR AAG CTC CRG GAC   287
Cys Tyr Leu Lys Ala Ser Ala Ala Cys Arg Ala Ala Lys Leu Xae Asp
 80                  85                  90                  95
TGC ACR ATG CTC GTG TGC GGR GAC GAC CTT GTC GTY ATC TGT GAR AGC   335
Cys Thr Met Leu Val Cys Gly Asp Asp Leu Val Val Ile Cys Glu Ser
                100                 105                 110
GCG GGR ACC CAG GAG GAC GCG GCR ARC CTA CGA GTC TTC ACG GAG GCT   383
Ala Gly Thr Gln Glu Asp Ala Ala Xaa Leu Arg Val Phe Thr Glu Ala
            115                 120                 125
ATG ACC AGG AAT TCC GCC                                           401
Met Thr Arg Asn Ser Ala
        130
```

Y : C or T    R : A or G    H : A or T or C

Xaa : Asn or Ser Xab : Thr or Ile or Val
Xac : Asp or Val or Ala Xad : Ala or Val Xae : Gln or Arg

SEQ ID NO:69
SEQUENCE LENGTH: 1171 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O30

```
TG GGG ATC CCG TAT GAT ACC CGC TGC TTT GAC TCA ACR GTC ACT GAG     47
   Gly Ile Pro Tyr Asp Thr Arg Cys Phe Asp Ser Thr Val Thr Glu
     1               5                  10                  15
AAT GAC ATC CGT GTY GAG GAG TCA ATT TAC CAA TGT TGT GAC TTG GCC    95
Asn Asp Ile Arg Val Glu Glu Ser Ile Tyr Gln Cys Cys Asp Leu Ala
                20                  25                  30
CCC GAG GCC AGA CAG GCC ATA AGG TCR CTC ACA GAG CGG CTT TAC ATC   143
Pro Glu Ala Arg Gln Ala Ile Arg Ser Leu Thr Glu Arg Leu Tyr Ile
            35                  40                  45
GGG GGC CCC CTG ACT AAT TCA AAR GGG CAG AAC TGC GGY TAT CGC CGG   191
Gly Gly Pro Leu Thr Asn Ser Lys Gly Gln Asn Cys Gly Tyr Arg Arg
        50                  55                  60
TGC CGC GYC AGC GGC GTG CTG ACG ACT AGC TGC GGY AAT ACC CTC ACA   239
Cys Arg Xaa Ser Gly Val Leu Thr Thr Ser Cys Gly Asn Thr Leu Thr
    65                  70                  75
TGT TAC TTG AAG GCC TCT GCA GCC TGT CGA GCT GCA AAG CTC CAG GAC   287
Cys Tyr Leu Lys Ala Ser Ala Ala Cys Arg Ala Ala Lys Leu Gln Asp
80                  85                  90                  95
TGC ACG ATG CTT GTG TGC GGA GAC GAC CTT GTC GTT ATC TGT GAW AGC   335
Cys Thr Met Leu Val Cys Gly Asp Asp Leu Val Val Ile Cys Xab Ser
                100                 105                 110
GCG GGA ACT CAG GAG GAC GCG GCG AGC CTA CGA GTC TTC ACG GAG GCT   383
Ala Gly Thr Gln Glu Asp Ala Ala Ser Leu Arg Val Phe Thr Glu Ala
            115                 120                 125
```

```
ATG ACT AGG TAC TCT GCC CCC CCC GGG GAC CCG CCC CAA CCA GAA TAC  431
Met Thr Arg Tyr Ser Ala Pro Pro Gly Asp Pro Pro Gln Pro Glu Tyr
        130                 135                 140
GAC TTG GAG CTG ATA ACA TCA TGY TCC TCC AAY GTG TCG GTC GCG CAC  479
Asp Leu Glu Leu Ile Thr Ser Cys Ser Ser Asn Val Ser Val Ala His
    145                 150                 155
GAC GCA TCA GGC AAA CGG GTG TAC TAY CTC ACC CGT GAC CCC MCC ACC  527
Asp Ala Ser Gly Lys Arg Val Tyr Tyr Leu Thr Arg Asp Pro Xac Thr
160                 165                 170                 175
CCC CTW GCG CGG GCT GCG TGG GAG ACA GCT AGA CAC ACT CCA GTC AAC  575
Pro Leu Ala Arg Ala Ala Trp Glu Thr Ala Arg His Thr Pro Val Asn
                180                 185                 190
TCC TGG CTA GGC AAC ATC ATC ATG TAY GCG CCC ACC TTA TGG GCA AGG  623
Ser Trp Leu Gly Asn Ile Ile Met Tyr Ala Pro Thr Leu Trp Ala Arg
            195                 200                 205
ATG ATT CTG ATG ACC CAC TTC TTC TCC ATC CTT CTA GCC CAG GAG CAA  671
Met Ile Leu Met Thr His Phe Phe Ser Ile Leu Leu Ala Gln Glu Gln
        210                 215                 220
CTT GAA AAA GCC CTA GAT TGT CAG ATC TAY GGG GCC ACT TAC TCC ATT  719
Leu Glu Lys Ala Leu Asp Cys Gln Ile Tyr Gly Ala Thr Tyr Ser Ile
    225                 230                 235
GAG CCA CTT GAC CTA CCT CAG ATC ATT CAA CGA CTC CAY GGT CTT AGC  767
Glu Pro Leu Asp Leu Pro Gln Ile Ile Gln Arg Leu His Gly Leu Ser
240                 245                 250                 255
GCA TTT TCA CTC CAT AGT TAC TCT CCA GGT GAG ATC AAT AGG GTG GCT  815
Ala Phe Ser Leu His Ser Tyr Ser Pro Gly Glu Ile Asn Arg Val Ala
                260                 265                 270
TCA TGC CTC AGG AAA CTT GGG GTA CCG CCC TTG CGA GTC TGG AGA CAT  863
Ser Cys Leu Arg Lys Leu Gly Val Pro Pro Leu Arg Val Trp Arg His
            275                 280                 285
CGG GCC AGA AGC GTC CGC GCT AAG CTA CTG TCC CAG GGG GGG AGG GCC  911
Arg Ala Arg Ser Val Arg Ala Lys Leu Leu Ser Gln Gly Gly Arg Ala
        290                 295                 300
GCC ACC TGT GGC AAA TAC CTC TTC AAC TGG GCA GTA AAG ACC AAG CTC  959
Ala Thr Cys Gly Lys Tyr Leu Phe Asn Trp Ala Val Lys Thr Lys Leu
    305                 310                 315
AAA CYC ACT CCA ATC CCR GAA GCG TCC CAG CTG GAC TTG TCC GGC TGG 1007
```

```
Lys Xad Thr Pro Ile Pro Glu Ala Ser Gln Leu Asp Leu Ser Gly Trp
320                 325                 330                 335
TTC GTT GCT GGT TAC AGC GGG GGA GAC ATA TAT CAC AGC CTG TCT CGT 1055
Phe Val Ala Gly Tyr Ser Gly Gly Asp Ile Tyr His Ser Leu Ser Arg
                340                 345                 350
GCC CGA CCC CGC TGG TTY ATG TGG TGC CTA CTC CTA CTT TCC GTA GGG 1103
Ala Arg Pro Arg Trp Phe Met Trp Cys Leu Leu Leu Leu Ser Val Gly
            355                 360                 365
GTA GGC ATC TAC CTG CTC CCC AAC CGA TGA GCG GGG AGC TAA ACA CTC 1151
Val Gly Ile Tyr Leu Leu Pro Asn Arg StopAla Gly Ser StopThr Leu
        370                 375                 380
CAG GCC AAT AGG CCA TCC C C                                     1171
Gln Ala Asn Arg Pro Ser
    385
Y : C or T      R : A or G         M : A or C      W : A or T
Xaa : Val or Ala          Xab : Asp or Glu    Xac : Thr or Pro
Xad : Leu or Pro
```

SEQ ID NO:70
SEQUENCE LENGTH: 1084 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: 2217

```
GG CAT GTG GGC CCA GGG GAG GGG GCT GTG CAG TGG ATG AAC CGG CTG    47
   His Val Gly Pro Gly Glu Gly Ala Val Gln Trp Met Asn Arg Leu
     1               5                  10                  15
ATA GCG TTT GCT TCG CGG GGC AAC CAT GTC TCC CCC ACG CAC TAT GTG   95
Ile Ala Phe Ala Ser Arg Gly Asn His Val Ser Pro Thr His Tyr Val
                20                  25                  30
CCT GAA AGC GAC GCC GCA GCG CGC GTC ACC CAG ATC CTC TCC AAC CTT  143
Pro Glu Ser Asp Ala Ala Ala Arg Val Thr Gln Ile Leu Ser Asn Leu
```

35 40 45

ACC ATC ACT CAG CTG TTG AAG AGG CTT CAC CAG TGG ATT AAT GAG GAC 191

Thr Ile Thr Gln Leu Leu Lys Arg Leu His Gln Trp Ile Asn Glu Asp

50 55 60

TGC TCC ACG CCA TGC TCC GGC TCG TGG CTC AGG GAT GTT TGG GAC TGG 239

Cys Ser Thr Pro Cys Ser Gly Ser Trp Leu Arg Asp Val Trp Asp Trp

65 70 75

ATA TGC ACG GTA TTG GCT GAT TTC AAG ACC TGG CTC CAG TCC AAG CTC 287

Ile Cys Thr Val Leu Ala Asp Phe Lys Thr Trp Leu Gln Ser Lys Leu

80 85 90 95

CTG CCG CGG TTA CCG GGG GTC CCT TTT TTC TCA TGC CAG CGT GGG TAC 335

Leu Pro Arg Leu Pro Gly Val Pro Phe Phe Ser Cys Gln Arg Gly Tyr

100 105 110

AAG GGG GTT TGG CGG GGA GAT GGC ATC ATG TAT ACC ACC TGC CCA TGT 383

Lys Gly Val Trp Arg Gly Asp Gly Ile Met Tyr Thr Thr Cys Pro Cys

115 120 125

GGA GCA CAA ATC ACC GGA CAT GTC AAA AAC GGT TCT ATG AGG ATC GTT 431

Gly Ala Gln Ile Thr Gly His Val Lys Asn Gly Ser Met Arg Ile Val

130 135 140

GGG CCT AGA ACC TGT AGC AAC ACG TGG CAC GGA ACA TTT CCC ATC AAC 479

Gly Pro Arg Thr Cys Ser Asn Thr Trp His Gly Thr Phe Pro Ile Asn

145 150 155

GCG TAC ACC ACA GGC CCC TGC ACA CCC TCC CCG GCG CCA AAC TAT TCC 527

Ala Tyr Thr Thr Gly Pro Cys Thr Pro Ser Pro Ala Pro Asn Tyr Ser

160 165 170 175

AGG GCG TTG TGG CGG GTG GCC ATT GAG GAG TAT GTG GAG GTC ACG CGG 575

Arg Ala Leu Trp Arg Val Ala Ile Glu Glu Tyr Val Glu Val Thr Arg

180 185 190

GTG GGG GAT TTC CAC TAC GTG ACG GGC ATG ACC ACT GAC AAC GTG AAA 623

Val Gly Asp Phe His Tyr Val Thr Gly Met Thr Thr Asp Asn Val Lys

195 200 205

TGC CCA TGC CAG GTT CCG GCC CCC GAA TTC TTC ACA GAA TTG GAT GGG 671

Cys Pro Cys Gln Val Pro Ala Pro Glu Phe Phe Thr Glu Leu Asp Gly

210 215 220

GTG CGG CTG CAC AGG TAC GCT CCG GCG TGC AAA CCT CTC CTG CGG GAT 719

Val Arg Leu His Arg Tyr Ala Pro Ala Cys Lys Pro Leu Leu Arg Asp

225 230 235

```
GAG GTC ACA TTC CAG GTC GGG CTC AAC CAA TAT ACG GTT GGG TCA CAG  767
Glu Val Thr Phe Gln Val Gly Leu Asn Gln Tyr Thr Val Gly Ser Gln
240                 245                 250                 255
CTC CCA TGT GAG CCC GAA CCG GAT GTA ACA GTG GTC ACC TCC ATG CTC  815
Leu Pro Cys Glu Pro Glu Pro Asp Val Thr Val Val Thr Ser Met Leu
                260                 265                 270
ACC GAC CCC TCC CAC ATT ACA GCA GAG GCG GCT AGG CGT AGG CTG ACC  863
Thr Asp Pro Ser His Ile Thr Ala Glu Ala Ala Arg Arg Arg Leu Thr
            275                 280                 285
AGA GGG TCT CCC CCT TCC TCG ACC AGT TCT TCA GCT AGT CAG TTG TCT  911
Arg Gly Ser Pro Pro Ser Ser Thr Ser Ser Ser Ala Ser Gln Leu Ser
        290                 295                 300
GCG CTT TCT TCG CAG GCA ACA TGC ACT ACC CAT CAG GGC GCC CCA GAC  959
Ala Leu Ser Ser Gln Ala Thr Cys Thr Thr His Gln Gly Ala Pro Asp
    305                 310                 315
ACT GAC CTC ATC GAG GCC AAC CTC CTG TGG CGG CAG GAG ATG GGC GGA 1007
Thr Asp Leu Ile Glu Ala Asn Leu Leu Trp Arg Gln Glu Met Gly Gly
320                 325                 330                 335
AAC ATC ACC CGC GTG GAG TCA GAG AAC AAG ATA GTA ATT CTA GAC TCT 1055
Asn Ile Thr Arg Val Glu Ser Glu Asn Lys Ile Val Ile Leu Asp Ser
                340                 345                 350
TTT GAA CCG CTT CGA GCG GAG GAG GAT GA                          1084
Phe Glu Pro Leu Arg Ala Glu Glu Asp
            355                 360
```

SEQ ID NO:71
SEQUENCE LENGTH: 1004 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: 1728

```
TGT GAG CCC GAA CCG GAT GTA ACA GTG GTC ACC TCC ATG CTC ACC GAC   48
```

```
Cys Glu Pro Glu Pro Asp Val Thr Val Val Thr Ser Met Leu Thr Asp
  1               5                  10                  15
CCC TCC CAC ATT ACA GCA GAG GCG GCT AGG CGT AGG CTG ACC AGA GGG    96
Pro Ser His Ile Thr Ala Glu Ala Ala Arg Arg Arg Leu Thr Arg Gly
             20                  25                  30
TCT CCC CCT TCC TCG ACC AGT TCT TCA GCT AGT CAG TTG TCT GCG CTT   144
Ser Pro Pro Ser Ser Thr Ser Ser Ser Ala Ser Gln Leu Ser Ala Leu
         35                  40                  45
TCT TCG CAG GCA ACA TGC ACT ACC CAT CAG GGC GCC CCA GAC ACT GAC   192
Ser Ser Gln Ala Thr Cys Thr Thr His Gln Gly Ala Pro Asp Thr Asp
     50                  55                  60
CTC ATC GAG GCC AAC CTC CTG TGG CGG CAG GAG ATG GGC GGA AAC ATC   240
Leu Ile Glu Ala Asn Leu Leu Trp Arg Gln Glu Met Gly Gly Asn Ile
 65                  70                  75                  80
ACC CGC GTG GAG TCA GAG AAC AAG ATA GTA ATT CTA GAC TCT TTT GAA   288
Thr Arg Val Glu Ser Glu Asn Lys Ile Val Ile Leu Asp Ser Phe Glu
                 85                  90                  95
CCG CTT CGA GCG GAG GAG GAT GAG AGG GAA GTG TCC GTT GCG GCG GAG   336
Pro Leu Arg Ala Glu Glu Asp Glu Arg Glu Val Ser Val Ala Ala Glu
            100                 105                 110
ATC CTG CGG AAG ACC AGG AAA TTC CCC GCA GCG ATG CCC GTA TGG GCA   384
Ile Leu Arg Lys Thr Arg Lys Phe Pro Ala Ala Met Pro Val Trp Ala
        115                 120                 125
CGC CCG GAC TAC AAC CCA CCA TTA CTA GAG TCT TGG AAG AAC CCG GAC   432
Arg Pro Asp Tyr Asn Pro Pro Leu Leu Glu Ser Trp Lys Asn Pro Asp
    130                 135                 140
TAC GTC CCT CCA GTG GTA CAC GGG TGC CCA TTG CCG CCT ACC AAG GCC   480
Tyr Val Pro Pro Val Val His Gly Cys Pro Leu Pro Pro Thr Lys Ala
145                 150                 155                 160
CCT CCA ATA CCA CCT CCA CGA AGA AAG AGA ACG GTT GTC CTG ACA GAA   528
Pro Pro Ile Pro Pro Pro Arg Arg Lys Arg Thr Val Val Leu Thr Glu
                165                 170                 175
TCC TCC GTG TCC TCT GCC TTG GCG GAG CTT GCT ACA AAG ACC TTT GGC   576
Ser Ser Val Ser Ser Ala Leu Ala Glu Leu Ala Thr Lys Thr Phe Gly
            180                 185                 190
AGT TCC GGA TCG TCG GCC GTC GAC AGC GGC ACG GCG ACC GGC CCT CCT   624
Ser Ser Gly Ser Ser Ala Val Asp Ser Gly Thr Ala Thr Gly Pro Pro
```

```
        195                 200                 205
GAC CAG GCC TCC GCC GAA GGA GAT GCA GGA TCC GAC GCT GAG TCG TAC   672
Asp Gln Ala Ser Ala Glu Gly Asp Ala Gly Ser Asp Ala Glu Ser Tyr
    210                 215                 220
TCC TCC ATG CCC CCC CTT GAG GGA GAG CCG GGG GAC CCC GAT CTC AGC   720
Ser Ser Met Pro Pro Leu Glu Gly Glu Pro Gly Asp Pro Asp Leu Ser
225                 230                 235                 240
GAC GGG TCT TGG TCT ACC GTA AGC GAG GAG GCC AGC GAG GAC GTC GTC   768
Asp Gly Ser Trp Ser Thr Val Ser Glu Glu Ala Ser Glu Asp Val Val
                245                 250                 255
TGC TGC TCG ATG TCC TAC ACA TGG ACA GGC GCC TTA ATT ACA CCA TGC   816
Cys Cys Ser Met Ser Tyr Thr Trp Thr Gly Ala Leu Ile Thr Pro Cys
            260                 265                 270
GCC GCG GAG GAG AGC AAG CTG CCC ATT AAT GCG CTG AGC AAC CCT TTG   864
Ala Ala Glu Glu Ser Lys Leu Pro Ile Asn Ala Leu Ser Asn Pro Leu
        275                 280                 285
CTG CGC CAC CAC AAC ATG GTC TAT GCC ACA ACA TCC CGC AGC GCA AGC   912
Leu Arg His His Asn Met Val Tyr Ala Thr Thr Ser Arg Ser Ala Ser
    290                 295                 300
CAG CGG CAG AAA AAG GTC ACA TTT GAC AGA CTG CAA GTC CTG GAT GAC   960
Gln Arg Gln Lys Lys Val Thr Phe Asp Arg Leu Gln Val Leu Asp Asp
305                 310                 315                 320
CAC TAC CGG GAC GTG CTC AAG GAC ATG AAG GCC AAG GCG TCC AC       1004
His Tyr Arg Asp Val Leu Lys Asp Met Lys Ala Lys Ala Ser
                325                 330
```

SEQ ID NO:72
SEQUENCE LENGTH: 857 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: 2918

```
AC TAC CGG GAC GTG CTG AAG GAC ATG AAG GCC AAG GCG TCC ACA GTT    47
   Tyr Arg Asp Val Leu Lys Asp Met Lys Ala Lys Ala Ser Thr Val
     1               5                  10                  15
AAG GCT AAA CTT CTA TCT GTA GAG GAA GCC TGC AAG CTG ACG CCC CCA   95
Lys Ala Lys Leu Leu Ser Val Glu Glu Ala Cys Lys Leu Thr Pro Pro
                20                  25                  30
CAC TCG GCC AGA TCT AAA TTT GAC TAC GGG GCA AAG GAC GTC CAG AGC  143
His Ser Ala Arg Ser Lys Phe Asp Tyr Gly Ala Lys Asp Val Gln Ser
            35                  40                  45
CTG TCC AGC AAG GCC GTT AAC CAC ATC CAC TCC GTG TGG AAG GAC TTG  191
Leu Ser Ser Lys Ala Val Asn His Ile His Ser Val Trp Lys Asp Leu
        50                  55                  60
CCG GAA GAC ACT GAG ACA CCA ATC GAC ACC ACC ATC ATG GCA AAA AAT  239
Pro Glu Asp Thr Glu Thr Pro Ile Asp Thr Thr Ile Met Ala Lys Asn
    65                  70                  75
GAG GTT TTT TGT GTT CAA CCA GAG AAA GGA GGC CGC AAG CCA GCT CGC  287
Glu Val Phe Cys Val Gln Pro Glu Lys Gly Gly Arg Lys Pro Ala Arg
 80                  85                  90                  95
CTT ATC GTA TTC CCA GAC TTG GGG GTT CGT GTG TGC GAG AAA ATG GCC  335
Leu Ile Val Phe Pro Asp Leu Gly Val Arg Val Cys Glu Lys Met Ala
                100                 105                 110
CTC TAC GAC GTG GTC TCC ACT CTT CCT CAG GCC GTG ATG GGC TCC TCA  383
Leu Tyr Asp Val Val Ser Thr Leu Pro Gln Ala Val Met Gly Ser Ser
            115                 120                 125
TAC AGA TTT CAG TGC TCC CCT GGA CAG CGG GTC GAG TTC CTG GTG AAT  431
Tyr Arg Phe Gln Cys Ser Pro Gly Gln Arg Val Glu Phe Leu Val Asn
        130                 135                 140
GCC TGG AAG TCA AAG AAG AGC CCT ATG GGC TTT GCA TAT GAC ACC CGC  479
Ala Trp Lys Ser Lys Lys Ser Pro Met Gly Phe Ala Tyr Asp Thr Arg
    145                 150                 155
TGT TTT GAC TCA ACG GTC ACC GAG AAC GAC ATC CGT ACT GAG GAG TCA  527
Cys Phe Asp Ser Thr Val Thr Glu Asn Asp Ile Arg Thr Glu Glu Ser
160                 165                 170                 175
ATT TAT CAA TGT TGT GAC TTG GAC CCC GAG GCC AGA CAG GCC ATA AGG  575
Ile Tyr Gln Cys Cys Asp Leu Asp Pro Glu Ala Arg Gln Ala Ile Arg
                180                 185                 190
TCG CTC ACA GAG CGG CTT TAT ATC GGG GGC CCC TTG ACC AAT TCA AAA  623
```

```
Ser Leu Thr Glu Arg Leu Tyr Ile Gly Gly Pro Leu Thr Asn Ser Lys
            195                 200                 205
GGG CAA AAC TGC GGC TAT CGC CGG TGC CGC GCC AGC GGC GTG CTG ACG   671
Gly Gln Asn Cys Gly Tyr Arg Arg Cys Arg Ala Ser Gly Val Leu Thr
        210                 215                 220
ACT AGC TGC GGT AAT ACC CTC ACA TGT TAC TTG AAG GCC TCT GCA GCC   719
Thr Ser Cys Gly Asn Thr Leu Thr Cys Tyr Leu Lys Ala Ser Ala Ala
    225                 230                 235
TGT CGA GCT GCG AAG CTC CAG GAC TGC ACG ATG CTC GTG TGC GGA GAC   767
Cys Arg Ala Ala Lys Leu Gln Asp Cys Thr Met Leu Val Cys Gly Asp
240                 245                 250                 255
GAC CTT GTC GTT ATC TGT GAA AGC GCG GGA ACC CAG GAG GAC GCG GCA   815
Asp Leu Val Val Ile Cys Glu Ser Ala Gly Thr Gln Glu Asp Ala Ala
                260                 265                 270
AAC CTA CGA GTC TTC ACG GAG GCT ATG ACC AGG AAT TCC GCC           857
Asn Leu Arg Val Phe Thr Glu Ala Met Thr Arg Asn Ser Ala
            275                 280                 285
```

SEQ ID NO:73
SEQUENCE LENGTH: 1818 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: 1718

```
TGT GAG CCC GAA CCG GAT GTA ACA GTG GTC ACC TCC ATG CTC ACC GAC    48
Cys Glu Pro Glu Pro Asp Val Thr Val Val Thr Ser Met Leu Thr Asp
  1               5                  10                  15
CCC TCC CAC ATT ACA GCA GAG GCG GCT AGG CGT AGG CTG ACC AGA GGG    96
Pro Ser His Ile Thr Ala Glu Ala Ala Arg Arg Arg Leu Thr Arg Gly
            20                  25                  30
TCT CCC CCT TCC TCG ACC AGT TCT TCA GCT AGT CAG TTG TCT GCG CTT   144
Ser Pro Pro Ser Ser Thr Ser Ser Ser Ala Ser Gln Leu Ser Ala Leu
```

```
            35                                      40                                      45
TCT TCG CAG GCA ACA TGC ACT ACC CAT CAG GGC GCC CCA GAC ACT GAC   192
Ser Ser Gln Ala Thr Cys Thr Thr His Gln Gly Ala Pro Asp Thr Asp
        50                                      55                                      60
CTC ATC GAG GCC AAC CTC CTG TGG CGG CAG GAG ATG GGC GGA AAC ATC   240
Leu Ile Glu Ala Asn Leu Leu Trp Arg Gln Glu Met Gly Gly Asn Ile
    65                                      70                                      75                                      80
ACC CGC GTG GAG TCA GAG AAC AAG ATA GTA ATT CTA GAC TCT TTT GAA   288
Thr Arg Val Glu Ser Glu Asn Lys Ile Val Ile Leu Asp Ser Phe Glu
                                    85                                      90                                      95
CCG CTT CGA GCG GAG GAG GAT GAG AGG GAA GTG TCC GTT GCG GCG GAG   336
Pro Leu Arg Ala Glu Glu Asp Glu Arg Glu Val Ser Val Ala Ala Glu
                        100                                     105                                     110
ATC CTG CGG AAG ACC AGG AAA TTC CCC GCA GCG ATG CCC GTA TGG GCA   384
Ile Leu Arg Lys Thr Arg Lys Phe Pro Ala Ala Met Pro Val Trp Ala
            115                                     120                                     125
CGC CCG GAC TAC AAC CCA CCA TTA CTA GAG TCT TGG AAG AAC CCG GAC   432
Arg Pro Asp Tyr Asn Pro Pro Leu Leu Glu Ser Trp Lys Asn Pro Asp
        130                                     135                                     140
TAC GTC CCT CCA GTG GTA CAC GGG TGC CCA TTG CCG CCT ACC AAG GCC   480
Tyr Val Pro Pro Val Val His Gly Cys Pro Leu Pro Pro Thr Lys Ala
145                                     150                                     155                                     160
CCT CCA ATA CCA CCT CCA CGA AGA AAG AGA ACG GTT GTC CTG ACA GAA   528
Pro Pro Ile Pro Pro Pro Arg Arg Lys Arg Thr Val Val Leu Thr Glu
                                165                                     170                                     175
TCC TCC GTG TCC TCT GCC TTG GCG GAG CTT GCT ACA AAG ACC TTT GGC   576
Ser Ser Val Ser Ser Ala Leu Ala Glu Leu Ala Thr Lys Thr Phe Gly
                        180                                     185                                     190
AGT TCC GGA TCG TCG GCC GTC GAC AGC GGC ACG GCG ACC GGC CCT CCT   624
Ser Ser Gly Ser Ser Ala Val Asp Ser Gly Thr Ala Thr Gly Pro Pro
            195                                     200                                     205
GAC CAG GCC TCC GCC GAA GGA GAT GCA GGA TCC GAC GCT GAG TCG TAC   672
Asp Gln Ala Ser Ala Glu Gly Asp Ala Gly Ser Asp Ala Glu Ser Tyr
        210                                     215                                     220
TCC TCC ATG CCC CCC CTT GAG GGA GAG CCG GGG GAC CCC GAT CTC AGC   720
Ser Ser Met Pro Pro Leu Glu Gly Glu Pro Gly Asp Pro Asp Leu Ser
225                                     230                                     235                                     240
```

```
GAC GGG TCT TGG TCT ACC GTA AGC GAG GAG GCC AGC GAG GAC GTC GTC  768
Asp Gly Ser Trp Ser Thr Val Ser Glu Glu Ala Ser Glu Asp Val Val
                245                 250                 255
TGC TGC TCG ATG TCC TAC ACA TGG ACA GGC GCC TTA ATT ACA CCA TGC  816
Cys Cys Ser Met Ser Tyr Thr Trp Thr Gly Ala Leu Ile Thr Pro Cys
            260                 265                 270
GCC GCG GAG GAG AGC AAG CTG CCC ATT AAT GCG CTG AGC AAC CCT TTG  864
Ala Ala Glu Glu Ser Lys Leu Pro Ile Asn Ala Leu Ser Asn Pro Leu
        275                 280                 285
CTG CGC CAC CAC AAC ATG GTC TAT GCC ACA ACA TCC CGC AGC GCA AGC  912
Leu Arg His His Asn Met Val Tyr Ala Thr Thr Ser Arg Ser Ala Ser
    290                 295                 300
CAG CGG CAG AAA AAG GTC ACA TTT GAC AGA CTG CAA GTC CTG GAT GAC  960
Gln Arg Gln Lys Lys Val Thr Phe Asp Arg Leu Gln Val Leu Asp Asp
305                 310                 315                 320
CAC TAC CGG GAC GTG CTS AAG GAC ATG AAG GCC AAG GCG TCC ACA GTT 1008
His Tyr Arg Asp Val Xaa Lys Asp Met Lys Ala Lys Ala Ser Thr Val
                325                 330                 335
AAG GCT AAA CTT CTA TCT GTA GAG GAA GCC TGC AAG CTG ACG CCC CCA 1056
Lys Ala Lys Leu Leu Ser Val Glu Glu Ala Cys Lys Leu Thr Pro Pro
            340                 345                 350
CAC TCG GCC AGA TCT AAA TTT GAC TAC GGG GCA AAG GAC GTC CAG AGC 1104
His Ser Ala Arg Ser Lys Phe Asp Tyr Gly Ala Lys Asp Val Gln Ser
        355                 360                 365
CTG TCC AGC AAG GCC GTT AAC CAC ATC CAC TCC GTG TGG AAG GAC TTG 1152
Leu Ser Ser Lys Ala Val Asn His Ile His Ser Val Trp Lys Asp Leu
    370                 375                 380
CCG GAA GAC ACT GAG ACA CCA ATC GAC ACC ACC ATC ATG GCA AAA AAT 1200
Pro Glu Asp Thr Glu Thr Pro Ile Asp Thr Thr Ile Met Ala Lys Asn
385                 390                 395                 400
GAG GTT TTT TGT GTT CAA CCA GAG AAA GGA GGC CGC AAG CCA GCT CGC 1248
Glu Val Phe Cys Val Gln Pro Glu Lys Gly Gly Arg Lys Pro Ala Arg
                405                 410                 415
CTT ATC GTA TTC CCA GAC TTG GGG GTT CGT GTG TGC GAG AAA ATG GCC 1296
Leu Ile Val Phe Pro Asp Leu Gly Val Arg Val Cys Glu Lys Met Ala
            420                 425                 430
CTC TAC GAC GTG GTC TCC ACT CTT CCT CAG GCC GTG ATG GGC TCC TCA 1344
```

```
Leu Tyr Asp Val Val Ser Thr Leu Pro Gln Ala Val Met Gly Ser Ser
        435                 440                 445
TAC AGA TTT CAG TGC TCC CCT GGA CAG CGG GTC GAG TTC CTG GTG AAT 1392
Tyr Arg Phe Gln Cys Ser Pro Gly Gln Arg Val Glu Phe Leu Val Asn
    450                 455                 460
GCC TGG AAG TCA AAG AAG AGC CCT ATG GGC TTT GCA TAT GAC ACC CGC 1440
Ala Trp Lys Ser Lys Lys Ser Pro Met Gly Phe Ala Tyr Asp Thr Arg
465                 470                 475                 480
TGT TTT GAC TCA ACG GTC ACC GAG AAC GAC ATC CGT ACT GAG GAG TCA 1488
Cys Phe Asp Ser Thr Val Thr Glu Asn Asp Ile Arg Thr Glu Glu Ser
                485                 490                 495
ATT TAT CAA TGT TGT GAC TTG GAC CCC GAG GCC AGA CAG GCC ATA AGG 1536
Ile Tyr Gln Cys Cys Asp Leu Asp Pro Glu Ala Arg Gln Ala Ile Arg
            500                 505                 510
TCG CTC ACA GAG CGG CTT TAT ATC GGG GGC CCC TTG ACC AAT TCA AAA 1584
Ser Leu Thr Glu Arg Leu Tyr Ile Gly Gly Pro Leu Thr Asn Ser Lys
        515                 520                 525
GGG CAA AAC TGC GGC TAT CGC CGG TGC CGC GCC AGC GGC GTG CTG ACG 1632
Gly Gln Asn Cys Gly Tyr Arg Arg Cys Arg Ala Ser Gly Val Leu Thr
    530                 535                 540
ACT AGC TGC GGT AAT ACC CTC ACA TGT TAC TTG AAG GCC TCT GCA GCC 1680
Thr Ser Cys Gly Asn Thr Leu Thr Cys Tyr Leu Lys Ala Ser Ala Ala
545                 550                 555                 560
TGT CGA GCT GCG AAG CTC CAG GAC TGC ACG ATG CTC GTG TGC GGA GAC 1728
Cys Arg Ala Ala Lys Leu Gln Asp Cys Thr Met Leu Val Cys Gly Asp
                565                 570                 575
GAC CTT GTC GTT ATC TGT GAA AGC GCG GGA ACC CAG GAG GAC GCG GCA 1776
Asp Leu Val Val Ile Cys Glu Ser Ala Gly Thr Gln Glu Asp Ala Ala
            580                 585                 590
AAC CTA CGA GTC TTC ACG GAG GCT ATG ACC AGG AAT TCC GCC         1818
Asn Leu Arg Val Phe Thr Glu Ala Met Thr Arg Asn Ser Ala
        595                 600                 605
```

SEQ ID NO:74
SEQUENCE LENGTH: 2591 base pairs
SEQUENCE TYPE: nucleic acid
STRANDEDNESS: double

TOPOLOGY: linear
ANTI-SENSE: No
ORIGINAL SOURCE
ORGANISM: Hepatitis C virus
IMMEDIATE EXPERIMENTAL SOURCE
CLONE: 2218

```
GG CAT GTG GGC CCA GGG GAG GGG GCT GTG CAG TGG ATG AAC CGG CTG     47
   His Val Gly Pro Gly Glu Gly Ala Val Gln Trp Met Asn Arg Leu
     1               5                  10                  15
ATA GCG TTT GCT TCG CGG GGC AAC CAT GTC TCC CCC ACG CAC TAT GTG    95
Ile Ala Phe Ala Ser Arg Gly Asn His Val Ser Pro Thr His Tyr Val
                 20                  25                  30
CCT GAA AGC GAC GCC GCA GCG CGC GTC ACC CAG ATC CTC TCC AAC CTT   143
Pro Glu Ser Asp Ala Ala Ala Arg Val Thr Gln Ile Leu Ser Asn Leu
             35                  40                  45
ACC ATC ACT CAG CTG TTG AAG AGG CTT CAC CAG TGG ATT AAT GAG GAC   191
Thr Ile Thr Gln Leu Leu Lys Arg Leu His Gln Trp Ile Asn Glu Asp
         50                  55                  60
TGC TCC ACG CCA TGC TCC GGC TCG TGG CTC AGG GAT GTT TGG GAC TGG   239
Cys Ser Thr Pro Cys Ser Gly Ser Trp Leu Arg Asp Val Trp Asp Trp
     65                  70                  75
ATA TGC ACG GTA TTG GCT GAT TTC AAG ACC TGG CTC CAG TCC AAG CTC   287
Ile Cys Thr Val Leu Ala Asp Phe Lys Thr Trp Leu Gln Ser Lys Leu
 80                  85                  90                  95
CTG CCG CGG TTA CCG GGG GTC CCT TTT TTC TCA TGC CAG CGT GGG TAC   335
Leu Pro Arg Leu Pro Gly Val Pro Phe Phe Ser Cys Gln Arg Gly Tyr
                100                 105                 110
AAG GGG GTT TGG CGG GGA GAT GGC ATC ATG TAT ACC ACC TGC CCA TGT   383
Lys Gly Val Trp Arg Gly Asp Gly Ile Met Tyr Thr Thr Cys Pro Cys
            115                 120                 125
GGA GCA CAA ATC ACC GGA CAT GTC AAA AAC GGT TCT ATG AGG ATC GTT   431
Gly Ala Gln Ile Thr Gly His Val Lys Asn Gly Ser Met Arg Ile Val
        130                 135                 140
GGG CCT AGA ACC TGT AGC AAC ACG TGG CAC GGA ACA TTT CCC ATC AAC   479
Gly Pro Arg Thr Cys Ser Asn Thr Trp His Gly Thr Phe Pro Ile Asn
    145                 150                 155
```

```
GCG TAC ACC ACA GGC CCC TGC ACA CCC TCC CCG GCG CCA AAC TAT TCC  527
Ala Tyr Thr Thr Gly Pro Cys Thr Pro Ser Pro Ala Pro Asn Tyr Ser
160                 165                 170                 175
AGG GCG TTG TGG CGG GTG GCC ATT GAG GAG TAT GTG GAG GTC ACG CGG  575
Arg Ala Leu Trp Arg Val Ala Ile Glu Glu Tyr Val Glu Val Thr Arg
                180                 185                 190
GTG GGG GAT TTC CAC TAC GTG ACG GGC ATG ACC ACT GAC AAC GTG AAA  623
Val Gly Asp Phe His Tyr Val Thr Gly Met Thr Thr Asp Asn Val Lys
            195                 200                 205
TGC CCA TGC CAG GTT CCG GCC CCC GAA TTC TTC ACA GAA TTG GAT GGG  671
Cys Pro Cys Gln Val Pro Ala Pro Glu Phe Phe Thr Glu Leu Asp Gly
        210                 215                 220
GTG CGG CTG CAC AGG TAC GCT CCG GCG TGC AAA CCT CTC CTG CGG GAT  719
Val Arg Leu His Arg Tyr Ala Pro Ala Cys Lys Pro Leu Leu Arg Asp
    225                 230                 235
GAG GTC ACA TTC CAG GTC GGG CTC AAC CAA TAT ACG GTT GGG TCA CAG  767
Glu Val Thr Phe Gln Val Gly Leu Asn Gln Tyr Thr Val Gly Ser Gln
240                 245                 250                 255
CTC CCA TGT GAG CCC GAA CCG GAT GTA ACA GTG GTC ACC TCC ATG CTC  815
Leu Pro Cys Glu Pro Glu Pro Asp Val Thr Val Val Thr Ser Met Leu
                260                 265                 270
ACC GAC CCC TCC CAC ATT ACA GCA GAG GCG GCT AGG CGT AGG CTG ACC  863
Thr Asp Pro Ser His Ile Thr Ala Glu Ala Ala Arg Arg Arg Leu Thr
            275                 280                 285
AGA GGG TCT CCC CCT TCC TCG ACC AGT TCT TCA GCT AGT CAG TTG TCT  911
Arg Gly Ser Pro Pro Ser Ser Thr Ser Ser Ser Ala Ser Gln Leu Ser
        290                 295                 300
GCG CTT TCT TCG CAG GCA ACA TGC ACT ACC CAT CAG GGC GCC CCA GAC  959
Ala Leu Ser Ser Gln Ala Thr Cys Thr Thr His Gln Gly Ala Pro Asp
    305                 310                 315
ACT GAC CTC ATC GAG GCC AAC CTC CTG TGG CGG CAG GAG ATG GGC GGA 1007
Thr Asp Leu Ile Glu Ala Asn Leu Leu Trp Arg Gln Glu Met Gly Gly
320                 325                 330                 335
AAC ATC ACC CGC GTG GAG TCA GAG AAC AAG ATA GTA ATT CTA GAC TCT 1055
Asn Ile Thr Arg Val Glu Ser Glu Asn Lys Ile Val Ile Leu Asp Ser
                340                 345                 350
TTT GAA CCG CTT CGA GCG GAG GAG GAT GAG AGG GAA GTG TCC GTT GCG 1103
```

```
Phe Glu Pro Leu Arg Ala Glu Glu Asp Glu Arg Glu Val Ser Val Ala
            355                 360                 365
GCG GAG ATC CTG CGG AAG ACC AGG AAA TTC CCC GCA GCG ATG CCC GTA 1151
Ala Glu Ile Leu Arg Lys Thr Arg Lys Phe Pro Ala Ala Met Pro Val
        370                 375                 380
TGG GCA CGC CCG GAC TAC AAC CCA CCA TTA CTA GAG TCT TGG AAG AAC 1199
Trp Ala Arg Pro Asp Tyr Asn Pro Pro Leu Leu Glu Ser Trp Lys Asn
    385                 390                 395
CCG GAC TAC GTC CCT CCA GTG GTA CAC GGG TGC CCA TTG CCG CCT ACC 1247
Pro Asp Tyr Val Pro Pro Val Val His Gly Cys Pro Leu Pro Pro Thr
400                 405                 410                 415
AAG GCC CCT CCA ATA CCA CCT CCA CGA AGA AAG AGA ACG GTT GTC CTG 1295
Lys Ala Pro Pro Ile Pro Pro Pro Arg Arg Lys Arg Thr Val Val Leu
                420                 425                 430
ACA GAA TCC TCC GTG TCC TCT GCC TTG GCG GAG CTT GCT ACA AAG ACC 1343
Thr Glu Ser Ser Val Ser Ser Ala Leu Ala Glu Leu Ala Thr Lys Thr
            435                 440                 445
TTT GGC AGT TCC GGA TCG TCG GCC GTC GAC AGC GGC ACG GCG ACC GGC 1391
Phe Gly Ser Ser Gly Ser Ser Ala Val Asp Ser Gly Thr Ala Thr Gly
        450                 455                 460
CCT CCT GAC CAG GCC TCC GCC GAA GGA GAT GCA GGA TCC GAC GCT GAG 1439
Pro Pro Asp Gln Ala Ser Ala Glu Gly Asp Ala Gly Ser Asp Ala Glu
    465                 470                 475
TCG TAC TCC TCC ATG CCC CCC CTT GAG GGA GAG CCG GGG GAC CCC GAT 1487
Ser Tyr Ser Ser Met Pro Pro Leu Glu Gly Glu Pro Gly Asp Pro Asp
480                 485                 490                 495
CTC AGC GAC GGG TCT TGG TCT ACC GTA AGC GAG GAG GCC AGC GAG GAC 1535
Leu Ser Asp Gly Ser Trp Ser Thr Val Ser Glu Glu Ala Ser Glu Asp
                500                 505                 510
GTC GTC TGC TGC TCG ATG TCC TAC ACA TGG ACA GGC GCC TTA ATT ACA 1583
Val Val Cys Cys Ser Met Ser Tyr Thr Trp Thr Gly Ala Leu Ile Thr
            515                 520                 525
CCA TGC GCC GCG GAG GAG AGC AAG CTG CCC ATT AAT GCG CTG AGC AAC 1631
Pro Cys Ala Ala Glu Glu Ser Lys Leu Pro Ile Asn Ala Leu Ser Asn
        530                 535                 540
CCT TTG CTG CGC CAC CAC AAC ATG GTC TAT GCC ACA ACA TCC CGC AGC 1679
Pro Leu Leu Arg His His Asn Met Val Tyr Ala Thr Thr Ser Arg Ser
```

```
    545                 550                 555
GCA AGC CAG CGG CAG AAA AAG GTC ACA TTT GAC AGA CTG CAA GTC CTG 1727
Ala Ser Gln Arg Gln Lys Lys Val Thr Phe Asp Arg Leu Gln Val Leu
560                 565                 570                 575
GAT GAC CAC TAC CGG GAC GTG CTS AAG GAC ATG AAG GCC AAG GCG TCC 1775
Asp Asp His Tyr Arg Asp Val Xaa Lys Asp Met Lys Ala Lys Ala Ser
                580                 585                 590
ACA GTT AAG GCT AAA CTT CTA TCT GTA GAG GAA GCC TGC AAG CTG ACG 1823
Thr Val Lys Ala Lys Leu Leu Ser Val Glu Glu Ala Cys Lys Leu Thr
            595                 600                 605
CCC CCA CAC TCG GCC AGA TCT AAA TTT GAC TAC GGG GCA AAG GAC GTC 1871
Pro Pro His Ser Ala Arg Ser Lys Phe Asp Tyr Gly Ala Lys Asp Val
        610                 615                 620
CAG AGC CTG TCC AGC AAG GCC GTT AAC CAC ATC CAC TCC GTG TGG AAG 1919
Gln Ser Leu Ser Ser Lys Ala Val Asn His Ile His Ser Val Trp Lys
    625                 630                 635
GAC TTG CCG GAA GAC ACT GAG ACA CCA ATC GAC ACC ACC ATC ATG GCA 1967
Asp Leu Pro Glu Asp Thr Glu Thr Pro Ile Asp Thr Thr Ile Met Ala
640                 645                 650                 655
AAA AAT GAG GTT TTT TGT GTT CAA CCA GAG AAA GGA GGC CGC AAG CCA 2015
Lys Asn Glu Val Phe Cys Val Gln Pro Glu Lys Gly Gly Arg Lys Pro
                660                 665                 670
GCT CGC CTT ATC GTA TTC CCA GAC TTG GGG GTT CGT GTG TGC GAG AAA 2063
Ala Arg Leu Ile Val Phe Pro Asp Leu Gly Val Arg Val Cys Glu Lys
            675                 680                 685
ATG GCC CTC TAC GAC GTG GTC TCC ACT CTT CCT CAG GCC GTG ATG GGC 2111
Met Ala Leu Tyr Asp Val Val Ser Thr Leu Pro Gln Ala Val Met Gly
        690                 695                 700
TCC TCA TAC AGA TTT CAG TGC TCC CCT GGA CAG CGG GTC GAG TTC CTG 2159
Ser Ser Tyr Arg Phe Gln Cys Ser Pro Gly Gln Arg Val Glu Phe Leu
    705                 710                 715
GTG AAT GCC TGG AAG TCA AAG AAG AGC CCT ATG GGC TTT GCA TAT GAC 2207
Val Asn Ala Trp Lys Ser Lys Lys Ser Pro Met Gly Phe Ala Tyr Asp
720                 725                 730                 735
ACC CGC TGT TTT GAC TCA ACG GTC ACC GAG AAC GAC ATC CGT ACT GAG 2255
Thr Arg Cys Phe Asp Ser Thr Val Thr Glu Asn Asp Ile Arg Thr Glu
                740                 745                 750
```

```
GAG TCA ATT TAT CAA TGT TGT GAC TTG GAC CCC GAG GCC AGA CAG GCC 2303
Glu Ser Ile Tyr Gln Cys Cys Asp Leu Asp Pro Glu Ala Arg Gln Ala
            755                 760                 765
ATA AGG TCG CTC ACA GAG CGG CTT TAT ATC GGG GGC CCC TTG ACC AAT 2351
Ile Arg Ser Leu Thr Glu Arg Leu Tyr Ile Gly Gly Pro Leu Thr Asn
        770                 775                 780
TCA AAA GGG CAA AAC TGC GGC TAT CGC CGG TGC CGC GCC AGC GGC GTG 2399
Ser Lys Gly Gln Asn Cys Gly Tyr Arg Arg Cys Arg Ala Ser Gly Val
    785                 790                 795
CTG ACG ACT AGC TGC GGT AAT ACC CTC ACA TGT TAC TTG AAG GCC TCT 2447
Leu Thr Thr Ser Cys Gly Asn Thr Leu Thr Cys Tyr Leu Lys Ala Ser
800                 805                 810                 815
GCA GCC TGT CGA GCT GCG AAG CTC CAG GAC TGC ACG ATG CTC GTG TGC 2495
Ala Ala Cys Arg Ala Ala Lys Leu Gln Asp Cys Thr Met Leu Val Cys
                820                 825                 830
GGA GAC GAC CTT GTC GTT ATC TGT GAA AGC GCG GGA ACC CAG GAG GAC 2543
Gly Asp Asp Leu Val Val Ile Cys Glu Ser Ala Gly Thr Gln Glu Asp
            835                 840                 845
GCG GCA AAC CTA CGA GTC TTC ACG GAG GCT ATG ACC AGG AAT TCC GCC 2591
Ala Ala Asn Leu Arg Val Phe Thr Glu Ala Met Thr Arg Asn Ser Ala
        850                 855                 860
```

SEQ ID NO:75

SEQUENCE LENGTH: 4296 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: 1530U

```
GCGGATCCT CCA CCT CCA TCG TGG GAT CAA ATG TGG AAG TGT CTC ATA CGG 51
          Pro Pro Pro Ser Trp Asp Gln Met Trp Lys Cys Leu Ile Arg
           1               5                  10
CTG AAG CCT ACG CTA CAC GGG CCA ACG CCC CTG TTG TAT AGG TTA GGA   99
```

```
Leu Lys Pro Thr Leu His Gly Pro Thr Pro Leu Leu Tyr Arg Leu Gly
 15                  20                  25                  30
GCC GTT CAG AAC GAG GTT ACC CTC ACA CAC CCC ATA ACC AAG TTC ATC   147
Ala Val Gln Asn Glu Val Thr Leu Thr His Pro Ile Thr Lys Phe Ile
                 35                  40                  45
ATG GCA TGC ATG TCG GCT GAC CTA GAG GTC GTC ACT AGC ACT TGG GTG   195
Met Ala Cys Met Ser Ala Asp Leu Glu Val Val Thr Ser Thr Trp Val
             50                  55                  60
CTG GTA GGC GGG GTC CTC GCG GCT CTG GCC GCG TAC TGC CTG ACA ACG   243
Leu Val Gly Gly Val Leu Ala Ala Leu Ala Ala Tyr Cys Leu Thr Thr
         65                  70                  75
GGC AGC GTG GTC ATT GTG GGC AGG ATC ATC TTG TCC GGG AGG CCG GCC   291
Gly Ser Val Val Ile Val Gly Arg Ile Ile Leu Ser Gly Arg Pro Ala
     80                  85                  90
GTT ATT CCC GAC AGG GAA GTT CTC TAC CAA GAG TTC GAT GAA ATG GAA   339
Val Ile Pro Asp Arg Glu Val Leu Tyr Gln Glu Phe Asp Glu Met Glu
 95                 100                 105                 110
GAG TGC GCC TCG CAC CTC CCT TAC ATC GAA CAA GGA ATG CAG CTC GCC   387
Glu Cys Ala Ser His Leu Pro Tyr Ile Glu Gln Gly Met Gln Leu Ala
                115                 120                 125
GAG CAA TTC AAG CAG AAG GCG CTC GGT TTG CTG CAA ACA GCC ACC AAG   435
Glu Gln Phe Lys Gln Lys Ala Leu Gly Leu Leu Gln Thr Ala Thr Lys
            130                 135                 140
CAA GCG GAG GCT GCT GCT CCC GTG GTG GAG TCC AAG TGG CGA GCC CTT   483
Gln Ala Glu Ala Ala Ala Pro Val Val Glu Ser Lys Trp Arg Ala Leu
        145                 150                 155
GAG ACC TTC TGG GCG AAG CAC ATG TGG AAT TTC ATC AGC GGG ATA CAG   531
Glu Thr Phe Trp Ala Lys His Met Trp Asn Phe Ile Ser Gly Ile Gln
    160                 165                 170
TAC TTA GCA GGC TTG TCC ACT CTG CCT GGA AAC CCC GCA ATA GCA TCA   579
Tyr Leu Ala Gly Leu Ser Thr Leu Pro Gly Asn Pro Ala Ile Ala Ser
175                 180                 185                 190
CTG ATG GCA TTC ACA GCC TCT ATC ACC AGC CCG CTC ACC ACC CAA TAT   627
Leu Met Ala Phe Thr Ala Ser Ile Thr Ser Pro Leu Thr Thr Gln Tyr
                195                 200                 205
ACC CTC CTG TTT AAC ATC TTG GGG GGA TGG GTG GCC GCC CAA CTC GCC   675
Thr Leu Leu Phe Asn Ile Leu Gly Gly Trp Val Ala Ala Gln Leu Ala
```

```
              210                      215                      220
CCC CCC AGT GCC GCT TCA GCC TTC GTG GGC GCC GGT ATA GCT GGC GCG   723
Pro Pro Ser Ala Ala Ser Ala Phe Val Gly Ala Gly Ile Ala Gly Ala
            225                      230                      235
GCT GTT GGC AGC ATA GGC CTC GGG AAG GTG CTT GTG GAC ATT CTG GCG   771
Ala Val Gly Ser Ile Gly Leu Gly Lys Val Leu Val Asp Ile Leu Ala
        240                      245                      250
GGT TAT GGA GCA GGG GTG GCA GGC GCG CTC GTG GCC TTT AAG GTC ATG   819
Gly Tyr Gly Ala Gly Val Ala Gly Ala Leu Val Ala Phe Lys Val Met
255                      260                      265                      270
AGC GGT GAC ATG CCC TCC ACC GAG GAC CTG GTC AAC TTA CTC CCC GCC   867
Ser Gly Asp Met Pro Ser Thr Glu Asp Leu Val Asn Leu Leu Pro Ala
                    275                      280                      285
ATC CTC TCT CCT GGT GCC CTG GTC GTC GGG GTC GTG TGC GCA GCA ATA   915
Ile Leu Ser Pro Gly Ala Leu Val Val Gly Val Val Cys Ala Ala Ile
              290                      295                      300
CTG CGT CGG CAT GTG GGC CCA GGG GAG GGG GCT GTG CAG TGG ATG AAC   963
Leu Arg Arg His Val Gly Pro Gly Glu Gly Ala Val Gln Trp Met Asn
            305                      310                      315
CGG CTG ATA GCG TTT GCT TCG CGG GGC AAC CAT GTC TCC CCC ACG CAC  1011
Arg Leu Ile Ala Phe Ala Ser Arg Gly Asn His Val Ser Pro Thr His
        320                      325                      330
TAT GTG CCT GAA AGC GAC GCC GCA GCG CGC GTC ACC CAG ATC CTC TCC  1059
Tyr Val Pro Glu Ser Asp Ala Ala Ala Arg Val Thr Gln Ile Leu Ser
335                      340                      345                      350
AAC CTT ACC ATC ACT CAG CTG TTG AAG AGG CTT CAC CAG TGG ATT AAT  1107
Asn Leu Thr Ile Thr Gln Leu Leu Lys Arg Leu His Gln Trp Ile Asn
                    355                      360                      365
GAG GAC TGC TCC ACG CCA TGC TCC GGC TCG TGG CTC AGG GAT GTT TGG  1155
Glu Asp Cys Ser Thr Pro Cys Ser Gly Ser Trp Leu Arg Asp Val Trp
              370                      375                      380
GAC TGG ATA TGC ACG GTA TTG GCT GAT TTC AAG ACC TGG CTC CAG TCC  1203
Asp Trp Ile Cys Thr Val Leu Ala Asp Phe Lys Thr Trp Leu Gln Ser
            385                      390                      395
AAG CTC CTG CCG CGG TTA CCG GGG GTC CCT TTT TTC TCA TGC CAG CGT  1251
Lys Leu Leu Pro Arg Leu Pro Gly Val Pro Phe Phe Ser Cys Gln Arg
        400                      405                      410
```

```
GGG TAC AAG GGG GTT TGG CGG GGA GAT GGC ATC ATG TAT ACC ACC TGC 1299
Gly Tyr Lys Gly Val Trp Arg Gly Asp Gly Ile Met Tyr Thr Thr Cys
415                 420                 425                 430
CCA TGT GGA GCA CAA ATC ACC GGA CAT GTC AAA AAC GGT TCT ATG AGG 1347
Pro Cys Gly Ala Gln Ile Thr Gly His Val Lys Asn Gly Ser Met Arg
                435                 440                 445
ATC GTT GGG CCT AGA ACC TGT AGC AAC ACG TGG CAC GGA ACA TTT CCC 1395
Ile Val Gly Pro Arg Thr Cys Ser Asn Thr Trp His Gly Thr Phe Pro
            450                 455                 460
ATC AAC GCG TAC ACC ACA GGC CCC TGC ACA CCC TCC CCG GCG CCA AAC 1443
Ile Asn Ala Tyr Thr Thr Gly Pro Cys Thr Pro Ser Pro Ala Pro Asn
        465                 470                 475
TAT TCC AGG GCG TTG TGG CGG GTG GCC ATT GAG GAG TAT GTG GAG GTC 1491
Tyr Ser Arg Ala Leu Trp Arg Val Ala Ile Glu Glu Tyr Val Glu Val
    480                 485                 490
ACG CGG GTG GGG GAT TTC CAC TAC GTG ACG GGC ATG ACC ACT GAC AAC 1539
Thr Arg Val Gly Asp Phe His Tyr Val Thr Gly Met Thr Thr Asp Asn
495                 500                 505                 510
GTG AAA TGC CCA TGC CAG GTT CCG GCC CCC GAA TTC TTC ACA GAA TTG 1587
Val Lys Cys Pro Cys Gln Val Pro Ala Pro Glu Phe Phe Thr Glu Leu
                515                 520                 525
GAT GGG GTG CGG CTG CAC AGG TAC GCT CCG GCG TGC AAA CCT CTC CTG 1635
Asp Gly Val Arg Leu His Arg Tyr Ala Pro Ala Cys Lys Pro Leu Leu
            530                 535                 540
CGG GAT GAG GTC ACA TTC CAG GTC GGG CTC AAC CAA TAT ACG GTT GGG 1683
Arg Asp Glu Val Thr Phe Gln Val Gly Leu Asn Gln Tyr Thr Val Gly
        545                 550                 555
TCA CAG CTC CCA TGT GAG CCC GAA CCG GAT GTA ACA GTG GTC ACC TCC 1731
Ser Gln Leu Pro Cys Glu Pro Glu Pro Asp Val Thr Val Val Thr Ser
    560                 565                 570
ATG CTC ACC GAC CCC TCC CAC ATT ACA GCA GAG GCG GCT AGG CGT AGG 1779
Met Leu Thr Asp Pro Ser His Ile Thr Ala Glu Ala Ala Arg Arg Arg
575                 580                 585                 590
CTG ACC AGA GGG TCT CCC CCT TCC TCG ACC AGT TCT TCA GCT AGT CAG 1827
Leu Thr Arg Gly Ser Pro Pro Ser Ser Thr Ser Ser Ser Ala Ser Gln
                595                 600                 605
TTG TCT GCG CTT TCT TCG CAG GCA ACA TGC ACT ACC CAT CAG GGC GCC 1875
```

```
Leu Ser Ala Leu Ser Ser Gln Ala Thr Cys Thr Thr His Gln Gly Ala
            610                 615                 620
CCA GAC ACT GAC CTC ATC GAG GCC AAC CTC CTG TGG CGG CAG GAG ATG 1923
Pro Asp Thr Asp Leu Ile Glu Ala Asn Leu Leu Trp Arg Gln Glu Met
        625                 630                 635
GGC GGA AAC ATC ACC CGC GTG GAG TCA GAG AAC AAG ATA GTA ATT CTA 1971
Gly Gly Asn Ile Thr Arg Val Glu Ser Glu Asn Lys Ile Val Ile Leu
    640                 645                 650
GAC TCT TTT GAA CCG CTT CGA GCG GAG GAG GAT GAG AGG GAA GTG TCC 2019
Asp Ser Phe Glu Pro Leu Arg Ala Glu Glu Asp Glu Arg Glu Val Ser
655                 660                 665                 670
GTT GCG GCG GAG ATC CTG CGG AAG ACC AGG AAA TTC CCC GCA GCG ATG 2067
Val Ala Ala Glu Ile Leu Arg Lys Thr Arg Lys Phe Pro Ala Ala Met
                675                 680                 685
CCC GTA TGG GCA CGC CCG GAC TAC AAC CCA CCA TTA CTA GAG TCT TGG 2115
Pro Val Trp Ala Arg Pro Asp Tyr Asn Pro Pro Leu Leu Glu Ser Trp
            690                 695                 700
AAG AAC CCG GAC TAC GTC CCT CCA GTG GTA CAC GGG TGC CCA TTG CCG 2163
Lys Asn Pro Asp Tyr Val Pro Pro Val Val His Gly Cys Pro Leu Pro
        705                 710                 715
CCT ACC AAG GCC CCT CCA ATA CCA CCT CCA CGA AGA AAG AGA ACG GTT 2211
Pro Thr Lys Ala Pro Pro Ile Pro Pro Pro Arg Arg Lys Arg Thr Val
    720                 725                 730
GTC CTG ACA GAA TCC TCC GTG TCC TCT GCC TTG GCG GAG CTT GCT ACA 2259
Val Leu Thr Glu Ser Ser Val Ser Ser Ala Leu Ala Glu Leu Ala Thr
735                 740                 745                 750
AAG ACC TTT GGC AGT TCC GGA TCG TCG GCC GTC GAC AGC GGC ACG GCG 2307
Lys Thr Phe Gly Ser Ser Gly Ser Ser Ala Val Asp Ser Gly Thr Ala
                755                 760                 765
ACC GGC CCT CCT GAC CAG GCC TCC GCC GAA GGA GAT GCA GGA TCC GAC 2355
Thr Gly Pro Pro Asp Gln Ala Ser Ala Glu Gly Asp Ala Gly Ser Asp
            770                 775                 780
GCT GAG TCG TAC TCC TCC ATG CCC CCC CTT GAG GGA GAG CCG GGG GAC 2403
Ala Glu Ser Tyr Ser Ser Met Pro Pro Leu Glu Gly Glu Pro Gly Asp
        785                 790                 795
CCC GAT CTC AGC GAC GGG TCT TGG TCT ACC GTA AGC GAG GAG GCC AGC 2451
Pro Asp Leu Ser Asp Gly Ser Trp Ser Thr Val Ser Glu Glu Ala Ser
```

```
    800                 805                 810
GAG GAC GTC GTC TGC TGC TCG ATG TCC TAC ACA TGG ACA GGC GCC TTA 2499
Glu Asp Val Val Cys Cys Ser Met Ser Tyr Thr Trp Thr Gly Ala Leu
815                 820                 825                 830
ATT ACA CCA TGC GCC GCG GAG GAG AGC AAG CTG CCC ATT AAT GCG CTG 2547
Ile Thr Pro Cys Ala Ala Glu Glu Ser Lys Leu Pro Ile Asn Ala Leu
                835                 840                 845
AGC AAC CCT TTG CTG CGC CAC CAC AAC ATG GTC TAT GCC ACA ACA TCC 2595
Ser Asn Pro Leu Leu Arg His His Asn Met Val Tyr Ala Thr Thr Ser
            850                 855                 860
CGC AGC GCA AGC CAG CGG CAG AAA AAG GTC ACA TTT GAC AGA CTG CAA 2643
Arg Ser Ala Ser Gln Arg Gln Lys Lys Val Thr Phe Asp Arg Leu Gln
        865                 870                 875
GTC CTG GAT GAC CAC TAC CGG GAC GTG CTG AAG GAC ATG AAG GCC AAG 2691
Val Leu Asp Asp His Tyr Arg Asp Val Leu Lys Asp Met Lys Ala Lys
    880                 885                 890
GCG TCC ACA GTT AAG GCT AAA CTT CTA TCT GTA GAG GAA GCC TGC AAG 2739
Ala Ser Thr Val Lys Ala Lys Leu Leu Ser Val Glu Glu Ala Cys Lys
895                 900                 905                 910
CTG ACG CCC CCA CAC TCG GCC AGA TCT AAA TTT GAC TAC GGG GCA AAG 2787
Leu Thr Pro Pro His Ser Ala Arg Ser Lys Phe Asp Tyr Gly Ala Lys
                915                 920                 925
GAC GTC CAG AGC CTG TCC AGC AAG GCC GTT AAC CAC ATC CAC TCC GTG 2835
Asp Val Gln Ser Leu Ser Ser Lys Ala Val Asn His Ile His Ser Val
            930                 935                 940
TGG AAG GAC TTG CCG GAA GAC ACT GAG ACA CCA ATC GAC ACC ACC ATC 2883
Trp Lys Asp Leu Pro Glu Asp Thr Glu Thr Pro Ile Asp Thr Thr Ile
        945                 950                 955
ATG GCA AAA AAT GAG GTT TTT TGT GTT CAA CCA GAG AAA GGA GGC CGC 2931
Met Ala Lys Asn Glu Val Phe Cys Val Gln Pro Glu Lys Gly Gly Arg
    960                 965                 970
AAG CCA GCT CGC CTT ATC GTA TTC CCA GAC TTG GGG GTT CGT GTG TGC 2979
Lys Pro Ala Arg Leu Ile Val Phe Pro Asp Leu Gly Val Arg Val Cys
975                 980                 985                 990
GAG AAA ATG GCC CTC TAC GAC GTG GTC TCC ACT CTT CCT CAG GCC GTG 3027
Glu Lys Met Ala Leu Tyr Asp Val Val Ser Thr Leu Pro Gln Ala Val
                995                1000                1005
```

```
ATG GGC TCC TCA TAC AGA TTT CAG TGC TCC CCT GGA CAG CGG GTC GAG 3075
Met Gly Ser Ser Tyr Arg Phe Gln Cys Ser Pro Gly Gln Arg Val Glu
               1010                1015                1020
TTC CTG GTG AAT GCC TGG AAG TCA AAG AAG AGC CCT ATG GGC TTT GCA 3123
Phe Leu Val Asn Ala Trp Lys Ser Lys Lys Ser Pro Met Gly Phe Ala
          1025                1030                1035
TAT GAC ACC CGC TGT TTT GAC TCA ACG GTC ACC GAG AAC GAC ATC CGT 3171
Tyr Asp Thr Arg Cys Phe Asp Ser Thr Val Thr Glu Asn Asp Ile Arg
     1040                1045                1050
ACT GAG GAG TCA ATT TAT CAA TGT TGT GAC TTG GAC CCC GAG GCC AGA 3219
Thr Glu Glu Ser Ile Tyr Gln Cys Cys Asp Leu Asp Pro Glu Ala Arg
1055                1060                1065                1070
CAG GCC ATA AGG TCG CTC ACA GAG CGG CTT TAT ATC GGG GGC CCC CTG 3267
Gln Ala Ile Arg Ser Leu Thr Glu Arg Leu Tyr Ile Gly Gly Pro Leu
                    1075                1080                1085
ACT AAT TCA AAG GGG CAG AAC TGC GGT TAT CGC CGG TGC CGC GTC AGC 3315
Thr Asn Ser Lys Gly Gln Asn Cys Gly Tyr Arg Arg Cys Arg Val Ser
               1090                1095                1100
GGC GTG CTG ACG ACT AGC TGC GGT AAT ACC CTC ACA TGT TAC TTG AAG 3363
Gly Val Leu Thr Thr Ser Cys Gly Asn Thr Leu Thr Cys Tyr Leu Lys
          1105                1110                1115
GCC TCT GCA GCC TGT CGA GCT GCA AAG CTC CAG GAC TGC ACG ATG CTT 3411
Ala Ser Ala Ala Cys Arg Ala Ala Lys Leu Gln Asp Cys Thr Met Leu
     1120                1125                1130
GTG TGC GGA GAC GAC CTT GTC GTT ATC TGT GAT AGC GCG GGA ACT CAG 3459
Val Cys Gly Asp Asp Leu Val Val Ile Cys Asp Ser Ala Gly Thr Gln
1135                1140                1145                1150
GAG GAC GCG GCG AGC CTA CGA GTC TTC ACG GAG GCT ATG ACT AGG TAC 3507
Glu Asp Ala Ala Ser Leu Arg Val Phe Thr Glu Ala Met Thr Arg Tyr
                    1155                1160                1165
TCT GCC CCC CCC GGG GAC CCG CCC CAA CCA GAA TAC GAC TTG GAG CTG 3555
Ser Ala Pro Pro Gly Asp Pro Pro Gln Pro Glu Tyr Asp Leu Glu Leu
               1170                1175                1180
ATA ACA TCA TGT TCC TCC AAT GTG TCG GTC GCG CAC GAC GCA TCA GGC 3603
Ile Thr Ser Cys Ser Ser Asn Val Ser Val Ala His Asp Ala Ser Gly
          1185                1190                1195
AAA CGG GTG TAC TAT CTC ACC CGT GAC CCC ACC ACC CCC CTA GCG CGG 3651
```

```
Lys Arg Val Tyr Tyr Leu Thr Arg Asp Pro Thr Thr Pro Leu Ala Arg
    1200                1205                1210
GCT GCG TGG GAG ACA GCT AGA CAC ACT CCA GTC AAC TCC TGG CTA GGC 3699
Ala Ala Trp Glu Thr Ala Arg His Thr Pro Val Asn Ser Trp Leu Gly
1215                1220                1225                1230
AAC ATC ATC ATG TAC GCG CCC ACC TTA TGG GCA AGG ATG ATT CTG ATG 3747
Asn Ile Ile Met Tyr Ala Pro Thr Leu Trp Ala Arg Met Ile Leu Met
                1235                1240                1245
ACC CAC TTC TTC TCC ATC CTT CTA GCC CAG GAG CAA CTT GAA AAA GCC 3795
Thr His Phe Phe Ser Ile Leu Leu Ala Gln Glu Gln Leu Glu Lys Ala
            1250                1255                1260
CTA GAT TGT CAG ATC TAC GGG GCC ACT TAC TCC ATT GAG CCA CTT GAC 3843
Leu Asp Cys Gln Ile Tyr Gly Ala Thr Tyr Ser Ile Glu Pro Leu Asp
        1265                1270                1275
CTA CCT CAG ATC ATT CAA CGA CTC CAC GGT CTT AGC GCA TTT TCA CTC 3891
Leu Pro Gln Ile Ile Gln Arg Leu His Gly Leu Ser Ala Phe Ser Leu
    1280                1285                1290
CAT AGT TAC TCT CCA GGT GAG ATC AAT AGG GTG GCT TCA TGC CTC AGG 3939
His Ser Tyr Ser Pro Gly Glu Ile Asn Arg Val Ala Ser Cys Leu Arg
1295                1300                1305                1310
AAA CTT GGG GTA CCG CCC TTG CGA GTC TGG AGA CAT CGG GCC AGA AGC 3987
Lys Leu Gly Val Pro Pro Leu Arg Val Trp Arg His Arg Ala Arg Ser
                1315                1320                1325
GTC CGC GCT AAG CTA CTG TCC CAG GGG GGG AGG GCC GCC ACC TGT GGC 4035
Val Arg Ala Lys Leu Leu Ser Gln Gly Gly Arg Ala Ala Thr Cys Gly
            1330                1335                1340
AAA TAC CTC TTC AAC TGG GCA GTA AAG ACC AAG CTC AAA CTC ACT CCA 4083
Lys Tyr Leu Phe Asn Trp Ala Val Lys Thr Lys Leu Lys Leu Thr Pro
        1345                1350                1355
ATC CCA GAA GCG TCC CAG CTG GAC TTG TCC GGC TGG TTC GTT GCT GGT 4131
Ile Pro Glu Ala Ser Gln Leu Asp Leu Ser Gly Trp Phe Val Ala Gly
    1360                1365                1370
TAC AGC GGG GGA GAC ATA TAT CAC AGC CTG TCT CGT GCC CGA CCC CGC 4179
Tyr Ser Gly Gly Asp Ile Tyr His Ser Leu Ser Arg Ala Arg Pro Arg
1375                1380                1385                1390
TGG TTC ATG TGG TGC CTA CTC CTA CTT TCC GTA GGG GTA GGC ATC TAC 4227
Trp Phe Met Trp Cys Leu Leu Leu Leu Ser Val Gly Val Gly Ile Tyr
```

```
                 1395                1400                1405
CTG CTC CCC AAC CGA TGA GCGGG GAGCTAAACA CTCCAGGCCA ATAGGCCATC  4280
Leu Leu Pro Asn Arg Stop
            1410
CCCCTTTTTT TTTTTT                                                4296
```

SEQ ID NO:76
SEQUENCE LENGTH: 818 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N22-1

```
 GG CAT GTG GGC CCA GGG GAG GGG GCT GTG CAG TGG ATG AAC CGG CTG    47
    His Val Gly Pro Gly Glu Gly Ala Val Gln Trp Met Asn Arg Leu
      1               5                  10                  15
ATA GCG TTT GCT TCG CGG GGC AAC CAT GTC TCC CCC ACG CAC TAT GTG    95
Ile Ala Phe Ala Ser Arg Gly Asn His Val Ser Pro Thr His Tyr Val
                 20                  25                  30
CCT GAA AGC GAC GCC GCA GCG CGC GTC ACC CAG ATC CTC TCC AAC CTT   143
Pro Glu Ser Asp Ala Ala Ala Arg Val Thr Gln Ile Leu Ser Asn Leu
             35                  40                  45
ACC ATC ACT CAG CTG TTG AAG AGG CTT CAC CAG TGG ATT AAT GAG GAC   191
Thr Ile Thr Gln Leu Leu Lys Arg Leu His Gln Trp Ile Asn Glu Asp
         50                  55                  60
TGC TCC ACG CCA TGC TCC GGC TCG TGG CTC AGG GAT GTT TGG GAC TGG   239
Cys Ser Thr Pro Cys Ser Gly Ser Trp Leu Arg Asp Val Trp Asp Trp
     65                  70                  75
ATA TGC ACG GTA TTG GCT GAT TGC AAG ACC TGG CTC CAG TCC AAG CTC   287
Ile Cys Thr Val Leu Ala Asp Cys Lys Thr Trp Leu Gln Ser Lys Leu
 80                  85                  90                  95
CTG CCG CGG TTA CCG GGG GTC CCT TTT TTC TCA TGC CAG CGT GGG TAC   335
Leu Pro Arg Leu Pro Gly Val Pro Phe Phe Ser Cys Gln Arg Gly Tyr
```

```
                        100                      105                      110
 AAG GGG GTT TGG CGG GGA GAT GGC ATC ATG TAT ACC ACC TGC CCA TGT    383
 Lys Gly Val Trp Arg Gly Asp Gly Ile Met Tyr Thr Thr Cys Pro Cys
                   115                      120                      125
 GGA GCA CAA ATC ACC GGA CAT GTC AAA AAC GGT TCT ATG AGG ATC GTT    431
 Gly Ala Gln Ile Thr Gly His Val Lys Asn Gly Ser Met Arg Ile Val
           130                      135                      140
 GGG CCT AGA ACC TGT AGC AAC ACG TGG CAC GGA ACA TTT CCC ATC AAC    479
 Gly Pro Arg Thr Cys Ser Asn Thr Trp His Gly Thr Phe Pro Ile Asn
     145                      150                      155
 GCG TAC ACC ACA GGC CCC TGC ACA CCC TCC CCG GCG CCA AAC TAT TCC    527
 Ala Tyr Thr Thr Gly Pro Cys Thr Pro Ser Pro Ala Pro Asn Tyr Ser
 160                      165                      170                      175
 AGG GCG TTG TGG CGG GTG GCC ATT GAG GAG TAT GTG GAG GTC ACG CGG    575
 Arg Ala Leu Trp Arg Val Ala Ile Glu Glu Tyr Val Glu Val Thr Arg
                        180                      185                      190
 GTG GGG GAT TTC CAC TAC GTG ACG GGC ATG ACC ACT GAC AAC GTG AAA    623
 Val Gly Asp Phe His Tyr Val Thr Gly Met Thr Thr Asp Asn Val Lys
                   195                      200                      205
 TGC CCA TGC CAG GTT CCG GCC CCC GAA TTC TTC ACA GAA TTG GAT GGG    671
 Cys Pro Cys Gln Val Pro Ala Pro Glu Phe Phe Thr Glu Leu Asp Gly
           210                      215                      220
 GTG CGG CTG CAC AGG TAC GCT CCG GCG TGC AAA CCT CTC CTG CGG GAT    719
 Val Arg Leu His Arg Tyr Ala Pro Ala Cys Lys Pro Leu Leu Arg Asp
     225                      230                      235
 GAG GTC ACA TTC CAG GTC GGG CTC AAC CAA TAT ACG GTT GGG TCA CAG    767
 Glu Val Thr Phe Gln Val Gly Leu Asn Gln Tyr Thr Val Gly Ser Gln
 240                      245                      250                      255
 CTC CCA TGT GAG CCC GAA CCG GAT GTA ACA GTG GTC ACC TCC ATG CTC    815
 Leu Pro Cys Glu Pro Glu Pro Asp Val Thr Val Val Thr Ser Met Leu
                        260                      265                      270
 ACC                                                                818
 Thr
```

SEQ ID NO:77

SEQUENCE LENGTH: 818 base pairs

SEQUENCE TYPE: nucleic acid

STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N22-3

```
 GG CAT GTG GGC CCA GGG GAG GGG GCT GTG CAG TGG ATG AAC CGG CTG    47
    His Val Gly Pro Gly Glu Gly Ala Val Gln Trp Met Asn Arg Leu
     1               5                  10                  15
ATA GCG TTT GCT TCG CGG GGC AAC CAT GTC TCC CCC ACG CAC TAT GTG    95
Ile Ala Phe Ala Ser Arg Gly Asn His Val Ser Pro Thr His Tyr Val
                20                  25                  30
CCT GAA AGC GAC GCC GCA GCG CGC GTC ACC CAG ATC CTC TCC AAC CTT   143
Pro Glu Ser Asp Ala Ala Ala Arg Val Thr Gln Ile Leu Ser Asn Leu
            35                  40                  45
ACC ATC ACT CAG TTG TTG AAG AGG CTC CAC CAG TGG ATT AAT GAG GAC   191
Thr Ile Thr Gln Leu Leu Lys Arg Leu His Gln Trp Ile Asn Glu Asp
        50                  55                  60
TGC TCC ACG CCA TGC TCC GGC TCG TGG CTC AGG GAT GTT TGG GAC TGG   239
Cys Ser Thr Pro Cys Ser Gly Ser Trp Leu Arg Asp Val Trp Asp Trp
    65                  70                  75
ATA TGC ACG GTA TTG GCT GAT TTC AAG ACC TGG CTC CAG TCC AAG CTC   287
Ile Cys Thr Val Leu Ala Asp Phe Lys Thr Trp Leu Gln Ser Lys Leu
 80                  85                  90                  95
CTG CCG CGG TTA CCG GGG GTC CCT TTC TTC TCA TGC CAG CGT GGG TAC   335
Leu Pro Arg Leu Pro Gly Val Pro Phe Phe Ser Cys Gln Arg Gly Tyr
                100                 105                 110
AAG GGG GTT TGG CGG GGA GAC GGC ATC ATG TAT ACC ACC TGC CCA TGT   383
Lys Gly Val Trp Arg Gly Asp Gly Ile Met Tyr Thr Thr Cys Pro Cys
            115                 120                 125
GGA GCA CAA ATC ACC GGA CAT GTC AAA AAC GGT TCT ATG AGG ATC GTT   431
Gly Ala Gln Ile Thr Gly His Val Lys Asn Gly Ser Met Arg Ile Val
        130                 135                 140
GGG CTT AGA ACC TGT AGC AAC ACG TGG CAC GGA ACA TTC CCC ATC AAC   479
Gly Leu Arg Thr Cys Ser Asn Thr Trp His Gly Thr Phe Pro Ile Asn
```

```
    145                 150                 155
GCG TAC ACC ACA GGC CCC TGC ACA CCC TCT CCA GCG CCG AAC TAC TCC   527
Ala Tyr Thr Thr Gly Pro Cys Thr Pro Ser Pro Ala Pro Asn Tyr Ser
160                 165                 170                 175
AGG GCG TTA TGG CGG GTA GCC GCT GAG GAG TAT GTG GAG GTC ACG CGG   575
Arg Ala Leu Trp Arg Val Ala Ala Glu Glu Tyr Val Glu Val Thr Arg
                180                 185                 190
GTG GGG GAT TTC CAC TAC GTG ACG GGC ATG ACC ACT GAC AAC GTA AAA   623
Val Gly Asp Phe His Tyr Val Thr Gly Met Thr Thr Asp Asn Val Lys
            195                 200                 205
TGC CCA TGC CAG GTT CCG GCC CCC GAA TTC TTC ACA GAA TTG GAT GGG   671
Cys Pro Cys Gln Val Pro Ala Pro Glu Phe Phe Thr Glu Leu Asp Gly
        210                 215                 220
GTG CGG CTG CGC AGG TAC GCT CCG GCG TGC AAA CCT CTC CTG CGG GAT   719
Val Arg Leu Arg Arg Tyr Ala Pro Ala Cys Lys Pro Leu Leu Arg Asp
    225                 230                 235
GAG GTC ACA TTC CAG GTC GGG CTC AAC CAA TAT ACG GTT GGG TCA CAG   767
Glu Val Thr Phe Gln Val Gly Leu Asn Gln Tyr Thr Val Gly Ser Gln
240                 245                 250                 255
CTC CCA TGT GAG CCC GAA CCG GAT GTA ACG GTG GTC ACC TCC ATG CTC   815
Leu Pro Cys Glu Pro Glu Pro Asp Val Thr Val Val Thr Ser Met Leu
                260                 265                 270
ACC                                                               818
Thr
```

SEQ ID NO:78

SEQUENCE LENGTH: 818 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: H22-3

```
 GG CAT GTG GGC CCA GGG GAG GGG GCT GTG CAG TGG ATG AAC CGG CTG   47
```

```
    His Val Gly Pro Gly Glu Gly Ala Val Gln Trp Met Asn Arg Leu
     1               5                  10                  15
ATA GCG TTC GCT TCG CGG GGT AAC CAC GTC TCC CCC ACG CAT TAT GTG    95
Ile Ala Phe Ala Ser Arg Gly Asn His Val Ser Pro Thr His Tyr Val
                 20                  25                  30
CCT GAG AGC GAC GCC GCA GCG CGT GTC ACC CAG ATC CTC TCC AGC CTT   143
Pro Glu Ser Asp Ala Ala Ala Arg Val Thr Gln Ile Leu Ser Ser Leu
             35                  40                  45
ACC ATC ACT CAG CTG CTG AAG AGG CTC CAC CAG TGG ATT GAT GAG GAC   191
Thr Ile Thr Gln Leu Leu Lys Arg Leu His Gln Trp Ile Asp Glu Asp
         50                  55                  60
TGC TCC ACG CCA TGT TCT GGT TCG TGG CTC AGG GAT GTT TGG GAC TGG   239
Cys Ser Thr Pro Cys Ser Gly Ser Trp Leu Arg Asp Val Trp Asp Trp
     65                  70                  75
ATA TGC ACG GTG TTG AGT GAC TTC AAG ACC TGG CTC CAG TCC AAG CTC   287
Ile Cys Thr Val Leu Ser Asp Phe Lys Thr Trp Leu Gln Ser Lys Leu
 80                  85                  90                  95
CTG CCG CGG CTA CCG GGA GTC CCT TTC CTC TCA TGC CAA CGT GGG TAC   335
Leu Pro Arg Leu Pro Gly Val Pro Phe Leu Ser Cys Gln Arg Gly Tyr
                100                 105                 110
AAG GGA GTC TGG CGG GGA GAT GGC ATC ATG CAG ACC ACC TGC CCA TGC   383
Lys Gly Val Trp Arg Gly Asp Gly Ile Met Gln Thr Thr Cys Pro Cys
            115                 120                 125
GGA GCA CAA ATC GCC GGA CAT GTC AAA AAT GGT TCT ATG AGG ATC ACT   431
Gly Ala Gln Ile Ala Gly His Val Lys Asn Gly Ser Met Arg Ile Thr
        130                 135                 140
GGC CCC AGA ACC TGT AGC AAC ACG TGG CAC GGA ACG TTC CCC ATC AAC   479
Gly Pro Arg Thr Cys Ser Asn Thr Trp His Gly Thr Phe Pro Ile Asn
    145                 150                 155
GCG TAC ACC ACA GGC CCC TGC ACA CCC TCC CCA GCG CCG AAC TAC TCC   527
Ala Tyr Thr Thr Gly Pro Cys Thr Pro Ser Pro Ala Pro Asn Tyr Ser
160                 165                 170                 175
AGG GCG TTA TGG CGG GTA GCT GCT GAG GAG TAT GTG GAG GTC ACG CGG   575
Arg Ala Leu Trp Arg Val Ala Ala Glu Glu Tyr Val Glu Val Thr Arg
                180                 185                 190
GTG GGG GAC TTC CAC TAC GTG ACG GGC ATG ACC ACT GAC AAC TTG AAA   623
Val Gly Asp Phe His Tyr Val Thr Gly Met Thr Thr Asp Asn Leu Lys
```

```
              195                     200                     205
    TGC CCA TGC CAG GTC CCG GCC CCC GAA TTC TTC ACG GAG TTG GAT GGG    671
    Cys Pro Cys Gln Val Pro Ala Pro Glu Phe Phe Thr Glu Leu Asp Gly
            210                     215                     220
    GTA CGG CTA CAC AGG TAC GCT CCG GCG TGC AAA CCT CTC CTA CGG GAT    719
    Val Arg Leu His Arg Tyr Ala Pro Ala Cys Lys Pro Leu Leu Arg Asp
        225                     230                     235
    GAG GTC ACA TTC CAG GTC GGG CTC AAC CAA TTC CCG GTT GGG TCG CAG    767
    Glu Val Thr Phe Gln Val Gly Leu Asn Gln Phe Pro Val Gly Ser Gln
    240                     245                     250                     255
    CTC CCA TGC GAG CCC GAA CCG GAT GTA ATA GTG GTC ACC TCC ATG CTC    815
    Leu Pro Cys Glu Pro Glu Pro Asp Val Met Val Val Thr Ser Met Leu
                    260                     265                     270
    ACC                                                                818
    Thr
```

SEQ ID NO:79
SEQUENCE LENGTH: 818 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H22-8

```
     GG CAT GTG GGC CCA GGG GAG GGG GCT GTG CAG TGG ATG AAC CGG CTG    47
        His Val Gly Pro Gly Glu Gly Ala Val Gln Trp Met Asn Arg Leu
          1               5                  10                  15
    ATA GCG TTC GCC TCG CGG GGT AAC CAC GTC TCC CCC ACG CAT TAT GTG    95
    Ile Ala Phe Ala Ser Arg Gly Asn His Val Ser Pro Thr His Tyr Val
                    20                  25                  30
    CCT GAG AGC GAC GCC GCG GCG CGT GTC ACC CAG ATC CTC TCC AGC CTC    143
    Pro Glu Ser Asp Ala Ala Ala Arg Val Thr Gln Ile Leu Ser Ser Leu
                35                  40                  45
    ACC ATC ACT CAG CTG CTG AAG AGG CTC CAC CAG TGG ATT AAT GAG GAC    191
```

```
Thr Ile Thr Gln Leu Leu Lys Arg Leu His Gln Trp Ile Asn Glu Asp
        50                  55                  60
TGC TCC ACG CCA TGT TCT GGT TCG TGG CTC AGG GAT GTT TGG GAC TGG    239
Cys Ser Thr Pro Cys Ser Gly Ser Trp Leu Arg Asp Val Trp Asp Trp
    65                  70                  75
ATA TGC ACG GTG TTG AGT GAC TTC AAG ACC TGG CTC CAG TCC AAG CTC    287
Ile Cys Thr Val Leu Ser Asp Phe Lys Thr Trp Leu Gln Ser Lys Leu
80                  85                  90                  95
CTG CCG CGG CTA CCG GGA GTC CCT TTC CTT TCA TGC CAA CGT GGG TAC    335
Leu Pro Arg Leu Pro Gly Val Pro Phe Leu Ser Cys Gln Arg Gly Tyr
                100                 105                 110
AAG GGA GTC TGG CGG GGA GAT GGC ATC ATG CAA ACC ACC TGC CCA TGC    383
Lys Gly Val Trp Arg Gly Asp Gly Ile Met Gln Thr Thr Cys Pro Cys
            115                 120                 125
GGA GCA CAA ATC GCC GGA CAT GTC AAA AAT GGT TCC ATG AGG ATC ACT    431
Gly Ala Gln Ile Ala Gly His Val Lys Asn Gly Ser Met Arg Ile Thr
        130                 135                 140
GGC CCC AGA ACC TGT AGC AAC ACG TGG CAC GGA ACG TTC CCC ATC AAC    479
Gly Pro Arg Thr Cys Ser Asn Thr Trp His Gly Thr Phe Pro Ile Asn
    145                 150                 155
GCG TAC ACC ACA GGC CCC TGC ACA CCC TCC CCA GCG CCG AAC TAT TCT    527
Ala Tyr Thr Thr Gly Pro Cys Thr Pro Ser Pro Ala Pro Asn Tyr Ser
160                 165                 170                 175
AGG GCG TTG TGG CGG GTA GCT GCT GAG GAG TAT GTG GAG GTC ACG CGG    575
Arg Ala Leu Trp Arg Val Ala Ala Glu Glu Tyr Val Glu Val Thr Arg
                180                 185                 190
GTG GGG GAT TTC CAC TAC GTG ACG GGC ATG ACC ACT GAC AAC TTG AAA    623
Val Gly Asp Phe His Tyr Val Thr Gly Met Thr Thr Asp Asn Leu Lys
            195                 200                 205
TGC CCA TGC CAG GTC CCG GCC CCC GAA TTC TTC ACG GAG TTG GAT GGG    671
Cys Pro Cys Gln Val Pro Ala Pro Glu Phe Phe Thr Glu Leu Asp Gly
        210                 215                 220
GTA CGG CTA CAC AGA TAC GCT CCG GCG TGC AAA CCT CTC CTA CGG GAT    719
Val Arg Leu His Arg Tyr Ala Pro Ala Cys Lys Pro Leu Leu Arg Asp
    225                 230                 235
GAG GTC ACA TTC CAG GTC GGG CTC AAC CAA TTC CCG GTT GGG TCG CAG    767
Glu Val Thr Phe Gln Val Gly Leu Asn Gln Phe Pro Val Gly Ser Gln
```

```
240                  245                  250                  255
CTC CCA TGC GAG CCC GAA CCG GAT GTA ACA GTG GTC ACC TCC ATG CTC   815
Leu Pro Cys Glu Pro Glu Pro Asp Val Thr Val Val Thr Ser Met Leu
                260                  265                  270
ACC                                                               818
Thr
```

SEQ ID NO:80
SEQUENCE LENGTH: 818 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H22-9

```
 GG CAT GTG GGC CCA GGG GAG GGG GCT GTG CAG TGG ATG AAC CGG CTG    47
    His Val Gly Pro Gly Glu Gly Ala Val Gln Trp Met Asn Arg Leu
      1               5                  10                  15
ATA GCG TTC GCT TCG CGG GGT AAC CAC GTC TCC CCC ACG CAT TAT GTG   95
Ile Ala Phe Ala Ser Arg Gly Asn His Val Ser Pro Thr His Tyr Val
                20                  25                  30
CCT GAG AGC GAC GCC GCA GCG CGT GTC ACC CAG ATC CTC TCC AGC CTT  143
Pro Glu Ser Asp Ala Ala Ala Arg Val Thr Gln Ile Leu Ser Ser Leu
            35                  40                  45
ACC ATC ACT CAG CTG TTG AAG AGG CTC CAC CAG TGG ATT AAT GAT GAC  191
Thr Ile Thr Gln Leu Leu Lys Arg Leu His Gln Trp Ile Asn Asp Asp
        50                  55                  60
TGC TCC ACG CCA TGT TCT GGT TCG TGG CTC AGG GAT GTT TGG GAC TGG  239
Cys Ser Thr Pro Cys Ser Gly Ser Trp Leu Arg Asp Val Trp Asp Trp
    65                  70                  75
ATA TGC ACG GTG TTG AGT GAC TTC AAG ACC TGG CTC CAG TCC AAG CTC  287
Ile Cys Thr Val Leu Ser Asp Phe Lys Thr Trp Leu Gln Ser Lys Leu
 80                  85                  90                  95
CTG CCG CGG CTA CCG GGA GTC CCT TTC CTC TCA TGC CAA CGT GGG TAC  335
```

```
Leu Pro Arg Leu Pro Gly Val Pro Phe Leu Ser Cys Gln Arg Gly Tyr
                100                 105                 110
AAG GGA GTC TGG CGG GGA GAT GGC ATC ATG CAT ACC ACC TGC CCA TGC    383
Lys Gly Val Trp Arg Gly Asp Gly Ile Met His Thr Thr Cys Pro Cys
            115                 120                 125
GGA GCA CAA ATC GCC GGA CAT GTC AAA AAT GGT TCC ATG AGG ATC ACT    431
Gly Ala Gln Ile Ala Gly His Val Lys Asn Gly Ser Met Arg Ile Thr
        130                 135                 140
GGC CCC AGA ACC TGT AGC AAC ACG TGG CGC GGA ACG TTC CCC ATC AAC    479
Gly Pro Arg Thr Cys Ser Asn Thr Trp Arg Gly Thr Phe Pro Ile Asn
    145                 150                 155
GCG TAC ACC ACA GGC CCC TGC ACA CCC TCC CCA GCG CCG AAC TAT TCT    527
Ala Tyr Thr Thr Gly Pro Cys Thr Pro Ser Pro Ala Pro Asn Tyr Ser
160                 165                 170                 175
AAG GCG TTG TGG CGG GTA GCT GCT GAG GAG TAT GTG GAG GTC ACG CGG    575
Lys Ala Leu Trp Arg Val Ala Ala Glu Glu Tyr Val Glu Val Thr Arg
                180                 185                 190
GTG GGG GAT TTC CAC TAC GTG ACG GGC ATG ACC ACT GAC AAC TTG AAA    623
Val Gly Asp Phe His Tyr Val Thr Gly Met Thr Thr Asp Asn Leu Lys
            195                 200                 205
TGC CCA TGC CAG GTC CCG GCC CCC GAA TTT TTC ACG GAG TTG GAT GGG    671
Cys Pro Cys Gln Val Pro Ala Pro Glu Phe Phe Thr Glu Leu Asp Gly
        210                 215                 220
GTA CGG CTA CAC AGG TAC GCT CCG GCG TGC AAA CCT CTC CTA CGG GAT    719
Val Arg Leu His Arg Tyr Ala Pro Ala Cys Lys Pro Leu Leu Arg Asp
    225                 230                 235
GAG GTC ACA TTC CAG GTC GGG CTC AAC CAA TTC CCG GTT GGG TCG CAG    767
Glu Val Thr Phe Gln Val Gly Leu Asn Gln Phe Pro Val Gly Ser Gln
240                 245                 250                 255
CTA CCA TGC GAG CCC GAA CCG GAT GTA GCA GTG GTC ACC TCC ATG CTC    815
Leu Pro Cys Glu Pro Glu Pro Asp Val Ala Val Val Thr Ser Met Leu
                260                 265                 270
ACC                                                                818
Thr
```

SEQ ID NO:81

SEQUENCE LENGTH: 311 base pairs

SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N17-3

```
TGT GAG CCC GAA CCG GAT GTA ACA GTG GTC ACC TCC ATG CTC ACC GAC   48
Cys Glu Pro Glu Pro Asp Val Thr Val Val Thr Ser Met Leu Thr Asp
 1               5                  10                  15
CCC TCC CAC ATT ACA GCA GAG GCG GCT AGG CGT AGG CTG ACC AGA GGG   96
Pro Ser His Ile Thr Ala Glu Ala Ala Arg Arg Arg Leu Thr Arg Gly
            20                  25                  30
TCT CCC CCT TCC TCG ACC AGT TCT TCA GCT AGT CAG TTG TCT GCG CTT  144
Ser Pro Pro Ser Ser Thr Ser Ser Ser Ala Ser Gln Leu Ser Ala Leu
        35                  40                  45
TCT TCG CAG GCA ACA TGC ACT ACC CAT CAG GGC GCC CCA GAC ACT GAC  192
Ser Ser Gln Ala Thr Cys Thr Thr His Gln Gly Ala Pro Asp Thr Asp
    50                  55                  60
CTC ATC GAG GCC AAC CTC CTG TGG CGG CAG GAG ATG GGC GGA AAC ATC  240
Leu Ile Glu Ala Asn Leu Leu Trp Arg Gln Glu Met Gly Gly Asn Ile
65                  70                  75                  80
ACC CGC GTG GAG TCA GAG AAC AAG ATA GTA ATT CTA GAC TCT TTT GAA  288
Thr Arg Val Glu Ser Glu Asn Lys Ile Val Ile Leu Asp Ser Phe Glu
                85                  90                  95
CCG CTT CGA GCG GAG GAG GAT G A                                  311
Pro Leu Arg Ala Glu Glu Asp
            100
```

SEQ ID NO:82
SEQUENCE LENGTH: 311 base pairs
SEQUENCE TYPE: nucleic acid
STRANDEDNESS: double
TOPOLOGY: linear
ANTI-SENSE: No

ORIGINAL SOURCE
ORGANISM: Hepatitis C virus
IMMEDIATE EXPERIMENTAL SOURCE
CLONE: N17-1

```
TGT GAG CCC GAA CCG GAT GTA ACA GTG GTC ACC TCC ATG CTC ACC GAC   48
Cys Glu Pro Glu Pro Asp Val Thr Val Val Thr Ser Met Leu Thr Asp
 1               5                  10                  15
CCC TCC CAC ATC ACA GCA GAG GCG GCT AGG CGT AGG CTG GCC AGA GGG   96
Pro Ser His Ile Thr Ala Glu Ala Ala Arg Arg Arg Leu Ala Arg Gly
            20                  25                  30
TCT CCT CCT TCT TCG GCC AGC TCT TCA GCT AGC CAG TTG TCT GCG CCA  144
Ser Pro Pro Ser Ser Ala Ser Ser Ser Ala Ser Gln Leu Ser Ala Pro
        35                  40                  45
TCT TTG AAG GCG ACA TGT ACT ACC CAT CAA GAC TCC CCA GAC GCT GAC  192
Ser Leu Lys Ala Thr Cys Thr Thr His Gln Asp Ser Pro Asp Ala Asp
    50                  55                  60
CTC ATC GAG GCC AAC CTC CTG TGG CGG CAG GAG ATG GGC GGG AAC ATC  240
Leu Ile Glu Ala Asn Leu Leu Trp Arg Gln Glu Met Gly Gly Asn Ile
 65                  70                  75                  80
ACC CGC GTG GAG TCA GAG AAC AAG ATA GTG ATT CTA GAC TCT TCT GAA  288
Thr Arg Val Glu Ser Glu Asn Lys Ile Val Ile Leu Asp Ser Ser Glu
                85                  90                  95
CCG CTT CGA GCG GAG GAG GAT G A                                  311
Pro Leu Arg Ala Glu Glu Asp
           100
```

SEQ ID NO:83
SEQUENCE LENGTH: 311 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N17-2

```
TGT GAG CCC GAA CCG GAT GTA ACA GTG GTC ACC TCC ATG CTC ACC GAC    48
Cys Glu Pro Glu Pro Asp Val Thr Val Val Thr Ser Met Leu Thr Asp
  1               5                  10                  15
CCC TCC CAC ATC ACA GCA GAG GCG GCT AGG CGT AGG CTG GCC AGA GGG    96
Pro Ser His Ile Thr Ala Glu Ala Ala Arg Arg Arg Leu Thr Arg Gly
             20                  25                  30
TCT CCT CCT TCT TTG GCC AGC TCT TCA GCT AGT CAG TTG TCT GCG CCA   144
Ser Pro Pro Ser Leu Ala Ser Ser Ser Ala Ser Gln Leu Ser Ala Pro
         35                  40                  45
TCT TTG AAG GCG ACA TGC ACT ACC CAT CAT GAC TCC CCA GAC GCT GAC   192
Ser Leu Lys Ala Thr Cys Thr Thr His His Asp Ser Pro Asp Ala Asp
     50                  55                  60
CTC ATC GAG GCC AAC CTC CTG TGG CGG CAG GAG ATG GGC GGG AAC ATC   240
Leu Ile Glu Ala Asn Leu Leu Trp Arg Gln Glu Met Gly Gly Asn Ile
 65                  70                  75                  80
ACC CGC GTG GAG TTA GAG AAC AAG ATA GTA ATT CTA GAC TCT TTT GAA   288
Thr Arg Val Glu Leu Glu Asn Lys Ile Val Ile Leu Asp Ser Phe Glu
                 85                  90                  95
CCG CTT CGA GCG GAG GAG GAT G A                                   311
Pro Leu Arg Ala Glu Glu Asp
            100
```

SEQ ID NO:84
SEQUENCE LENGTH: 311 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H17-1

```
TGT GAG CCC GAA CCG GAT GTA ACA GTG CTC ACT TCC ATG CTC ACC GAC    48
Cys Glu Pro Glu Pro Asp Val Thr Val Leu Thr Ser Met Leu Thr Asp
  1               5                  10                  15
CCC TCC CAC ATT ACA GCA GAG ACG GCT AAG CGT AGG CTG GCC AGA GGG    96
```

```
Pro Ser His Ile Thr Ala Glu Thr Ala Lys Arg Arg Leu Ala Arg Gly
            20                  25                  30
TCT CCC CCT CCC TTG GCC AGC TCT TCA GCT AGT CAG TTG TCT GCG CCC  144
Ser Pro Pro Pro Leu Ala Ser Ser Ser Ala Ser Gln Leu Ser Ala Pro
        35                  40                  45
TCC CTG AAG GCG ACA TGC ACT ACC CAT CAT GAC TCC CCG GAC GCT GAC  192
Ser Leu Lys Ala Thr Cys Thr Thr His His Asp Ser Pro Asp Ala Asp
    50                  55                  60
CTC ATC GAG GCC AAC CTC CTG TGG CGG CAG GAG ATG GGA GGG AAC ATC  240
Leu Ile Glu Ala Asn Leu Leu Trp Arg Gln Glu Met Gly Gly Asn Ile
65                  70                  75                  80
ACC CGT GTG GAG TCA GAG AAC AAG GTA GTA ATT CTG GAC TCT TTC GAC  288
Thr Arg Val Glu Ser Glu Asn Lys Val Val Ile Leu Asp Ser Phe Asp
                85                  90                  95
CCG CTT CGA GCG GAG GAG GAT G A                                  311
Pro Leu Arg Ala Glu Glu Asp
            100
```

SEQ ID NO:85
SEQUENCE LENGTH: 311 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H17-3

```
TGT GAG CCC GAA CCG GAT GTA ACA GTG GTC ACT TCC ATG CTC ACC GAC   48
Cys Glu Pro Glu Pro Asp Val Thr Val Val Thr Ser Met Leu Thr Asp
1               5                   10                  15
CCC TCC CAC ATT ACA GCA GAG GCG GCT GGG CGT AGG CTG GCC AGA GGG   96
Pro Ser His Ile Thr Ala Glu Ala Ala Gly Arg Arg Leu Ala Arg Gly
            20                  25                  30
TCT CCC CCT TCC TTG GCC AGC TCT TCA GCT AGT CAG TTG TCT GCG CCC  144
Ser Pro Pro Ser Leu Ala Ser Ser Ser Ala Ser Gln Leu Ser Ala Pro
```

```
            35                       40                       45
    TCT CTG AAG GCG ACA TGC ACT ACC CAT CAT GAC TCC CCG GAC GCT GAC  192
    Ser Leu Lys Ala Thr Cys Thr Thr His His Asp Ser Pro Asp Ala Asp
        50                       55                       60
    CTC ATC GAG GCC AAC CTC CTA TGG CGG CAG GAG ATG GGA GGG AAC ATC  240
    Leu Ile Glu Ala Asn Leu Leu Trp Arg Gln Glu Met Gly Gly Asn Ile
    65                       70                       75                  80
    ACC CGC GTG GAG TCA GAG AGC AAG GTA GTA ATT CTG GAC TCT TTC GAC  288
    Thr Arg Val Glu Ser Glu Ser Lys Val Val Ile Leu Asp Ser Phe Asp
                        85                       90                       95
    CCG CTT CGA GCG GAG GAG GAT G A                                   311
    Pro Leu Arg Ala Glu Glu Asp
                    100
```

SEQ ID NO:86
SEQUENCE LENGTH: 740 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O28-1

```
    GTG GTA GTC CTG GAC TCG TTG GAG CCG CTT CAA GCG AAG GAA GGT GAG   48
    Val Val Val Leu Asp Ser Leu Glu Pro Leu Gln Ala Lys Glu Gly Glu
     1               5                  10                  15
    AGG GAA GTG TCC GTT GCG GCG GAG ATC CTG CGG AAG ACC AGG AAA TTC   96
    Arg Glu Val Ser Val Ala Ala Glu Ile Leu Arg Lys Thr Arg Lys Phe
                    20                  25                  30
    CCC GCA GCG ATG CCC GTA TGG GCA CGC CCG GAC TAC AAC CCA CCA TTA  144
    Pro Ala Ala Met Pro Val Trp Ala Arg Pro Asp Tyr Asn Pro Pro Leu
                35                  40                  45
    CTA GAG TCT TGG AAG AAC CCG GAC TAC GTC CCT CCA GTG GTA CAC GGG  192
    Leu Glu Ser Trp Lys Asn Pro Asp Tyr Val Pro Pro Val Val His Gly
        50                  55                  60
```

```
TGC CCA TTG CCG CCT ACC AAG GCC CCT CCA ATA CCA CCT CCA CGA AGA   240
Cys Pro Leu Pro Pro Thr Lys Ala Pro Pro Ile Pro Pro Pro Arg Arg
 65                  70                  75                  80
AAG AGA ACG GTT GTC CTG ACA GAA TCC TCC GTG TCC TCT GCC TTG GCG   288
Lys Arg Thr Val Val Leu Thr Glu Ser Ser Val Ser Ser Ala Leu Ala
                 85                  90                  95
GAG CTT GCT ACA AAG ACC TTT GGC AGT TCC GGA TCG TCG GCC GTC GAC   336
Glu Leu Ala Thr Lys Thr Phe Gly Ser Ser Gly Ser Ser Ala Val Asp
            100                 105                 110
AGC GGC ACG GCG ACC GGC CCT CCT GAC CAG GCC TCC GCC GAA GGA GAT   384
Ser Gly Thr Ala Thr Gly Pro Pro Asp Gln Ala Ser Ala Glu Gly Asp
        115                 120                 125
GCA GGA TCC GAC GCT GAG TCG TAC TCC TCC ATG CCC CCC CTT GAG GGA   432
Ala Gly Ser Asp Ala Glu Ser Tyr Ser Ser Met Pro Pro Leu Glu Gly
    130                 135                 140
GAG CCG GGG GAC CCC GAT CTC AGC GAC GGG TCT TGG TCT ACC GTA AGC   480
Glu Pro Gly Asp Pro Asp Leu Ser Asp Gly Ser Trp Ser Thr Val Ser
145                 150                 155                 160
GAG GAG GCC AGC GAG GAC GTC GTC TGC TGC TCG ATG TCC TAC ACA TGG   528
Glu Glu Ala Ser Glu Asp Val Val Cys Cys Ser Met Ser Tyr Thr Trp
                165                 170                 175
ACA GGC GCC TTA ATT ACA CCA TGC GCC GCG GAG GAG AGC AAG CTG CCC   576
Thr Gly Ala Leu Ile Thr Pro Cys Ala Ala Glu Glu Ser Lys Leu Pro
            180                 185                 190
ATT AAT GCG CTG AGC AAC CCT TTG CTG CGC CAC CAC AAC ATG GTC TAT   624
Ile Asn Ala Leu Ser Asn Pro Leu Leu Arg His His Asn Met Val Tyr
        195                 200                 205
GCC ACA ACA TCC CGC AGC GCA AGC CAG CGG CAG AAA AAG GTC ACA TTT   672
Ala Thr Thr Ser Arg Ser Ala Ser Gln Arg Gln Lys Lys Val Thr Phe
    210                 215                 220
GAC AGA CTG CAA GTC CTG GAT GAC CAC TAC CGG GAC GTG CTC AAG GAC   720
Asp Arg Leu Gln Val Leu Asp Asp His Tyr Arg Asp Val Leu Lys Asp
225                 230                 235                 240
ATG AAG GCC AAG GCG TCC AC                                        740
Met Lys Ala Lys Ala Ser
                245
```

SEQ ID NO:87
SEQUENCE LENGTH: 740 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: 028-2

```
GTG GTA GTC CTG GAC TCG TTG GAC CCG CTT CGA GCG GAG GAA GAT GAG    48
Val Val Val Leu Asp Ser Leu Asp Pro Leu Arg Ala Glu Glu Asp Glu
  1               5                  10                  15
AGG GAA GTG TCC GTT GCG GCG GAG ATC CTG CGA AAG ACC AAG AAA TTC    96
Arg Glu Val Ser Val Ala Ala Glu Ile Leu Arg Lys Thr Lys Lys Phe
             20                  25                  30
CCC GCA GCG ATG CCC GTA TGG GCA CGC CCG GAC TAC AAC CCA CCA TTA   144
Pro Ala Ala Met Pro Val Trp Ala Arg Pro Asp Tyr Asn Pro Pro Leu
         35                  40                  45
CTA GAG TCT TGG AAG AAC CCG GAC TAC GTC CCT CCG GTG GTA CAC GGG   192
Leu Glu Ser Trp Lys Asn Pro Asp Tyr Val Pro Pro Val Val His Gly
     50                  55                  60
TGC CCA TTG CCG CCT ACC AAG GCC CCT CCA ATA CCA CCT CCA CGG AGA   240
Cys Pro Leu Pro Pro Thr Lys Ala Pro Pro Ile Pro Pro Pro Arg Arg
 65                  70                  75                  80
AAG AGG ACG GTT GCC CTG ACA GAA TCC ACC GTG TCC TCT GCC TTG GCG   288
Lys Arg Thr Val Ala Leu Thr Glu Ser Thr Val Ser Ser Ala Leu Ala
                 85                  90                  95
GAG CTT GCT ACA AAG ACC TTT GGC AGT TCC GGA TCG TCG GCC GTC GAC   336
Glu Leu Ala Thr Lys Thr Phe Gly Ser Ser Gly Ser Ser Ala Val Asp
            100                 105                 110
AGC GGC ACG GCG ACT GGC CCT CCT GAC CAG GCC TCC GCC GAA GGA GAT   384
Ser Gly Thr Ala Thr Gly Pro Pro Asp Gln Ala Ser Ala Glu Gly Asp
        115                 120                 125
GCA GGA TCC GAC GCT GAG TCG TAC TCC TCC ATG CCC CCC CTT GAG GGA   432
Ala Gly Ser Asp Ala Glu Ser Tyr Ser Ser Met Pro Pro Leu Glu Gly
```

```
    130                 135                 140
GAG CCG GGG GAC CCT GAT CTC AGC GAC GGG TCT TGG TCT ACT GTA AGC  480
Glu Pro Gly Asp Pro Asp Leu Ser Asp Gly Ser Trp Ser Thr Val Ser
145                 150                 155                 160
GAG GAG GCC GGC GAG GAC GTC GTC TGC TGC TCG ATG TCC TAC ACA TGG  528
Glu Glu Ala Gly Glu Asp Val Val Cys Cys Ser Met Ser Tyr Thr Trp
                165                 170                 175
ACA GGC GCC TTA ATT ACA CCA TGC GCC GCG GAG GAG AGC AAG CTG CCC  576
Thr Gly Ala Leu Ile Thr Pro Cys Ala Ala Glu Glu Ser Lys Leu Pro
            180                 185                 190
ATT AAT GCG CTG AGC AAC TCT TTG CTG CGC CAC CAC AAC ATG GTC TAT  624
Ile Asn Ala Leu Ser Asn Ser Leu Leu Arg His His Asn Met Val Tyr
        195                 200                 205
GCC ACA ACA TCC CGC AGC GCA AGC CAG CGG CAG AAA AAG GTC ACA TTT  672
Ala Thr Thr Ser Arg Ser Ala Ser Gln Arg Gln Lys Lys Val Thr Phe
    210                 215                 220
GAC AGA CTG CAA GTC CTG GAT GAC CAC TAC CGG GAC GTG CTC AAG GAC  720
Asp Arg Leu Gln Val Leu Asp Asp His Tyr Arg Asp Val Leu Lys Asp
225                 230                 235                 240
ATG AAG GCC AAG GCG TCC AC                                       740
Met Lys Ala Lys Ala Ser
                245
```

SEQ ID NO:88
SEQUENCE LENGTH: 740 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O28-4

```
GTG GTA GTC CTG GAC TCG TTG GAC CCG CTT CGA GCG GAG GAA GAT GAG   48
Val Val Val Leu Asp Ser Leu Asp Pro Leu Arg Ala Glu Glu Asp Glu
  1               5                  10                  15
```

```
AGG GAA GTG TCC GTT GCG GCG GAG ATC CTG CGA AAG ACC AAG AAA TTC   96
Arg Glu Val Ser Val Ala Ala Glu Ile Leu Arg Lys Thr Lys Lys Phe
            20                  25                  30
CCC GCA GCG ATG CCC GTA TGG GCA CGC CCG GAC TAC AAC CCA CCA TTA  144
Pro Ala Ala Met Pro Val Trp Ala Arg Pro Asp Tyr Asn Pro Pro Leu
        35                  40                  45
CTA GAG TCT TGG AAG AAC CCG GAC TAC GTC CCT CCG GTG GTA CAC GGG  192
Leu Glu Ser Trp Lys Asn Pro Asp Tyr Val Pro Pro Val Val His Gly
    50                  55                  60
TGC CCA TTG CCG CCT ATC AAG GCC CCT CCA ATA CCA CCT CCA CGG AGA  240
Cys Pro Leu Pro Pro Ile Lys Ala Pro Pro Ile Pro Pro Pro Arg Arg
65                  70                  75                  80
AAG AGG ACG GTT GTC CTG ACA GAA TCC ACC GTG TCC TCT GCC TTG GCG  288
Lys Arg Thr Val Val Leu Thr Glu Ser Thr Val Ser Ser Ala Leu Ala
                85                  90                  95
GAG CTT GCT ACA AAG ACC TTT GGC AGT TCC GGA TCG TCG GCC GTC GAC  336
Glu Leu Ala Thr Lys Thr Phe Gly Ser Ser Gly Ser Ser Ala Val Asp
            100                 105                 110
AGC GGC ACG GCG ACC GGC CCT CCT GAC CAG GCC TCC GCC GAA GGA GAT  384
Ser Gly Thr Ala Thr Gly Pro Pro Asp Gln Ala Ser Ala Glu Gly Asp
        115                 120                 125
GCA GGA TCC GAC GCT GAG TCG TAC TCC TCC ATG CCC CCC CTT GAG GGA  432
Ala Gly Ser Asp Ala Glu Ser Tyr Ser Ser Met Pro Pro Leu Glu Gly
    130                 135                 140
GAG CCG GGG GAC CCT GAT CTC AGC GAC GGG TCT TGG TCT ACT GTA AGC  480
Glu Pro Gly Asp Pro Asp Leu Ser Asp Gly Ser Trp Ser Thr Val Ser
145                 150                 155                 160
GAG GAG GCC GGC GAG GAC GTC GTC TGC TGC TCG ATG TCC TAC ACA TGG  528
Glu Glu Ala Gly Glu Asp Val Val Cys Cys Ser Met Ser Tyr Thr Trp
                165                 170                 175
ACA GGC GCC TTA ATT ACA CCA TGC ACC GCG GAG GAG AGC AAG CTG CCC  576
Thr Gly Ala Leu Ile Thr Pro Cys Thr Ala Glu Glu Ser Lys Leu Pro
            180                 185                 190
ATT AAT GCG CTG AGC AAC TCT TTG CTG CGT CAC CAC AAC ATG GTC TAT  624
Ile Asn Ala Leu Ser Asn Ser Leu Leu Arg His His Asn Met Val Tyr
        195                 200                 205
GCC ACA ACA TCC CGC AGC GCA AGC CAG CGG CAG AAA AAG GTC ACA TTT  672
```

```
Ala Thr Thr Ser Arg Ser Ala Ser Gln Arg Gln Lys Lys Val Thr Phe
    210                 215                 220
GAC AGA CTG CAA GTC CTG GAT GAC CAC TAC CGG GAC GTG CTC AAG GAC   720
Asp Arg Leu Gln Val Leu Asp Asp His Tyr Arg Asp Val Leu Lys Asp
225                 230                 235                 240
ATG AAG GCC AAG GCG TCC AC                                        740
Met Lys Ala Lys Ala Ser
                245
```

SEQ ID NO:89

SEQUENCE LENGTH: 515 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: N29-1

```
AC TAC CGG GAC GTG CTG AAG GAG ATG AAG GCG AAG GCG TCC ACA GTT     47
   Tyr Arg Asp Val Leu Lys Glu Met Lys Ala Lys Ala Ser Thr Val
     1               5                  10                  15
AAG GCT AAA CTT CTA TCT GTA GAG GAA GCC TGC AAG CTG ACG CCC CCA   95
Lys Ala Lys Leu Leu Ser Val Glu Glu Ala Cys Lys Leu Thr Pro Pro
                20                  25                  30
CAC TCG GCC AGA TCT AAA TTT GGC TAC GGG GCA AAG GAC GTC CGG AGC  143
His Ser Ala Arg Ser Lys Phe Gly Tyr Gly Ala Lys Asp Val Arg Ser
            35                  40                  45
CTG TCC AGC AAG GCC GTT AAC CAC ATC CGC TCC GTG TGG AAG GAC TTG  191
Leu Ser Ser Lys Ala Val Asn His Ile Arg Ser Val Trp Lys Asp Leu
        50                  55                  60
CTG GAA GAC ACT GAG ACA CCA ATT GAC ACC ACC ATC ATG GCA AAA AAT  239
Leu Glu Asp Thr Glu Thr Pro Ile Asp Thr Thr Ile Met Ala Lys Asn
    65                  70                  75
GAG GTT TTC TGT GTT CAA CCA GAG AAA GGA GGC CGC AAG CCA GCT CGC  287
Glu Val Phe Cys Val Gln Pro Glu Lys Gly Gly Arg Lys Pro Ala Arg
```

```
80                  85                  90                  95
CTT ATC GTA TTC CCA GAC TTG GGG GTT CGT GTG TGC GAG AAA ATG GCC  335
Leu Ile Val Phe Pro Asp Leu Gly Val Arg Val Cys Glu Lys Met Ala
                100                 105                 110
CTC TAC GAC GTG GTC TCC ACT CTT CCT CAG GCC GTG ATG GGC TCC TCA  383
Leu Tyr Asp Val Val Ser Thr Leu Pro Gln Ala Val Met Gly Ser Ser
            115                 120                 125
TAC GGA TTC CAG TAC TCC CCT GGA CAG CGG GTC GAG TTC CTG GTG AAT  431
Tyr Gly Phe Gln Tyr Ser Pro Gly Gln Arg Val Glu Phe Leu Val Asn
        130                 135                 140
GCC TGG AAG TCA AAG AAG AGC CCT ATG GGC TTT GCA TAT GAC ACC CGC  479
Ala Trp Lys Ser Lys Lys Ser Pro Met Gly Phe Ala Tyr Asp Thr Arg
    145                 150                 155
TGT TTT GAC TCA ACG GTC ACC GAG AAC GAC ATC CGT                  515
Cys Phe Asp Ser Thr Val Thr Glu Asn Asp Ile Arg
160                 165                 170
```

SEQ ID NO:90
SEQUENCE LENGTH: 515 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N29-2

```
AC TAC CGG GAC GTG CTG AAG GAG ATG AAG GCG AAG GCG TCC ACA GTT  47
   Tyr Arg Asp Val Leu Lys Glu Met Lys Ala Lys Ala Ser Thr Val
    1               5                  10                  15
AAG GCT AAA CTT CTA TCT GTA GAG GAA GCC TGT AAG CTG ACG CCC CCA  95
Lys Ala Lys Leu Leu Ser Val Glu Glu Ala Cys Lys Leu Thr Pro Pro
                20                  25                  30
CAC TCG GCC AGA TCT AAA TTT GGC TAC GGG GCA AAG GAC GTC CGG AGC  143
His Ser Ala Arg Ser Lys Phe Gly Tyr Gly Ala Lys Asp Val Arg Ser
            35                  40                  45
```

```
CTG TCC AGC AAG GCC GTT AAC CAC ATC CGC TCC GTG TGG AAG GAC TTG  191
Leu Ser Ser Lys Ala Val Asn His Ile Arg Ser Val Trp Lys Asp Leu
        50                  55                  60
CTG GAA GAC ACT GAG ACA CCA ATT GAC ACC ACC ATC ATG GCA AAA AAT  239
Leu Glu Asp Thr Glu Thr Pro Ile Asp Thr Thr Ile Met Ala Lys Asn
    65                  70                  75
GAG GTT TTC TGT GTT CAA CCA GAG AAA GGA GGC CGC AAG CCA GCT CGC  287
Glu Val Phe Cys Val Gln Pro Glu Lys Gly Gly Arg Lys Pro Ala Arg
80                  85                  90                  95
CTT ATC GTA TTC CCA GAC TTG GGG GTT CGT GTG TGC GAG AAA ATG GCC  335
Leu Ile Val Phe Pro Asp Leu Gly Val Arg Val Cys Glu Lys Met Ala
                100                 105                 110
CTC TAC GAC GTG GTC TCC ACT CTT CCT CAG GCC GTG ATG GGC TCC TCA  383
Leu Tyr Asp Val Val Ser Thr Leu Pro Gln Ala Val Met Gly Ser Ser
            115                 120                 125
TAC GGA TTC CAG TAC TCC CCT GGA CAG CGG GTC GAG TTC CTG GTG AAT  431
Tyr Gly Phe Gln Tyr Ser Pro Gly Gln Arg Val Glu Phe Leu Val Asn
        130                 135                 140
GCC TGG AAG TCA AAG AAG AGT CCT ATG GGC TTT GCA TAT GAC ACC CGC  479
Ala Trp Lys Ser Lys Lys Ser Pro Met Gly Phe Ala Tyr Asp Thr Arg
    145                 150                 155
TGT TTT GAC TCA ACG GTC ACC GAG AAC GAC ATC CGT                  515
Cys Phe Asp Ser Thr Val Thr Glu Asn Asp Ile Arg
160                 165                 170
```

SEQ ID NO:91
SEQUENCE LENGTH: 503 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N29-3

```
AC TAC CGG GAC GTG CTG AAG GAG ATG AAG GCG AAG GCG TCC ACA GTT    47
```

```
   Tyr Arg Asp Val Leu Lys Glu Met Lys Ala Lys Ala Ser Thr Val
     1               5                  10                  15
AAG GCT AAA CTT CTA TCT GTA GAG GAA GCC TGT AAG CTG ACG CCC CCA    95
Lys Ala Lys Leu Leu Ser Val Glu Glu Ala Cys Lys Leu Thr Pro Pro
                 20                  25                  30
CAC TCG GCC AGA TCT AAG TTT GGC TAC GGG GCA AAG GAC GTC CGG AGC   143
His Ser Ala Arg Ser Lys Phe Gly Tyr Gly Ala Lys Asp Val Arg Ser
             35                  40                  45
CTG TCC AGC AAG GCC GTT AAC CAC ATC CGC TCC GTG TGG AGG GAC TTG   191
Leu Ser Ser Lys Ala Val Asn His Ile Arg Ser Val Trp Glu Asp Leu
         50                  55                  60
CTG GAA GAC ACT GAA ACA CCA ATT GAC ACC ACC ATC ATG GCA AAA AAT   239
Leu Glu Asp Thr Glu Thr Pro Ile Asp Thr Thr Ile Met Ala Lys Asn
     65                  70                  75
GAG GTT TTC TGT GTT CAA CCA GAG AAA GGA GGC CGC AAG CCA GCT CGC   287
Glu Val Phe Cys Val Gln Pro Glu Lys Gly Gly Arg Lys Pro Ala Arg
 80                  85                  90                  95
CTT ATC GTA TTC CCA GAC TTG GGG GTT CGT GTG TGC GAG AAA ATG GCC   335
Leu Ile Val Phe Pro Asp Leu Gly Val Arg Val Cys Glu Lys Met Ala
                100                 105                 110
CTC TAC GAC GTG GTC TCC ACT CTT CCT CAG GCC GTG ATG GGC TCC TCA   383
Leu Tyr Asp Val Val Ser Thr Leu Pro Gln Ala Val Met Gly Ser Ser
            115                 120                 125
TAC GGA TTC CAG TAC TCC CCT GGA CAG CGG GTC GAG TTC CTG GTG AAT   431
Tyr Gly Phe Gln Tyr Ser Pro Gly Gln Arg Val Glu Phe Leu Val Asn
        130                 135                 140
GCC TGG AAG TCA AAG AAG AGT CCT ATG GGC TTT TCA TAT GAC ACC CGC   479
Ala Trp Lys Ser Lys Lys Ser Pro Met Gly Phe Ser Tyr Asp Thr Arg
    145                 150                 155
TGT TTT GAC TCA ACG GTC ACC GAG                                   503
Cys Phe Asp Ser Thr Val Thr Glu
160                 165
```

SEQ ID NO:92
SEQUENCE LENGTH: 401 base pairs
SEQUENCE TYPE: nucleic acid
STRANDEDNESS: double

TOPOLOGY: linear
ANTI-SENSE: No
ORIGINAL SOURCE
ORGANISM: Hepatitis C virus
IMMEDIATE EXPERIMENTAL SOURCE
CLONE: N18-4

```
TG GGG ATC CCG TAT GAT ACC CGC TGC TTT GAC TCA ACG GTC ACT GAG    47
   Gly Ile Pro Tyr Asp Thr Arg Cys Phe Asp Ser Thr Val Thr Glu
    1               5                  10                  15
AAT GAC ATC CGT ACT GAG GAG TCA ATT TAT CAA TGT TGT GAC TTG GAC   95
Asn Asp Ile Arg Thr Glu Glu Ser Ile Tyr Gln Cys Cys Asp Leu Asp
                20                  25                  30
CCC GAG GCC AGA CAG GCC ATA AGG TCG CTC ACA GAG CGG CTT TAT ATC  143
Pro Glu Ala Arg Gln Ala Ile Arg Ser Leu Thr Glu Arg Leu Tyr Ile
            35                  40                  45
GGG GGC CCC TTG ACC AAT TCA AAA GGG CAA AAC TGC GGC TAT CGC CGG  191
Gly Gly Pro Leu Thr Asn Ser Lys Gly Gln Asn Cys Gly Tyr Arg Arg
        50                  55                  60
TGC CGC GCC AGC GGC GTG CTG ACG ACT AGC TGC GGT AAT ACC CTC ACA  239
Cys Arg Ala Ser Gly Val Leu Thr Thr Ser Cys Gly Asn Thr Leu Thr
    65                  70                  75
TGT TAC TTG AAG GCC TCT GCA GCC TGT CGA GCT GCG AAG CTC CAG GAC  287
Cys Tyr Leu Lys Ala Ser Ala Ala Cys Arg Ala Ala Lys Leu Gln Asp
80                  85                  90                  95
TGC ACG ATG CTC GTG TGC GGA GAC GAC CTT GTC GTT ATC TGT GAA AGC  335
Cys Thr Met Leu Val Cys Gly Asp Asp Leu Val Val Ile Cys Glu Ser
                100                 105                 110
GCG GGA ACC CAG GAG GAC GCG GCA AAC CTA CGA GTC TTC ACG GAG GCT  383
Ala Gly Thr Gln Glu Asp Ala Ala Asn Leu Arg Val Phe Thr Glu Ala
            115                 120                 125
ATG ACC AGG AAT TCC GCC                                          401
Met Thr Arg Asn Ser Ala
        130
```

SEQ ID NO:93
SEQUENCE LENGTH: 401 base pairs

SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N18-2

```
TG GGG ATC CCG TAT GAT ACC CGC TGC TTT GAC TCA ACA GTC ACT GAG    47
   Gly Ile Pro Tyr Asp Thr Arg Cys Phe Asp Ser Thr Val Thr Glu
     1               5                  10                  15
AAC GAC ATC CGT ATT GAG GAG TCA ATT TAT CAA TGC TGT GAC TTG GTC   95
Asn Asp Ile Arg Ile Glu Glu Ser Ile Tyr Gln Cys Cys Asp Leu Val
                20                  25                  30
CCC GAG GCC AGA CAG GCC ATA AGG TCG CTC ACA GAG CGG CTT TAT ATC  143
Pro Glu Ala Arg Gln Ala Ile Arg Ser Leu Thr Glu Arg Leu Tyr Ile
            35                  40                  45
GGG GGC CCC TTG ACC AAT TCA AAA GGG CAA AAC TGC GGC TAT CGC CGG  191
Gly Gly Pro Leu Thr Asn Ser Lys Gly Gln Asn Cys Gly Tyr Arg Arg
        50                  55                  60
TGC CGC GCC AGC GGC GTG CTG ACG ACT AGC TGC GGT AAT ACC CTC ACA  239
Cys Arg Ala Ser Gly Val Leu Thr Thr Ser Cys Gly Asn Thr Leu Thr
    65                  70                  75
TGT TAC TTG AAG GCC TCT GCA GCC TGT CGA GCT GCG AAG CTC CGG GAC  287
Cys Tyr Leu Lys Ala Ser Ala Ala Cys Arg Ala Ala Lys Leu Arg Asp
 80                  85                  90                  95
TGC ACG ATG CTC GTG TGC GGA GAC GAC CTT GTC GTT ATC TGT GAA AGC  335
Cys Thr Met Leu Val Cys Gly Asp Asp Leu Val Val Ile Cys Glu Ser
                100                 105                 110
GCG GGG ACC CAG GAG GAC GCG GCA AGC CTA CGA GTC TTC ACG GAG GCT  383
Ala Gly Thr Gln Glu Asp Ala Ala Ser Leu Arg Val Phe Thr Glu Ala
            115                 120                 125
ATG ACC AGG AAT TCC GCC                                          401
Met Thr Arg Asn Ser Ala
        130
```

SEQ ID NO:94
SEQUENCE LENGTH: 401 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: N18-3

```
TG GGG ATC CCG TAT GAT ACC CGC TGC TTT GAC TCA ACG GTC ACT GAG     47
   Gly Ile Pro Tyr Asp Thr Arg Cys Phe Asp Ser Thr Val Thr Glu
     1               5                  10                  15
AAT GAC ATC CGT ACT GAG GAG TCA ATT TAT CAA TGT TGT GAC TTG GAC    95
Asn Asp Ile Arg Thr Glu Glu Ser Ile Tyr Gln Cys Cys Asp Leu Asp
                20                  25                  30
CCC GAG GCC AGA CAG GCC ATA AGG TCG CTC ACA GAG CGG CTT TAT ATC   143
Pro Glu Ala Arg Gln Ala Ile Arg Ser Leu Thr Glu Arg Leu Tyr Ile
            35                  40                  45
GGG GGC CCC TTG ACC AAT TCA AAA GGG CAG AAC TGC GGT TAT CGC CGG   191
Gly Gly Pro Leu Thr Asn Ser Lys Gly Gln Asn Cys Gly Tyr Arg Arg
        50                  55                  60
TGC CGC GCC AGC GGC GTG CTG ACG ACT AGC TGC GGT AAT ACC CTT ACA   239
Cys Arg Ala Ser Gly Val Leu Thr Thr Ser Cys Gly Asn Thr Leu Thr
    65                  70                  75
TGT TAC TTG AAG GCC TCT GCA GCC TGT CGA GCT GCG AAG CTC CAG GAC   287
Cys Tyr Leu Lys Ala Ser Ala Ala Cys Arg Ala Ala Lys Leu Gln Asp
80                  85                  90                  95
TGC ACG ATG CTC GTG TGC GGA GAC GAC CTT GTC GTT ATC TGT GAA AGC   335
Cys Thr Met Leu Val Cys Gly Asp Asp Leu Val Val Ile Cys Glu Ser
                100                 105                 110
GCG GGA ACC CAG GAG GAC GCG GCA AAC CTA CGA GTC TTC ACG GAG GCT   383
Ala Gly Thr Gln Glu Asp Ala Ala Asn Leu Arg Val Phe Thr Glu Ala
            115                 120                 125
ATG ACC AGG AAT TCC GCC                                           401
Met Thr Arg Asn Ser Ala
```

130

SEQ ID NO:95
SEQUENCE LENGTH: 401 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H18-1

```
TG GGG ATC CCG TAT GAT ACC CGC TGC TTT GAC TCA ACA GTC ACT GAG    47
   Gly Ile Pro Tyr Asp Thr Arg Cys Phe Asp Ser Thr Val Thr Glu
     1               5                  10                  15
AGT GAT ATC CGT GTT GAG GAG TCA ATC TAC CAA TGT TGT GAC TTG GCC   95
Ser Asp Ile Arg Val Glu Glu Ser Ile Tyr Gln Cys Cys Asp Leu Ala
                20                  25                  30
CCC GAG GCC AGA CAG GCC ATA AGG TCG CTC ACA GAG CGG CTT TAT ATC  143
Pro Glu Ala Arg Gln Ala Ile Arg Ser Leu Thr Glu Arg Leu Tyr Ile
            35                  40                  45
GGG GGC CCC CTG ACT AAT TCA AAA GGG CAG AAC TGC GGT TAT CGC CGG  191
Gly Gly Pro Leu Thr Asn Ser Lys Gly Gln Asn Cys Gly Tyr Arg Arg
        50                  55                  60
TGC CGC GTC AGC GGC GTG CTG ACG ACC AGC TGC GGT AAT ACT CTT ACA  239
Cys Arg Val Ser Gly Val Leu Thr Thr Ser Cys Gly Asn Thr Leu Thr
     65                  70                  75
TGT TAC TTG AAG GCC TCT GCA GCC TGT CGA GCT GCA AAG CTC CAG GAC  287
Cys Tyr Leu Lys Ala Ser Ala Ala Cys Arg Ala Ala Lys Leu Gln Asp
 80                  85                  90                  95
TGC ACA ATG CTC GTG TGC GGG GAC GAC CTT GTC GTC ATC TGT GAG AGC  335
Cys Thr Met Leu Val Cys Gly Asp Asp Leu Val Val Ile Cys Glu Ser
                100                 105                 110
GCG GGA ACC CAG GAG GAC GCG GCG AAC CTA CGA GTC TTC ACG GAG GCT  383
Ala Gly Thr Gln Glu Asp Ala Ala Asn Leu Arg Val Phe Thr Glu Ala
            115                 120                 125
```

```
ATG ACC AGG AAT TCC GCC                                                 401
Met Thr Arg Asn Ser Ala
        130
```

SEQ ID NO:96
SEQUENCE LENGTH: 401 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: H18-2

```
TG GGG ATC CCG TAT GAT ACC CGC TGC TTT GAC TCA ACA GTC ACT GAG      47
   Gly Ile Pro Tyr Asp Thr Arg Cys Phe Asp Ser Thr Val Thr Glu
     1               5                  10                  15
AGT GAT ATC CGT GTT GAG GAG TCA ATC TAC CAA TGT TGT GAC TTG GCC     95
Ser Asp Ile Arg Val Glu Glu Ser Ile Tyr Gln Cys Cys Asp Leu Ala
                    20                  25                  30
CCC GAG GCC AGA CAG GCC ATA AGG TCG CTC ACA GAG CGG CTT TAT ATC    143
Pro Glu Ala Arg Gln Ala Ile Arg Ser Leu Thr Glu Arg Leu Tyr Ile
                35                  40                  45
GGG GGC CCC CTG ACT AAT TCA AAG GGG CAG AAC TGC GGT TAT CGC CGG    191
Gly Gly Pro Leu Thr Asn Ser Lys Gly Gln Asn Cys Gly Tyr Arg Arg
            50                  55                  60
TGC CGC GTC AGC GGC GTG CTG ACG ACC AGC TGC GGT AAT ACC CTT ACA    239
Cys Arg Val Ser Gly Val Leu Thr Thr Ser Cys Gly Asn Thr Leu Thr
        65                  70                  75
TGT TAC TTG AAG GCC TCT GCA GCC TGT CGA GCT GCA AAG CTC CAG GAC    287
Cys Tyr Leu Lys Ala Ser Ala Ala Cys Arg Ala Ala Lys Leu Gln Asp
 80                  85                  90                  95
TGC ACA ATG CTC GTG TGC GGG GAC GAC CTT GTC GTC ATC TGT GAA AGC    335
Cys Thr Met Leu Val Cys Gly Asp Asp Leu Val Val Ile Cys Glu Ser
                100                 105                 110
GCG GGA ACC CAG GAG GAC GCG GCG AAC CTA CGA GTC TTC ACG GAG GCT    383
```

```
Ala Gly Thr Gln Glu Asp Ala Ala Asn Leu Arg Val Phe Thr Glu Ala
            115                 120                 125
ATG ACC AGG AAT TCC GCC                                              401
Met Thr Arg Asn Ser Ala
        130
```

SEQ ID NO:97

SEQUENCE LENGTH: 401 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: H18-3

```
TG GGG ATC CCG TAT GAT ACC CGC TGC TTT GAC TCA ACA GTC ACC GAG     47
   Gly Ile Pro Tyr Asp Thr Arg Cys Phe Asp Ser Thr Val Thr Glu
     1               5                  10                  15
AGT GAT ATC CGT GTT GAG GAG TCA ATC TAC CAA TGT TGT GAC TTG GCC    95
Ser Asp Ile Arg Val Glu Glu Ser Ile Tyr Gln Cys Cys Asp Leu Ala
                20                  25                  30
CCC GAG GCC AGA CAG GCT ATA AGG TCG CTC ACA GAG CGG CTT TAT ATC   143
Pro Glu Ala Arg Gln Ala Ile Arg Ser Leu Thr Glu Arg Leu Tyr Ile
            35                  40                  45
GGG GGC CCC CTG ACT AAT TCA AAA GGG CAG AAC TGC GGT TAT CGC CGG   191
Gly Gly Pro Leu Thr Asn Ser Lys Gly Gln Asn Cys Gly Tyr Arg Arg
        50                  55                  60
TGC CGC GTC AGC GGC GTG CTG ACG ACC AGC TGC GGT AAT ACC CTT ACA   239
Cys Arg Val Ser Gly Val Leu Thr Thr Ser Cys Gly Asn Thr Leu Thr
    65                  70                  75
TGT TAC TTG AAG GCC TCT GCA GCC TGT CGA GCT GCA AAG CTC CAG GAC   287
Cys Tyr Leu Lys Ala Ser Ala Ala Cys Arg Ala Ala Lys Leu Gln Asp
80                  85                  90                  95
TGC ACA ATG CTC GTG TGC GGG GAC GAC CTT GTC GTC ATC TGT GAA AGC   335
Cys Thr Met Leu Val Cys Gly Asp Asp Leu Val Val Ile Cys Glu Ser
```

```
                    100                     105                     110
GCG GGA ACC CAG GAG GAC GCG GCG AAC CTA CGA GTC TTC ACG GAG GCT   383
Ala Gly Thr Gln Glu Asp Ala Ala Asn Leu Arg Val Phe Thr Glu Ala
                115                     120                     125
ATG ACC AGG AAT TCC GCC                                           401
Met Thr Arg Asn Ser Ala
        130
```

SEQ ID NO:98
SEQUENCE LENGTH: 1171 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O30-3

```
TG GGG ATC CCG TAT GAT ACC CGC TGC TTT GAC TCA ACG GTC ACT GAG     47
   Gly Ile Pro Tyr Asp Thr Arg Cys Phe Asp Ser Thr Val Thr Glu
    1               5                  10                  15
AAT GAC ATC CGT GTC GAG GAG TCA ATT TAC CAA TGT TGT GAC TTG GCC    95
Asn Asp Ile Arg Val Glu Glu Ser Ile Tyr Gln Cys Cys Asp Leu Ala
                    20                  25                  30
CCC GAG GCC AGA CAG GCC ATA AGG TCA CTC ACA GAG CGG CTT TAC ATC   143
Pro Glu Ala Arg Gln Ala Ile Arg Ser Leu Thr Glu Arg Leu Tyr Ile
                35                  40                  45
GGG GGC CCC CTG ACT AAT TCA AAG GGG CAG AAC TGC GGT TAT CGC CGG   191
Gly Gly Pro Leu Thr Asn Ser Lys Gly Gln Asn Cys Gly Tyr Arg Arg
            50                  55                  60
TGC CGC GTC AGC GGC GTG CTG ACG ACT AGC TGC GGT AAT ACC CTC ACA   239
Cys Arg Val Ser Gly Val Leu Thr Thr Ser Cys Gly Asn Thr Leu Thr
        65                  70                  75
TGT TAC TTG AAG GCC TCT GCA GCC TGT CGA GCT GCA AAG CTC CAG GAC   287
Cys Tyr Leu Lys Ala Ser Ala Ala Cys Arg Ala Ala Lys Leu Gln Asp
80                  85                  90                  95
```

```
TGC ACG ATG CTT GTG TGC GGA GAC GAC CTT GTC GTT ATC TGT GAT AGC   335
Cys Thr Met Leu Val Cys Gly Asp Asp Leu Val Val Ile Cys Asp Ser
                100                 105                 110
GCG GGA ACT CAG GAG GAC GCG GCG AGC CTA CGA GTC TTC ACG GAG GCT   383
Ala Gly Thr Gln Glu Asp Ala Ala Ser Leu Arg Val Phe Thr Glu Ala
            115                 120                 125
ATG ACT AGG TAC TCT GCC CCC CCC GGG GAC CCG CCC CAA CCA GAA TAC   431
Met Thr Arg Tyr Ser Ala Pro Pro Gly Asp Pro Pro Gln Pro Glu Tyr
        130                 135                 140
GAC TTG GAG CTG ATA ACA TCA TGT TCC TCC AAT GTG TCG GTC GCG CAC   479
Asp Leu Glu Leu Ile Thr Ser Cys Ser Ser Asn Val Ser Val Ala His
    145                 150                 155
GAC GCA TCA GGC AAA CGG GTG TAC TAT CTC ACC CGT GAC CCC ACC ACC   527
Asp Ala Ser Gly Lys Arg Val Tyr Tyr Leu Thr Arg Asp Pro Thr Thr
160                 165                 170                 175
CCC CTA GCG CGG GCT GCG TGG GAG ACA GCT AGA CAC ACT CCA GTC AAC   575
Pro Leu Ala Arg Ala Ala Trp Glu Thr Ala Arg His Thr Pro Val Asn
                180                 185                 190
TCC TGG CTA GGC AAC ATC ATC ATG TAC GCG CCC ACC TTA TGG GCA AGG   623
Ser Trp Leu Gly Asn Ile Ile Met Tyr Ala Pro Thr Leu Trp Ala Arg
            195                 200                 205
ATG ATT CTG ATG ACC CAC TTC TTC TCC ATC CTT CTA GCC CAG GAG CAA   671
Met Ile Leu Met Thr His Phe Phe Ser Ile Leu Leu Ala Gln Glu Gln
        210                 215                 220
CTT GAA AAA GCC CTA GAT TGT CAG ATC TAC GGG GCC ACT TAC TCC ATT   719
Leu Glu Lys Ala Leu Asp Cys Gln Ile Tyr Gly Ala Thr Tyr Ser Ile
    225                 230                 235
GAG CCA CTT GAC CTA CCT CAG ATC ATT CAA CGA CTC CAC GGT CTT AGC   767
Glu Pro Leu Asp Leu Pro Gln Ile Ile Gln Arg Leu His Gly Leu Ser
240                 245                 250                 255
GCA TTT TCA CTC CAT AGT TAC TCT CCA GGT GAG ATC AAT AGG GTG GCT   815
Ala Phe Ser Leu His Ser Tyr Ser Pro Gly Glu Ile Asn Arg Val Ala
                260                 265                 270
TCA TGC CTC AGG AAA CTT GGG GTA CCG CCC TTG CGA GTC TGG AGA CAT   863
Ser Cys Leu Arg Lys Leu Gly Val Pro Pro Leu Arg Val Trp Arg His
            275                 280                 285
CGG GCC AGA AGC GTC CGC GCT AAG CTA CTG TCC CAG GGG GGG AGG GCC   911
```

```
Arg Ala Arg Ser Val Arg Ala Lys Leu Leu Ser Gln Gly Gly Arg Ala
        290                 295                 300
GCC ACC TGT GGC AAA TAC CTC TTC AAC TGG GCA GTA AAG ACC AAG CTC   959
Ala Thr Cys Gly Lys Tyr Leu Phe Asn Trp Ala Val Lys Thr Lys Leu
    305                 310                 315
AAA CTC ACT CCA ATC CCA GAA GCG TCC CAG CTG GAC TTG TCC GGC TGG 1007
Lys Leu Thr Pro Ile Pro Glu Ala Ser Gln Leu Asp Leu Ser Gly Trp
320                 325                 330                 335
TTC GTT GCT GGT TAC AGC GGG GGA GAC ATA TAT CAC AGC CTG TCT CGT 1055
Phe Val Ala Gly Tyr Ser Gly Gly Asp Ile Tyr His Ser Leu Ser Arg
                340                 345                 350
GCC CGA CCC CGC TGG TTC ATG TGG TGC CTA CTC CTA CTT TCC GTA GGG 1103
Ala Arg Pro Arg Trp Phe Met Trp Cys Leu Leu Leu Leu Ser Val Gly
            355                 360                 365
GTA GGC ATC TAC CTG CTC CCC AAC CGA TGA GCG GGG AGC TAA ACA CTC 1151
Val Gly Ile Tyr Leu Leu Pro Asn Arg StopAla Gly Ser StopThr Leu
        370                 375                 380
CAG GCC AAT AGG CCA TCC C C                                     1171
Gln Ala Asn Arg Pro Ser
    385
```

SEQ ID NO:99
SEQUENCE LENGTH: 1170 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: O30-2

```
 G GGG ATC CCG TAT GAT ACC CGC TGC TTT GAC TCA ACG GTC ACT GAG    46
   Gly Ile Pro Tyr Asp Thr Arg Cys Phe Asp Ser Thr Val Thr Glu
     1               5                  10                  15
AAT GAC ATC CGT GTT GAG GAG TCA ATT TAC CAA TGT TGT GAC TTG GCC   94
Asn Asp Ile Arg Val Glu Glu Ser Ile Tyr Gln Cys Cys Asp Leu Ala
```

```
                         20                      25                      30
CCC GAG GCC AGA CAG GCC ATA AGG TCG CTC ACA GAG CGG CTT TAC ATC  142
Pro Glu Ala Arg Gln Ala Ile Arg Ser Leu Thr Glu Arg Leu Tyr Ile
                    35                      40                      45
GGG GGC CCC CTG ACT AAT TCA AAA GGG CAG AAC TGC GGC TAT CGC CGG  190
Gly Gly Pro Leu Thr Asn Ser Lys Gly Gln Asn Cys Gly Tyr Arg Arg
            50                      55                      60
TGC CGC GTC AGC GGC GTG CTG ACG ACT AGC TGC GGC AAT ACC CTC ACA  238
Cys Arg Val Ser Gly Val Leu Thr Thr Ser Cys Gly Asn Thr Leu Thr
        65                      70                      75
TGT TAC TTG AAG GCC TCT GCA GCC TGT CGA GCT GCA AAG CTC CAG GAC  286
Cys Tyr Leu Lys Ala Ser Ala Ala Cys Arg Ala Ala Lys Leu Gln Asp
 80                      85                      90                      95
TGC ACG ATG CTT GTG TGC GGA GAC GAC CTT GTC GTT ATC TGT GAA AGC  334
Cys Thr Met Leu Val Cys Gly Asp Asp Leu Val Val Ile Cys Glu Ser
                        100                     105                     110
GCG GGA ACT CAG GAG GAC GCG GCG AGC CTA CGA GTC TTC ACG GAG GCT  382
Ala Gly Thr Gln Glu Asp Ala Ala Ser Leu Arg Val Phe Thr Glu Ala
                115                     120                     125
ATG ACT AGG TAC TCT GCC CCC CCC GGG GAC CCG CCC CAA CCA GAA TAC  430
Met Thr Arg Tyr Ser Ala Pro Pro Gly Asp Pro Pro Gln Pro Glu Tyr
            130                     135                     140
GAC TTG GAG CTG ATA ACA TCA TGC TCC TCC AAC GTG TCG GTC GCG CAC  478
Asp Leu Glu Leu Ile Thr Ser Cys Ser Ser Asn Val Ser Val Ala His
        145                     150                     155
GAC GCA TCA GGC AAA CGG GTG TAC TAC CTC ACC CGT GAC CCC ACC ACC  526
Asp Ala Ser Gly Lys Arg Val Tyr Tyr Leu Thr Arg Asp Pro Thr Thr
160                     165                     170                     175
CCC CTT GCG CGG GCT GCG TGG GAG ACA GCT AGA CAC ACT CCA GTC AAC  574
Pro Leu Ala Arg Ala Ala Trp Glu Thr Ala Arg His Thr Pro Val Asn
                        180                     185                     190
TCC TGG CTA GGC AAC ATC ATC ATG TAT GCG CCC ACC TTA TGG GCA AGG  622
Ser Trp Leu Gly Asn Ile Ile Met Tyr Ala Pro Thr Leu Trp Ala Arg
                195                     200                     205
ATG ATT CTG ATG ACC CAC TTC TTC TCC ATC CTT CTA GCC CAG GAG CAA  670
Met Ile Leu Met Thr His Phe Phe Ser Ile Leu Leu Ala Gln Glu Gln
            210                     215                     220
```

```
CTT GAA AAA GCC CTA GAT TGT CAG ATC TAT GGG GCC ACT TAC TCC ATT   718
Leu Glu Lys Ala Leu Asp Cys Gln Ile Tyr Gly Ala Thr Tyr Ser Ile
    225                 230                 235
GAG CCA CTT GAC CTA CCT CAG ATC ATT CAA CGA CTC CAT GGT CTT AGC   766
Glu Pro Leu Asp Leu Pro Gln Ile Ile Gln Arg Leu His Gly Leu Ser
240                 245                 250                 255
GCA TTT TCA CTC CAT AGT TAC TCT CCA GGT GAG ATC AAT AGG GTG GCT   814
Ala Phe Ser Leu His Ser Tyr Ser Pro Gly Glu Ile Asn Arg Val Ala
                260                 265                 270
TCA TGC CTC AGG AAA CTT GGG GTA CCG CCC TTG CGA GTC TGG AGA CAT   862
Ser Cys Leu Arg Lys Leu Gly Val Pro Pro Leu Arg Val Trp Arg His
            275                 280                 285
CGG GCC AGA AGC GTC CGC GCT AAG CTA CTG TCC CAG GGG GGG AGG GCC   910
Arg Ala Arg Ser Val Arg Ala Lys Leu Leu Ser Gln Gly Gly Arg Ala
        290                 295                 300
GCC ACC TGT GGC AAA TAC CTC TTC AAC TGG GCA GTA AAG ACC AAG CTC   958
Ala Thr Cys Gly Lys Tyr Leu Phe Asn Trp Ala Val Lys Thr Lys Leu
    305                 310                 315
AAA CCC ACT CCA ATC CCG GAA GCG TCC CAG CTG GAC TTG TCC GGC TGG 1006
Lys Pro Thr Pro Ile Pro Glu Ala Ser Gln Leu Asp Leu Ser Gly Trp
320                 325                 330                 335
TTC GTT GCT GGT TAC AGC GGG GGA GAC ATA TAT CAC AGC CTG TCT CGT 1054
Phe Val Ala Gly Tyr Ser Gly Gly Asp Ile Tyr His Ser Leu Ser Arg
                340                 345                 350
GCC CGA CCC CGC TGG TTT ATG TGG TGC CTA CTC CTA CTT TCC GTA GGG 1102
Ala Arg Pro Arg Trp Phe Met Trp Cys Leu Leu Leu Leu Ser Val Gly
            355                 360                 365
GTA GGC ATC TAC CTG CTC CCC AAC CGA TGA GCG GGG AGC TAA ACA CTC 1150
Val Gly Ile Tyr Leu Leu Pro Asn Arg StopAla Gly Ser StopThr Leu
        370                 375                 380
CAG GCC AAT AGG CCA TCC C C                                     1170
Gln Ala Asn Arg Pro Ser
    385
```

SEQ ID NO:100

SEQUENCE LENGTH: 1171 base pairs

SEQUENCE TYPE: nucleic acid

STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: 030-4

```
TG GGG ATC CCG TAT GAT ACC CGC TGC TTT GAC TCA ACA GTC ACT GAG     47
   Gly Ile Pro Tyr Asp Thr Arg Cys Phe Asp Ser Thr Val Thr Glu
     1               5                  10                  15
AAT GAC ATC CGT GTT GAG GAG TCA ATT TAC CAA TGT TGT GAC TTG GCC    95
Asn Asp Ile Arg Val Glu Glu Ser Ile Tyr Gln Cys Cys Asp Leu Ala
                20                  25                  30
CCC GAG GCC AGA CAG GCC ATA AGG TCG CTC ACA GAG CGG CTT TAC ATC   143
Pro Glu Ala Arg Gln Ala Ile Arg Ser Leu Thr Glu Arg Leu Tyr Ile
            35                  40                  45
GGG GGC CCC CTG ACT AAT TCA AAG GGG CAG AAC TGC GGT TAT CGC CGG   191
Gly Gly Pro Leu Thr Asn Ser Lys Gly Gln Asn Cys Gly Tyr Arg Arg
        50                  55                  60
TGC CGC GCC AGC GGC GTG CTG ACG ACT AGC TGC GGT AAT ACC CTC ACA   239
Cys Arg Ala Ser Gly Val Leu Thr Thr Ser Cys Gly Asn Thr Leu Thr
    65                  70                  75
TGT TAC TTG AAG GCC TCT GCA GCC TGT CGA GCT GCA AAG CTC CAG GAC   287
Cys Tyr Leu Lys Ala Ser Ala Ala Cys Arg Ala Ala Lys Leu Gln Asp
80                  85                  90                  95
TGC ACG ATG CTT GTG TGC GGA GAC GAC CTT GTC GTT ATC TGT GAA AGC   335
Cys Thr Met Leu Val Cys Gly Asp Asp Leu Val Val Ile Cys Glu Ser
                100                 105                 110
GCG GGA ACT CAG GAG GAC GCG GCG AGC CTA CGA GTC TTC ACG GAG GCT   383
Ala Gly Thr Gln Glu Asp Ala Ala Ser Leu Arg Val Phe Thr Glu Ala
            115                 120                 125
ATG ACT AGG TAC TCT GCC CCC CCC GGG GAC CCG CCC CAA CCA GAA TAC   431
Met Thr Arg Tyr Ser Ala Pro Pro Gly Asp Pro Pro Gln Pro Glu Tyr
        130                 135                 140
GAC TTG GAG CTG ATA ACA TCA TGC TCC TCC AAT GTG TCG GTC GCG CAC   479
Asp Leu Glu Leu Ile Thr Ser Cys Ser Ser Asn Val Ser Val Ala His
```

```
    145                 150                 155
GAC GCA TCA GGC AAA CGG GTG TAC TAT CTC ACC CGT GAC CCC CCC ACC   527
Asp Ala Ser Gly Lys Arg Val Tyr Tyr Leu Thr Arg Asp Pro Pro Thr
160                 165                 170                 175
CCC CTT GCG CGG GCT GCG TGG GAG ACA GCT AGA CAC ACT CCA GTC AAC   575
Pro Leu Ala Arg Ala Ala Trp Glu Thr Ala Arg His Thr Pro Val Asn
                180                 185                 190
TCC TGG CTA GGC AAC ATC ATC ATG TAC GCG CCC ACC TTA TGG GCA AGG   623
Ser Trp Leu Gly Asn Ile Ile Met Tyr Ala Pro Thr Leu Trp Ala Arg
            195                 200                 205
ATG ATT CTG ATG ACC CAC TTC TTC TCC ATC CTT CTA GCC CAG GAG CAA   671
Met Ile Leu Met Thr His Phe Phe Ser Ile Leu Leu Ala Gln Glu Gln
        210                 215                 220
CTT GAA AAA GCC CTA GAT TGT CAG ATC TAC GGG GCC ACT TAC TCC ATT   719
Leu Glu Lys Ala Leu Asp Cys Gln Ile Tyr Gly Ala Thr Tyr Ser Ile
    225                 230                 235
GAG CCA CTT GAC CTA CCT CAG ATC ATT CAA CGA CTC CAT GGT CTT AGC   767
Glu Pro Leu Asp Leu Pro Gln Ile Ile Gln Arg Leu His Gly Leu Ser
240                 245                 250                 255
GCA TTT TCA CTC CAT AGT TAC TCT CCA GGT GAG ATC AAT AGG GTG GCT   815
Ala Phe Ser Leu His Ser Tyr Ser Pro Gly Glu Ile Asn Arg Val Ala
                260                 265                 270
TCA TGC CTC AGG AAA CTT GGG GTA CCG CCC TTG CGA GTC TGG AGA CAT   863
Ser Cys Leu Arg Lys Leu Gly Val Pro Pro Leu Arg Val Trp Arg His
            275                 280                 285
CGG GCC AGA AGC GTC CGC GCT AAG CTA CTG TCC CAG GGG GGG AGG GCC   911
Arg Ala Arg Ser Val Arg Ala Lys Leu Leu Ser Gln Gly Gly Arg Ala
        290                 295                 300
GCC ACC TGT GGC AAA TAC CTC TTC AAC TGG GCA GTA AAG ACC AAG CTC   959
Ala Thr Cys Gly Lys Tyr Leu Phe Asn Trp Ala Val Lys Thr Lys Leu
    305                 310                 315
AAA CTC ACT CCA ATC CCG GAA GCG TCC CAG CTG GAC TTG TCC GGC TGG 1007
Lys Leu Thr Pro Ile Pro Glu Ala Ser Gln Leu Asp Leu Ser Gly Trp
320                 325                 330                 335
TTC GTT GCT GGT TAC AGC GGG GGA GAC ATA TAT CAC AGC CTG TCT CGT 1055
Phe Val Ala Gly Tyr Ser Gly Gly Asp Ile Tyr His Ser Leu Ser Arg
                340                 345                 350
```

```
GCC CGA CCC CGC TGG TTC ATG TGG TGC CTA CTC CTA CTT TCC GTA GGG 1103
Ala Arg Pro Arg Trp Phe Met Trp Cys Leu Leu Leu Leu Ser Val Gly
            355                 360                 365
GTA GGC ATC TAC CTG CTC CCC AAC CGA TGA GCG GGG AGC TAA ACA CTC 1151
Val Gly Ile Tyr Leu Leu Pro Asn Arg StopAla Gly Ser StopThr Leu
        370                 375                 380
CAG GCC AAT AGG CCA TCC C C                                     1171
Gln Ala Asn Arg Pro Ser
    385
```

SEQ ID NO:101

SEQUENCE LENGTH: 7911 base pairs

SEQUENCE TYPE: nucleic acid

STRANDEDNESS: double

TOPOLOGY: linear

ANTI-SENSE: No

ORIGINAL SOURCE

ORGANISM: Hepatitis C virus

IMMEDIATE EXPERIMENTAL SOURCE

CLONE: T7N1-30

```
ACTAGTTAAT ACGACTCACT ATAGGGTGCC AGCCCCCTGA TGGGGGCGAC ACTCCACCAT 60
AGATCACTCC CCTGTGAGGA ACTACTGTCT TCACGCAGAA AGCGTCTAGC CATGGCGTTA 120
GTATGAGTGT CGTGCAGCCT CCAGGACCCC CCCTCCCGGG AGAGCCATAG TGGTCTGCGG 180
AACCGGTGAG TACACCGGAA TTGCCAGGAC GACCGGGTCC TTTCTTGGAT CAACCCGCTC 240
AATGCCTGGA GATTTGGGCG TGCCCCCGCG AGACTGCTAG CCGAGTAGTG TTGGGTCGCG 300
AAAGGCCTTG TGGTACTGCC TGATAGGGTG CTTGCGAGTG CCCCGGGAGG TCTCGTAGAC 360
CGTGCATC ATG AGC ACA AAT CCA AAA CCC CAA AGA AAA ATC AAA CGT AAC  410
         Met Ser Thr Asn Pro Lys Pro Gln Arg Lys Ile Lys Arg Asn
          1               5                  10
ACC AAC CGC CGC CCA CAG GAC GTT AAG TTC CCG GGC GGT GGT CAG ATC  458
Thr Asn Arg Arg Pro Gln Asp Val Lys Phe Pro Gly Gly Gly Gln Ile
 15                  20                  25                  30
GTT GGT GGA GTT TAC CTG TTG CCG CGC AGG GGC CCC AGG TTG GGT GTG  506
Val Gly Gly Val Tyr Leu Leu Pro Arg Arg Gly Pro Arg Leu Gly Val
                 35                  40                  45
CGC GCG ACT AGG AAG ACT TCC GAG CGG CCG CAA CCT CGT GGA AGG CGA  554
```

```
Arg Ala Thr Arg Lys Thr Ser Glu Arg Pro Gln Pro Arg Gly Arg Arg
            50                  55                  60
CAA CCT ATC CCC AAG GCT CGC CAA CCC GAG GGT AGG GCC TGG GCT CAG     602
Gln Pro Ile Pro Lys Ala Arg Gln Pro Glu Gly Arg Ala Trp Ala Gln
        65                  70                  75
CCC GGG TAC CCT TGG CCC CTC TAT GGC AAT GAG GGC TTG GGG TGG GCA     650
Pro Gly Tyr Pro Trp Pro Leu Tyr Gly Asn Glu Gly Leu Gly Trp Ala
    80                  85                  90
GGA TGG CTC CTG TCA CCC CGC GGC TCC CGG CCT AGT TGG GGC CCC ACG     698
Gly Trp Leu Leu Ser Pro Arg Gly Ser Arg Pro Ser Trp Gly Pro Thr
95                  100                 105                 110
GAC CCC CGG CGT AGG TCG CGT AAT TTG GGT AAG GTC ATC GAT ACC CTC     746
Asp Pro Arg Arg Arg Ser Arg Asn Leu Gly Lys Val Ile Asp Thr Leu
                115                 120                 125
ACA TGC GGC TTC GCC GAC CTC ATG GGG TAC ATT CCG CTC GTC GGC GCC     794
Thr Cys Gly Phe Ala Asp Leu Met Gly Tyr Ile Pro Leu Val Gly Ala
            130                 135                 140
CCC CTA GGG GGC GCT GCC AGG GCT CTA GCG CAT GGC GTC CGG GTT CTG     842
Pro Leu Gly Gly Ala Ala Arg Ala Leu Ala His Gly Val Arg Val Leu
        145                 150                 155
GAG GAC GGC GTG AAC TAT GCA ACA GGG AAT CTG CCT GGT TGC TCC TTT     890
Glu Asp Gly Val Asn Tyr Ala Thr Gly Asn Leu Pro Gly Cys Ser Phe
    160                 165                 170
TCT ATC TTC CTT TTG GCT TTG CTG TCC TGT TTG ACC ATC CCA GCT TCC     938
Ser Ile Phe Leu Leu Ala Leu Leu Ser Cys Leu Thr Ile Pro Ala Ser
175                 180                 185                 190
GCC TAC CAA GTG CGC AAC GCG TCC GGG GTG TAC CAT GTC ACG AAC GAC     986
Ala Tyr Gln Val Arg Asn Ala Ser Gly Val Tyr His Val Thr Asn Asp
                195                 200                 205
TGC TCC AAC TCA AGT ATT GTG TAT GAG GCG GCG GAC GTG ATT ATG CAC    1034
Cys Ser Asn Ser Ser Ile Val Tyr Glu Ala Ala Asp Val Ile Met His
            210                 215                 220
ACC CCC GGG TGC GTG CCC TGC GTC CGG GAG AAC AAT TCC TCC CGC TGC    1082
Thr Pro Gly Cys Val Pro Cys Val Arg Glu Asn Asn Ser Ser Arg Cys
        225                 230                 235
TGG GTA GCG CTC ACT CCC ACG CTT GCG GCC AGG AAC AGC AGC ATC CCC    1130
Trp Val Ala Leu Thr Pro Thr Leu Ala Ala Arg Asn Ser Ser Ile Pro
```

```
240                     245                     250
ACT ACG ACA ATA CGG CGT CAT GTC GAC TTG CTC GTT GGG GCA GCT GCT  1178
Thr Thr Thr Ile Arg Arg His Val Asp Leu Leu Val Gly Ala Ala Ala
255                 260                     265                 270
CTC TGT TCC GCT ATG TAT GTG GGG GAT TTT TGC GGA TCT GTT TTC CTC  1226
Leu Cys Ser Ala Met Tyr Val Gly Asp Phe Cys Gly Ser Val Phe Leu
                275                     280                 285
GTC TCC CAG CTG TTC ACT TTC TCA CCT CGC CGG TAT GAG ACG GTG CAA  1274
Val Ser Gln Leu Phe Thr Phe Ser Pro Arg Arg Tyr Glu Thr Val Gln
            290                     295                 300
GAC TGC AAT TGC TCA ATC TAT CCC GGC CAT GTA TCA GGC CAT CGC ATG  1322
Asp Cys Asn Cys Ser Ile Tyr Pro Gly His Val Ser Gly His Arg Met
        305                     310                     315
GCT TGG GAT ATG ATA ATG AAT TGG TCA CCT ACA ACA GCC CTA GTG GTA  1370
Ala Trp Asp Met Ile Met Asn Trp Ser Pro Thr Thr Ala Leu Val Val
    320                     325                     330
TCG CAG CTA CTC CGG ATC CCA CAA GCC GTG GTG GAT ATG GTG GCA GGG  1418
Ser Gln Leu Leu Arg Ile Pro Gln Ala Val Val Asp Met Val Ala Gly
335                 340                     345                 350
GCC CAC TGG GGA GTC CTG GCG GGC CTT GCC TAC TAT TCC ATG GTG GGG  1466
Ala His Trp Gly Val Leu Ala Gly Leu Ala Tyr Tyr Ser Met Val Gly
                355                     360                 365
AAC TGG GCT AAG GTC TTG GTT GTG ATG CTG CTC TTC GCC GGT GTT GAC  1514
Asn Trp Ala Lys Val Leu Val Val Met Leu Leu Phe Ala Gly Val Asp
            370                     375                 380
GGG GGG ACC CAC GTG ACA GGG GGG AAG GTA GCC TAC ACC ACC CAG GGC  1562
Gly Gly Thr His Val Thr Gly Gly Lys Val Ala Tyr Thr Thr Gln Gly
        385                     390                     395
TTT ACA TCC TTC TTT TCA CGA GGG CCG TCT CAG AAA ATC CAA CTT GTA  1610
Phe Thr Ser Phe Phe Ser Arg Gly Pro Ser Gln Lys Ile Gln Leu Val
    400                     405                     410
AAC ACT AAC GGC AGC TGG CAC ATC AAT AGG ACT GCC CTC AAT TGC AAT  1658
Asn Thr Asn Gly Ser Trp His Ile Asn Arg Thr Ala Leu Asn Cys Asn
415                 420                     425                 430
GAC TCC CTT AAC ACC GGG TTC CTT GCC GCG CTG TTC TAC ACC CAC AGC  1706
Asp Ser Leu Asn Thr Gly Phe Leu Ala Ala Leu Phe Tyr Thr His Ser
                435                     440                     445
```

```
TTC AAC GCG TCC GGA TGT CCG GAG CGT ATG GCC GGT TGC CGC CCC ATT  1754
Phe Asn Ala Ser Gly Cys Pro Glu Arg Met Ala Gly Cys Arg Pro Ile
            450                 455                 460
GAC GAG TTC GCT CAG GGG TGG GGT CCC ATC ACT CAT GTT GTG CCT AAC  1802
Asp Glu Phe Ala Gln Gly Trp Gly Pro Ile Thr His Val Val Pro Asn
        465                 470                 475
ATC TCG GAC CAG AGG CCC TAT TGC TGG CAC TAC GCG CCT CGA CCG TGT  1850
Ile Ser Asp Gln Arg Pro Tyr Cys Trp His Tyr Ala Pro Arg Pro Cys
    480                 485                 490
GGT ATC GTA CCC GCG TCG CAG GTG TGT GGT CCG GTG TAT TGC TTC ACC  1898
Gly Ile Val Pro Ala Ser Gln Val Cys Gly Pro Val Tyr Cys Phe Thr
495                 500                 505                 510
CCA AGC CCT GTT GTG GTG GGG ACG ACC GAT CGT TTC GGC GCC CCC ACG  1946
Pro Ser Pro Val Val Val Gly Thr Thr Asp Arg Phe Gly Ala Pro Thr
                515                 520                 525
TAC AAC TGG GGA AAC AAT GAG ACG GAT GTG CTA CTC CTC AAC AAC ACA  1994
Tyr Asn Trp Gly Asn Asn Glu Thr Asp Val Leu Leu Leu Asn Asn Thr
            530                 535                 540
CGG CCG CCG CAG GGC AAC TGG TTC GGT TGT ACC TGG ATG AAT GGC ACT  2042
Arg Pro Pro Gln Gly Asn Trp Phe Gly Cys Thr Trp Met Asn Gly Thr
        545                 550                 555
GGG TTC ACA AAG ACG TGC GGG GGC CCC CCG TGC AAC ATC GGG GGG GTC  2090
Gly Phe Thr Lys Thr Cys Gly Gly Pro Pro Cys Asn Ile Gly Gly Val
    560                 565                 570
GGC AAC AAT ACC TTG ACT TGC CCC ACG GAC TGC TTC CGG AAG CAC CCC  2138
Gly Asn Asn Thr Leu Thr Cys Pro Thr Asp Cys Phe Arg Lys His Pro
575                 580                 585                 590
GAG GCC ACT TAC ACA AAA TGT GGT TCG GGG CCT TGG TTG ACA CCT AGG  2186
Glu Ala Thr Tyr Thr Lys Cys Gly Ser Gly Pro Trp Leu Thr Pro Arg
                595                 600                 605
TGC CTA GTT CAT TAC CCA TAC AGG CTC TGG CAC TAT CCC TGC ACT GTC  2234
Cys Leu Val His Tyr Pro Tyr Arg Leu Trp His Tyr Pro Cys Thr Val
            610                 615                 620
AAC TTT ACC ATC TTC AAG GTT AGG ATG TAT GTG GGG GGC GTG GAA CAC  2282
Asn Phe Thr Ile Phe Lys Val Arg Met Tyr Val Gly Gly Val Glu His
        625                 630                 635
AGG CTT GAA GCT GCA TGC AAT TGG ACC CGA GGA GAG CGT TGT GAC TTG  2330
```

```
Arg Leu Glu Ala Ala Cys Asn Trp Thr Arg Gly Glu Arg Cys Asp Leu
    640                 645                 650
GAG GAC AGG GAT AGA TCA GAG CTT AGC CCG CTA TTG CTG TCC ACA ACA  2378
Glu Asp Arg Asp Arg Ser Glu Leu Ser Pro Leu Leu Leu Ser Thr Thr
655                 660                 665                 670
GAG TGG CAG GTA CTG CCC TGT TCC TTC ACC ACC CTG CCG GCT CTG TCC  2426
Glu Trp Gln Val Leu Pro Cys Ser Phe Thr Thr Leu Pro Ala Leu Ser
                675                 680                 685
ACT GGT TTG ATT CAT CTC CAT CAG AAC ATC GTG GAC GTG CAA TAT CTG  2474
Thr Gly Leu Ile His Leu His Gln Asn Ile Val Asp Val Gln Tyr Leu
            690                 695                 700
TAC GGC ATA GGG TCG GCG GTT GTC TCC TTC GCA ATC AAA TGG GAA TAT  2522
Tyr Gly Ile Gly Ser Ala Val Val Ser Phe Ala Ile Lys Trp Glu Tyr
        705                 710                 715
ATT CTG TTG CTT TTC CTC CCC CTG GCG GAC GCG CGC GTC TGT GCC TGG  2570
Ile Leu Leu Leu Phe Leu Pro Leu Ala Asp Ala Arg Val Cys Ala Trp
    720                 725                 730
TTG TGG ATG ATG CTG CTG ATA GCC CAA GCT GAG GCC GCC TTG GAG AAC  2618
Leu Trp Met Met Leu Leu Ile Ala Gln Ala Glu Ala Ala Leu Glu Asn
735                 740                 745                 750
CTG GTG GTC CTC AAT GCA GCA TCC ATG GCG GGA GCG CAT GGC ATC CTC  2666
Leu Val Val Leu Asn Ala Ala Ser Met Ala Gly Ala His Gly Ile Leu
                755                 760                 765
TCT TTC CTT GTG TTC TTC TGT GCC GCC TGG TAC ATC AAA GGC AGG CTG  2714
Ser Phe Leu Val Phe Phe Cys Ala Ala Trp Tyr Ile Lys Gly Arg Leu
            770                 775                 780
GTC CCT GGG GCG GCA TAC GCT TTC TAT GGC GTA TGG CCG CTG CTC CTG  2762
Val Pro Gly Ala Ala Tyr Ala Phe Tyr Gly Val Trp Pro Leu Leu Leu
        785                 790                 795
CTC TTG ATG GCG CTA CCC GCA CGG GCG TAC GCC ATG GAC CGG GAG ATG  2810
Leu Leu Met Ala Leu Pro Ala Arg Ala Tyr Ala Met Asp Arg Glu Met
    800                 805                 810
GCT GCA TCG TGC GGA GGC GCG GTT TTT GTA GGT CTG GTA CTC TTG ACC  2858
Ala Ala Ser Cys Gly Gly Ala Val Phe Val Gly Leu Val Leu Leu Thr
815                 820                 825                 830
TTG TCA CCA TAC TAC AAA GTG TTC CTC GCT AAG CTC ATA TGG TGG TTG  2906
Leu Ser Pro Tyr Tyr Lys Val Phe Leu Ala Lys Leu Ile Trp Trp Leu
```

```
                    835                     840                     845
CAA TAT CTC ATC ACC AGG GCC GAG GCG CAC TTG CAA GTG TGG ATC CCC    2954
Gln Tyr Leu Ile Thr Arg Ala Glu Ala His Leu Gln Val Trp Ile Pro
                850                     855                     860
CCC CTC AAC GTT CGG GGG GGC CGC GAT GCC ATC ATC CTT CTC ACA TGT    3002
Pro Leu Asn Val Arg Gly Gly Arg Asp Ala Ile Ile Leu Leu Thr Cys
            865                     870                     875
GCG GTC CAC CCG GAG CTG ATC TTT GAC ATC ACC AAG CTC TTG CTC GCC    3050
Ala Val His Pro Glu Leu Ile Phe Asp Ile Thr Lys Leu Leu Leu Ala
        880                     885                     890
ATA CTC GGT CCG CTC ATG GTA CTC CAG GCT GGC CTA ACC CAA ATG CCG    3098
Ile Leu Gly Pro Leu Met Val Leu Gln Ala Gly Leu Thr Gln Met Pro
895                     900                     905                     910
TAC TTT GTG CGT GCT CAA GGG CTC ATT CGT ATG TGC ATG TTG GTG CGG    3146
Tyr Phe Val Arg Ala Gln Gly Leu Ile Arg Met Cys Met Leu Val Arg
                    915                     920                     925
AAA GCC GCT GGG GGT CAT TAT GTC CAG ATG GCT CTC ATG AAG CTG GCT    3194
Lys Ala Ala Gly Gly His Tyr Val Gln Met Ala Leu Met Lys Leu Ala
                930                     935                     940
GCA CTG ACA GGT ACG TAC GTT TAT GAC CAT CTT ACT CCA CTG CAG GAC    3242
Ala Leu Thr Gly Thr Tyr Val Tyr Asp His Leu Thr Pro Leu Gln Asp
            945                     950                     955
TGG GCC CAC GCG GGC CTA CGA GAC CTT GCG GTA GCA GTT GAG CCC GTT    3290
Trp Ala His Ala Gly Leu Arg Asp Leu Ala Val Ala Val Glu Pro Val
        960                     965                     970
GTC TTC TCT GAT ATG GAG ACT AAG ATC ATC ACG TGG GGG GCA GAG ACG    3338
Val Phe Ser Asp Met Glu Thr Lys Ile Ile Thr Trp Gly Ala Glu Thr
975                     980                     985                     990
GCG GCG TGT GGG GAC ATC ATC TCG AGT CTA CCC GTT TCC GCC CGA AGG    3386
Ala Ala Cys Gly Asp Ile Ile Ser Ser Leu Pro Val Ser Ala Arg Arg
                    995                    1000                    1005
GGG AGG GAG CTG CTT TTG GGA CCG GCC GAT AGT TTT GAC GGG CAG GGG    3434
Gly Arg Glu Leu Leu Leu Gly Pro Ala Asp Ser Phe Asp Gly Gln Gly
               1010                    1015                    1020
TGG CGA CTC CTT GCG CCT ATC ACG GCC TAC TCC CAG CAG ACG CGG GGC    3482
Trp Arg Leu Leu Ala Pro Ile Thr Ala Tyr Ser Gln Gln Thr Arg Gly
           1025                    1030                    1035
```

```
CTG CTT GGT TGC ATC ATC ACC AGC CTT ACG GGC CGG GAT AAG AAC CAG  3530
Leu Leu Gly Cys Ile Ile Thr Ser Leu Thr Gly Arg Asp Lys Asn Gln
    1040                1045                1050
GTC GAG GGG GAG GTT CAA GTG GTC TCT ACC GCA ACA CAA TCT TTC CTG  3578
Val Glu Gly Glu Val Gln Val Val Ser Thr Ala Thr Gln Ser Phe Leu
1055                1060                1065                1070
GCG ACC TGC ATC AAC GGC GTT TGC TGG ACT GTT TTC CAC GGC GCC GGC  3626
Ala Thr Cys Ile Asn Gly Val Cys Trp Thr Val Phe His Gly Ala Gly
                1075                1080                1085
TCG AAG ACC TTA GCC GGC CCA AAA GGC CCA ATC ACC CAA ATG TAC ACC  3674
Ser Lys Thr Leu Ala Gly Pro Lys Gly Pro Ile Thr Gln Met Tyr Thr
            1090                1095                1100
AAT GTA GAT CAG GAC CTC GTC GGC TGG TCG GCG CCC CCC GGG GCG CGT  3722
Asn Val Asp Gln Asp Leu Val Gly Trp Ser Ala Pro Pro Gly Ala Arg
        1105                1110                1115
TCC TTG ACA CCT TGC ACC TGC GGC AGC TCG GAC CTT TAT TTG GTC ACG  3770
Ser Leu Thr Pro Cys Thr Cys Gly Ser Ser Asp Leu Tyr Leu Val Thr
    1120                1125                1130
AGG CAT GCT GAT GTC ATT CCG GTG CAC CGG CGG GGC GAC AGC AGG GGG  3818
Arg His Ala Asp Val Ile Pro Val His Arg Arg Gly Asp Ser Arg Gly
1135                1140                1145                1150
AGC CTC CTC TCC CCC GGG CCC ATC TCT TAC TTG AAG GGT TCC TCG GGT  3866
Ser Leu Leu Ser Pro Gly Pro Ile Ser Tyr Leu Lys Gly Ser Ser Gly
                1155                1160                1165
GGT CCG CTG CCT TGC CCC TCG GGC CGT GTT GTG GGC ATC TTC CGG GCT  3914
Gly Pro Leu Pro Cys Pro Ser Gly Arg Val Val Gly Ile Phe Arg Ala
            1170                1175                1180
GCC GTG TGC ACC CGG GGG GTT GCG AAG GCG GTG GAC TTT GTG CCC GTT  3962
Ala Val Cys Thr Arg Gly Val Ala Lys Ala Val Asp Phe Val Pro Val
        1185                1190                1195
GAG TCT ATG GAA ACC ACC ATG CGG TCT CCG GTC TTC ACG GAT AAC TCA  4010
Glu Ser Met Glu Thr Thr Met Arg Ser Pro Val Phe Thr Asp Asn Ser
    1200                1205                1210
ACC CCC CCG GCC GTA CCG CAG ACA TTC CAA GTG GCC CAC CTA CAC GCT  4058
Thr Pro Pro Ala Val Pro Gln Thr Phe Gln Val Ala His Leu His Ala
1215                1220                1225                1230
CCC ACT GGC AGC GGC AAA AGC ACC AGG GTG CCG GCT GCG TAT GCG GCC  4106
```

```
Pro Thr Gly Ser Gly Lys Ser Thr Arg Val Pro Ala Ala Tyr Ala Ala
                1235                1240                1245
CAA GGG TAC AAG GTA CTC GTC CTG AAC CCG TCC GTT GCT GCC ACT TTG   4154
Gln Gly Tyr Lys Val Leu Val Leu Asn Pro Ser Val Ala Ala Thr Leu
            1250                1255                1260
GGC TTT GGG GCG TAC ATG TCC AAG GCA CAT GGT GTT GAC CCT AAC ATC   4202
Gly Phe Gly Ala Tyr Met Ser Lys Ala His Gly Val Asp Pro Asn Ile
        1265                1270                1275
AGA ACT GGG GTG AGG ACC ATC ACC ACG GGC GCT CCC ATC ACG TAC TCC   4250
Arg Thr Gly Val Arg Thr Ile Thr Thr Gly Ala Pro Ile Thr Tyr Ser
    1280                1285                1290
ACC TAC GGT AAG TTC CTC GCC GAC GGT GGC TGT TCT GGG GGT GCC TAT   4298
Thr Tyr Gly Lys Phe Leu Ala Asp Gly Gly Cys Ser Gly Gly Ala Tyr
1295                1300                1305                1310
GAC ATC ATA ATA TGT GAT GAG TGT CAT TCA ACT GAC TCG ACT TCC ATC   4346
Asp Ile Ile Ile Cys Asp Glu Cys His Ser Thr Asp Ser Thr Ser Ile
                1315                1320                1325
TTG GGC ATT GGT ACA GTC CTG GAC CAA GCG GAG ACG GCT GGA GCG CGC   4394
Leu Gly Ile Gly Thr Val Leu Asp Gln Ala Glu Thr Ala Gly Ala Arg
            1330                1335                1340
CTT GTC GTG CTC GCC ACC GCT ACG CCT CCG GGA TCG GTC ACC GTG CCG   4442
Leu Val Val Leu Ala Thr Ala Thr Pro Pro Gly Ser Val Thr Val Pro
        1345                1350                1355
CAT CCT AAT ATT GAG GAG GTG GCC TTG TCC AAC ACT GGA GAG ATC CCC   4490
His Pro Asn Ile Glu Glu Val Ala Leu Ser Asn Thr Gly Glu Ile Pro
    1360                1365                1370
TTC TAT GGC AAG GCC ATC CCC CTC GAG GCC ATC AAG GGG GGG AGG CAT   4538
Phe Tyr Gly Lys Ala Ile Pro Leu Glu Ala Ile Lys Gly Gly Arg His
1375                1380                1385                1390
CTC ATT TTC TGC CAT TCC AAG AAG AAA TGT GAC GAG CTC GCT GCG AAG   4586
Leu Ile Phe Cys His Ser Lys Lys Lys Cys Asp Glu Leu Ala Ala Lys
                1395                1400                1405
CTG TCG GCC CTC GGA GTC AAC GCT GTA GCA TAT TAC CGG GGT CTT GAT   4634
Leu Ser Ala Leu Gly Val Asn Ala Val Ala Tyr Tyr Arg Gly Leu Asp
            1410                1415                1420
GTG TCC ATC ATA CCG ACA AGC GGG GAC GTC GTT GTC GTG GCA ACA GAC   4682
Val Ser Ile Ile Pro Thr Ser Gly Asp Val Val Val Val Ala Thr Asp
```

```
        1425                1430                1435
GCT CTA ATG ACA GGC TAT ACC GGT GAC TTT GAC TCG GTG ATC GAC TGC  4730
Ala Leu Met Thr Gly Tyr Thr Gly Asp Phe Asp Ser Val Ile Asp Cys
    1440                1445                1450
AAC ACA TGT GTC ACC CAA ACA GTC GAT TTC AGC TTG GAC CCT ACT TTC  4778
Asn Thr Cys Val Thr Gln Thr Val Asp Phe Ser Leu Asp Pro Thr Phe
1455                1460                1465                1470
ACC ATC GAG ACG ACG ACC GTA CCC CAA GAT GCG GTG TCG CGC TCG CAG  4826
Thr Ile Glu Thr Thr Thr Val Pro Gln Asp Ala Val Ser Arg Ser Gln
                1475                1480                1485
CGG CGA GGC AGG ACT GGT AGG GGC AGG GGG GGC ATA TAC AGG TTT GTA  4874
Arg Arg Gly Arg Thr Gly Arg Gly Arg Gly Gly Ile Tyr Arg Phe Val
            1490                1495                1500
ACT CCA GGG GAA CGG CCC TCA GGC ATG TTC GAT TCT TCG GTC CTG TGT  4922
Thr Pro Gly Glu Arg Pro Ser Gly Met Phe Asp Ser Ser Val Leu Cys
        1505                1510                1515
GAA TGT TAT GAC GCG GGC TGT GCT TGG TAC GAG CTC ACG CCC GCC GAG  4970
Glu Cys Tyr Asp Ala Gly Cys Ala Trp Tyr Glu Leu Thr Pro Ala Glu
    1520                1525                1530
ACC TCG GTT AGG TTG CGG GCT TAC CTA AAT ACA CCT GGG CTG CCC GTC  5018
Thr Ser Val Arg Leu Arg Ala Tyr Leu Asn Thr Pro Gly Leu Pro Val
1535                1540                1545                1550
TGC CAG GAC CAT CTG GAG TTC TGG GAG AGC GTC TTC ACC GGC CTC ACC  5066
Cys Gln Asp His Leu Glu Phe Trp Glu Ser Val Phe Thr Gly Leu Thr
                1555                1560                1565
CAC ATA GAT GCC CAC TTC TTG TCC CAG ACC AAA CAG GCA GGA GAC AAC  5114
His Ile Asp Ala His Phe Leu Ser Gln Thr Lys Gln Ala Gly Asp Asn
            1570                1575                1580
TTC CCC TAC CTG GTA GCA TAC CAG GCT ACA GTG TGC GCC AGG GCC AAG  5162
Phe Pro Tyr Leu Val Ala Tyr Gln Ala Thr Val Cys Ala Arg Ala Lys
        1585                1590                1595
GCT CCA CCT CCA TCG TGG GAT CAG ATG TGG AAG TGT CTC ATA CGG CTG  5210
Ala Pro Pro Pro Ser Trp Asp Gln Met Trp Lys Cys Leu Ile Arg Leu
    1600                1605                1610
AAG CCT ACG CTA CAC GGG CCA ACG CCC CTG TTG TAT AGG TTA GGA GCC  5258
Lys Pro Thr Leu His Gly Pro Thr Pro Leu Leu Tyr Arg Leu Gly Ala
1615                1620                1625                1630
```

GTT CAG AAC GAG GTT ACC CTC ACA CAC CCC ATA ACC AAG TTC ATC ATG 5306
Val Gln Asn Glu Val Thr Leu Thr His Pro Ile Thr Lys Phe Ile Met
1635 1640 1645

GCA TGC ATG TCG GCT GAC CTA GAG GTC GTC ACT AGC ACT TGG GTG CTG 5354
Ala Cys Met Ser Ala Asp Leu Glu Val Val Thr Ser Thr Trp Val Leu
1650 1655 1660

GTA GGC GGG GTC CTC GCG GCT CTG GCC GCG TAC TGC CTG ACA ACG GGC 5402
Val Gly Gly Val Leu Ala Ala Leu Ala Ala Tyr Cys Leu Thr Thr Gly
1665 1670 1675

AGC GTG GTC ATT GTG GGC AGG ATC ATC TTG TCC GGG AGG CCG GCC GTT 5450
Ser Val Val Ile Val Gly Arg Ile Ile Leu Ser Gly Arg Pro Ala Val
1680 1685 1690

ATT CCC GAC AGG GAA GTT CTC TAC CAA GAG TTC GAT GAA ATG GAA GAG 5498
Ile Pro Asp Arg Glu Val Leu Tyr Gln Glu Phe Asp Glu Met Glu Glu
1695 1700 1705 1710

TGC GCC TCG CAC CTC CCT TAC ATC GAA CAA GGA ATG CAG CTC GCC GAG 5546
Cys Ala Ser His Leu Pro Tyr Ile Glu Gln Gly Met Gln Leu Ala Glu
1715 1720 1725

CAA TTC AAG CAG AAG GCG CTC GGT TTG CTG CAA ACA GCC ACC AAG CAA 5594
Gln Phe Lys Gln Lys Ala Leu Gly Leu Leu Gln Thr Ala Thr Lys Gln
1730 1735 1740

GCG GAG GCT GCT GCT CCC GTG GTG GAG TCC AAG TGG CGA GCC CTT GAG 5642
Ala Glu Ala Ala Ala Pro Val Val Glu Ser Lys Trp Arg Ala Leu Glu
1745 1750 1755

ACC TTC TGG GCG AAG CAC ATG TGG AAT TTT ATC AGC GGG ATA CAG TAC 5690
Thr Phe Trp Ala Lys His Met Trp Asn Phe Ile Ser Gly Ile Gln Tyr
1760 1765 1770

TTA GCA GGC TTG TCC ACT CTG CCT GGA AAC CCC GCA ATA GCA TCA CTG 5738
Leu Ala Gly Leu Ser Thr Leu Pro Gly Asn Pro Ala Ile Ala Ser Leu
1775 1780 1785 1790

ATG GCA TTC ACA GCC TCT ATC ACC AGC CCG CTC ACC ACC CAA TAT ACC 5786
Met Ala Phe Thr Ala Ser Ile Thr Ser Pro Leu Thr Thr Gln Tyr Thr
1795 1800 1805

CTC CTG TTT AAC ATC TTG GGG GGA TGG GTG GCC GCC CAA CTC GCC CCC 5834
Leu Leu Phe Asn Ile Leu Gly Gly Trp Val Ala Ala Gln Leu Ala Pro
1810 1815 1820

CCC AGT GCC GCT TCA GCC TTC GTG GGC GCC GGT ATA GCT GGC GCG GCT 5882

```
Pro Ser Ala Ala Ser Ala Phe Val Gly Ala Gly Ile Ala Gly Ala Ala
        1825                1830                1835
GTT GGC AGC ATA GGC CTC GGG AAG GTG CTT GTG GAC ATT CTG GCG GGT  5930
Val Gly Ser Ile Gly Leu Gly Lys Val Leu Val Asp Ile Leu Ala Gly
    1840                1845                1850
TAT GGA GCA GGG GTG GCA GGC GCG CTC GTG GCC TTT AAG GTC ATG AGC  5978
Tyr Gly Ala Gly Val Ala Gly Ala Leu Val Ala Phe Lys Val Met Ser
1855                1860                1865                1870
GGT GAC ATG CCC TCC ACC GAG GAC CTG GTC AAC TTA CTC CCC GCC ATC  6026
Gly Asp Met Pro Ser Thr Glu Asp Leu Val Asn Leu Leu Pro Ala Ile
                1875                1880                1885
CTC TCT CCT GGT GCC CTG GTC GTC GGG GTC GTG TGC GCA GCA ATA CTG  6074
Leu Ser Pro Gly Ala Leu Val Val Gly Val Val Cys Ala Ala Ile Leu
            1890                1895                1900
CGT CGG CAT GTG GGC CCA GGG GAG GGG GCT GTG CAG TGG ATG AAC CGG  6122
Arg Arg His Val Gly Pro Gly Glu Gly Ala Val Gln Trp Met Asn Arg
        1905                1910                1915
CTG ATA GCG TTT GCT TCG CGG GGC AAC CAT GTC TCC CCC ACG CAC TAT  6170
Leu Ile Ala Phe Ala Ser Arg Gly Asn His Val Ser Pro Thr His Tyr
    1920                1925                1930
GTG CCT GAA AGC GAC GCC GCA GCG CGC GTC ACC CAG ATC CTC TCC AAC  6218
Val Pro Glu Ser Asp Ala Ala Ala Arg Val Thr Gln Ile Leu Ser Asn
1935                1940                1945                1950
CTT ACC ATC ACT CAG CTG TTG AAG AGG CTT CAC CAG TGG ATT AAT GAG  6266
Leu Thr Ile Thr Gln Leu Leu Lys Arg Leu His Gln Trp Ile Asn Glu
                1955                1960                1965
GAC TGC TCC ACG CCA TGC TCC GGC TCG TGG CTC AGG GAT GTT TGG GAC  6314
Asp Cys Ser Thr Pro Cys Ser Gly Ser Trp Leu Arg Asp Val Trp Asp
            1970                1975                1980
TGG ATA TGC ACG GTA TTG GCT GAT TTC AAG ACC TGG CTC CAG TCC AAG  6362
Trp Ile Cys Thr Val Leu Ala Asp Phe Lys Thr Trp Leu Gln Ser Lys
        1985                1990                1995
CTC CTG CCG CGG TTA CCG GGG GTC CCT TTT TTC TCA TGC CAG CGT GGG  6410
Leu Leu Pro Arg Leu Pro Gly Val Pro Phe Phe Ser Cys Gln Arg Gly
    2000                2005                2010
TAC AAG GGG GTT TGG CGG GGA GAT GGC ATC ATG TAT ACC ACC TGC CCA  6458
Tyr Lys Gly Val Trp Arg Gly Asp Gly Ile Met Tyr Thr Thr Cys Pro
```

```
2015                2020                2025                2030
TGT GGA GCA CAA ATC ACC GGA CAT GTC AAA AAC GGT TCT ATG AGG ATC  6506
Cys Gly Ala Gln Ile Thr Gly His Val Lys Asn Gly Ser Met Arg Ile
                2035                2040                2045
GTT GGG CCT AGA ACC TGT AGC AAC ACG TGG CAC GGA ACA TTT CCC ATC  6554
Val Gly Pro Arg Thr Cys Ser Asn Thr Trp His Gly Thr Phe Pro Ile
            2050                2055                2060
AAC GCG TAC ACC ACA GGC CCC TGC ACA CCC TCC CCG GCG CCA AAC TAT  6602
Asn Ala Tyr Thr Thr Gly Pro Cys Thr Pro Ser Pro Ala Pro Asn Tyr
        2065                2070                2075
TCC AGG GCG TTG TGG CGG GTG GCC GCT GAG GAG TAT GTG GAG GTC ACG  6650
Ser Arg Ala Leu Trp Arg Val Ala Ala Glu Glu Tyr Val Glu Val Thr
    2080                2085                2090
CGG GTG GGG GAT TTC CAC TAC GTG ACG GGC ATG ACC ACT GAC AAC GTG  6698
Arg Val Gly Asp Phe His Tyr Val Thr Gly Met Thr Thr Asp Asn Val
2095                2100                2105                2110
AAA TGC CCA TGC CAG GTT CCG GCC CCC GAA TTC TTC ACA GAA TTG GAT  6746
Lys Cys Pro Cys Gln Val Pro Ala Pro Glu Phe Phe Thr Glu Leu Asp
                2115                2120                2125
GGG GTG CGG CTG CAC AGG TAC GCT CCG GCG TGC AAA CCT CTC CTG CGG  6794
Gly Val Arg Leu His Arg Tyr Ala Pro Ala Cys Lys Pro Leu Leu Arg
            2130                2135                2140
GAT GAG GTC ACA TTC CAG GTC GGG CTC AAC CAA TAT ACG GTT GGG TCA  6842
Asp Glu Val Thr Phe Gln Val Gly Leu Asn Gln Tyr Thr Val Gly Ser
        2145                2150                2155
CAG CTC CCA TGT GAG CCC GAA CCG GAT GTA ACA GTG GTC ACC TCC ATG  6890
Gln Leu Pro Cys Glu Pro Glu Pro Asp Val Thr Val Val Thr Ser Met
    2160                2165                2170
CTC ACC GAC CCC TCC CAC ATT ACA GCA GAG GCG GCT AGG CGT AGG CTG  6938
Leu Thr Asp Pro Ser His Ile Thr Ala Glu Ala Ala Arg Arg Arg Leu
2175                2180                2185                2190
GCC AGA GGG TCT CCC CCT TCC TTG GCC AGT TCT TCA GCT AGT CAG TTG  6986
Ala Arg Gly Ser Pro Pro Ser Leu Ala Ser Ser Ser Ala Ser Gln Leu
                2195                2200                2205
TCT GCG CTT TCT TTG TAG GCG ACA TGC ACT ACC CAT CAT GGC GCC CCA  7034
Ser Ala Leu Ser Leu StopAla Thr Cys Thr Thr His His Gly Ala Pro
            2210                2215                2220
```

```
GAC ACT GAC CTC ATC GAG GCC AAC CTC CTG TGG CGG CAG GAG ATG GGC  7082
Asp Thr Asp Leu Ile Glu Ala Asn Leu Leu Trp Arg Gln Glu Met Gly
        2225                2230                2235
GGA AAC ATC ACC CGC GTG GAG TCA GAG AAC AAG ATA GTA ATT CTA GAC  7130
Gly Asn Ile Thr Arg Val Glu Ser Glu Asn Lys Ile Val Ile Leu Asp
    2240                2245                2250
TCT TTT GAA CCG CTT CGA GCG GAG GAG GAT GAG AGG GAA GTG TCC GTT  7178
Ser Phe Glu Pro Leu Arg Ala Glu Glu Asp Glu Arg Glu Val Ser Val
2255                2260                2265                2270
GCG GCG GAG ATC CTG CGG AAG ACC AGG AAA TTC CCC GCA GCG ATG CCC  7226
Ala Ala Glu Ile Leu Arg Lys Thr Arg Lys Phe Pro Ala Ala Met Pro
                2275                2280                2285
GTA TGG GCA CGC CCG GAC TAC AAC CCA CCA TTA CTA GAG TCT TGG AAG  7274
Val Trp Ala Arg Pro Asp Tyr Asn Pro Pro Leu Leu Glu Ser Trp Lys
            2290                2295                2300
AAC CCG GAC TAC GTC CCT CCA GTG GTA CAC GGG TGC CCA TTG CCG CCT  7322
Asn Pro Asp Tyr Val Pro Pro Val Val His Gly Cys Pro Leu Pro Pro
        2305                2310                2315
ACC AAG GCC CCT CCA ATA CCA CCT CCA CGG AGA AAG AGA ACG GTT GTC  7370
Thr Lys Ala Pro Pro Ile Pro Pro Pro Arg Arg Lys Arg Thr Val Val
    2320                2325                2330
CTG ACA GAA TCC ACC GTG TCC TCT GCC TTG GCG GAG CTT GCT ACA AAG  7418
Leu Thr Glu Ser Thr Val Ser Ser Ala Leu Ala Glu Leu Ala Thr Lys
2335                2340                2345                2350
ACC TTT GGC AGT TCC GGA TCG TCG GCC GTC GAC AGC GGC ACG GCG ACC  7466
Thr Phe Gly Ser Ser Gly Ser Ser Ala Val Asp Ser Gly Thr Ala Thr
                2355                2360                2365
GGC CCT CCT GAC CAG GCC TCC GCC GAA GGA GAT GCA GGA TCC GAC GCT  7514
Gly Pro Pro Asp Gln Ala Ser Ala Glu Gly Asp Ala Gly Ser Asp Ala
            2370                2375                2380
GAG TCG TAC TCC TCC ATG CCC CCC CTT GAG GGA GAG CCG GGG GAC CCC  7562
Glu Ser Tyr Ser Ser Met Pro Pro Leu Glu Gly Glu Pro Gly Asp Pro
        2385                2390                2395
GAT CTC AAC GAC GGG TCT TGG TCT ACC GTA AGC GAG GAG GCC AGC GAG  7610
Asp Leu Asn Asp Gly Ser Trp Ser Thr Val Ser Glu Glu Ala Ser Glu
    2400                2405                2410
GAC GTC GTC TGC TGC TCG ATG TCC TAC ACA TGG ACA GGC GCC TTA ATT  7658
```

```
Asp Val Val Cys Cys Ser Met Ser Tyr Thr Trp Thr Gly Ala Leu Ile
2415                2420                2425                2430
ACA CCA TGC GCC GCG GAG GAG AGC AAG CTG CCC ATT AAT GCG CTG AGC  7706
Thr Pro Cys Ala Ala Glu Glu Ser Lys Leu Pro Ile Asn Ala Leu Ser
                2435                2440                2445
AAC CCT TTG CTG CGC CAC CAC AAC ATG GTC TAT GCC ACA ACA TCC CGC  7754
Asn Pro Leu Leu Arg His His Asn Met Val Tyr Ala Thr Thr Ser Arg
            2450                2455                2460
AGC GCA AGC CAG CGG CAG AAA AAG GTC ACA TTT GAC AGA CTG CAA GTC  7802
Ser Ala Ser Gln Arg Gln Lys Lys Val Thr Phe Asp Arg Leu Gln Val
        2465                2470                2475
CTG GAT GAC CAC TAC CGG GAC GTG CTG AAG GAC ATG AAG GCC AAG GCG  7850
Leu Asp Asp His Tyr Arg Asp Val Leu Lys Asp Met Lys Ala Lys Ala
    2480                2485                2490
TCC ACA GTT AAG GCT AAA CTT CTA TCT GTA GAG GAA GCC TGC AAG CTG  7898
Ser Thr Val Lys Ala Lys Leu Leu Ser Val Glu Glu Ala Cys Lys Leu
2495                2500                2505                2510
ACG CCC CCA CAC T  7911
```

SEQ ID NO:102
SEQUENCE LENGTH: 1123 base pairs
SEQUENCE TYPE: nucleic acid
STRANDEDNESS: double
TOPOLOGY: linear
ANTI-SENSE: No
ORIGINAL SOURCE
ORGANISM: Hepatitis C virus
IMMEDIATE EXPERIMENTAL SOURCE
CLONE: CN23

```
AAGCTT ATG AGC ACA AAT CCA AAA CCC CAA AGA AAA ACC AAA CGT AAC   48
       Met Ser Thr Asn Pro Lys Pro Gln Arg Lys Thr Lys Arg Asn
         1               5                  10
ACC AAC CGC CGC CCA CAG GAC GTC AAG TTC CCG GGC GGT GGT CAG ATC   96
Thr Asn Arg Arg Pro Gln Asp Val Lys Phe Pro Gly Gly Gly Gln Ile
 15                 20                  25                  30
GTT GGT GGA GTT TAC CTG TTG CCG CGC AGG GGC CCC AGG TTG GGT GTG  144
```

```
Val Gly Gly Val Tyr Leu Leu Pro Arg Arg Gly Pro Arg Leu Gly Val
                35                  40                  45
CGC GCG ACT AGG AAG ACT TCC GAG CGG TCG CAA CCT CGT GGA AGG CGA    192
Arg Ala Thr Arg Lys Thr Ser Glu Arg Ser Gln Pro Arg Gly Arg Arg
            50                  55                  60
CAA CCT ATC CCC AAG GCT CGC CAA CCC GAG GGC AGG GCC TGG GCT CAG    240
Gln Pro Ile Pro Lys Ala Arg Gln Pro Glu Gly Arg Ala Trp Ala Gln
        65                  70                  75
CCC GGG TAC CCT TGG CCC CTC TAT GGC AAT GAG GGC TTG GGG TGG GCA    288
Pro Gly Tyr Pro Trp Pro Leu Tyr Gly Asn Glu Gly Leu Gly Trp Ala
    80                  85                  90
GGA TGG CTC CTG TCA CCC CGG GGG GTT GCG AAG GCG GTG GAC TTT GTG    336
Gly Trp Leu Leu Ser Pro Arg Gly Val Ala Lys Ala Val Asp Phe Val
 95                 100                 105                 110
CCC GTT GAG TCT ATG GAA ACC ACC ATG CGG TCT CCG GTC TTC ACG GAT    384
Pro Val Glu Ser Met Glu Thr Thr Met Arg Ser Pro Val Phe Thr Asp
                115                 120                 125
AAC TCA ACC CCC CCG GCC GTA CCG CAG ACA TTC CAA GTG GCC CAC CTA    432
Asn Ser Thr Pro Pro Ala Val Pro Gln Thr Phe Gln Val Ala His Leu
            130                 135                 140
CAC GCT CCC ACT GGC AGC GGC AAA AGC ACC AGG GTG CCG GCT GCG TAT    480
His Ala Pro Thr Gly Ser Gly Lys Ser Thr Arg Val Pro Ala Ala Tyr
        145                 150                 155
GCG GCC CAA GGG TAC AAG GTA CTC GTC CTG AAC CCG TCC GTT GCT GCC    528
Ala Ala Gln Gly Tyr Lys Val Leu Val Leu Asn Pro Ser Val Ala Ala
    160                 165                 170
ACT TTG GGC TTT GGG GCG TAC ATG TCC AAG GCA CAT GGT GTT GAC CCT    576
Thr Leu Gly Phe Gly Ala Tyr Met Ser Lys Ala His Gly Val Asp Pro
175                 180                 185                 190
AAC ATC AGA ACT GGG GTG AGG ACC ATC ACC ACG GGC GCT CCC ATC ACG    624
Asn Ile Arg Thr Gly Val Arg Thr Ile Thr Thr Gly Ala Pro Ile Thr
                195                 200                 205
TAC TCC ACC TAC GGT AAG TTC CTC GCC GAC GGT GGC TGT TCT GGG GGT    672
Tyr Ser Thr Tyr Gly Lys Phe Leu Ala Asp Gly Gly Cys Ser Gly Gly
            210                 215                 220
GCC TAT GAC ATC ATA ATA TGT GAT GAG TGT CAT TCA ACT GAC TCG ACT    720
Ala Tyr Asp Ile Ile Ile Cys Asp Glu Cys His Ser Thr Asp Ser Thr
```

```
        225                 230                 235
TCC ATC TTG GGC ATT GGT ACA GTC CTG GAC CAA GCG GAG ACG GCT GGA    768
Ser Ile Leu Gly Ile Gly Thr Val Leu Asp Gln Ala Glu Thr Ala Gly
    240                 245                 250
GCG CGC CTT GTC GTG CTC GCC ACC GCT ACG CCT CCG GGA TCG GTC ACC    816
Ala Arg Leu Val Val Leu Ala Thr Ala Thr Pro Pro Gly Ser Val Thr
255                 260                 265                 270
GTG CCG CAT CCT AAT ATT GAG GAG GTG GCC TTG TCC AAC ACT GGA GAG    864
Val Pro His Pro Asn Ile Glu Glu Val Ala Leu Ser Asn Thr Gly Glu
                275                 280                 285
ATC CCC TTC TAT GGC AAG GCC ATC CCC CTC GAG GCC ATC AAG GGG GGG    912
Ile Pro Phe Tyr Gly Lys Ala Ile Pro Leu Glu Ala Ile Lys Gly Gly
            290                 295                 300
AGG CAT CTC ATT TTC TGC CAT TCC AAG AAG AAA TGT GAC GAG CTC GCT    960
Arg His Leu Ile Phe Cys His Ser Lys Lys Lys Cys Asp Glu Leu Ala
        305                 310                 315
GCG AAG CTG TCG GCC CTC GGA GTC AAC GCT GTA GCA TAT TAC CGG GGT   1008
Ala Lys Leu Ser Ala Leu Gly Val Asn Ala Val Ala Tyr Tyr Arg Gly
    320                 325                 330
CTT GAT GTG TCC ATC ATA CCG ACA AGC GGG GAC GTC GTT GTC GTG GCA   1056
Leu Asp Val Ser Ile Ile Pro Thr Ser Gly Asp Val Val Val Val Ala
335                 340                 345                 350
ACA GAC GCT CTA ATG ACG GGC TAT ACC GGT GAC TTT GAC TCG GTG ATC   1104
Thr Asp Ala Leu Met Thr Gly Tyr Thr Gly Asp Phe Asp Ser Val Ile
                355                 360                 365
GAC TGC AAC ACA TGA TAA AGATCT                                    1128
Asp Cys Asn Thr StopStop
            370
```

SEQ ID NO:103
SEQUENCE LENGTH: 974 base pairs
SEQUENCE TYPE: nucleic acid
STRANDEDNESS: double
TOPOLOGY: linear
ANTI-SENSE: No
ORGANISM: Hepatitis C virus
IMMEDIATE EXPERIMENTAL SOURCE

CLONE: O15-1

```
GCGGATCCT CCA CCT CCA TCG TGG GAC CAA ATG TGG AAG TGT CTC ATA CGG 51

          Pro Pro Pro Ser Trp Asp Gln Met Trp Lys Cys Leu Ile Arg
            1               5                10
CTG AAA CCT ACG CTA CAC GGG CCA ACA CCC CTG TTG TAT AGG TTA GGA  99
Leu Lys Pro Thr Leu His Gly Pro Thr Pro Leu Leu Tyr Arg Leu Gly
 15                  20                  25                  30
GCC GTT CAA AAC GAG GTC ACC CTC ACA CAC CCC ATA ACC AAA TTC ATC 147
Ala Val Gln Asn Glu Val Thr Leu Thr His Pro Ile Thr Lys Phe Ile
                 35                  40                  45
ATG GCA TGC ATG TCG GCT GAC CTA GAG GTC GTC ACT AGC ACC TGG GTG 195
Met Ala Cys Met Ser Ala Asp Leu Glu Val Val Thr Ser Thr Trp Val
             50                  55                  60
CTG GTA GGC GGG GTC CTC GCA GCT CTG GCC GCG TAC TGC CTG ACA ACG 243
Leu Val Gly Gly Val Leu Ala Ala Leu Ala Ala Tyr Cys Leu Thr Thr
         65                  70                  75
GGC AGC GTG GTC ATC GTG GGC AGG ATC ATC TTG TCC GGG AGG CCG GCT 291
Gly Ser Val Val Ile Val Gly Arg Ile Ile Leu Ser Gly Arg Pro Ala
     80                  85                  90
ATC ATT CCC GAC AGG GAA GTT CTC TAC CGT GAG TTC GAT GAA ATG GAG 339
Ile Ile Pro Asp Arg Glu Val Leu Tyr Arg Glu Phe Asp Glu Met Glu
 95                 100                 105                 110
GAG TGC GCC TCA CAC CTC CCC TAC ATC GAA CAG GGA ATG CAG CTC GCC 387
Glu Cys Ala Ser His Leu Pro Tyr Ile Glu Gln Gly Met Gln Leu Ala
                115                 120                 125
GAG CAG TTC AAG CAG AAG GCG CTC GGT TTG TTG CAA ACA GCT ACC CAG 435
Glu Gln Phe Lys Gln Lys Ala Leu Gly Leu Leu Gln Thr Ala Thr Gln
            130                 135                 140
CAA GCG GAG GCT GCT GCT CCC GTG GTG GAG TCC AAA TGG CGA GCC CTA 483
Gln Ala Glu Ala Ala Ala Pro Val Val Glu Ser Lys Trp Arg Ala Leu
        145                 150                 155
GAG GCC TTC TGG GCA AAG CAC ATG TGG AAT TTC ATC AGC GGG ATA CAG 531
Glu Ala Phe Trp Ala Lys His Met Trp Asn Phe Ile Ser Gly Ile Gln
    160                 165                 170
TAC TTG GCA GGC TTG TCC ACT CTG CCT GGA AAC CCC GCG ATA GCA TCA 579
```

```
Tyr Leu Ala Gly Leu Ser Thr Leu Pro Gly Asn Pro Ala Ile Ala Ser
175                 180                 185                 190
CTG ATG GCA TTC ACA GCC TCT ATC ACC AGC CCT CTC ACC ACC CAA CAT 627
Leu Met Ala Phe Thr Ala Ser Ile Thr Ser Pro Leu Thr Thr Gln His
                195                 200                 205
ACC CTC CTG TTT AAC ATC TTG GGG GGA TGG GTA GCC GCC CAA CTC GCC 675
Thr Leu Leu Phe Asn Ile Leu Gly Gly Trp Val Ala Ala Gln Leu Ala
            210                 215                 220
CCT CCC AGC GCT GCT TCA GCT TTT GTG GGC GCC GGC ATA GCT GGC GCG 723
Pro Pro Ser Ala Ala Ser Ala Phe Val Gly Ala Gly Ile Ala Gly Ala
        225                 230                 235
GCT GTT GGC AGC ATA GGC CTT GGG AAG GTG CTT GTG GAC ATC CTG GCG 771
Ala Val Gly Ser Ile Gly Leu Gly Lys Val Leu Val Asp Ile Leu Ala
    240                 245                 250
GGT TAT GGA GCA GGG GTG GCA GGC GCA CTC GTG GCC TTT AAG GTC ATG 819
Gly Tyr Gly Ala Gly Val Ala Gly Ala Leu Val Ala Phe Lys Val Met
255                 260                 265                 270
AGT GGC GAG ATG CCC TCC ACC GAG GAC TTG GTC AAC CTA CTC CCT GCC 867
Ser Gly Glu Met Pro Ser Thr Glu Asp Leu Val Asn Leu Leu Pro Ala
                275                 280                 285
ATC CTC TCT CCT GGC GCC CTG GTC GTC GGA GTC GTG TGC GCA GCA ATA 915
Ile Leu Ser Pro Gly Ala Leu Val Val Gly Val Val Cys Ala Ala Ile
            290                 295                 300
CTG CGT CGA CAT GTG GGC CCA GGG GAG GGG GCT GTG CAG TGG ATG AAC 963
Leu Arg Arg His Val Gly Pro Gly Glu Gly Ala Val Gln Trp Met Asn
        305                 310                 315
CGG CTG C AGCC                                                  974
Arg Leu
    320
```

SEQ ID NO:104
SEQUENCE LENGTH: 974 base pairs
SEQUENCE TYPE: nucleic acid
STRANDEDNESS: double
TOPOLOGY: linear
ANTI-SENSE: No
ORGANISM: Hepatitis C virus

IMMEDIATE EXPERIMENTAL SOURCE
CLONE: 015-2

```
GCGGATCCT CCA CCT CCA TCG TGG GAC CAA ATG TGG AAG TGT CTC ATA CGG 51

          Pro Pro Pro Ser Trp Asp Gln Met Trp Lys Cys Leu Ile Arg
            1               5                  10
CTA AAA CCT ACG CTA CAC GGG CCA ACA CCC CTG TTG TAT AGG TTA GGA  99
Leu Lys Pro Thr Leu His Gly Pro Thr Pro Leu Leu Tyr Arg Leu Gly
 15                  20                  25                  30
GCC GTT CAA AAC GAG GTC ACC CTC ACA CAC CCC ATA ACC AAG TTC ATC 147
Ala Val Gln Asn Glu Val Thr Leu Thr His Pro Ile Thr Lys Phe Ile
                 35                  40                  45
ATG GCA TGC ATG TCG GCT GAC CTA GAG GTC GTC ACT AGC ACC TGG GTG 195
Met Ala Cys Met Ser Ala Asp Leu Glu Val Val Thr Ser Thr Trp Val
             50                  55                  60
CTG GTA GGC GGG GTC CTC GCA GCT CTG GCC GCG TAC TGC CTG ACA ACG 243
Leu Val Gly Gly Val Leu Ala Ala Leu Ala Ala Tyr Cys Leu Thr Thr
         65                  70                  75
GGC AGC GTG GTC ATC GTG GGC AGA ATC ATC TTG TCC GGG AGG CCG GCT 291
Gly Ser Val Val Ile Val Gly Arg Ile Ile Leu Ser Gly Arg Pro Ala
     80                  85                  90
ATC ATT CCC GAC AGG GAG GTT CTC TAC CGG GAG TTC GAT GAA ATG GAG 339
Ile Ile Pro Asp Arg Glu Val Leu Tyr Arg Glu Phe Asp Glu Met Glu
 95                 100                 105                 110
GAG TGC GCC TCA CAC CTC CCC TAC ATC GAA CAG GGA ATG CAG CTC GCC 387
Glu Cys Ala Ser His Leu Pro Tyr Ile Glu Gln Gly Met Gln Leu Ala
                115                 120                 125
GAG CAA TTC AAG CAG AAG GCG CTC GGT TTG TTG CAA ACA GCT ACC AAG 435
Glu Gln Phe Lys Gln Lys Ala Leu Gly Leu Leu Gln Thr Ala Thr Lys
            130                 135                 140
CAA GCG GAG GCT GCT GCT CCC GTG GTG GAG TCC AAA TGG CGA GCC CTT 483
Gln Ala Glu Ala Ala Ala Pro Val Val Glu Ser Lys Trp Arg Ala Leu
        145                 150                 155
GAG ACC TTC TGG GCA AAG CAC ATG TGG AAT TTC ATC AGC GGG ATA CAG 531
Glu Thr Phe Trp Ala Lys His Met Trp Asn Phe Ile Ser Gly Ile Gln
    160                 165                 170
```

```
TAC TTG GCA GGC TTG TCC ACT CTG CCT GGA AAC CCC GCG ATA GCA TCA 579
Tyr Leu Ala Gly Leu Ser Thr Leu Pro Gly Asn Pro Ala Ile Ala Ser
175                 180                 185                 190
CTG ATG GCA TTC ACA GCC TCT ATC ACC AGC CCT CTC ACC ACC CAA CAT 627
Leu Met Ala Phe Thr Ala Ser Ile Thr Ser Pro Leu Thr Thr Gln His
                195                 200                 205
ACC CTC CTG TTT AAC ATC TTT GGG GGA TGG GTG GCC GCC CAA CTC GCC 675
Thr Leu Leu Phe Asn Ile Phe Gly Gly Trp Val Ala Ala Gln Leu Ala
            210                 215                 220
CCT CCC AGC GCT GCT TCA GCT TTT GTG GGC GCC GGC ATA GCT GGC GCG 723
Pro Pro Ser Ala Ala Ser Ala Phe Val Gly Ala Gly Ile Ala Gly Ala
        225                 230                 235
GCT GTT GGC AGC ATA GGC CTT GGG AAG GTG CTT GTG GAC ATC CTG GCG 771
Ala Val Gly Ser Ile Gly Leu Gly Lys Val Leu Val Asp Ile Leu Ala
    240                 245                 250
GGT TAT GGA GCA GGG GTG GCA GGC GCA CTC GTG GCC TTT AAG GTC ATG 819
Gly Tyr Gly Ala Gly Val Ala Gly Ala Leu Val Ala Phe Lys Val Met
255                 260                 265                 270
AGT GGC GAG ATG CCC TCC ACC GAG GAC TTG GTC AAC TTA CTC CCT GCC 867
Ser Gly Glu Met Pro Ser Thr Glu Asp Leu Val Asn Leu Leu Pro Ala
                275                 280                 285
ATC CTC TCT CCT GGC GCC CTG GTC GTC GGA GTC GTG TGC GCA GCA ATA 915
Ile Leu Ser Pro Gly Ala Leu Val Val Gly Val Val Cys Ala Ala Ile
            290                 295                 300
CTG CGT CGA CAT GTG GGC CCA GGG GAG GGG GCT GTG CAG TGG ATG AAC 963
Leu Arg Arg His Val Gly Pro Gly Glu Gly Ala Val Gln Trp Met Asn
        305                 310                 315
CGG CTG C AGCC                                                  974
Arg Leu
    320
```

SEQ ID NO: 105
SEQUENCE LENGTH: 19 base pairs
SEQUENCE TYPE: nucleic acid
TOPOLOGY: linear
MOLECULE TYPE: cDNA
ORIGINAL SOURCE

ORGANISM: Hepatitis C virus

CTCCACCATAGATCACTCC 19

SEQ ID NO: 106
SEQUENCE LENGTH: 18 base pairs
SEQUENCE TYPE: nucleic acid
TOPOLOGY: linear
MOLECULE TYPE: cDNA
ORIGINAL SOURCE
ORGANISM: Hepatitis C virus

AGGTCTAGTAGACCGTGC 18

SEQ ID NO: 107
SEQUENCE LENGTH: 18 base pairs
SEQUENCE TYPE: nucleic acid
TOPOLOGY: linear
MOLECULE TYPE: cDNA
ORIGINAL SOURCE
ORGANISM: Hepatitis C virus

AGGAAGACTTCCGAGCGG 18

SEQ ID NO: 108
SEQUENCE LENGTH: 19 base pairs
SEQUENCE TYPE: nucleic acid
TOPOLOGY: linear
MOLECULE TYPE: cDNA
ORIGINAL SOURCE
ORGANISM: Hepatitis C virus

CGTGAACTATGCAACAGGG 19

SEQ ID NO: 109
SEQUENCE LENGTH: 18 base pairs
SEQUENCE TYPE: nucleic acid

TOPOLOGY: linear
MOLECULE TYPE: cDNA
ORIGINAL SOURCE
ORGANISM: Hepatitis C virus

ACCGCTCGGAAGTCTTCC 18

SEQ ID NO: 110
SEQUENCE LENGTH: 18 base pairs
SEQUENCE TYPE: nucleic acid
TOPOLOGY: linear
MOLECULE TYPE: cDNA
ORIGINAL SOURCE
ORGANISM: Hepatitis C virus

GGGCAAGTTCCCTGTTGC 18

SEQ ID NO: 111
SEQUENCE LENGTH: 18 base pairs
SEQUENCE TYPE: nucleic acid
TOPOLOGY: linear
MOLECULE TYPE: cDNA
ORIGINAL SOURCE
ORGANISM: Hepatitis C virus

GCTGGATTCTCTGAGACG 18

SEQ ID NO: 112
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

GAGGCCGTGAACTGCGATGA 23

SEQ ID NO: 113
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

TTCTCTAAGGTGGCNTCNGCNTG 23
N: inosine

SEQ ID NO: 114
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

CCGGACGCGTTGAANCTNGNGT 21
N: inosine

SEQ ID NO: 115
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

CATCCAGGTACAACCGAACCA 23

SEQ ID NO: 116
SEQUENCE LENGTH: 24 base pairs
SEQUENCE TYPE: nucleic acid
TOPOLOGY: linear
MOLECULE TYPE: cDNA

ORIGINAL SOURCE
ORGANISM: Hepatitis C virus

AACACACGGCCGCCNCANGGNAA 24
N: inosine

SEQ ID NO: 117
SEQUENCE LENGTH: 19 base pairs
SEQUENCE TYPE: nucleic acid
TOPOLOGY: linear
MOLECULE TYPE: cDNA
ORIGINAL SOURCE
ORGANISM: Hepatitis C virus

CCGGATCCCACAAGCCGTNGTNGA 19
N: inosine

SEQ ID NO: 118
SEQUENCE LENGTH: 20 base pairs
SEQUENCE TYPE: nucleic acid
TOPOLOGY: linear
MOLECULE TYPE: cDNA
ORIGINAL SOURCE
ORGANISM: Hepatitis C virus

GACATGCATGTCATGATGTA 20

SEQ ID NO: 119
SEQUENCE LENGTH: 26 base pairs
SEQUENCE TYPE: nucleic acid
TOPOLOGY: linear
MOLECULE TYPE: cDNA
ORIGINAL SOURCE
ORGANISM: Hepatitis C virus

GGCTGCAGCCGGTTCATCCACTGCAC 26

SEQ ID NO: 120
SEQUENCE LENGTH: 26 base pairs
SEQUENCE TYPE: nucleic acid
TOPOLOGY: linear
MOLECULE TYPE: cDNA
ORIGINAL SOURCE
ORGANISM: Hepatitis C virus

GCGGATCCTGCTTCGCCCAGAAGGTC 26

SEQ ID NO: 121
SEQUENCE LENGTH: 22 base pairs
SEQUENCE TYPE: nucleic acid
TOPOLOGY: linear
MOLECULE TYPE: cDNA
ORIGINAL SOURCE
ORGANISM: Hepatitis C virus

GACACATGTGTTGCAGTCGATC 22

SEQ ID NO: 122
SEQUENCE LENGTH: 24 base pairs
SEQUENCE TYPE: nucleic acid
TOPOLOGY: linear
MOLECULE TYPE: cDNA
ORIGINAL SOURCE
ORGANISM: Hepatitis C virus

CGGTCCNAGNAGTATCTCNTTNCC 24
N: inosine

SEQ ID NO: 123
SEQUENCE LENGTH: 35 base pairs
SEQUENCE TYPE: nucleic acid
TOPOLOGY: linear
MOLECULE TYPE: cDNA
ORIGINAL SOURCE

ORGANISM: Hepatitis C virus

ATGGGCCCGGGNGANAGNAGNCTCCCCCTNCTNTC 35
N: inosine

SEQ ID NO: 124
SEQUENCE LENGTH: 20 base pairs
SEQUENCE TYPE: nucleic acid
TOPOLOGY: linear
MOLECULE TYPE: cDNA
ORIGINAL SOURCE
ORGANISM: Hepatitis C virus

GGCTATACCGGCGACTTCGA 20

SEQ ID NO: 125
SEQUENCE LENGTH: 27 base pairs
SEQUENCE TYPE: nucleic acid
TOPOLOGY: linear
MOLECULE TYPE: cDNA
ORIGINAL SOURCE
ORGANISM: Hepatitis C virus

GCGGATCCGGCCTCACCCACATAGATG 27

SEQ ID NO: 126
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

GCGGATCCTCCACCTCCATCGTG 23

SEQ ID NO: 127
SEQUENCE LENGTH: 20 base pairs

SEQUENCE TYPE: nucleic acid
TOPOLOGY: linear
MOLECULE TYPE: cDNA
ORIGINAL SOURCE
ORGANISM: Hepatitis C virus

CTGCTGTCGCCCNGNCCCAT 20
N: inosine

SEQ ID NO: 128
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

ATCACGTGGGGNGCAGANACNGC 23
N: inosine

SEQ ID NO: 129
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

TGTGCCTGNTTNTGGATGATG 21
N: inosine

SEQ ID NO: 130
SEQUENCE LENGTH: 21 base pairs
SEQUENCE TYPE: nucleic acid
TOPOLOGY: linear
MOLECULE TYPE: cDNA
ORIGINAL SOURCE

ORGANISM: Hepatitis C virus

GGTGAGCATGGAGGTGACCAC 21

SEQ ID NO: 131
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

TCATCCTCCTCCGCTCGAAGC 21

SEQ ID NO: 132
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

GTGGACGCCTTNGCCTTCATNTC 23
N: inosine

SEQ ID NO: 133
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

ACGGATGTCNTTCTCNGTNAC 21
N: inosine

SEQ ID NO: 134

SEQUENCE LENGTH: 30 base pairs
SEQUENCE TYPE: nucleic acid
TOPOLOGY: linear
MOLECULE TYPE: cDNA
ORIGINAL SOURCE
ORGANISM: Hepatitis C virus

GGCGGAATTCCTGGTCATAGCCTCCGTGAA 30

SEQ ID NO: 135
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

GGGGNATGGCCTATTGGCCTG 21
N: inosine

SEQ ID NO: 136
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

GGCATGTGGGCCCAGGGGAGG 21

SEQ ID NO: 137
SEQUENCE LENGTH: 20 base pairs
SEQUENCE TYPE: nucleic acid
TOPOLOGY: linear
MOLECULE TYPE: cDNA
ORIGINAL SOURCE
ORGANISM: Hepatitis C virus

TGTGAGCCCGAACCGGATGT 20

SEQ ID NO: 138
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

GTGGTANTCCTGGACTCNTTNGA 23
N: inosine

SEQ ID NO: 139
SEQUENCE LENGTH: 22 base pairs
SEQUENCE TYPE: nucleic acid
TOPOLOGY: linear
MOLECULE TYPE: cDNA
ORIGINAL SOURCE
ORGANISM: Hepatitis C virus

ACTACCGNGACGTGCTNAANGA 22
N: inosine

SEQ ID NO: 140
SEQUENCE LENGTH: 30 base pairs
SEQUENCE TYPE: nucleic acid
TOPOLOGY: linear
MOLECULE TYPE: cDNA
ORIGINAL SOURCE
ORGANISM: Hepatitis C virus

TGGGGATCCCGTATGATACCCGCTGCTTTG 30

SEQ ID NO: 141
SEQUENCE LENGTH: 24 base pairs
SEQUENCE TYPE: nucleic acid

TOPOLOGY: linear
MOLECULE TYPE: cDNA
ORIGINAL SOURCE
ORGANISM: Hepatitis C virus

ATTGTCAGATCTACGGGGCCACTT 24

SEQ ID NO: 142
SEQUENCE LENGTH: 43 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTAAAAAAAAAAAAGGGGGATGGCCTATTGGCCTGGA 43

SEQ ID NO: 143
SEQUENCE LENGTH: 17 base pairs
SEQUENCE TYPE: nucleic acid
TOPOLOGY: linear
MOLECULE TYPE: cDNA
ORIGINAL SOURCE
ORGANISM: Hepatitis C virus

GTAAAACGACGGCCAGT 17

SEQ ID NO: 144
SEQUENCE LENGTH: 17 base pairs
SEQUENCE TYPE: nucleic acid
TOPOLOGY: linear
MOLECULE TYPE: cDNA
ORIGINAL SOURCE
ORGANISM: Hepatitis C virus

CAGGAAACAGCTATGAC 17

SEQ ID NO: 145
SEQUENCE LENGTH: 35 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGAGCACAAATCCAAAACCCCAAAGA 35

SEQ ID NO: 146
SEQUENCE LENGTH: 38 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCACCTACGCCGGGGGTCCGTGGG 38

SEQ ID NO: 147
SEQUENCE LENGTH: 39 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCAGATTCTCTGAGACGGCCCTCGT 39

SEQ ID NO: 148
SEQUENCE LENGTH: 17 base pairs
SEQUENCE TYPE: nucleic acid
TOPOLOGY: linear
MOLECULE TYPE: cDNA
ORIGINAL SOURCE
ORGANISM: Hepatitis C virus

GCTACTCCGGATACCAC 17

SEQ ID NO: 149
SEQUENCE LENGTH: 35 base pairs
SEQUENCE TYPE: nucleic acid
TOPOLOGY: linear
MOLECULE TYPE: cDNA
ORIGINAL SOURCE
ORGANISM: Hepatitis C virus

GCGTCGACGCTAGCATGAGCACAAATCCAAAACCC 35

SEQ ID NO: 150
SEQUENCE LENGTH: 35 base pairs
SEQUENCE TYPE: nucleic acid
TOPOLOGY: linear
MOLECULE TYPE: cDNA
ORIGINAL SOURCE
ORGANISM: Hepatitis C virus

GCGTCGACGCTAGCAGGTCTCGTAGACCGTGCATC 35

SEQ ID NO: 151
SEQUENCE LENGTH: 40 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCGCTAGCTCAGGATTCTCTGAGACGGCCCTCGA 40

SEQ ID NO: 152
SEQUENCE LENGTH: 35 base pairs
SEQUENCE TYPE: nucleic acid
TOPOLOGY: linear
MOLECULE TYPE: cDNA

ORIGINAL SOURCE
ORGANISM: Hepatitis C virus

GCAAGCTTATGCGGATCCCACAAGCCGTGGTGGAT 35

SEQ ID NO: 153
SEQUENCE LENGTH: 24 base pairs
SEQUENCE TYPE: nucleic acid
TOPOLOGY: linear
MOLECULE TYPE: cDNA
ORIGINAL SOURCE
ORGANISM: Hepatitis C virus

CGGATCCCACAAGCCGTGGTGGAT 24

SEQ ID NO: 154
SEQUENCE LENGTH: 43 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCATCACTCTAAGGTGGCGTCGGCGTGGG 43

SEQ ID NO: 155
SEQUENCE LENGTH: 11 base pairs
SEQUENCE TYPE: nucleic acid
STRANDEDNESS: double
TOPOLOGY: linear
MOLECULE TYPE: cDNA
ORIGINAL SOURCE
ORGANISM: Hepatitis C virus

GCAAGCTTATG 11

SEQ ID NO: 156

SEQUENCE LENGTH: 20 base pairs
SEQUENCE TYPE: nucleic acid
STRANDEDNESS: double
TOPOLOGY: linear
MOLECULE TYPE: cDNA
ORIGINAL SOURCE
ORGANISM: Hepatitis C virus

TGATGAAGATCTGAATTCGC 20

SEQ ID NO: 157
SEQUENCE LENGTH: 34 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGTTCAACGCGTCCGGATGTCCGGA 34

SEQ ID NO: 158
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

TTCAACGCGTCCGGATGTCCGGA 23

SEQ ID NO: 159
SEQUENCE LENGTH: 43 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCATCAACAACCGAACCAGTTGCCCTGCG 43

SEQ ID NO: 160
SEQUENCE LENGTH: 34 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGATCGGGGGGGTCGGCAACAATAC 34

SEQ ID NO: 161
SEQUENCE LENGTH: 23 base pairs
SEQUENCE TYPE: nucleic acid
TOPOLOGY: linear
MOLECULE TYPE: cDNA
ORIGINAL SOURCE
ORGANISM: Hepatitis C virus

ATCGGGGGGGTCGGCAACAATAC 23

SEQ ID NO: 162
SEQUENCE LENGTH: 43 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCATCAAAGCTCTGATCTATCCCTGTCCT 43

SEQ ID NO: 163
SEQUENCE LENGTH: 41 base pairs
SEQUENCE TYPE: nucleic acid
TOPOLOGY: linear
MOLECULE TYPE: cDNA

ORIGINAL SOURCE
ORGANISM: Hepatitis C virus

GCGTCGACGCTAGCATGCGGATCCCACAAGCCGTGGTGGAT 41

SEQ ID NO: 164
SEQUENCE LENGTH: 40 base pairs
SEQUENCE TYPE: nucleic acid
TOPOLOGY: linear
MOLECULE TYPE: cDNA
ORIGINAL SOURCE
ORGANISM: Hepatitis C virus

GCGTCGACGCTAGCATGTTCAACGCGTCCGGATGTCCGGA 40

SEQ ID NO: 165
SEQUENCE LENGTH: 40 base pairs
SEQUENCE TYPE: nucleic acid
TOPOLOGY: linear
MOLECULE TYPE: cDNA
ORIGINAL SOURCE
ORGANISM: Hepatitis C virus

GCGTCGACGCTAGCATGATCGGGGGGTCGGCAACAATAC 40

SEQ ID NO: 166
SEQUENCE LENGTH: 40 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCGCTAGCTCACTCTAAGGTGGCGTCGGCGTGGG 40

SEQ ID NO: 167
SEQUENCE LENGTH: 40 base pairs

SEQUENCE TYPE: nucleic acid
TOPOLOGY: linear
MOLECULE TYPE: cDNA
ORIGINAL SOURCE
ORGANISM: Hepatitis C virus

GCGAATTCGCTAGCTCAACAACCGAACCAGTTGCCCTGCG 40

SEQ ID NO: 168
SEQUENCE LENGTH: 40 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCGCTAGCTCAAAGCTCTGATCTATCCCTGTCCT 40

SEQ ID NO: 169
SEQUENCE LENGTH: 32 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGTGGTTGTGGATGATGCTGCTG 32

SEQ ID NO: :170
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

TGGTTGTGGATGATGCTGCTG 21

SEQ ID NO: 171
SEQUENCE LENGTH: 44 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCATCACCTCCGGGCGGAGACNGGNAGNCC 44
N: inosine

SEQ ID NO: 172
SEQUENCE LENGTH: 31 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGGGCAACGAGNTNCTNCTNGG 31
N: inosine

SEQ ID NO: 173
SEQUENCE LENGTH: 20 base pairs
SEQUENCE TYPE: nucleic acid
TOPOLOGY: linear
MOLECULE TYPE: cDNA
ORIGINAL SOURCE
ORGANISM: Hepatitis C virus

GGCAACGAGNTNCTNCTNGG 20
N: inosine

SEQ ID NO: 174
SEQUENCE LENGTH: 41 base pairs
SEQUENCE TYPE: nucleic acid
TOPOLOGY: linear

MOLECULE TYPE: cDNA
ORIGINAL SOURCE
ORGANISM: Hepatitis C virus

GCGAATTCAGATCTTCATCACTTCAGCCGTATGAGACACTT 41

SEQ ID NO: 175
SEQUENCE LENGTH: 31 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGCTGTCGCCCGGGCCCATCTC 31

SEQ ID NO: 176
SEQUENCE LENGTH: 20 base pairs
SEQUENCE TYPE: nucleic acid
TOPOLOGY: linear
MOLECULE TYPE: cDNA
ORIGINAL SOURCE
ORGANISM: Hepatitis C virus

CTGTCGCCCGGGCCCATCTC 20

SEQ ID NO: 177
SEQUENCE LENGTH: 41 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCATCAACATGTGTTGCAGTCGATCAC 41

SEQ ID NO: 178

SEQUENCE LENGTH: 32 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGGGCTATACCGGNGACTTNGAC 32
N: inosine

SEQ ID NO: 179
SEQUENCE LENGTH: 21 base pairs
SEQUENCE TYPE: nucleic acid
TOPOLOGY: linear
MOLECULE TYPE: cDNA
ORIGINAL SOURCE
ORGANISM: Hepatitis C virus

GGCTATACCGGNGACTTNGAC 21
N: inosine

SEQ ID NO: 180
SEQUENCE LENGTH: 35 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCAGTGCTTCGCCCAGAAGGT 35

SEQ ID NO: 181
SEQUENCE LENGTH: 29 base pairs
SEQUENCE TYPE: nucleic acid
TOPOLOGY: linear
MOLECULE TYPE: cDNA
ORIGINAL SOURCE

ORGANISM: Hepatitis C virus

GCGCTAGCATGTGGTTGTGGATGATGCTG 29

SEQ ID NO: 182
SEQUENCE LENGTH: 38 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCGCTAGCTCACAGCCGGTTCATCCACTGCAC 38

SEQ ID NO: 183
SEQUENCE LENGTH: 32 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGCAGCGTGGGTACAAGGGGGTT 32

SEQ ID NO: 184
SEQUENCE LENGTH: 47 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCATCAGAGCTGTGACCCAACCGTATATTGGTT 47

SEQ ID NO: 185
SEQUENCE LENGTH: 33 base pairs
SEQUENCE TYPE: nucleic acid

TOPOLOGY: linear
MOLECULE TYPE: cDNA
ORIGINAL SOURCE
ORGANISM: Hepatitis C virus

GCGCTAGCATGGGGTACAAGGGGGTTTGGCGGG 33

SEQ ID NO: 186
SEQUENCE LENGTH: 32 base pairs
SEQUENCE TYPE: nucleic acid
TOPOLOGY: linear
MOLECULE TYPE: cDNA
ORIGINAL SOURCE
ORGANISM: Hepatitis C virus

GCGCTAGCTCATCGGTTGGGGAGCAGGTAGAT 32

SEQ ID NO: 187
SEQUENCE LENGTH: 26 base pairs
SEQUENCE TYPE: nucleic acid
TOPOLOGY: linear
MOLECULE TYPE: cDNA
ORIGINAL SOURCE
ORGANISM: Hepatitis C virus

GGATCCCCCCAAGCTTGGGGGAATTC 26

SEQ ID NO:188
SEQUENCE LENGTH: 31 base pairs
SEQUENCE TYPE: nucleic acid
TOPOLOGY: linear
MOLECULE TYPE: cDNA
ORIGINAL SOURCE
ORGANISM: Hepatitis C virus

AGCTTACTAGTTAATACGACTCACTATAGGG 31

SEQ ID NO:189
SEQUENCE LENGTH: 33 base pairs
SEQUENCE TYPE: nucleic acid
TOPOLOGY: linear
MOLECULE TYPE: cDNA
ORIGINAL SOURCE
ORGANISM: Hepatitis C virus

CTGGCACCCTATAGTGAGTCGTATTAACTAGTA 33

SEQ ID NO:190
SEQUENCE LENGTH: 44 base pairs
SEQUENCE TYPE: nucleic acid
TOPOLOGY: linear
MOLECULE TYPE: cDNA
ORIGINAL SOURCE
ORGANISM: Hepatitis C virus

TGCCAGCCCCCTGATGGGGGCGACACTCCACCATAGATCACTCC 44

SEQ ID NO:191
SEQUENCE LENGTH: 45 base pairs
SEQUENCE TYPE: nucleic acid
TOPOLOGY: linear
MOLECULE TYPE: cDNA
ORIGINAL SOURCE
ORGANISM: Hepatitis C virus

TCACAGGGGAGTGATCTATGGTGGAGTGTCGCCCCCATCAGGGGG 45

SEQ ID NO:192
SEQUENCE LENGTH: 40 base pairs
SEQUENCE TYPE: nucleic acid
TOPOLOGY: linear
MOLECULE TYPE: cDNA
ORIGINAL SOURCE
ORGANISM: Hepatitis C virus

CCTGTGAGGAACTACTGTCTTCACGCAGAAAGCGTCTAGC 40

SEQ ID NO:193
SEQUENCE LENGTH: 37 base pairs
SEQUENCE TYPE: nucleic acid
TOPOLOGY: linear
MOLECULE TYPE: cDNA
ORIGINAL SOURCE
ORGANISM: Hepatitis C virus

CATGGCTAGACGCTTTCTGCGTGAAGACAGTAGTTCC 37

SEQ ID NO:194
SEQUENCE LENGTH: 33 base pairs
SEQUENCE TYPE: nucleic acid
TOPOLOGY: linear
MOLECULE TYPE: cDNA
ORIGINAL SOURCE
ORGANISM: Hepatitis C virus

GCAAGCTTATGCTGCTGTCGCCCGGGCCCATCT 33

SEQ ID NO:195
SEQUENCE LENGTH: 38 base pairs
SEQUENCE TYPE: nucleic acid
TOPOLOGY: linear
MOLECULE TYPE: cDNA
ORIGINAL SOURCE
ORGANISM: Hepatitis C virus

GCGAATTCAGATCTTCATCATGTGTTGCAGTCGATCAC 38

## Claims

1. An isolated gene encoding a polypeptide originated from hepatitis C virus, wherein said polypeptide has an amino acid sequence of SEQ ID NO 101.

2. An isolated gene encoding a polypeptide originated from hepatitis C virus, wherein said polypeptide has an amino acid sequence of SEQ ID NO 102.

3. An isolated DNA originated from hepatitis C virus, wherein said DNA has a base sequence of SEQ ID NO 101.

4. An isolated DNA originated from hepatitis C virus, wherein said DNA has a base sequence of SEQ ID NO 102.

5. A polypeptide which comprises 115 amino acids from No. 1 to No. 115 of amino acid sequence of SEQ ID NO 3 or 7.

6. An isolated DNA which encodes a polypeptide of Claim 127.

7. A polypeptide of 10 to 25 amino acid residues derived from amino acid sequence of SEQ ID NO 31 or 32 , wherein said polypeptide comprises at least 6 amino acids from No. 182 to No. 187 of amino acid sequence of SEQ ID NO 31 or 32.

8. A polypeptide of 10 to 25 amino acid residues derived from amino acid sequence of SEQ ID NO 31 or 32 , wherein said polypeptide comprises at least 8 amino acids from Nos. 202 to 209 of amino acid sequence of SEQ ID NO 31 or 32.

9. A polypeptide which comprises 106 amino acids from No. 109 to No. 214 of amino acid sequence of SEQ ID NO 31 or 32.

10. A polypeptide which comprises 92 amino acids from No. 233 to No. 324 of amino acid sequence of SEQ ID NO 31 or 32.

11. A polypeptide of 10 to 25 amino acid residues derived from amino acid sequence of SEQ ID NO 31 or 32 , wherein said polypeptide comprises at least 5 amino acids from No. 252 to No. 256 of amino acid sequence of SEQ ID NO 31 or 32.

12. A polypeptide of 10 to 25 amino acid residues derived from amino acid sequence of SEQ ID NO 31 or 32 , wherein said polypeptide comprises at least 7 amino acids from No. 273 to No. 279 of amino acid sequence of SEQ ID NO 31 or 32.

13. A polypeptide of 10 to 25 amino acid residues derived from amino acid sequence of SEQ ID NO 31 or 32 , wherein said polypeptide comprises at least 7 amino acids from No. 136 to No. 142 of amino acid sequence of SEQ ID NO 31 or 32.

14. A polypeptide of 17 to 25 amino acid residues derived from amino acid sequence of SEQ ID NO 31 or 32 , wherein said polypeptide comprises at least 17 amino acids from No. 53 to No. 69 of amino acid sequence of SEQ ID NO 31 or 32.

15. A polypeptide which comprises all or 266 amino acids from No. 461 to No. 726 of amino acid sequence of SEQ ID NO 43.

16. A polypeptide which comprises all or 42 amino acids from No. 963 to No. 1004 of amino acid sequence of SEQ ID NO 43.

17. A polypeptide which comprises all or 45 amino acids from No. 283 to No. 327 of amino acid sequence of SEQ ID NO 43.

18. A polypeptide which comprises all or 74 amino acids from No. 477 to No. 550 of amino acid sequence of SEQ ID NO 43.

19. A polypeptide which comprises 61 amino acids from No. 215 to No. 275 of amino acid sequence of SEQ ID NO 43.

**20.** A polypeptide which comprises all or 74 amino acids from No. 413 to No. 486 of amino acid sequence of SEQ ID NO 75.

**21.** A polypeptide which comprises all or 997 amino acids from No. 415 to No. 1411 of amino acid sequence of SEQ ID NO 75.

**22.** A polypeptide which comprises all or 19 amino acids from No. 247 to No. 265 of amino acid sequence of SEQ ID NO 75.

**23.** A polypeptide which comprises all or 74 amino acids from No. 655 to No. 728 of amino acid sequence of SEQ ID NO 75.

**24.** A polypeptide which comprises all or 54 amino acids from No. 763 to No. 816 of amino acid sequence of SEQ ID NO 75.

**25.** A polypeptide shown by at least 20 amino acid residues from No. 324 to No. 343 of amino acid sequence of SEQ ID NO 75, wherein said polypeptide comprises at least 8 amino acids.

**26.** A polypeptide which comprises all or 98 amino acids from No. 858 to No. 955 of amino acid sequence of SEQ ID NO 75.

**27.** A polypeptide of 14 to 25 amino acid residues derived from amino acid sequence of SEQ ID NO 75 , wherein said polypeptide comprises at least 14 amino acids from No. 356 to No. 369 of amino acid sequence of SEQ ID NO 75.

**28.** A polypeptide which comprises all or 92 amino acids from No. 1009 to No. 1100 of amino acid sequence of SEQ ID NO 75.

**29.** A polypeptide which comprises all or 66 amino acids from No. 1160 to No. 1225 of amino acid sequence of SEQ ID NO 75.

**30.** A polypeptide of 18 to 25 amino acid residues derived from amino acid sequence of SEQ ID NO 75 , wherein said polypeptide comprises at least 18 amino acids from No. 584 to No. 601 of amino acid sequence of SEQ ID NO 75.

**31.** A polypeptide which comprises 42 amino acids from No. 615 to No. 656 of amino acid sequence of SEQ ID NO 75.

**32.** A polypeptide of 11 to 25 amino acid residues derived from amino acid sequence of SEQ ID NO 75 , wherein said polypeptide comprises at least 11 amino acids from No. 326 to No. 337 of amino acid sequence of SEQ ID NO 75.

**33.** A single-stranded DNA fragment or an antisense DNA fragment thereof which contains at least 15 nucleotides selected from 317 nucleotides from No. 1 to No. 317 of SEQ ID NO 1, 9, 11 or 12.

**34.** The DNA fragment of Claim 221 comprising 16 to 30 base pairs.

**35.** The DNA fragment of Claim 221 comprising 17 to 23 base pairs.

**36.** The use of a DNA and/or a polypeptide as claimed in any of the preceding claims for the preparation of a vaccine against hepatitis C virus.

**37.** The use of a DNA and/or a polypeptide as claimed in many of the preceding claims for the serodiagnosis of hepatitis C related diseases.

**38.** The use of a DNA as claimed in any of the preceding claims for a in vitro and/or in vivo screening system for a substance capable of specifically suppressing or controlling a proteolytic processing of a precursor protein of hepatitis C virus.